(19) Europäisches Patentamt
European Patent Office
Office européen des brevets

(11) EP 2 386 565 A2

(12) EUROPEAN PATENT APPLICATION

(43) Date of publication:
**16.11.2011 Bulletin 2011/46**

(51) Int Cl.:
***C07K 4/00*** *(2006.01)*

(21) Application number: **10012546.7**

(22) Date of filing: **12.01.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **12.01.1999 US 229071**
**17.03.1999 US 271192**
**01.12.1999 US 452406**

(62) Document number(s) of the earlier application(s) in accordance with Art. 76 EPC:
**00904325.8 / 1 141 011**

(71) Applicant: **Cambridge Enterprise Ltd.**
**Cambridge**
**Cambridgeshire CB2 1TN (GB)**

(72) Inventors:
- **Grainger, David J.**
  **Duxford,**
  **Cambridge CB2 4RA (GB)**
- **Tatalick, Lauren Marie**
  **Redmond, WA 98053 (US)**
- **Kanaly, Suzanne T.**
  **Seattle, WA 98126 (US)**

(74) Representative: **Roberts, Michael Austin**
**Reddie & Grose**
**16 Theobalds Road**
**London**
**WC1X 8PL (GB)**

Remarks:
This application was filed on 30-09-2010 as a divisional application to the application mentioned under INID code 62.

(54) **Compounds and methods to inhibit or augment an inflammatory response**

(57) Isolated and purified chemokine peptides, variants, and derivatives thereof, as well as chemokine peptide analogs, are provided.



Printed by Jouve, 75001 PARIS (FR)

SEQUENCE LISTING

<110> Cambridge Enterprise Limited

<120> Compounds and methods to inhibit or augment an inflammatory response

<130> P/52583.EP02/MAR

<140> Divisional of EP 00904325.8
<141> 2000-01-12

<150> PCT/US00/00821
<151> 2000-01-12

<150> US 09/452,406
<151> 1999-12-01

<150> US 09/271,192
<151> 1999-03-17

<150> US 09/229,071
<151> 1999-01-12

<160> 104

<170> FastSEQ for Windows Version 4.0

<210> 1
<211> 12
<212> PRT
<213> Homo sapiens

<400> 1
Glu Ile Cys Ala Asp Pro Lys Gln Lys Trp Val Gln
1 5 10

<210> 2
<211> 13
<212> PRT

<213> Homo sapiens

<400> 2

Ala Gln Pro Asp Ala Ile Asn Ala Pro Val Thr Cys Cys
1 5 10

<210> 3
<211> 15
<212> PRT
<213> Homo sapiens

<400> 3

Ser Tyr Arg Arg Ile Thr Ser Ser Lys Cys Pro Lys Glu Ala Val
1 5 10 15

<210> 4
<211> 15
<212> PRT
<213> Homo sapiens

<400> 4

His Leu Lys Ile Leu Asn Thr Pro Asn Cys Ala Leu Gln Ile Val
1 5 10 15

<210> 5
<211> 14
<212> PRT
<213> Homo sapiens

<400> 5

Asp Tyr Phe Glu Thr Ser Ser Gln Cys Ser Lys Pro Gly Val
1 5 10

<210> 6
<211> 16
<212> PRT
<213> Homo sapiens

<400> 6

Glu Leu Arg Val Ile Glu Ser Gly Pro His Cys Ala Asn Thr Glu Ile
1 5 10 15

<210> 7
<211> 10
<212> PRT
<213> Homo sapiens

<400> 7
Cys Ala Asp Pro Lys Gln Lys Trp Val Gln
1 5 10

<210> 8
<211> 6
<212> PRT
<213> Homo sapiens

<400> 8
Glu Ile Cys Ala Asp Pro
1 5

<210> 9
<211> 6
<212> PRT
<213> Homo sapiens

<400> 9
Lys Gln Lys Trp Val Gln
1 5

<210> 10
<211> 12
<212> PRT
<213> Artificial Sequence

<220>
<223> A chemokine peptide variant

<400> 10

Glu Ile Cys Leu Asp Pro Lys Gln Lys Trp Val Gln
1 5 10

<210> 11
<211> 12
<212> PRT
<213> Artificial Sequence

<220>
<223> A chemokine peptide variant

<400> 11

Glu Ile Cys Ala Asp Pro Ser Gln Lys Trp Val Gln
1 5 10

<210> 12
<211> 12
<212> PRT
<213> Artificial Sequence

<220>
<223> A chemokine peptide variant

<400> 12

Glu Ile Cys Ala Asp Pro Ser Glu Glu Trp Val Gln
1 5 10

<210> 13
<211> 12
<212> PRT
<213> Artificial Sequence

<220>
<223> A chemokine peptide variant

<400> 13

Glu Ile Cys Ala Asp Pro Lys Gln Lys Trp Ile Gln

1 5 10

<210> 14
<211> 12
<212> PRT
<213> Artificial Sequence

<220>
<223> A chemokine peptide variant

<400> 14
Glu Ile Cys Leu Asp Pro Lys Gln Lys Trp Ile Gln
1 5 10

<210> 15
<211> 12
<212> PRT
<213> Homo sapiens

<400> 15
Cys Pro Ser Leu Glu Asp Ser Phe Ile Gln Val Ala
1 5 10

<210> 16
<211> 99
<212> PRT
<213> Homo sapiens

<400> 16
Met Lys Val Ser Ala Ala Leu Leu Cys Leu Leu Leu Ile Ala Ala Thr
1 5 10 15
Phe Ile Pro Gln Gly Leu Ala Gln Pro Asp Ala Ile Asn Ala Pro Val
20 25 30
Thr Cys Cys Tyr Asn Phe Thr Asn Arg Lys Ile Ser Val Gln Arg Leu
35 40 45
Ala Ser Tyr Arg Arg Ile Thr Ser Ser Lys Cys Pro Lys Glu Ala Val
50 55 60
Ile Phe Lys Thr Ile Val Ala Lys Glu Ile Cys Ala Asp Pro Lys Gln

65 70 75 80

Lys Trp Val Gln Asp Ser Met Asp His Leu Asp Lys Gln Thr Gln Thr

85 90 95

Pro Lys Thr

<210> 17

<211> 77

<212> PRT

<213> Homo sapiens

<400> 17

Ala Gln Pro Asp Ser Val Ser Ile Pro Ile Thr Cys Cys Phe Asn Val

1 5 10 15

Ile Asn Arg Lys Ile Pro Ile Gln Arg Leu Glu Ser Tyr Thr Arg Ile

20 25 30

Thr Asn Ile Gln Cys Pro Lys Glu Ala Val Ile Phe Lys Thr Lys Arg

35 40 45

Gly Lys Glu Val Cys Ala Asp Pro Lys Glu Arg Trp Val Arg Asp Ser

50 55 60

Met Lys His Leu Asp Gln Ile Phe Gln Asn Leu Lys Pro

65 70 75

<210> 18

<211> 99

<212> PRT

<213> Homo sapiens

<400> 18

Met Lys Ala Ser Ala Ala Leu Leu Cys Leu Leu Leu Thr Ala Ala Ala

1 5 10 15

Phe Ser Pro Gln Gly Leu Ala Gln Pro Val Gly Ile Asn Thr Ser Thr

20 25 30

Thr Cys Cys Tyr Arg Phe Ile Asn Lys Lys Ile Pro Lys Gln Arg Leu

35 40 45

Glu Ser Tyr Arg Arg Thr Thr Ser Ser His Cys Pro Arg Glu Ala Val

50 55 60

Ile Phe Lys Thr Lys Leu Asp Lys Glu Ile Cys Ala Asp Pro Thr Gln

65 70 75 80

Lys Trp Val Gln Asp Phe Met Lys His Leu Asp Lys Lys Thr Gln Thr
85 90 95
Pro Lys Leu

<210> 19
<211> 92
<212> PRT
<213> Homo sapiens

<400> 19
Met Gln Val Ser Thr Ala Ala Leu Ala Val Leu Leu Cys Thr Met Ala
1 5 10 15
Leu Cys Asn Gln Phe Ser Ala Ser Leu Ala Ala Asp Thr Pro Thr Ala
20 25 30
Cys Cys Phe Ser Tyr Thr Ser Arg Gln Ile Pro Gln Asn Phe Ile Ala
35 40 45
Asp Tyr Phe Glu Thr Ser Ser Gln Cys Ser Lys Pro Gly Val Ile Phe
50 55 60
Leu Thr Lys Arg Ser Arg Gln Val Cys Ala Asp Pro Ser Glu Glu Trp
65 70 75 80
Val Gln Lys Tyr Val Ser Asp Leu Glu Leu Ser Ala
85 90

<210> 20
<211> 92
<212> PRT
<213> Homo sapiens

<400> 20
Met Lys Leu Cys Val Thr Val Leu Ser Leu Leu Met Leu Val Ala Ala
1 5 10 15
Phe Cys Ser Pro Ala Leu Ser Ala Pro Met Gly Ser Asp Pro Pro Thr
20 25 30
Ala Cys Cys Phe Ser Tyr Thr Ala Arg Lys Leu Pro Arg Asn Phe Val
35 40 45
Val Asp Tyr Tyr Glu Thr Ser Ser Leu Cys Ser Gln Pro Ala Val Val
50 55 60

Phe Gln Thr Lys Arg Ser Lys Gln Val Cys Ala Asp Pro Ser Glu Ser
65 70 75 80
Trp Val Gln Glu Tyr Val Tyr Asp Leu Glu Leu Asn
85 90

<210> 21
<211> 91
<212> PRT
<213> Homo sapiens

<400> 21
Met Lys Val Ser Ala Ala Arg Leu Ala Val Ile Leu Ile Ala Thr Ala
1 5 10 15
Leu Cys Ala Pro Ala Ser Ala Ser Pro Tyr Ser Ser Asp Thr Thr Pro
20 25 30
Cys Cys Phe Ala Tyr Ile Ala Arg Pro Leu Pro Arg Ala His Ile Lys
35 40 45
Glu Tyr Phe Tyr Thr Ser Gly Lys Cys Ser Asn Pro Ala Val Val Phe
50 55 60
Val Thr Arg Lys Asn Arg Gln Val Cys Ala Asn Pro Glu Lys Lys Trp
65 70 75 80
Val Arg Glu Tyr Ile Asn Ser Leu Glu Met Ser
85 90

<210> 22
<211> 89
<212> PRT
<213> Homo sapiens

<400> 22
Met Asn Ala Lys Val Val Val Val Leu Val Leu Val Leu Thr Ala Leu
1 5 10 15
Cys Leu Ser Asp Gly Lys Pro Val Ser Leu Ser Tyr Arg Cys Pro Cys
20 25 30
Arg Phe Phe Glu Ser His Val Ala Arg Ala Asn Val Lys His Leu Lys
35 40 45
Ile Leu Asn Thr Pro Asn Cys Ala Leu Gln Ile Val Ala Arg Leu Lys
50 55 60

Asn Asn Asn Arg Gln Val Cys Ile Asp Pro Lys Leu Lys Trp Ile Gln
65 70 75 80
Glu Tyr Leu Glu Lys Ala Leu Asn Lys
85

<210> 23
<211> 99
<212> PRT
<213> Homo sapiens

<400> 23
Met Thr Ser Lys Leu Ala Val Ala Leu Leu Ala Ala Phe Leu Ile Ser
1 5 10 15
Ala Ala Leu Cys Glu Gly Ala Val Leu Pro Arg Ser Ala Lys Glu Leu
20 25 30
Arg Cys Gln Cys Ile Lys Thr Tyr Ser Lys Pro Phe His Pro Lys Phe
35 40 45
Ile Lys Glu Leu Arg Val Ile Glu Ser Gly Pro His Cys Ala Asn Thr
50 55 60
Glu Ile Ile Val Lys Leu Ser Asp Gly Arg Glu Leu Cys Leu Asp Pro
65 70 75 80
Lys Glu Asn Trp Val Gln Arg Val Val Glu Lys Phe Leu Lys Arg Ala
85 90 95
Glu Asn Ser

<210> 24
<211> 107
<212> PRT
<213> Homo sapiens

<400> 24
Met Ala Arg Ala Ala Leu Ser Ala Ala Pro Ser Asn Pro Arg Leu Leu
1 5 10 15
Arg Val Ala Leu Leu Leu Leu Leu Leu Val Ala Ala Gly Arg Arg Ala
20 25 30
Ala Gly Ala Ser Val Ala Thr Glu Leu Arg Cys Gln Cys Leu Gln Thr
35 40 45

Leu Gln Gly Ile His Pro Lys Asn Ile Gln Ser Val Asn Val Lys Ser
    50                  55                  60
Pro Gly Pro His Cys Ala Gln Thr Glu Val Ile Ala Thr Leu Lys Asn
65                  70                  75                  80
Gly Arg Lys Ala Cys Leu Asn Pro Ala Ser Pro Ile Val Lys Lys Ile
                85                  90                  95
Ile Glu Lys Met Leu Asn Ser Asp Lys Ser Asn
            100                 105

<210> 25
<211> 97
<212> PRT
<213> Homo sapiens

<400> 25
Met Lys Val Ser Ala Ala Leu Leu Trp Leu Leu Leu Ile Ala Ala Ala
 1               5                  10                  15
Phe Ser Pro Gln Gly Leu Ala Gly Pro Ala Ser Val Pro Thr Thr Cys
            20                  25                  30
Cys Phe Asn Leu Ala Asn Arg Lys Ile Pro Leu Gln Arg Leu Glu Ser
        35                  40                  45
Tyr Arg Arg Ile Thr Ser Gly Lys Cys Pro Gln Lys Ala Val Ile Phe
    50                  55                  60
Lys Thr Lys Leu Ala Lys Asp Ile Cys Ala Asp Pro Lys Lys Lys Trp
65                  70                  75                  80
Val Gln Asp Ser Met Lys Tyr Leu Asp Gln Lys Ser Pro Thr Pro Lys
                85                  90                  95
Pro

<210> 26
<211> 148
<212> PRT
<213> Mus musculus

<400> 26
Met Gln Val Pro Val Met Leu Leu Gly Leu Leu Phe Thr Val Ala Gly
 1               5                  10                  15

Trp Ser Ile His Val Leu Ala Gln Pro Asp Ala Val Asn Ala Pro Leu
20 25 30
Thr Cys Cys Tyr Ser Phe Thr Ser Lys Met Ile Pro Met Ser Arg Leu
35 40 45
Glu Ser Tyr Lys Arg Ile Thr Ser Ser Arg Cys Pro Lys Glu Ala Val
50 55 60
Val Phe Val Thr Lys Leu Lys Arg Glu Val Cys Ala Asp Pro Lys Lys
65 70 75 80
Glu Trp Val Gln Thr Tyr Ile Lys Asn Leu Asp Arg Asn Gln Met Arg
85 90 95
Ser Glu Pro Thr Thr Leu Phe Lys Thr Ala Ser Ala Leu Arg Ser Ser
100 105 110
Ala Pro Leu Asn Val Lys Leu Thr Arg Lys Ser Glu Ala Asn Ala Ser
115 120 125
Thr Thr Phe Ser Thr Thr Thr Ser Ser Thr Ser Val Gly Val Thr Ser
130 135 140
Val Thr Val Asn
145

<210> 27
<211> 10
<212> PRT
<213> Artificial Sequence

<220>
<223> A chemokine peptide variant

<400> 27
Cys Leu Asp Pro Lys Lys Glu Trp Ile Gln
1 5 10

<210> 28
<211> 825
<212> DNA
<213> Homo sapiens

<220>
<221> CDS

<222> (34)...(327)

<400> 28

```
acattgtgaa atctccaact cttaaccttc aac atg aaa gtc tct gca gtg ctt       54
                                     Met Lys Val Ser Ala Val Leu
                                      1               5

ctg tgc ctg ctg ctc atg aca gca gct ttc aac ccc cag gga ctt gct       102
Leu Cys Leu Leu Leu Met Thr Ala Ala Phe Asn Pro Gln Gly Leu Ala
        10                  15                  20

cag cca gat gca ctc aac gtc cca tct act tgc tgc ttc aca ttt agc       150
Gln Pro Asp Ala Leu Asn Val Pro Ser Thr Cys Cys Phe Thr Phe Ser
    25                  30                  35

agt aag aag atc tcc ttg cag agg ctg aag agc tat gtg atc acc acc       198
Ser Lys Lys Ile Ser Leu Gln Arg Leu Lys Ser Tyr Val Ile Thr Thr
40                  45                  50                  55

agc agg tgt ccc cag aag gct gtc atc ttc aga acc aaa ctg ggc aag       246
Ser Arg Cys Pro Gln Lys Ala Val Ile Phe Arg Thr Lys Leu Gly Lys
                60                  65                  70

gag atc tgt gct gac cca aag gag aag tgg gtc cag aat tat atg aaa       294
Glu Ile Cys Ala Asp Pro Lys Glu Lys Trp Val Gln Asn Tyr Met Lys
            75                  80                  85

cac ctg ggc cgg aaa gct cac acc ctg aag act tgaactctgc taccccctact    347
His Leu Gly Arg Lys Ala His Thr Leu Lys Thr
        90                  95

gaaatcaagc tggagtacgt gaaatgactt ttccattctc ctctggcctc ctcttctatg    407
ctttggaata cttctaccat aattttcaaa taggatgcat tcggttttgt gattcaaaat    467
gtactatgtg ttaagtaata ttggctatta tttgacttgt tgctggtttg gagtttattt    527
gagtattgct gatcttttct aaagcaaggc cttgagcaag taggttgctg tctctaagcc    587
cccttccctt ccactatgag ctgctggcag tgggttgtat tcggttccca ggggttgaga    647
gcatgcctgt gggagtcatg gacatgaagg gatgctgcaa tgtaggaagg agagctcttt    707
gtgaatgtga ggttgttgct aaattattgt ttattgtgga aagatgaatg caatagtagg    767
```

actgctgaca ttttgcagaa aatacatttt atttaaaatc tcctaaaaaa aaaaaaa 825

<210> 29
<211> 3524
<212> DNA
<213> Homo sapiens

<220>
<221> CDS
<222> (80)...(358)

<400> 29
tctccgtcag ccgcattgcc cgctcggcgt ccggcccccg acccgtgctc gtccgcccgc 60
ccgcccgccc gcccgcgcc atg aac gcc aag gtc gtg gtc gtg ctg gtc ctc 112
Met Asn Ala Lys Val Val Val Val Leu Val Leu
1 5 10

gtg ctg acc gcg ctc tgc ctc agc gac ggg aag ccc gtc agc ctg agc 160
Val Leu Thr Ala Leu Cys Leu Ser Asp Gly Lys Pro Val Ser Leu Ser
15 20 25

tac aga tgc cca tgc cga ttc ttc gaa agc cat gtt gcc aga gcc aac 208
Tyr Arg Cys Pro Cys Arg Phe Phe Glu Ser His Val Ala Arg Ala Asn
30 35 40

gtc aag cat ctc aaa att ctc aac act cca aac tgt gcc ctt cag att 256
Val Lys His Leu Lys Ile Leu Asn Thr Pro Asn Cys Ala Leu Gln Ile
45 50 55

gta gcc cgg ctg aag aac aac aac aga caa gtg tgc att gac ccg aag 304
Val Ala Arg Leu Lys Asn Asn Asn Arg Gln Val Cys Ile Asp Pro Lys
60 65 70 75

cta aag tgg att cag gag tac ctg gag aaa gct tta aac aag agg ttc 352
Leu Lys Trp Ile Gln Glu Tyr Leu Glu Lys Ala Leu Asn Lys Arg Phe
80 85 90

aag atg tgagagggtc agacgcctga ggaaccctta cagtaggagc ccagctctga 408

Lys Met

aaccagtgtt agggaagggc ctgccacagc ctcccctgcc agggcagggc cccaggcatt 468
gccaagggct ttgttttgca cactttgcca tattttcacc atttgattat gtagcaaaat 528
acatgacatt tatttttcat ttagtttgat tattcagtgt cactggcgac acgtagcagc 588
ttagactaag gccattattg tacttgcctt attagagtgt ctttccacgg agccactcct 648
ctgactcagg gctcctgggt tttgtattct ctgagctgtg caggtgggga gactgggctg 708
agggagcctg gccccatggt cagccctagg gtggagagcc accaagaggg acgcctgggg 768
gtgccaggac cagtcaacct gggcaaagcc tagtgaaggc ttctctctgt gggatgggat 828
ggtggagggc cacatgggag gctcaccccc ttctccatcc acatgggagc cgggtctgcc 888
tcttctggga gggcagcagg gctaccctga gctgaggcag cagtgtgagg ccagggcaga 948
gtgagaccca gccctcatcc cgagcacctc cacatcctcc acgttctgct catcattctc 1008
tgtctcatcc atcatcatgt gtgtccacga ctgtctccat ggccccgcaa aaggactctc 1068
aggaccaaag ctttcatgta aactgtgcac caagcaggaa atgaaaatgt cttgtgttac 1128
ctgaaaacac tgtgcacatc tgtgtcttgt gtggaatatt gtccattgtc caatcctatg 1188
tttttgttca aagccagcgt cctcctctgt gaccaatgtc ttgatgcatg cactgttccc 1248
cctgtgcagc cgctgagcga ggagatgctc cttgggccct ttgagtgcag tcctgatcag 1308
agccgtggtc ctttggggtg aactaccttg gttcccccac tgatcacaaa aacatggtgg 1368
gtccatgggc agagcccaag ggaattcggt gtgcaccagg gttgacccca gaggattgct 1428
gccccatcag tgctccctca catgtcagta ccttcaaact agggccaagc ccagcactgc 1488
ttgaggaaaa caagcattca caacttgttt ttggttttta aaacccagtc cacaaaataa 1548
ccaatcctgg acatgaagat tctttcccaa ttcacatcta acctcatctt cttcaccatt 1608
tggcaatgcc atcatctcct gccttcctcc tgggccctct ctgctctgcg tgtcacctgt 1668
gcttcgggcc cttcccacag gacatttctc taagagaaca atgtgctatg tgaagagtaa 1728
gtcaacctgc ctgacatttg gagtgttccc ctcccactga gggcagtcga tagagctgta 1788
ttaagccact taaaatgttc acttttgaca aaggcaagca cttgtgggtt tttgttttgt 1848
ttttcattca gtcttacgaa tacttttgcc ctttgattaa agactccagt taaaaaaaat 1908
tttaatgaag aaagtggaaa acaaggaagt caaagcaagg aaactatgta acatgtagga 1968
agtaggaagt aaattatagt gatgtaatct tgaattgtaa ctgttcgtga atttaataat 2028
ctgtagggta attagtaaca tgtgttaagt attttcataa gtatttcaaa ttggagcttc 2088
atggcagaag gcaaacccat caacaaaaat tgtcccttaa acaaaaatta aaatcctcaa 2148
tccagctatg ttatattgaa aaaatagagc ctgagggatc tttactagtt ataaagatac 2208
agaactcttt caaaaccttt tgaaattaac ctctcactat accagtataa ttgagttttc 2268
agtggggcag tcattatcca ggtaatccaa gatattttaa aatctgtcac gtagaacttg 2328
gatgtacctg cccccaatcc atgaaccaag accattgaat tcttggttga ggaaacaaac 2388
atgaccctaa atcttgacta cagtcaggaa aggaatcatt tctatttctc ctccatggga 2448
gaaaatagat aagagtagaa actgcaggga aaattatttg cataacaatt cctctactaa 2508

caatcagctc cttcctggag actgcccagc taaagcaata tgcatttaaa tacagtcttc 2568
catttgcaag ggaaaagtct cttgtaatcc gaatctcttt ttgctttcga actgctagtc 2628
aagtgcgtcc acgagctgtt tactagggat ccctcatctg tccctccggg acctggtgct 2688
gcctctacct gacactccct tgggctccct gtaacctctt cagaggccct cgctgccagc 2748
tctgtatcag gacccagagg aaggggccag aggctcgttg actggctgtg tgttgggatt 2808
gagtctgtgc cacgtgtatg tgctgtggtg tgtccccctc tgtccaggca ctgagatacc 2868
agcgaggagg ctccagaggg cactctgctt gttattagag attacctcct gagaaaaaag 2928
cttccgcttg gagcagaggg gctgaatagc agaaggttgc acctccccca accttagatg 2988
ttctaagtct ttccattgga tctcattgga cccttccatg gtgtgatcgt ctgactggtg 3048
ttatcaccgt gggctccctg actgggagtt gatcgccttt cccaggtgct acacccttttt 3108
ccagctggat gagaatttga gtgctctgat ccctctacag agcttccctg actcattctg 3168
aaggagcccc attcctggga aatattccct agaaacttcc aaatccccta agcagaccac 3228
tgataaaacc atgtagaaaa tttgttattt tgcaacctcg ctggactctc agtctctgag 3288
cagtgaatga ttcagtgtta aatgtgatga atactgtatt ttgtattgtt tcaagtgcat 3348
ctcccagata atgtgaaaat ggtccaggag aaggccaatt cctatacgca gcgtgcttta 3408
aaaaataaat aagaaacaac tctttgagaa acaacaattt ctactttgaa gtcataccaa 3468
tgaaaaaatg tatatgcact tataattttc ctaataaagt tctgtactca aatgta 3524

<210> 30
<211> 4259
<212> DNA
<213> Mus musculus

<220>
<221> CDS
<222> (2070)...(2130)

<221> CDS
<222> (2669)...(2795)

<221> CDS
<222> (2990)...(3079)

<221> CDS
<222> (3491)...(3506)

<400> 30
gaattcggag gtctacctgc ctctgcctcc caagtactgg aattactctt acggcttact 60

```
tttcctcaag ggtcttcaaa gcacttctga ccatagggag cggactctta ggaaattcca    120
tatagattct tttatttcag tcatttgaca ttaactttat agtcataaat tttgatattt    180
ttttacagat ctgaatatgt ttgaagaaat gttcattttt cccctagcca caaaaatccc    240
atgacatgta taccatactt gccataacct gctataattc agtgcaaaat ggcctgttct    300
agtcaaaggg ctgctttcaa gtacgagtga ctatttactg gatacaatgt atgtatacta    360
cagtaacttt cattttagca tgaaattctg tgtaaaacaa gtccaattta aaccccttct    420
aggtgtttag ttccagaaac accaaagaaa catcaaagat aacttcagcc aagcagacac    480
aggcaagtct ggagcctgaa ccaaggatct gctctttgga gacactgcat aaccaagtgt    540
ttgagagcgt gggctacaga gatggagttt ctggattcct agagggttga atgagggaaa    600
actcttgact aacagatgct taacactacg caatagttac taaactcttt tctcctcagt    660
aagggactat atatccactt gctaactgtt ttttcttaac atcctcaaat ctgtttttcc    720
ttcacatctt gactgagaac ttgtacaata acaaaataaa ataaaactgt caccatgtat    780
ctttctttat tgtttaatgt aaaactgctc agcacctctt tccactctaa aattctgagg    840
ttcctacagc gcagcgtcta ttctgcagaa gcaggttggg agagactgct catggcgtcc    900
tctcacagta gccttctccc agcagaaggt tgcaaaacga aagtctcttg gcttgtctct    960
gcccccattc ccttctgtct gtccttccag attccacggc tgtcccgtga tttgctccct   1020
tgaaagctta gctctgttct agtccttgga ctacatcaat gtggattttg ctctctagaa   1080
atttctatct ttcccaagca gcaactccct tcctgttctg gtgacagctc ctgtttccca   1140
gttcccattg gtgggtagag gtttcactct tggcctctgt tgtcaagttt tgaaccggta   1200
cactccaggc tcctgtccca ttcccaggat gagtcacctc catccccttc atcactgtca   1260
cctctatgcg agatctatgt aacctcacct actcttccag gtcccaggta tctgctgcct   1320
tgggtgctct gcagagtgac atccacattc actttcatcc ttgaccctgt taccacacac   1380
tcacaaagaa gacaatcaaa gcatccctga gagaatcagc acggggcaac aattagcttt   1440
gcaattcctc ttgtaacttg taaacattag tgtgacactt ccggcttctg ttctgaatgc   1500
ctgctctaac tgttcactaa gttgtataga ctttggatct ggctaaattt ggcatgtgat   1560
ttttttttta aagtttattc caagtatttt ataataaagc ctatgaagta aaaagcaaaa   1620
ataaacagtg taaacacaac aaatgtaaaa accatggtta gaacctgact tagatatcag   1680
ctctgtgttt tatttatgag agaaggaaga tgagaattaa agccatttcc agacttattc   1740
tgcaaggcac tcatctgatt tctcaaacag ctcacgcttt ggaaagtgaa acctacctca   1800
ctcgttaaaa attaaaagga gcacaagagg ggagagggaa attccaagtt catgggtcac   1860
aataaacaca agcaatgccc tcggtttaca ggggacttcc ctcgggttgc ggagccttgc   1920
tgagtcatct ccaaagtcag ccaatcagga ctcagggagg gaaactcttt gcagataaat   1980
actcctcagc agccggcact cgagaagcgc ttcatccacc gctgagagac atcccgagcc   2040
aaccttccgg aagcctcccc atcagcacc atg aac cca agt gct gcc gtc att     2093
                                Met Asn Pro Ser Ala Ala Val Ile
                                1               5

ttc tgc ctc atc ctg ctg ggt ctg agt ggg act caa g gtaagggaca        2140
```

```
Phe Cys Leu Ile Leu Leu Gly Leu Ser Gly Thr Gln
    10                  15                  20

ccaaggccat ttaattaacg aagtcagaag tcagacgatt aagctcagtt ctaaacacag   2200
catgtattta agctttaatg tgtgtaccta taaagaagag ggaagcagga agaaatccct   2260
tcagcttgca gagtttatca taggctggtt gaagtcagag aaaaatagaa taaaagaaaa   2320
ggaacgaaga agggaagaaa gggagaaaag gaggagggag gaggaagaag gagggaagag   2380
agagtcagga gaaagggcga aagagtggga tggggtaagg catggatgcc tccttgcctg   2440
agcctaacca atactgtgag cagtgcataa atgcaggatt tcgtaactga caagttgcag   2500
atctctcttt accatgacca agatattcaa acactcagcc ctatgatacg atgggatgcg   2560
tctctccaca gatcagacag ggtctgctaa acactacctc atccatttta agtgcctaaa   2620
atgaaaccgt gtgctgacct tcctggctct ccccctctct tcctgcag gg atc cct     2676
                                                    Gly Ile Pro

ctc gca agg acg gtc cgc tgc aac tgc atc cat atc gat gac ggg cca     2724
Leu Ala Arg Thr Val Arg Cys Asn Cys Ile His Ile Asp Asp Gly Pro
    25                  30                  35

gtg aga atg agg gcc ata ggg aag ctt gaa atc atc cct gcg agc cta     2772
Val Arg Met Arg Ala Ile Gly Lys Leu Glu Ile Ile Pro Ala Ser Leu
40                  45                  50                  55

tcc tgc cca cgt gtt gag atc at gtgagtacaa gcccacctgc cgataaacgt     2825
Ser Cys Pro Arg Val Glu Ile Ile
                60

ccctcccgta accacacagt aaataagtga gggaaaccag gaaagatggg gacgggtctg   2885
tgactctaac taaggcacag tgcctgaact ctgacatgga cctgcagggc catcagctct   2945
gttggcctga cgttaatctg agtatctcac tcttatttct atag t gcc acg atg      2999
                                                   Ala Thr Met
                                                       65

aaa aag aat gat gag cag aga tgt ctg aat ccg gaa tct aag acc atc     3047
Lys Lys Asn Asp Glu Gln Arg Cys Leu Asn Pro Glu Ser Lys Thr Ile
            70                  75                  80

aag aat tta atg aaa gcg ttt agc caa aaa ag gtaggtttga tgttgctttt    3099
```

```
Lys Asn Leu Met Lys Ala Phe Ser Gln Lys Arg
        85                  90

tcaggaaatg gtggtctggg gagcagcgcc tgccctgggc tttgctgtgg gcatctgccc   3159
taaactcatg gcaccggcat gtgcctttgt ctctccattt acacagacac tgaggtgcct   3219
tcttaggtca tacattccta gtgtctagaa gtggagcagt tattatacct gtcacgggta   3279
aagctgccaa atgcccaccc ccccacttcc tcacttaaaa aaaaaaaacc aaaaacaaac   3339
aaacccattc tgtcccctca acccccaccc acccgtgacc catggagatt gtgtagcaga   3399
ggaaaatgca ccaggccttt tgccccaggg tctttgggtt ccaaagtgaa agcagagtct   3459
atccgctcaa tacagtttcc tcttcctaca g g tct aaa agg gct cct            3506
                                     Ser Lys Arg Ala Pro
                                         95

taactggaga gaagccacgc acacaccccg gtgctgtgat ggacagcaga gagcctgtct   3566
ctccatcact ccccttttacc cagtggatgg ctagtcctaa ttgcccttgg tcttctgaaa  3626
ggtgaccagc cgtggtcaca tcagctgcta ctcctcctgc aggatgatgg ttaagccatg   3686
gtcctgagac aaaagtaact gccgaagcaa gaattcttta agggctggtc tgagtcctca   3746
ctcaagtggc tgggatggct gtcctagctc tgtactgtaa gctatgtgga ggtgcgacgc   3806
ccttcaccat gtgccacgcc ccaggctgct ccccacaccc tccttgtcct ccctagctca   3866
ggctcgtcag ttctgagttt acctgagctc ttttatttca gatgtaagac tacaaattta   3926
agtttgtaag gacaaactta accaccatct tcccaagggg ttatcaagat actcagagga   3986
acctggaaat gtatgtgtaa atactattta atgaacgact gtacaaagta gaattcctag   4046
atgtattttt tgtatgcttt gcattgtata tggaagaact tgtgtcatca agtatgtatc   4106
aatgggtagt taaagtttat ttttaaaacc gtccaatacc ttttgtatta tgtaacattc   4166
aaaagacaat gtactgtatt gaaagtagta agagacccaa aatgtaataa agtaataata   4226
actgacatga aatggtcatg tgactgagaa ttc                                 4259

<210> 31
<211> 1081
<212> DNA
<213> Homo sapiens

<220>
<221> CDS
<222> (43)...(363)

<400> 31
ctctcctcct cgcacagccg ctcgaaccgc ctgctgagcc cc atg gcc cgc gcc        54
```

```
                                                Met Ala Arg Ala
                                                 1

acg ctc tcc gcc gcc ccc agc aat ccc cgg ctc ctg cgg gtg gcg ctg      102
Thr Leu Ser Ala Ala Pro Ser Asn Pro Arg Leu Leu Arg Val Ala Leu
 5                  10                  15                  20

ctg ctc ctg ctc ctg gtg gcc gcc agc cgg cgc gca gca gga gcg ccc      150
Leu Leu Leu Leu Leu Val Ala Ala Ser Arg Arg Ala Ala Gly Ala Pro
                25                  30                  35

ctg gcc act gaa ctg cgc tgc cag tgc ttg cag acc ctg cag gga att      198
Leu Ala Thr Glu Leu Arg Cys Gln Cys Leu Gln Thr Leu Gln Gly Ile
            40                  45                  50

cac ctc aag aac atc caa agt gtg aag gtg aag tcc ccc gga ccc cac      246
His Leu Lys Asn Ile Gln Ser Val Lys Val Lys Ser Pro Gly Pro His
        55                  60                  65

tgc gcc caa acc gaa gtc ata gcc aca ctc aag aat ggg cag aaa gct      294
Cys Ala Gln Thr Glu Val Ile Ala Thr Leu Lys Asn Gly Gln Lys Ala
    70                  75                  80

tgt ctc aac ccc gca tcg ccc atg gtt aag aaa atc atc gaa aag atg      342
Cys Leu Asn Pro Ala Ser Pro Met Val Lys Lys Ile Ile Glu Lys Met
 85                  90                  95                 100

ctg aaa aat ggc aaa tcc aac tgaccagaag gaaggaggaa gcttattggt         393
Leu Lys Asn Gly Lys Ser Asn
                105

ggctgttcct gaaggaggcc ctgcccttac aggaacagaa gaggaaagag agacacagct    453
gcagaggcca cctggattgc gcctaatgtg tttgagcatc acttaggaga agtcttctat    513
ttatttattt atttatttat ttgtttgttt tagaagattc tatgttaata ttttatgtgt    573
aaaataaggt tatgattgaa tctacttgca cactctccca ttatatttat tgtttatttt    633
aggtcaaacc caagttagtt caatcctgat tcatatttaa tttgaagata gaaggtttgc    693
agatattctc tagtcatttg ttaatatttc ttcgtgatga catatcacat gtcagccact    753
gtgatagagg ctgaggaatc caagaaaatg gccagtgaga tcaatgtgac ggcagggaaa    813
```

```
tgtatgtgtg tctattttgt aactgtaaag atgaatgtca gttgttattt attgaaatga    873
tttcacagtg tgtggtcaac atttctcatg ttgaagcttt aagaactaaa atgttctaaa    933
tatcccttgg acattttatg tctttcttgt aaggcatact gccttgttta atgttaatta    993
tgcagtgttt ccctctgtgt tagagcagag aggtttcgat atttattgat gttttcacaa   1053
agaacaggaa aataaaatat ttaaaaat                                      1081

<210> 32
<211> 1173
<212> DNA
<213> Homo sapiens

<220>
<221> CDS
<222> (107)...(448)

<400> 32
cggcacgagc acagtgctcc ggatcctcca atcttcgctc ctccaatctc cgctcctcca     60
cccagttcag gaacccgcga ccgctcgcag cgctctcttg accact atg agc ctc       115
                                                   Met Ser Leu
                                                    1

ctg tcc agc cgc gcg gcc cgt gtc ccc ggt cct tcg agc tcc ttg tgc      163
Leu Ser Ser Arg Ala Ala Arg Val Pro Gly Pro Ser Ser Ser Leu Cys
     5                  10                  15

gcg ctg ttg gtg ctg ctg ctg ctg ctg acg cag cca ggg ccc atc gcc      211
Ala Leu Leu Val Leu Leu Leu Leu Leu Thr Gln Pro Gly Pro Ile Ala
 20                  25                  30                  35

agc gct ggt cct gcc gct gct gtg ttg aga gag ctg cgt tgc gtt tgt      259
Ser Ala Gly Pro Ala Ala Ala Val Leu Arg Glu Leu Arg Cys Val Cys
                 40                  45                  50

tta cag acc acg cag gga gtt cat ccc aaa atg atc agt aat ctg caa      307
Leu Gln Thr Thr Gln Gly Val His Pro Lys Met Ile Ser Asn Leu Gln
             55                  60                  65

gtg ttc gcc ata ggc cca cag tgc tcc aag gtg gaa gtg gta gcc tcc      355
```

Val Phe Ala Ile Gly Pro Gln Cys Ser Lys Val Glu Val Val Ala Ser
70 75 80

ctg aag aac ggg aag gaa att tgt ctt gat cca gaa gcc cct ttt cta 403
Leu Lys Asn Gly Lys Glu Ile Cys Leu Asp Pro Glu Ala Pro Phe Leu
85 90 95

aag aaa gtc atc cag aaa att ttg gac ggt gga aac aag gaa aac 448
Lys Lys Val Ile Gln Lys Ile Leu Asp Gly Gly Asn Lys Glu Asn
100 105 110

tgattaagag aaatgagcac gcatggaaaa gtttcccagt ctacagcaga gaagttttct 508
ggaggtctct gaacccaggg aagacaagaa ggaaagattt tgttgttgtt tgtttatttg 568
gtttccccag tagttagctt tcttccctgg attcctcact tttgaagagt gtgaggaaaa 628
cctatgtttg gcgcttaagc tttcagctca gcttaatgaa gtgtttagca tagtacctct 688
gctatttgct gttattttat ctgctatgct attgaagttt tggcaattga ctatagtgtg 748
agccaggaat cactggctgt taatcttaca aagtgtcttg gaattgtagg tgactattat 808
ttttccaaga aatatccctt aagatattaa ctgagaaggc tgggggttta atgtggaaat 868
gatgtttcaa aaggaatcct gtgatggaaa tacaactggt atcttcactt ttttaggaat 928
tgggaaatat tttaatgttt cttggggaat atgttagaga attcccttac tcttgattgt 988
gggatactat ttaattattt cactttagaa agctgagtgt ttcacacctt atctatgtag 1048
aatatatttc cttattcaga atttctaaaa gtttaagttc tatgagggct aatatcttat 1108
cttcctataa ttttagacat tgctttaact ttttagtaaa aaaaaaaaaa aaaaaaaaaa 1168
aaaaa 1173

<210> 33
<211> 825
<212> DNA
<213> Homo sapiens

<220>
<221> CDS
<222> (34)...(327)

<400> 33
acattgtgaa atctccaact cttaaccttc aac atg aaa gtc tct gca gtg ctt 54
Met Lys Val Ser Ala Val Leu
1 5

```
ctg tgc ctg ctg ctc atg aca gca gct ttc aac ccc cag gga ctt gct       102
Leu Cys Leu Leu Leu Met Thr Ala Ala Phe Asn Pro Gln Gly Leu Ala
         10                  15                  20

cag cca gat gca ctc aac gtc cca tct act tgc tgc ttc aca ttt agc       150
Gln Pro Asp Ala Leu Asn Val Pro Ser Thr Cys Cys Phe Thr Phe Ser
     25                  30                  35

agt aag aag atc tcc ttg cag agg ctg aag agc tat gtg atc acc acc       198
Ser Lys Lys Ile Ser Leu Gln Arg Leu Lys Ser Tyr Val Ile Thr Thr
 40                  45                  50                  55

agc agg tgt ccc cag aag gct gtc atc ttc aga acc aaa ctg ggc aag       246
Ser Arg Cys Pro Gln Lys Ala Val Ile Phe Arg Thr Lys Leu Gly Lys
                 60                  65                  70

gag atc tgt gct gac cca aag gag aag tgg gtc cag aat tat atg aaa       294
Glu Ile Cys Ala Asp Pro Lys Glu Lys Trp Val Gln Asn Tyr Met Lys
             75                  80                  85

cac ctg ggc cgg aaa gct cac acc ctg aag act tgaactctgc tacccctact     347
His Leu Gly Arg Lys Ala His Thr Leu Lys Thr
         90                  95

gaaatcaagc tggagtacgt gaaatgactt ttccattctc ctctggcctc ctcttctatg     407
ctttggaata cttctaccat aattttcaaa taggatgcat tcggttttgt gattcaaaat     467
gtactatgtg ttaagtaata ttggctatta tttgacttgt tgctggtttg gagtttattt     527
gagtattgct gatcttttct aaagcaaggc cttgagcaag taggttgctg tctctaagcc     587
cccttccctt ccactatgag ctgctggcag tgggttgtat tcggttccca ggggttgaga     647
gcatgcctgt gggagtcatg gacatgaagg gatgctgcaa tgtaggaagg agagctcttt     707
gtgaatgtga ggttgttgct aaattattgt ttattgtgga aagatgaatg caatagtagg     767
actgctgaca ttttgcagaa aatacatttt atttaaaatc tcctaaaaaa aaaaaaaa       825
```

<210> 34
<211> 3112
<212> DNA
<213> Homo sapiens

<220>
<221> CDS
<222> (1192)...(1267)
<221> CDS
<222> (1953)...(2067)

<221> CDS
<222> (2488)...(2575)

<400> 34
```
ttagagactt aataataaag gatcttgtgg ataatttatc attccctgat agagaaaaat      60
ttagctttgc ttattttaga gttataaatg atgctgggtc aggtatcttt atgtttgaag     120
atggctccat atttgggttg tttccacaga actctttccc agaaatgctt tttctaggtt     180
aatggctaca catatttcta ggcacctgac atactgacac ccacctctaa agtattttta     240
tgatccacaa ctagcgttta acacagcgcc ccagtcactc cgagactaat aaatagacaa     300
atgactgaaa cgtgacctca tgctttctat tcctccagct ttcattgagt tcctttcctc     360
tgggaggact ggggggttgtc tagccctcca cagcatcagc ccattgaccc tatccttgtg    420
gttatagcag ctgaggaagc agaattacag ctctgtggga aggaatgggg ctggagagtt     480
catgcataga ccaattcttt tttttttttt tttttgagat ggagtttcac ttttgttgcc     540
caggctggag tgcaatggca tgatctcagc tcaccacagc ccccacctcc tgggttcaag     600
cgattctcct gccctcagcc tcccgagtag ctgggattac aggcatgtgc caccacgcct     660
gactactttt gtatttttag tagagatgga gtttctcttt cttggtcagg ttggtctcaa     720
actcctgacc tcaggtgatc cgcagcctcg gcctcccaaa gtgttgggat tacaggtgtg     780
agcgaccatg cctggctgca tagaccagtt cttatgagaa gggatcaact aagaatagcc     840
ttgggttgac acacacccct cttcacactc acaggagaaa ccccatgaag ctagaaccag     900
tcatgagttg agagctgaga gttagagagt agctcagaga tgctattctt ggatatcctg     960
agcccctgtg gtcaccaggg accctgagtt gtgcaacact cagcatgaca gcatcactac    1020
acttaaaaat ttccctcctc acccccagat tccatttccc catccgccag ggctgcctat    1080
aaagaggaga gatggcttca gacatcagaa ggacgcaggc agcaaagagt agtcagtccc    1140
ttcttggctc tgctgacact cgagcccaca ttccatcacc tgctcccaat c atg cag     1197
                                                         Met Gln
                                                          1

gtc tcc act gct gcc ctt gcc gtc ctc ctc tgc acc atg gct ctc tgc      1245
Val Ser Thr Ala Ala Leu Ala Val Leu Leu Cys Thr Met Ala Leu Cys
        5                  10                  15
```

```
aac cag gtc ctc tct gca cca c gtgagtccat gttgttgttg tgggtatcac      1297
Asn Gln Val Leu Ser Ala Pro
     20                 25

cactctctgg ccatggttag accacatcag tctttttttg cggcctgaga gccccgaaga   1357
gaaaagaagg aagttcttaa agcgctgcca aacaccttgg tctttttctt cacaactttt   1417
atttttatct ctagaagggg tcttagccct cctagtctcc aggtatgaga atctaggcag   1477
gggcagggga gttacagtcc cttgtacaga tagaaaaaca gggttcaaaa cgaatcagtt   1537
tgcaagaggc agaatccagg gctgcttact tcccagtggg gtctgttgtt cactctccag   1597
ctcaccctag gtctcccagg agccctgtcc cttggatgtc ttatgagaga tgtccagggc   1657
ttctcttggg ctggggtatg acttcttgaa ccgacaaaat tccatgaaga gagctaagag   1717
aacagtccat tcaggtatct ggatcacata gagaaacaga gaacccacta tgaagagtca   1777
aggggaaaga ggaatataga cagaaacaaa gagacatttc tctgcaaaac cccccaaatg   1837
ccttgcagtc acttggtctg agcaagcctg ccctcctcaa ccactcaggg atcagaagct   1897
gcctggcctt ttcttctgag ctgtgactcg ggcttattct ctcctttctc cgcag tt     1954
                                                            Leu

gct gct gac acg ccg acc gcc tgc tgc ttc agc tac acc tcc cga cag     2002
Ala Ala Asp Thr Pro Thr Ala Cys Cys Phe Ser Tyr Thr Ser Arg Gln
            30                  35                  40

att cca cag aat ttc ata gct gac tac ttt gag acg agc agc cag tgc     2050
Ile Pro Gln Asn Phe Ile Ala Asp Tyr Phe Glu Thr Ser Ser Gln Cys
        45                  50                  55

tcc aag ccc agt gtc at gtaagtgcca gtcttcctgc tcacctctag             2097
Ser Lys Pro Ser Val Ile
     60

ggaggtaggg agtgtcaggg tgggggcaga aacaggccag aaggccatcc tggaaaggcc   2157
cagccttcag gagcctatcg gggatacagg acgcagggca ctgaggtgtg acctgacttg   2217
gggctggagt gaggtgggtg ttacagagtc aggaagggct gccccaggcc agaggaaagg   2277
aacaggaaga aggaggcagc aggacactct gagggccccc ttgcctggag tcactgagag   2337
aagctctcta gacggagata ggcagggggc ccctgagaga ggagcaggcc ttgagctgcc   2397
caggacagag agcaggatgt caggccatgg tgggcccagg attccccggc tggattcccc   2457
agtgcttaac tcttcctccc ttctccacag c ttc cta acc aag aga ggc cgg       2509
                                   Phe Leu Thr Lys Arg Gly Arg
```

65 70

cag gtc tgt gct gac ccc agt gag gag tgg gtc cag aaa tac gtc agt 2557
Gln Val Cys Ala Asp Pro Ser Glu Glu Trp Val Gln Lys Tyr Val Ser
75 80 85

gac ctg gag ctg agt gcc tgaggggtcc agaagcttcg aggcccagcg 2605
Asp Leu Glu Leu Ser Ala
90

acctcagtgg gcccagtggg gaggagcagg agcctgagcc ttgggaacat gcgtgtgacc 2665
tctacagcta cctcttctat ggactggtta ttgccaaaca gccacactgt gggactcttc 2725
ttaacttaaa ttttaattta tttatactat ttagttttta taatttattt ttgatttcac 2785
agtgtgtttg tgattgtttg ctctgagagt tccccctgtc ccctccacct tccctcacag 2845
tgtgtctggt gacgaccgag tggctgtcat cggcctgtgt aggcagtcat ggcaccaaag 2905
ccaccagact gacaaatgtg tatcagatgc ttttgttcag ggctgtgatc ggcctgggga 2965
aataataaag atgttctttt aaacggtaaa ccagtattga gtttggtttt gtttttctgg 3025
caaatcaaaa tcactagtta agaggaatca taggcaaaga ttaggaagag gtgaaatgga 3085
gggaaactgg gagagatggg gagcgct 3112

<210> 35
<211> 481
<212> DNA
<213> Homo sapiens

<220>
<221> CDS
<222> (55)...(333)

<400> 35
agcctctgaa gctcccacca ggccagctct cctcccacaa cagcttccca cagc atg 57
Met
1

aag atc tcc gtg gct gcc att ccc ttc ttc ctc ctc atc acc atc gcc 105
Lys Ile Ser Val Ala Ala Ile Pro Phe Phe Leu Leu Ile Thr Ile Ala
5 10 15

```
cta ggg acc aag act gaa tcc tcc tca cgg gga cct tac cac ccc tca      153
Leu Gly Thr Lys Thr Glu Ser Ser Ser Arg Gly Pro Tyr His Pro Ser
         20                  25                  30

gag tgc tgc ttc acc tac act acc tac aag atc ccg cgt cag cgg att      201
Glu Cys Cys Phe Thr Tyr Thr Thr Tyr Lys Ile Pro Arg Gln Arg Ile
     35                  40                  45

atg gat tac tat gag acc aac agc cag tgc tcc aag ccc gga att gtc      249
Met Asp Tyr Tyr Glu Thr Asn Ser Gln Cys Ser Lys Pro Gly Ile Val
 50                  55                  60                  65

ttc atc acc aaa agg ggc cat tcc gtc tgt acc aac ccc agt gac aag      297
Phe Ile Thr Lys Arg Gly His Ser Val Cys Thr Asn Pro Ser Asp Lys
                 70                  75                  80

tgg gtc cag gac tat atc aag gac atg aag gag aac tgagtgaccc           343
Trp Val Gln Asp Tyr Ile Lys Asp Met Lys Glu Asn
             85                  90

agaaggggtg gcgaaggcac agctcagaga cataaagaga agatgccaag gccccctcct    403
ccacccaccc ctaactctca gccccagtca ccctcttgga gcttccctgc tttgaattaa    463
agaccactca tgctcttc                                                  481

<210> 36
<211> 3709
<212> DNA
<213> Homo sapiens

<220>
<221> CDS
<222> (885)...(960)

<221> CDS
<222> (2149)...(2260)

<221> CDS
<222> (3383)...(3482)
```

<400> 36

```
tctagaaaaa aaaaaacaaa aagggaaaat tcccctggca ggactctcag atgctgctga     60
gtagctctca gtcctctctg taacccaaac ataacacatc tatctccgtg cttacactgg    120
gtggctttca cttgtttatc tgtgaattga agagaagttg cttgaggtca ggcagtgctc    180
ctcattggta actgccttct ctggggctaa ccaaggacct agaacagaat aagctattga    240
aaattgttga ggattgaaaa aaatagaaaa aatagaaatg gcaaatatct aggccagtca    300
ctggacatag agaatgttat ttaattctta tcgcacgtcc ttgagacatg tattgctatt    360
tgcattttgt gtgaatatgc atttgggtaa gtttatgtaa tccctcctct gcagaactgg    420
gattcaaatg caggtgtatc tctgttcagg tccagactct tctgccctga agcagtagta    480
cttggatgga atgacgtagg gttggacaag ccacacagag gccacttcct ctcacttact    540
tttctttgct tcccactcaa ccaggacagt tcccacgcac tttttcaaga ttcttatctg    600
ctcccacact tggggaagtt cccaatgcaa cctatcaatc catcaccacc acgaatacca    660
gccaggagag gtggggaaag gagtttacca catggtcgct gggtgtgagc aactgttccc    720
tgtccctatg gcttcccact tgtggctccc accatggcct ggagttttgg gtggagtttt    780
tcaaataaaa gccctcagca ttgcaggacg gcacagtggt gagctcttag cttcaccagg    840
ctcatcaaag ctgctccagg aaggcccaag ccagaccaga agac atg cag atc atc     896
                                                 Met Gln Ile Ile
                                                 1

acc aca gcc ctg gtg tgc ttg ctg cta gct ggg atg tgg ccg gaa gat      944
Thr Thr Ala Leu Val Cys Leu Leu Leu Ala Gly Met Trp Pro Glu Asp
 5                  10                  15                  20

gtg gac agc aag agc a gtgagtgtgg caggcatcat tttgcttctc tctggggagg   1000
Val Asp Ser Lys Ser
                25

gcagaaacgt ggtcagccac tctggggttg gagcaggctt ctccttgaac tcaccaactc   1060
tatctcccct cttcctacct aaagaggagg aatggtgaac ttggacaggc tggggtgagg   1120
gctagtagga gaaccatgag ttggggcaaa cacagagaac tgaactgaca gcttcagtac   1180
aaggagctct gcttcatcca gacccaagga agggaacctg tgaggttact cgggtaaagc   1240
tgggaggccc aaggtccagg ggacagcctg ggtgtagctt ctacagtgtg acagacacca   1300
agtagagtca gaaggcaaga ccgggctcta acaattggtc actcttgggc aagtcacttt   1360
agctctcaaa ctctactttc tctatcagtg aaatggagtt gatgatgtct gccctccaag   1420
actgtttgga gaataccaac ctagtaagag gcatgaaagg gggtgcaaac agaaaaacta   1480
ggaggaagaa gctgggattg gaatgcaggt ctcttgcggg atgtggtgtg ggaggagaat   1540
gcacaaatgg acagagtggg ggttgggggc tgggaaagag ctaaggacca gggcaggagg   1600
```

```
ggattcaaga gactgagtag ggcagctagc tagttcctgg gagctcttcc cttgtcatct   1660
catcagtttg gactcctcga acaattccta atcttcccca gatcaggtct gtgaactgtg   1720
gaccactgtg tcctgcatca gactaaccag gtccccaggg tgtggggtcc agagcccttg   1780
gacatgaata ctggggcaga accatgcaca tgtggtgaaa taccaaaact ggatgagcct   1840
ttagaaacca ggctccaaaa agttttattt tacagatggg aagtctgggg ccagggtgaa   1900
ggcacatctt cctcagggcc actcagctgg ggtgcgggga gctcagatct gaaccccaat   1960
cttctgactc tttacctagc cccagaacaa ggtggctgat gaggcagagc tatgccggca   2020
ccgtctggat gtggtcccca agccagggct tgtcctggga ggcgtttttt tgtttgtttt   2080
ttaaaaattg tgctacaggt gagaggttga gaaatggatg caaaccatcg tctgtgttcc   2140
tcttctag tg cag gta ccc ttc tcc aga tgt tgc ttc tca ttt gcg gag     2189
        Met Gln Val Pro Phe Ser Arg Cys Cys Phe Ser Phe Ala Glu
                        30                  35

caa gag att ccc ctg agg gca atc ctg tgt tac aga aat acc agc tcc     2237
Gln Glu Ile Pro Leu Arg Ala Ile Leu Cys Tyr Arg Asn Thr Ser Ser
 40                  45                  50                  55

atc tgc tcc aat gag ggc tta at gtaagtgatc acctgctcaa tctctcccta     2290
Ile Cys Ser Asn Glu Gly Leu Ile
                60

gagaacagaa ccccgccagc ctggaattac aagagtagac actagatgac agtattttac   2350
tggaataagg tttctaaacc cagagctgcc agcacctggg tgcaagccac acttgggcgc   2410
tagagggagc gctgagcttc ctagcaggtg tgaggaagga tgcatctgtg ctcctgcagt   2470
ggcttgtgtt tcctgaaact ccaaggtgcc aagtattgta tcccagcatt atgagctcag   2530
aggtttaaca aaagcatgag gggttattgt gcactggaaa gagcaaggga accaggatga   2590
gttcctgccc ctggatttgg aacccatagt cttgggtgac cgtggacagg taactccttt   2650
gtactgaatt gtctgtgtat ccttctgtat tccttatctg tgaagggtca taaacatagc   2710
tgcatcacag ggtctttaca aacttaattg gagtagcttt cacataccag tcagtatttt   2770
aaggcttttt catgtatatt ctctctgtcg atcctcttgg ggcacatatt tttgttatca   2830
tgaaaagtga ggttcaggaa ggtagagata tttgtctaag atcaaccaga tagtaagaga   2890
tagagttggt ctatagattg gacaatagtc cagtttagga agtagacaga tcagaagaga   2950
aaaatacaca cccacacaca cacacaaggc gcgcgcacac acacacacac aaacacatga   3010
gtccaacgca acaagtaagg ccccagatgg acacagaaac attaaagttg gtgcaaagag   3070
ctattccagg atgggaattt ctcatctcac ctaatcgtca gaatgttttc agctgttcag   3130
ccccaccctg atacaccaaa ttgaaaccag gagaggggtc caggaaattc aattcataag   3190
ctcctggtgc tttggctgtt ccccagcgtg caaaccacac atcctgtgca gcaacttcat   3250
ttacagaggg gagcccaagg cctagcaaga gcagtttagg ggacctggca gccggaggag   3310
```

gcggggcttg tgcgttgccc acccagtggt ggcttgggtg gctcagcctt ctctcttatt 3370
ctctgttcac ag a ttc aag ctg aag aga ggc aaa gag gcc tgc gcc ttg 3419
Phe Lys Leu Lys Arg Gly Lys Glu Ala Cys Ala Leu
65 70 75

gac aca gtt gga tgg gtt cag agg cac aga aaa atg ctg agg cac tgc 3467
Asp Thr Val Gly Trp Val Gln Arg His Arg Lys Met Leu Arg His Cys
80 85 90

ccg tca aaa aga aaa tgagcagatt tctttccatt gtgggctctg gaaaccacat 3522
Pro Ser Lys Arg Lys
95

ggcttcacct gtccccgaaa ctaccagccc tacaccattc cttctgccct gcttttgcta 3582
ggtcacagag gatctgcttg gtcttgataa gctatgttgt tgcactttaa acatttaaat 3642
tatacaatca tcaacccccca accctctggg ctcttggatt tcagagtgaa aacttgatgg 3702
cattgag 3709

<210> 37
<211> 673
<212> DNA
<213> Homo sapiens

<220>
<221> CDS
<222> (67)...(450)

<400> 37
gggcaactca ccctcactca gaggtcttct ggttctggaa acaactctag ctcagccttc 60
tccacc atg agc ctc aga ctt gat acc acc cct tcc tgt aac agt gcg 108
Met Ser Leu Arg Leu Asp Thr Thr Pro Ser Cys Asn Ser Ala
1 5 10

aga cca ctt cat gcc ttg cag gtg ctg ctg ctt ctg tca ttg ctg ctg 156
Arg Pro Leu His Ala Leu Gln Val Leu Leu Leu Leu Ser Leu Leu Leu
15 20 25 30

act gct ctg gct tcc tcc acc aaa gga caa act aag aga aac ttg gcg 204

Thr Ala Leu Ala Ser Ser Thr Lys Gly Gln Thr Lys Arg Asn Leu Ala
35 40 45

aaa ggc aaa gag gaa agt cta gac agt gac ttg tat gct gaa ctc cgc 252
Lys Gly Lys Glu Glu Ser Leu Asp Ser Asp Leu Tyr Ala Glu Leu Arg
50 55 60

tgc atg tgt ata aag aca acc tct gga att cat ccc aaa aac atc caa 300
Cys Met Cys Ile Lys Thr Thr Ser Gly Ile His Pro Lys Asn Ile Gln
65 70 75

agt ttg gaa gtg atc ggg aaa gga acc cat tgc aac caa gtc gaa gtg 348
Ser Leu Glu Val Ile Gly Lys Gly Thr His Cys Asn Gln Val Glu Val
80 85 90

ata gcc aca ctg aag gat ggg agg aaa atc tgc ctg gac cca gat gct 396
Ile Ala Thr Leu Lys Asp Gly Arg Lys Ile Cys Leu Asp Pro Asp Ala
95 100 105 110

ccc aga atc aag aaa att gta cag aaa aaa ttg gca ggt gat gaa tct 444
Pro Arg Ile Lys Lys Ile Val Gln Lys Lys Leu Ala Gly Asp Glu Ser
115 120 125

gct gat taatttgttc tgtttctgcc aaacttcttt aactcccagg aagggtagaa 500
Ala Asp

ttttgaaacc ttgattttct agagttctca tttattcagg atacctattc ttactgtatt 560
aaaatttgga tatgtgtttc attctgtctc aaaaatcaca ttttattctg agaaggttgg 620
ttaaaagatg gcagaaagaa gatgaaaata aataagcctg gtttcaaccc tct 673

<210> 38
<211> 12
<212> PRT
<213> Homo sapiens

<400> 38
Gln Val Cys Ile Asp Pro Lys Leu Lys Trp Ile Gln

```
 1               5                   10

<210> 39
<211> 2545
<212> DNA
<213> Homo sapiens

<220>
<221> CDS
<222> (40)...(414)

<400> 39
atccaataca ggagtgactt ggaactccat tctatcact atg aag aaa agt ggt        54
                                           Met Lys Lys Ser Gly
                                            1               5

gtt ctt ttc ctc ttg ggc atc atc ttg ctg gtt ctg att gga gtg caa      102
Val Leu Phe Leu Leu Gly Ile Ile Leu Leu Val Leu Ile Gly Val Gln
                 10                  15                  20

gga acc cca gta gtg aga aag ggt cgc tgt tcc tgc atc agc acc aac      150
Gly Thr Pro Val Val Arg Lys Gly Arg Cys Ser Cys Ile Ser Thr Asn
             25                  30                  35

caa ggg act atc cac cta caa tcc ttg aaa gac ctt aaa caa ttt gcc      198
Gln Gly Thr Ile His Leu Gln Ser Leu Lys Asp Leu Lys Gln Phe Ala
         40                  45                  50

cca agc cct tcc tgc gag aaa att gaa atc att gct aca ctg aag aat      246
Pro Ser Pro Ser Cys Glu Lys Ile Glu Ile Ile Ala Thr Leu Lys Asn
     55                  60                  65

gga gtt caa aca tgt cta aac cca gat tca gca gat gtg aag gaa ctg      294
Gly Val Gln Thr Cys Leu Asn Pro Asp Ser Ala Asp Val Lys Glu Leu
 70                  75                  80                  85

att aaa aag tgg gag aaa cag gtc agc caa aag aaa aag caa aag aat      342
Ile Lys Lys Trp Glu Lys Gln Val Ser Gln Lys Lys Lys Gln Lys Asn
```

```
                90                  95                  100

ggg aaa aaa cat caa aaa aag aaa gtt ctg aaa gtt cga aaa tct caa      390
Gly Lys Lys His Gln Lys Lys Lys Val Leu Lys Val Arg Lys Ser Gln
            105                 110                 115

cgt tct cgt caa aag aag act aca taagagacca cttcaccaat aagtattctg     444
Arg Ser Arg Gln Lys Lys Thr Thr
        120                 125

tgttaaaaat gttctatttt aattataccg ctatcattcc aaaggaggat ggcatataat    504
acaaaggctt attaatttga ctagaaaatt taaaacatta ctctgaaatt gtaactaaag    564
ttagaaagtt gattttaaga atccaaacgt taagaattgt taaaggctat gattgtcttt    624
gttcttctac cacccaccag ttgaatttca tcatgcttaa ggccatgatt ttagcaatac    684
ccatgtctac acagatgttc acccaaccac atcccactca caacagctgc ctggaagagc    744
agccctaggc ttccacgtac tgcagcctcc agagagtatc tgaggcacat gtcagcaagt    804
cctaagcctg ttagcatgct ggtgagccaa gcagtttgaa attgagctgg acctcaccaa    864
gctgctgtgg ccatcaacct ctgtatttga atcagcctac aggcctcaca cacaatgtgt    924
ctgagagatt catgctgatt gttattgggt atcaccactg gagatcacca gtgtgtggct    984
ttcagagcct cctttctggc tttggaagcc atgtgattcc atcttgcccg ctcaggctga   1044
ccactttatt tctttttgtt cccctttgct tcattcaagt cagctcttct ccatcctacc   1104
acaatgcagt gcctttcttc tctccagtgc acctgtcata tgctctgatt tatctgagtc   1164
aactcctttc tcatcttgtc cccaacaccc cacagaagtg ctttcttctc ccaattcatc   1224
ctcactcagt ccagcttagt tcaagtcctg cctcttaaat aaaccttttt ggacacacaa   1284
attatcttaa aactcctgtt tcacttggtt cagtaccaca tgggtgaaca ctcaatggtt   1344
aactaattct tgggtgttta tcctatctct ccaaccagat tgtcagctcc ttgagggcaa   1404
gagccacagt atatttccct gtttcttcca cagtgcctaa taatactgtg gaactaggtt   1464
ttaataattt tttaattgat gttgttatgg gcaggatggc aaccagacca ttgtctcaga   1524
gcaggtgctg gctctttcct ggctactcca tgttggctag cctctggtaa cctcttactt   1584
attatcttca ggacactcac tacagggacc agggatgatg caacatcctt gtctttttat   1644
gacaggatgt ttgctcagct tctccaacaa taagaagcac gtggtaaaac acttgcggat   1704
attctggact gtttttaaaa aatatacagt ttaccgaaaa tcatataatc ttacaatgaa   1764
aaggacttta tagatcagcc agtgaccaac cttttcccaa ccatacaaaa attccttttc   1824
ccgaaggaaa agggctttct caataagcct cagctttcta agatctaaca agatagccac   1884
cgagatcctt atcgaaactc attttaggca aatatgagtt ttattgtccg tttacttgtt   1944
tcagagtttg tattgtgatt atcaattacc acaccatctc ccatgaagaa agggaacggt   2004
gaagtactaa gcgctagagg aagcagccaa gtcggttagt ggaagcatga ttggtgccca   2064
gttagcctct gcaggatgtg gaaacctcct tccaggggag gttcagtgaa ttgtgtagga   2124
```

gaggttgtct gtggccagaa tttaaaccta tactcacttt cccaaattga atcactgctc 2184
acactgctga tgatttagag tgctgtccgg tggagatccc acccgaacgt cttatctaat 2244
catgaaactc cctagttcct tcatgtaact tccctgaaaa atctaagtgt ttcataaatt 2304
tgagagtctg tgacccactt accttgcatc tcacaggtag acagtatata actaacaacc 2364
aaagactaca tattgtcact gacacacacg ttataatcat ttatcatata tatacataca 2424
tgcatacact ctcaaagcaa ataatttttc acttcaaaac agtattgact tgtatacctt 2484
gtaatttgaa atattttctt tgttaaaata gaatggtatc aataaataga ccattaatca 2544
g 2545

<210> 40
<211> 12
<212> PRT
<213> Homo sapiens

<400> 40
Glu Leu Cys Leu Asp Pro Lys Glu Asn Trp Val Gln
1 5 10

<210> 41
<211> 12
<212> PRT
<213> Homo sapiens

<400> 41
Glu Ile Cys Leu Asp Pro Glu Ala Pro Phe Leu Lys
1 5 10

<210> 42
<211> 12
<212> PRT
<213> Homo sapiens

<400> 42
Gln Val Cys Ala Asp Pro Ser Glu Glu Trp Val Gln
1 5 10

<210> 43
<211> 12

<212> PRT

<213> Homo sapiens

<400> 43

Gln Val Cys Ala Asp Pro Ser Glu Ser Trp Val Gln
1 5 10

<210> 44

<211> 12

<212> PRT

<213> Homo sapiens

<400> 44

Gln Val Cys Ala Asp Pro Ser Glu Ser Trp Val Gln
1 5 10

<210> 45

<211> 125

<212> PRT

<213> Homo sapiens

<400> 45

Met Lys Lys Ser Gly Val Leu Phe Leu Leu Gly Ile Ile Leu Leu Val
1 5 10 15

Leu Ile Gly Val Gln Gly Thr Pro Val Val Arg Lys Gly Arg Cys Ser
20 25 30

Cys Ile Ser Thr Asn Gln Gly Thr Ile His Leu Gln Ser Leu Lys Asp
35 40 45

Leu Lys Gln Phe Ala Pro Ser Pro Ser Cys Glu Lys Ile Glu Ile Ile
50 55 60

Ala Thr Leu Lys Asn Gly Val Gln Thr Cys Leu Asn Pro Asp Ser Ala
65 70 75 80

Asp Val Lys Glu Leu Ile Lys Lys Trp Glu Lys Gln Val Ser Gln Lys
85 90 95

Lys Lys Gln Lys Asn Gly Lys Lys His Gln Lys Lys Lys Val Leu Lys
100 105 110

Val Arg Lys Ser Gln Arg Ser Arg Gln Lys Lys Thr Thr
115 120 125

<210> 46
<211> 128
<212> PRT
<213> Homo sapiens

<400> 46
Met Ser Leu Arg Leu Asp Thr Thr Pro Ser Cys Asn Ser Ala Arg Pro
1               5                   10                  15
Leu His Ala Leu Gln Val Leu Leu Leu Leu Ser Leu Leu Leu Thr Ala
            20                  25                  30
Leu Ala Ser Ser Thr Lys Gly Gln Thr Lys Arg Asn Leu Ala Lys Gly
        35                  40                  45
Lys Glu Glu Ser Leu Asp Ser Asp Leu Tyr Ala Glu Leu Arg Cys Met
    50                  55                  60
Cys Ile Lys Thr Thr Ser Gly Ile His Pro Lys Asn Ile Gln Ser Leu
65                  70                  75                  80
Glu Val Ile Gly Lys Gly Thr His Cys Asn Gln Val Glu Val Ile Ala
                85                  90                  95
Thr Leu Lys Asp Gly Arg Lys Ile Cys Leu Asp Pro Asp Ala Pro Arg
            100                 105                 110
Ile Lys Lys Ile Val Gln Lys Lys Leu Ala Gly Asp Glu Ser Ala Asp
        115                 120                 125

<210> 47
<211> 96
<212> PRT
<213> Homo sapiens
<400> 47
Met Gln Ile Ile Thr Thr Ala Leu Val Cys Leu Leu Leu Ala Gly Met
1               5                   10                  15
Trp Pro Glu Asp Val Asp Ser Lys Ser Met Gln Val Pro Phe Ser Arg
            20                  25                  30
Cys Cys Phe Ser Phe Ala Glu Gln Glu Ile Pro Leu Arg Ala Ile Leu
        35                  40                  45
Cys Tyr Arg Asn Thr Ser Ser Ile Cys Ser Asn Glu Gly Leu Ile Phe
    50                  55                  60
Lys Leu Lys Arg Gly Lys Glu Ala Cys Ala Leu Asp Thr Val Gly Trp

65 70 75 80

Val Gln Arg His Arg Lys Met Leu Arg His Cys Pro Ser Lys Arg Lys

85 90 95

<210> 48

<211> 93

<212> PRT

<213> Homo sapiens

<400> 48

Met Lys Ile Ser Val Ala Ala Ile Pro Phe Phe Leu Leu Ile Thr Ile

1 5 10 15

Ala Leu Gly Thr Lys Thr Glu Ser Ser Ser Arg Gly Pro Tyr His Pro

20 25 30

Ser Glu Cys Cys Phe Thr Tyr Thr Thr Tyr Lys Ile Pro Arg Gln Arg

35 40 45

Ile Met Asp Tyr Tyr Glu Thr Asn Ser Gln Cys Ser Lys Pro Gly Ile

50 55 60

Val Phe Ile Thr Lys Arg Gly His Ser Val Cys Thr Asn Pro Ser Asp

65 70 75 80

Lys Trp Val Gln Asp Tyr Ile Lys Asp Met Lys Glu Asn

85 90

<210> 49

<211> 93

<212> PRT

<213> Homo sapiens

<400> 49

Met Gln Val Ser Thr Ala Ala Leu Ala Val Leu Leu Cys Thr Met Ala

1 5 10 15

Leu Cys Asn Gln Val Leu Ser Ala Pro Leu Ala Ala Asp Thr Pro Thr

20 25 30

Ala Cys Cys Phe Ser Tyr Thr Ser Arg Gln Ile Pro Gln Asn Phe Ile

35 40 45

Ala Asp Tyr Phe Glu Thr Ser Ser Gln Cys Ser Lys Pro Ser Val Ile

50 55 60

Phe Leu Thr Lys Arg Gly Arg Gln Val Cys Ala Asp Pro Ser Glu Glu

65 70 75 80
Trp Val Gln Lys Tyr Val Ser Asp Leu Glu Leu Ser Ala
85 90

<210> 50
<211> 98
<212> PRT
<213> Homo sapiens

<400> 50
Met Lys Val Ser Ala Val Leu Leu Cys Leu Leu Leu Met Thr Ala Ala
1 5 10 15
Phe Asn Pro Gln Gly Leu Ala Gln Pro Asp Ala Leu Asn Val Pro Ser
20 25 30
Thr Cys Cys Phe Thr Phe Ser Ser Lys Lys Ile Ser Leu Gln Arg Leu
35 40 45
Lys Ser Tyr Val Ile Thr Thr Ser Arg Cys Pro Gln Lys Ala Val Ile
50 55 60
Phe Arg Thr Lys Leu Gly Lys Glu Ile Cys Ala Asp Pro Lys Glu Lys
65 70 75 80
Trp Val Gln Asn Tyr Met Lys His Leu Gly Arg Lys Ala His Thr Leu
85 90 95
Lys Thr

<210> 51
<211> 839
<212> DNA
<213> Homo sapiens

<220>
<221> CDS
<222> (54)...(344)

<400> 51
caacccagaa accaccacct ctcacgccaa agctcacacc ttcagcctcc aac atg 56
Met
1

aag gtc tcc gca gca ctt ctg tgg ctg ctg ctc ata gca gct gcc ttc 104
Lys Val Ser Ala Ala Leu Leu Trp Leu Leu Leu Ile Ala Ala Ala Phe
5 10 15

agc ccc cag ggg ctc gct ggg cca gct tct gtc cca acc acc tgc tgc 152
Ser Pro Gln Gly Leu Ala Gly Pro Ala Ser Val Pro Thr Thr Cys Cys
20 25 30

ttt aac ctg gcc aat agg aag ata ccc ctt cag cga cta gag agc tac 200
Phe Asn Leu Ala Asn Arg Lys Ile Pro Leu Gln Arg Leu Glu Ser Tyr
35 40 45

agg aga atc acc agt ggc aaa tgt ccc cag aaa gct gtg atc ttc aag 248
Arg Arg Ile Thr Ser Gly Lys Cys Pro Gln Lys Ala Val Ile Phe Lys
50 55 60 65

acc aaa ctg gcc aag gat atc tgt gcc gac ccc aag aag aag tgg gtg 296
Thr Lys Leu Ala Lys Asp Ile Cys Ala Asp Pro Lys Lys Lys Trp Val
70 75 80

cag gat tcc atg aag tat ctg gac caa aaa tct cca act cca aag cca 344
Gln Asp Ser Met Lys Tyr Leu Asp Gln Lys Ser Pro Thr Pro Lys Pro
85 90 95

taaataatca ccatttttga aaccaaacca gagcctgagt gttgcctaat ttgttttccc 404
ttcttacaat gcattctgag gtaacctcat tatcagtcca aagggcatgg gttttattat 464
atatatatat atatattttt ttttaaaaaa aaacgtattg catttaattt attgaggctt 524
taaaacttat cctccatgaa tatcagttat ttttaaactg taaagctttg tgcagattct 584
ttacccccctg ggagccccaa ttcgatcccc tgtcacgtgt gggcaatgtt ccccctctcc 644
tctcttcctc cctggaatct tgtaaaggtc ctggcaaaga tgatcagtat gaaaatgtca 704
ttgttcttgt gaacccaaag tgtgactcat taaatggaag taatgttgtt ttaggaatac 764
ataaagtatg tgcatatttt attatagtca ctagttgtaa tttttttgtg ggaaatccac 824
actgagctga ggggg 839

<210> 52
<211> 114
<212> PRT
<213> Homo sapiens

<400> 52

Met Ser Leu Leu Ser Ser Arg Ala Ala Arg Val Pro Gly Pro Ser Ser
1               5                   10                  15
Ser Leu Cys Ala Leu Leu Val Leu Leu Leu Leu Leu Thr Gln Pro Gly
            20                  25                  30
Pro Ile Ala Ser Ala Gly Pro Ala Ala Ala Val Leu Arg Glu Leu Arg
        35                  40                  45
Cys Val Cys Leu Gln Thr Thr Gln Gly Val His Pro Lys Met Ile Ser
    50                  55                  60
Asn Leu Gln Val Phe Ala Ile Gly Pro Gln Cys Ser Lys Val Glu Val
65                  70                  75                  80
Val Ala Ser Leu Lys Asn Gly Lys Glu Ile Cys Leu Asp Pro Glu Ala
                85                  90                  95
Pro Phe Leu Lys Lys Val Ile Gln Lys Ile Leu Asp Gly Gly Asn Lys
            100                 105                 110
Glu Asn

<210> 53
<211> 107
<212> PRT
<213> Homo sapiens

<400> 53

Met Ala Arg Ala Thr Leu Ser Ala Ala Pro Ser Asn Pro Arg Leu Leu
1               5                   10                  15
Arg Val Ala Leu Leu Leu Leu Leu Leu Val Ala Ala Ser Arg Arg Ala
            20                  25                  30
Ala Gly Ala Pro Leu Ala Thr Glu Leu Arg Cys Gln Cys Leu Gln Thr
        35                  40                  45
Leu Gln Gly Ile His Leu Lys Asn Ile Gln Ser Val Lys Val Lys Ser
    50                  55                  60
Pro Gly Pro His Cys Ala Gln Thr Glu Val Ile Ala Thr Leu Lys Asn
65                  70                  75                  80
Gly Gln Lys Ala Cys Leu Asn Pro Ala Ser Pro Met Val Lys Lys Ile
                85                  90                  95
Ile Glu Lys Met Leu Lys Asn Gly Lys Ser Asn

100 105

<210> 54
<211> 98
<212> PRT
<213> Homo sapiens

<400> 54
Met Asn Pro Ser Ala Ala Val Ile Phe Cys Leu Ile Leu Leu Gly Leu
1 5 10 15
Ser Gly Thr Gln Gly Ile Pro Leu Ala Arg Thr Val Arg Cys Asn Cys
20 25 30
Ile His Ile Asp Asp Gly Pro Val Arg Met Arg Ala Ile Gly Lys Leu
35 40 45
Glu Ile Ile Pro Ala Ser Leu Ser Cys Pro Arg Val Glu Ile Ile Ala
50 55 60
Thr Met Lys Lys Asn Asp Glu Gln Arg Cys Leu Asn Pro Glu Ser Lys
65 70 75 80
Thr Ile Lys Asn Leu Met Lys Ala Phe Ser Gln Lys Arg Ser Lys Arg
85 90 95
Ala Pro

<210> 55
<211> 1041
<212> DNA
<213> Homo sapiens

<220>
<221> CDS
<222> (18)...(338)

<400> 55
cccgcctgct gagcccc atg gcc cgc gct gct ctc tcc gcc gcc ccc agc 50
Met Ala Arg Ala Ala Leu Ser Ala Ala Pro Ser
1 5 10

aat ccc cgg ctc ctg cga gtg gca ctg ctg ctc ctg ctc ctg gta gcc 98

Asn Pro Arg Leu Leu Arg Val Ala Leu Leu Leu Leu Leu Leu Val Ala
15 20 25

gct ggc cgg cgc gca gca gga gcg tcc gtg gcc act gaa ctg cgc tgc 146
Ala Gly Arg Arg Ala Ala Gly Ala Ser Val Ala Thr Glu Leu Arg Cys
30 35 40

cag tgc ttg cag acc ctg cag gga att cac ccc aag aac atc caa agt 194
Gln Cys Leu Gln Thr Leu Gln Gly Ile His Pro Lys Asn Ile Gln Ser
45 50 55

gtg aac gtg aag tcc ccc gga ccc cac tgc gcc caa acc gaa gtc ata 242
Val Asn Val Lys Ser Pro Gly Pro His Cys Ala Gln Thr Glu Val Ile
60 65 70 75

gcc aca ctc aag aat ggg cgg aaa gct tgc ctc aat cct gca tcc ccc 290
Ala Thr Leu Lys Asn Gly Arg Lys Ala Cys Leu Asn Pro Ala Ser Pro
80 85 90

ata gtt aag aaa atc atc gaa aag atg ctg aac agt gac aaa tcc aac 338
Ile Val Lys Lys Ile Ile Glu Lys Met Leu Asn Ser Asp Lys Ser Asn
95 100 105

tgaccagaag ggaggaggaa gctcactggt ggctgttcct gaaggaggcc ctgcccttat 398
aggaacagaa gaggaaagag agacacagct gcagaggcca cctggattgt gcctaatgtg 458
tttgagcatc gcttaggaga agtcttctat ttatttattt attcattagt tttgaagatt 518
ctatgttaat attttaggtg taaaataatt aagggtatga ttaactctac ctgcacactg 578
tcctattata ttcattcttt ttgaaatgtc aaccccaagt tagttcaatc tggattcata 638
tttaatttga aggtagaatg ttttcaaatg ttctccagtc attatgttaa tatttctgag 698
gagcctgcaa catgccagcc actgtgatag aggctggcgg atccaagcaa atggccaatg 758
agatcattgt gaaggcaggg gaatgtatgt gcacatctgt tttgtaactg tttagatgaa 818
tgtcagttgt tatttattga aatgatttca cagtgtgtgg tcaacatttc tcatgttgaa 878
actttaagaa ctaaaatgtt ctaaatatcc cttggacatt ttatgtcttt cttgtaaggc 938
atactgcctt gtttaatggt agttttacag tgtttctggc ttagaacaaa ggggcttaat 998
tattgatgtt ttcatagaga atataaaaat aaagcactta tag 1041

<210> 56
<211> 93

<212> PRT

<213> Homo sapiens

<400> 56

Met Asn Ala Lys Val Val Val Val Leu Val Leu Val Leu Thr Ala Leu
1 5 10 15

Cys Leu Ser Asp Gly Lys Pro Val Ser Leu Ser Tyr Arg Cys Pro Cys
20 25 30

Arg Phe Phe Glu Ser His Val Ala Arg Ala Asn Val Lys His Leu Lys
35 40 45

Ile Leu Asn Thr Pro Asn Cys Ala Leu Gln Ile Val Ala Arg Leu Lys
50 55 60

Asn Asn Asn Arg Gln Val Cys Ile Asp Pro Lys Leu Lys Trp Ile Gln
65 70 75 80

Glu Tyr Leu Glu Lys Ala Leu Asn Lys Arg Phe Lys Met
85 90

<210> 57

<211> 107

<212> PRT

<213> Homo sapiens

<400> 57

Met Ala Arg Ala Ala Leu Ser Ala Ala Pro Ser Asn Pro Arg Leu Leu
1 5 10 15

Arg Val Ala Leu Leu Leu Leu Leu Leu Val Ala Ala Gly Arg Arg Ala
20 25 30

Ala Gly Ala Ser Val Ala Thr Glu Leu Arg Cys Gln Cys Leu Gln Thr
35 40 45

Leu Gln Gly Ile His Pro Lys Asn Ile Gln Ser Val Asn Val Lys Ser
50 55 60

Pro Gly Pro His Cys Ala Gln Thr Glu Val Ile Ala Thr Leu Lys Asn
65 70 75 80

Gly Arg Lys Ala Cys Leu Asn Pro Ala Ser Pro Ile Val Lys Lys Ile
85 90 95

Ile Glu Lys Met Leu Asn Ser Asp Lys Ser Asn
100 105

<210> 58

<211> 1560
<212> DNA
<213> Homo sapiens

<220>
<221> CDS
<222> (102)...(398)

<400> 58

```
ctccataagg cacaaacttt cagagacagc agagcacaca agcttctagg acaagagcca     60
ggaagaaacc accggaagga accatctcac tgtgtgtaaa c atg act tcc aag ctg    116
                                              Met Thr Ser Lys Leu
                                               1               5

gcc gtg gct ctc ttg gca gcc ttc ctg att tct gca gct ctg tgt gaa      164
Ala Val Ala Leu Leu Ala Ala Phe Leu Ile Ser Ala Ala Leu Cys Glu
                 10                  15                  20

ggt gca gtt ttg cca agg agt gct aaa gaa ctt aga tgt cag tgc ata      212
Gly Ala Val Leu Pro Arg Ser Ala Lys Glu Leu Arg Cys Gln Cys Ile
             25                  30                  35

aag aca tac tcc aaa cct ttc cac ccc aaa ttt atc aaa gaa ctg aga      260
Lys Thr Tyr Ser Lys Pro Phe His Pro Lys Phe Ile Lys Glu Leu Arg
         40                  45                  50

gtg att gag agt gga cca cac tgc gcc aac aca gaa att att gta aag      308
Val Ile Glu Ser Gly Pro His Cys Ala Asn Thr Glu Ile Ile Val Lys
     55                  60                  65

ctt tct gat gga aga gag ctc tgt ctg gac ccc aag gaa aac tgg gtg      356
Leu Ser Asp Gly Arg Glu Leu Cys Leu Asp Pro Lys Glu Asn Trp Val
 70                  75                  80                  85

cag agg gtt gtg gag aag ttt ttg aag agg gct gag aat tca              398
Gln Arg Val Val Glu Lys Phe Leu Lys Arg Ala Glu Asn Ser
                 90                  95
```

taaaaaaatt cattctctgt ggtatccaag aatcagtgaa gatgccagtg aaacttcaag 458
caaatctact tcaacacttc atgtattgtg tgggtctgtt gtagggttgc cagatgcaat 518
acaagattcc tggttaaatt tgaatttcag taaacaatga atagtttttc attgtaccat 578
gaaatatcca gaacatactt atatgtaaag tattatttat ttgaatctac aaaaaacaac 638
aaataatttt taaatataag gattttccta gatattgcac gggagaatat acaaatagca 698
aaattgggcc aagggccaag agaatatccg aactttaatt tcaggaattg aatgggtttg 758
ctagaatgtg atatttgaag catcacataa aaatgatggg acaataaatt ttgccataaa 818
gtcaaattta gctggaaatc ctggattttt ttctgttaaa tctggcaacc ctagtctgct 878
agccaggatc cacaagtcct tgttccactg tgccttggtt tctcctttat ttctaagtgg 938
aaaaagtatt agccaccatc ttacctcaca gtgatgttgt gaggacatgt ggaagcactt 998
taagtttttt catcataaca taaattattt tcaagtgtaa cttattaacc tatttattat 1058
ttatgtattt atttaagcat caaatatttg tgcaagaatt tggaaaaata gaagatgaat 1118
cattgattga atagttataa agatgttata gtaaatttat tttattttag atattaaatg 1178
atgttttatt agataaattt caatcagggt ttttagatta aacaaacaaa caattgggta 1238
cccagttaaa ttttcatttc agatatacaa caaataattt tttagtataa gtacattatt 1298
gtttatctga aattttaatt gaactaacaa tcctagtttg atactcccag tcttgtcatt 1358
gccagctgtg ttggtagtgc tgtgttgaat tacggaataa tgagttagaa ctattaaaac 1418
agccaaaact ccacagtcaa tattagtaat ttcttgctgg ttgaaacttg tttattatgt 1478
acaaatagat tcttataata ttatttaaat gactgcattt ttaaatacaa ggctttatat 1538
ttttaacttt aaaaaaaacc gg 1560

<210> 59
<211> 15
<212> PRT
<213> Homo sapiens

<400> 59
Asn Leu Gln Val Phe Ala Ile Gly Pro Gln Cys Ser Lys Val Glu
1 5 10 15

<210> 60
<211> 14
<212> PRT
<213> Homo sapiens

<400> 60
Val Asp Tyr Tyr Glu Thr Ser Ser Leu Cys Ser Gln Pro Ala
1 5 10

<210> 61
<211> 15
<212> PRT
<213> Homo sapiens

<400> 61
Val Asp Tyr Tyr Glu Thr Ser Ser Leu Cys Ser Gln Pro Ala Val
1 5 10 15

<210> 62
<211> 15
<212> PRT
<213> Homo sapiens

<400> 62
Glu Ser Tyr Arg Arg Ile Thr Asn Ile Gln Cys Pro Lys Glu Ala
1 5 10 15

<210> 63
<211> 15
<212> PRT
<213> Homo sapiens

<400> 63
Glu Ser Tyr Arg Arg Thr Thr Ser Ser His Cys Pro Arg Glu Ala
1 5 10 15

<210> 64
<211> 15
<212> PRT
<213> Homo sapiens

<400> 64
Lys Ser Tyr Val Ile Thr Thr Ser Arg Cys Pro Gln Lys Ala Val
1 5 10 15

<210> 65

<211> 12
<212> PRT
<213> Homo sapiens

<400> 65
Glu Ile Cys Ala Asp Pro Lys Glu Lys Trp Val Gln
1 5 10

<210> 66
<211> 12
<212> PRT
<213> Homo sapiens

<400> 66
Glu Ile Cys Ala Asp Pro Thr Gln Lys Trp Val Gln
1 5 10

<210> 67
<211> 12
<212> PRT
<213> Homo sapiens

<400> 67
Glu Ile Cys Ala Asp Pro Lys Glu Arg Trp Val Arg
1 5 10

<210> 68
<211> 12
<212> PRT
<213> Homo sapiens

<220>
<221> VARIANT
<222> (1)...(12)
<223> Xaa = Any Amino Acid

<400> 68
Met Ile Cys Ala Asp Pro Lys Xaa Xaa Trp Val Gln

1 5 10

<210> 69
<211> 15
<212> PRT
<213> Homo sapiens

<400> 69
Ser Val Asn Val Lys Ser Pro Gly Pro His Cys Ala Gln Thr Glu
1 5 10 15

<210> 70
<211> 15
<212> PRT
<213> Homo sapiens

<400> 70
Ser Val Lys Val Lys Ser Pro Gly Pro His Cys Ala Gln Thr Glu
1 5 10 15

<210> 71
<211> 15
<212> PRT
<213> Homo sapiens

<400> 71
Ser Val Asn Val Arg Ser Pro Gly Pro His Cys Ala Gln Thr Glu
1 5 10 15

<210> 72
<211> 12
<212> PRT
<213> Homo sapiens

<400> 72
Lys Ala Cys Leu Asn Pro Ala Ser Pro Ile Val Lys
1 5 10

<210> 73
<211> 12
<212> PRT
<213> Homo sapiens

<400> 73
Lys Ala Cys Leu Asn Pro Ala Ser Pro Met Val Lys
1 5 10

<210> 74
<211> 12
<212> PRT
<213> Homo sapiens

<400> 74
Lys Ala Cys Leu Asn Pro Ala Ser Pro Met Val Gln
1 5 10

<210> 75
<211> 12
<212> PRT
<213> Homo sapiens

<400> 75
Lys Ser Tyr Lys Ile Ile Thr Ser Ser Lys Cys Pro
1 5 10

<210> 76
<211> 661
<212> DNA
<213> Homo sapiens

<220>
<221> CDS
<222> (32)...(331)

<400> 76
tcaaactgaa gctcgcactc tcgcctccag c atg aaa gtc tct gcc gcc ctt 52

```
                                      Met Lys Val Ser Ala Ala Leu
                                       1               5

ctg tgc ctg ctg ctc ata gca gcc acc ttc att ccc caa ggg ctc gct      100
Leu Cys Leu Leu Leu Ile Ala Ala Thr Phe Ile Pro Gln Gly Leu Ala
         10                  15                  20

cag cca gat gca atc aat gcc cca gtc acc tgc tgc tat aac ttc acc      148
Gln Pro Asp Ala Ile Asn Ala Pro Val Thr Cys Cys Tyr Asn Phe Thr
     25                  30                  35

aat agg aag atc tca gtg cag agg ctc gcg agc tat aga aga atc acc      196
Asn Arg Lys Ile Ser Val Gln Arg Leu Ala Ser Tyr Arg Arg Ile Thr
 40                  45                  50                  55

agc agc aag tgt ccc aaa gaa gct gtg atc ttc aag acc att gtg gcc      244
Ser Ser Lys Cys Pro Lys Glu Ala Val Ile Phe Lys Thr Ile Val Ala
                 60                  65                  70

aag gag atc tgt gct gac ccc aag cag aag tgg gtt cag gat tcc atg      292
Lys Glu Ile Cys Ala Asp Pro Lys Gln Lys Trp Val Gln Asp Ser Met
             75                  80                  85

gac cac ctg gac aag caa acc caa act ccg aag act tga acactcactc       341
Asp His Leu Asp Lys Gln Thr Gln Thr Pro Lys Thr  *
         90                  95

cacaacccaa gaatctgcag ctaacttatt ttcccctagc tttccccaga catcctgttt    401
tattttatta taatgaattt tgtttgttga tgtgaaacat tatgccttaa gtaatgttaa    461
ttcttattta agttattgat gttttaagtt tatctttcat ggtactagtg ttttttagat    521
acagagactt ggggaaattg cttttcctct tgaaccacag ttctacccct gggatgtttt    581
gagggtcttt gcaagaatca ttttttaac attccaatgc atttaataca aagaattgct     641
aaaatattat tgtggaaatg                                                661
```

<210> 77
<211> 1847
<212> DNA
<213> Homo sapiens

<220>
<221> CDS
<222> (80)...(346)

<400> 77
tctccgtcag ccgcattgcc cgctcggcgt ccggcccccg acccgtgctc gtccgcccgc 60
ccgcccgccc gcccgcgcc atg aac gcc aag gtc gtg gtc gtg ctg gtc ctc 112
Met Asn Ala Lys Val Val Val Val Leu Val Leu
1 5 10

gtg ctg acc gcg ctc tgc ctc agc gac ggg aag ccc gtc agc ctg agc 160
Val Leu Thr Ala Leu Cys Leu Ser Asp Gly Lys Pro Val Ser Leu Ser
15 20 25

tac aga tgc cca tgc cga ttc ttc gaa agc cat gtt gcc aga gcc aac 208
Tyr Arg Cys Pro Cys Arg Phe Phe Glu Ser His Val Ala Arg Ala Asn
30 35 40

gtc aag cat ctc aaa att ctc aac act cca aac tgt gcc ctt cag att 256
Val Lys His Leu Lys Ile Leu Asn Thr Pro Asn Cys Ala Leu Gln Ile
45 50 55

gta gcc cgg ctg aag aac aac aac aga caa gtg tgc att gac ccg aag 304
Val Ala Arg Leu Lys Asn Asn Asn Arg Gln Val Cys Ile Asp Pro Lys
60 65 70 75

cta aag tgg att cag gag tac ctg gag aaa gct tta aac aag 346
Leu Lys Trp Ile Gln Glu Tyr Leu Glu Lys Ala Leu Asn Lys
80 85

taagcacaac agccaaaaag gactttccgc tagacccact cgaggaaaac taaaaccttg 406
tgagagatga aagggcaaag acgtggggga gggggcctta accatgagga ccaggtgtgt 466
gtgtggggtg ggcacattga tctgggatcg ggcctgaggt ttgcagcatt tagaccctgc 526
atttatagca tacggtatga tattgcagct tatattcatc catgccctgt acctgtgcac 586
gttggaactt ttattactgg ggtttttcta agaaagaaat tgtattatca acagcatttt 646
caagcagtta gttccttcat gatcatcaca atcatcatca ttctcattct catttttaa 706
atcaacgagt acttcaagat ctgaatttgg cttgtttgga gcatctcctc tgctcccctg 766

gggagtctgg gcacagtcag gtggtggctt aacagggagc tggaaaaagt gtcctttctt 826
cagacactga ggctcccgca gcagcgcccc tcccaagagg aaggcctctg tggcactcag 886
ataccgactg gggctggggc gccgccactg ccttcacctc ctctttcaaa cctcagtgat 946
tggctctgtg ggctccatgt agaagccact attactggga ctgtctcaga gacccctctc 1006
ccagctattc ctactctctc cccgactccg agagcatgct taatcttgct tctgcttctc 1066
atttctgtag cctgatcagc gccgcaccag ccgggaagag ggtgattgct ggggctcgtg 1126
ccctgcatcc ctctcctccc agggcctgcc ccacagctcg ggccctctgt gagatccgtc 1186
tttggcctcc tccagaatgg agctggccct ctcctgggga tgtgtaatgg tccccctgct 1246
tacccgcaaa agacaagtct ttacagaatc aaatgcaatt ttaaatctga gagctcgctt 1306
gagtgactgg gtttgtgatt gcctctgaag cctatgtatg ccatggaggc actaacaaac 1366
tctgaggttt ccgaaatcag aagcgaaaaa atcagtgaat aaaccatcat cttgccacta 1426
ccccctcctg aagccacagc aggggttcag gttccaatca gaactgttgg caaggtgaca 1486
tttccatgca tagatgcgat ccacagaagg tcctggtggt atttgtaact ttttgcaagg 1546
catttttta tatatatttt tgtgcacatt tttttttacg attctttaga aaacaaatgt 1606
atttcaaaat atatttatag tcgaacaagt catatatatg aatgagagcc atatgaatgt 1666
cagtagttta tacttctcta ttatctcaaa ctactggcaa tttgtaaaga aatatatatg 1726
atatataaat gtgattgcag cttttcaatg ttagccacag tgtatttttt cacttgtact 1786
aaaattgtat caaatgtgac attatatgca ctagcaataa aatgctaatt gtttcatggt 1846
a 1847

<210> 78
<211> 1160
<212> DNA
<213> Homo sapiens

<220>
<221> CDS
<222> (27)...(299)

<400> 78
cctccgacag cctctccaca ggtacc atg aag gtc tcc gcg gca cgc ctc gct 53
Met Lys Val Ser Ala Ala Arg Leu Ala
1 5

gtc atc ctc att gct act gcc ctc tgc gct cct gca tct gcc tcc cca 101
Val Ile Leu Ile Ala Thr Ala Leu Cys Ala Pro Ala Ser Ala Ser Pro
10 15 20 25

```
tat tcc tcg gac acc aca ccc tgc tgc ttt gcc tac att gcc cgc cca      149
Tyr Ser Ser Asp Thr Thr Pro Cys Cys Phe Ala Tyr Ile Ala Arg Pro
                  30                  35                  40

ctg ccc cgt gcc cac atc aag gag tat ttc tac acc agt ggc aag tgc      197
Leu Pro Arg Ala His Ile Lys Glu Tyr Phe Tyr Thr Ser Gly Lys Cys
              45                  50                  55

tcc aac cca gca gtc gtc ttt gtc acc cga aag aac cgc caa gtg tgt      245
Ser Asn Pro Ala Val Val Phe Val Thr Arg Lys Asn Arg Gln Val Cys
          60                  65                  70

gcc aac cca gag aag aaa tgg gtt cgg gag tac atc aac tct ttg gag      293
Ala Asn Pro Glu Lys Lys Trp Val Arg Glu Tyr Ile Asn Ser Leu Glu
      75                  80                  85

atg agc taggatggag agtccttgaa cctgaactta cacaaatttg cctgtttctg       349
Met Ser
 90

cttgctcttg tcctagcttg ggaggcttcc cctcactatc ctacccccacc cgctccttga    409
agggcccaga ttctgaccac gacgagcagc agttacaaaa accttcccca ggctggacgt    469
ggtggctcag ccttgtaatc ccagcacttt gggaggccaa ggtgggtgga tcacttgagg    529
tcaggagttc gagacagcct ggccaacatg atgaaacccc atgtgtacta aaaatacaaa    589
aaattagccg ggcgtggtag cgggcgcctg tagtcccagc tactcgggag gctgaggcag    649
gagaatggcg tgaacccggg agcggagctt gcagtgagcc gagatcgcgc cactgcactc    709
cagcctgggc gacagagcga gactccgtct caaaaaaaaa aaaaaaaaaa aaaaaaatac    769
aaaaattagc cgcgtggtgg cccacgcctg taatcccagc tactcgggag gctaaggcag    829
gaaaattgtt tgaacccagg aggtggaggc tgcagtgagc tgagattgtg ccacttcact    889
ccagcctggg tgacaaagtg agactccgtc acaacaacaa caacaaaaag cttccccaac    949
taaagcctag aagagcttct gaggcgctgc tttgtcaaaa ggaagtctct aggttctgag   1009
ctctggcttt gccttggctt tgcaagggct ctgtgacaag gaaggaagtc agcatgcctc   1069
tagaggcaag gaagggagga acactgcact cttaagcttc cgccgtctca acccctcaca   1129
ggagcttact ggcaaacatg aaaaatcggg g                                  1160
```

<210> 79
<211> 696
<212> DNA

<213> Homo sapiens

<220>
<221> CDS
<222> (109)...(384)

<400> 79

```
ttcccccccc cccccccccc ccccgcccga gcacaggaca cagctgggtt ctgaagcttc     60
tgagttctgc agcctcacct ctgagaaaac ctcttttcca ccaatacc atg aag ctc     117
                                                     Met Lys Leu
                                                      1

tgc gtg act gtc ctg tct ctc ctc atg cta gta gct gcc ttc tgc tct     165
Cys Val Thr Val Leu Ser Leu Leu Met Leu Val Ala Ala Phe Cys Ser
     5                  10                  15

cca gcg ctc tca gca cca atg ggc tca gac cct ccc acc gcc tgc tgc     213
Pro Ala Leu Ser Ala Pro Met Gly Ser Asp Pro Pro Thr Ala Cys Cys
 20                  25                  30                  35

ttt tct tac acc gcg agg aag ctt cct cgc aac ttt gtg gta gat tac     261
Phe Ser Tyr Thr Ala Arg Lys Leu Pro Arg Asn Phe Val Val Asp Tyr
                 40                  45                  50

tat gag acc agc agc ctc tgc tcc cag cca gct gtg gta ttc caa acc     309
Tyr Glu Thr Ser Ser Leu Cys Ser Gln Pro Ala Val Val Phe Gln Thr
             55                  60                  65

aaa aga agc aag caa gtc tgt gct gat ccc agt gaa tcc tgg gtc cag     357
Lys Arg Ser Lys Gln Val Cys Ala Asp Pro Ser Glu Ser Trp Val Gln
         70                  75                  80

gag tac gtg tat gac ctg gaa ctg aac tgagctgctc agagacagga           404
Glu Tyr Val Tyr Asp Leu Glu Leu Asn
     85                  90

agtcttcagg gaaggtcacc tgagcccgga tgcttctcca tgagacacat ctcctccata     464
ctcaggactc ctctccgcag ttcctgtccc ttctcttaat ttaatctttt ttatgtgccg     524
```

tgttattgta ttaggtgtca tttccattat ttatattagt ttagccaaag gataagtgtc 584
ctatggggat ggtccactgt cactgtttct ctgctgttgc aaatacatgg ataacacatt 644
tgattctgtg tgttttccat aataaaactt taaaataaaa tgcagacagt ta 696

<210> 80
<211> 2738
<212> DNA
<213> Homo sapiens

<220>
<221> CDS
<222> (123)...(353)

<400> 80
gaacaaccca gaaaccttca cctctcatgc tgaagctcac acccttgccc tccaagatga 60
aggtttctgc agcgcttctg tgcctgctgc tcatggcagc cactttcagc cctcagggac 120
tt gct cag cca gat tca gtt tcc att cca atc acc tgc tgc ttt aac 167
Ala Gln Pro Asp Ser Val Ser Ile Pro Ile Thr Cys Cys Phe Asn
1 5 10 15

gtg atc aat agg aaa att cct atc cag agg ctg gag agc tac aca aga 215
Val Ile Asn Arg Lys Ile Pro Ile Gln Arg Leu Glu Ser Tyr Thr Arg
20 25 30

atc acc aac atc caa tgt ccc aag gaa gct gtg atc ttc aag acc caa 263
Ile Thr Asn Ile Gln Cys Pro Lys Glu Ala Val Ile Phe Lys Thr Gln
35 40 45

cgg ggc aag gag gtc tgt gct gac ccc aag gag aga tgg gtc agg gat 311
Arg Gly Lys Glu Val Cys Ala Asp Pro Lys Glu Arg Trp Val Arg Asp
50 55 60

tcc atg aag cat ctg gac caa ata ttt caa aat ctg aag cca 353
Ser Met Lys His Leu Asp Gln Ile Phe Gln Asn Leu Lys Pro
65 70 75

tgagccttca tacatggact gagagtcaga gcttgaagaa aagcttattt attttcccca 413
acctccccca ggtgcagtgt gacattattt tattataaca tccacaaaga gattattttt 473

```
aaataattta aagcataata tttcttaaaa agtatttaat tatatttaag ttgttgatgt    533
tttaactcta tctgtcatac atcctagtga atgtaaaatg caaaatcctg gtgatgtgtt    593
ttttgttttt gttttcctgt gagctcaact aagttcacgg caaaatgtca ttgttctccc    653
tcctacctgt ctgtagtgtt gtggggtcct cccatggatc atcaaggtga aacactttgg    713
tattctttgg caatcagtgc tcctgtaagt caaatgtgtg ctttgtactg ctgttgttga    773
aattgatgtt actgtatata actatggaat tttgaaaaaa aatttcaaaa agaaaaaaat    833
atatataatt taaaactaag aaaaaaaaaa aaaaaaaaaa aaaaaggttt ctattgactt    893
gggttaatcg tgtgaccgcg gtggctggca cgaaattgac caaccctggg gttagtatag    953
cttagttaaa ctttcgttta ttgctaaagg ttaatcactg ctgtttcccg tgggggtgtg   1013
gctaggctaa gcgttttgag ctgcattgct gcgtgcttga tgcttgtccc ttttgatcgt   1073
ggtgatttag agggtgaact cactggaatg gggatgcttg catgtgtaat cttactaaga   1133
gctaatagaa aggctaggac caaaccagaa acctccaatt ctcatgtgga agcccatgcc   1193
ctcaccctcc aacatgaaag cctctgcagc acttctgtgt ctgctgctca cagcagctgc   1253
tttcagcccc caggggcttg ctcagccagt tgggattaat acttcaacta cctgctgcta   1313
cagatttatc aataagaaaa tccctaagca gaggctggag agctacagaa ggaccaccag   1373
tagccactgt ccccgggaag ctgtaatctt caagaccaaa ctggacaagg agatctgtgc   1433
tgaccccaca cagaagtggg tccaggactt tatgaagcac ctggacaaga aaacccaaac   1493
tccaaagctt tgaacattca tgactgaact gaaaacaagc catgacttga gaaacaaata   1553
atttgtatac cctgtccttt ctcagagtgg ttctgagatt attttaatct aattctaagg   1613
aatatgagct ttatgtaata atgtgaatca tggtttttct tagtagattt taaaagttat   1673
taatatttta atttaatctt ccatggattt tggtgggttt tgaacataaa gccttggatg   1733
tatatgtcat ctcagtgctg taaaaactgt gggatgctcc tcccttctct acctcatggg   1793
ggtattgtat aagtccttgc aagaatcagt gcaaagattt gctttaattg ttaagatatg   1853
atgtccctat ggaagcatat tgttattata taattacata tttgcatatg tatgactccc   1913
aaattttcac ataaaataga tttttgtata acaaaaaaaa aaaaaaaaaa aaggacacgg   1973
gcagcagaca gtggtcagtc ctttcttggc tctgctgaca ctcgagccca cattccgtca   2033
cctgctcaga atcatgcagg tctccactgc tgcccttgct gtcctcctct gcaccatggc   2093
tctctgcaac cagttctctg catcacttgc tgctgacacg ccgaccgcct gctgcttcag   2153
ctacacctcc cggcagattc cacagaattt catagctgac tactttgaga cgagcagcca   2213
gtgctccaag cccggtgtca tcttcctaac caagcgaagc cggcaggtct gtgctgaccc   2273
cagtgaggag tgggtccaga aatatgtcag cgacctggag ctgagtgcct gaggggtcca   2333
gaagcttcga ggcccagcga cctcggtggg cccagtgggg aggagcagga gcctgagcct   2393
tgggaacatg cgtgtgacct ccacagctac ctcttctatg gactggttgt tgccaaacag   2453
ccacactgtg ggactcttct taacttaaat tttaatttat ttatactatt tagtttttgt   2513
aatttatttt cgatttcaca gtgtgtttgt gattgtttgc tctgagagtt cccctgtccc   2573
ctccccccttc cctcacaccg cgtctggtga caaccgagtg gctgtcatca gcctgtgtag   2633
gcagtcatgg caccaaagcc accagactga caaatgtgta tcggatgctt ttgttcaggg   2693
ctgtgatcgg cctggggaaa taataaagat gctcttttaa aaggt                   2738
```

<210> 81
<211> 1085
<212> DNA
<213> Homo sapiens

<220>
<221> CDS
<222> (329)...(625)

<400> 81

```
ggtttctatt gacttgggtt aatcgtgtga ccgcggtggc tggcacgaaa ttgaccaacc      60
ctggggttag tatagcttag ttaaactttc gtttattgct aaaggttaat cactgctgtt     120
tcccgtgggg gtgtggctag gctaagcgtt ttgagctgca ttgctgcgtg cttgatgctt     180
gtcccttttg atcgtggtga tttagagggt gaactcactg gaatggggat gcttgcatgt     240
gtaatcttac taagagctaa tagaaaggct aggaccaaac cagaaacctc caattctcat     300
gtggaagccc atgccctcac cctccaac atg aaa gcc tct gca gca ctt ctg        352
                                Met Lys Ala Ser Ala Ala Leu Leu
                                 1               5

tgt ctg ctg ctc aca gca gct gct ttc agc ccc cag ggg ctt gct cag       400
Cys Leu Leu Leu Thr Ala Ala Ala Phe Ser Pro Gln Gly Leu Ala Gln
     10                  15                  20

cca gtt ggg att aat act tca act acc tgc tgc tac aga ttt atc aat       448
Pro Val Gly Ile Asn Thr Ser Thr Thr Cys Cys Tyr Arg Phe Ile Asn
 25                  30                  35                  40

aag aaa atc cct aag cag agg ctg gag agc tac aga agg acc acc agt       496
Lys Lys Ile Pro Lys Gln Arg Leu Glu Ser Tyr Arg Arg Thr Thr Ser
                 45                  50                  55

agc cac tgt ccc cgg gaa gct gta atc ttc aag acc aaa ctg gac aag       544
Ser His Cys Pro Arg Glu Ala Val Ile Phe Lys Thr Lys Leu Asp Lys
             60                  65                  70

gag atc tgt gct gac ccc aca cag aag tgg gtc cag gac ttt atg aag       592
Glu Ile Cys Ala Asp Pro Thr Gln Lys Trp Val Gln Asp Phe Met Lys
```

75 80 85

cac ctg gac aag aaa acc caa act cca aag ctt tgaacattca tgactgaact 645
His Leu Asp Lys Lys Thr Gln Thr Pro Lys Leu
90 95

gaaaacaagc catgacttga gaaacaaata atttgtatac cctgtccttt ctcagagtgg 705
ttctgagatt attttaatct aattctaagg aatatgagct ttatgtaata atgtgaatca 765
tggtttttct tagtagattt taaaagttat taatatttta atttaatctt ccatggattt 825
tggtgggttt tgaacataaa gccttggatg tatatgtcat ctcagtgctg taaaaactgt 885
gggatgctcc tcccttctct acctcatggg ggtattgtat aagtccttgc aagaatcagt 945
gcaaagattt gctttaattg ttaagatatg atgtccctat ggaagcatat tgttattata 1005
taattacata tttgcatatg tatgactccc aaattttcac ataaaataga tttttgtata 1065
acaaaaaaaa aaaaaaaaaa 1085

<210> 82
<211> 775
<212> DNA
<213> Homo sapiens

<220>
<221> CDS
<222> (84)...(359)

<400> 82
aaggacacgg gcagcagaca gtggtcagtc ctttcttggc tctgctgaca ctcgagccca 60
cattccgtca cctgctcaga atc atg cag gtc tcc act gct gcc ctt gct gtc 113
Met Gln Val Ser Thr Ala Ala Leu Ala Val
1 5 10

ctc ctc tgc acc atg gct ctc tgc aac cag ttc tct gca tca ctt gct 161
Leu Leu Cys Thr Met Ala Leu Cys Asn Gln Phe Ser Ala Ser Leu Ala
15 20 25

gct gac acg ccg acc gcc tgc tgc ttc agc tac acc tcc cgg cag att 209
Ala Asp Thr Pro Thr Ala Cys Cys Phe Ser Tyr Thr Ser Arg Gln Ile
30 35 40

cca cag aat ttc ata gct gac tac ttt gag acg agc agc cag tgc tcc 257
Pro Gln Asn Phe Ile Ala Asp Tyr Phe Glu Thr Ser Ser Gln Cys Ser
45 50 55

aag ccc ggt gtc atc ttc cta acc aag cga agc cgg cag gtc tgt gct 305
Lys Pro Gly Val Ile Phe Leu Thr Lys Arg Ser Arg Gln Val Cys Ala
60 65 70

gac ccc agt gag gag tgg gtc cag aaa tat gtc agc gac ctg gag ctg 353
Asp Pro Ser Glu Glu Trp Val Gln Lys Tyr Val Ser Asp Leu Glu Leu
75 80 85 90

agt gcc tgaggggtcc agaagcttcg aggcccagcg acctcggtgg gcccagtggg 409
Ser Ala

gaggagcagg agcctgagcc ttgggaacat gcgtgtgacc tccacagcta cctcttctat 469
ggactggttg ttgccaaaca gccacactgt gggactcttc ttaacttaaa ttttaattta 529
tttatactat ttagtttttg taatttattt tcgatttcac agtgtgtttg tgattgtttg 589
ctctgagagt tcccctgtcc cctcccccctt ccctcacacc gcgtctggtg acaaccgagt 649
ggctgtcatc agcctgtgta ggcagtcatg gcaccaaagc caccagactg acaaatgtgt 709
atcggatgct tttgttcagg gctgtgatcg gcctggggaa ataataaaga tgctctttta 769
aaaggt 775

<210> 83
<211> 98
<212> PRT
<213> Homo sapiens

<400> 83
Met Lys Val Ser Ala Val Leu Leu Cys Leu Leu Leu Met Thr Ala Ala
1 5 10 15
Phe Asn Pro Gln Gly Leu Ala Gln Pro Asp Ala Leu Asn Val Pro Ser
20 25 30
Thr Cys Cys Phe Thr Phe Ser Ser Lys Lys Ile Ser Leu Gln Arg Leu
35 40 45
Lys Ser Tyr Val Ile Thr Thr Ser Arg Cys Pro Gln Lys Ala Val Ile
50 55 60

Phe Arg Thr Lys Leu Gly Lys Glu Ile Cys Ala Asp Pro Lys Glu Lys
65 70 75 80
Trp Val Gln Asn Tyr Met Lys His Leu Gly Arg Lys Ala His Thr Leu
85 90 95
Lys Thr

<210> 84
<211> 10
<212> PRT
<213> Artificial Sequence

<220>
<223> A chemokine peptide variant

<400> 84
Leu Asp Pro Lys Gln Lys Trp Ile Gln Cys
1 5 10

<210> 85
<211> 4
<212> PRT
<213> Homo sapiens

<400> 85
Trp Val Gln Cys
1

<210> 86
<211> 4
<212> PRT
<213> Artificial Sequence

<220>
<223> A chemokine peptide variant

<400> 86
Trp Ile Gln Cys

1

<210> 87
<211> 10
<212> PRT
<213> Homo sapiens

<400> 87
Ser Tyr Arg Arg Ile Thr Ser Ser Lys Cys
1 5 10

<210> 88
<211> 6
<212> PRT
<213> Homo sapiens

<400> 88
Cys Pro Lys Glu Ala Val
1 5

<210> 89
<211> 5
<212> PRT
<213> Homo sapiens

<400> 89
Ser Tyr Arg Arg Ile
1 5

<210> 90
<211> 5
<212> PRT
<213> Homo sapiens

<400> 90
Thr Ser Ser Lys Cys
1 5

<210> 91
<211> 9
<212> PRT
<213> Homo sapiens

<400> 91
Asp Tyr Phe Glu Thr Ser Ser Gln Cys
1 5

<210> 92
<211> 6
<212> PRT
<213> Homo sapiens

<400> 92
Cys Ser Lys Pro Gly Val
1 5

<210> 93
<211> 17
<212> PRT
<213> Artificial Sequence

<220>
<223> A chemokine peptide variant

<400> 93
Cys Ser Tyr Arg Arg Ile Thr Ser Ser Lys Ser Pro Lys Glu Ala Val
1 5 10 15
Cys

<210> 94
<211> 9
<212> PRT
<213> Homo sapiens

<400> 94

Ser Tyr Arg Arg Ile Thr Ser Ser Lys
1 5

<210> 95
<211> 11
<212> PRT
<213> Unknown

<220>
<223> A peptide sequence from an unidentified source.

<400> 95
Cys Gln Val Trp Lys Gln Lys Pro Asp Ala Cys
1 5 10

<210> 96
<211> 4
<212> PRT
<213> Unknown

<220>
<223> A peptide sequence from an unidentified source.

<400> 96
Lys Lys Phe Lys
1

<210> 97
<211> 4
<212> PRT
<213> Unknown

<220>
<223> A peptide sequence from an unidentified source.

<400> 97
Arg Lys Pro Lys
1

<210> 98
<211> 4
<212> PRT
<213> Unknown
<220>
<223> A peptide sequence from an unidentified source.

<400> 98
Lys Arg Phe Lys
1

<210> 99
<211> 13
<212> PRT
<213> Unknown

<220>
<223> A peptide sequence from an unidentified source.

<400> 99
Leu Tyr Ile Asp Phe Arg Gln Asp Leu Gly Trp Lys Trp
1 5 10

<210> 100
<211> 11
<212> PRT
<213> Unknown

<220>
<223> A peptide sequence from an unidentified source.

<400> 100
His Glu Pro Lys Gly Tyr His Ala Asn Phe Cys
1 5 10

<210> 101
<211> 7
<212> PRT

<213> Unknown

<220>
<223> A peptide sequence from an unidentified source.

<400> 101
Val Tyr Tyr Val Gly Arg Lys
1 5

<210> 102
<211> 13
<212> PRT
<213> Unknown

<220>
<223> A peptide sequence from an unidentified source.

<400> 102
Lys Val Glu Gln Leu Ser Asn Met Val Val Lys Ser Cys
1 5 10

<210> 103
<211> 23
<212> PRT
<213> Unknown

<220>
<223> A peptide sequence from an unidentified source.

<400> 103
Tyr Asn Phe Thr Asn Arg Lys Ile Ser Val Gln Arg Leu Ala Ser Tyr
1 5 10 15
Arg Arg Ile Thr Ser Ser Lys
20

<210> 104
<211> 15
<212> PRT

<213> Artificial Sequence

<220>
<223> A chemokine peptide variant

<400> 104
Ser Tyr Arg Arg Ile Thr Ser Ser Lys Ser Pro Lys Glu Ala Val
1               5                   10                  15

## Description

### Cross-Reference to Related Applications

This application is a continuation-in-part application of U.S. application Serial No. 09/271,192, filed March 17, 1999 the disclosure of which is incorporated by reference herein. This is a divisional application of EP application No. 00904325.8.

### Background of the Invention

Macrophage/monocyte recruitment plays a role in the morbidity and mortality of a broad spectrum of diseases, including autoimmune diseases, granulomatous diseases, allergic diseases, infectious diseases, osteoporosis and coronary artery disease. For example, in atherosclerosis early during lipid lesion formation, circulating monocytes adhere to the activated endothelium overlying the incipient plaque. Under appropriate conditions, the monocytes then migrate into the developing intima. In the intima, macrophage accumulate lipoprotein and excrete an excess of proteases relative to protease inhibitors. If the lipoproteins are oxidized, they are toxic to macrophage, which results in macrophage death and an increase in an unstable, necrotic, extracellular lipid pool. An excess of proteases results in loss of extracellular matrix and destabilization of the fibrous plaque. Plaque instability is the acute cause of myocardial infarction.

Many molecules have been identified that are necessary for the recruitment of monocytes and other inflammatory cell types to sites of injury or insult. These molecules represent targets for the inhibition of monocyte recruitment. One class of such molecules is adhesion molecules, e.g., receptors, for monocytes. Another class of molecules includes inflammatory mediators, such as TNF-$\alpha$ and related molecules, the interleukins, e.g., IL-1$\beta$, and chemokines, e.g., monocyte chemoattractant protein-1 (MCP-1). As a result, agents which modulate the activity of chemokines are likely to be useful to prevent and treat a wide range of diseases. For example, Rollins et al. (U.S. Patent No. 5,459,128) generally disclose analogs of MCP-1 that inhibit the monocyte chemoattractant activity of endogenous MCP-1. Analogs that are effective to inhibit endogenous MCP-1 are disclosed as analogs which are modified at 28-tyrosine, 24-arginine, 3-aspartate and/or in amino acids between residues 2-8 of MCP-1. In particular, Rollins et al. state that "[s]uccessful inhibition of the activity is found where MCP-1 is modified in one or more of the following ways: a) the 28-tyrosine is substituted by aspartate, b) the 24-arginine is substituted by phenylalanine, c) the 3-aspartate is substituted by alanine, and/or d) the 2-8 amino acid sequence is deleted" (col. 1, lines 49-54). The deletion of amino acids 2-8 of MCP-1 ("MCP-1 (Δ2-8)") results in a polypeptide that is inactive, i.e., MCP-1(△2-8) is not a chemoattractant (col. 5, lines 22-23). The only effective MCP-1 inhibitor disclosed in Rollins et al. is MCP-1(△2-8).

Recent studies suggest that MCP-1(△2-8) exhibits a dominant negative effect, i.e., it forms heterodimers with wild-type MCP-1 that cannot elicit a biological effect (Zhang et al., J. Biol_ Chem., 269,15918 (1994); Zhang et al., Mol. Cell. Biol., 15, 4851 (1995)). Thus, MCP-1(△2-8) does not exhibit properties of a classic receptor antagonist. Moreover, MCP-1(Δ2-8) is unlikely to be widely useful for inhibition of MCP-1 activity *in vivo,* as MCP-1(△2-8) is a large polypeptide with undesirable pharmacodynamic properties. Furthermore, it is unknown whether MCP-1(Δ2-8) is active as a dominant-negative inhibitor of other chemokines associated with inflammation.

Thus, there is a need to identify agents that inhibit or enhance chemokine-induced macrophage and/or monocyte recruitment and which have desirable pharmacodynamic properties. Moreover, there is a need to identify agents that inhibit or enhance chemokine-induced activities of other cell types, such as lymphocytes, neutrophils or eosinophils. Further, there is a need to identify agents that are pan-selective chemokine inhibitors.

### Summary of the Invention

The invention provides a therapeutic agent comprising an isolated and purified chemokine peptide, chemokine peptide variant, chemokine analog, or a derivative thereof. Preferably, the therapeutic agent of the invention inhibits the activity of more than one chemokine, although the agent may not inhibit the activity of all chemokines to the same extent. Alternatively, a preferred therapeutic agent of the invention specifically inhibits the activity of one chemokine to a greater extent than other chemokines. Yet another preferred therapeutic agent of the invention mimics the activity of a chemokine, e.g., it acts as an agonist. Thus, therapeutic agents that are chemokine antagonists and agonists are within the scope of the invention. A further preferred therapeutic agent of the invention is an agent that does not inhibit or mimic the activity of a chemokine but binds to or near the receptor for that chemokine, i.e., it is a neutral agent.

A preferred embodiment of the invention is an isolated and purified CC chemokine peptide 3, e.g., a peptide derived from MCP- which corresponds to about residue 46 to about residue 67 of mature MCP-1 ("peptide 3 [MCP-1]"), a variant, an analog, or a derivative thereof. It is contemplated that chemokine peptide 3, a variant, an analog or a derivative thereof is a chemokine receptor antagonist, although these therapeutic agents may exert their effect by a different mechanism, e.g., by inhibiting the arachidonic acid pathway (e.g., inhibition of leukotriene, thromboxane, or

prostaglandin synthesis or stability) or by elevating TGF-beta levels, or by more than one mechanism.

A preferred peptide 3 of the invention is a compound of formula (I):

$$[[(X^2)\text{-}(X^3)\text{-}C\text{-}(X)\text{-}(X^7)\text{-}P]_a\text{-}[(X^4)\text{-}(Z)\text{-}(X^5)\text{-}W\text{-}(X^1)\text{-}(X^6)]_b]_c$$

wherein $X^2$ is E, Q, D, N, L, P, I or M, wherein $X^3$ is I, V, M, A, P, norleucine or L, wherein X is A, L, V, M, P, norleucine or I, wherein $X^4$ is K, S, R, Q, N or T, wherein Z is Q, K, E, N, R, I, V, M, A, P, norleucine or L, wherein $X^7$ is D or P, wherein $X^5$ is K, E, R, S, Q, D, T, G, H or N, wherein $X^1$ is V, L, M, P, A, norleucine, or I, wherein $X^6$ is Q, N, K or R, wherein a is 0-6, wherein b is 0-6, and wherein c is 1-6, with the proviso that a and b cannot both be 0. The letters in formulas (I)-(III) that are not X, Y or Z represent peptidyl residues as shown in Figure 9. A more preferred peptide 3 of the invention is a compound of formula (I):

$$[[(X^2)\text{-}(X^3)\text{-}C\text{-}(X)\text{-}D\text{-}P]_a\text{-}[(X^4)\text{-}(Z)\text{-}(X^5)\text{-}W\text{-}(X^1)\text{-}(X^6)]_b]_c$$

wherein $X^2$ is E, Q or M, wherein $X^3$ is I, V or L, wherein X is A, L or I, wherein $X^4$ is K, S or T, wherein Z is Q, K, E or L, wherein $X^5$ is K, E, R, S or T, wherein $X^1$ is V or I, wherein $X^6$ is Q or R, wherein a is 0-6, wherein b is 0-6, and wherein c is 1-6, with the proviso that a and b cannot both be 0.

Yet another preferred peptide 3 of the invention is a compound of formula (II):

$$[[(X^4)\text{-}(Z)\text{-}(X^5)]_a\text{-}[W\text{-}(X^1)\text{-}(X^6)]_b]_c$$

wherein $X^4$ is K, S or T, wherein Z is Q, K, E or L, wherein $X^5$ is K, E, R, S or T, wherein $X^1$ is V or I, wherein $X^6$ is Q or R, wherein a is 0-6, wherein b is 0-6, and wherein c is 1-6, with the proviso that a and b cannot both be 0.

Another preferred peptide 3 of the invention is a compound of formula (II):

$$[[(X^4)\text{-}(Z)\text{-}(X^5)]_a\text{-}[W\text{-}(X^1)\text{-}(X^6)]_b]_c$$

wherein $X^4$ is K, S, R, Q, N or T, wherein Z is Q, K, E, N, R, I, V, M, A, P, norleucine or L, wherein $X^5$ is K, E, R, S, Q, D, T, G, H or N, wherein $X^1$ is V, L, M, P, A, norleucine, or I, wherein $X^6$ is Q, N, K or R, wherein a is 0-6, wherein b is 0-6, and wherein c is 1-6, with the proviso that a and b cannot both be 0.

A more preferred peptide 3 of the invention is a compound of formula (XIII):

$$(X^8)\text{-}(X)\text{-}D\text{-}(X^2)\text{-}(X^4)\text{-}(Z)\text{-}(X^5)\text{-}W\text{-}(X^1)\text{-}Q\text{-}(X^7)$$

wherein X is A, L, V or I, wherein $X^2$ is P, G or L, wherein $X^4$ is K, T, R or N, wherein Z is Q, K, A or L, wherein $X^5$ is K, E, R, Q or P, wherein $X^1$ is V, L, A, M, F or I, and wherein $X^8$ and $X^7$ are independently C or absent.

A preferred embodiment of the invention is an isolated and purified CC chemokine peptide 3, e.g., a peptide derived from MCP-1 which corresponds to SEQ ID NO:1 ("peptide 3(1-12)[MCP-1]") or SEQ ID NO:7 ("peptide 3(3-12)[MCP-1]"), a fragment, a variant, an analog, or a derivative thereof. As described hereinbelow, peptide 3(1-12)[MCP-1] (SEQ ID NO:1) and peptide 3(3-12)[MCP-1] (SEQ ID NO:7) are pan-chemokine inhibitors, bioavailable, and have desirable pharmacokinetics. Another preferred CC chemokine peptide 3 of the invention is peptide 3[MIP1$\alpha$], and more preferably peptide 3(1-12)[MIP1$\alpha$] which has an amino acid sequence corresponding to SEQ ID NO:42, a variant, an analog, a fragment or a derivative thereof.

Further preferred embodiments of the invention are a CC chemokine peptide 3 such as peptide 3(1-12)[MCP-4] (e.g., SEQ ID NO:65), peptide 3(1-12)[MCP-3](e.g., SEQ ID NO:66), peptide 3(1-12)[MCP-2] (e.g., SEQ ID NO:67), peptide 3(1-12)[eotaxin] (e.g., SEQ ID NO:68), peptide 3(1-12)[MIP1$\alpha$],(e.g., SEQ ID NO:42), peptide 3(1-12)[MIP1$\beta$] (e.g., SEQ ID NO:43), peptide 3(1-12)[RANTES](e.g., SEQ ID NO:44), or a fragment thereof.

Another preferred embodiment of the invention includes a CXC chemokine peptide 3, a variant, an analog or

a derivative thereof. A preferred CXC peptide 3 of the invention is a compound of formula (III):

$$[[(X^2)-(X^3)-C-L-(X)-(X^7)]_a-[(X^4)-(Z)-(X^5)-(X^8)-(X^1)-(X^6)]_b]_c$$

wherein $X^2$ is E or K, wherein $X^3$ is I, A, R or L, wherein X is D or N, wherein $X^7$ is Q, P or L, wherein $X^4$ is E, K, D, A or Q, wherein Z is A, R, S or E, wherein $X^5$ is P, N or K, wherein $X^8$ is F, W, R, I, M, L or A, wherein $X^1$ is L, V, Y or I, wherein $X^6$ is K or Q, wherein a is 0-6, wherein b is 0-6, and wherein c is 1-6, with the proviso that a and b cannot both be 0.

Further preferred embodiments of the invention are a CXC chemokine peptide 3 such as peptide 3(1-12)[IL8] (e.g., SEQ ID NO:40), peptide 3(1-12)[SDF-1](e.g., SEQ ID NO:38), peptide 3(1-12)[ENA-78](e.g., SEQ ID NO:41), peptide 3(1-12)[GROα](e.g., SEQ ID NO:72), peptide 3(1-12)[GROβ](e.g., SEQ ID NO:73), peptide 3(1-12)[GROγ](e.g., SEQ ID NO:74), or fragments thereof.

Yet other preferred embodiments of the invention are a $CX_2C$, $CX_3C$ or C chemokine peptide 3, a variant, an analog or a derivative thereof.

Preferably, a chemokine peptide 3, its variants, analogs or derivatives inhibits the arachidonic acid pathway, e.g., inhibits the synthesis or stability, or binding, of thromboxane, prostaglandin, leukotriene, or any combination thereof.

Another preferred embodiment of the invention includes a chemokine peptide 3 that is at least a tripeptide, a variant thereof or a derivative thereof. A preferred embodiment of the invention is the MCP-1 tripeptide KQK (i.e., peptide 3(9-12)[MCP-1], which specifically inhibits MCP-1, but not MIP1α, IL8 and SDF1α, chemokine-induced activity. Other preferred embodiments of the invention include isolated and purified chemokine tripeptides that specifically inhibit IL8, MIP1α, SDF1, MCP-1, MCP-2, MCP-3, and MIP1β, e.g., KEN, SEE, KLK, KKE, KER, TQK, and SES, respectively. A further preferred embodiment of the invention is a chemokine peptide 3 tripeptide that inhibits the activity of more than one chemokine, e.g., WVQ or WIQ. Preferably, a tripeptide of the invention is not RFK.

Yet another embodiment of the invention is a peptide which includes the amino acid sequence KXK, wherein X is an amino acid, preferably one of the twenty naturally occurring amino acids, and which peptide is a chemokine antagonist, activates TGF-beta (TGF-betal, TGF-beta2, TGF-beta3, or a combination thereof), or a combination thereof. Preferably, the peptide increases the activation of TGF-betal. It is preferred that a peptide which includes the amino acid sequence KXK is less than about 15, preferably about 10, and more preferably about 8 amino acid residues in length. Preferably, the peptide is not KKFK or RKPK. A further embodiment of the invention is a peptide which includes a basic amino acid residue followed by phenylalanine followed by another basic residue, wherein the peptide is not RFK, is not KRFK, or does not contain RFK or KRFK.

Another preferred peptide of the invention is a compound of formula (VII):

$$(X^1)-(Y)-(K)-(X^2)-K-(X^3)$$

wherein $X^2$ is V, A, D, E, P, R, C, H, M, F, K, L, N, Q, Y, or I; wherein Y is absent or is an amino acid that is not R or K; and wherein $X^1$ and $X^3$ are independently 0-20 amino acid residues or absent. Preferably, $X^2$ is F, K, L, N, Q, Y, or 1. More preferably, $X^2$ is F, K, L, N, Q, Y, or I, and Y, $X^1$ and $X^3$ are absent.

To identify a peptide of the invention useful in the methods of the invention, a sequence comparison of the receptor ligand under study from a variety of different species is performed, then the cross-reactivity of the receptor ligand from each species to the human receptor is assessed. The preferred sequence(s) are then obtained from the species which has the least sequence homology to the corresponding human receptor ligand, but which still binds and signals through the human receptor. The sequence of this most divergent but still functional receptor ligand is then aligned with the human sequence in order to identify regions that are conserved. Such conserved regions represent peptides of the invention useful in the methods of the invention. For example, the process has been applied to identify peptides in human MCP-1 having, for example, antagonists, agonists or neutral properties. Such peptides include, but are not limited to, peptide 3, peptide 2 (described below) and related molecules (see below).

Another example is the identification of peptides in the sequence of the cytokine TGF-beta having antagonist, agonist, or neutral receptor binding properties. The amino acid sequence of human TGF-betal was compared to that of *Xenopus.* Peptides identified by this method include LYIDFRQDLGWKW ("T1"; SEQ ID NO:111); HEPKGYHANFC ("T2"; SEQ ID NO:112); VYYVGRK ("T3"; SEQ ID NO:113) and KVEQLSNMVVKSC ("T4"; SEQ ID NO:114). Biotinylated T1 bound to the TGF-beta receptor of THP-1 cells with an $ED_{50}$ of 18 μM and is a receptor neutral agent (i.e., neither agonist nor antagonist). Biotinylated T2 bound to the TGF-beta receptor of THP-1 cells with an $ED_{50}$ of 30 μM and is a weak receptor antagonist.

Also provided is an isolated and purified chemokine peptide variant, or a derivative thereof. A chemokine

peptide variant has at least about 50%, preferably at least about 80%, and more preferably at least about 90% but less than 100%, contiguous amino acid sequence homology or identity to the amino acid sequence of the corresponding native chemokine, e.g., $Ser_7$ peptide 3(1-12)[MCP1] (SEQ ID NO:11) has less than 100% contiguous homology to the corresponding amino acid sequence of MCP-1, i.e., a peptide having SEQ ID NO:1. A preferred peptide 3 variant is $Leu_4Ile_{11}$peptide 3(3-12)[MCP-1], i.e., it is a ten amino acid peptide derived from peptide 3(1-12)[MCP-1] that lacks amino acid residues 1 and 2 of peptide 3(1-12)[MCP-1], and which has a leucine rather than alanine at position 4 of peptide 3(1-12)[MCP-1], and an isoleucine rather than valine at position eleven of peptide 3(1-12)[MCP-1].

The invention also provides derivatives of chemokine peptides and peptide variants. A preferred derivative is a cyclic reverse sequence D isomer (CRD) derivative of a chemokine peptide, a variant or an analog thereof of the invention. For example, CRD-$Cys_{13}Leu_4Ile_{11}$peptide 3(3-12)[MCP-1] are compounds of the invention that are particularly useful in the practice of the methods of the invention, as described hereinbelow.

Also provided are certain analogs of chemokines. In particular, analogs of chemokine peptide 3, or variants thereof are contemplated. A preferred analog of chemokine peptide 3 is an analog of WIQ, including a compound of formula (IV):

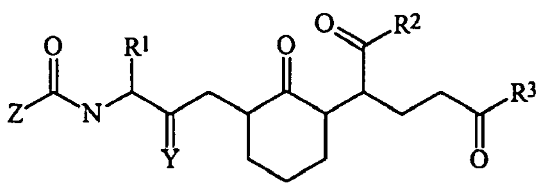

(IV)

wherein $R^1$ is aryl, heteroaryl, aryl($C_1$-$C_3$)alkyl, heteroaryl($C_1$-$C_3$)alkyl, coumaryl, coumaryl($C_1$-$C_3$)alkyl, chromanyl or chromanyl($C_1$-$C_3$)alkyl; wherein any aryl or heteroaryl group, or the benz-ring of any coumaryl or chromanyl group may optionally be substituted with one, two or three substituents selected from the group consisting of halo, nitro, cyano, hydroxy, ($C_1$-$C_6$)alkyl, ($C_1$-$C_6$)alkoxy, ($C_1$-$C_6$)alkanoyl, ($C_2$-$C_6$)alkanoyloxy, -C(=O)($C_1$-$C_6$)alkoxy, C(=O)$NR^gR^h$, $NR^iR^j$;

wherein $R^2$ is ($C_1$-$C_{10}$)alkyl, ($C_3$-$C_6$)cycloalkyl, ($C_3$ $C_6$)cycloalkyl($C_1$-$C_6$)alkyl, ($C_1$-$C_{10}$)alkoxy, ($C_3$-$C_6$)cycloalkyl($C_1$-$C_6$) alkoxy or N($R^a$)($R^b$);

wherein $R^3$ is ($C_1$-$C_{10}$)alkyl, ($C_3$-$C_6$)cycloalkyl, ($C_3$-$C_6$)cycloalkyl($C_1$-$C_6$)alkyl, ($C_1$-$C_{10}$)alkoxy, ($C_3$-$C_6$)cycloalkyl($C_1$-$C_6$) alkoxy or N($R^c$)($R^d$);

wherein Y is oxo or thioxo;

wherein Z is ($C_1$-$C_{15}$)alkyl, ($C_3$-$C_6$)cycloalkyl, ($C_3$-$C_6$)cycloalkyl($C_1$-$C_6$)alkyl, ($C_1$-$C_{15}$)alkoxy, ($C_3$-$C_6$)cycloalkyl($C_1$-$C_6$) alkoxy or N($R^e$)($R^f$); and

wherein $R^a$-$R^j$ are each independently hydrogen, ($C_1$-$C_{10}$)alkyl, ($C_1$-$C_{10}$)alkanoyl, phenyl, benzyl, or phenethyl; or $R^a$ and $R^b$, $R^c$ and $R^d$, $R^e$ and $R^f$, $R^g$ and $R^h$, or $R^i$ and $R^i$ together with the nitrogen to which they are attached form a ring selected from pyrrolidino, piperidino, or morpholino; or a pharmaceutically acceptable salt thereof.

A preferred embodiment of a compound of formula (IV) includes a compound of a formula (IV) wherein $R^1$ is aryl, heteroaryl, coumaryl, or chromanyl. Preferably aryl is phenyl; and heteroaryl is indolyl or pyridinyl. Another preferred embodiment of a compound of formula (IV) includes a compound of a formula (IV) wherein $R^2$ is N($R^a$)($R^b$); and $R^3$ is N($R^c$)($R^d$). Yet another preferred embodiment of a compound of formula (IV) includes a compound of a formula (IV) wherein Z is ($C_1$-$C_{15}$)alkyl.

Another preferred compound of formula (IV) is wherein $R^1$ is aryl, heteroaryl, coumaryl or chromanyl; wherein $R^2$ is N($R^a$)($R^b$); wherein $R^3$ is N($R^c$)($R^d$); wherein Y is oxo or thioxo; wherein Z is ($C_1$-$C_{15}$)alkyl; wherein $R^a$-$R^d$ are each independently hydrogen, ($C_1$-$C_{10}$)alkyl, ($C_1$-$C_{10}$)alkanoyl, phenyl, benzyl or phenethyl; or wherein $R^a$ and $R^b$, or $R^c$ and $R^d$, together with the nitrogen to which they are attached form a pyrrolidino, piperidino or morpholino ring; or a pharmaceutically acceptable salt thereof.

A further preferred compound is a compound of formula (IV) wherein $R^1$ is indolyl; $R^2$ is N($R^a$)($R^b$); $R^3$ is N($R^c$)($R^d$); Y is S; Z is hydrogen; and $R^a$, $R^b$, $R^c$, and $R^d$ are each methyl.

A further preferred analog of WIQ is a compound of formula (XIV):

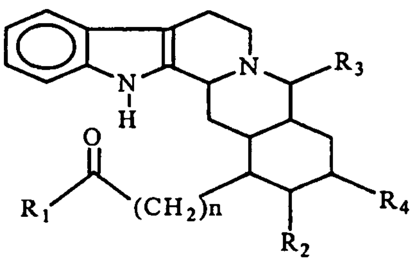

(XIV)

wherein $R_1$ is O(Ra) wherein Ra is H, $(C_1\text{-}C_6)$alkyl, $(C_1\text{-}C_6)$alkanoyl, $(C_1\text{-}C_6)$alkanoyloxy, $(C_6\text{-}C_{10})$aryl or $(C_6\text{-}C_{10})$heteroaryl; or N(Rb)(Rc) wherein each Rb and Rc is independently H or $(C_1\text{-}C_6)$alkyl; $R_2$ is O(Ra) wherein Ra is H, $(C_1\text{-}C_6)$alkyl, $(C_1\text{-}C_6)$alkanoyl, $(C_1\text{-}C_6)$alkanoyloxy, $(C_6\text{-}C_{10})$aryl or $(C_6\ C_{10})$heteroaryl; or N(Rb)(Rc) wherein each Rb and Rc is independently H or $(C_1\text{-}C_6)$alkyl; $R_3$ is H, C(=O) or C(=S); $R_4$ is H, C(=O), C(=S), O(Ra), or N(Rb)(Rc); each Ra is H, $(C_1\text{-}C_6)$alkyl, $(C_1\text{-}C_6)$alkanoyloxy, $(C_1\text{-}C_6)$alkanoyl, $(C_6\text{-}C_{10})$aryl or $(C_6\text{-}C_{10})$heteroaryl; each Rb and Rc is independently H or $(C_1\text{-}C_6)$alkyl; and n is an integer between 0 and 6, inclusive; wherein any $(C_6\text{-}C_{10})$aryl, $(C_1\text{-}C_6)$alkyl, $(C_1\text{-}C_6)$alkanoyloxy, $(C_6\text{-}C_{10})$heteroaryl or $(C_1\text{-}C_6)$alkanoyl or the benzo ring in formula (XIV) is optionally substituted with at least one substituent (e.g. 1, 2, 3, or 4) selected from the group consisting of halo, cyano, nitro, trifluoromethyl, trifluoromethoxy, $(C_1\text{-}C_6)$alkyl, $(C_1\text{-}C_6)$alkoxy, $(C_1\text{-}C_6)$alkanoyl, $(C_1\text{-}C_6)$alkanoyloxy, $(C_1\text{-}C_6)$alkoxycarbonyl, methoxydioxy, hydroxy, C(=O), sulfino ($SO_2H$), sulfo ($SO_3H$), and N(Rb)(Rc) wherein or a pharmaceutically acceptable salt thereof.

Another preferred analog of WIQ is a compound of formula (XIV) wherein $R_1$ is $O(R_a)$, an amino acid, or $N(R_b)(R_c)$; $R_2$ is $O(R_a)$ or $N(R_b)(R_c)$; $R_3$ is H, oxo or thioxo; $R_4$ is H, oxo, thioxo, $O(R_a)$ or $N(R_b)(R_c)$; n is 0, 1, 2, 3, 4, 5 or 6; each $R_a$ is independently hydrogen, $(C_1\text{-}C_6)$alkyl, $(C_1\text{-}C_6)$alkanoyl, aryl, heteroaryl, or a saccharide; $R_b$ and $R_c$ are each independently H or $(C_1\text{-}C_6)$alkyl; wherein any $(C_1\text{-}C_6)$alkyl, $(C_1\text{-}C_6)$alkanoyl, $(C_1\text{-}C_6)$alkanoyloxy,aryl or heteroaryl is optionally substituted with one or more (e.g. 1, 2, 3, or 4) substituents selected from the group consisting of halo, cyano, nitro, trifluoromethyl, trifluoromethoxy, $(C_1\text{-}C_6)$alkyl, $(C_1\text{-}C_6)$allcoxy, $(C_1\text{-}C_6)$alkanoyl, $(C_1\text{-}C_6)$alkanoyloxy, $(C_1\text{-}C_6)$alkoxycarbonyl, hydroxy, oxo, sulfino ($SO_2H$), sulfo ($SO_3H$), and $N(R_b)(R_c)$; or a pharmaceutically acceptable salt thereof.

For a compound of formula (XIV), $R_1$ can specifically be N(Rb)(Rc) wherein Rb is H and Rc is H; $R_2$ can specifically be O(Ra) wherein Ra is H, $-CH_2C(OH)C(OH)C(OH)C(OH)CHO$ or $-CH_2C(OH)C(OH)C(OH)C(NH)CHO$; $R_3$ can specifically be H; $R_4$ can specifically be H; and n can specifically be 1.

Specific compounds of formula (XIV) are compounds wherein $R_1$ is amino, methylamino, dimethylamino or N-linked-glutamine; $R_2$ is hydroxy, $R_3$ is hydrogen, $R_4$ is hydrogen, and n is 0 or 1; or a pharmaceutically acceptable salt thereof.

Other specific compounds of formula (XIV) are compounds wherein the benzo ring of formula (XIV) is optionally substituted.

Preferred compounds of formula (XIV) possess the ring stereochemistry of yohimbine, however, the invention also provides compounds of formula (XIV) having the alloyohimbine and the rauwolscine ring systems.

The compounds of the invention preferably exclude compounds of formula (XIV) wherein $R_1$ is a β- amino, hydroxy, or methoxy, when n is 0, $R_2$ is an α- methoxy, $R_3$ is hydrogen, and $R_4$ is hydroxy (Baxter et al., J.Am. Chem. Soc., 1990, 112, 7682-7692); compounds of formula (XIV) wherein $R_1$ is methoxy, when n is 0, $R_2$ is hydroxy or acetoxy, $R_3$ is hydrogen, and $R_4$ is hydrogen (Szantay et al., Chem. Ber., 1976, 109, 1737-1748; Toke et al., J. Org. Chem., 1973, 38, 2496-2500 and 2501-2509); compounds of formula (XIV) wherein $R_1$, is methoxy or amino, when n is 0, $R_2$ is hydroxy, $R_3$ is hydrogen, and $R_4$ is hydrogen (Toke et al., J. Org. Chem., 1973, 38, 2501-2509; Toke et al, Chem. Ber., 1969, 102, 3249-3259); and compounds of formula (XIV) wherein $R_1$ is a β- $O(R_a)$ or $N(R_b)(R_c)$ wherein $R_a$ is hydrogen or $(C_1\text{-}C_6)$alkyl and $R_b$ and $R_c$ are each hydrogen, when, n is 0, $R_2$ is an α- $O(R_a)$ wherein $R_a$ is hydrogen, $(C_1\text{-}C_6)$alkyl, or $(C_1\text{-}C_6)$alkanoyl, $R_3$ is hydrogen, and $R_4$ is hydrogen (U.S. Patent Number 5,807,482).

Another analog of WGQ useful in the methods of the invention is a compound of formula (X):

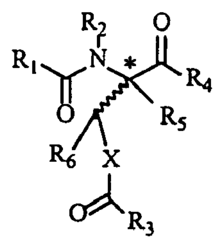

(X)

wherein: $R_1$ is $(C_1-C_{15})$alkyl, $(C_2-C_{15})$alkenyl, $(C_2-C_{15})$alkynyl, $(C_1-C_{15})$alkoxy, aryl, heteroaryl, aryl$(C_1-C_6)$alkyl, heteroaryl$(C_1-C_6)$alkyl; and $R_2$ is hydrogen or $(C_1-C_{15}$ is)alkyl; or $R_1$ and $R_2$ together with the atoms to which they are attached are a five or six membered heterocyclic ring comprising four or five carbon atoms, optionally substituted on carbon with oxo, and optionally substituted with a fused benzo group; $R_3$ is hydroxy, $(C_1-C_6)$alkoxy, or $N(R_{a)}(R_b)$; and $R_4$ is hydroxy, $(C_1-C_6)$alkoxy, or $N(R_a)(R_b)$ ; or $R_3$ and $R_4$ together with the atoms to which they are attached are a five or six membered heterocyclic ring comprising four or five carbon atoms and $N(R_c)$; $R_5$ is hydrogen or $(C_1-C_6)$alkyl; $R_6$ is hydrogen or oxo; X is a direct bond, N(H), or methylene ($-CH_2-$); each $R_a$ and $R_b$ is independently hydrogen, $(C_1-C_6)$alkyl, $(C_1-C_6)$alkanoyl, $(C_1-C_6)$alkoxycarbonyl, aryl, aryl$(C_1-C_6)$allcyl, aryl$(C_1-C_6)$alkoxy, aryl$(C_1-C_6)$alkanoyl, aryl$(C_1-C_6)$alkoxycarbonyl, heteroaryl, heteroaryl$(C_1-C_6)$alkyl heteroaryl$(C_1-C_6)$alkoxy, heteroaryl$(C_1-C_6)$alkanoyl, or heteroaryl$(C_1-C_6)$alkoxycarbonyl; and $R_c$ is hydrogen or $(C_1-C_6)$alkyl; wherein any $(C_1-C_{15})$alkyl, $(C_2-C_{15})$alkenyl, $(C_2-C_{15})$alkynyl, or $(C_1-C_{15})$alkoxy of $R_1$ is optionally substituted with one or more substituents selected from the group consisting of halo, cyano, nitro, carboxy, trifluoromethyl, trifluoromethoxy, $(C_1-C_6)$alkyl, $(C_1-C_6)$alkoxy, $(C_1-C_6)$alkanoyl, $(C_1-C_6)$alkanoyloxy, $(C_1-C_6)$alkoxycarbonyl, hydroxy, oxo, and $N(R_a)(R_b)$; and wherein any aryl, heteroaryl, or benzo of $R_1$, $R_a$, and $R_b$, is optionally substituted with 1, 2, 3, or 4 substituents independently selected from the group consisting of halo, cyano, hydroxy, nitro, trifluoromethyl, trifluoromethoxy, $(C_1-C_6)$alkyl, $(C_1-C_6)$alkoxy, $(C_1-C_6)$alkanoyl, $(C_1-C_6)$alkanoyloxy, $(C_1-C_6)$alkoxycarbonyl, sulfino ($SO_2H$), sulfo ($SO_3H$), and methylenedioxy; or a pharmaceutically acceptable salt thereof.

A preferred group of compounds of formula (X) are compounds wherein $R_1$ is 9-decenyl, *tert*-butoxy, phenyl, 4-hydroxyphenyl, *tert*-butylcarbonylaminomethyl, benzoylaminomethyl, 4-hydroxybenzyloxycarbonylaminomethyl; and $R_2$ is hydrogen; or $R_1$ and $R_2$ together with the atoms to which they are attached are 2-(1,3-dioxo-1H-isoindolyl); $R_3$ is amino or hydroxy; and $R_4$ is hydroxy, amino, or 4-hydroxybenzylamino; or $R_3$ and $R_4$ together with the atoms to which they are attached are a six membered heterocyclic ring comprising five carbon atoms and N(H); $R_5$ is hydrogen; and $R_6$ is hydrogen. Preferably the center marked * has the (S) absolute configuration in a compound of formula (X).

A preferred group of compounds of formula (X) are compounds wherein $R_5$ is $(C_1-C_6)$alkyl.

Another analog of WGQ useful in the methods of the invention is a compound of formula (XI):

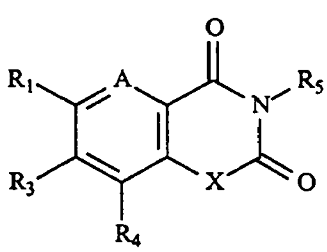

(XI)

wherein: A is $C(R_2)$ or N; $R_1$ is $(C_1-C_{15})$alkyl, $(C_2-C_{15})$alkenyl, $(C_2-C_{15})$alkynyl, $(C_1-C_{15})$alkoxy, $(C_1-C_{15})$alkanoyl, $(C_2-C_{15})$alkenylcarbonyl, $(C_2-C_{15})$alkynylcarbonyl, $(C_1-C_{15})$alkoxycarbonyl, or $N(R_a)(R_b)$; $R_2$, $R_3$, and $R_4$ are each independently hydrogen, halo, cyano, hydroxy, nitro, trifluoromethyl, trifluoromethoxy, $(C_1-C_6)$alkyl, $(C_1-C_6)$alkoxy, $(C_1-C_6)$alkanoyl, $(C_1-C_6)$alkanoyloxy, $(C_1-C_6)$alkoxycarbonyl, sulfino ($SO_2H$), or sulfo ($SO_3H$),; $R_5$ is hydrogen or $(C_1-C_6)$alkyl; X is a direct bond or methylene ($-CH_2-$); each $R_a$ and $R_b$ is independently hydrogen, $(C_1-C_{15})$alkyl, $(C_2-C_{15})$alkenyl, $(C_2-C_{15})$alkynyl, $(C_1-C_{15})$alkanoyl, $(C_1-C_{15})$alkoxycarbonyl, $(C_2-C_{15})$alkenylcarbonyl, $(C_2-C_{15})$alkynylcarbonyl; wherein any $(C_1-C_{15})$alkyl, $(C_2-C_{15})$alkenyl, $(C_2-C_{15})$alkynyl, or $(C_1-C_{15})$alkoxy of $R_1$ is optionally substituted with one or more substituents (e.g. 1, 2, 3, or 4) selected from the group consisting of halo, cyano, nitro, carboxy, trifluoromethyl, trifluoromethoxy, $(C_1-C_6)$alkyl, $(C_1-C_6)$alkoxy, $(C_1-C_6)$alkanoyl, $(C_1-C_6)$alkanoyloxy, $(C_1-C_6)$alkoxycarbonyl, hydroxy, oxo, and $N(R_a)(R_b)$; or a pharmaceutically acceptable salt thereof.

For a compound of formula (XI) $R_1$ can specifically be $(C_6-C_{15})$alkenyl, $(C_6-C_{15})$alkenylcarbonyl, or $N(R_a)(R_b)$, wherein $R_a$ is hydrogen and $R_b$ is $(C_6\ C_{15})$alkenyl, or $(C_6-C_{15})$alkenylcarbonyl; or $R_1$ can specifically be 10-undecenoylamino or 9-decenoyl; $R_2$ can specifically be hydrogen; $R_3$ can specifically be sulfo; $R_4$ can specifically be hydrogen;

and $R_5$ can specifically be hydrogen.

An analog of WAQ useful in the methods of the invention is a compound of formula (XII):

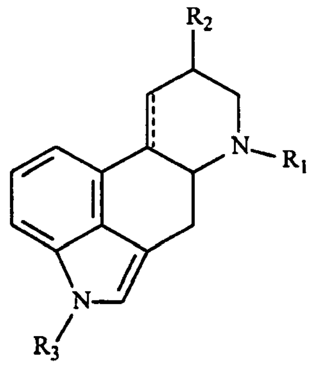

**(XII)**

wherein: $R_1$ is hydrogen or $(C_1-C_6)$alkyl; $R_2$ is hydrogen, $(C_1-C_6)$alkyl, $(C_1-C_6)$alkanoyl, an amino acid, (amino acid)$(C_1-C_6)$alkyl, (amino acid)$(C_1-C_6)$alkanoyl, or $N(R_a)(R_b)$, wherein each $R_a$ and $R_b$ is independently hydrogen, $(C_1-C_6)$alkanoyl, an amino acid, phenyl, benzyl or phenethyl; $R_3$ is hydrogen, $(C_1-C_6)$alkyl, $(C_1-C_6)$alkanoyl, phenyl, benzyl, or phenethyl; and the bond represented by ---- is absent or present; wherein the benz ring of formula (XII) may optionally be substituted with 1, 2, or 3 substituents independently selected from the group consisting of halo, cyano, hydroxy, nitro, trifluoromethyl, trifluoromethoxy, $(C_1-C_6)$alkyl, $(C_1-C_6)$alkoxy, $(C_1-C_6)$alkanoyl, $(C_1-C_6)$alkanoyloxy, $(C_1-C_6)$alkoxycarbonyl, sulfino ($SO_2H$), sulfo ($SO_3H$), and methylenedioxy; or a pharmaceutically acceptable salt thereof.

A specific compound of formula (XII) is a compound of the following formula:

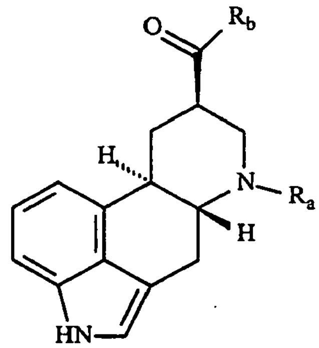

wherein: $R_a$ is hydrogen or $(C_1-C_6)$alkyl; and $R_b$ is hydrogen or an amino acid; or a pharmaceutically acceptable salt thereof.

For a compound of formula (XII) $R_1$ can specifically be methyl or ethyl; and $R_2$ can specifically be glutamine linked through the amine nitrogen to form an amide.

Specific compounds of formula (XII) are compounds wherein the carbon bearing $R_2$ has the R absolute configuration, however, the invention also provides the corresponding compounds of the S absolute configuration.

Specific compounds of formula (XII) are compounds wherein $R_2$ is -C(=O)-(N-glutamine), or $N(R_a)(R_b)$, wherein $R_a$ is hydrogen and $R_b$ is glutamine linked to N through the carboxy terminus to form an amide.

Specific compounds of formula (XII) are compounds wherein $R_3$ is hydrogen.

Other compounds of the invention include compounds of formula (VIII):

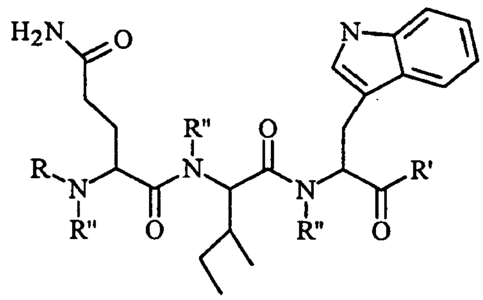

wherein

R is ($C_1$-$C_6$)alkyl, ($C_1$-$C_6$)alkanoyl, aryl, heteroaryl, ($C_1$-$C_6$)alkoxycarbonyl, or benzyloxycarbonyl, wherein aryl, heteroaryl, and the phenyl ring of the benzyloxycarbonyl can optionally be substituted with one or more (e.g. 1, 2, 3, or 4), halo, hydroxy, cyano nitro, trifluoromethyl, trifluoromethoxy, ($C_1$-$C_6$)alkyl, ($C_1$-$C_6$)alkoxy, ($C_1$-$C_6$)alkanoyl, ($C_2$-$C_6$)alkanoyloxy or ($C_1$-$C_6$)alkoxycarbonyl;

R' is ($C_1$-$C_6$)alkoxy, aryloxy, or $NR_aR_b$, wherein $R_a$ and $R_b$ are each independently hydrogen, ($C_1$-$C_6$)alkyl, aryl, benzyl, or phenethyl; or $R_a$, and $R_b$ together with the nitrogen to which they are attached are a 5-6 membered heterocyclic ring (e.g. pyrrolidino, piperidino, or morpholino); and

each R" is independently hydrogen, ($C_1$-$C_6$)alkyl, phenyl, benzyl, or phenethyl;

or a pharmaceutically acceptable salt thereof. Preferably, R is benzyloxycarbonyl and R' is dimethyl amino or diethylamine, or R is benzyloxycarbonyl; and R' is benzyloxy.

Other compounds of the invention include compounds of formula (IX):

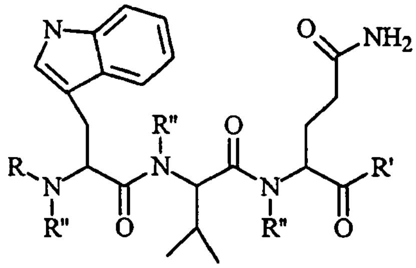

wherein

R is ($C_1$-$C_6$)alkyl, ($C_1$-$C_6$)alkanoyl, aryl, heteroaryl, ($C_1$-$C_6$)alkoxycarbonyl, or benzyloxycarbonyl, wherein aryl, heteroaryl, and the phenyl ring of the benzyloxycarbonyl can optionally be substituted with one or more (e.g. 1, 2, 3, or 4), halo, hydroxy, cyano nitro, trifluoromethyl, trifluoromethoxy, ($C_1$-$C_6$)alkyl, ($C_1$-$C_6$)alkoxy, ($C_1$-$C_6$)alkanoyl, ($C_2$-$C_6$)alkanoyloxy or ($C_1$-$C_6$)alkoxycarbonyl;

R' is ($C_1$-$C_6$)alkoxy, aryloxy, **or** $NR_aR_b$, wherein $R_a$ and $R_b$ are each independently hydrogen, ($C_1$-$C_6$)alkyl, aryl, benzyl, or phenethyl; or $R_a$, and $R_b$ together with the nitrogen to which they are attached are a 5-6 membered heterocyclic ring (e.g. pyrrolidino, piperidino, or morpholino); and

each R" is independently hydrogen, ($C_1$-$C_6$)alkyl, phenyl, benzyl, or phenethyl;

or a pharmaceutically acceptable salt thereof. Preferably, R is benzyloxycarbonyl and R' is dimethyl amino or diethylamine, or R is benzyloxycarbonyl; and R' is benzyloxy.

Another preferred analog of chemokine peptide 3 is an analog of KXK. Thus, the invention includes a compound of formula (V):

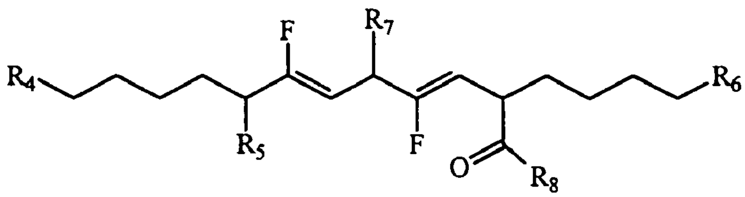

wherein
$R^4$ is $NR_kR_1$ $R^5$ is $NR_mR_n$; $R^6$ is $NR_oR_p$; $R^7$ is the side chain of a natural or unnatural amino acid or is $-(CH_2)_2C(=O)NR_qR_r$; $R^8$ is hydrogen, hydroxy, $(C_1-C_{10})$alkyl, $(C_1-C_6)$cycloalkyl, $(C_3-C_6)$cycloalkyl$(C_1-C_6)$akl, $(C_1-C_{10})$alkoxy, $(C_3-C_6)$cycloalkyl$(C_1-C_6)$alkoxy, $NR_sR_t$, the amino terminus of an amino acid or the N-terminal residue of a peptide of 2 to about 25 amino acid residues; $R_k$, $R_l$, $R_o$, and $R_p$ are each independently hydrogen, $(C_1-C_{10})$alkyl, $(C_3-C_6)$cycloalkyl, $(C_3-C_6)$cycloalkyl$(C_1-C_6)$alkyl, $(C_1-C_{10})$alkanoyl, phenyl, benzyl or phenethyl; $R_m$ are $R_n$ are each independently hydrogen, $(C_1-C_{10})$alkyl, $(C_3-C_6)$cycloalkyl, $(C_3-C_6)$cycloalkyl$(C_1-C_6)$alkyl, $(C_1-C_{10})$alkoxy, $(C_1-C_{10})$alkanoyl, $(C_1-C_{10})$alkoxycarbonyl, 9-fluorenylmethoxycarbonyl, phenyl, benzyl, phenethyl, the C-terminal residue of an amino acid or a peptide of 2 to about 25 amino acid residues; $R_q$ are $R_r$ are each independently hydrogen, $(C_1-C_{10})$alkyl, $(C_3-C_6)$cycloalkyl, $(C_3-C_6)$cycloalkyl$(C_1-C_6)$alkyl, phenyl, benzyl or phenethyl; wherein $R_s$ are $R_t$ are each independently hydrogen, $(C_1-C_{10})$alkyl, $(C_3-C_6)$cycloalkyl, $(C_3-C_6)$cycloalkyl$(C_1-C_6)$alkyl, phenyl, benzyl or phenethyl; or a pharmaceutically acceptable salt thereof.

Preferably $R_k$, $R_l$, $R_o$, and $R_p$ are each hydrogen; $R_m$ are $R_n$ are each independently hydrogen, acetyl, $(C_1-C_{10})$alkyl, $(C_3-C_6)$cycloalkyl, propoxy, butoxy, tert-butoxycarbonyl, 9-fluorenylmethoxycarbonyl or the C-terminal residue of an amino acid or a peptide of 2 to about 25 amino acid residues; and $R_q$ are $R_r$ are each independently hydrogen, $(C_1-C_{10})$alkyl, or $(C_3-C_6)$cycloalkyl.

Preferably, $R^7$ is $-(CH_2)_2C(=O)NR_qR_{r.}$

Preferably, $R^7$ is methyl, 3-guanidinopropyl, aminocarbonylmethyl, carboxymethyl, mercaptomethyl, (2-carboxyl-2-aminoethyl)dithiomethyl, 2-carboxyethyl, 2-(aminocarbonyl)ethyl, hydrogen, 5-imadazoylmethyl, 4-amino-3-hydroxy propyl, 2-butyl, 2-methylprop-1-yl, 4-amino butyl, 2-(methylthio)ethyl, benzyl, hydroxy methyl, 1-hydroxyethyl, 3-indolylmethyl, 4-hydroxybenzyl, or isopropyl.

More preferably, $R^7$ is hydrogen, benzyl, 4-hydroxybenzyl, methyl, 2-hydroxy methyl, or mercaptomethyl.

A preferred compound of formula (V) includes an analog of KGK, KFK, KYK, KAK, KSK, KCK or KQK. For example, an analog of KQK includes a compound of formula (V):

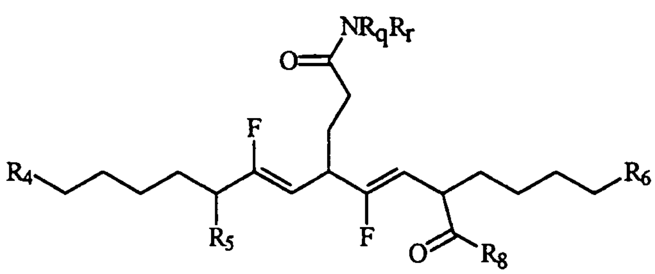

wherein $R^4$ is $NR_kR_1$; $R^5$ is $NR_mR_n$; $R^6$ is $NR_oR_p$; $R^7$ is $NR_qR_r$; $R^8$ is hydrogen, hydroxy, $(C_1-C_{10})$alkyl, $(C_3-C_6)$cycloalkyl, $(C_3-C_6)$cycloalkyl$(C_1-C_6)$alkyl, $(C_1-C_{10})$alkoxy, $(C_3-C_6)$cycloalkyl$(C_1-C_6)$alkoxy, $NR_sR_t$, the amino terminus of an amino acid or the N-terminal residue of a peptide of 2 to about 25 amino acid residues; $R_k$, $R_1$, $R_o$, and $R_p$ are each independently hydrogen, $(C_1-C_{10})$alkyl, $(C_3-C_6)$cycloalkyl, $(C_3-C_6)$cycloalkyl$(C_1-C_6)$alkyl, $(C_1-C_{10})$alkanoyl, phenyl, benzyl or phenethyl; $R_m$ are $R_n$, are each independently hydrogen, $(C_1-C_{10})$alkyl, $(C_3-C_6)$cycloalkyl, $(C_3-C_6)$cycloalkyl$(C_1-C_6)$alkyl, $(C_1-C_{10})$alkoxy, $(C_1-C_{10})$alkanoyl, $(C_1-C_{10})$alkoxycarbonyl, 9-fluorenylmethoxycarbonyl, phenyl, benzyl, phenethyl, the C-terminal residue of an amino acid or a peptide of 2 to about 25 amino acid residues; $R_q$ are $R_r$ are each independently hydrogen, $(C_1-C_{10})$alkyl, $(C_3-C_6)$cycloalkyl, $(C_3-C_6)$cycloalkyl$(C_1-C_6)$alkyl, phenyl, benzyl or phenethyl; $R_s$ are $R_t$ are each independently hydrogen, $(C_1-C_{10})$alkyl, $(C_3-C_6)$cycloalkyl, $(C_3-C_6)$cycloalkyl$(C_1-C_6)$alkyl, phenyl, benzyl or phenethyl; or a pharmaceutically acceptable salt thereof.

Preferably $R_k$, $R_t$, $R_o$, and $R_p$ are each hydrogen; $R_m$ are $R_n$ are each independently hydrogen, acetyl, $(C_1-C_{10})$alkyl, $(C_3-C_6)$cycloalkyl, propoxy, butoxy, tert-butoxycarbonyl, 9-fluorenylmethoxycarbonyl or the C-terminal residue of an amino acid or a peptide of 2 to about 25 amino acid residues; and $R_q$ are $R_r$ are each independently hydrogen,

$(C_1-C_{10})$alkyl, or $(C_3-C_6)$cycloalkyl

Another preferred analog of chemokine peptide 3 is an analog of WVQ. Thus, the invention provides a compound of formula (VI):

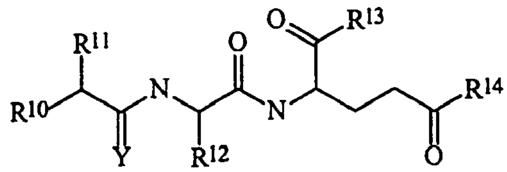

(VI)

wherein

$R^{10}$is $NR^iR^j$; $R^{11}$ is aryl, heteroaryl, aryl$(C_1-C_3)$alkyl, heteroaryl$(C_1-C_3)$alkyl, coumaryl, coumaryl$(C_1-C_3)$alkyl. chromanyl or chromanyl$(C_1-C_3)$alkyl; wherein any aryl or heteroaryl group, or the benz-ring of any coumaryl or chromanyl group may optionally be substituted with one, two or three substituents selected from the group consisting of halo, nitro, cyano, hydroxy, $(C_1-C_6)$alkyl, $(C_1-C_6)$alkoxy, $(C_1-C_6)$alkanoyl, $(C_2-C_6)$alkanoyloxy, $-C(=O)(C_1-C_6)$alkoxy, $C(=O)NR^gR^h$, $NR^eR^f$; $R^{12}$ is $(C_1-C_6)$alkyl; $R^{12}$ is $(C_1-C_{10})$alkyl, $(C_3-C_6)$cycloalkyl, $(C_3-C_6)$cycloalkyl$(C_1-C_6)$alkyl, $(C_1-C_{10})$alkoxy, $(C_3-C_6)$cycloalkyl$(C_1-C_6)$alkoxy, hydroxy, or $N(R^a)(R^b)$; $R^{14}$ is $(C_1-C_{10})$alkyl, $(C_3-C_6)$cycloalkyl, $(C_3-C_6)$cycloalkyl$(C_1-C_6)$alkyl, $(C_1-C_{10)}$alkoxy, $(C_3\ C_6)$cycloalkyl$(C_1-C_6)$alkoxy or $N(R^c)(R^d)$; Y is oxo or thioxo; wherein $R^a$-$R^j$ are each independently hydrogen, $(C_1-C_{10})$alkyl, $(C_1-C_{10})$alkanoyl, phenyl, benzyl, or phenethyl; or $R^a$ and $R^b$, $R^c$ and $R^d$, $R^c$ and $R^f$, $R^g$ and $R^h$ or $R^i$ and $R^j$ together with the nitrogen to which they are attached form a ring selected from pyrrolidino, piperidino, or morpholino; or a pharmaceutically acceptable salt thereof. Preferably, $R^{10}$ is amino; $R^{11}$ is 2-benzimidazolyl; $R^{12}$ is $(C_1-C_6)$alkyl; $R^{13}$ is hydroxy; and $R^{14}$ is amino.

Also provided is a compound of formula (VI):

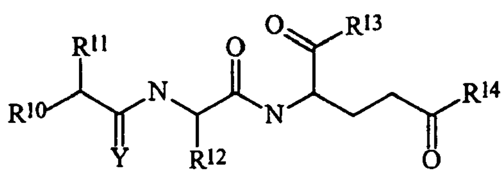

(VI)

wherein

$R^{10}$ is $NR^iR^j$;

R" is aryl, heteroaryl, aryl$(C_1-C_3)$alkyl, heteroaryl$(C_1-C_3)$alkyl, coumaryl, coumaryl$(C_1-C_3)$alkyl, chromanyl or chromanyl $(C_1-C_3)$alkyl; wherein any aryl or heteroaryl group, or the benz-ring of any coumaryl or chromanyl group may optionally be substituted with one, two or three substituents selected from the group consisting of halo, nitro, cyano, hydroxy, $(C_1-C_6)$alkyl, $(C_1-C_6)$alkoxy, $(C_1-C_6)$alkanoyl, $(C_2-C_6)$alkanoyloxy, $-C(=O)(C_1-C_6)$alkoxy, $C(=O)NR^gR^h$, $NR^eR^f$;

$R^{12}$ is $(C_1-C_6)$alkyl;

$R^{13}$ is $(C_1-C_{10})$alkyl, $(C_3-C_6)$cycloalkyl, $(C_3-C_6)$cycloalkyl$(C_1-C_6)$alkyl, $(C_1-C_{10})$alkoxy, $(C_3-C_6)$cycloalkyl$(C_1-C_6)$alkoxy, hydroxy, or $N(R^a)(R^b)$;

$R^{14}$ is $(C_1-C_{10})$alkyl, $(C_3-C_6)$cycloalkyl, $(C_3-C_6)$cycloalkyl$(C_1-C_6)$alkyl, $(C_1-C_{10})$alkoxy, $(C_3-C_6)$cycloalkyl$(C_1-C_6)$alkoxy or $N(R^c)(R^d)$;

Y is oxo or thioxo;

wherein $R^a$-$R^j$ are each independently hydrogen, $(C_1-C_{10})$alkyl, $(C_1-C_{10})$alkanoyl, phenyl, benzyl, or phenethyl; or $R^a$ and $R^b$, $R^c$ and $R^d$, $R^e$ and $R^f$, $R^g$ and $R^h$, or $R^i$ and $R^j$ together with the nitrogen to which they are attached form a ring selected from pyrrolidino, piperidino, or morpholino; or a pharmaceutically acceptable salt thereof.

Yet another analog of chemokine peptide 3 is an analog of WGQ. Thus, the invention provides a compound of formula (XV):

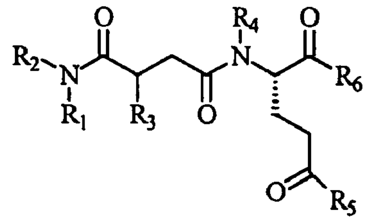

wherein $R_1$ is aryl, heteroaryl, aryl($C_1$-$C_{10}$)alkyl, aryl($C_1$-$C_{10}$)alkanoyl, heteroaryl($C_1$-$C_{10}$)alkyl, or heteroaryl($C_1$-$C_{10}$)alkanoyl; $R_2$ is hydrogen, ($C_1$-$C_{15}$)alkyl, ($C_3$-$C_8$)cycloalkyl, ($C_3$-$C_8$)cycloalkyl($C_1$-$C_{10}$)alkyl, aryl, or aryl($C_1$-$C_{10}$)alkyl; $R_3$ is hydrogen, or ($C_1$-$C_{10}$)alkyl, $R_4$ is hydrogen, or ($C_1$-$C_{10}$)alkyl; $R_5$ is N($R^a$)($R^b$); $R_6$ is N($R^a$)($R^b$); and each $R^a$ and $R^b$ is independently hydrogen, ($C_1$-$C_{10}$)alkyl, ($C_1$-$C_{10}$)alkanoyl, or aryl($C_1$-$C_{10}$)alkyl; or $R^a$ and $R^b$ together with the nitrogen to which they are attached form a pyrrolidino, piperidino or morpholino ring; wherein any aryl or heteroaryl is optionally substituted with 1, 2, 3, or 4 substituents independently selected from the group consisting of halo, cyano, hydroxy, nitro, trifluoromethyl, trifluoromethoxy, ($C_1$-$C_6$)alkyl, ($C_1$-$C_6$)alkoxy, ($C_1$-$C_6$)alkanoyl, ($C_1$-$C_6$)alkanoyloxy, ($C_1$-$C_6$)alkoxycarbonyl, and methylenedioxy; or a pharmaceutically acceptable salt thereof.

For a compound of formula (XV), $R_1$ can specifically be 3-indolylmethyl; $R_2$ can specifically be isopropyl, *tert*-butyl, or phenyl; $R_3$ can specifically be methyl; $R_4$ can specifically be hydrogen; $R_5$ can specifically be amino; and $R_6$ can specifically be dimethylamino, benzylamino, or hydroxybenzylamino.

Yet another analog of WGQ useful in the methods of the invention is a compound of formula (XIX):

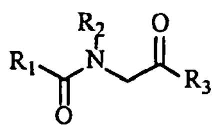

(XIX)

wherein: $R_1$ is ($C_7$-$C_{15}$)alkyl, ($C_7$-$C_{15}$)alkenyl, ($C_7$-$C_{15}$)alkynyl ($C_7$-$C_{15}$)alkoxy, aryl($C_5$-$C_{10}$)alkyl, or heteroaryl($C_5$-$C_{10}$)alkyl; $R_2$ is hydrogen or ($C_1$-$C_{15}$)alkyl; and $R_3$ is hydroxy, ($C_1$-$C_6$)alkoxy, or N($R_a$)($R_b$) , wherein each $R_a$ and $R_b$ is independently hydrogen, ($C_1$-$C_6$)alkyl, ($C_1$-$C_6$)alkanoyl, ($C_1$-$C_6$)alkoxycarbonyl, aryl, aryl($C_1$-$C_6$)alkyl, aryl($C_1$-$C_6$)alkoxy, aryl($C_1$-$C_6$)alkanoyl, aryl($C_1$-$C_6$)alkoxycarbonyl, heteroaryl, heteroaryl($C_1$-$C_6$)alkyl, heteroaryl($C_1$-$C_6$)alkoxy, heteroaryl ($C_1$-$C_6$)alkanoyl, or heteroaryl($C_1$-$C_6$)alkoxycarbonyl; wherein any ($C_7$-$C_{15}$)alkyl, ($C_7$-$C_{15}$)alkenyl, ($C_7$-$C_{15}$)alkynyl, or ($C_7$-$C_{15}$)alkoxy of $R_1$ is optionally substituted with one or more substituents selected from the group consisting of halo, cyano, nitro, carboxy, trifluoromethyl, trifluoromethoxy, ($C_1$-$C_6$)alkyl, ($C_1$-$C_6$)alkoxy, ($C_1$-$C_6$)alkanoyl, ($C_1$-$C_6$)alkanoyloxy, ($C_1$-$C_6$)alkoxycarbonyl, hydroxy, oxo, and N($R^a$($R^b$); and wherein any aryl or heteroaryl of $R_1$, $R^a$, and $R_b$, is optionally substituted with 1, 2, 3, or 4 substituents independently selected from the group consisting of halo, cyano, hydroxy, nitro, trifluoromethyl, trifluoromethoxy, ($C_1$-$C_6$)alkyl, ($C_1$-$C_6$)alkoxy, ($C_1$-$C_6$)alkanoyl, ($C_1$-$C_6$)alkanoyloxy, ($C_1$-$C_6$)alkoxycarbonyl, sulfino ($SO_2H$), sulfo ($SO_3H$), and methylenedioxy; or a pharmaceutically acceptable salt thereof.

A preferred group of compounds of formula (XIX) are compounds wherein $R_1$ is 9-decenyl, $R_2$ is hydrogen, and $R_3$ is hydroxy, amino, or methoxy. A more preferred compound of formula (XIX) is a compound wherein $R_1$ is 9-decenyl, $R_2$ is hydrogen, and $R_3$ is amino; or a pharmaceutically acceptable salt thereof.

It is envisioned that the therapeutic agents of the invention include compounds having a chiral center that can be isolated in optically active and racemic forms.

Also provided are pharmaceutical compositions, delivery systems, and kits comprising the therapeutic agents of the invention.

The invention further provides methods to treat chemokine-associated indications. For example, the invention provides a method of preventing or inhibiting an indication associated with chemokine-induced activity. The method comprises administering to a mammal afflicted with, or at risk of, the indication an amount of a chemokine peptide 3, a fragment thereof, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof, effective to prevent or inhibit said activity. Preferably, the peptide is not an IL-8 peptide, a NAP-2 peptide, or a PF4 peptide. Preferably, the administration is effective to inhibit the activity of more than one chemokine (i.e., the peptide is a pan-selective inhibitor). Preferred pan-chemokine inhibitors are analogs of WVQ, e.g., YII (see page 54), analogs of WIQ, analogs of WGQ, e.g., AII (see

page 56), $Leu_4Ile_{11}$peptide 3(3-12)[MCP-1], $Leu_4Ile_{11}$peptide 3(1-12)[MCP-1] and CRD-$Cys_{13}Leu_4Ile_{11}$peptide 3(3-12). These agents are useful to treat indications such as multiple sclerosis, asthma, psoriasis, allergy, rheumatoid arthritis, organ transplant rejection, and autoimmune disorders. Preferred chemokine peptides useful to treat or inhibit these indications include peptide 2 and/or peptide 3 from MCP-1, MCP-2, MCP-3, MCP-4, RANTES, MIP1α, ENA78, MIG, GROα, GROβ, GROγ, GCP-1, HCC-1, I-309, SCM-1, eotaxin, IP10, MIPβ and SDF-1.

Moreover, as peptide 3, its variants and derivatives may decrease Th2 responses and increase Th1 responses, these compounds may be particularly useful to treat or prevent specific diseases in which a decrease in Th2 response and an increase in Th1 response is indicated.

The invention also provides a method of preventing or inhibiting an indication associated with histamine release from basophils or mast cells. The method comprises administering to a mammal at risk of, or afflicted with, the indication an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof.

Also provided is a method of preventing or inhibiting an indication associated with monocyte, macrophage, neutrophil, B cell, T cell or eosinophil recruitment, or B cell or T cell activation or proliferation. The method comprises administering an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof. For example, a chemokine peptide 3, a variant thereof, or a derivative thereof may be useful to prevent or treat autoimmune or granulomatous indications.

Further provided is a therapeutic method to prevent or treat vascular indications, comprising: administering to a mammal in need of such therapy an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof, wherein the indication is coronary artery disease, myocardial infarction, unstable angina pectoris, atherosclerosis or vasculitis, e.g., Behçet's syndrome, giant cell arteritis, polymyalgia rheumatica, Wegener's granulomatosis, Churg-Strauss syndrome vasculitis, Henoch-Schönlein purpura and Kawasaki disease. Preferred chemokine peptides for this embodiment of the invention include chemokine peptides of MCP-1, RANTES, GROα, GROβ, GROγ, GCP-1, HCC-1,1-309, SCM-1, MIP1α;, IP10, MCP-4, and MIP1β.

The invention also provides a method to prevent or treat an autoimmune disorder. The method comprises administering to a mammal in need of said therapy an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof. A preferred variant of peptide 3 useful to prevent or treat autoimmune disorders is $Leu_4Ile_{11}$peptide 3(1-12)[MCP-1] (SEQ ID NO: 14) or peptide 3 having WVQ. A preferred chemokine peptide 3 for use in preventing or treating multiple sclerosis includes SEE and peptide 3(1-14)[MIP1α] (SEQ ID NO:42). Other preferred peptides are chemokine peptides of RANTES.

Further provided is a method to modulate the chemokine-induced activity of macrophage, B cells, T cells or other hematopoietic cells, e.g., neutrophils, eosinophils or mast cells, at a preselected physiological site. The method comprises administering a dosage form comprising an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof, wherein the dosage form is linked, either covalently or noncovalently, to a targeting moiety. The targeting moiety binds to a cellular component at the preselected physiological site, e.g., to an antigen that is specific for tumor cells.

Moreover, it is also envisioned that an agent of the invention may be a targeting moiety, as some of the agents are selective chemokine inhibitors, rather than pan-chemokine inhibitors. For example, an agent of the invention, e.g., peptide 3, may be useful in the targeted delivery of an isotope or other cytotoxic molecule to certain cells. Similarly, an agent of the invention that specifically targets a particular cell type may be useful in diagnostics. Thus, these agents can be radiolabeled (Chianelli et al., Nucl, Med Comm, 18, 437 (1997)), or labeled with any other detectable signal, such as those useful in diagnostic imaging (e.g., MRI and CAT) to image sites of inflammation in disorders like rheumatoid arthritis and diabetes mellitus (type I).

The invention also provides a therapeutic method to prevent or inhibit asthma. The method comprises administering to a mammal in need of such therapy an effective amount of an agent that inhibits airway reactivity and a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof. As described hereinbelow in Example 12, a peptide of the invention inhibited cellular inflammation and IgE responses in the lung of mice exposed to ovalbumin. Preferably, in this embodiment of the invention, a therapeutic agent is administered to the upper and/or lower respiratory tract. Preferred peptides useful in this embodiment of the invention are chemokine peptides of RANTES, MCP-1 and MIP1α.

Further provided is a therapeutic method to prevent or inhibit viral, e.g., poxvirus, herpesvirus (e.g., *Herpesvirus samiri*), cytomegalovirus (CMV) or lentivirus, infection or replication. The method comprises administering to a mammal in need of such therapy an effective amount of a chemokine peptide 3, a variant thereof, a derivative thereof, a compound of formula (IV), or a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof. Preferably, the therapeutic agents are employed to prevent or treat HIV. More preferably, the agent is administered before, during or after the administration of an anti-viral agent, e.g., for HIV AZT, a non-nucleoside reverse transcriptase inhibitor, a protease inhibitor or a combination thereof. It is also envisioned that a combination of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), or a compound of formula (XIX) may be useful in the anti-viral methods and compositions of the invention. Preferred chemokine peptides useful to prevent or inhibit viral infection are those from IP10, MIP1α, MIP1β, SDF-1, IL-8, GROα, GROβ, GROγ, GCP-1, HCC-1, I-309, SCM-1, RANTES, and MCP-1.

A therapeutic method to prevent or treat low bone mineral density is also provided. The method comprises administering to a mammal in need of such therapy an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof. A preferred derivative of a variant of peptide 3 to prevent or treat low mineral bone density is CRD-$Cys_{13}Leu_4Ike_{11P}$eptide 3(3-12)[MCP-1]. A preferred fragment of SEQ ID NO:1 useful in preventing or treating low mineral bone density is KQK.

Also provided is a method of suppressing tumor growth in a vertebrate animal comprising administering to said vertebrate a therapeutically effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof. Preferably, the method increases or enhances macrophage, B cell-, T cell- or other immune cell-associated activity at a tumor site. A preferred peptide for use in this embodiment of the invention is a MCP-1 peptide.

Further provided is a method for preventing or treating rheumatoid arthritis in a mammal, comprising: administering to the mammal an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof. For this embodiment of the invention, a preferred peptide is a MCP-1, MIP1α, MIP1β, GROα, and ENA78 peptide.

Also provided is a method to prevent or treat organ transplant rejection, and/or delayed organ or graft function, e.g., in renal transplant patients. The method comprises administering an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof.

Further provided is a method for preventing or treating psoriasis in a mammal, comprising: administering to the mammal an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof. Preferred peptides to prevent or treat psoriasis are peptides of MCP-1, RANTES,, MIP1α MIG, IP10, GROUP, GROα, GROγ, GCP-1, HCC-1, I-309, SCM-1, or MCP-3. A preferred derivative to prevent or treat psoriasis is a CRD-derivative

of peptide 3.

Also provided is a method to enhance wound healing. The method comprises administering to a mammal an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof.

The invention also provides a method to modulate a chemokine-induced activity, e.g., to treat malaria, tuberculosis or other disorders caused by intracellular parasites. The method comprises administering to a mammal an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof.

Further provided is a method for preventing or treating an allergy in a mammal, comprising: administering to the mammal an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX), a compound of formula (X), a compound of formula (XI), a compound of formula (XII), a compound of formula (XIV), a compound of formula (XV), a compound of formula (XIX), or a combination thereof. Preferred peptides to prevent or treat allergies include peptides of RANTES, MIP1$\alpha$, MCP-1, MCP-2, MCP-3, MCP-4, eotaxin or MIP1$\beta$.

Yet another embodiment of the invention is a method to prevent or inhibit an indication associated with elevated TNF-$\alpha$. The method comprises administering an effective amount of a chemokine peptide 3, a variant thereof, a derivative thereof, a compound of formula (IV), a compound of formula (V), a compound of formula (VI), a compound of formula (VII), a compound of formula (VIII), a compound of formula (IX) a compound of formula (X), a compound of formula (XI), a compound of formula (XII), a compound of formula (XIV), a compound of formula (XV), a compound of formula (XIX), or a combination thereof.

The invention also provides methods in which the nucleic acid molecules of the invention are administered to a mammal afflicted with, or at risk of, an indication associated with a chemokine-induced activity.

The invention also provides a method to identify a region of a chemokine receptor which binds to a chemokine peptide, a variant, derivative or analog thereof. The method comprises contacting a chemokine receptor with an amount of the chemokine peptide, a variant, derivative or analog thereof so as to result in a complex between the receptor and the chemokine peptide, a variant, derivative or analog thereof. Then it is determined which region of the receptor is bound to the chemokine peptide, variant, derivative or analog thereof.

The invention further provides a method to identify a molecule which binds to a region of a chemokine receptor that is bound by a specific chemokine peptide, a variant, derivative or analog thereof. The method contacting the region with a population of molecules, and detecting or determining whether at least one molecule of the population of molecules specifically binds to the region.

Yet another embodiment of the invention is a method to identify a molecule that binds to a chemokine receptor but which molecule does not form a heterodimer with at least one chemokine that binds to the receptor. The method comprises contacting the chemokine receptor with the molecule so as to form a complex between the receptor and the molecule. The complex is contacted with at least one chemokine. Then it is determined whether the molecule in the complex forms a heterodimer with the chemokine.

A further embodiment of the invention also is a method to identify a molecule that binds to a chemokine receptor but which molecule does not form a heterodimer with a chemokine that binds to the receptor. The method comprises contacting the chemokine receptor with the molecule and at least one chemokine, and detecting or determining whether the molecule forms a heterodimer with the chemokine.

The invention also provides a method to identify an agent that inhibits antigen-induced recall response. The method comprises administering to a mammal which is sensitized to an antigen an agent selected from 1) a compound of formula (XIV), (X), (XI), or (XII); or 2) a saccharide conjugate of a chemokine peptide 3, a variant thereof, a derivative thereof, a compound of formula (IV), a compound of formula (V), a compound of formula (VI), a compound of formula (X), a compound of formula (XI), a compound of formula (XII), a compound of formula (XIV), or a compound of formula (XIX); or a pharmaceutically acceptable salt thereof; or a combination thereof. It is then determined whether the agent inhibits the recall response.

The invention further provides a method of preventing or inhibiting a recall response to an antigen, comprising: administering to a mammal which is sensitized to the antigen an amount of an agent effective to inhibit or decrease IL-4 levels in the mammal or to inhibit or decrease immunoglobulin levels in the mammal; wherein the agent is selected from: 1) a compound of formula (XIV), (X), (XI), (XIX), or (XII); and 2) a saccharide conjugate of a chemokine peptide 3, a variant thereof, a derivative thereof, a compound of formula (IV), a compound of formula (V), a compound of formula (VI), a compound of formula (X), a compound of formula (XI), a compound of formula (XII), a compound of formula (XIV),

or a compound of formula (XIX); or a pharmaceutically acceptable salt thereof; or a combination thereof.

Yet a further embodiment of the invention is a method of suppressing the immune response of a mammal subjected to a therapy which employs an immunogenic therapeutic molecule, comprising: administering to the mammal an amount of an agent effective to inhibit antigen-induced recall response to the immunogenic therapeutic molecule; wherein the agent is selected from: 1) a compound of formula (XIV), (X), (XI), (XIX), and (XII); and 2) a saccharide conjugate of a chemokine peptide 3, a variant thereof, a derivative thereof, a compound of formula (IV), a compound of formula (V), a compound of formula (VI), a compound of formula (X), a compound of formula (XI), a compound of formula (XII), a compound of formula (XIV), or a compound of formula (XIX); or a pharmaceutically acceptable salt thereof; or a combination thereof.

The invention also provides a method to identify an agent which inhibits chemokine activity but does not compete with native chemokine for its receptor. The method comprises contacting cells with the agent, wherein the cells comprise receptors that bind native chemokine; and detecting or determining whether the agent specifically binds to a receptor which is not the receptor which binds native chemokine and inhibits chemokine activity.

The invention also provides a method to prevent or inhibit stroke, comprising: administering to a mammal an effective amount of: 1) a compound of formula (XIV), (X), (XI), (XIX), or (XII); or 2) a saccharide conjugate of a chemokine peptide 3, a variant thereof, a derivative thereof, a compound of formula (IV), a compound of formula (V), a compound of formula (VI), a compound of formula (X), a compound of formula (XI), a compound of formula (XII), a compound of formula (XIV), or a compound of formula (XIX); or a pharmaceutically acceptable salt thereof; or a combination thereof.

The invention further provides a method to increased *in vivo* half-life of compound selected from chemokine peptide 3, a variant thereof, a derivative thereof, a compound of formula (IV), a compound of formula (V), a compound of formula (VI), a compound of formula (XIV), a compound of formula (X), a compound of formula (XI), a compound of formula (XIX), and a compound of formula (XII), comprising linking a saccharide to the compound. The invention also provides a saccharide linked compound prepared by the above method.

## Brief Description of the Figure

Figure 1 is a schematic of the trans-well migration assay. In most experiments, the peptide (wavy line) is added to the upper well with about 50,000 cells (O). The upper and lower wells are separated by a 5 μm or 8 μm pore size PVP-free membrane (- - - -). Chemokine (•) is added to the lower well. After 4 hours, the number of cells that have migrated through the membrane are measured (O in lower well).

Figure 2 shows a dose-response curve for the peptide 3 (SEQ ID NO:1) inhibition of MCP-1-induced THP-1 cell migration.

Figure 3 shows the structure of CRD-$Cys_{13}Leu_4Ile_{11P}$eptide 3(3-12)[MCP-1], which is cyclized via disulphide bonds. The main chain $\alpha$ carbons are indicated by $C_D$ which indicates that the D form of the amino acid is present.

Figure 4 depicts a schematic of inhibition of cell migration by a therapeutic agent of the invention, e.g., by inhibiting the functional activity induced by the binding of native chemokine to its chemokine receptor. $C^RC$ = a therapeutic agent of the invention. Chemokine receptors are shown as blackened rectangles.

Figure 5 shows the dose-dependent inhibition of inflammation (A) and endotoxemia (B) in animal models by peptide 3 (CRD-$Cys_{13}Leu_4Ile_{11}$ peptide 3(3-12)[MCP-1] =NR58-3.14.3).

Figure 6 shows an analog of peptide WVQ.

Figure 7A shows a graph of the percent fractional area staining for macrophage at the site of MCP-1 administration in a rat in the presence or absence of a peptide of the invention.

Figure 7B shows a graph of the percent fractional area staining for B cells at the site of LPS and MCP-1 administration in a rat in the presence or absence of a peptide of the invention.

Figure 8 depicts codons for various amino acids.

Figure 9 depicts exemplary amino acid substitutions.

Figure 10 shows exemplary therapeutic agents of the invention.

Figure 11 summarizes binding and $ED_{50}$ data for selected peptides of the invention.

Figure 12 shows an exemplary protocol to test agents in a rat dermal inflammation model (CRD-$Cys_{13}Leu_4Ile_{11}$ peptide 3(3-12)[MCP-1] = NR58-3.14.3).

Figure 13 depicts the structure and $ED_{50}$ of CRD derivatives of peptide 3, CRD-$Cys_{13}Leu_4Ile_{11}$peptide 3(3-12)[MCP-1] and the D-ala derivative thereof ("inactive").

Figure 14 shows the inhibition of monocyte infiltration induced by MCP-1 in rat skin by CRD-$Cys_{13}Leu_4Ile_{11}$peptide 3(3-12)[MCP-1] in contrast to the D-ala derivative thereof ("inactive").

Figure 15 depicts the inhibition of neutrophils, monocytes and lymphocytes, and the reduction in TNF-$\alpha$ levels, in the skin of rats exposed to LPS in the presence of CRD-$Cys_{13}Leu_4Ile_{11}$peptide 3(3-12)[MCP-1]. Inactive = the D-ala derivative of CRD-$Cys_{13}Leu_4Ile_{11}$peptide 3(3-12)[MCP-1].

Figure 16 shows the results from ovalbumin-sensitized mice treated with an agent of the invention. (A) IgE, IL-4, total cells, and macrophages in ovalbumin-sensitized mice treated with two different amounts of CRD-$Leu_4Ile_{11}Cys_{11}$peptide 3(3-12)[MCP-1]. (B) Results from FACS analysis of ovalbumin-sensitized mice treated with CRD-$Leu_4Ile_{11}$ $Cys_{13}$peptide 3(3-12)[MCP-1]. There were significantly lower numbers of macrophages in the lungs of mice treated with CRD-$Leu_4Ile_{11}$ $Cys_{13}$peptide 3(3-12)[MCP-1] as compared to mice that received only PBS prior to ovalbumin challenge. (C) Serum IgE and IL-4 levels from ovalbumin-sensitized mice treated with CRD-$Leu_4Ile_{11}Cys_{13}$peptide 3(3-12)[MCP-1]. Serum IgE and IL-4 levels were significantly lower in mice treated with CRD-$Leu_4Ile_{11}$ $Cys_{13}$peptide 3(3-12)[MCP-1] than in mice that received only PBS prior to ovalbumin challenge.

Figure 17 is a graph of results obtained in the THP-1 migration assay with 100 $\mu$M peptide. 3.14.3 is CRD-$Cys_{13}Leu_4Ile_{11}$peptide 3(3-12)[MCP-1], 3.14.5 is L-$Leu_4$CRD-$Cys_{13}Ile_{11}$peptide 3(3-12)[MCP-1], 3.14.6 is CRD-$Tyr_3Leu_4Ile_{11}\beta$-$Ala_1\beta Ala_2$peptide 3(3-12)[MCP-1] (N-terminal extension), 3.14.7 is CRD-$Leu_4Asn_5Ile_{11}$ $\beta$-$Ala_3\beta Ala_4Tyr_{15}$peptide 3(3-12)[MCP-1] (C-terminal extension), 3.3.0 is peptide 3(7-12)[MCP-1], 3.19.2 is LRD-peptide 3(7-12)[MCP-1], 3.20.1 is CFL-peptide 3(7-12)[MCP-1], and 3.20.3 is CRD-peptide 3(7-12)[MCP-1].

Figure 18 depicts exemplary agents of the invention.

Figure 19 shows the $ED_{50}$ for the inhibition of THP-1 migration in a transwell assay induced by either 50 ng/ml MCP-1,100 ng/ml IL-8 or 100 nM fMLP by agents of the invention. Peptides 1 and 2 had no significant effect on THP-1 migration induced by either CC or CXC chemokines even at 100 $\mu$M. The $ED_{50}$ for peptide 3 and its variants shown are the mean $\pm$ SEM of three separate experiments. All the variants inhibited migration in response to both CC and CXC chemokines by more than 90% at 100 $\mu$M demonstrating that peptide 3 and its derivatives are pan-chemokine inhibitors. None of the peptides showed statistically significant inhibition of migration in response to non-chemokine chemoattractants; fMet-Leu-Phe (fMLP) or TGF-$\beta$1 at 100 $\mu$M (the highest concentration tested). Furthermore, the peptides exhibit similar properties to other CC and CXC chemokines (MEP-1$\alpha$ and SDF-1$\alpha$).

Figure 20 shows the inhibition of MCP-1 induced migration by peptide 3 and biotinylated peptide 3.

Figure 21 shows a graph of the inhibition of binding of biotinylated mouse IgG by increasing amounts of labeled peptide 2 and 3.

Figure 22A depicts the equilibrium binding of RANTES to various cells.

Figure 22B shows a comparison between the binding of labeled RANTES to various cells in the presence or absence of unlabeled RANTES.

Figure 23 depicts FITC-anti-CCR5 antibody staining of CCR5 expressing cells.

Figure 24 depicts FITC-anti-CXCR1 antibody and FITC-anti-CXCR2 antibody staining of CXCR1 and CXCR2 expressing cells, respectively.

Figure 25 shows peptide 2 and 3 binding to parent and recombinant chemokine receptor expressing cell lines.

Figure 26A shows a bar graph of the binding of biotinylated peptide 3 to THP-1 cells using $^{125}$I-streptavidin.

Figure 26B shows a dose-response curve for binding ofbiotinylated peptide 3 to THP-1 cells using $^{35}$S-streptavidin.

Figure 27 shows total cell number and cell types in the lung of unchallenged and ovalbumin-challenged mice administered CRD-$Leu_4Ile_{11}Cys_{13}$ peptide 3(3-12)[MCP-1]. A) total cells, B) macrophage, C) B cells, and D) CD4 T cells.

Figure 28 shows IgE levels in unchallenged and ovalbumin-challenged mice administered CRD-$Leu_4Ile_{11}Cys_{13}$ peptide 33(3-12)[MCP-1].

Figure 29 shows IgE and total IgM and IgG in spleen recall responses in unchallenged and ovalbumin-challenged mice administered CRD-$Leu_4Ile_{11}Cys_{13}$ peptide 3(3-12)[MCP-1]. A) IgE, and B) total IgG and IgM.

Figure 30 depicts IL-4 levels in spleen recall responses from unchallenged and ovalbumin-challenged mice administered CRD-$Leu_4Ile_{11}Cys_{13}$ peptide 3(3-12)[MCP-1]. A) IL-4 levels after single dose. B) IL-4 levels after OVA challenge and boost.

Figure 31 shows total cells in the lung of unchallenged and ovalbumin-challenged mice.

Figure 32 depicts IgE in spleen recall responses of unchallenged and ovalbumin-challenged mice.

Figure 33 depicts antibody-specific recall responses in monkeys treated with CRD-$Leu_4Ile_{11}Cys_{13}$ peptide 3(3-12) [MCP-1]. Animals #137 and #144 were diluent (PBS) controls. Animals #138, #141 and #143 were treated with CRD-$Leu_4Ile_{11}Cys_{13}$ peptide 3(3-12)[MCP-1].

Figure 34 depicts lesion volume by slice (rostral to caudal) in an ischemic reperfusion injury model in rats 24 hours post ictus. N=7. Results are mean $\pm$ SD:

Figure 35 shows intracellular calcium influx in THP-1 cells loaded with Fura2. CRD-$Leu_4Ile_{11}Cys_{13}$ peptide 3(3-12) [MCP-1] at 6.7 $\mu$g/ml was incubated with THP-1 cells (2 x $10^6$ cells/ml) for 25 minutes prior to exposure to agonist (10 ng/ml) and measurement of flux. The second exposure to agonist is 2 minutes later. Fluorescence is measured at 510 nm.

Figure 36 depicts the inhibition of T cell-dependent antibody response in mice by CRD-$Leu_4Ile_{11}Cys_{13}$peptide 3 (3-12)[MCP-1].

Figure 37 depicts results from male and female, i.v. cannulated, Sprague Dawley rats which were given a single intravenous dose (100 μg) of tritiated $^3$H-CRD-L-$Leu_4Ile_{11}Cys_{13}$peptide 3(3-12)[MCP-1] via the lateral tail vein at time = 0. Blood and urine were collected over time to establish pharmacokinetic parameters. $^3$H-CRD-L-$Leu_4Ile_{11}Cys_{13}$peptide 3(3-12)[MCP-1] levels were measured in whole blood, serum, cell pellets and urine. A: ng/ml $^3$H-CRD-L-$Leu_4Ile_{11}Cys_{13}$ peptide 3(3-12)[MCP-1] in serum during the first 8 hours. B & C: ng $^3$H-CRD-L-$Leu_4Ile_{11}Cys_{13}$ peptide 3(3-12)[MCP-1]/gram of sample in serum, cell pellets, and serum + cells pellets for 48 hours after dosing males=B, females = C). D) representative urinary excretion of $^3$H-CRD-L-$Leu_4Ile_{11}Cys_{13}$ peptide 3 (3-12)[MCP-1] in rats after a single i.v bolus.

Figure 38 shows whole blood clearance over time after a single subcutaneous bolus dose of either 10.3, 103 or 1030 μg $^3$H-CRD-L-$Leu_4Ile_{11}Cys_{13}$ peptide 3(3-12)[MCP-1]. Values are represented as % of the injected dose per gram of blood.

Figure 39 shows the biodistribution of $^3$H-CRD-L-$Leu_4Ile_{11}Cys_{13}$ peptide 3(3-12)[MCP-1] in mice after a single 100.3 μg subcutaneous bolus dose. Values are represented as μg $^3$H-CRD-L-$Leu_4Ile_{11}Cys_{13}$ peptide 3(3-12)[MCP-1] per gram of tissue. A) Tissues from left to right are: PL= blood pellet, SE =serum, Inj= injection site, SK = skin, HE= heart, TH =thymus, LU=lung, LV=liver, SP=spleen,

ST=stomach, KD=kidney, AD=adrenal gland, DU=duodenum, JE=jejunum, IL= ileum, CE=cecum + colon, ND=mesenteric lymph nodes, FT= abdominal fat, MA=bone marrow, MU=skeletal muscle, BR=brain, OV=ovaries, UT=uterus. B) Data for biodistribution of 10.3 μg, 103 μg, and 1030 μg of agent at 30 minutes, 3 hours and 24 hours.

Figures 40A-C show the total cells, macrophages and B cells in the lungs of ovalbumin-treated mice.

Figures 40 D-E depicts the IL-4 levels in spleen recall responses, serum IgE levels, and levels of thromboxane, LTB4 and $PGE_2$ in BAL from ovalbumin-treated mice.

Figure 41 shows serum levels of CRD-$Leu_4Ile_{11}Cys_{13}$ peptide 3(3-12)[MCP-1] over time in monkeys administered the agent.

Figure 42 depicts a competitive assay using $^{125}$I-MCP-1 in the presence of MCP-1 or CRD-$Leu_4Ile_{11}Cys_{13}$peptide 3(3-12)[MCP-1].

Figure 43 shows the effect of WGQ on THP-1 migration.

Figure 44 depicts the effect of WGQ analogs at 100 μM on THP-1 migration induced by MCP-1. Values are represented as % inhibition of THP-1 migration (as a % of control) ± SEM and are the mean of two separate experiments. The shaded boxes indicate compounds that inhibited THP-1 migration greater than 50%.

Figure 45 shows $ED_{50}$ of WGQ analogs on THP-1 migration induced by MCP-1.

Figure 46 shows serum TNF-α levels in mice treated with various agents or PBS and then exposed to LPS. All agents were administered subcutaneously 45 minutes prior to LPS (1 mg/mouse) administration. THD = thalidomide. Data is pooled from three replicate experiments. NR58,4 was administered as the potassium salt.

Figure 47 shows neutrophil data from a rat model for stroke (see Figure 34).

Figure 48 depicts levels of CRD-$Leu_4Ile_{11}Cys_{13}$ peptide 3(3-12)[MCP-1]-glucoside in serum.

Figure 49 depicts effect of CRD-$Leu_4Ile_{11}Cys_{13}$ peptide 3(3-12)[MCP-1] on MIP-1α-induced chemotaxis.

Figure 50 shows the inhibitory effect of A-I in the presence of A) MCP-1, B) MCP-1, C) RANTES, and D) MIP-1α.

Figure 51 depicts the competitive binding of A-I to chemokine receptors in the presence of chemokine A) MCP-1 and CCR-2, and B) IL-8 and CXCR-2.

Figure 52 shows the reverse transcriptase activity present in the culture medium at day 21 after infection of Jurkat cells with a T-tropic HIV. Peptides were added on day 0, one hour prior to infection of the cells with HIV isolate. The full length chemokine SDF-1α was used as a positive control.

Figure 53 shows a graph of the fraction of HIV infected THP-1 cells in the presence of peptide 2 or peptide 3 using a quantitative immunofluorescent (QIF) assay.

Figure 54 shows chemokine peptide inhibition of HIV infectivity *in vitro.* (a) HIV (IIIb) replication in cultures of Jurkat T-cells was estimated by measuring the supernatant reverse transcriptase activity two weeks after infection. Peptide 2 and peptide 3 were at 100 μM final concentration and SDF-1α was added at 100 ng/ml final concentration 1 hour prior to exposure to virus. Values are mean ± SEM from 12 wells, expressed as the percentage of the reverse transcriptase activity in the supernatant from the control wells. The experiment shown is typical of six separate experiments. (b) HIV (IIIb) infectivity of Jurkat T-cells was estimated by staining cells treated identically to those in (a) for $p24^{gag}$ expression. Values are mean ± S.D. percentage of cells stained for p24gag averaged from 12 fields of view from each of two separate wells. (c) HIV (MN) infectivity of THP-1 cells measured as in (b). MIP-1α was used at 100 ng/ml final concentration. (d) Immunofluorescence micrographs of THP-1 cells 48 hours after exposure to HIV (MN) in the absence (top panel) or presence (lower panel) of 100 μM peptide 3, stained for $p24^{gag}$. Several cells in the upper panel, but not the lower panel, show strong, punctate staining for $p24^{gag}$ antigen. Both fields of view contain 40-50 cells (assessed by Hoeschst 33345 staining viewed under UV illumination conditions). Figure 55 shows alkaphore skeletons.

## Detailed Description of the Invention

### Definitions

"Chemokines" refers to a family of proinflammatory signaling molecules which act on macrophage, B cells, T cells, neutrophils, eosinophils, basophils, mast cells, smooth muscle cells, e.g., vascular smooth muscle cells, and the like (e.g., by affecting their migration, proliferation, or degranulation, or the immunomodulation of T cell development to Th1 and Th2 subtypes). Preferred chemokines are primate in origin, e.g., human, although the invention includes other mammalian chemokines, such as those of bovine, ovine, equine, canine, feline or rodent origin, as well as virally encoded chemokines. Chemokines include, but are not limited to, MCP-1 (SEQ ID NO: 16), MCP-2 (SEQ ID NO: 17), MCP-3 (SEQ ID NO: 18), MIG (SEQ ID NO:45), MIP1α (SEQ ID NO:19), MIP1β (SEQ ID NO:20), RANTES (SEQ ID NO:21), PF4 (SEQ ID NO:46), I-309 (SEQ ID NO:47), HCC-1 (SEQ ID NO:48), eotaxin (SEQ ID NO:25), C10 (SEQ ID NO:49), CCR-2 (SEQ ID NO:50), ENA-78 (SEQ ID NO:52), GROα (SEQ ID NO:24), GROβ (SEQ ID NO:53), IL-8 (SEQ ID NO: 23), IP, e.g., IP-10 (SEQ ID NO:54), SDF1α (SEQ ID NO:22), SDF1β (SEQ ID NO:56), GROα (SEQ ID NO:57), MIP3α, TCA-3, CTAPIII, NAP, MARC/FYK, β-thromboglobulin, GCP, e.g., GCP-2, PBP, HC14, MDC, Zsig-35, β-6, TECK, PARC, 6Ckine, fractakine, DC-CK1, LIX, LKN, TARC, LARC, SCM-1, STCP1, LKN, SLC, LMC, IBICK, ILINCK, MCIF, MPIF, MIG, Zchemo-8 (see WO98/54326), Ckβ, e.g., Ckβ8, Ckβ4, and Ckβ13, CCF18/MRP-2, C10, CCIII, CKα2, ENA, H1305, HCC, Dvic-1, MGSA, DGWCC, TCA4, dendrokine (see WO 97/29192), CC2/HCC1, CC3, and MIP1τ, as well as virally encoded chemokines such as ELC, vMIP-I, vMIP-II and vMIP-III (see Kledal et al., Science, 277, 1656 (1997)). "CXC" or "α" chemokines include, but are not limited to, IL-8, PF4, IP10, NAP-2, GROα, GROβ, GROγ, SDF1, MIP2, MGSA, γIP, CTAPIII, β-thromboglobulin, MIG, PBP, NAP-2 and ENA78. "CC" or "β" chemokines include, but are not limited to, MCP-1, MCP-2, MCP-3, MCP-4, MCP-5, RANTES, eotaxin, LARC, TARC, C10, MIP1α, MIP1β, I309, HCC-1, CKβ8, CCF18/MRP-2, MIP1τ. A third type of chemokines are "C" chemokines, e.g., lymphotactin. A fourth type of chemokines are "$CX_3C$" chemokines such as fractakine or neurotactin (Rollins et al., Blood, 90, 404 (1997)). A fifth type of chemokines, $CX_2C$ chemokines, include CCIII.

"Peptide 3" refers to a peptide derived from a chemokine, which is generally located in (derived from) the carboxy-terminal half of the chemokine, and which inhibits the activity of at least the corresponding native chemokine, as determined by methods well known to the art. Peptide 3 comprises no more than 30, preferably about 3 to about 25, more preferably about 3 to about 15, and even more preferably about 3 to about 11, peptidyl residues which have 100% contiguous amino acid sequence homology or identity to the amino acid sequence of the corresponding native chemokine, preferably a mammalian chemokine, e.g., a primate chemokine such as a human chemokine, or a virally-encoded chemokine. For example, a preferred peptide 3 of MCP-1 that inhibits at least the activity of MCP-1 is peptide 3(1-12) [MCP-1], e.g., a peptide which has an amino acid sequence corresponding to SEQ ID NO:1, or a fragment or derivative thereof. Another preferred embodiment of the invention is peptide 3(3-12)[MCP-1], e.g., a peptide having an amino acid sequence corresponding to SEQ ID NO:7, or a fragment or derivative thereof. Preferably, a chemokine peptide 3 of the invention does not include a peptide of IL-8, PF-4 or NAP-2.

An alignment of chemokine amino acid sequences, such as the alignment depicted in Table 1, provides a general method to identify the location of peptide 3 sequences in chemokines. Generally, peptide 3 in non-MCP-1 chemokines corresponds to about residue 46 to about residue 67 of mature human MCP-1. Moreover, it is envisioned that peptide 3 may comprise moieties other than the amino acid sequence which inhibits chemokine activity, e.g., amino acid residues not present in the native chemokine (i.e., a fusion protein), nucleic acid molecules or targeting moieties such as antibodies or fragments thereof or biotin, so long as these moieties do not substantially reduce the biological activity of peptide 3. A substantial reduction in activity means a reduction in activity of greater than about 99%.

Also preferably, a peptide, variant, analog or derivative of the invention, has increased affinity for at least one chemokine receptor, e.g., about 1 μM to about 1 nM, more preferably about 1 nM to about 1 pM, and also preferably has decreased Duffy binding, relative to a corresponding peptide having the native ("wild-type") sequence or relative to the corresponding native chemokine. However, certain populations have individuals who are $Duffy^-$, e.g., a certain percentage of African Americans are $Duffy^-$. Thus, agents useful to treat these populations may have Duffy binding affinity that is equal to or greater than that of the corresponding native chemokine.

As used herein, the terms "isolated and/or purified" refer to *in vitro* preparation, isolation and/or purification of a therapeutic agent of the invention, so that it is not associated with *in vivo* substances. Thus, with respect to an "isolated nucleic acid molecule", which includes a polynucleotide of genomic, cDNA, or synthetic origin or some combination thereof, the "isolated nucleic acid molecule" (1) is not associated with all or a portion of a polynucleotide in which the "isolated nucleic acid molecule" is found in nature, (2) is operably linked to a polynucleotide which it is not linked to in nature, or (3) does not occur in nature as part of a larger sequence. An isolated nucleic acid molecule means a polymeric form of nucleotides of at least 10 bases in length, either ribonucleotides or deoxynucleotides or a modified form of either type of nucleotide. The term includes single and double stranded forms of DNA. The term "oligonucleotide" referred to herein includes naturally occurring, and modified nucleotides linked together by naturally occurring, and non-naturally

occurring oligonucleotide linkages. Oligonucleotides are a polynucleotide subset with 200 bases or fewer in length. Preferably, oligonucleotides are 10 to 60 bases in length and most preferably 12, 13, 14, 15, 16, 17, 18, 19, or 20 to 40 bases in length. Oligonucleotides are usually single stranded, e.g., for probes; although oligonucleotides may be double stranded, e.g., for use in the construction of a variant. Oligonucleotides of the invention can be either sense or antisense oligonucleotides. The term "naturally occurring nucleotides" referred to herein includes deoxyribonucleotides and ribonucleotides. The term "modified nucleotides" referred to herein includes nucleotides with modified or substituted sugar groups and the like. The term "oligonucleotide linkages" referred to herein includes oligonucleotides linkages such as phosphorothioate, phosphorodithioate, phophoroselenoate, phosphorodiselenoate, phosphoroanilothioate, phosphoraniladate, phosphoranidate, and the like. An oligonucleotide can include a label for detection, if desired.

The term "isolated polypeptide" means a polypeptide encoded by cDNA or recombinant RNA, or is synthetic origin, or some combination thereof, which isolated polypeptide (1) is not associated with proteins found in nature, (2) is free of other proteins from the same source, e.g., free of human proteins, (3) is expressed by a cell from a different species, or (4) does not occur in nature.

The term "sequence homology" means the proportion of base matches between two nucleic acid sequences or the proportion amino acid matches between two amino acid sequences. When sequence homology is expressed as a percentage, e.g., 50%, the percentage denotes the proportion of matches over the length of sequence from a chemokine that is compared to some other sequence. Gaps (in either of the two sequences) are permitted to maximize matching; gap lengths of 15 bases or less are usually used, 6 bases or less are preferred with 2 bases or less more preferred. When using oligonucleotides as probes or treatments, the sequence homology between the target nucleic acid and the oligonucleotide sequence is generally not less than 17 target base matches out of 20 possible oligonucleotide base pair matches (85%); preferably not less than 9 matches out of 10 possible base pair matches (90%), and more preferably not less than 19 matches out of 20 possible base pair matches (95%).

The term "selectively hybridize" means to detectably and specifically bind. Polynucleotides, oligonucleotides and fragments of the invention selectively hybridize to nucleic acid strands under hybridization and wash conditions that minimize appreciable amounts of detectable binding to nonspecific nucleic acids. High stringency conditions can be used to achieve selective hybridization conditions as known in the art and discussed herein. Generally, the nucleic acid sequence homology between the polynucleotides, oligonucleotides, and fragments of the invention and a nucleic acid sequence of interest is at least 65%, and more typically with preferably increasing homologies of at least about 70%, about 90%, about 95%, about 98%, and 100%.

Two amino acid sequences are homologous if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are preferred with 2 or less being more preferred. Alternatively and preferably, two protein sequences (or polypeptide sequences derived from them of at least 30 amino acids in length) are homologous, as this term is used herein, if they have an alignment score of at more than 5 (in standard deviation units) using the program ALIGN with the mutation data matrix and a gap penalty of 6 or greater. See Dayhoff, M. O., in Atlas of Protein Sequence and Structure, 1972, volume 5, National Biomedical Research Foundation, pp. 101-110, and Supplement 2 to this volume, pp. 1-10. The two sequences or parts thereof are more preferably homologous if their amino acids are greater than or equal to 50% identical when optimally aligned using the ALIGN program.

The term "corresponds to" is used herein to mean that a polynucleotide sequence is homologous (i.e., is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

The following terms are used to describe the sequence relationships between two or more polynucleotides: "reference sequence", "comparison window", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing, or may comprise a complete cDNA or gene sequence. Generally, a reference sequence is at least 20 nucleotides in length, frequently at least 25 nucleotides in length, and often at least 50 nucleotides in length. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity.

A "comparison window", as used herein, refers to a conceptual segment of at least 20 contiguous nucleotides and wherein the portion of the polynucleotide sequence in the comparison window may comprise additions or deletions

(i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman (1981) Adv. Appl. Math. 2: 482, by the homology alignment algorithm of Needleman and Wunsch (1970) J. Mol Biol. 48: 443, by the search for similarity method of Pearson and Lipman (1988) Proc. Natl_ Acad_ Sci. (USA.) 85: 2444, by computerized implementations of these algorithms (GAP, BESTFIT, FASTA, and TFASTA in the Wisconsin Genetics Software Package Release 7.0, Genetics Computer Group, 575 Science Dr., Madison, Wis.), or by inspection, and the best alignment (i.e., resulting in the highest percentage of homology over the comparison window) generated by the various methods is selected.

The term "sequence identity" means that two polynucleotide sequences are identical (i.e., on a nucleotide-by-nucleotide basis) over the window of comparison. The term "percentage of sequence identity" means that two polynucleotide sequences are identical (i.e., on a nucleotide-by-nucleotide basis) over the window of comparison. The term "percentage of sequence identity" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparison (i.e., the window size), and multiplying the result by 100 to yield the percentage of sequence identity. The terms "substantial identity" as used herein denote a characteristic of a polynucleotide sequence, wherein the polynucleotide comprises a sequence that has at least 85 percent sequence identity, preferably at least 90 to 95 percent sequence identity, more usually at least 99 percent sequence identity as compared to a reference sequence over a comparison window of at least 20 nucleotide positions, frequently over a window of at least 20-50 nucleotides, wherein the percentage of sequence identity is calculated by comparing the reference sequence to the polynucleotide sequence which may include deletions or additions which total 20 percent or less of the reference sequence over the window of comparison. The reference sequence may be a subset of a larger sequence, for example, as a segment of human MCP-1.

As applied to polypeptides, the term "substantial identity" means that two peptide sequences, when optimally aligned, such as by the programs GAP or BESTFIT using default gap weights, share at least about 80 percent sequence identity, preferably at least about 90 percent sequence identity, more preferably at least about 95 percent sequence identity, and most preferably at least about 99 percent sequence identity.

As used herein, the terms "label" or "labeled" refer to incorporation of a detectable marker, e.g., by incorporation of a radiolabeled amino acid or attachment to a polypeptide of biotinyl moieties that can be detected by marked avidin (e.g., streptavidin containing a fluorescent marker or enzymatic activity that can be detected by optical or colorimetric methods). Various methods of labeling polypeptides are known in the art and may be used. Examples of labels for polypeptides include, but are not limited to, the following: radioisotopes (e.g., $^{3}H$, $^{14}C$, $^{35}S$, $^{125}I$, $^{131}I$), fluorescent labels (e.g., FITC, rhodamine, lanthanide, phosphors), enzymatic labels (e.g., horseradish peroxidase, β-galactosidase, luciferase, alkaline phosphatase), chemiluminescent, biotinyl groups, predetermined polypeptide epitopes recognized by a secondary reporter (e.g., leucine zipper pair sequences, binding sites for secondary antibodies, metal binding domains, epitope tags). In some embodiments, labels are attached by spacer arms of various lengths to reduce potential steric hindrance.

As used herein, "substantially pure" means an object species is the predominant species present (i.e., on a molar basis it is more abundant than any other individual species in the composition), and preferably a substantially purified fraction is a composition wherein the object species comprises at least about 50 percent (on a molar basis) of all macromolecular species present. Generally, a substantially pure composition will comprise more than about 80 percent of all macromolecular species present in the composition, more preferably more than about 85%, about 90%, about 95%, and about 99%. Most preferably, the object species is purified to essential homogeneity (contaminant species cannot be detected in the composition by conventional detection methods) wherein the composition consists essentially of a single macromolecular species.

An isolated "chemokine peptide variant" of peptide 3 or peptide 2 is a peptide comprising no more than 30, preferably about 3 to about 25, and more preferably about 3 to about 18, and even more preferably about 3 to about 11, peptidyl residues which have at least 50%, preferably at least about 80%, and more preferably at least about 90% but less than 100%, contiguous amino acid sequence homology or identity to the amino acid sequence of the corresponding native chemokine, e.g., $Ser_7$ peptide 3(1-12)[MCP1] (SEQ ID NO:11) has less than 100% homology to the corresponding amino acid sequence of MCP-1, i.e., peptide 3(1-12)[MCP-1] (SEQ ID NO:1). A variant of the invention may include amino acid residues not present in the corresponding native chemokine, and internal deletions relative to the corresponding native chemokine. Chemokine peptide variants include peptides having at least one D-amino acid.

Chemokine peptides or peptide variants which are subjected to chemical modifications, such as esterification, amidation, reduction, protection and the like, are referred to as chemokine "derivatives." For example, a modification known to improve the stability and bioavailability of peptides *in vivo* is the cyclization of the peptide, for example through one or more disulfide bonds. A preferred modification is the synthesis of a cyclic reverse sequence derivative (CRD) of a peptide of the invention. A linear peptide is synthesized with all D-form amino acids using the reverse (i.e., C-terminal

to N-terminal) sequence of the peptide. If necessary, additional cysteine residues are added to the N and C termini (if the peptide sequence does not already have N and C terminal cys residues), thereby allowing oxidative cyclization. However, the term "CRD" includes cyclization by other mechanisms, e.g., via a peptidyl bond, and the like. A preferred derivative of the invention is CRD-$Cys_0Cys_{13}Leu_4Ile_{11}$peptide 3[MCP-1] or CRD-$Cys_{13}Leu_4Ile_{11}$ peptide 3(3-12)[MCP-1].

Also included within the scope of the term "derivative" is linear reverse D (LRD) and cyclized forward L (CFL) derivatives. LRD derivatives have the reverse (i.e., C-terminal to N-terminal) sequence of the peptide with all D-form amino acids, but are not cyclized. CFL derivatives have the forward (i.e., N-terminal to C-terminal) sequence of the peptide with all L-form amino acids, but with additional N and C terminal cys residues (if the peptide sequence does not already have cys residues at either the N or the C terminal position), followed by oxidative cyclization, or cyclization by an alternative method. Other "derivatives" of the invention include branched peptides, circular, branched and branched circular peptides.

A "chemokine analog" means a moiety that mimics or inhibits a chemokine-induced activity, or binds to or near a chemokine receptor but does not mimic or inhibit chemokine activity (neutral), wherein the portion of the moiety that mimics or inhibits the chemokine-induced activity, or binds to or near the receptor but is neutral, is not a peptide, and wherein the active portion of the analog is not a nucleic acid molecule. As used herein, the term "mimics" means that the moiety induces an activity that is induced by a native chemokine, but that the induction by the analog is not necessarily of the same magnitude as the induction of activity by the native chemokine.

It is also envisioned that the chemokine peptides, variants, analogs and derivatives thereof, of the invention may comprise moieties other than the portion which inhibits or mimics chemokine activity, or binds to or near a chemokine receptor without eliciting or inhibiting signaling, e.g., peptide or polypeptide molecules, such as antibodies or fragments thereof or fusion proteins, nucleic acid molecules, sugars, lipids, fats, a detectable signal molecule such as a radioisotope, e.g., gamma emitters, paramagnetic molecules or sound wave emitters, small chemicals, metals, salts, synthetic polymers, e.g., polylactide and polyglycolide, surfactants and glycosaminoglycans, which preferably are covalently attached or linked to the portion of the peptide, variant, analog or derivative that mimics or inhibits the chemokine-induced activity, so long as the other moieties do not alter the biological activity of the peptide, variant, analog or derivative. Also envisioned is a chemokine peptide, variant, analog or derivative that is non-covalently associated with the moieties described above.

As used herein the term "saccharide" includes monosaccharides, disaccharides, trisaccharides and polysaccharides. The term includes glucose, sucrose fructose and ribose, as well as deoxy sugars such as deoxyribose and the like. Saccharide derivatives can conveniently be prepared as described in International Patent Applications Publication Numbers WO 96/34005 and WO97/03995. A saccharide can conveniently be linked to the remainder of a compound of formula I through an ether bond.

As used herein, halo is fluoro, chloro, bromo, or iodo. The terms alkyl and alkoxy denote both straight and branched groups, but reference to an individual radical such as "propyl" embraces only the straight chain radical, a branched chain isomer such as "isopropyl" being specifically referred to. Aryl denotes a phenyl radical or an ortho-fused bicyclic carbocyclic radical having about nine to ten ring atoms in which at least one ring is aromatic. Heteroaryl encompasses a radical attached via a ring carbon of a monocyclic aromatic ring containing five or six ring atoms consisting of carbon and one to four heteroatoms each selected from the group consisting of non-peroxide oxygen, sulfur, and $N(R^4)$ wherein $R^4$ is absent or is hydrogen, $(C_1-C_4)$alkyl, phenyl or benzyl, as well as a radical of an ortho-fused bicyclic heterocycle of about eight to ten ring atoms derived therefrom, particularly a benz-derivative or one derived by fusing a propylene, trimethylene, or tetra methylene diradical thereto.

A preferred chemokine analog of the invention is a compound of formula (IV):

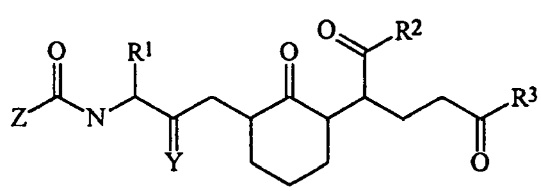

(IV)

wherein $R^1$ is aryl, heteroaryl, aryl$(C_1-C_3)$alkyl, heteroaryl$(C_1-C_3)$alkyl, coumaryl, coumaryl$(C_1-C_3)$alkyl, chromanyl or chromanyl$(C_1-C_3)$alkyl; wherein any aryl or heteroaryl group, or the benz-ring of any coumaryl or chromanyl group may optionally be substituted with one, two or three substituents selected from the group consisting of halo, nitro, cyano, hydroxy, $(C_1-C_6)$alkyl, $(C_1-C_6)$alkoxy, $(C_1-C_6)$alkanoyl, $(C_2-C_6)$alkanoyloxy, $-C(=O)(C_1-C_6)$alkoxy, $C(=O)NR^gR^h$, $NR^iR^j$;
wherein $R^2$ is $(C_1-C_{10})$alkyl, $(C_3-C_6)$cycloalkyl, $(C_3-C_6)$cycloalkyl$(C_1-C_6)$alkyl, $(C_1-C_{10})$alkoxy, $(C_3-C_6)$cycloalkyl$(C_1-C_6)$ alkoxy or $N(R^a)(R^b)$;
wherein $R^3$ is $(C_1-C_{10})$alkyl, $(C_3-C_6)$cycloalkyl, $(C_3-C_6)$cycloallcyl$(C_1-C_6)$alkyl, $(C_1-C_{10})$alkoxy, $(C_3-C_6)$cycloalkyl$(C_1-C_6)$

alkoxy or N($R^c$)($R^d$);
wherein Y is oxo or thioxo;
wherein Z is ($C_1$-$C_{15}$)alkyl, ($C_1$-$C_6$)cycloalkyl, ($C_3$-$C_6$)cycloalkyl($C_1$-$C_6$)alkyl, ($C_1$-$C_{15}$)alkoxy, ($C_3$-$C_6$)cycloalkyl($C_1$-$C_6$) alkoxy or N($R^e$)($R^f$); and
wherein $R^a$-$R^j$ are each independently hydrogen, ($C_1$-$C_{10}$)alkyl, ($C_1$-$C_{10}$)alkanoyl, phenyl, benzyl, or phenethyl; or $R^a$ and $R^b$, $R^c$ and $R^d$, $R^c$ and $R^f$, $R^g$ and $R^h$, or $R^i$ and $R^j$ together with the nitrogen to which they are attached form a ring selected from pyrrolidino, piperidino, or morpholino; or a pharmaceutically acceptable salt thereof.

A preferred embodiment of a compound of formula (IV) includes a compound of formula (IV) wherein $R^1$ is aryl, heteroaryl, coumaryl, or chromanyl. Preferably aryl is phenyl; and heteroaryl is indolyl or pyridinyl. Another preferred embodiment of a compound of formula (IV) includes a compound of a formula (IV) wherein $R^2$ is N($R^a$)($R^b$); and $R^3$ is N($R^c$)($R^d$). Yet another preferred embodiment of a compound of formula (IV) includes a compound of a formula (IV) wherein Z is ($C_1$-$C_{15}$)alkyl.

A further preferred compound is a compound of formula (IV) wherein $R^1$ is indolyl; $R^2$ is N($R^a$)($R^b$); $R^3$ is N($R^c$)($R^d$); Y is S; Z is hydrogen; and $R^a$, $R^b$, $R^c$, and $R^d$ are each methyl.

Yet another preferred compound of formula (IV) includes a compound wherein $R^1$ is 2-benzimidazolyl; for $R^2$ is N($R^a$)($R^b$); $R^3$is N($R^c$)($R^d$); Y is oxo; and Z is N($R^e$)($R^f$) or a pharmaceutically acceptable salt thereof. Another preferred compound of formula (IV) is a compound wherein $R^1$ is 2-benzimidazolyl; $R^2$ is $N(Me)_2$; $R^3$ is $N(Me)_2$; Y is oxo; and Z is $N(Me)_2$; or a pharmaceutically acceptable salt thereof.

Another preferred chemokine analog of the invention is a compound of formula (V):

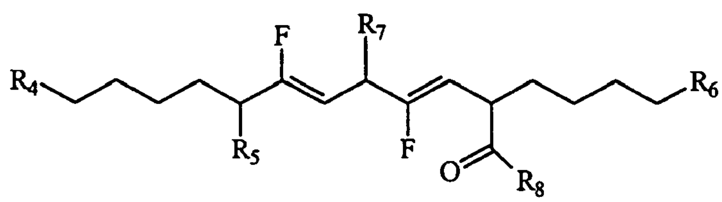

wherein $R^4$ is $NR_kR_l$; wherein $R^5$ is $NR_mR_n$; wherein $R^6$ is $NR_oR_p$; wherein $R^7$ is $Nr_qR_r$; wherein $R^8$ is hydrogen, hydroxy, ($C_1$-$C_{10}$)alkyl, ($C_3$-$C_6$)cycloalkyl, ($C_3$ $C_6$)cycloalkyl($C_1$-$C_6$)alkyl, ($C_1$-$C_{10}$)alkoxy, ($C_3$-$C_6$)cycloalkyl($C_1$-$C_6$)alkoxy, $NR_sR_t$, the amino terminus of an amino acid or the N-terminal residue of a peptide of 2 to about 25 amino acid residues; wherein $R_k$, $R_l$, $R_o$, and $R_p$ are each independently hydrogen, ($C_1$-$C_{10}$)alkyl, ($C_3$-$C_6$)cycloalkyl, ($C_3$-$C_6$)cycloalkyl($C_1$-$C_6$)alkyl, ($C_1$-$C_{10}$)alkanoyl, phenyl, benzyl or phenethyl; wherein $R_m$ are $R_n$ are each independently hydrogen, ($C_1$-$C_{10}$)alkyl, ($C_3$-$C_6$)cycloalkyl, ($C_3$-$C_6$)cycloalkyl($C_1$-$C_6$)alkyl, ($C_1$-$C_{10}$)alkoxy, ($C_1$-$C_{10}$)alkanoyl, ($C_1$-$C_{10}$)alkoxycarbonyl, 9-fluorenylmethoxycarbonyl, phenyl, benzyl, phenethyl, the C-terminal residue of an amino acid or a peptide of 2 to about 25 amino acid residues; wherein Rq are $R_r$ are each independently hydrogen, ($C_1$-$C_{10}$)alkyl, ($C_3$ $C_6$)cycloalkyl, ($C_3$-$C_6$)cycloalkyl($C_1$-$C_6$)alkyl, phenyl, benzyl or phenethyl; wherein $R_s$ are $R_t$ are each independently hydrogen, ($C_{1,}$-$C_{10}$)alkyl, ($C_3$-$C_6$)cycloalkyl, ($C_1$-$C_6$)cycloalkyl($C_1$-$C_6$)alkyl, phenyl, benzyl or phenethyl; or a pharmaceutically acceptable salt thereof.

Preferably $R_k$, $R_1$, $R_o$, and $R_p$ are each hydrogen; $R_m$ are $R_n$ are each independently hydrogen, acetyl, ($C_1$-$C_{10}$) alkyl, ($C_3$-$C_6$)cycloalkyl, propoxy, butoxy, tert-butoxycarbonyl, 9-fluorenylmethoxycarbonyl, the C-terminal residue of an amino acid or a peptide of 2 to about 25 amino acid residues; and $R_q$ are $R_r$ are each independently hydrogen, ($C_1$-$C_{10}$) alkyl, or ($C_3$-$C_6$cycloalkyl.

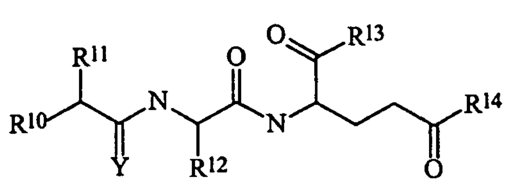

(VI)

Another preferred analog of a chemokine is a compound of formula (XIII):

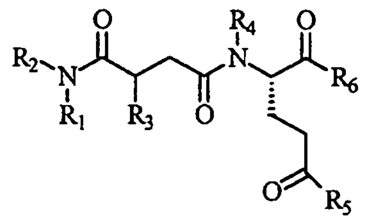

wherein $R_1$ is aryl, heteroaryl, aryl($C_1$-$C_{10}$)alkyl, aryl($C_1$-$C_{10}$)alkanoyl, heteroaryl($C_1$-$C_{10}$)alkyl, or heteroaryl($C_1$-$C_{10}$) alkanoyl; $R_2$ is hydrogen, ($C_1$-$C_{15}$)alkyl, ($C_3$-$C_8$)cycloalkyl, ($C_3$-$C_8$)cycloalkyl($C_1$-$C_{10}$)alkyl, aryl, or aryl($C_1$-$C_{10}$)alkyl; $R_3$ is hydrogen, or ($C_1$-$C_{10}$)alkyl, $R_4$ is hydrogen, or ($C_1$-$C_{10}$)alkyl; $R_5$ is N($R^a$)($R^a$); $R_5$ is N($R^a$)($R^b$); and each $R^a$ and $R^b$ is independently hydrogen, ($C_1$-$C_{10}$)alkyl, ($C_1$-$C_{10}$)alkanoyl, or aryl($C_1$-$C_{10}$)alkyl; or $R^a$ and $R^b$ together with the nitrogen to which they are attached form a pyrrolidino, piperidino or morpholino ring; wherein any aryl or heteroaryl is optionally substituted with 1, 2, 3, or 4 substituents independently selected from the group consisting of halo, cyano, hydroxy, nitro, trifluoromethyl, trifluoromethoxy, ($C_1$-$C_6$)alkyl, ($C_1$-$C_6$)alkoxy, ($C_1$-$C_6$)alkanoyl, ($C_1$-$C_6$)alkanoyloxy, ($C_1$-$C_6$)alkoxycarbonyl, and methylenedioxy; or a pharmaceutically acceptable salt thereof.

For a compound of formula (XIII), $R_1$ can specifically be 3-indolylmethyl; $R_2$ can specifically be isopropyl, tert-butyl, or phenyl; $R_3$ can specifically be methyl; $R_4$ can specifically be hydrogen; $R_5$ can specifically be amino; and $R_6$ can specifically be dimethylamino, benzylamino, or hydroxybenzylamino.

Another preferred analog of the invention is a compound of formula (XI):

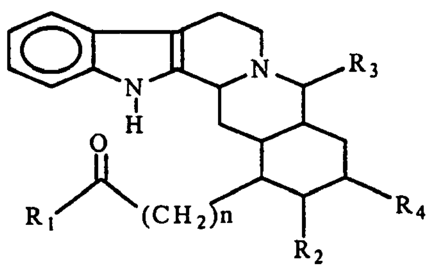

**(XI)**

wherein $R_1$ is O(Ra) wherein Ra is H, ($C_1$-$C_6$)alkyl, ($C_1$-$C_6$)alkanoyl, ($C_1$-$C_6$)alkanoyloxy, ($C_6$-$C_{10}$)aryl or ($C_6$-$C_{10}$)heteroaryl; or N(Rb)(Rc) wherein each Rb and Rc is independently H or ($C_1$-$C_6$)alkyl; $R_2$ is O(Ra) wherein Ra is H, ($C_1$-$C_6$) alkyl, ($C_1$-$C_6$)alkanoyl, ($C_1$-$C_6$)alkanoyloxy, ($C_6$-$C_{10}$)aryl or ($C_6$-$C_{10}$)heteroaryl; or N(Rb)(Rc) wherein each Rb and Rc is independently H or ($C_1$-$C_6$)alkyl; $R_3$ is H, C(=O) or C(=S); $R_4$ is H; C(=O); C(=S); O(Ra) wherein Ra is H, ($C_1$-$C_6$) alkyl, ($C_1$-$C_6$)alkanoyloxy, ($C_1$-$C_6$)alkanoyl, ($C_6$-$C_{10}$)aryl or ($C_6$-$C_{10}$)heteroaryl; or N(Rb)(Rc) wherein each Rb and Rc is independently H or ($C_1$-$C_6$)alkyl; and n is an integer between 0 and 6, inclusive; wherein any ($C_6$ $C_{10}$)aryl, ($C_1$-$C_6$) alkyl, ($C_1$-$C_6$)alkanoyloxy, ($C_6$-$C_{10}$)heteroaryl or ($C_1$-$C_6$)alkanoyl is optionally substituted with at least one substituent selected from the group consisting of halo, cyano, nitro, trifluoromethyl, trifluoromethoxy, ($C_1$-$C_6$)alkyl, ($C_1$-$C_6$)alkoxy, ($C_1$-$C_6$)alkanoyl, ($C_1$-$C_6$alkanoyloxy, ($C_1$-$C_6$)alkoxycarbonyl, methoxydioxy, hydroxy, C(=O), and N(Rb)(Rc) wherein each Rb and Rc is independently H or ($C_1$-$C_6$)alkyl; or a pharmaceutically acceptable salt thereof.

Specific compounds of formula XIV are shown in the following table.

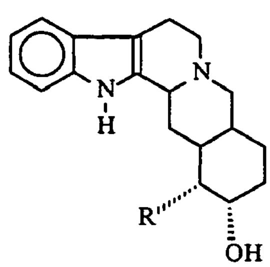

| Compound | R |
|---|---|
| Y-I | R = methoxycarbonyl |
| Y-II | R = aminocarbonyl |
| Y-III | R = methylaminocarbonyl |
| Y-IV | R = dimethylaminocarbonyl |
| Y-V | R = N-(methyl)glutamino |
| Y-VI | R = aminocarbonylmethyl |

Specific compounds of formula (X) are shown in the following table.

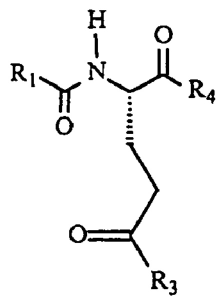

| Compound | $R_1$ | $R_3$ | $R_4$ |
|---|---|---|---|
| NR58,1 | 9-decenyl | amino | hydroxy |
| NR58,2 | 9-decenyl | amino | amino |
| NR58,3 | 9-decenyl | amino | 4-hydroxybenzyl-amino |
| NR58,5 | *tert*-butyl | amino | hydroxy |
| NR58,6 | *tert*-butyl | amino | amino |
| NR58,7 | *tert*-butyl | amino | 4-hydroxybenzyl-amino |
| NR58,9 | phenyl | amino | hydroxy |
| NR58,10 | phenyl | amino | amino |
| NR58,11 | 4-hydroxyphenyl | amino | 4-hydroxybenzyl-amino |
| NR58,13 | *tert*-butyl | amino | hydroxy |
| NR58,14 | benzoylaminomethyl | amino | hydroxy |
| NR58,15 | 4-hydroxybenzyloxy-carbonylaminomethyl | amino | hydroxy |

Other specific compounds of formula (X) include [3S]-3-(undec-10-enoylamino)piperidine-2,6-dione (compound 58,4) and N-benzoyl-L-pyroglutamate (compound 58,12).

Other specific compounds of formula (X) are shown in the following table.

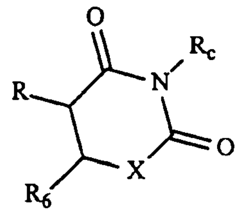

| Compound | X | $R_6$ | $R_c$ | R |
|---|---|---|---|---|
| A-I (NR58,4) | $CH_2$ | H | H | 10-undecenoylamino |
| A-II | $CH_2$ | H | H | 10-undecanoylamino |
| A-III | $CH_2$ | H | H | 8-nonenoylamino |
| A-IV | $CH_2$ | H | H | 6-heptenoylamino |
| A-V | $CH_2$ | H | H | 4-pentenoylamino |
| A-VI | $CH_2$ | H | H | 2-propenoylamino |
| A-VIII | $CH_2$ | H | H | N-methyl-10-undecenoylamino |
| A-IX | $CH_2$ | H | Me | 10-undecenoylamino |
| A-X | $CH_2$ | H | Me | N-methyl-10-undecenoylamino |
| A-XI | direct bond | H | H | 10-undecenoylamino |
| A-XII | NH | oxo | H | 10-undecenoylamino |
| A-XIII | NH | oxo | H | N-methyl-10-undecenoylamino |

Specific compounds of formula (XI) are shown in the following table.

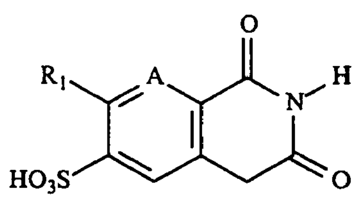

| Compound | $R_1$ | A |
|---|---|---|
| B-1 | 10-undecenoylamino | CH |
| B-II | 9-decenyl | N |
| B-III | 9-decenyl | CH |

Specific compounds of formula (XII) are shown in the following table.

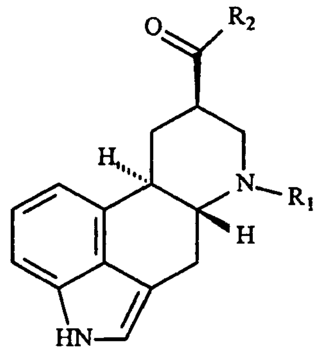

| Compound | $R_1$ | $R_2$ |
|---|---|---|
| L-I | Me | OH |
| L-II | Me | L-glutamino |

It will be appreciated by those skilled in the art that compounds of formula (IV), (V), (VI), (XIII), (XIV), (X), (XI), (XIX), and (XII), and compounds of the invention which are peptides having chiral centers, may exist in and be isolated in optically active and racemic forms. For example, compounds of the invention comprise $\alpha$-amino acid residues in D or L form, or mixtures thereof. Some compounds may exhibit polymorphism. It is to be understood that the present invention encompasses any racemic, optically-active, polymorphic, or stereoisomeric form, or mixtures thereof, of a compound of the invention, which possess the useful properties described herein. It is well known in the art how to prepare optically active forms (for example, by resolution of the racemic form by recrystallization techniques, by synthesis, from optically-active starting materials, by chiral synthesis, or by chromatographic separation using a chiral stationary phase). It is also well known to the art how to determine a compounds ability to inhibit or enhance chemokine-induced activity using the standard tests described herein, or using other tests which are well known in the art.

Specific and preferred values listed herein for radicals, substituents, and ranges, are for illustration only and they do not exclude other defined values or other values within defined ranges for the radicals and substituents. Specifically, ($C_1$-$C_{15}$)alkyl can be methyl, ethyl, propyl, isopropyl, butyl, iso-butyl, sec-butyl, pentyl, 3-pentyl, hexyl, heptyl, octyl, nonyl, decyl, undecyl, 9-methylundecyl, dodecyl; ($C_1$-$C_6$)alkyl can be methyl, ethyl, propyl, isopropyl, butyl, iso-butyl, sec-butyl, pentyl, 3-pentyl, or hexyl; ($C_1$-$C_3$)alkyl can be methyl, ethyl, or propyl; ($C_3$-$C_6$)cycloalkyl can be cyclopropyl, cyclobutyl, cyclopentyl, or cyclohexyl; ($C_1$-$C_{10}$)alkoxy can be methoxy, ethoxyl, propoxy, isopropoxy, butoxy, iso-butoxy, sec-butoxy, pentoxy, 3-pentoxy, hexyloxy, heptyloxy, octyloxy, nonyloxy, or decyloxy; ($C_1$-$C_6$)alkoxy can be methoxy, ethoxyl, propoxy, isopropoxy, butoxy, iso-butoxy, sec-butoxy, pentoxy, 3-pentoxy, or hexoxy; ($C_1$-$C_{10}$)alkanoyl can be formyl, acetyl, propanoyl, butanoyl, pentanoyl, hexanoyl, heptanoyl, octanoyl, nonanoyl, or decanoyl; ($C_1$-$C_6$) alkanoyl can be formyl, acetyl, propanoyl, butanoyl, pentanoyl, or hexanoyl; ($C_2$-$C_6$)alkanoyloxy can be acetoxy, propanoyloxy, butanoyloxy, pentanoyloxy, or hexanoyloxy; aryl can be phenyl, indenyl, or naphthyl; and heteroaryl can be furyl, imidazolyl, tetrazolyl, pyridyl, (or its N-oxide), thienyl, benzimidazolyl (or its N-oxide), pyrimidinyl (or its N-oxide), indolyl, or quinolyl (or its N-oxide).

In addition, it is understood that the agents of the invention (e.g. peptide 3, varients or derivatives thereof, a compound of formula (IV), a compound of formula (V), a compound of formula (VI), a compound of formula (XIII), a compound of formula (XIV), a compound of formula (X), a compound of formula (XI), a compound of formula (XIX), or a compound of formula (XII)), may be modified to include O-linked sugars and sugar chains (e.g. a saccharide), e.g., at hydroxyl, amide and/or ester groups, so as to yield a "saccharide conjugate". A preferred saccharide conjugate is the peptide derivative CRD-$Leu_4Ile_{11}Cys_{13}$ peptide 3(3-12)[MCP-1] linked to a one or more saccharides. For example the peptide derivative may be linked to the saccharide by an aminoglycosidic bond to either or both the amino terminus and one or both lysine $\in$-amino groups (for example as prepared in example 18).

Preferably, the therapeutic agents of the invention are biologically active. The biological activity of a chemokine peptide, peptide variant, analog or derivative thereof, can be measured by methods known to the art, some of which are described hereinbelow. For example, biologically active peptide 3[MCP-1] variants falling within the scope of the invention have at least about 1%, preferably at least about 10%, more preferably at least about 50%, and even more preferably at least about 90%, the activity of the corresponding native peptide sequence, e.g., peptide 3(1-12)[MCP-1] (SEQ ID NO:1), or the native chemokine, e.g., MCP-1 (SEQ ID NO:16). Thus, a peptide 3 variant, e.g., $Leu_4Ile_{11}$peptide

3(1-12)[MCP-1], falling within the scope of the invention has an $ED_{50}$ for inhibition that is at least about 1%, preferably at least about 10%, more preferably at least about 50%, and even more preferably at least about 90%, the maximal activity of peptide 3(1-12)[MCP-1] (SEQ ID NO:1) at 100 μM.

As used herein, "a chemokine-induced activity" includes, but is not limited to, an activity that is elicited through the binding of a chemokine, a therapeutic agent of the invention or other moiety, e.g., viral protein, to a chemokine receptor, or the binding of a therapeutic agent or other moiety in close physical proximity to the receptor so that the activity is altered. Chemokine receptors include, but are not limited to, CCR1 (CC-CKRI), CCR2a, CCR2b, CCR3, CCR4, CCR5, CCR6, CCR7, CCR8, HGBER32 (WO9S/09171), CXCR1 (IL8RI), CXCR2, CXCR3, $CX_3CR1$, CXCR4 and CXCR5. Chemotide receptors play a role in cell migration, cell activation, viral or parasite entry, release of proinflammatory compounds, and the like.

As used herein, "indications associated with chemokine-induced activity" includes, but is not limited to, atherosclerosis and other forms of local or systemic vasculitis, e.g., Behçet's syndrome, giant cell arteritis, polymyalgia rheumatica, Wegener's granulomatosis, Churg-Strauss syndrome vasculitis, Henoch-Schönlein purpura, Kawasaki disease, microscopic polyanglitis, Takayasu's arteritis, essential cryoglobulinemic vasculitis, cutaneous leukocytoclastic anglitis, polyarteritis nodosa, primary granulomatous central nervous system vasculitis, drug-induced antineutrophil cytoplasmic autoantibodies (ANCA)-associated vasculitis, cryoglobulinemic vasculitis, lupus vasculitis, rheumatoid vasculitis, Sjögren's syndrome vasculitis, hypocomplemtemic urticarial vasculitis, Goodpasture's syndrome, serum-sickness vasculitis, drug-induced immune complex vasculitis, paraneoplastic small vessel vasculitis (e.g., lymphoproliferative neoplasm-induced vasculitis, myeloproliferative neoplasm-induced vasculitis, and carcinoma-induced vasculitis), and inflammatory bowel disease vasculitis, diseases such as myocardial infarction, stroke, acute ischemia which is secondary to atherosclerosis; hypertension; reperfusion injury (Kumar et al., Circulation, 95, 693 (1997)); aortic aneurysms; vein graft hyperplasia (Stark et al., Athero., Thrombosis. and Vascular Biology, 17, 1614 (1997)); angiogenesis; hypercholesterolemia; congestive heart failure; Kawasaki's disease; stenosis or restenosis, particularly in patients undergoing angioplasty; pathologically low bone mineral density, such as osteoporosis (Posner et al., Bone, 21, 321 (1997)); ulcerative colitis; chronic obstructive pulmonary disease; stroke; infection with human immunodeficiency virus (HIV), other lentiviruses or retroviruses with similar mechanisms of cell entry via chemokine receptor(s), or infection with other viruses, e.g., cytomegalovirus (Sozzani et al., J. Leukoc. Biol., 62, 30 (1997)), or viral infection resulting in viral meningitis; organ transplantation, such as acute transplant rejection or delayed graft function, allograft rejection and graft versus host disease; transplant vasculopathy; malaria and other consequences of infection by parasites related to plasmodium; asthma; allergic diseases, such as atopy (IgE-mediated components), allergic rhinitis, atopic dermatitis, anaphylaxis, allergic bronchopulmonary aspergillosis (IgE-mediated), and hypersensitivity pneumonitis (high IgG and reactive T cells) (pigeon breeders disease, farmer's lung disease, humidifier lung disease, malt workers' lung disease); allergies, including flea allergy dermatitis in mammals such as domestic animals, e.g., dogs and cats, contact allergens including mosquito bites or other insect sting allergies, poison ivy, poison oak, poison sumac, or other skin allergens; urticaria; eczema; pulmonary fibrosis such as idiopathic pulmonary fibrosis; cystic fibrosis; hemolytic uremic syndrome (Van Setten et al., Pediatr. Res., 43, 759 (1998)); autoimmune disorders, including, but not limited to, type I diabetes, Crohn's disease, multiple sclerosis, arthritis, rheumatoid arthritis (Ogata et al., J. Pathol., 182, 106 (1997); Gong et al., J. Exp. Med., 186, 131 (1997)), systemic lupus erythematosus, autoimmune (Hasimoto's) thyroiditis, autoimmune liver diseases such as hepatitis and primary biliary cirrhosis, hyperthyroidism (Graves' disease; thyrotoxicosis), insulin-resistant diabetes, autoimmune adrenal insufficiency (Addison's disease), autoimmune oophoritis, autoimmune orchitis, autoimmune hemolytic anemia, paroxysmal cold hemoglobinuria, Behcet's disease, autoimmune thrombocytopenia, autoimmune neutropenia, pernicious anemia, pure red cell anemia, autoimmune coagulopathies, myasthenia gravis, experimental allergic encephalomyelitis, autoimmune polyneuritis, pemphigus and other bullous diseases, rheumatic carditis, Goodpasture's syndrome, postcardiotomy syndrome, Sjogren's syndrome, polymyositis, dermatomyositis, and scleroderma; eye diseases such as uveitis or blinding Herpes stromal keratitis; liver disease; erhlichiosis or Lyme disease including Lyme arthritis; aberrant hematopoiesis; otitis externa, e.g., formulated as a topical ear cleaner for domestic animals such as dogs and cats; intraperitoneal adhesions, e.g., adhesions which develop post-surgery, particularly after gynecologic or intestinal surgeries (Zeyneloglu et al., Am. J. Obstet. Gynecol., 179, 438 (1998)); scarring after surgery; radiation-induced fibrosis; renal disorders; post-trauma inflammation, e.g., post-surgical inflammation such as that following orthopedic surgeries, e.g., prosthetic implants, as well as atherectomy, circulatory surgeries, and tissue replacements; nephritis due to, for example, autosomal dominant polycystic kidney disease, diabetic nephropathy, IgA nephropathy, interstitial fibrosis, or lupus; as well as other disease states resulting from inappropriate inflammation, either local or systemic, for example, irritable or inflammatory bowel syndrome (Mazzucchelli et al., J Pathol., 178, 201 (1996)), skin diseases such as psoriasis (Gillitzer et al., Arch. Dermatol. Res., 284, 26 (1992); Yu et al., Lab Investig., 71, 226 (1994)) and lichen planus, delayed type hypersensitivity, Alzheimer's disease (Johnstone et al., J. Neuroimmunol., 93, 182 (1999)), chronic pulmonary inflammation, e.g., pulmonary alveolitis and pulmonary granuloma, gingival inflammation or other periodontal disease, and osseous inflammation associated with lesions of endodontic origin (Volejnikova et al., Am. J. Pathol)., 150, 1711 (1997)), hypersensitivity lung diseases such as hypersensitivity pneumonitis (Sugiyama et al., Eur. Respir. J., 8,

1084 (1995)), and inflammation related to histamine release from basophils (Dvorak et al., L Allergy Clin. Immunol., 98, 355 (1996)), such as hay fever, histamine release from mast cells (Galli et al., Ciba Foundation Symposium, 147, 53 (1989)), or mast cell tumors, types of type 1 hypersensitivity reactions (anaphylaxis, skin allergy, hives, allergic rhinitis, and allergic gastroenteritis); glomerulonephritis (Gesualdo et al., Kidney International, 51, 155 (1997)); inflammation associated with peritoneal dialysis (Sach et al., Nephrol, Dial. Transplant, 12, 315 (1997)); emphysema; and pancreatitis.

Other indications falling within the scope of the invention include, but are not limited to, neoplasia, e.g., histocytoma, glioma, sarcoma, osteosarcoma, osteoma (Zheng et al., J. Cell Biochem., 70, 121 (1998)), melanoma, Kaposi's sarcoma, small cell lung cancer, and ovarian carcinoma as well as myelosuppression and mucositis associated with chemotherapy; inflammatory pseudotumor of the lung; brain or spinal cord trauma, such as after disc surgery (Ghirnikar et al., J.Neurosci Res., 46, 727 (1996); Berman et al., J. Immunol., 156, 3017 (1996)); gout; lung disease, e.g., due to respiratory syncicial virus infection of humans, cattle, pigs and the like, or lung injury (Lukacs et al., Adv. Immunol., 62, 257 (1996)); adult respiratory distress syndrome (see Robbins, Pathologic Basis of Disease, Cotran et al. (Eds.), 5th ed.); strokes; Loeffler's syndrome; chronic eosinophilic pneumonia; pulmonary fibrosis; wound healing; bacterial infection, e.g., bacterial peritonitis, meningitis or gram negative sepsis; toxic shock syndrome; granulomatous diseases such as Mycobacteriosis, Pneumocystosis, Histoplasmosis, Blastomycosis, Coccidiomycosis, Cryptococcosis, Aspergillosis, granulomatous enteritis, Candidiasis, foreign body granulomas and peritonitis, pulmonary granulomatosis, Wegener's granulomatosis (Del Papa et al., Arthritis Rheum., 39, 758 (1996)), leprosy, syphilis, cat-scratch disease, schistosomiasis (Jacobs et al., Am. J. Pathol., 150, 2033 (1997)), silicosis, sarcoidosis (Iida et al., Thorax, 52,431 (1997); Car et al., Am. J. Respir. Crit. Care Med., 149, 655 (1994)) and berylliosis; lethal endotoxemia (Zisman et al., J._ Clin._ Invest., 99, 2832 (1997)); and indications associated with a weak inflammatory response, e.g., which occur in parasitic infection, e.g., *Leishmaniasis* (Moll, Biol Abs., 104, 21765 (1997)), trypanosome, *Mycobacterium leprae* or *Mycobacterium tuberculosis* infection, helminth infections, such as nematodes (round worms); (Trichuriasis, Enterobiasis, Ascariasis, Hookworm, Strongyloidiasis, Trichinosis, filariasis); trematodes (fluxes) (Schistosomiasis, Clonorchiasis), cestode (tape worms) (Echinococcosis, Taeniasis saginata, Cysticercosis); visceral works, visceral larva migrans (e.g., *Toxocara),* eosinophilic gastroenteritis (e.g., *Anisaki* spp., *Phocanema* ssp.), cutaneous larva migrans *(Ancylostoma braziliense, Ancylostoma caninum*)*,* or fungal infection.

The peptides of the invention may also be useful as contraceptives or to induce abortion, in acute respiratory distress syndrome, and diseases where steroids are routinely used (e.g., relapsing Beheers colitis and asthma).

Also included within the scope of the invention are indications associated with tumor necrosis factor α (TNFα), e.g., rheumatoid arthritis or endotoxemia, or indications associated with elevated levels of TNFα. These indications include, but are not limited to, endotoxic shock; Crohn's disease; fever, and flu-like symptoms; acute interstitial pneumonitis; septic and nonseptic shock; acute respiratory distress syndrome; thromboembolic conditions; bone resorption; arthritis; acute graft versus host disease; leprosy; malaria; cerebral malaria; cachexia of tuberculosis or cancer; and idiopathic fibrosis.

I. Identification of Therapeutic Agents Falling within the Scope of the Invention

Agents useful in the practice of the invention include agents that inhibit or reduce (e.g., chemokine receptor antagonists), or increase, augment or enhance (e.g., chemokine receptor agonists), chemokine-induced activity, e.g., monocyte or macrophage recruitment. These agents can be identified by *in vitro* and *in vivo* assays, such as the assays described hereinbelow. It is recognized that not all agents falling within the scope of the invention can inhibit or enhance chemokine-induced activity *in vitro* and *in vivo.* The therapeutic agents of the invention may be direct receptor binding agonists and/or antagonists, or may act by a different mechanism, e.g., duplex formation of antisense nucleic acid with chemokine mRNA, or by more than one mechanism, so as to result in the alteration of chemokine-induced activity.

A. Peptides, Variants, Derivatives and Analogs

1*. In vitro* chemotaxis

To determine whether an agent inhibits a chemokine-induced activity, such as macrophage recruitment, varying amounts of the agent are mixed with cells in the presence of a known chemoattractant. For example, a range of known concentrations of an agent, e.g., a chemokine peptide, is incubated with a defined number (e.g., $10^4$ - 106) of human THP-1 monocyte cells in individual wells of the top compartment of a trans-well plate. Chemokine (such as MCP-1, MIP1α, IL8 or SDF-1α), at a concentration known to cause significant migration of THP-1 cells in the trans-well migration assay, is placed in the lower compartment (Figure 1). Cells are then incubated at 37˚C for a period sufficient to allow migration, e.g., 4 hours. After incubation, the cells are gently removed from the top of the filter with a pipette, 20 μl of 20 mM EDTA in simple PBS is added into each top well, and incubated for 20 minutes at 4˚C. The filter is carefully flushed with media using a gentle flow, and removed. A standard curve consisting of a two-fold dilution series of THP-1 cells (in 29 μl) is prepared to accurately quantify the number of cells that have migrated. Migrated cells are stained with 3 μl of MTT stock dye solution which is added directly into each well (5 mg/ml in RPMI-1640 without phenol red, Sigma Chemical Co.) and incubated at 37°C for 4 hours. The media is carefully aspirated from each well, and the converted dye is solubilized by 20 μl of DMSO. Absorbance of converted dye is measured at a wavelength of 595 nm using an ELISA plate reader. The number of migrated cells in each well is then determined by interpolation of the standard curve (see also Imai et al.,J. Biol. Chem., 272, 15036 (1997)).

Any method suitable for counting cells can be used, for example, counting with a hemocytometer, incubation of the cells with MTT (see above), or FACS analysis. A negative control assay is also performed, using TGF-β or another non-chemokine chemoattractant (e.g., IL1β or TNFα). To assess whether the agent is cytotoxic, the same concentrations of agent are incubated with THP-1 cells. Agents which 1) are not cytotoxic at levels which inhibit migration, 2) are ineffective at inhibiting the negative control-induced migration, and 3) reduce or inhibit chemokine-induced THP-1 migration, are agents which fall within the scope of the invention.

Agents may also be screened in a chemotactic assay which employs human neutrophils, eosinophils, mast cells, basophils, platelets, lymphocytes or monocytes. For monocytes, 9 mls of fresh blood are transferred to a tube containing 1 ml of 3.8% sodium citrate, and left at room temperature for 15 minutes. Five mls of this anti-coagulated blood are carefully layered over 3.5 ml Polymorphprep® (Nycomed Pharma, Oslo), and centrifuged at 500 g for 35 minutes per the manufacturer's instructions. The top band at the sample/medium interface contains monocytes. The monocytes are carefully removed with a glass pipette, and reconstituted to the original volume (5 ml). The cells are washed with PBS plus 10% fetal calf serum, and centrifuged at 400 g for 10 minutes. The washing step is repeated three times before the cells are counted. Cells are resuspended at $1 \times 10^7$ cells/ml in RPMI-1640 + 10% fetal calf serum (FCS). The monocytes are cultured for two days at 37°C in a humidified atmosphere of 5% $CO_2$.

On day 2, the cells are counted, spun down, and reconstituted to $1 \times 10^7$ cells/ml in Gey's balanced salt solution + 1 mg/ml bovine serum albumin (BSA). Chemotaxis is induced in a 48 or 96-well disposable chemotaxis chamber fitted with a 5-8 μm polycarbonate filter for monocytes, neutrophils or eosinophils, or a 3 μm filter for lymphocytes (Uguccioni et al, Eur. J. Immunol., 25, 64 (1995); Loetscher et al., J. Exp. Med., 184, 569 (1996); Weber et al., L Immunol., 4166 (1995)) (PVP free, ChemoTX, Neuroprobe Inc., Cabin John, MD). Twenty-nine μl of chemoattractant or control are added to the lower compartment of each well. The framed filter is aligned with the holes in the corner of the filter frame and placed over the wells. Two and one-half $\times 10^5$ monocytes in 25 μl of Gey's balanced salt solution + 1 mg/ml BSA are added to the upper compartment. The agent is dissolved in MilliQ water and then serially diluted in the Gey's balanced salt solution. In most cases, the serially diluted agent is added to the upper compartment of the chemotaxis chamber. The chamber is incubated at 37°C in a humidified atmosphere of 5% $CO_2$ for 1.5 hours.

2. Enzyme release

The release of *N*-acetyl-β-D-glucosaminidase from monocytes may be employed to determine whether a therapeutic agent inhibits a cytokine-associated activity. Samples of 1.2 x $10^6$ monocytes in 0.3 ml of prewarmed medium (136 mM NaCl, 4.8 mM KCl, 1.2 mM $KH_2PO_4$, 1 mM $CaCl_2$, 20 mM Hepes, pH 7.4, 5 mM D-glucose, and 1 mg/ml fatty acid-free BSA) are pretreated for 2 minutes with cytochalasin B (2.7 mg/ml) and then stimulated with a chemokine in the presence or absence of the therapeutic agent. The reaction is stopped after 3 minutes by cooling on ice and centrifugation, and the enzyme activity is determined in the supernatant (Uguccioni et al., Eur. J. Immunol., 25, 64 (1995)).

The release of elastase from neutrophils may also be employed to determine whether a therapeutic agent inhibits a cytokine-associated activity (Pereri et al., J. Exp Med., 1547 (1988); Clark-Lewis et al., J. Biol. Chem., 269, 16075 (1994)).

3. Cytosolic free $Ca^{2+}$ concentration ($[Ca^{2+}]_i$ changes

Monocytes, eosinophils, neutrophils and lymphocytes loaded with Fura-2 (0.1 mmol/$10^5$ cells) are stimulated with a chemokine in the presence or absence of the therapeutic agent, and $[Ca^{2+}]_i$-related fluorescence changes are recorded (Von Tschanner et al., Nature, 324, 369 (1986)). For example, to determine cytosolic $Ca^{2+}$ concentrations in monocytes, monocytes are incubated with 0.5 μM Fura-2/AM for 30 minutes at 37°C in HEPES-buffered saline (145 mM NaCl, 5 mM KCl, 1 mM $MgCl_2$, 10 mM HEPES, and 10 mM glucose), pH 7.4, at 37°C, supplemented with 1% albumin (w/v) and 1 mM $CaCl_2$. After loading with Fura-2, the cells are centrifuged for 5 minutes at 300 x g and then resuspended in buffer containing no added albumin, to a cell density of 1.5 x $10^6$ cells/ml, and kept at room temperature until use. This protocol results in a cytosolic Fura-2 concentration of about 100 μM. Serial dilutions of chemokines in PBS plus 0.1 % albumin (w/v) (sterile filtered) are added to aliquots (0.7 ml) of cell suspension. The Fura-2 fluorescence of the monocyte suspension is measured at 37°C in a single excitation, single emission (500 nm) wavelength Perkin-Elmer LS5 fluorometer. $[Ca^{2+}]_i$ is calculated from changes in fluorescence measured at a single excitation wavelength of 340 nm.

$[Ca^{2+}]_i$ measurements in cells that are stably transformed with a molecularly cloned chemokine receptor which

is not expressed in the corresponding non-transformed cells are performed essentially as described above. After loading with Fura-2/AM, cells (1 × $10^6$/ml) are kept in ice-cold medium (118 mM NaCl, 4.6 mM KCl, 25 mM $NaHCO_3$, 1 mM $KH_2PO_4$, 11 mM glucose, 50 mM HEPES, 1 mM $MgCl_2$, 1 mM $CaCl_2$, 0.1% gelatin (pH 7.4). Aliquots (2 ml) of cell suspension are prewarmed at 37°C for 5 minutes in 3-ml plastic cuvettes, and fluorescence is measured in a fluorometer (Johnson Foundation Biomedical Group) with magnetic stirring and temperature controlled at 37°C. Excitation is set at 340 nm, and emission is set at 510 nm. $[Ca^{2+}]_i$ is calculated as described above. For studies in monocytes on cross-desensitization of calcium responses, chemokines are added sequentially with a 2-minute interval, and $[Ca^{2+}]_i$ transients are recorded. The concentrations used in these types of studies vary for each chemokine and are set at levels known to induce the maximal response for $[Ca^{2+}]_i$ mobilization (see Forssmann et al., FEBS Lett., 408, 211 (1997); Sozzani et al., J. Leukoc. Biol., 57, 788 (1995); Berkhout et al., J. Biol. Chem., 272, 16404 (1997)).

4. Chemokine binding and binding displacement

In general, specific binding is calculated as the amount of labeled agent bound in the absence of cold competitor minus the amount of labeled agent bound in the presence of cold competitor. The amount of specific binding in the presence of varied amounts of cold competitor can be used to determine the association constant for the agent, as well as the number of binding sites on the cell for the agent, using, for example, Scatchard Analysis. The agent may be labeled by radiolabeling (e.g., iodination) or with a suitable biochemical tag (e.g., biotin) or by addition of a photoactivatable crosslinking group. Agents with an association constant lower than 100 μM (i.e., which bind more strongly than an agent with an association constant of 100 μM) and which have at least about 2,500, preferably at least about 10,000, and more preferably greater than 25,000, binding sites per cell for at least one cell type which expresses a chemokine receptor, fall under the scope of this invention. THP-1 cells have at least about 5,000 MCP-1 receptors/cell.

For example, monocytes are suspended in RPMI 1640 medium without bicarbonate containing 0.2% bovine serum albumin and 0.1% azide. Radiolabeled chemokine peptide is incubated with 1-2 x $10^6$ cells, e.g., THP-1 cells, in the presence or absence of increasing concentrations of unlabeled chemokine (MCP-1, MCP-3, MCP-4, RANTES or MIP-1α) for 15 minutes at 37°C in a 96-well plate in a final volume of 0.2 ml (e.g., PBS + 0.5% FCS). After the incubation, 0.5 ml of ice-cold wash buffer (20 mM Tris, 0.5 M NaCl, pH 7.4) is added, and cells are collected onto a polyethyleneimine-treated Whatman GF/C filter using a Brandall cell harvester. Filters are washed with 4 ml of cold wash buffer, and the radioactivity bound to the filters is counted in a γ-counter.

For competition studies, the $IC_{50}$ is calculated with a curve fitting program (GraFit, Erithacus Software, London), using a four-parameter logistic, $cpm_{bound} = cpm_{max}/(1 + ([L]/IC_{50})^s) + cpm_{ns}$, where $cpm_{max}$ represents the binding without competitor, [L] is the competitor concentration, $cpm_{ns}$ is the non-specific binding, and s is the slope factor. The $cmp_{bound}$ is corrected for "no cell" controls. To obtain the $K_d$ and capacity of binding specific binding, data from homologous displacement experiments are fitted into a single-site ligand binding equation using the GraFit best fit program.

Chemokine binding to cells stably transformed with a molecularly cloned chemokine receptor is performed essentially as described above except that radiolabeled agent is diluted with unlabeled chemokine. Cells are incubated with radiolabeled agent plus or minus unlabeled chemokines for 30 minutes at 37°C (see also, Imai et al., *supra;* Sozzani et al. (1995), *supra;* Berkhout et al., *supra;* WO 97/22698).

5. Binding to the Duffy Antigen Receptor for Chemokines (DARC)

The affinity of the therapeutic agent to DARC may be determined by any method known in the art, e.g., the ability of the agent to inhibit the binding of radio-iodinated MCP-1 to red blood cells. Agents which bind to DARC with a lower association constant (i.e., stronger binding) than they bind to chemokine receptors (i.e., a DARC selectivity ratio of < 1), and which bind to DARC with an association constant lower than 100 μM, preferably lower than 10 μM and more preferably lower than 1 μM, are useful in particular embodiments of the methods of the invention. In contrast, agents which do not bind DARC, or do not bind to DARC with an affinity that is greater than their affinity for chemokine receptors (i.e., a selectivity ratio > 1), are useful in the practice of other embodiments of the methods of the invention.

6. Inhibition of the co-mitogenic activity of chemokines

Many chemokines are co-mitogenic with low concentrations of FCS, e.g., 50 ng/ml MCP-1 + 0.5% FCS is a mitogen for smooth muscle cells. Assays well known to the art for determination of DNA synthesis induced by any known chemokine plus a low concentration (< 5%) of FCS on suitable cells (e.g., smooth muscle cells) in the presence and absence of the agent may be employed to screen agents for such inhibitory activity. See Porreca et al., J Vasc. Res., 34, 58 (1997), the disclosure of which is incorporated by reference herein.

7. Agonists

To determine whether an agent of the invention is a chemokine receptor agonist, varying amounts of a labeled form of the agent, e.g., biotinylated, are mixed with cells that express the receptor, e.g., THP-1 cells express receptors for MCP-1, MIPIα, SDF-1α and IL-8, while Jurkat cells express functional receptors for SDF-1. The affinity of the labeled agent for the cells is then determined. Agents that bind to receptors with a reasonable affinity and interact with the receptor by inducing signaling, are within the scope of the invention. While not encompassed by the term "agonist" or "antagonist", agents that bind to or near the receptor but elicit no response are also within the scope of the invention, and are termed "neutral" agents.

Agents with agonist activity may also be identified using the transwell migration assay, where the cells are placed in the upper compartment (see Figure 1) in the absence of agent, and the agent, e.g. peptide 2[MCP-1], is placed at varying concentrations in the lower compartment in place of the chemokine. If the agent(s) have agonist activity, more cells are found in the lower compartment at the end of the assay in wells containing the agent(s) than in wells containing inactive control, i.e., agent or medium alone. Preferably, agents having agonist activity also stimulate migration of primary human cells, e.g., monocytes, in a transwell migration assay.

Moreover, weak agonists or neutral agonists (agents which bind to the receptor but do not inhibit binding of native chemokine and its subsequent signaling, nor do they induce signaling themselves) can be identified by screening the agents for ability to displace the binding of HIV gp 120, specifically the V3 loop of gp120, to the surface of THP-1 cells or Jurkat cells. Cells are incubated with labeled (for example, radioiodinated) recombinant gp 120 protein in an amount effective to bind to the virus receptor, in the presence and absence of various concentrations of the agent(s). Agents which reduce or abolish gp120 binding are agonists or neutral agonists within the scope of the invention.

8. *In vivo*

A rapid method to determine whether an agent of the invention inhibits or augments an inflammatory response is to inject a selected chemokine into the skin of an animal in the presence or absence of an agent of the invention. At some later point in time, animals are sacrificed and the number of inflammatory cells at the chemokine injection site in animals exposed to both chemokine and the test agent is compared to the number of inflammatory cells at the chemokine injection site in animals exposed to chemokine alone, e.g., by quantitative immunofluorescence, relative to control animals.

B. Nucleic acid molecules of the invention

1. Sources of the Nucleic Acid Molecules of the Invention

Sources of nucleotide sequences from which the present nucleic acid molecules encoding a chemokine peptide, a variant thereof or the nucleic acid complement thereof, include total or poly $A^+$ RNA from any eukaryotic, preferably mammalian, cellular source from which cDNAs can be derived by methods known in the art. Other sources of the DNA molecules of the invention include genomic libraries derived from any eukaryotic cellular source. Moreover, the present DNA molecules may be prepared *in vitro,* e.g., by synthesizing an oligonucleotide of about 100, preferably about 75, more preferably about 50, and even more preferably about 40, nucleotides in length, or by subcloning a portion of a DNA segment that encodes a particular chemokine.

2. Isolation of a Gene Encoding a Chemokine

A nucleic acid molecule encoding a chemokine can be identified and isolated using standard methods, as described by Sambrook et al., Molecular Cloning: A Laboratory Manual, Cold Spring Harbor, NY (1989). For example, reverse-transcriptase PCR (RT-PCR) can be employed to isolate and clone chemokine cDNAs. Oligo-dT can be employed as a primer in a reverse transcriptase reaction to prepare first-strand cDNAs from isolated RNA which contains RNA sequences of interest, e.g., total RNA isolated from human tissue. RNA can be isolated by methods known to the art, e.g., using TRIZOL™ reagent (GIBCO-BRL/Life Technologies, Gaithersburg, MD). Resultant first-strand cDNAs are then amplified in PCR reactions.

"Polymerase chain reaction" or "PCR" refers to a procedure or technique in which amounts of a preselected fragment of nucleic acid, RNA and/or DNA, are amplified as described in U.S. Patent No. 4,683,195. Generally, sequence information from the ends of the region of interest or beyond is employed to design oligonucleotide primers comprising at least 7-8 nucleotides. These primers will be identical or similar in sequence to opposite strands of the template to be amplified. PCR can be used to amplify specific RNA sequences, specific DNA sequences from total genomic DNA, and cDNA transcribed from total cellular RNA, bacteriophage or plasmid sequences, and the like. See generally Mullis et

al., Cold Spring Harbor Symp. Quant.Biol., 51, 263 (1987); Erlich, ed., PCR Technology, (Stockton Press, NY, 1989). Thus, PCR-based cloning approaches rely upon conserved sequences deduced from alignments of related gene or polypeptide sequences.

Primers are made to correspond to highly conserved regions of polypeptides or nucleotide sequences which were identified and compared to generate the primers, e.g., by a sequence comparison of other eukaryotic chemokines. One primer is prepared which is predicted to anneal to the antisense strand, and another primer prepared which is predicted to anneal to the sense strand, of a DNA molecule which encodes a chemokine.

The products of each PCR reaction are separated via an agarose gel and all consistently amplified products are gel-purified and cloned directly into a suitable vector, such as a known plasmid vector. The resultant plasmids are subjected to restriction endonuclease and dideoxy sequencing of double-stranded plasmid DNAs.

Another approach to identify, isolate and clone cDNAs which encode a chemokine is to screen a cDNA library. Screening for DNA fragments that encode all or a portion of a cDNA encoding a chemokine can be accomplished by probing the library with a probe which has sequences that are highly conserved between genes believed to be related to the chemokine, e.g., the homolog of a particular chemokine from a different species, or by screening of plaques for binding to antibodies that specifically recognize the chemokine. DNA fragments that bind to a probe having sequences which are related to the chemokine, or which are immunoreactive with antibodies to the chemokine, can be subcloned into a suitable vector and sequenced and/or used as probes to identify other cDNAs encoding all or a portion of the chemokine.

As used herein, the terms "isolated and/or purified" refer to *in vitro* isolation of a DNA or polypeptide molecule from its natural cellular environment, and from association with other components of the cell, such as nucleic acid or polypeptide, so that it can be sequenced, replicated, and/or expressed. For example, "isolated chemokine nucleic acid" is RNA or DNA containing greater than 9, preferably 36, and more preferably 45 or more, sequential nucleotide bases that encode at least a portion of a chemokine, or a variant thereof, or a RNA or DNA complementary thereto, that is complementary or hybridizes, respectively, to RNA or DNA encoding the chemokine and remains stably bound under stringent conditions, as defined by methods well known in the art, e.g., in Sambrook et al., *supra.* Thus, the RNA or DNA is "isolated" in that it is free from at least one contaminating nucleic acid with which it is normally associated in the natural source of the RNA or DNA and is preferably substantially free of any other mammalian RNA or DNA. The phrase "free from at least one contaminating source nucleic acid with which it is normally associated" includes the case where the nucleic acid is reintroduced into the source or natural cell but is in a different chromosomal location or is otherwise flanked by nucleic acid sequences not normally found in the source cell. An example of isolated chemokine nucleic acid is RNA or DNA that encodes human MCP-1 and shares at least about 80%, preferably at least about 90%, and more preferably at least about 95%, sequence identity with the MCP-1 polypeptide having SEQ ID NO:16.

As used herein, the term "recombinant nucleic acid" or "preselected nucleic acid," e.g., "recombinant DNA sequence or segment" or "preselected DNA sequence or segment" refers to a nucleic acid, e.g., to DNA, that has been derived or isolated from any appropriate tissue source, that may be subsequently chemically altered *in vitro,* so that its sequence is not naturally occurring, or corresponds to naturally occurring sequences that are not positioned as they would be positioned in a genome which has not been transformed with exogenous DNA. An example of preselected DNA "derived" from a source, would be a DNA sequence that is identified as a useful fragment within a given organism, and which is then chemically synthesized in essentially pure form. An example of such DNA "isolated" from a source would be a useful DNA sequence that is excised or removed from said source by chemical means, e.g., by the use of restriction endonucleases, so that it can be further manipulated, e.g., amplified, for use in the invention, by the methodology of genetic engineering.

Thus, recovery or isolation of a given fragment of DNA from a restriction digest can employ separation of the digest on polyacrylamide or agarose gel by electrophoresis, identification of the fragment of interest by comparison of its mobility versus that of marker DNA fragments of known molecular weight, removal of the gel section containing the desired fragment, and separation of the gel from DNA. See Lawn et al., Nucleic Acids Res., 9, 6103 (1981), and Goeddel et al., Nucleic Acids Res., 8, 4057 (1980). Therefore, "preselected DNA" includes completely synthetic DNA sequences, semi-synthetic DNA sequences, DNA sequences isolated from biological sources, and DNA sequences derived from RNA, as well as mixtures thereof.

As used herein, the term "derived" with respect to a RNA molecule means that the RNA molecule has complementary sequence identity to a particular DNA molecule.

3. Variants of the Nucleic Acid Molecules of the Invention

Nucleic acid molecules encoding amino acid sequence variants of a chemokine peptide are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant

version of the chemokine peptide.

Oligonucleotide-mediated mutagenesis is a preferred method for preparing amino acid substitution variants of a chemokine peptide. This technique is well known in the art as described by Adelman et al., DNA, 2,183 (1983). Briefly, chemokine DNA is altered by hybridizing an oligonucleotide encoding the desired mutation to a DNA template, where the template is the single-stranded form of a plasmid or bacteriophage containing the unaltered or native DNA sequence of the chemokine. After hybridization, a DNA polymerase is used to synthesize an entire second complementary strand of the template that will thus incorporate the oligonucleotide primer, and will code for the selected alteration in the chemokine DNA.

Generally, oligonucleotides of at least 25 nucleotides in length are used. An optimal oligonucleotide will have 12 to 15 nucleotides that are completely complementary to the template on either side of the nucleotide(s) coding for the mutation. This ensures that the oligonucleotide will hybridize properly to the single-stranded DNA template molecule. The oligonucleotides are readily synthesized using techniques known in the art such as that described by Crea et al., Proc. Natl. Acad Sci U.S.A., 75, 5765 (1978).

The DNA template can be generated by those vectors that are either derived from bacteriophage M13 vectors (the commercially available M13mp18 and M13mp19 vectors are suitable), or those vectors that contain a single-stranded phage origin of replication as described by Viera et al., Meth. Enzymol., 153, 3 (1987). Thus, the DNA that is to be mutated may be inserted into one of these vectors to generate single-stranded template. Production of the single-stranded template is described in Sections 4.21-4.41 of Sambrook et al., Molecular Cloning: A Laboratory Manual (Cold Spring Harbor Laboratory Press, N.Y. 1989).

Alternatively, single-stranded DNA template may be generated by denaturing double-stranded plasmid (or other) DNA using standard techniques.

For alteration of the native DNA sequence (to generate amino acid sequence variants, for example), the oligonucleotide is hybridized to the single-stranded template under suitable hybridization conditions. A DNA polymerizing enzyme, usually the Klenow fragment of DNA polymerase I, is then added to synthesize the complementary strand of the template using the oligonucleotide as a primer for synthesis. A heteroduplex molecule is thus formed such that one strand of DNA encodes the mutated form of the chemokine, and the other strand (the original template) encodes the native, unaltered sequence of the chemokine. This heteroduplex molecule is then transformed into a suitable host cell, usually a prokaryote such as *E. coli* JM101. After the cells are grown, they are plated onto agarose plates and screened using the oligonucleotide primer radiolabeled with 32-phosphate to identify the bacterial colonies that contain the mutated DNA. The mutated region is then removed and placed in an appropriate vector for peptide or polypeptide production, generally an expression vector of the type typically employed for transformation of an appropriate host.

The method described immediately above may be modified such that a homoduplex molecule is created wherein both strands of the plasmid contain the mutations(s). The modifications are as follows: The single-stranded oligonucleotide is annealed to the single-stranded template as described above. A mixture of three deoxyribonucleotides, deoxyriboadenosine (dATP), deoxyriboguanosine (dGTP), and deoxyribothymidine (dTTP), is combined with a modified thiodeoxyribocytosine called dCTP-($\alpha$S) (which can be obtained from the Amersham Corporation). This mixture is added to the template-oligonucleotide complex. Upon addition of DNA polymerase to this mixture, a strand of DNA identical to the template except for the mutated bases is generated. In addition, this new strand of DNA will contain dCTP-($\alpha$S) instead of dCTP, which serves to protect it from restriction endonuclease digestion.

After the template strand of the double-stranded heteroduplex is nicked with an appropriate restriction enzyme, the template strand can be digested with ExoIII nuclease or another appropriate nuclease past the region that contains the site(s) to be mutagenized. The reaction is then stopped to leave a molecule that is only partially single-stranded. A complete double-stranded DNA homoduplex is then formed using DNA polymerase in the presence of all four deoxyribonucleotide triphosphates, ATP, and DNA ligase. This homoduplex molecule can then be transformed into a suitable host cell such as E. *coli* JM101.

For example, a preferred embodiment of the invention is an isolated and purified DNA molecule comprising a preselected DNA segment encoding peptide 3 (1-12)[MCP-1] having SEQ ID NO:1, wherein the DNA segment comprises SEQ ID NO:76, or variants of SEQ ID NO:76, having nucleotide substitutions which are "silent" (see Figure 8). That is, when silent nucleotide substitutions are present in a codon, the same amino acid is encoded by the codon with the nucleotide substitution as is encoded by the codon without the substitution. For example, valine is encoded by the codon GTT, GTC, GTA and GTG. A variant of SEQ ID NO:79 at the tenth codon in the mature polypeptide (GTC in SEQ ID NO:79) includes the substitution of GTT, GTA or GTG for GTC. Other "silent" nucleotide substitutions in SEQ ID NO: 76 which can encode peptide 3 (1-12)[MCP-1] having SEQ ID NO:1 can be ascertained by reference to Figure 8 and page D1 in Appendix D in Sambrook et al., Molecular Cloning: A Laboratory Manual (1989). Nucleotide substitutions can be introduced into DNA segments by methods well known to the art. See, for example, Sambrook et al., *supra.* Likewise, nucleic acid molecules encoding other mammalian, preferably human, chemokines may be modified in a similar manner. Thus, nucleic acid molecules encoding at least a portion of, for example, MCP-2 (SEQ ID NO:80), MCP-3 (SEQ ID NO:81), MCP-4 (SEQ ID NO:100), MIP1$\alpha$ (SEQ ID NO:82), MIP1$\beta$ (SEQ ID NO:83), RANTES (SEQ ID NO:

84), SDF1α (SEQ ID NO:85), IL8 (SEQ ID NO:86), GROα (SEQ ID NO:87), eotaxin (SEQ ID NO:88), MIG (SEQ ID NO: 89), PF-4 (SEQ ID NO:90), I309 (SEQ ID N0:91), HCC-1 (SEQ ID NO:92), C10 (SEQ ID NO:93), CCR-2 (SEQ ID NO: 94), ENA-78 (SEQ ID NO:95), GROβ (SEQ ID NO:96), IP10 (SEQ ID NO:97), SDF1β (SEQ ID NO:98), GROα (SEQ ID NO:99), MIP3α, TCA-3, CTAPIII, MARC/FYK, β-thromboglobulin, GCP-2, PBP, HC14, MDC, TECK, PARC, 6Ckine, fracktakine, DC-CK1, LIX, TARC, LARC, MIG, Ckβ8, CCF18/MRP-2, CCIII, CKα2, H1305, Dvic-1, DGWCC, TCA4, dendrokine, CC2/HCC1, CC3, and MIP1τ, as well as virally encoded chemokines such as vMIP-I, vMIP-II and vMIP-III, or the complement thereto, may be modified so as to yield nucleic acid molecules of the invention having silent nucleotide substitutions, or to yield nucleic acid molecules having nucleotide substitutions that result in amino acid substitutions (see peptide variants hereinbelow).

C. *In vivo* studies

To further determine whether a particular agent is useful in the practice of the methods of the invention, an animal model is identified for a human disease. Transgenic animal models for human disease may also be employed to identify agents useful in the methods of the invention. For example, models of chemokine-induced macrophage recruitment associated with human atherosclerosis include, but are not limited to, mice with a homozygous deletion of the apolipoprotein E (apoE) gene, mice overexpressing human apoB and Watanabe heritable hyperlipidemic rabbits. Models for autoimmune disease include the collagen-induced arthritis in DBA/1 mice and myelin basic protein-induced experimental autoimmune encephalomyelitis. Models for osteoporosis include ovariectomized female rats, mice, monkeys, rats treated with heparin or with glucocorticoids as well as suspension-induced osteoporosis in rats. Models for HIV infection include infection of monkeys with SIV, SIV isolates, HIV or HIV isolates, SCID-Hu mice with HIV or HIV isolates, or rabbits with HIV or HIV isolates. Other animal models for lentiviral infection include cats infected with FIV, horses with EIAV, and goats infected with CAEV (which is also an animal model for arthritis).

The efficacy of an agent of the invention for anti-inflammatory therapy may be assessed by measuring the extent of inflammation, or the extent of macrophage infiltration of affected tissues. Macrophage infiltration can be detected by staining tissue sections with antibodies which specifically detect macrophages (e.g., mac-1 antiserum). Inflammation or other symptoms of disease may be detected by measuring appropriate clinical parameters, using techniques which are well known to those skilled in the art. For example, apoE knockout mice are treated with an agent, such as CRD-$leu_4ile_{11}$peptide 3, e.g., by intraperitoneal injection, for a period of twelve weeks, while control litter mates receive a suitable control peptide with no known biological activity. At the end of twelve weeks, the animals are sacrificed and the effect of the agent is assessed by measuring the reduction in macrophage recruitment into the vessel wall by quantitative immunohistochemistry using mac-1 antiserum, and by measuring the reduction in the extent of vascular lipid lesion formation by histochemistry using oil red O staining in accordance with Paigen, Arteriosclerosis, 10, 316 (1990).

Apo(a) transgenic mice develop lesions when fed a lipid-rich diet. These lesions do not contain any macrophages. In contrast, C57B16 inbred mice develop lipid lesions of similar size and severity to those in apo(a) transgenic mice, but these lesions are rich in infiltrating macrophage. Lesions of apo(a) mice, C57B16 mice, and 6 other strains of mice which develop lipid lesions rich with macrophage, were screened by quantitative immunofluorescence for levels of pro-inflammatory mediators, e.g., TNF-α, MCP-1, MIP-1α, IL1β, ICAM-1, VCAM-1, and P-selectin. TNF-α, MIP-1α, IL1β, ICAM-1, VCAM-1 and P-selectin were all expressed at identical levels in the apo(a) mouse lesions and the C57B16 lesions. Thus, while these pro-inflammatory mediators may be necessary to infiltration, they are not sufficient alone. In marked contrast, MCP-1 was completely absent from the lesions of apo(a) mice, but expressed at high levels in lesions from all other mouse lines which had macrophage-rich lesions.

Confocal microscopic analysis of sections of blood vessel wall with lesions triple stained with antibodies specific for SM-α-actin (smooth muscle cells; IA4 antibody), macrophages (Mac-1 antibodies) and MCP-1, showed that MCP-1 is not exclusively expressed by macrophage. That is, both smooth muscle cells and macrophages expressed MCP-1. Thus, MCP-1 may be the missing "inflammatory mediator" in the apo(a) mouse model of atherosclerosis. These results suggest that the lack of MCP-1 in apo(a) mice lesions may not be a consequence of the absence of macrophages, but instead contribute to the cause of lack of monocyte infiltration. Moreover, these results provide evidence that the chemokine MCP-1 plays a role in atherosclerotic vascular inflammation. Thus, MCP-1 can provide the basis for analogs which block the recruitment activity of this chemokine.

Chemokines other than MCP-1 may also be involved in macrophage recruitment, inflammation and pathogenesis of atherosclerosis, and in other diseases associated with inappropriate proliferation. For example, MIP1α has been implicated in the inappropriate inflammation in multiple sclerosis. Thus, sequences analogous to peptide 2 and 3 from MIP1α may be particularly useful to treat or prevent multiple sclerosis. Therefore, when a particular chemokine is implicated in a particular disease, sequences from that particular chemokine may be especially useful to treat or prevent that disease. Preferred agents falling within the scope of the invention are inhibitors of signaling of more than one chemokine, and preferably of all chemokines. Thus, it may be preferable to prepare chemokine peptide analogs having sequences from a chemokine other than the one(s) associated with a particular disease process. Selection of a particular

agent to treat a particular disease may be based on bioavailability, toxicity, DARC binding or other similar criteria.

Other models include, but are not limited to those reported by Lukacs (Adv. Immunol., pp. 257-304, Academic Press (1996)), for lung injury; Lloyd et al. J. Leuko. Biol., 185, 1371 (1997)) and Tam et al. (Kid. Int., 49, 715 (1996)), for nephritis; Volejnikova (Am. J.Pathol., 150, 1711 (1997), for bone; Ghinikar et al. (J. Neurosci Res., 46, 727 (1996)) and Ransoholf et al. (J. Leuko Biol., 62, 645 (1997)), for brain; Kaul et al. (Am. J. Trop. Med. Hyg., 58, 240 (1995)), for malaria; Ajeubar et al. (J. Leuko. Biol., 63, 108 (1998)), for peritonitis; Furukawa et al. (Lupus, 6, 193 (1997)), for systemic lupus; Suzuki et al. (J. Heart & Lung Transpl., 16, 1141 (1967)), Abbott et al. (Arch Surg., 89, 645 (1964)), Corry et al. (Transpl., 16,343 (1973)), Dworkin et al. (J. Heart Lung Transpl., 10, 591 (1991)), Laden et al. (Arch. Path., 93, 240 (1972)) and Mitchell et al. (Transpl., 49, 835 (1990)), for transplants; U.S. Patent No. 5,661,132 for wound healing; Burhardt et al. (Rheum. Int., 17, 91 (1997)) for autoimmunity; Elson et al. (Gastroenter., 109, 1344 (1998)) for inflammatory bowel disease; Hayes et al. (Arterio. Thromb. Vasc. Biol.,18, 397 (1998)) and Wang et al. (Arterio. Thromb., 11, 1166 (1991)), for cardiovascular disease; Wegner et al. (Science, 247, 456 (1990) for eosinophilic infiltration into the lung; Brahn (Ciinorth and Rel. Res., 265, 42 (1991)), Wooley (Curr.Op. Rheum., 3, 407 (1991)) and Gay et al. (Curr. Op. Rheum., 7, 199 (1995), SCID-human synovial implant model)) for rheumatoid arthritis); Beamer et al. (Blood, 86, 3220 (1998)), Nakaguma (Int. 1. Exp. Path., 76, 65 (1998)), Nanney et al. (J. Invest. Dermat., 106, 1169 (1996)), Nickoff et al. (AJP, 146, 580 (1995)), Sundberg et al. (Pathobiol., 65, 271 (1997)), and Wolf et al. (Int J. Dermat., 30, 448 (1998)) for psoriasis; and Conti et al. (Blood, 89, 4120 (1997)), Gonzalo et al. (JCI, 98, 2332 (1996)), Teiyeira et al. (JCI, 100, 1657 (1997)), Ceri et al. (Allergy, 52, 739 (1997)), Freed (Eur. Res. J., 8, 1770 (1998)), Griffiths-Johnson et al. (Meth. Enzy., 288, 241 (1991)), Herz et al. (New Horizons in Allergy Immunoth., 25-32 Plenum Press, 1996) and Kane (Eur. Resp. J., 7, 555 (1991)) for allergy. II. Preparation of Agents Falling Within the Scope of the Invention

A. Nucleic acid molecules

1. Chimeric Expression Cassettes

To prepare expression cassettes for transformation herein, the recombinant or preselected DNA sequence or segment may be circular or linear, double-stranded or single-stranded. A preselected DNA sequence which encodes an RNA sequence that is substantially complementary to a mRNA sequence encoding a chemokine is typically a "sense" DNA sequence cloned into a cassette in the opposite orientation (i.e., 3' to 5' rather than 5' to 3'). Generally, the preselected DNA sequence or segment is in the form of chimeric DNA, such as plasmid DNA, that can also contain coding regions flanked by control sequences which promote the expression of the preselected DNA present in the resultant cell line.

As used herein, "chimeric" means that a vector comprises DNA from at least two different species, or comprises DNA from the same species, which is linked or associated in a manner which does not occur in the "native" or wild type of the species.

Aside from preselected DNA sequences that serve as transcription units for a chemokine, or portions thereof, a portion of the preselected DNA may be untranscribed, serving a regulatory or a structural function. For example, the preselected DNA may itself comprise a promoter that is active in mammalian cells, or may utilize a promoter already present in the genome that is the transformation target. Such promoters include the CMV promoter, as well as the SV40 late promoter and retroviral LTRs (long terminal repeat elements), although many other promoter elements well known to the art may be employed in the practice of the invention.

Other elements functional in the host cells, such as introns, enhancers, polyadenylation sequences and the like, may also be a part of the preselected DNA. Such elements may or may not be necessary for the function of the DNA, but may provide improved expression of the DNA by affecting transcription, stability of the mRNA, or the like. Such elements may be included in the DNA as desired to obtain the optimal performance of the transforming DNA in the cell.

"Control sequences" is defined to mean DNA sequences necessary for the expression of an operably linked coding sequence in a particular host organism. The control sequences that are suitable for prokaryotic cells, for example, include a promoter, and optionally an operator sequence, and a ribosome binding site. Eukaryotic cells are known to utilize promoters, polyadenylation signals, and enhancers.

"Operably linked" is defined to mean that the nucleic acids are placed in a functional relationship with another nucleic acid sequence. For example, DNA for a pre-sequence or secretory leader is operably linked to DNA for a peptide or polypeptide if it is expressed as a preprotein that participates in the secretion of the peptide or polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accord with conventional practice.

The preselected DNA to be introduced into the cells further will generally contain either a selectable marker gene or a reporter gene or both to facilitate identification and selection of transformed cells from the population of cells sought to be transformed. Alternatively, the selectable marker may be carried on a separate piece of DNA and used in a co-transformation procedure. Both selectable markers and reporter genes may be flanked with appropriate regulatory sequences to enable expression in the host cells. Useful selectable markers are well known in the art and include, for example, antibiotic and herbicide-resistance genes, such as *neo, hpt, dhfr, bar, aroA, dapA* and the like. See also, the genes listed on Table 1 of Lundquist et al. (U.S. Patent No. 5,848,956).

Reporter genes are used for identifying potentially transformed cells and for evaluating the functionality of regulatory sequences. Reporter genes which encode for easily assayable proteins are well known in the art. In general, a reporter gene is a gene which is not present in or expressed by the recipient organism or tissue and which encodes a protein whose expression is manifested by some easily detectable property, e.g., enzymatic activity. Preferred genes include the chloramphenicol acetyl transferase gene (cat) from Tn9 of *E. coli,* the beta-glucuronidase gene (gus) of the *uidA* locus of *E. coli,* and the luciferase gene from firefly *Photinus pyralis.* Expression of the reporter gene is assayed at a suitable time after the DNA has been introduced into the recipient cells.

The general methods for constructing recombinant DNA which can transform target cells are well known to those skilled in the art, and the same compositions and methods of construction may be utilized to produce the DNA useful herein. For example, J. Sambrook et al., Molecular Cloning- A Laboratory Manual, Cold Spring Harbor Laboratory Press (2d ed., 1989), provides suitable methods of construction.

2 Transformation into Host Cells

The recombinant DNA can be readily introduced into the host cells, e.g., mammalian, bacterial, yeast or insect cells by transfection with an expression vector comprising DNA encoding a chemokine or its complement, by any procedure useful for the introduction into a particular cell, e.g., physical or biological methods, to yield a transformed cell having the recombinant DNA stably integrated into its genome, so that the DNA molecules, sequences, or segments, of the present invention are expressed by the host cell.

Physical methods to introduce a preselected DNA into a host cell include calcium phosphate precipitation, lipofection, particle bombardment, microinjection, electroporation, and the like. Biological methods to introduce the DNA of interest into a host cell include the use of DNA and RNA viral vectors. The main advantage of physical methods is that they are not associated with pathological or oncogenic processes of viruses. However, they are less precise, often resulting in multiple copy insertions, random integration, disruption of foreign and endogenous gene sequences, and unpredictable expression. For mammalian gene therapy, it is desirable to use an efficient means of precisely inserting a single copy gene into the host genome. Viral vectors, and especially retroviral vectors, have become the most widely used method for inserting genes into mammalian, e.g., human cells. Other viral vectors can be derived from poxviruses, herpes simplex virus I, adenoviruses and adeno-associated viruses, and the like.

As used herein, the term "cell line" or "host cell" is intended to refer to well-characterized homogenous, biologically pure populations of cells. These cells may be eukaryotic cells that are neoplastic or which have been "immortalized" *in vitro* by methods known in the art, as well as primary cells, or prokaryotic cells. The cell line or host cell is preferably of mammalian origin, but cell lines or host cells of non-mammalian origin may be employed, including plant, insect, yeast, fungal or bacterial sources. Generally, the preselected DNA sequence is related to a DNA sequence which is resident in the genome of the host cell but is not expressed, or not highly expressed, or, alternatively, over expressed.

"Transfected" or "transformed" is used herein to include any host cell or cell line, the genome of which has been altered or augmented by the presence of at least one preselected DNA sequence, which DNA is also referred to in the art of genetic engineering as "heterologous DNA," "recombinant DNA," "exogenous DNA," "genetically engineered," "non-native," or "foreign DNA," wherein said DNA was isolated and introduced into the genome of the host cell or cell line by the process of genetic engineering. The host cells of the present invention are typically produced by transfection with a DNA sequence in a plasmid expression vector, a viral expression vector, or as an isolated linear DNA sequence. Preferably, the Transfected DNA is a chromosomally integrated recombinant DNA sequence, which comprises a gene encoding the chemokine or its complement, which host cell may or may not express significant levels of autologous or "native" chemokine.

To confirm the presence of the preselected DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular chemokine, e.g., by immunological means (ELISAs and Western blots) or by assays described hereinabove to identify agents falling within the scope of the invention.

To detect and quantitate RNA produced from introduced preselected DNA segments, RT-PCR may be employed. In this application of PCR, it is first necessary to reverse transcribe RNA into DNA, using enzymes such as reverse transcriptase, and then through the use of conventional PCR techniques amplify the DNA. In most instances

PCR techniques, while useful, will not demonstrate integrity of the RNA product. Further information about the nature of the RNA product may be obtained by Northern blotting. This technique demonstrates the presence of an RNA species and gives information about the integrity of that RNA. The presence or absence of an RNA species can also be determined using dot or slot blot Northern hybridizations. These techniques are modifications of Northern blotting and only demonstrate the presence or absence of an RNA species.

While Southern blotting and PCR may be used to detect the preselected DNA segment in question, they do not provide information as to whether the preselected DNA segment is being expressed. Expression may be evaluated by specifically identifying the peptide products of the introduced preselected DNA sequences or evaluating the phenotypic changes brought about by the expression of the introduced preselected DNA segment in the host cell.

B. Peptides, peptide variants, and derivatives thereof

The present isolated, purified chemokine peptides, peptide variants or derivatives thereof, can be synthesized *in vitro,* e.g., by the solid phase peptide synthetic method or by recombinant DNA approaches (see above). The solid phase peptide synthetic method is an established and widely used method, which is described in the following references: Stewart et al., Solid Phase Peptide Synthesis, W. H. Freeman Co., San Francisco (1969); Merrifield, J. Am. Chem. Soc., 85 2149 (1963); Meienhofer in "Hormonal Proteins and Peptides," ed.; C.H. Li, Vol. 2 (Academic Press, 1973), pp. 48-267; Bavaay and Merrifield, "The Peptides," eds. E. Gross and F. Meienhofer, Vol. 2 (Academic Press, 1980) pp. 3-285; and Clark-Lewis et al., Meth Enzymol., 231, 233 (1997). These peptides can be further purified by fractionation on immunoaffinity or ionexchange columns; ethanol precipitation; reverse phase HPLC; chromatography on silica or on an anion-exchange resin such as DEAE; chromatofocusing; SDS-PAGE; ammonium sulfate precipitation; gel filtration using, for example, Sephadex G-75; or ligand affinity chromatography.

Once isolated and characterized, derivatives, e.g., chemically derived derivatives, of a given chemokine peptide can be readily prepared. For example, amides of the chemokine peptide or chemokine peptide variants of the present invention may also be prepared by techniques well known in the art for converting a carboxylic acid group or precursor, to an amide. A preferred method for amide formation at the C-terminal carboxyl group is to cleave the peptide from a solid support with an appropriate amine, or to cleave in the presence of an alcohol, yielding an ester, followed by aminolysis with the desired amine.

Salts of carboxyl groups of a peptide or peptide variant of the invention may be prepared in the usual manner by contacting the peptide with one or more equivalents of a desired base such as, for example, a metallic hydroxide base, e.g., sodium hydroxide; a metal carbonate or bicarbonate base such as, for example, sodium carbonate or sodium bicarbonate; or an amine base such as, for example, triethylamine, triethanolamine, and the like.

N-acyl derivatives of an amino group of the chemokine peptide or peptide variants may be prepared by utilizing an N-acyl protected amino acid for the final condensation, or by acylating a protected or unprotected peptide. O-acyl derivatives may be prepared, for example, by acylation of a free hydroxy peptide or peptide resin. Either acylation may be carried out using standard acylating reagents such as acyl halides, anhydrides, acyl imidazoles, and the like. Both N- and O-acylation may be carried out together, if desired.

Formyl-methionine, pyroglutamine and trimethyl-alanine may be substituted at the N-terminal residue of the peptide or peptide variant. Other amino-terminal modifications include aminooxypentane modifications (see Simmons et al., Science, 276, 276 (1997)).

In addition, the amino acid sequence of a chemokine peptide can be modified so as to result in a chemokine peptide variant. The modification includes the substitution of at least one amino acid residue in the peptide for another amino acid residue, including substitutions which utilize the D rather than L form, as well as other well known amino acid analogs, e.g., unnatural amino acids such as $\alpha$, $\alpha$-disubstituted amino acids, N-alkyl amino acids, lactic acid, and the like. These analogs include phosphoserine, phosphothreonine, phosphotyrosine, hydroxyproline, gamma-carboxyglutamate; hippuric acid, octahydroindole-2-carboxylic acid, statine, 1,2,3,4,-tetrahydroisoquinoline-3-carboxylic acid, penicillamine, ornithine, citruline, $\alpha$-methyl-alanine, para-benzoyl-phenylalanine, phenylglycine, propargylglycine, sarcosine, $\varepsilon$-N,N,N-trimethyllysine, $\in$-N-acetyllysine, N-acetylserine, N-formylmethionine, 3-methylhistidine, 5-hydroxylysine, $\omega$-N-methylarginine, and other similar amino acids and imino acids and tert-butylglycine.

One or more of the residues of the peptide can be altered, so long as the peptide variant is biologically active. For example, for peptide 3[MCP-1] variants, e.g., $Ser_7$peptide 3(1-12)[MCP-1], it is preferred that the variant has at least about 10% of the biological activity of the corresponding non-variant peptide, e.g., a peptide having SEQ ID NO: 1. Conservative amino acid substitutions are preferred--that is, for example, aspartic-glutamic as acidic amino acids; lysine/arginine/histidine as basic amino acids; leucine/isoleucine, methionine/valine, alanine/valine as hydrophobic amino acids; serine/glycine/alanine/threonine as hydrophilic amino acids. Conservative amino acid substitution also includes groupings based on side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having

aromatic side chains is phenylalanine, tyrosine, and tryptophan; a group of amino acids having basic side chains is lysine, arginine, and histidine; and a group of amino acids having sulfur-containing side chains is cysteine and methionine. For example, it is reasonable to expect that replacement of a leucine with an isoleucine or valine, an aspartate with a glutamate, a threonine with a serine, or a similar replacement of an amino acid with a structurally related amino acid will not have a major effect on the properties of the resulting variant polypeptide. Whether an amino acid change results in a functional peptide can readily be determined by assaying the specific activity of the peptide variant. Assays are described in detail herein.

Conservative substitutions are shown in Figure 9 under the heading of exemplary substitutions. More preferred substitutions are under the heading of preferred substitutions. After the substitutions are introduced, the variants are screened for biological activity.

Amino acid substitutions falling within the scope of the invention, are, in general, accomplished by selecting substitutions that do not differ significantly in their effect on maintaining (a) the structure of the peptide backbone in the area of the substitution, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic; trp, tyr, phe.

The invention also envisions peptide variants with non-conservative substitutions. Non-conservative substitutions entail exchanging a member of one of the classes described above for another.

Acid addition salts of the peptide or variant peptide or of amino residues of the peptide or variant peptide may be prepared by contacting the peptide or amine with one or more equivalents of the desired inorganic or organic acid, such as, for example, hydrochloric acid. Esters of carboxyl groups of the peptides may also be prepared by any of the usual methods known in the art.

Moreover, it is also envisioned that the agents of the invention, e.g., chemokine peptides, are modified in a manner that increases their stability *in vivo*, e.g., their half-life or bioavailability. These modified agents are termed "derivatives." Methods to prepare such derivatives are well known to the art. One method to stabilize peptides is to prepare derivatives which are cyclized peptides (see EPA 471,453 (amide bonds), such as that between lysine and aspartic acid side chains; EPA 467,701 (disulfide bonds); EPA 467,699 (thioether bonds). Other modifications which may increase *in vivo* stability are disclosed in Jameson et al. (Nature, 368, 744 (1994)); U.S. Patent No. 4,992,463; U.S. Patent No. 5,596,078 and U.S. Patent No. 5,091,396. A preferred embodiment of the invention is a chemokine peptide or variant that has been cyclized by addition of one or more cysteine residues to the N and/or C terminus of the peptide, as well as peptides which are constructed of the reverse sequence (i.e., reading C-terminal to N-terminal) of D-form amino acids. A more preferred embodiment of this invention is a peptide which is both cyclized and constructed with the reverse sequence of D-form amino acids, i.e., a CRD derivative.

It is also envisioned that the invention includes antibodies specific for the therapeutic agents of the invention. For example, rabbits were immunized with CRD-$Leu_4Ile_{11}Cys_{13}$ peptide 3(3-12)[MCP-1]. The resulting antisera had a high titer but did not cross react with MCP-1. The antibodies may be useful in an immunoassay to detect CRD-$Leu_4Ile_{11}Cys_{13}$ peptide 3(3-12)[MCP-1].

C. Chemokine analogs

Chemokine analogs have properties analogous to those of the corresponding peptide. These analogs can be referred to as "peptide mimetics" or "peptidomimetics" (Fauchere, J. (1986) Adv. Dru Res., 15:29; Veber and Freidinger (1985) TINS p. 392; and Evans et al. (1987) J. Med. Chem, 30:1229, which are incorporated herein by reference) and can be developed with the aid of computerized molecular modeling. These analogs include structures having one or more peptide linkages optionally replaced by a linkage selected from the group consisting of: $-CH_2NH-$, $-CH_2S-$, $-CH_2-CH_2-$ -CH=CH-(cis and trans), -CH=CF-(trans), $-CoCH_2-$, $-CH(OH)CH_2$ and $-CH_2SO-$, by methods known in the art and further described in the following references: Spatola, A. F. in "Chemistry and Biochemistry of Amino Acids, Peptides, and Proteins," B. Weinstein, eds. Marcel Dekker, New York, P. 267 (1983); Spatola, A. F., Vega Data (March 1983), Vol. 1, Issue 3, "Peptide Backbone Modifications" (general review); Morley, J. S., Trends Pharm. Sci. (1980) pp. 463-468 (general review); Hudson, D. et al., Int. J. Pept. Prot. Res. (1979) 14:177-185 ($-CH_2NH-$, $CH_2CH_2-$); Spatola, A. F. et al., Life Sci (1986) 38:1243-1249 ($-CH_2-S$); Hann, M. M., J. Chem. Soc. Perkin Trans I (1982) 307-314 (-CH-CH-, cis and trans); Almquist, R. G. et al., J. Med. Chem. (1980) 23:1392-1398 ($-COCH_2-$); Jennings-White, C. et al.,

Tetrahedron Lett. (1982) 23:2533 (-$COCH_2$-); Szelke, M. et al. European Appln. EP 45665 (1982) CA; 97:39405 (1982) (-$CH(OH)CH_2$-); Holladay, M. W. et al., Tetrahedron Lett. (1983) 24:4401-4404 (-$C(OH)CH_2$-); and Hruby, V. J., Life Sci. (1982) 31:189-199 (-$CH_2S$-); each of which is incorporated herein by reference. A particularly preferred non-peptide linkage is -$CH_2NH$-. Such analogs may have greater chemical stability, enhanced pharmacological properties (half-life, absorption, potency, efficacy, etc.), altered specificity (e.g., a broad-spectrum of biological activities), reduced antigenicity, and be economically prepared. Labeling of analogs usually involves covalent attachment of one or more labels, directly or through a spacer (e.g., an amide group), to non-interfering positions(s) on the analog that are predicted by quantitative structure-activity data and/or molecular modeling. Such non-interfering positions generally are positions that do not form direct contacts with the macromolecule(s) to which the analog binds to produce the therapeutic effect. Systematic substitution of one or more amino acids of a consensus sequence with a D-amino acid of the same type (e.g., D-lysine in place of L-lysine) may also be used to generate more stable peptides.

1. Isosteres of chemokine tripeptides (a compound of formula (IV))

A compound of formula (IV), wherein Z=$CH_3$; $R^1$=indolyl; Y=O; and X=$CH_3$. can be prepared from N-tBOC-NinBOC-L-tryptophan-OH and cyclohexenone. For example, 2-cyclohexen-1-one (Aldrich C10,281-4) can be reacted with lithium dimethylcuprate in the presence of trimethylsilyl chloride prior to use in the reaction, by methods well known to those skilled in the art (e.g., House et al., J. Org. Chem., 40,1460 (1975)). The addition of $\alpha$-$\beta$ unsaturated ketones by organocuprates is described, for example, in House et al., Org.Chem., 31, 3128 (1966). Similarly, capture of the enolate by trimethyl silyl chloride is described in House et al., J. Org. Chem., 36, 2361 (1971). The trapped enolate is then resolved to the $\alpha$-iododerivative, for example, by addition of molecular iodine in the presence of acetoxy-silver and tetrabutylammonium fluoride, according to the method of Rubottom (J. Org. Chem., 44, 1731 (1979)) to give the trans-disubstituted cyclohexanone of formula 100.

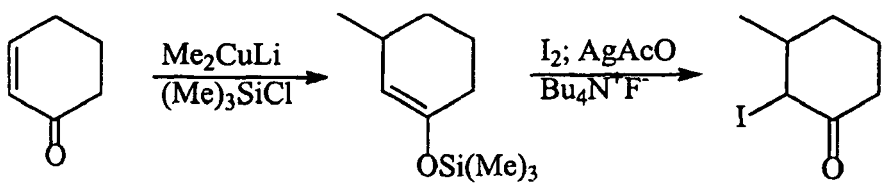


**100**

Conversion of the iodide of formula **100**, to a secondary alcohol, and formation of an ester, for example, with acetic anhydride yields a compound of formula **101**.

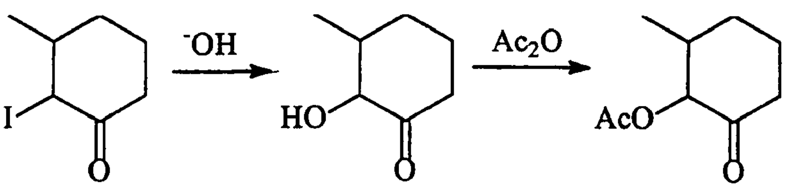


**101**

A compound of formula **101** can alternatively be prepared by conversion of the above trimethylsilyl ether enolate to the $\alpha$-hydroxy ketone followed by formation of the ester, using procedures which are well known in the art.

A compound of formula **101** can be alkylated, for example, with vinyl magnesium bromide under standard conditions, and dehydrated (for example, in the presence of molecular iodine and heat) to yield a diene of formula **102**:

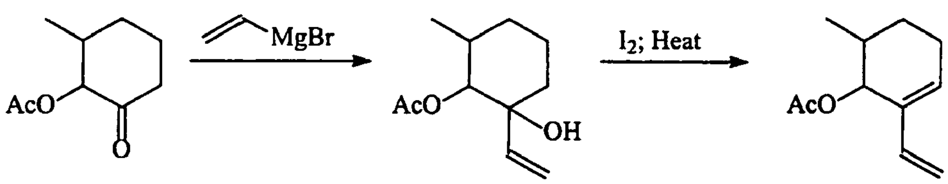


**102**

Diels-Alder reaction between the diene of formula **102** and ethyl acrylate (Aldrich E970-6) gives a stereospecific and regiospecific product of formula **103**.

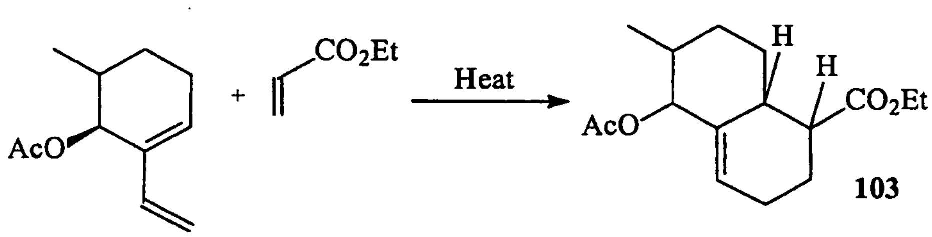


**103**

For example, the cyclization reaction can be performed by mixing the compound of formula **102** and ethyl acrylate in a sealed tube and heating, essentially as described by Green et al. (Adv. Pest control Res., 3, 129 (1960)).

Oxidative cleavage of the double bond in a compound of formula **103** gives a diacid of formula **104.**

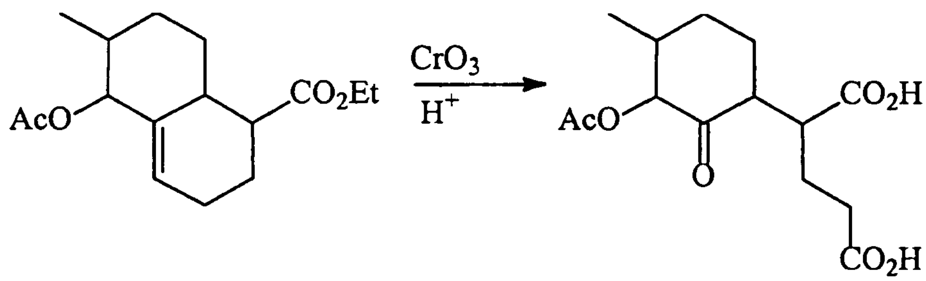


**104**

Such an oxidative cleavage may conveniently be carried out by ozonolysis or by oxidation with an acid chromate. For example, using $CrO_3$ in acid, the compound of formula **104** may be prepared, essentially as described by Eschenmoser & Winter, Science, 196, 1410 (1977).

Activation of the diacid with $POC1_5$ and subsequent reaction with dimethylamine gives a di-amide of formula **105**.

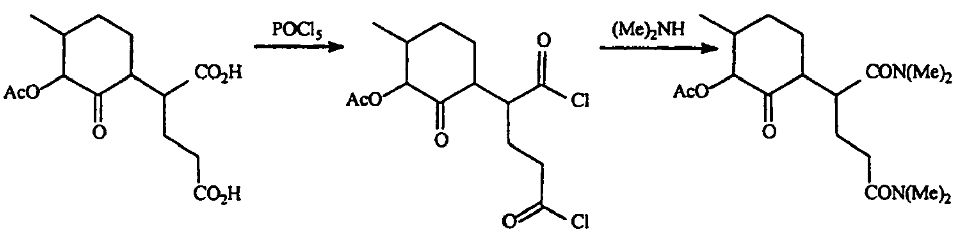


**105**

Hydrolysis of the acetoxy group of a compound of formula **105** followed by formation of the mesylate (or other suitable leaving group) and addition of sodium iodide in THF gives a compound of formula **106**.

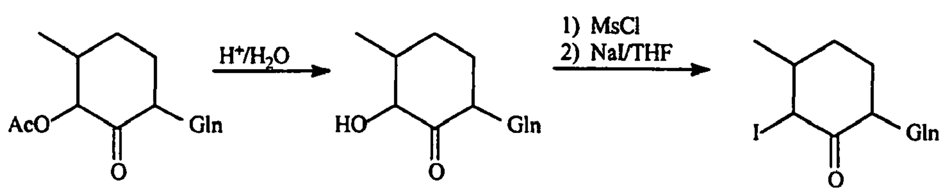


**106**

Reaction of a compound of formula **106** and a compound of formula **107** in the presence of anhydrous potassium carbonate in dry DMF, essentially as described by Lygo and Rudd (Tetrahedron Lett., 36, 3577 (1995)) followed by

removal of the sulfone, for example, using $SmI_2$, gives a compound of formula **108** which can be deprotected and acylated to give a compound of formula (IV) wherein $R^2$ and $R^3$ are $NMe_2$.

(1)

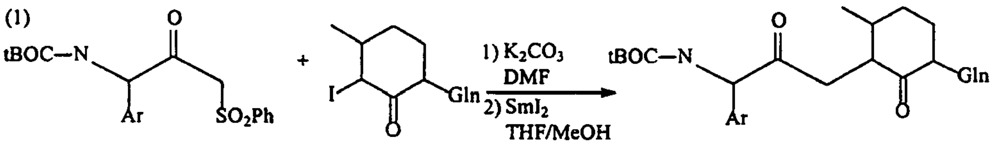


107 108

(2)

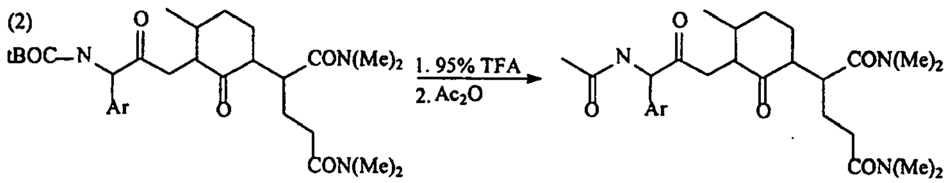


IV

Trypilamine

An intermediate of formula **107** may conveniently be prepared from a protected tryptophan (for example, N-α-tBOC-$N_{in}$tBOC-L-tryptophan-OH; Novabiochem 04-12-0201) by reaction with the dianion derived of phenylmethylsulfone.

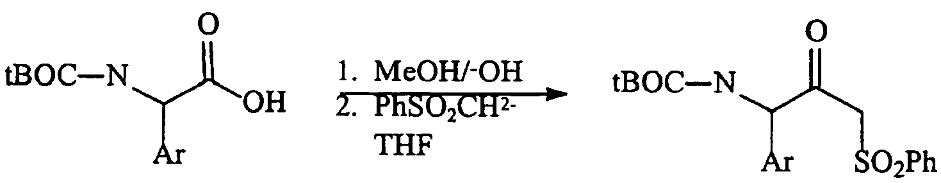


107

Ar = N-tBOC-Indolyl

A preferred synthesis for a compound of formula (IV) illustrated in the following diagram.

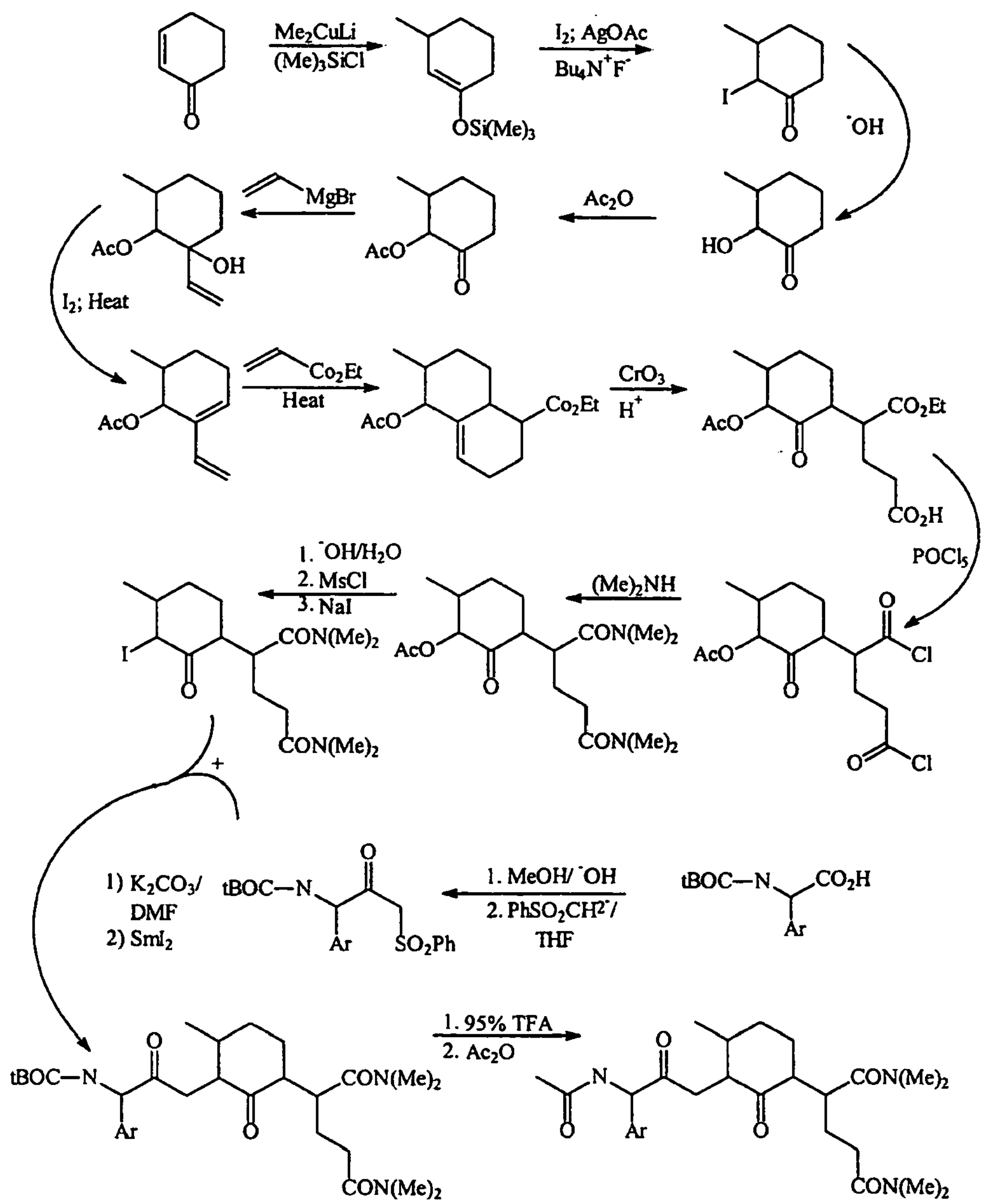


(IV)

Thioketone derivatives (Y=S) may be synthesized by insertion of an additional reaction, in which the β-ketosulfone derivative of protected tryptophan **107** is converted to the thioketone derivative. For example, reaction with a dithiol, such as 1,2-ethanedithiol, forms a thioacetal which can be hydrolyzed in the presence of $H_2S$ under anhydrous conditions, to yield the thioketone.

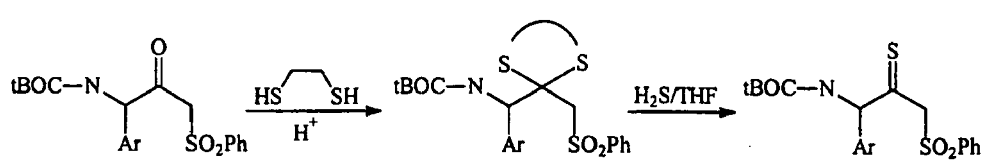


The conversion may also be carried out using [2,4-bis(4-methoxy-phenyl)-1,3-dithia-2,4-diphos-phetane-2,4 disulfide] (Lawesson's Reagent). Reaction of the thioketone derivative with the compound of formula **106** gives a compound of formula (IV) wherein Y=S.

Aryl substituents other than indolyl require preparation of suitably protected β-ketosulfone derivatives of the appropriate amino acid. Where the amino acid is readily available, the reaction can be performed using the appropriate tBOC or Fmoc protected amino acid (phenylalanine and tyrosine, respectively), for example, from Novabiochem. When the amino acid is not readily available (e.g., R=coumaryl), the suitably protected amino acid must first be prepared by methods well established in the art for synthesis of non-standard amino acids (for example, see Yuan and Hruby, Tetrahedron Lett., 38, 3853 (1997)).

As illustrated below, a compound of formula (V) can conveniently be prepared from an ester of formula 13. Deprotonation with lithium diisopropylamide followed by alkylation with bromide 14 gives a compound of formula 15. Selective reduction of the ester, for example with diisobutylaluminum hydride, gives an aldehyde of formula 16, which can be converted to the difluoroalkene 17 by a Wittig reaction with $PPh_3{=}CF_2$ (Hayashi et al., Chemistry Letters, 1980, pages 935-938) .

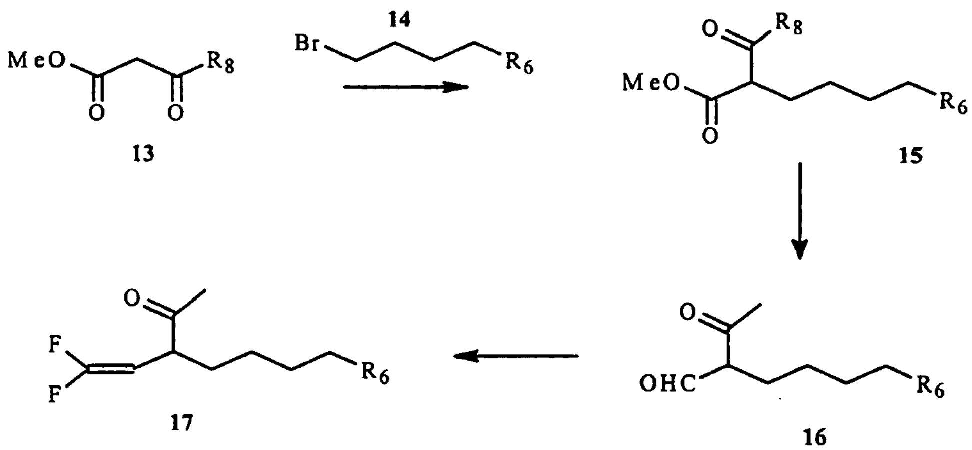
13
14
15
16
17

Aldehyde 18 can be converted to bromide 19 using a procedure similar to that described in Visweswariah et al., Synthesis, 1982, pages 309-310, by treatment with phenyltrimethylammonium tribromide, followed by formation of the acetal under standard conditions. Conversion of the bromide to the corresponding alkyllithium by treatment with n-butyllithium, followed by reaction with difluoride 17, yields a compound of formula 20 (Chemistry Letters, 1980, pages 935-940). Deprotection under acidic conditions gives aldehyde 21, which can be reacted with $PPh_3{=}CF_2$ to give trifluoride 22. Subsequent treatment of 22, with the alkyllithium derived from bromide 23 yields a compound of formula (V). It will be understood by one skilled in the art that a variety of other known protecting groups can be utilized in the above procedures and that certain protecting groups may be preferred over others depending on the structure of the groups $R_4$-$R_8$.

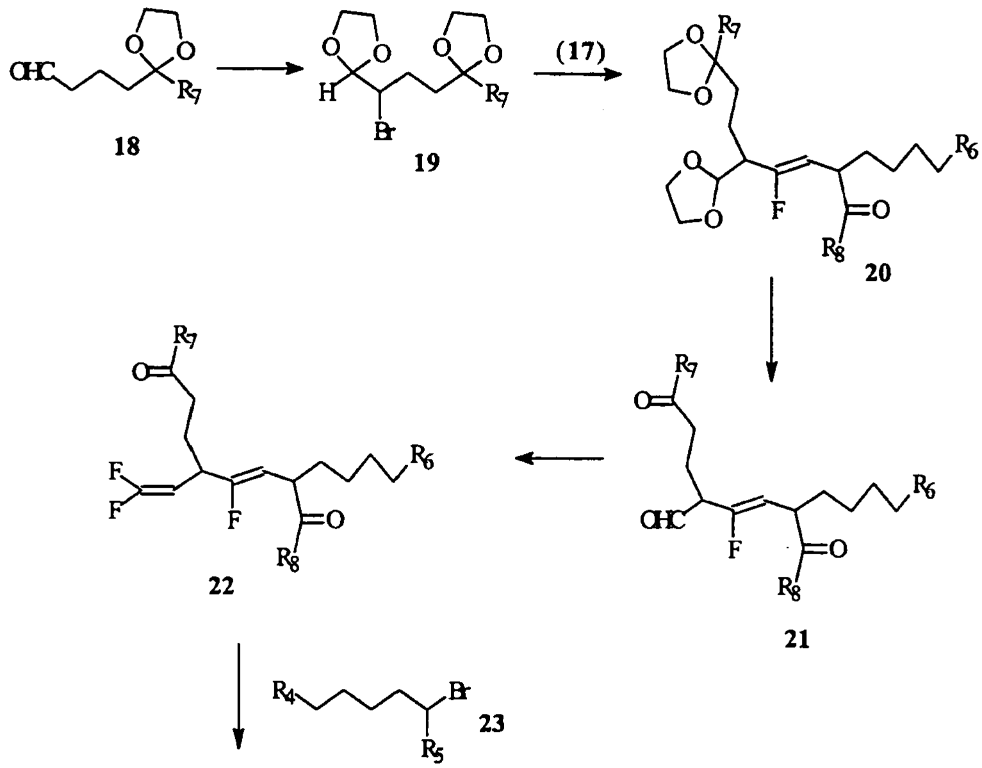


18 19 20 21 22 23

(V)

A compound of formula (XIII) can be prepared as shown in the following scheme.

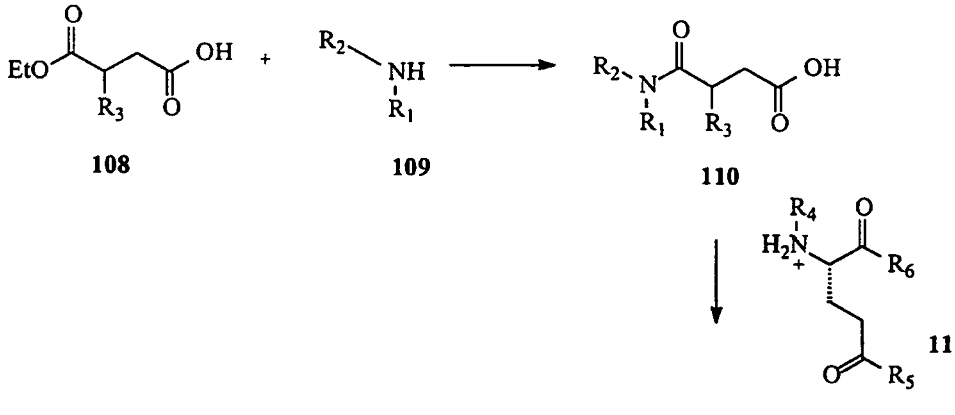

108 109 110 111

XIII

Reaction of ester **108** with an amine of formula **109** yields an acid of formula **110**. Activation of the carboxylic acid using techniques known in the art (e.g. with N-bromosuccinimide) and coupling with an amine of formula **111** yields a compound of formula (XIII).

A compound of formula (XIV) can be prepared from Yohimbine using procedures similar to those known in the art. As illustrated in Example 23, treatment of yohimbine with sodamide provides the corresponding amide of formula (XIV) wherein $R_1$ is amino. Further alkylation or acylation of this amide using standard conditions provides other compounds of formula (XIV).

A compound of formula (X) wherein $R_3$ and $R_4$ form a ring can be prepared using the following general scheme.

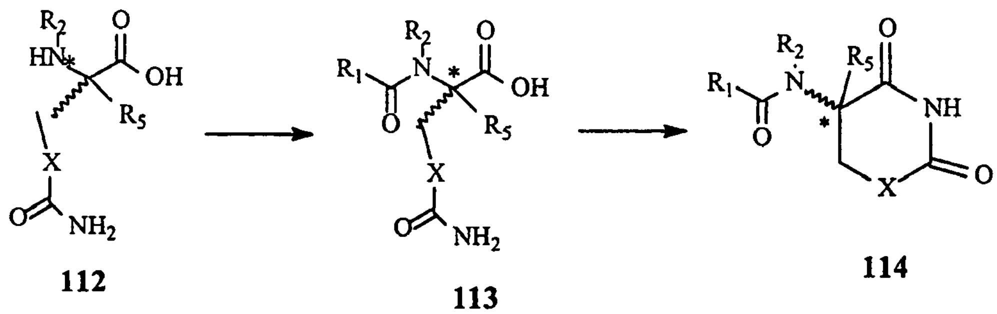

112 113 114

Acylation of the amine **112** under standard conditions followed by ring closure provides a compound of formula **114**, which is a compound of formula (X). A compound of formula **114** can be used as a starting material for preparing other compounds of formula (X).

A compound of formula (X) wherein $R_3$ and $R_4$ form a ring can also be prepared using the following general scheme.

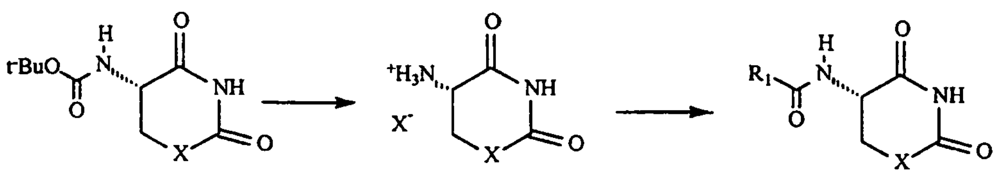

115 116 117

Hydrolysis of the carbamate **115** under standard conditions, for example using trifluoroacetic acid, yields a salt of formula **116** wherein X is a suitable counterion (e.g. triflate). Treatment of the amine salt **116** with the requisite activated acid ($R_1COOH$) gives a compound of formula **117**, which is a compound of formula (X). The amine salt of formula **116** is a particularly useful intermediate for preparing compounds of formula (X).

Compounds of formula (XII) can conveniently be prepared from lysergic acid using procedures similar to those known in the art. For example, compounds of formula (XII) can be prepared as described in Example 24.

Other useful chemokine analogs may be identified by the methods described hereinabove. In particular, chemokine analogs that are orally bioavailable, and stable and potent inhibitors of chemokine activity are preferred.

D. Targeting of the therapeutic agent

Chemokine peptides, variants, analogs or derivatives thereof may be targeted to a specific therapeutic site by linking the therapeutic agent to a moiety that specifically binds to a cellular component, e.g., antibodies or fragments thereof, lectins, transferrin (for liver targeting) and small molecule drugs, so as to form a therapeutic conjugate. Targeting of the therapeutic agents of the invention can result in increased concentration of the therapeutic agent at a specific anatomic location. Moreover, the linking of a therapeutic agent of the invention to a binding moiety may increase the stability of the therapeutic agent *in vivo.* For example, an anti-CD4 mimetic that binds to the CD4 receptor may be linked to a therapeutic agent of the invention so as to result in a therapeutic conjugate, a portion of which binds to the HIV co-receptor. This may enhance the ability to target the therapeutic agent to a particular cell type and thus block HIV infection of that cell type.

For neoplasia, anti-tumor antibodies such as NR-LU-10 (anti-carcinoma), NR-ML-5 (anti-melanoma), or anti-CD45 (anti-lymphoma), may be useful to localize the therapeutic agent to a particular type of tumor. For infectious disease, antibodies which recognize a pathogen-specific epitope, such as mAb 17. 41 *(Cryptosporidium parvum),* may be employed. To target to joints for treating rheumatoid arthritis, anti-synovium or chondroitin sulfate (e.g., Catalog No. C8035, Sigma Chemical Co., St. Louis, MO) antibodies can be linked to a therapeutic agent of the invention). To treat or prevent asthma or pneumonia, antibodies to the bronchial epithelium may be useful to prepare immunoconjugates for use in the methods of the invention.

Other antibodies useful in targeting a therapeutic agent of the invention to a specific site or cell type include

antibodies specific for blood vessels or lymphatics (e.g., Ulex europaeus-I lectin, Catalog No. U4754, Sigma Chemical Co., St. Louis, MO), blood clots or platelets (e.g., Catalog Nos. F9902, F4639, F2506, F8512, Sigma Chemical Co., St. Louis, MO), T cells (e.g., Catalog Nos. C7048 (CD3); C1805 (CD4); C7173 (CD5); and C7298 (CD7), Sigma Chemical Co., St. Louis, MO), brain (e.g., Catalog Nos. S2644 and S2407, Sigma Chemical Co., St. Louis, MO), tumors (e.g., Catalog No. C2331, Sigma Chemical Co., St. Louis, MO), epithelial cells (e.g., Catalog Nos. E6011 and C1041, Sigma Chemical Co., St. Louis, MO), fibroblasts (e.g., Catalog Nos. F4771 and V4630, Sigma Chemical Co., St. Louis, MO), macrophage (e.g., Catalog No. M1919, Sigma Chemical Co., St. Louis, MO), stomach lumen (e.g., Catalog No. M5293, Sigma Chemical Co., St. Louis, MO), neutrophils (e.g., Catalog Nos. N1890 and N1765, Sigma Chemical Co., St. Louis, MO), tendons (e.g., Catalog No. E4013, Sigma Chemical Co., St. Louis, MO), skin (e.g., Catalog No. K4252, Sigma Chemical Co., St. Louis, MO) mammary tissue or epithelium (e.g., Catalog No. C6930, Sigma Chemical Co., St. Louis, MO) and skeletal muscle (e.g., Catalog Nos. D8281 and D1033, Sigma Chemical Co., St. Louis, MO).

To prepare immunoconjugates useful for targeting a malignant or virus-infected cell, an antibody or fragment thereof having a specificity for a surface antigen on a malignant cell or virus-infected is attached to a therapeutic agent of the invention. Preferably, a chemokine peptide or variant thereof is attached via peptide bonds to the carboxyl termini regions, e.g., CH3, of antibody heavy chains. The immunoconjugates can be prepared by genetic engineering techniques, i.e, by forming a nucleic acid construct encoding the chimeric immunoconjugate. Preferably, the gene construct encoding the immunoconjugate includes, in 5' to 3' orientation, a DNA segment which encodes a heavy chain variable region, a DNA segment encoding the heavy chain constant region, and a DNA segment coding for the chemokine peptide, peptide variant, or repeats thereof. The fused gene is inserted into an expression vector for transfection of the appropriate recipient cells where it is expressed. The hybrid chain can be combined with a light (or heavy) chain counterpart to form monovalent and divalent immunoconjugates.

The heavy chain constant region for the conjugates can be selected from any of the five isotypes: alpha, delta, epsilon, gamma or mu. Heavy chains or various subclasses (such as the IgG subclasses 1-4) can be used. The light chains can have either a kappa or lambda constant chain. DNA sequences for these immunoglobulin regions are well known in the art (see, e.g., Gillies et al., L Immunol. Meth., 125, 191 (1989)).

In preferred embodiments, the variable region is derived from an antibody specific for the target antigen (an antigen associated with a diseased cell such as a cancer cell or virus-infected cell), and the constant region includes the CH1, CH2 and CH3 domains. The gene encoding the chemokine peptide or variant is joined, e.g., by appropriate linkers, e.g., by DNA encoding $(Gly_4\text{-}Ser)_3$ in frame to the 3' end of the gene encoding the constant region (e.g., CH3 exon), either directly or through an intergenic region. In certain embodiments, the intergenic region can comprise a nucleotide sequence coding for a proteolytic cleavage site. This site, interposed between the immunoglobulin and the chemokine peptide or variant, can be designed to provide for proteolytic release of the chemokine peptide or variant at the target site. For example, it is well known that plasmin and trypsin cleave after lysine and arginine residues at sites that are accessible to the proteases. Many other site-specific endoproteases and the amino acid sequences they attack are well known.

The nucleic acid construct can include the endogenous promoter and enhancer for the variable region-encoding gene to regulate expression of the chimeric immunoglobulin chain. For example, the variable region encoding genes can be obtained as DNA fragments comprising the leader peptide, the VJ gene (functionally rearranged variable (V) regions with joining (J) segment) for the light chain or VDJ gene for heavy chain, and the endogenous promoter and enhancer for these genes. Alternatively, the gene coding for the variable region can be obtained apart from endogenous regulatory elements and used in an expression vector which provides these elements.

Variable region genes can be obtained by standard DNA cloning procedures from cells that produce the desired antibody. Screening of the genomic library for a specific functionally rearranged variable region can be accomplished with the use of appropriate DNA probes such as DNA segments containing the J region DNA sequence and sequences downstream. Identification and confirmation of correct clones are then achieved by DNA sequencing of the cloned genes and comparison of the sequence to the corresponding sequence of the full length, properly spliced mRNA.

Genes encoding appropriate variable regions can be obtained generally from Ig-producing lymphoid cells. For example, hybridoma cell lines producing Ig specific for tumor associated antigens or viral antigens can be produced by standard somatic cell hybridization techniques. These Ig-producing cell lines provide the source of variable region genes in functionally rearranged form. The variable region genes are typically of murine origin because the murine system lends itself to the production of a wide variety of Igs of desired specificity.

The DNA fragment containing the functionally rearranged variable region gene is linked to a DNA fragment containing the gene encoding the desired constant region (or a portion thereof). Ig constant regions (heavy and light chain) can be obtained from antibody-producing cells by standard gene cloning techniques. Genes for the two classes of human light chains and the five classes of human heavy chains have been cloned, and thus, constant regions of human origin are readily available from these clones.

The fused gene encoding the hybrid IgH chain is assembled or inserted into expression vectors for incorporation into a recipient cell. The introduction of gene construct into plasmid vectors can be accomplished by standard gene

splicing procedures.

The chimeric IgH chain can be co-expressed in the same cell with a corresponding L chain so that a complete immunoglobulin can be expressed and assembled simultaneously. For this purpose, the heavy and light chain constructs can be placed in the same or separate vectors.

Recipient cell lines are generally lymphoid cells. The preferred recipient cell is a myeloma (or hybridoma). Myelomas can synthesize, assemble, and secrete immunoglobulins encoded by transfected genes and they can glycosylate polypeptide. A particularly preferred recipient cell is the Sp2/0 myeloma which normally does not produce endogenous immunoglobulin. When transfected, the cell will produce only Ig encoded by the transfected gene constructs. Transfected myelomas can be grown in culture or in the peritoneum of mice where secreted immunoconjugate can be recovered from ascites fluid. Other lymphoid cells such as B lymphocytes can be used as recipient cells.

There are several methods for transfecting lymphoid cells with vectors containing the nucleic acid constructs encoding the chimeric Ig chain. A preferred way of introducing a vector into lymphoid cells is by spheroblast fusion (see Gillies et al., Biotechnol 2, 798-804 (1989)). Alternative methods include electroporation or calcium phosphate precipitation.

Other useful methods of producing the immunoconjugates include the preparation of an RNA sequence encoding the construct and its translation in an appropriate *in vivo* or *in vitro* system.

Methods for purifying recombinant immunoglobulins are well known. For example, a well known method of purifying antibodies involves protein A purification because of the propensity of protein A to bind the Fc region of antibodies. The antigen binding activity of the purified immunoconjugates can then be measured by methods well known to the art, such as described in Gillies et al. (J. Immunol Methol., 125, 191 (1989)). For example, immunoconjugate activity can be determined using antigen-coated plates in either a direct binding or competition assay format.

In particular, it is preferred that humanized antibodies are prepared and then assayed for their ability to bind antigen. Methods to determine the ability of the humanized antibodies to bind antigen may be accomplished by any of numerous known methods for assaying antigen-antibody affinity. For example, the murine antibody NR-LU-13 binds an approximately 40 kilodalton glycoprotein expressed on numerous carcinomas. This antigen has been characterized in Varki et al., Cancer Res., 44, 681 (1984); Okabe et al., Cancer Res., 44, 5273 (1989). Thus, it is routine to test the ability of humanized antibodies to bind the NR-LU-13 antigen. Moreover, methods for evaluating the ability of antibodies to bind to epitopes of this antigen are known.

Humanized antibodies (or fragments thereof) are useful tools in methods for therapeutic purposes. When determining the criteria for employing humanized antibodies or antibody conjugates for *in vivo* administration for therapeutic purposes, it is desirable that the general attainable targeting ratio is high and that the absolute dose of therapeutic agent delivered to the tumor is sufficient to elicit a significant tumor response. Methods for utilizing the humanized antibodies can be found, for example, in U.S. Patent Nos. 4,877,868, 5,175,343, 5,213,787, 5,120,526, and 5,202,169.

To target vascular smooth muscle cells (VSMC), VSMC binding proteins, e.g., polypeptides or carbohydrates, proteoglycans and the like, that are associated with the cell membranes of vascular smooth muscle cells can be employed to prepare therapeutic conjugates. In a preferred embodiment, the binding moiety is exemplified by chondroitin sulfate proteoglycans (CSPGs) synthesized by vascular smooth muscle cells and pericytes, and a discrete portion (termed an epitope herein) of the CSPG molecule having an apparent molecular weight of about 250 kD is especially preferred. The 250 kD target is an N-linked glycoprotein that is a component of a larger 400 kD proteoglycan complex. In one presently preferred embodiment of the invention, a vascular smooth muscle binding protein is provided by NR-AN-01 monoclonal antibody (a subculture of NR-ML-05) that binds to an epitope in a vascular smooth muscle CSPG target molecule. The monoclonal antibody designated NR-ML-05 reportedly binds a 250 kD CSPG synthesized by melanoma cells (Morgan et al., U.S. Pat. No. 4,897,255). Smooth muscle cells and pericytes also reportedly synthesize a 250 kD CSPG as well as other CSPGs. NR-ML-05 binding to smooth muscle cells has been disclosed (Fritzberg et al., U.S. Pat. No. 4,879,225). Subculture NR-ML-05 No. 85-41-4I-A2, freeze # A2106, has been deposited with the American Type Culture Collection, Rockville, MD and granted Accession No. HB-9350. NR-ML-05 is the parent of, and structurally and functionally equivalent to, subclone NR-AN-01, disclosed herein. It will be recognized that NR-AN-01 is just one example of a vascular smooth muscle binding protein that specifically associates with the 400 kD CSPG target, and that other binding proteins associating with this target and other epitopes in this target are also useful in the therapeutic conjugates and methods of the invention.

It will be recognized that the inventors also contemplate the utility of human monoclonal antibodies or "humanized" murine antibody as a vascular smooth muscle binding protein in the therapeutic conjugates of their invention. For example, murine monoclonal antibody may be "chimerized" by genetically recombining the nucleotide sequence encoding the murine Fv region (i.e., containing the antigen binding sites) with the nucleotide sequence encoding a human constant domain region and an Fc region, e.g., in a manner similar to that disclosed in European Patent Application No. 0,411,893 A2. Humanized vascular smooth muscle binding partners will be recognized to have the advantage of decreasing the immunoreactivity of the antibody or polypeptide in the host recipient, which may thereby be useful for increasing the *in vivo* half-life and reducing the possibility of adverse immune reactions. See also, N. Lonberg et al. (U.S. Patent Nos.

5,625,126; 5,545,806; and 5,569,825); and Surani et al. (U.S. Patent No. 5,545,807).

Useful binding peptides for cancer treatment embodiments of the present invention include those associated with cell membrane and cytoplasmic epitopes of cancer cells and the like. These binding peptides localize to the surface membrane of intact cells and internal epitopes of disrupted cells, respectively, and deliver the therapeutic agent for assimilation into the target cells. Minimal peptides, mimetic organic compounds and human or humanized antibodies that localize to the requisite tumor cell types are also useful as binding peptides of the present invention. Such binding peptides may be identified and constructed or isolated in accordance with known techniques. Preferred binding peptides of these embodiments of the present invention bind to a target epitope with an association constant of at least about $10^{-6}$M.

Methods useful to prepare antibody-peptide conjugates are well known to the art. See, for example U.S. Patent No. 5,650,150, the disclosure of which is incorporated by reference herein. Representative "coupling" methods for linking the therapeutic agent through covalent or non-covalent bonds to the targeting moiety include chemical cross-linkers and heterobifunctional cross-linking compounds (i.e., "linkers") that react to form a bond between reactive groups (such as hydroxyl, amino, amido, or sulfhydryl groups) in a therapeutic agent and other reactive groups (of a similar nature) in the targeting moiety. This bond may be, for example, a peptide bond, disulfide bond, thioester bond, amide bond, thioether bond, and the like. In one illustrative example, conjugates of monoclonal antibodies with drugs have been summarized by Morgan and Foon (Monoclonal Antibody Therapy to Cancer: Preclinical Models and Investigations, Basic and Clinical Tumor Immunology, Vol. 2, Kluwer Academic Publishers, Hingham, MA) and by Uhr, J. of Immunol. 133:i-vii, 1984). In another illustrative example where the conjugate contains a radionuclide cytostatic agent, U.S. Patent No. 4,897,255, Fritzberg et al., incorporated herein by reference, is instructive of coupling methods that may be useful. In one embodiment, the therapeutic conjugate contains a vascular smooth muscle binding protein coupled covalently to a chemokine peptide or variant. In this case, the covalent bond of the linkage may be formed between one or more amino, sulfhydryl, or carboxyl groups of the vascular smooth muscle binding protein and the chemokine peptide or variant.

In a preferred embodiment of the invention, an antibody conjugate is used in pretargeting methods. Essentially, such pretargeting methods are characterized by an improved targeting ratio or increased absolute dose to the target cell sites in comparison to conventional cancer diagnosis or therapy. A general description of pretargeting methods may be found in U.S. Patent No. 4,863,713, 5,578,287, and 5,630,996. Typical pretargeting approaches are summarized below.

Pretargeting methods are of two general types: three-step pretargeting methods and two-step pretargeting methods. A three-step pretargeting protocol includes the administration of a targeting moiety-ligand conjugate, which is allowed to localize at a target site and to dilute in the circulation. This is followed by administration of an anti-ligand which binds to the targeting moiety-ligand conjugate and clears unbound targeting moiety-ligand conjugate from the blood, as well as binds to targeting moiety-ligand conjugate at the target site. Thus, the anti-ligand fulfills a dual function by clearing targeting moiety-ligand conjugate not bound to the target site as well as attaches to the target site to form a targeting moiety-ligand : anti-ligand complex. Finally, a therapeutic agent-ligand conjugate that exhibits rapid whole body clearance is administered.

When the therapeutic agent-ligand conjugate in circulation comes into close proximity to the targeting moiety-ligand : anti-ligand complex bound to the target site, the anti-ligand portion of the complex binds to the ligand portion of the circulating therapeutic agent-ligand conjugate, thus producing a targeting moiety-ligand : anti-ligand : ligand-therapeutic agent "sandwich" at the target site. Furthermore, because the unbound therapeutic agent is attached to a rapidly clearing ligand (rather than a slowly clearing targeting moiety, such as antibody or antibody fragment), this technique provides decreased non-target exposure to the active agent.

Alternatively, two-step pretargeting methods eliminate the step of administering the above identified anti-ligand. These "two-step" procedures feature targeting moiety-ligand or targeting moiety-anti-ligand administration, followed by the administration of a therapeutic agent which is conjugated to the opposite member of the ligand/anti-ligand pair.

As an optional step in the two-step pretargeting method, ligand or anti-ligand, designed specifically to provide a clearance function, is administered to facilitate the clearance of circulating targeting moiety-ligand or targeting moiety-anti-ligand. Thus, in the two-step pretargeting approach, the clearing agent does not become bound to the target cell population, either directly or through the previously administered target cell bound targeting moiety-anti-ligand or targeting moiety-ligand conjugate.

A targeting moiety in a pretargeting method binds to a defined target cell population, such as tumor cells. Preferred targeting moieties useful in this regard are antibodies (polyclonal or monoclonal), such as human monoclonal antibodies, or "humanized" murine or chimeric antibodies. Some examples of humanized antibodies include those that are CHO produced, produced in hosts such as plant (for example corn, soybean, tobacco, and the like), insect, mammalian, yeast, and bacterial. The humanized antibodies may be those that bind to the antigen bound by antibody NR-LU-13. Preferably, the humanized antibody may not possess N-linked glycosylation or its N-linked glycosylation has been modified post expression to reduce immunogenicity or toxicity.

Ligand/anti-ligand pairs suitable for use in targeting protocols include biotin/avidin or streptavidin, haptens and epitopes/antibody, fragments or analogs thereof, including mimetics, lectins/carbohydrates, zinc finger proteins/dsDNA

fragments, enzyme inhibitors/enzymes; and analogs and derivatives thereof. Preferred ligands and anti-ligands bind to each other with an affinity of at least about $K_A \geq 10^9 M^{-1}$ or $K_D \leq 10^{-9} M$. Biotin/avidin or streptavidin is a preferred ligand/anti-ligand pair.

In general, such pretargeting methods preferably include the administration of an anti-ligand that provides a clearance function. The clearance is probably attributable to cross-linking and/or aggregation of conjugates that are circulating in the blood, which leads to complex/aggregate clearance by the recipient's RES (reticuloendothelial system). The anti-ligand clearance of this type is preferably accomplished with a multivalent molecule. However, a univalent molecule of sufficient size to be cleared by the RES on its own could also be employed.

Alternatively, receptor-based clearance mechanisms, e.g., Ashwell receptor or other receptors, may be exploited by addition of hexose residues, such as galactose or mannose residues, to provide for clearance of the anti-ligand, anti-ligand conjugate or humanized antibody via the liver. Such clearance mechanisms are less dependent upon the valency of the clearing agent than the RES complex/aggregate clearance mechanisms described above.

For example, if the targeting moiety-ligand or targeting moiety-anti-ligand has been derivatized to provide for clearance (i.e., addition of a hexose residue) a clearing agent should not be necessary. Preferred clearing agents are disclosed in U.S. Patent Nos. 5,624,896 and 5,616,690; as well as PCT Application Publication Number WO 95/15978.

One skilled in the art, based on the teachings herein and the applications referenced herein, can readily determine an effective therapeutic effective dosage and treatment protocol. This will depend upon factors such as the particular selected therapeutic agent, route of delivery, the type of target site(s), affinity of the targeting moiety for target site of interest, any cross-reactivity of the targeting moiety with normal tissue, condition of the patient, whether the treatment is effected alone or in combination with other treatments, among other factors.

For example, in the case of humanized antibody - avidin or streptavidin conjugates in pretargeting strategies, a suitable dosage ranges from about 10 to about 2500 mg, more preferably from about 50 to 1500 mg, and most preferably from about 100 to 800 mg. The dosage of the ligand-therapeutic agent conjugate, generally ranges from about 0.001 to about 10 mg and more preferably from about 0.1 to 2 mg.

In general, such pretargeting methods include the administration of a clearing agent. The dosage of the clearing agent is an amount which is sufficient to substantially clear the previously administered conjugate from the circulation, i.e., at least about 50%, more preferably at least about 90%, and most preferably approaching or at 100%. In general, the clearing agent is administered several days after administration of the humanized antibody - streptavidin conjugate, preferably about 1 to 5 days after, more preferably at least about 1 to 2 days after. Generally, the determination of when to administer the clearing agent depends on the target uptake and endogenous clearance of targeting moiety conjugate. Particularly preferred clearing agents are those which provide for Ashwell receptor mediated clearance, such as galactosylated proteins, e.g., galactosylated biotinylated human serum albumin (HSA) and small molecule clearing agents containing galactose and biotin. In the case of HSA based clearing agents, a typical dosage of the clearing agent will range from about 100 to 1000 mg, and more preferably about 200-500 mg. If a clearing agent is administered, the ligand-therapeutic agent conjugate is preferably administered about 2 to 12 hours after.

The conjugates may be administered by known methods of administration. Known methods of administration include, by way of example, intraperitoneal injection, intravenous injection, intramuscular injection, intranasal administration, among others. Intravenous administration is generally preferred.

## III. Indications Amenable to Treatment by the Agents of the Invention

The agents of the invention are useful to treat a mammal afflicted with, to inhibit in a mammal at risk of, or to augment in a mammal at risk of, an indication associated with chemokine-induced activity, such as aberrant or pathological inflammatory processes. The chemokines participate in a broad range of inflammatory processes, both physiological and pathological. Thus, broad specificity chemokine inhibitors may be useful to treat or prevent a wide range of inflammatory diseases. Moreover, the use of rationally designed chemokine inhibitors, i.e., inhibitors with relative specificity for various chemokines, may reduce or inhibit side-effects associated with chronic therapies of broad spectrum chemokine inhibitors. Thus, these inhibitors may be designed to treat particular diseases, thereby minimizing side effects resulting from disrupting unrelated physiological processes.

Atherosclerosis. Development of atherosclerosis is a complex process involving smooth muscle cells, endothelial cells and inflammatory cells, and, in particular, monocyte-derived tissue macrophages, B or T cells. Once endothelial cells are activated, they express adhesion molecules important for the extravasation of inflammatory cells. For example, in the TGF$\beta$1 knockout (-/-) mouse, the absence of this cytokine resulted in endothelial cell activation. The activated endothelial cells express, among other adhesion molecules, E-selectin, P-selectin, and ICAM-1, which in turn participate in the extravasation of leukocytes. Potent pro-inflammatory cytokines were also expressed at the sites of incipient vascular lesions. TNF-$\alpha$, IL-1, as well as several chemokines including IL-8 and MCP-1, have been detected at elevated levels in atherosclerotic lesions. Results described hereinabove show that the chemokine MCP-1 in particular plays a role in atherosclerotic vascular inflammation.

It is now well accepted that the acute stability of vascular lesions is a more important determinant of short-term, e.g., less than several years, risk of myocardial infarction than is total plaque burden. The degree of macrophage infiltration is probably the major determinant of relative plaque stability. At least two factors contribute to plaque stability: macrophages secrete an excess of matrix-degrading enzymes (such as the matrix metalloproteinases) over their inhibitors, resulting in the loss of extracellular matrix (ECM) in the macrophage-rich shoulder and fibrous cap regions, a common feature of unstable or ruptured plaques; and macrophage-derived foam cells become necrotic, possibly in response to toxic oxidative metabolites of lipids, resulting in a lipid-filled extracellular pool which further destabilizes the local vessel wall architecture.

Inhibitors of chemokine action, and in particular inhibitors of MCP-1, may improve plaque stability and thus rapidly reduce the risk of myocardial infarction, without necessarily reducing the total atherosclerotic plaque burden. In particular, the agents of the invention may decrease lipid lesion formation and/or lipid lesion progression as well as increasing plaque stability (Boring et al., Nature, 394, 894 (1998)). Thus, agents of the invention, e.g., peptide 3(1-12) [MCP-1] (SEQ ID NO:1), KQK, peptide 3[7-12] (SEQ ID NO:9), as well as variants, e.g., $Leu_4Ile_{11}$peptide 3(1-12)[MCP-1] (SEQ ID NO: 14), or derivatives thereof (e.g. NR58,4, Y-II, and L-II), may be useful to treat and/or prevent unstable angina pectoris, atherosclerosis, as well as other diseases characterized by local or systemic vasculitis, as well as the symptoms and diseases which occur secondarily to the vessel wall inflammation such as myocardial infarction.

Moreover, the agents of the invention are also useful in combination with lipid lowering agents, such as the statins, or TGF-beta elevating agents (see, for example, WO 96/40098, the disclosure of which is incorporated by reference herein).

Osteoporosis. Low bone mineral density, often categorized as osteoporosis, results from an imbalance between bone matrix deposition by osteoblasts and its subsequent resorption by osteoclasts. The balance between these two dynamic processes determines bone density. One strategy to increase bone density has been the use of analogs of tamoxifen, such as raloxifene, which mimic the effects of estrogen on bone and thus, promote osteoblast differentiation (increasing bone matrix deposition) and inhibit osteoclast recruitment (decreasing resorption). An alternative strategy is to decrease matrix resorption by directly inhibiting the mechanism by which osteoclasts are recruited to the bone. Measurement of bone matrix degradation products (such as the N-terminal and C-terminal telopeptides of collagen as well as pyridinium cross-links) in plasma and urine confirm that bone resorption is increased in osteoporosis, and hence inhibition of osteoclast activity is likely to prove an effective therapeutic strategy.

Unlike osteoblasts, which are locally derived, osteoclasts are continuously recruited to bone as precursor cells which circulate in the monocyte fraction, and which may be identical to monocytes. Once recruited, the precursors differentiate into osteoclasts which then resorb matrix until they die by apoptosis. Thus, the number of osteoclasts in bone tissue (and hence the osteoclast activity) can be rapidly regulated by modulating the osteoclast recruitment process.

A number of lines of evidence now suggest that the monocyte recruitment into bone is a molecular parallel of the pathological monocyte recruitment into the blood vessel wall that occurs during atherogenesis. In particular, the chemokine MCP-1 is implicated in both processes. Thus, MCP-1 inhibitors may act to reduce monocyte recruitment and thus decrease osteoclast recruitment and/or decrease the number of cells differentiating into osteoclasts, which would result in a rapid increase in bone density, for example, over a period of weeks rather than years. The ability of the present therapeutic agents to increase bone density contrasts with existing drugs which prevent a further decrease in bone density but do not increase bone density. Therefore, peptide 3, e.g., peptide 3(7-12)[MCP-1], and variants (e.g., $Leu_4Ile_{11}$peptide 3(1-12)[MCP-1]) and derivatives (e.g., CRD-$Cys_{13}Leu_4Ile_{11}$peptide 3(3-12)[MCP-1]) thereof, may be useful to inhibit or prevent low bone density. In particular, derivatives with specificity for CC chemokines, such as KQK analogs and the WAQ analogs are preferred agents for the treatment of osteoporosis.

HIV Infection and AIDS. In addition to the CD4 receptor, additional cell surface molecules (termed co-receptors) are required for the productive infection of a cell by HIV isolates. HIV isolates can be divided into two subtypes, which depend on whether they can infect monocyte/macrophages (M-tropic strains) or helper T lymphocytes (T-tropic strains). Experiments with chemokine ligands suggest that the chemokine receptors function as the HIV co-receptors: MIP1$\alpha$ and RANTES inhibited the infection of monocytes with M-tropic strains (but not infection of T-cells by T-tropic strains), while SDF-1 inhibited T cell infection (but not monocyte infection). Further molecular analyses confirmed that the MIP1$\alpha$/RANTES receptor CCR-5 is the HIV co-receptor on monocytes while the SDF-1 receptor CXCR-4 (also termed LESTR and fusin) is the co-receptor on T-cells. Early in infection, M-tropic virus predominates, a virus which is non-syncytium forming, less virulent and does not deplete T-cells. At a later time, selection favors conversion to the more virulent, syncytium forming T-tropic strain, a strain which depletes helper T cells and leads to acquired immunodeficiency (AIDS). Some isolates of HIV have been reported which can efficiently use other chemokine receptors (such as CCR2a or CCR3), while other isolates which predominantly use CCR5 can use CCR2a at lower efficiency (Doranz et al., Cell, 85, 1149 (1996); Ross et al., Proc Natl. Acad Sci USA, 95, 7682 (1998)). These findings suggest that a blockade of any one chemokine receptor may be ineffective, at least once an infection has been established and the viral load is relatively high (Cairns et al., Nat. Med., 4, 563 (1998)). This limits the likely impact of the small number of chemokine receptor antagonists described to date (reviewed in Cairns et al., Nat. Med., 4, 563 (1998)), all of which are specific for one, or

a small subset, of chemokine receptors. Thus, to provide an effective agent to inhibit HIV, the agent preferably inhibits virus binding to more than one receptor, i.e., an agent would have to have broad specificity for chemokine receptors.

Genetic studies have identified a mutation in CCR5 which renders individuals essentially immune to HIV infection. This mutation, termed CCR5Δ32, results in a truncated mRNA for CCR-5. The expression of the truncated CCR-5 does not produce any detectable CCR-5 protein on the cell surface. Individuals homozygous for this deficiency have been reported to be entirely resistant to HIV infection, even under exposure to extremely high viral challenge, although there is now a single report of a homozygous mutant individual seropositive for HIV infection. Thus, these observations demonstrate that effective blockade of the CCR-5 receptor may effectively prevent infection. Moreover, CCR-5 mediated chemokine signaling does not have a crucial role in normal physiology, since CCR-5Δ32 homozygotes have no detectable phenotype other than HIV resistance.

Therefore, inhibitors of chemokine receptors, such as peptide 3, its variants, analogs or derivatives, may inhibit HIV infection as these agents have broad specificity. As described hereinbelow (Example 5), peptide 3[MCP-1] inhibited HIV binding and infection of Jurkat cells and macrophage. A preferred agent to prevent or inhibit HIV infection and/or replication is CRD-$Cys_{13}Leu_4Ile_{11}$peptide 3(3-12)[MCP-1]. In particular, peptide 3, its variants, analogs or derivatives, e.g., CRD-$Cys_{13}Leu_4Ile_{11}$peptide 3(3-12)[MCP-1], may be especially useful to inhibit infection of M-tropic strains of HIV.

Peptide 2, its variants, analogs or derivatives, are also useful to prevent or inhibit HIV infection and/or replication, as peptide 2 inhibited HIV replication in T cells and macrophage. Preferred therapeutic agents have decreased Duffy binding and increased co-receptor affinity (in at least about the nM range) (see Example 5) relative to the corresponding chemokine or peptide having the native or wild-type sequence. Preferably, Peptide 2, its variants, analogs or derivatives, e.g., LRD derivatives, are useful to inhibit T-tropic strains of HIV.

Thus, a combination of peptide 3, its variants, analogs or derivatives, and peptide 2, its variants, analogs or derivatives, may be particularly useful to prevent or treat HIV infection.

Thus, these agents are useful for the treatment, as well as the prevention, of both HIV seropositives and of progression of seropositive patients to AIDS, when used, either alone, in combination, or in combination with other anti-viral therapies. When used in combination, it is preferred that an infected individual is pre-treated with viral inhibitors (such as a cocktail of reverse transcriptase and viral protease inhibitors) and then given doses of a general chemokine inhibitor, preferably peptide 3, peptide 2, their variants or derivatives, more preferably peptide 2[MIP1α], its analogs or derivatives. Moreover, since resistance to other therapies (such as protease inhibitors or reverse transcriptase inhibitors) arise because of viral replication, agents which reduce virus infectivity may drastically increase the success of these existing therapies. Specifically, unlike all currently exploited therapeutic targets such as reverse transcriptase or the viral protease, chemokine agonists and/or antagonists target the susceptible cell rather than the virus itself. Although the virus can rapidly mutate to generate strains resistant to the virus-targeted agents, cells mutate less readily and are under less or no selective pressure to mutate. The extent to which the mutations in the HIV virus must occur to circumvent the use of a chemokine co-receptor is likely to be much greater than the mutations necessary to render a reverse transcriptase resistant to a reverse transcriptase inhibitor. Thus, the administration of chemokine analogs is likely to prove effective either alone or in combination with the virus-targeted therapies. Furthermore, chemokine inhibitors may have limited side effects *in vivo,* i.e., limited physiological impact, and therefore have a good therapeutic index when used *in vivo.*

Stroke. Inflammatory processes have been implicated in the pathophysiology of stroke or cerebral ischemia. The effects of the inflammatory response following stroke are detrimental, thus, there is a benefit afforded by preventing or inhibiting the inflammatory response. Activated neutrophils promote cerebral ischaemic injury by vascular plugging and by production of cytotoxic substances. For example, the early post ischemic recruitment and influx of vascular leukocytes, mainly neutrophils, into the brain represents a therapeutic target for the agents of the invention. Selective chemokine expression by central nervous system cells is important for post-ischaemic vascular leukocyte targeting, e.g., MCP-1 as well as other chemokines are upregulated in the central nervous system of stroke patients.

Psoriasis. Psoriasis is an inflammatory disorder that is associated with MCP-1 and monocyte recruitment. Topical application of a therapeutic agent of the invention, e.g., peptide 3, is preferred to prevent or treat psoriasis as this delivery method reduces bioavailability problems. Derivatives of the therapeutic agents of the invention, e.g., CRD peptides, which are administered topically may exhibit enhanced bioavailability relative to non-derivatized counterparts. Alternatively, psoriasis may be treated by systemic administration of an agent of the invention such as for example, CRD-$Leu4Ile_{11}Cys_{13}$-peptide 3(3-13)[MCP-1], NR58,4, Y-II,and L-II).

Autoimmune Diseases. Autoimmune diseases, such as multiple sclerosis, Crohn's disease, rheumatoid arthritis and systemic lupus erythematosus, are characterized by inappropriate activation of the immune system, orchestrated by autoreactive leukocytes. Although it remains unclear what factors lead to the initial inappropriate recognition of self-antigens, a number of pro-inflammatory cytokines have been implicated in the continuing inflammation which underlies the tissue destruction that, in turn, leads to the morbidity and mortality associated with these diseases. Of these inflammatory cytokines, TNF-α and the chemokines (in particular MIP-1α) have been implicated.

For example, elevated MIP-1α expression is detected in experimental autoimmune encephalomyelitis, a model of T-cell mediated autoimmune disease with some common characteristics to human multiple sclerosis. Elevated MIP1α

activity is also detected in the cerebrospinal fluid of patients with multiple sclerosis. Antibody therapy to reduce chemokine levels has been shown to be effective in animal models of autoimmune diseases, but this method exhibits tachyphalaxis and only lowers chemokine levels for a short period, and is unlikely to be useful in human therapy. In contrast, a general antagonist of chemokine signaling is likely to suppress the inappropriate inflammation indefinitely. Thus, peptide 3, its derivatives and variants, may be useful to prevent and/or treat autoimmune disorders including, but not limited to, type I diabetes, multiple sclerosis, rheumatoid arthritis and systemic lupus erythematosus.

Moreover, different chemokine expression patterns may be associated with different autoimmune disorders, and hence each autoimmune disease may require a different derivative or variant of peptide 3. For example, MIP1$\alpha$ may play a central role in multiple sclerosis. MIP1$\alpha$ is a CC chemokine. Thus, the administration of a CC-selective agent of the invention can be used to treat multiple sclerosis (e.g., KQK compounds, compounds of formula (V), or $Ser_7Glu_8Glu_9$peptide 3(1-12)[MCP-1]).

Following wounding, there is a complex process of wound healing involving recruitment and proliferation of different cell types, elaboration of matrix, and increased immune surveillance. In the fetus (where increased immune surveillance is not required) this wound healing process leads to complete restoration of the normal tissue architecture (e.g., normal dermal architecture is restored after incisional wounding). In marked contrast, in the adult, incisional wounding results in a wound healing process that does not restore normal dermal architecture. Matrix is elaborated in excess amounts and in inappropriate spatial organization. The result is a scar. In some cases, such as in children following severe wounding such as from burns, the scars are hypertrophic having huge excess of matrix deposition and are particularly disfiguring.

In adults, the risk of infection following wounding is high. Leukocytes, particularly neutrophils, are recruited rapidly to the wound site, while monocyte/macrophages appear several days after wounding, resulting in a rapid formation of granulomatous tissue. Studies with antibodies have suggested that CXC chemokines such as IL-8 play an important role in neutrophil attraction to the wound site, and that inhibition of IL-8 production reduces both neutrophil accumulation and subsequent scarring. Experiments blocking CC chemokines have similarly shown that they have a role in the attraction of macrophages to the wound site, and these cells may also promote rapid healing at the expense of wound quality. Hence inhibition of either CXC or CC chemokines, or both, may result in a decrease in the wound-induced inflammatory reaction, and in turn promote a balance between fast healing and good restoration of dermal architecture.

To prevent or reduce scarring and/or enhance wound healing, a preferred embodiment of the invention is the topical application of a therapeutic agent of the invention that inhibits chemokine action at the site of the wound. Thus, a broad spectrum chemokine inhibitor, such as peptide 3(1-12)[MCP-1], $Leu_4Ile_{11}$peptide 3(1-12)[MCP-1], CRD-$Leu_4Ile_{11}$peptide 3[MCP-1], NR58,4,Y-II, L-II, or WVQ, or combinations thereof may be administered. Alternatively, a selective inhibitor of IL-8, such as KEN, or a selective inhibitor of MCP-1, such as KQK, as well as combinations thereof may be administered. In addition, a combination of a broad spectrum inhibitor and a selective inhibitor may be administered. In this way, the various components of the wound-induced inflammatory process may be controlled as desired and the wound may be allowed to heal more slowly (under conditions where it is protected from infection, e.g., by simultaneous use of antibiotics) but with enhanced recovery of dermal architecture. See U.S. Patent No. 5,202,118 for methods to determine the efficacy of an agent to treat or enhance wound healing.

Hypertension. Hypertension is a risk factor for atherosclerosis. To determine whether an agent of the invention is useful to inhibit or treat hypertension, a rabbit model is employed. New Zealand white rabbits are fed an atherogenic diet for three weeks to induce plaque formation. One half of each group of rabbits is administered an agent of the invention. Aortic coarctation is created in one group of the rabbits by wrapping a Dacron band around the midportion of the descending thoracic aorta (stenosis group). Another group of rabbits undergo the banding technique without aortic constriction. Yet another group of rabbits serve as nonoperated controls. Monocyte binding to the aortic endothelial surface is determined with epifluorescent microscopy on standard aortic segments proximal and distal to the band. Immunohistochemistry is performed using the following antibodies:VCAM-1, RAM11, CD11b, and factor VIII. In rabbits that did not receive the agent, hypertensive regions of the aorta proximal to the stenosis, monocyte adhesion and endothelial VCAM-1 expression are increased, with intimal thickening and accumulation of macrophage. In agent-treated rabbits, monocyte adhesion and endothelial VCAM-1 expression, intimal thickening and accumulation of macrophage are decreased relative to non-agent-treated rabbits. Thus, agents of the invention (e.g., CRD-$Leu_4Ile_{11}Cys_{13}$-peptide 3 (3-13)[MCP-1], NR58,4, Y-II, and L-II) may be useful to ameliorate the vascular remodeling which accompanies, and may cause, human hypertension. It is preferred, however, that agents of the invention that are used to treat hypertension have little or no adrenoreceptor binding. Thus, preferred agents for treating hypertension may exclude compounds of formula (XIV).

Basophil-mediated Asthma is a disease characterized by hyper-reactive airways and chronic inflammation resulting from an influx of many cell types and inflammatory mediators. The interaction and causal effects of all the inflammatory mediators in asthma is not entirely understood. MCP-1 can play a role in asthma through several different effector functions such as: monocyte recruitment, basophil recruitment, lymphocyte recruitment, monocyte activation or by triggering the release of histamine from basophils or resident mast cells (Bischoff et al., J. Exp. Med., 175(5), 1271

(1992)). Inhibition of these processes are likely to reduce the severity of the disease. Allergic diseases, like asthma, are manifested through a complex interaction of inflammatory mediators including monocytes/macrophages, lymphocytes and histamine release from mast cells and basophils.

A preferred mode for administration of a therapeutic agent of the invention to treat or inhibit the symptoms associated with asthma is by inhalation. As red blood cells are not normally present in the respiratory tract, the DARC specificity of the therapeutic agent is less important for administration to the respiratory tract than for other modes of administration.

Endotoxemia. Endotoxemia is an acute systemic illness often mediated by LPS, a major component in the cell wall of gram-negative bacteria. LPS stimulates the release of proinflammatory cytokines. MCP-1 and MCP-2 are expressed in endotoxemia and exert their effect by recruiting leukocytes to target organs. The intraperitoneal administration of recombinant murine MCP-1 to LPS-challenged mice protected them from endotoxic lethality (Zisman et al., J. Clin. Invest., 99, 2832 (1997)). Thus, preferred peptides for use in this embodiment of the invention are MCP-1 and MCP-2 peptides.

Myocardial Infarction/Acute Ischemia. Myocardial infarction is the result of acute closure of a coronary vessel usually due to thrombosis secondary to rupture of an atherosclerotic plaque. The damage to the adjacent myocardium and resultant heart failure is secondary to the period of ischemia and the damage caused during the reperfusion period. Reperfusion injuries are associated with increased oxygen free radicals and inflammatory mediators. MCP-1 is upregulated during the reperfusion period and is a key inflammatory mediator (Kumar et al., Circulation, 90, 1427 (1994); Kumar et al., Circulation, 95,693 (1997)). Inhibition of MCP-1 and the resultant inflammatory input may decease damage to the myocardium during recovery and reduce the incidence of heart failure.

Rheumatoid Arthritis. Rheumatoid arthritis is a multi-systemic inflammatory disease involving primarily the joints but also the skin, blood vessels, heart, lung and muscle. The characteristic pathology of rheumatoid arthritis involves the accumulation of non-suppurative inflammatory cell infiltrate consisting of macrophages and lymphocytes within the joint. MCP-1 is produced by both synovial cells and infiltrating monocyte/macrophages in rheumatoid arthritis and is thought to contribute to the accumulation of inflammatory cells within the joint. Native MCP-1 and an antagonist of MCP-1 (residues 9-76 of native MCP-1) have been assessed in the MRL-lpr model of chronic arthritis. Treatment with the antagonist MCP-1 (9-76) but not native MCP-1 resulted in a reduction of the symptoms and histopathology of chronic arthritis in this model (Gong et al., J. Exp Med., 186, 131 (1997); Plater-Zyberk et al., Immune. Lett., 57,117 (1997); Wilder, Clin- Rheumat., 10, 259 (1996)). Thus, peptide 3, its variants, analogs and derivatives may be especially useful to treat or prevent rheumatoid arthritis.

Contraception. Knockout mice for the CXCR4 chemokine receptor exhibit embryonic lethality. Agents of the invention have been identified which block the CXCR4 receptor (see Example 5) and other chemokine receptors. Thus, the agents of the invention may be useful in inducing abortion or providing contraception. Blockade of the CXCR4 receptor could provide an alternative to traditional contraceptives and could be used post-coitus.

## IV. Dosages, Formulations and Routes of Administration of the Agents of the Invention

The therapeutic agents of the invention, including a compound of formula (I)-(XV) and (XIX), including their salts, are preferably administered so as to achieve serum levels of about 0.01 pM to about 100 nM, more preferably at doses of about 0.01 pM to about 5 nM, and even more preferably at doses of about 0.1 pM to about 2 nM, of the therapeutic agent. To achieve these levels, the agent may be administered at dosages of at least about 0.01 to about 100 mg/kg, more preferably about 0.1 to about 50 mg/kg, and even more preferably about 0.1 to about 30 mg/kg, of body weight, although other dosages may provide beneficial results. The amount administered will vary depending on various factors including, but not limited to, the agent chosen, the disease, whether prevention or treatment is to be achieved, and if the agent is modified for bioavailability and *in vivo* stability.

Administration of sense or antisense nucleic acid molecule may be accomplished through the introduction of cells transformed with an expression cassette comprising the nucleic acid molecule (see, for example, WO 93/02556) or the administration of the nucleic acid molecule (see, for example, Felgner et al., U.S. Patent No. 5,580,859, Pardoll et al., Immunity 3, 165 (1995); Stevenson et al., Immunol. Rev., 145, 211 (1995); Molling, J. Mol, Med., 75, 242 (1997); Donnelly et al., Ann. N.Y. Acad. Sci., 772, 40 (1995); Yang et al., Mol. Med. Today, 2,476 (1996); Abdallah et al., Biol. Cell, 85, 1 (1995)). Pharmaceutical formulations, dosages and routes of administration for nucleic acids are generally disclosed, for example, in Felgner et al., *supra.*

The amount of therapeutic agent administered is selected to treat a particular indication. The therapeutic agents of the invention are also amenable to chronic use for prophylactic purposes, preferably by systemic administration.

Administration of the therapeutic agents in accordance with the present invention may be continuous or intermittent, depending, for example, upon the recipient's physiological condition, whether the purpose of the administration is therapeutic or prophylactic, and other factors known to skilled practitioners. The administration of the agents of the invention may be essentially continuous over a preselected period of time or may be in a series of spaced doses. Both

local and systemic administration is contemplated.

One or more suitable unit dosage forms comprising the therapeutic agents of the invention, which, as discussed below, may optionally be formulated for sustained release, can be administered by a variety of routes including oral, or parenteral, including by rectal, buccal, vaginal and sublingual, transdermal, subcutaneous, intravenous, intramuscular, intraperitoneal, intrathoracic, intrapulmonary and intranasal routes. The formulations may, where appropriate, be conveniently presented in discrete unit dosage forms and may be prepared by any of the methods well known to pharmacy. Such methods may include the step of bringing into association the therapeutic agent with liquid carriers, solid matrices, semi-solid carriers, finely divided solid carriers or combinations thereof, and then, if necessary, introducing or shaping the product into the desired delivery system.

When the therapeutic agents of the invention are prepared for oral administration, they are preferably combined with a pharmaceutically acceptable carrier, diluent or excipient to form a pharmaceutical formulation, or unit dosage form. The total active ingredients in such formulations comprise from 0.1 to 99.9% by weight of the formulation. By "pharmaceutically acceptable" it is meant the carrier, diluent, excipient, and/or salt must be compatible with the other ingredients of the formulation, and not deleterious to the recipient thereof. The active ingredient for oral administration may be present as a powder or as granules; as a solution, a suspension or an emulsion; or in achievable base such as a synthetic resin for ingestion of the active ingredients from a chewing gum. The active ingredient may also be presented as a bolus, electuary or paste.

Formulations suitable for vaginal administration may be presented as pessaries, tampons, creams, gels, pastes, douches, lubricants, foams or sprays containing, in addition to the active ingredient, such carriers as are known in the art to be appropriate. Formulations suitable for rectal administration may be presented as suppositories.

Pharmaceutical formulations containing the therapeutic agents of the invention can be prepared by procedures known in the art using well known and readily available ingredients. For example, the agent can be formulated with common excipients, diluents, or carriers, and formed into tablets, capsules, suspensions, powders, and the like. Examples of excipients, diluents, and carriers that are suitable for such formulations include the following fillers and extenders such as starch, sugars, mannitol, and silicic derivatives; binding agents such as carboxymethyl cellulose, HPMC and other cellulose derivatives, alginates, gelatin, and polyvinyl-pyrrolidone; moisturizing agents such as glycerol; disintegrating agents such as calcium carbonate and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonite; and lubricants such as talc, calcium and magnesium stearate, and solid polyethyl glycols.

For example, tablets or caplets containing the agents of the invention can include buffering agents such as calcium carbonate, magnesium oxide and magnesium carbonate. Caplets and tablets can also include inactive ingredients such as cellulose, pregelatinized starch, silicon dioxide, hydroxy propyl methyl cellulose, magnesium stearate, microcrystalline cellulose, starch, talc, titanium dioxide, benzoic acid, citric acid, corn starch, mineral oil, polypropylene glycol, sodium phosphate, and zinc stearate, and the like. Hard or soft gelatin capsules containing an agent of the invention can contain inactive ingredients such as gelatin, microcrystalline cellulose, sodium lauryl sulfate, starch, talc, and titanium dioxide, and the like, as well as liquid vehicles such as polyethylene glycols (PEGs) and vegetable oil. Moreover, enteric coated caplets or tablets of an agent of the invention are designed to resist disintegration in the stomach and dissolve in the more neutral to alkaline environment of the duodenum.

The therapeutic agents of the invention can also be formulated as elixirs or solutions for convenient oral administration or as solutions appropriate for parenteral administration, for instance by intramuscular, subcutaneous or intravenous routes.

The pharmaceutical formulations of the therapeutic agents of the invention can also take the form of an aqueous or anhydrous solution or dispersion, or alternatively the form of an emulsion or suspension.

Thus, the therapeutic agent may be formulated for parenteral administration (e.g., by injection, for example, bolus injection or continuous infusion) and may be presented in unit dose form in ampules, pre-filled syringes, small volume infusion containers or in multi-dose containers with an added preservative. The active ingredients may take such forms as suspensions, solutions, or emulsions in oily or aqueous vehicles, and may contain formulatory agents such as suspending, stabilizing and/or dispersing agents. Alternatively, the active ingredients may be in powder form, obtained by aseptic isolation of sterile solid or by lyophilization from solution, for constitution with a suitable vehicle, e.g., sterile, pyrogen-free water, before use.

These formulations can contain pharmaceutically acceptable vehicles and adjuvants which are well known in the prior art. It is possible, for example, to prepare solutions using one or more organic solvent(s) that is/are acceptable from the physiological standpoint, chosen, in addition to water, from solvents such as acetone, ethanol, isopropyl alcohol, glycol ethers such as the products sold under the name "Dowanol", polyglycols and polyethylene glycols, $C_1$-$C_4$ alkyl esters of short-chain acids, preferably ethyl or isopropyl lactate, fatty acid triglycerides such as the products marketed under the name "Miglyol", isopropyl myristate, animal, mineral and vegetable oils and polysiloxanes.

The compositions according to the invention can also contain thickening agents such as cellulose and/or

cellulose derivatives. They can also contain gums such as xanthan, guar or carbo gum or gum arabic, or alternatively polyethylene glycols, bentones and montmorillonites, and the like.

It is possible to add, if necessary, an adjuvant chosen from antioxidants, surfactants, other preservatives, film-forming, keratolytic or comedolytic agents, perfumes and colorings. Also, other active ingredients may be added, whether for the conditions described or some other condition.

For example, among antioxidants, t-butylhydroquinone, butylated hydroxyanisole, butylated hydroxytoluene and $\alpha$-tocopherol and its derivatives may be mentioned. The galenical forms chiefly conditioned for topical application take the form of creams, milks, gels, dispersion or microemulsions, lotions thickened to a greater or lesser extent, impregnated pads, ointments or sticks, or alternatively the form of aerosol formulations in spray or foam form or alternatively in the form of a cake of soap.

Additionally, the agents are well suited to formulation as sustained release dosage forms and the like. The formulations can be so constituted that they release

the active ingredient only or preferably in a particular part of the intestinal or respiratory tract, possibly over a period of time. The coatings, envelopes, and protective matrices may be made, for example, from polymeric substances, such as polylactide-glycolates, liposomes, microemulsions, microparticles, nanoparticles, or waxes. These coatings, envelopes, and protective matrices are useful to coat indwelling devices, e.g., stents, catheters, peritoneal dialysis tubing, and the like.

The therapeutic agents of the invention can be delivered via patches for transdermal administration. See U.S. Patent No. 5,560,922 for examples of patches suitable for transdermal delivery of a therapeutic agent. Patches for transdermal delivery can comprise a backing layer and a polymer matrix which has dispersed or dissolved therein a therapeutic agent, along with one or more skin permeation enhancers. The backing layer can be made of any suitable material which is impermeable to the therapeutic agent. The backing layer serves as a protective cover for the matrix layer and provides also a support function. The backing can be formed so that it is essentially the same size layer as the polymer matrix or it can be of larger dimension so that it can extend beyond the side of the polymer matrix or overlay the side or sides of the polymer matrix and then can extend outwardly in a manner that the surface of the extension of the backing layer can be the base for an adhesive means. Alternatively, the polymer matrix can contain, or be formulated of, an adhesive polymer, such as polyacrylate or acrylate/vinyl acetate copolymer. For long-term applications it might be desirable to use microporous and/or breathable backing laminates, so hydration or maceration of the skin can be minimized.

Examples of materials suitable for making the backing layer are films of high and low density polyethylene, polypropylene, polyurethane, polyvinylchloride, polyesters such as poly(ethylene phthalate), metal foils, metal foil laminates of such suitable polymer films, and the like. Preferably, the materials used for the backing layer are laminates of such polymer films with a metal foil such as aluminum foil. In such laminates, a polymer film of the laminate will usually be in contact with the adhesive polymer matrix.

The backing layer can be any appropriate thickness which will provide the desired protective and support functions. A suitable thickness will be from about 10 to about 200 microns.

Generally, those polymers used to form the biologically acceptable adhesive polymer layer are those capable of forming shaped bodies, thin walls or coatings through which therapeutic agents can pass at a controlled rate. Suitable polymers are biologically and pharmaceutically compatible, nonallergenic and insoluble in and compatible with body fluids or tissues with which the device is contacted. The use of soluble polymers is to be avoided since dissolution or erosion of the matrix by skin moisture would affect the release rate of the therapeutic agents as well as the capability of the dosage unit to remain in place for convenience of removal.

Exemplary materials for fabricating the adhesive polymer layer include polyethylene, polypropylene, polyurethane, ethylene/propylene copolymers, ethylene/ethylacrylate copolymers, ethylene/vinyl acetate copolymers, silicone elastomers, especially the medical-grade polydimethylsiloxanes, neoprene rubber, polyisobutylene, polyacrylates, chlorinated polyethylene, polyvinyl chloride, vinyl chloride-vinyl acetate copolymer, crosslinked polymethacrylate polymers (hydrogel), polyvinylidene chloride, poly(ethylene terephthalate), butyl rubber, epichlorohydrin rubbers, ethylenvinyl alcohol copolymers, ethylene-vinyloxyethanol copolymers; silicone copolymers, for example, polysiloxane-polycarbonate copolymers, polysiloxanepolyethylene oxide copolymers, polysiloxane-polymethacrylate copolymers, polysiloxane-alkylene copolymers

(e.g., polysiloxane-ethylene copolymers), polysiloxane-alkylenesilane copolymers (e.g., polysiloxane-ethylenesilane copolymers), and the like; cellulose polymers, for example methyl or ethyl cellulose, hydroxy propyl methyl cellulose, and cellulose esters; polycarbonates; polytetrafluoroethylene; and the like.

Preferably, a biologically acceptable adhesive polymer matrix should be selected from polymers with glass transition temperatures below room temperature. The polymer may, but need not necessarily, have a degree of crystallinity at room

temperature. Cross-linking monomeric units or sites can be incorporated into such polymers. For example, cross-linking monomers can be incorporated into polyacrylate polymers, which provide sites for cross-linking the matrix after dispersing

the therapeutic agent into the polymer. Known cross-linking monomers for polyacrylate polymers include polymethacrylic esters of polyols such as butylene diacrylate and dimethacrylate, trimethylol propane trimethacrylate and the like. Other monomers which provide such sites include allyl acrylate, allyl methacrylate, diallyl maleate and the like.

Preferably, a plasticizer and/or humectant is dispersed within the adhesive polymer matrix. Water-soluble polyols are generally suitable for this purpose. Incorporation of a humectant in the formulation allows the dosage unit to absorb moisture on the surface of skin which in turn helps to reduce skin irritation and to prevent the adhesive polymer layer of the delivery system from failing.

Therapeutic agents released from a transdermal delivery system must be capable of penetrating each layer of skin. In order to increase the rate of permeation of a therapeutic agent, a transdermal drug delivery system must be able in particular to increase the permeability of the outermost layer of skin, the stratum corneum, which provides the most resistance to the penetration of molecules. The fabrication of patches for transdermal delivery of therapeutic agents is well known to the art.

For administration to the upper (nasal) or lower respiratory tract by inhalation, the therapeutic agents of the invention are conveniently delivered from an insufflator, nebulizer or a pressurized pack or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount.

Alternatively, for administration by inhalation or insufflation, the composition may take the form of a dry powder, for example, a powder mix of the therapeutic agent and a suitable powder base such as lactose or starch. The powder composition may be presented in unit dosage form in, for example, capsules or cartridges, or, e.g., gelatine or blister packs from which the powder may be administered with the aid of an inhalator, insufflator or a metered-dose inhaler.

For intra-nasal administration, the therapeutic agent may be administered via nose drops, a liquid spray, such as via a plastic bottle atomizer or metered-dose inhaler. Typical of atomizers are the Mistometer (Wintrop) and the Medihaler (Riker).

The local delivery of the therapeutic agents of the invention can also be by a variety of techniques which administer the agent at or near the site of disease. Examples of site-specific or targeted local delivery techniques are not intended to be limiting but to be illustrative of the techniques available. Examples include local delivery catheters, such as an infusion or indwelling catheter, e.g., a needle infusion catheter, shunts and stents or other implantable devices, site specific carriers, direct injection, or direct applications.

For topical administration, the therapeutic agents may be formulated as is known in the art for direct application to a target area. Conventional forms for this purpose include wound dressings, coated bandages or other polymer coverings,

ointments, creams, lotions, pastes, jellies, sprays, and aerosols, as well as in toothpaste and mouthwash, or by other suitable forms, e.g., via a coated condom. Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents. The active ingredients can also be delivered via iontophoresis, e.g., as disclosed in U.S. Patent Nos. 4,140,122; 4,383,529; or 4,051,842. The percent by weight of a therapeutic agent of the invention present in a topical formulation will depend on various factors, but generally will be from 0.01% to 95% of the total weight of the formulation, and typically 0.1-25% by weight.

When desired, the above-described formulations can be adapted to give sustained release of the active ingredient employed, e.g., by combination with certain hydrophilic polymer matrices, e.g., comprising natural gels, synthetic polymer gels or mixtures thereof.

Drops, such as eye drops or nose drops, may be formulated with an aqueous or non-aqueous base also comprising one or more dispersing agents, solubilizing agents or suspending agents. Liquid sprays are conveniently delivered from pressurized packs. Drops can be delivered via a simple eye dropper-capped bottle, or via a plastic bottle adapted to deliver liquid contents dropwise, via a specially shaped closure.

The therapeutic agent may further be formulated for topical administration in the mouth or throat. For example, the active ingredients may be formulated as a lozenge further comprising a flavored base, usually sucrose and acacia or tragacanth; pastilles comprising the composition in an inert base such as gelatin and glycerin or sucrose and acacia; mouthwashes comprising the composition of the present invention in a suitable liquid carrier; and pastes and gels, e.g., toothpastes or gels, comprising the composition of the invention.

The formulations and compositions described herein may also contain other ingredients such as antimicrobial agents, or preservatives. Furthermore, the active ingredients may also be used in combination with other therapeutic agents, for example, oral contraceptives, bronchodilators, anti-viral agents e.g., ddI, ddC, AZT, protease inhibitors, or any combination thereof, steroids, leukotriene inhibitors, cyclosporin A, methotrexate, azathioprene, anti-IgE, Enbrel, Xenapax and the like.

Sustained Released Dosage Forms

Sustained release dosage forms of the invention may comprise microparticles and/or nanoparticles having a therapeutic agent dispersed therein. The therapeutic dosage forms of this aspect of the present invention may be of any configuration suitable for sustained release. Preferred sustained release therapeutic dosage forms exhibit one or more of the following characteristics:

- microparticles (e.g., from about 0.5 micrometers to about 100 micrometers in diameter, with about 0.5 to about 2 micrometers more preferred; or from about 0.01 micrometers to about 200 micrometers in diameter, preferably from about 0.5 to about 50 micrometers, and more preferably from about 2 to about 15 micrometers) or nanoparticles (e.g., from about 1.0 nanometer to about 1000 nanometers in diameter, with about 50 to about 250 nanometers being more preferred; or from about 0.01 nanometer to about 1000 nanometers in diameter, preferably from about 50 to about 200 nanometers), free flowing powder structure;
- biodegradable structure designed to biodegrade over a period of time preferably between from about 0.5 to about 180 days, preferably from about 1-3 to about 150 days, or from about 3 to about 180 days, with from about 10 to about 21 days more preferred; or non-biodegradable structure to allow therapeutic agent diffusion to occur over a time period of between from about 0.5 to about 180 days, more preferably from about 30 to about 120 days; or from about 3 to about 180 days, with from about 10 to about 21 days preferred;
- biocompatible with target tissue and the local physiological environment into which the dosage form to be administered, including yielding biocompatible biodegradation products;
- facilitate a stable and reproducible dispersion of therapeutic agent therein, preferably to form a therapeutic agent-polymer matrix, with active therapeutic agent release occurring by one or both of the following routes: (1) diffusion of the therapeutic agent through the dosage form (when the therapeutic agent is soluble in the shaped polymer or polymer mixture defining the dimensions of the dosage form); or (2) release of the therapeutic agent as the dosage form biodegrades; and/or
- for targeted dosage forms, capability to have, preferably, from about 1 to about 10,000 binding protein/peptide to dosage form bonds and more preferably, a maximum of about 1 binding peptide to dosage form bond per 150 square angstroms of particle surface area. The total number of binding protein/peptide to dosage form bonds depends upon the particle size used. The binding proteins or peptides are capable of coupling to the particles of the therapeutic dosage form through covalent ligand sandwich or non-covalent modalities as set forth herein.

Nanoparticle sustained release therapeutic dosage forms are preferably biodegradable and, optionally, bind to the vascular smooth muscle cells and enter those cells, primarily by endocytosis. The biodegradation of the nanoparticles occurs over time (e.g., 30 to 120 days; or 10 to 21 days) in prelysosomic vesicles and lysosomes. Preferred larger microparticle therapeutic dosage forms of the present invention release the therapeutic agents for subsequent target cell uptake with only a few of the smaller microparticles entering the cell by phagocytosis. A practitioner in the art will appreciate that the precise mechanism by which a target cell assimilates and metabolizes a dosage form of the present invention depends on the morphology, physiology and metabolic processes of those cells. The size of the particle sustained release therapeutic dosage forms is also important with respect to the mode of cellular assimilation. For example, the smaller nanoparticles can flow with the interstitial fluid between cells and penetrate the infused tissue. The larger microparticles tend to be more easily trapped interstitially in the infused primary tissue, and thus are useful to therapeutic agents.

Preferred sustained release dosage forms of the present invention comprise biodegradable microparticles or nanoparticles. More preferably, biodegradable microparticles or nanoparticles are formed of a polymer containing matrix that biodegrades by random, nonenzymatic, hydrolytic scissioning to release therapeutic agent, thereby forming pores within the particulate structure.

Polymers derived from the condensation of alpha hydroxycarboxylic acids and related lactones are preferred for use in the present invention. A particularly preferred moiety is formed of a mixture of thermoplastic polyesters (e.g., polylactide or polyglycolide) or a copolymer of lactide and glycolide components, such as poly(lactide-co-glycolide). An exemplary structure, a random poly(DL-lactide-co-glycolide), is shown below, with the values of x and y being manipulable by a practitioner in the art to achieve desirable microparticle or nanoparticle properties.

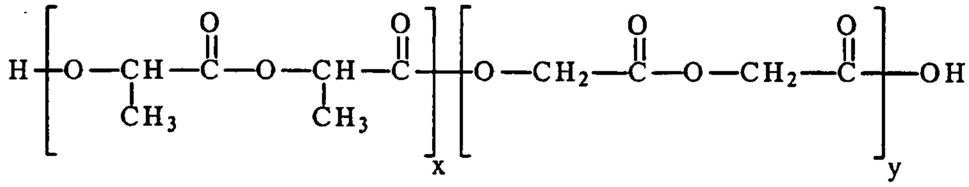

Other agents suitable for forming particulate dosage forms of the present invention include polyorthoesters and polyacetals (Polymer Letters,18:293 (1980) and polyorthocarbonates (U.S. Patent No. 4,093,709) and the like.

Preferred lactic acid/glycolic acid polymer containing matrix particles of the present invention are prepared by emulsion-based processes, that constitute modified solvent extraction processes, see, for example, processes described by Cowsar et al., "Poly(Lactide-Co-Glycolide) Microcapsules for Controlled Release of Steroids," Methods Enzymology, 112:101-116,1985 (steroid entrapment in microparticles); Eldridge et al., "Biodegradable and Biocompatible Poly(DL-Lactide-Co-Glycolide) Microspheres as an Adjuvant for Staphylococcal Enterotoxin B Toxoid Which Enhances the Level of Toxin-Neutralizing Antibodies," Infection and Immunity, 52:2978-2986, 1991 (toxoid entrapment); Cohen et al., "Controlled Delivery Systems for Proteins Based on Poly(Lactic/Glycolic Acid) Microspheres," Pharmaceutical Research, 8 (6):713-720, 1991 (enzyme entrapment); and Sanders et al., "Controlled Release of a Luteinizing Hormone-Releasing Hormone Analogue from Poly(D,L-Lactide-Co-Glycolide) Microspheres," J. Pharmaceutical Science, 73(9):1294-1297, 1984 (peptide entrapment).

In general, the procedure for forming particle dosage forms of the present invention involves dissolving the polymer in a halogenated hydrocarbon solvent, dispersing a therapeutic agent solution (preferably aqueous) therein, and adding an additional agent that acts as a solvent for the halogenated hydrocarbon solvent but not for the polymer. The polymer precipitates out from the polymer-halogenated hydrocarbon solution onto droplets of the therapeutic agent containing solution and entraps the therapeutic agent. Preferably the therapeutic agent is substantially uniformly dispersed within the sustained release dosage form of the present invention. Following particle formation, they are washed and hardened with an organic solvent. Water washing and aqueous nonionic surfactant washing steps follow, prior to drying at room temperature under vacuum.

For biocompatibility purposes, particulate dosage forms, characterized by a therapeutic agent dispersed in the matrix of the particles, are sterilized prior to packaging, storage or administration. Sterilization may be conducted in any convenient manner therefor. For example, the particles can be irradiated with gamma radiation, provided that exposure to such radiation does not adversely impact the structure or function of the therapeutic agent dispersed in the therapeutic agent-polymer matrix or the binding protein/peptide attached thereto. If the therapeutic agent or binding protein/peptide is so adversely impacted, the particle dosage forms can be produced under sterile conditions.

Release of the therapeutic agent from the particle dosage forms of the present invention can occur as a result of both diffusion and particle matrix erosion. The biodegradation rate directly effects the kinetics of therapeutic agent release. The biodegradation rate is regulable by alteration of the composition or structure of the sustained release dosage form. For example, alteration of the lactide/glycolide ratio in preferred dosage forms of the present invention can be conducted, as described by Tice et al., "Biodegradable Controlled-Release Parenteral Systems," Pharmaceutical Technology, pp. 26-35, 1984; by inclusion of agents that alter the rate of polymer hydrolysis, such as citric acid and sodium carbonate, as described by Kent et al., "Microencapsulation of Water Soluble Active Polypeptides," U.S. Patent No. 4,675,189; by altering the loading of therapeutic agent in the lactide/glycolide polymer, the degradation rate being inversely proportional to the amount of therapeutic agent contained therein, by judicious selection of an appropriate analog of a common family of therapeutic agents that exhibit different potencies so as to alter said core loadings; and by variation of particle size, as described by Beck et al., "Poly(DL-Lactide-Co-Glycolide)/Norethisterone Microcapsules: An Injectable Biodegradable Contraceptive," Biol. Reprod., 28:186-195, 1983, or the like. All of the aforementioned methods of regulating biodegradation rate influence the intrinsic viscosity of the polymer containing matrix, thereby altering the hydration rate thereof.

The preferred lactide/glycolide structure is biocompatible with the mammalian physiological environment. Also, these preferred sustained release dosage forms have the advantage that biodegradation thereof forms lactic acid and glycolic acid, both normal metabolic products of mammals.

Functional groups required for binding of the protein/peptide to the particle dosage form are optionally included in or on the particle matrix and are attached to the non-degradable or biodegradable polymeric units. Functional groups that are useful for this purpose include those that are reactive with peptides, e.g., carboxyl groups, amine groups, sulfhydryl groups and the like. Preferred binding enhancement moieties include the terminal carboxyl groups of the preferred (lactide-glycolide) polymer containing matrix or the like.

V. Detection of the Agents of the Invention in Physiological Fluid

Analysis of peptide 3 in blood and urine was performed on a semipermeable surface (SPS) HPLC column (restricted access media). Serum or other protein-containing samples can be injected directly onto an SPS column (e.g., SPS-C18 with a column size of 4.6 mm x 250 mm; using a mobile phase: A: 0.1% TFA in water, B: 0.1% TFA in acetonitrile: 0-5 min - 5% B, 5-30 min - 60% B, 30-40 min - 5% B detector; 215 nm). The outer phase of the column forms a semipermeable surface that prevents large molecules from reaching the inner phase. Small molecules penetrate the semipermeable surface and interact with the inner reversed phase.

Standards of peptide 3 (range of 1.5 μg/ml to 1000 μg/ml) in PBS were injected and a standard curve was created. 20 μl of serum and urine were injected and the areas under the peptide 3 peaks were obtained. The concentration was then calculated from the standard curve. This method can detect at least about 20 μg/ml of a peptide in physiological fluid samples.

The peptides of the invention may also be detected and/or quantitated in physiological fluid, e.g., urine or serum, using LC-MS. Using electrospray ionization (ESI), an LCQ ion trap mass spectrometer (Thermoquest Finnigan, San Jose, CA) is operated in the positive ion mode with the heated capillary set to 200˚C, and 4.25 kV applied to the electrospray needle. The sheath gas flow rate is set to 55 units, while the auxiliary gas is turned off. The data are acquired with a maximum ion time of 500 ms and 1 total microscan. The analysis is performed using a full scan MS with *m*/*z* [335-1400] and/or a full scan MS/MS with m/z [280-1500] generated by fragmentation of the doubly charged ion with *m*/*z* 680.1 set to an isolation width of 2.0 amu and a collisional energy of 28%.

HPLC grade solvents ('Baker Analyzed' from J.T. Baker, Phillipsburg, NJ), and formic acid (99%, ACS, Sigma, St. Louis, MO) were used. A Zorbax Eclipse XDB-C18 3.0 x 150 mm, 3.5 micron ('Zorbax', Hewlett-Packard, Palo Alto, CA) equipped with a 'SafeGuard' guard column containing a C18 cartridge (Phenomenex, Torrence, CA) is operated at a column temperature of 35˚C and a maximum pressure of 400 bar. The flow rate is set to 0.500 mL/min. An HP1100 binary system (Hewlett-Packard, Palo Alto, CA) generates a 20 minute gradient starting with 0% B (acetonitrile) and 100% A (water/0.1% formic acid) at 0.0 to 3.0 minutes, then ramps up to 15% B at 3.5 minutes and runs isocratically until 12.0 minutes. This elution step is followed by a high organic wash step ramping up to 95% B from 12.0 to 14.0 minutes while increasing the flow rate to 0.800 mL/min at 14.1 minutes. At 16.0 to 16.5 minutes the system is resetting to 0% B and re-equilibrates for 3.5 minutes at 0.800 mL/min. Alternatively, a 15 minute gradient is generated starting with 98% A (water/0.1% formic acid (acetonitrile)) at 0-2.5 minutes, then ramps to 17% B at 2.5 minutes up to 11 minutes, then ramps to 95% B at 11 minutes. The flow rate is increased to 0.800 mL/minute at 11.1 minutes. At 13.2 minutes, the system resets to 1% B until 15 minutes. The LCQ divert valve is set to direct the flow to the detector between 8 and 11 minutes. 10 μl of each sample is injected using an HP1100 autosampler (Hewlett-Packard, Palo Alto, CA). Under the first set of conditions, CRD-$Leu_4Ile_{11}Cys_{13}$ peptide 3(3-12)[MCP-1 elutes at a retention time of 9.69 minutes.

Under the second set, CRD-$Leu_4Ile_{11}Cys_{13}$ peptide 3(3-12)[MCP-1] (triacetate salt) eluted at a retention time of 8.3 minutes while CRD-L-$Leu_4Ile_{11}$,$Cys_{13}$ peptide 3(3-12)[MCP-1] (triacetate salt) eluted at 8.9 minutes. The standard analytes are prepared by adding different levels of CRD-$Leu_4Ile_{11}Cys_{13}$ peptide 3(3-12)[MCP-1] and a fixed amount of CRD-L-$Leu_4Ile_{11}Cys_{13}$ peptide 3(3-12)[MCP-1] triacetate salt to rat urine filtrated through a 'Sterile Acrodisc 13 0.2 μm' filter (Gelman Sciences, Prod. # 4454) or serum. The following levels of free base CRD-$Leu_4Ile_{11}Cys_{13}$ peptide 3(3-12) [MCP-1] in rat urine were each injected three times and processed using LCQuan to generate a standard curve: 0.05 μg/mL, 0.1 μg/mL, 0.5 μg/mL, 1.0 μg/mL, 2.0 μg/mL, 3.0 μg/mL, 5.0 μg/mL, 10 μg/mL, 20 μg/mL, 30 μg/mL, and 50 μg/mL. Rat urine samples are analyzed after filtration as described above.

Rat serum samples are analyzed as described above, for fast screens without prior purification, otherwise after liquid/liquid extraction with ice-cold acetonitrile followed by solvent removal in a speed vac over night and reconstitution in water/formic acid (0.1%) or HPLC grade water. For example, 400 μl of ice cold acetonitrile is mixed with serum (about 100 μl) and centrifuged for 10 minutes at 10,000 rpm. 400 μl of supernatants were transferred into fresh tubes, dried under vacuum and reconstituted in 80 μl of HPLC grade water. Samples were spun for 10 minutes at 10,000 rpm and 70 μl transferred into 100 μl glass inserts in 2 mL HPLC vials for LC-MS analysis. An internal standard such as deuterated CRD-$Leu_4Ile_{11}Cys_{13}$ peptide 3(3-12)[MCP-1] can be added to account for losses during sample preparation. The standard curves are prepared accordingly.

The invention will be further described by, but is not limited to, the following examples.

**Examples 1** Identification and Characterization of Pan-Chemokine Peptide Inhibitors

Both human and mouse MCP-1 bind to and positively signal through the human chemokine receptor. Thus, regions of homology between human and murine MCP-1 may represent regions that are involved in binding and/or signaling. Based on an alignment of human and murine MCP-1 sequences, three regions in MCP-1 were identified which were conserved between all the species examined. Three peptides (12-15mers) were prepared which had the greatest sequence homology between the human and mouse MCP-1 sequences (Table 1), and were purified to >95% purity.

These peptides were screened for their ability to inhibit hMCP-1 induced THP-1 migration. A similar analysis was done for a non-chemokine, i.e., TGF-beta. The sequences of *Xenopus laevis* TGF-betal and TGF-beta3 and human TGF-betal and TGF-beta3 were compared, and 3 regions (each 10mer) of perfect homology were identified.

For this assay, THP-1 cells were maintained at a density of $4 \times 10^5$ cells per ml in RPMI-1640 supplemented with 10% fetal calf serum + 20 μM 2-mercaptoethanol. Chemotaxis was induced in a 96-well disposable chemotaxis chamber fitted with a 5 μM polycarbonate filter (PVP free, ChemoTX, Neuroprobe Inc., Cabin John). Twenty-nine μl of chemoattractant (recombinant human chemokine; 50 ng/ml, i.e., 5.9 nM) or control (100 ng/ml TGFβ) was added to the lower compartment of each well. The framed filter was aligned with the holes in the corner of the filter frame and placed over the wells.

Five $\times 10^4$ THP-1 cells in 25 μl of RPMI-1640 were added to the upper compartment. Peptides were dissolved in Milli Q water and then serially diluted in culture medium. In most cases, the serially diluted peptides were added to the upper compartment of the chemotaxis chamber. The chamber was incubated at 37˚C in a humidified atmosphere of 5% $CO_2$ for 4 hours.

After incubation, the cells were gently removed from the top of the filter with a pipette, 20 μl of 20 mM EDTA in PBS was added into each top well, and the mixture was incubated for 20 minutes at 4˚C. The filter was then carefully flushed with media using a gentle flow, and removed. A standard curve was prepared to accurately quantify the number of THP-1 cells that had migrated. The curve was based on a two-fold dilution series of THP-1 cells (top standard 100,000 cells in 29 μl). Cells which had migrated, and in separate wells, the cells in the standards, were stained with 3 μl of MTT stock solution which was added directly into each well (5 mg/ml in RPMI 1640 without phenol red, Sigma Chemical Co.) and incubated at 37˚C for 4 hours. The media was carefully aspirated from each well, and the converted dye was solubilized by 20 μl of DMSO. Absorbance of converted dye was measured at a wavelength of 595 nM using an ELISA plate reader. The number of cells that had migrated in each well was determined by interpolation of the standard curve.

Peptide 1[MCP-1] (see Table 1; SEQ ID NO:2), i.e., the N-terminal peptide of human MCP-1, was only weakly active in the migration inhibition assay ($ED_{50}$ > 100 μM; 10% inhibition at 100 μM, p = 0.27). Peptide 2[MCP-1] (Table 1; SEQ ID NO:3) was also a weak inhibitor of chemokine-induced migration ($ED_{50}$ > 100 μM; 19% inhibition at 100 μM, p = 0.09). Thus, in the presence of a strong agonist, i.e., MCP-1, peptide 2[MCP-1] having SEQ ID NO:3, a weak agonist, displaces MCP-1 from its receptor. However, in the absence of a strong agonist, i.e., MCP-1, peptide 2[MCP-1] exhibited weak agonist properties, i.e., peptide 2[MCP-1] stimulated chemotaxis. Surprisingly, peptide 2(1-15)[SDF1α] had potent pan-chemokine antagonist properties.

In contrast, peptide 3(1-12)[MCP-1] (Table 1; SEQ ID NO:1) was a highly effective inhibitor of MCP-1 induced THP-1 migration with a dose giving 50% inhibition ($ED_{50}$) of 8 ± 1 μM (n=4). A typical dose response curve is shown in Figure 2. At concentrations above 50 μM, peptide 3(1-12)[MCP-1] having SEQ ID NO:1 abolished all of the MCP-1 induced THP-1 migration.

**TABLE 1 Alignment of Chemokine Sequences**

```
AQPDAINAPV TCCYNFTNRK ISVQRLASYR RITSSKCPKE AVIFKTIVAK EICADPKQKW VQDSMDHLDK QTQTPKT hMCP1 (SEQ ID NO:16)
*****.***. **** **  .  *    ** **. *****.**** ** * *  . . *.*****  * **      **.
AQPDAVNAPL TCCYSPTSKM IPMSRLESYK RITSSRCPKE AVVFVTKLKR EVCADPKKEW VQTYIKNLDR NQMR.....mMCP1 (SEQ ID NO:26)
__________                  ____________           __________
 Peptide 1                   Peptide 2              Peptide 3

                                                  GICADPKQKWVQ  rabbit MCP1
                                                  EICADPNKEWVQ  rat MCP1
                                                  EICADPNKEWVQ  dog MCP1
                                                  ELCADPKQKWVQ  bovine MCP1
                                                  EVCADPTQKWVQ  guinea pig MCP1
                                                  EICAEPKQKWVQ  pig MCP1

AQPDSVSIPI TCCPNVINRK IPIQRLESYR RITNIQCPKE AVIFKTKRGK EVCADPKERW VRDSNKHLDQ IFQNLKP  hMCP2 (SEQ ID NO:17)
AQPVGINTST TCCYRFINKK IPKQRLESYR RTTSSHCPRE AVIFKTKLDK EICADPTQKW VQDFMKHLDK KTQTPKL  hMCP3 (SEQ ID NO:18)

SASLAADTPT ACCFSYTSRQ IPQNFIADYF E-TSSQCSKP GVIFLTKRSR QVCADPSEEW VQKYVSDLEL SA      hMIP1a (SEQ ID NO:19)
SAPMGSDPPT ACCFSYTARK LPRNFVVDYY E-TSSLCSQP AVVFQTKRSK QVCADPSESW VQEYVYDLEL N       hMIP1b (SEQ ID NO:20)

SAPMGSDPPT ACCFSYTARK LPRNFVVDYY E-TSSLCSQP AVVFQTKRSK QVCADPSESW VQEYVYDLEL N       RANTES (SEQ ID NO:21)

    HPGIPS ACCYNFTNKK ISFQRLKSYK IITSSKCPQT AIVFEIKPDK MICADPKxxW VQDAKKYLDQ ISQxTKP Eotaxin (SEQ ID NO:25)

LPRSAKELRC QCIKTYSKPF HPKFIKELRV IESGPHCANT EIIVRLSDGR ELCLDPKENW VQRVEKFLKR AENS    hIL-8 (SEQ ID NO:23)

  GKPVSLSY RCPCRFFESH IARANVKHLK ILNTPNCALQ IVARLKNNNR QVCIDPKLKW IQEYEKALNK         hSDF1b (SEQ ID NO:22)
                                                    CALDTVGW VQ                     I-309
.......... .C...F.... I......... ..T...C... AVI......K .VCADP...W VQ.....L.. .....CONSENSUS
```

To determine whether the peptides were MCP-1 receptor antagonists, the peptides were introduced with the chemokine in the lower compartment (as opposed to with the cells in the upper compartment in the experiments described above; in the trans-well THP-1 migration assay. Under these conditions, peptide 1 [MCP-1] having SEQ ID NO:2 was a more efficient inhibitor of MCP-1 induced chemokine migration that it had been when it was incubated with the cells, inhibiting 48% of the MCP-1 induced migration at 100 μM compared to 10% inhibition when peptide 1[MCP-1] (SEQ ID

NO:2) was incubated with the cells. This result is consistent with published reports which show that peptide 1 [MCP-1] (SEQ ID NO:2) and its derivatives act by disrupting the MCP-1 dimer, forming inactive monomers. Peptide 1 [MCP-1] (SEQ ID NO:2) is not, therefore, a classical receptor-level antagonist of MCP-1 function. In marked contrast, peptide 3 (1-12)[MCP-1] having SEQ ID NO: was much less effective when incubated with the chemokine than with the cells (17% inhibition at 100 μM compared with >99% inhibition), suggesting that a peptide having SEQ ID NO:1 inhibits MCP-1 induced migration by directly interacting with the cells, rather than by binding to the chemokine ligand. To confirm this observation, the binding affinity of an N-terminally biotinylated derivative of peptide 3(1-12)[MCP-I] (SEQ ID NO:1) was determined. This derivative bound to the surface of THP-1 cells with a ka of about 10 μM.

Peptide 3(1-12)[MCP-1] (SEQ ID NO:1) also inhibited other functions of MCP-1, which may be mediated by different combinations of receptors. MCP-1 has been reported to be a weak co-mitogen with 0.5% fetal calf serum for cultured smooth muscle cells. It was found that 100 μM peptide 3(1-12)[MCP-1] (SEQ ID NO:1) completely abolished the co-mitogenic effect of MCP-1 for cultured smooth muscle cells, also consistent with the hypothesis that peptide 3 (1-12)[MCP-1] (SEQ ID NO:1) is an MCP-1 receptor antagonist. The observation that peptide 3(1-12)[MCP-1] (SEQ ID NO:1) completely inhibits different responses to MCP-1 in different cell types suggests that peptide 3 may be a general antagonist of all chemokine receptors capable of binding and signaling in response to MCP-1.

To investigate the receptor specificity of peptide 3 inhibition, the $ED_{50}$ was determined for peptide 3(1-12) [MCP-1] (SEQ ID NO:1) inhibition of THP-1 migration induced by chemokines which signal through different receptors than MCP-1 receptors. Representative chemokines included a beta-chemokine ("CC"), MIP-1α and RANTES, and two alpha-chemokines ("CXC"), IL-8 and SDF-1α. Additionally, to determine the specificity ofpeptide 3(1-12)[MCP-1] (SEQ ID NO:1) for chemokine receptors, TGF-beta was selected as a migration-inducing agent unrelated to the chemokine family, and as an agent which elicits a biological activity by signaling through identified, unrelated receptors.

Peptide 3(1-12)[MCP-1] (SEQ ID NO:1) inhibited the THP-1 migration induced response to all four of the selected chemokines, with the order of potency: MIP-1α≥ MCP-1 > SDF1α ≥ IL-8 (see Table 2). In contrast, peptide 1 [MCP-1] (SEQ ID NO:2) or peptide 2(1-15)[MCP-1] (SEQ ID NO:3) did not inhibit migration in response to any of these chemokines by more than 20%, even at 100 μM (Table 2). Peptide 3 binds to THP-1 cells with an association constant of about 10 μM.

**TABLE 2**

**(a) $ED_{50}$ for inhibition of THP-1 migration**

| PEPTIDE | $ED_{50}$ (μM) versus | | | | |
|---|---|---|---|---|---|
| | MCP-1 | MIP1α | IL8 | SDF-1α | TGFβ1 |
| Peptide 1 (SEQ ID NO:2) | n.s.[b] | n.s.[b] | n.s. | n.s. | n.s. |
| Peptide 2 (SEQ ID NO:3) | n.s. | n.s. | n.s. | n.s. | n.s. |
| Peptide 3[a] (SEQ ID NO:1) | 8 ± 1 | 8 ± 1 | 14 ± 1 | 10 ± 0 | n.s. |

**(b) Extent of inhibition of THP-1 migration at 100 μM**

| PEPTIDE | % inhibition at 100 μM versus | | | | |
|---|---|---|---|---|---|
| | MCP-1 | MIP1α | IL8 | SDF-1α | TGFβ1 |
| Peptide 1 (SEQ ID NO:2) | n.s.[b] | n.s.[b] | n.s. | n.s. | n.s. |
| Peptide 2 (SEQ ID NO:3) | n.s. | n.s. | n.s. | n.s. | n.s. |
| Peptide 3 (SEQ ID NO:1) | 112 | 99 | 103 | 107 | n.s. |

**(c) Extent of inhibition of human monocyte migration at 100 μM**

| PEPTIDE | % inhibition at 100 μM versus | | | | |
|---|---|---|---|---|---|
| | MCP-1 | MIP1α | IL8 | SDF-1α | TGFβ1 |
| Peptide 1 (SEQ ID NO:2) | n.s. | n.s. | n.s. | n.s. | n.s. |
| Peptide 2 (SEQ ID NO:3) | 23 | n.s. | n.s. | n.s. | n.s. |

(continued)

| (c) Extent of inhibition of human monocyte migration at 100 μM | | | | | |
|---|---|---|---|---|---|
| PEPTIDE | % inhibition at 100 μM versus | | | | |
| | MCP-1 | MIP1α | IL8 | SDF-1α | TGFβ1 |
| Peptide 3 (SEQ ID NO:1) | 108 | 120 | 106 | 108 | n.s. |

[a] mean + SEM of at least three determinations
[b] Peptide 1 caused significant inhibition only when added to the lower compartment
n.s. = no statistically significant inhibition ($p > 0.05$)

Furthermore, peptide 3(1-12)[MCP-1] having SEQ ID NO:1 (as well as peptide 1[MCP-1] (SEQ ID NO:2) and peptide 2(1-15)[MCP-1] (SEQ ID NO:3)) did not significantly inhibit THP-1 migration induced by TGF-beta even at 100 μM. Taken together, these results demonstrated that peptide 3(1-12)[MCP-1] (SEQ ID NO:1) is a general (i.e., inhibits all chemokines tested) and specific (i.e., only inhibits chemokines) inhibitor of chemokine signaling. Although peptide 3 (1-12)[MCP-1] (SEQ ID NO:1) shows weak selectivity for CC chemokines over CXC chemokines, nevertheless, at 100 μM, peptide 3(1-12)[MCP-1] (SEQ ID NO:1) inhibits >99% of the migration induced by any of the chemokines of either chemokine family tested (Table 2). Thus, although MCP-1 signals through multiple related receptors, peptide 3(1-12) [MCP-1] (SEQ ID NO:1) blocks all of the receptors which participate in the chemotactic and mitogenic signaling pathways elicited by MCP-1.

To exclude the possibility that peptide 3(1-12)[MCP-1] (SEQ ID NO:1) was more effective on THP-1 cells than primary human monocytes, the effect of peptide 3(1-12)[MCP-1] (SEQ ID NO:1) on the chemokine-induced migration of freshly prepared peripheral blood monocytes from 3 donors was tested. Similar to the results for THP-1 cells, 100 μM of peptide 3(1-12)[MCP-1] (SEQ ID NO:1), but not peptide 1[MCP-1] (SEQ ID NO:2) or peptide 2(1-15)[MCP-1] (SEQ ID NO:3), inhibited all or almost all (>95%) of the migration induced with each of the four chemokines, but did not affect TGF-beta induced migration. Thus, peptide 3(1-12)[MCP-1] (SEQ ID NO:1) is an inhibitor of a broad range of pro-inflammatory chemokines which act on a wide range of target cells (smooth muscle cells, THP-1, Jurkat T-cell line and primary human monocytes). Note that in contrast to THP-1 cells, peptide 2(1-15)[MCP-1] (SEQ ID NO:3) inhibition of MCP-1 induced migration of primary human monocytes (20%) was statistically significant (Table 2).

**Example 2**

Characterization of Fragments and Variants of Peptide 3(1-12)[MCP-1] and Peptide 2 [MCP-1]

To determine whether a fragment of peptide 3 has biological activity and selectivity, two 6mer "half-peptides" were analyzed (Table 3): EICADP (SEQ ID NO:8), corresponding to peptide 3(1-6)[MCP-1], and KQKWVQ (SEQ ID NO:9), corresponding to peptide 3(7-12)[MCP-1]. Peptide 3(7-12)[MCP-1] (SEQ ID NO:9) was as potent an inhibitor of CC chemokine signaling as peptide 3(1-12)[MCP-1] (SEQ ID NO:1), but was noticeably more potent as an inhibitor of CXC chemokines (Table 4). In contrast, peptide 3(1-6)[MCP-1] (SEQ ID NO:8) was much less potent as an inhibitor than peptide 3(1-12)[MCP-1] (SEQ ID NO:1).

**TABLE 3**

| NAME | SEQUENCE | SOURCE |
|---|---|---|
| Peptide 1 family | | |
| Pep1 | AQPDAINAPVTCC (SEQ ID NO:2) | Residues 1-13 of mature hMCP-1 |
| Peptide 2 family | | |
| Pep2(1-15)[MCP1] | SYRRITSSKCPKEAV (SEQ ID NO:3) | Residues 28-42 of mature hMCP-1 |
| Pep2(1-15)[SDF1] | HLKILNTPNCALQIV (SEQ ID NO:4) | Residues 26-40 of mature hSDF-1β |
| Pep2(1-14)[MIP1α] | DYFETSSQCSKPGV (SEQ ID NO:5) | Residues 28-41 of mature hMIP1α |
| Pep2(1-16)[IL8] | ELRVIESGPHCANTEI (SEQ ID NO:6) | Residues 27-42 of mature hIL-8 |

(continued)

| Peptide 3 family | | |
|---|---|---|
| Pep3(1-12)[MCP-1] | EICADPKQKWVQ (SEQ ID NO:1) | Residues 50-61 of mature hMCP-1 |
| Pep3(3-12)[MCP-1] | CADPKQKWVQ (SEQ ID NO:7) | Residues 52-61 of mature hMCP-1 |
| Pep3(1-6)[MCP-1] | EICADP (SEQ ID NO:8) | Residues 50-55 of mature hMCP-1 |
| Pep3(7-12)[MCP-1] | KQKWVQ (SEQ ID NO:9) | Residues 56-61 of mature hMCP-1 |
| $Leu_4$Pep3 (1-12)[MCP-1] | EICLDPKQKWVQ (SEQ ID NO:10) | Mutant of peptide 3 |
| $Ser_7$Pep3 (1-12)[MCP-1] | EICADPSQKWVQ (SEQ ID NO:11) | Mutant of peptide 3 |
| $Ser_7Glu_8Glu_9$Pep3 (1-12)[MCP-1] | EICADPSEEWVQ (SEQ ID NO:12) | Residues 50-61 of mature hMIP1α |
| $Ile_{11}$Pep3 (1-12)[MCP-1] | EICADPKQKWIQ (SEQ ID NO:13) | Mutant of peptide 3 |
| $Leu_4Ile_{11}$Pep3 (1-12)[MCP-1] | EICLDPKQKWIQ (SEQ ID NO:14) | Mutant of peptide 3 |
| Unrelated control peptide | | |
| Peptide C | CPSLEDSFIQVA (SEQ ID NO:15) | C-terminus of h Apo(a)RG-C protein |

**TABLE 4**

**Effect of Mutant Sequence Peptide 3 Derivatives on THP-1 Migration**

| PEPTIDE | $ED_{50}$ (μM) versus | | | | |
|---|---|---|---|---|---|
| | MCP1 | MIP1α | IL8 | SDF1α | TGFβ1 |
| Peptide 3 (SEQ ID NO:1) | 8 | 8 | 14 | 10 | n.s. |
| Peptide 3[3-12] (SEQ ID NO:7) | 8 | 7 | 9 | 9 | n.s. |
| Peptide 3[1-6] (SEQ ID NO:8) | 33 | 25 | 17 | 19 | n.s. |
| Peptide 3[7-12] (SEQ ID NO:9) | 7 | 5 | 6 | 6 | n.s. |
| $Leu_4$peptide 3 (SEQ ID NO:10) | 8 | 7 | 3 | 3 | n.s. |
| $Ser_7$peptide 3 (SEQ ID NO:11) | 7 | 6 | 3 | 4 | n.s. |
| $Ile_{11}$peptide 3 (SEQ ID NO:13) | 6 | 4 | 2 | 7 | n.s. |
| $Leu_4Ile_{11}$peptide 3 (SEQ ID NO:14) | 2 | 1 | 3 | 3 | n.s. |
| $Ser_7Glu_8Glu_9$pep3 (SEQ ID NO:12) | 7 | 2 | 9 | 5 | n.s. |
| WVQ | 8 | < 1 | < 1 | < 1 | n.s. |
| KQK | 7 | n.s. | n.s. | n.s. | n.s. |
| SEE | n.s. | 6 | n.s. | n.s. | n.s. |

Peptide 3(7-12)[MCP-1] (SEQ ID NO:9) showed essentially no selectivity, inhibiting migration by all chemokines tested with an $ED_{50}$ in the range of 7-9 μM, i.e., it was a pan-chemokine inhibitor. Peptide 3(1-6)[MCP-1] (SEQ ID NO: 8) was much less efficient at inhibiting the CC chemokines ($ED_{50}$ of about 30 μM) but only slightly less efficient at inhibiting CXC chemokines (18 μM) compared with peptide 3(1-12)[MCP-1] (SEQ ID NO:1). The selectivity ratio is defined as the average $ED_{50}$ for MCP-1 and MIP1α divided by the average $ED_{50}$ for IL-8 and SDF1α. Selectivity ratios of greater than 1 indicate greater inhibition of CC chemokines relative to CXC chemokines; selectivity ratios of less than 1 indicate greater inhibition of CXC chemokines relative to CC chemokines; and a selectivity ratio of 1 indicates that both families of cytokines are inhibited to the same extent. Hence, although it is overall a markedly weaker inhibitor of

chemokine signaling, peptide 3(1-6)[MCP-1] (SEQ ID NO:8) showed a 2-fold selectivity for CXC chemokines. Thus, peptide 3(1-6)[MCP-1] (SEQ ID NO:8) is a preferred inhibitor of the CXC chemokines, with a selectivity ratio of 0.7, while peptide 3(7-12)[MCP-1] (SEQ ID NO:9) is a preferred inhibitor of both classes of chemokines, with a selectivity ratio of 1.1. The selectivity ratio for peptide 3(1-12)[MCP-1] (SEQ ID NO:1) is 1.5.

Peptide 3(3-12)[MCP-1] (SEQ ID NO:7) had very similar properties to peptide 3(1-12)[MCP-1] (SEQ ID NO: 1). This result suggested that the glutamate (E) and isoleucine (I) residues at positions 1 and 2 of the peptide 3(1-12) [MCP-1] (SEQ ID NO:1) sequence, which are not conserved in chemokine sequences other than MCP-1, are unimportant for receptor binding. Alignment of all human chemokine sequences in the peptide 3 region indicate a common conserved motif present in almost all chemokines whether of the alpha or beta subfamily (Table 3). This motif is: $Cx_1DPx_2x_3x_4Wx_5Q$.

Furthermore, there is a pattern of amino acids in the variable positions $x_1$ through $x_5$ which suggests that the nature of the amino acid at these positions may play a role in determining the selectivity of receptor binding. For example, in the CC chemokine family, position $x_1$ is usually occupied by alanine (A), whereas this position is commonly leucine (L) in the CXC chemokines except in SDF1 (Isoleucine (I) in SDF-1). To test this hypothesis, the selectivity of $Leu_4$peptide 3(1-12)[MCP-1] (SEQ ID NO:10) was compared to peptide 3(1-12)[MCP-1] (SEQ ID NO:1). While $Leu_4$peptide 3(1-12) [MCP-1] (SEQ ID NO:10) showed an approximately 4-fold increase in potency as an inhibitor of CXC chemokines compared with ala-containing peptide 3(1-12)[MCP-1] (SEQ ID NO:1), there was no decrease in the potency of CC chemokine inhibition (Table 4). Thus, $Leu_4$peptide 3(1-12)[MCP-1] (SEQ ID NO:10) showed some CXC selectivity (a selectivity ratio of 0.37) and was the most CXC selective of all the derivatives tested other than the tripeptides (see below).

As noted for position $x_1$ above, only three different amino acids appear at position $x_5$ (Table 1). Most chemokines have valine (V) at position $x_5$ as do the CXC chemokines IL-8 and MIP. In contrast, SDF-1 and IP10 have isoleucine (I) at this position, while ENA78 is the only chemokine with leucine (L) at this position. The results showed that $Ile_{11}$peptide 3(1-12)[MCP-1] (SEQ ID NO:13) showed some CXC selectivity, though not as marked as $Leu_4$peptide 3(1-12)[MCP-1] (SEQ ID NO:10) (a selectivity ratio of 0.9), but surprisingly showed the greatest selectivity for IL-8 (which has valine at this position) not SDF-1. This analog was the most selective inhibitor of IL-8 signaling other than the tripeptides, i.e., the analog had a selectivity of IL-8 over other chemokines by about 3 fold.

An analog having both the $Leu_4$ and $Ile_{11}$ substitutions did not show any greater specificity as an inhibitor of CXC chemokines than either single mutant $Leu_4$peptide 3(1-12)[MCP-1] (SEQ ID NO:10) or $Ile_{11}$peptide 3(1-12)[MCP-1] (SEQ ID NO:13) (Table 6). However, $Leu_4Ile_{11}$peptide 3(1-12)[MCP-1] (SEQ ID NO:14) was approximately 5-fold more potent as an inhibitor of CC chemokines than peptide 3(1-12)[MCP-1] (SEQ ID NO:1), or the single mutants $Leu_4$peptide 3(1-12)[MCP-1] (SEQ ID NO:10) or $Ile_{11}$peptide 3(1-12)[MCP-1] (SEQ ID NO:13). Thus, $Leu_4Ile_{11}$peptide 3(1-12)[MCP-1] (SEQ ID NO:14) was a more potent general chemokine inhibitor, with an average $ED_{50}$ of 2.3 $\mu$M compared with 10 $\mu$M for peptide 3(1-12)[MCP-1] (SEQ ID NO:1). Furthermore, the $Leu_4Ile_{11}$peptide 3(1-12)[MCP-1] (SEQ ID NO:14) unexpectedly preserved the modest CC selectivity of peptide 3(1-12)[MCP-1] (SEQ ID NO:1) with a selectivity ratio of 2.0. Surprisingly, therefore, the $Leu_4Ile_{11}$peptide 3(1-12)[MCP-1] (SEQ ID NO:1) was approximately 5-fold more potent as an inhibitor of MCP-1 signaling than peptide 3(1-12)[MCP-1] (SEQ ID NO:1), despite the fact that peptide 3(1-12)[MCP-1] (SEQ ID NO:1) contains the cognate sequence from human MCP-1. Moreover, it was found that the $Leu_4Ile_{11}$peptide 3(3-12)[MCP-1] (SEQ ID NO:1), like $Leu_4Ile_{11}$peptide 3(1-12)[MCP-1] (SEQ ID NO:14), was a higher affinity peptide analog of peptide 3(1-12)[MCP-1] (SEQ ID NO:1).

For positions $x_2$ through $x_4$, all chemokines described to date have at least one charged amino acid in this tripeptide region (Table 1). Many chemokines have two basic residues occupying $x_2$ and $x_4$ (e.g., KQK in MCP-1, KER in MCP-2 and KLK in SDF-1) while others have two acidic residues (e.g., SEE in MIP1$\alpha$, SES in MIP1$\beta$, and SES in RANTES). A recent report (Nature Med., 3, 367 (1997)) suggested that the charge in the extracellular loops of the chemokine receptors may be an important determinant of ligand specificity, e.g., CXCR4 which binds SDF-1 is negatively charged, while CCR5, which binds MIP1$\alpha$, MIP1$\beta$ and RANTES is positively charged. Thus, residues $X_2$-$X_4$ may play an important role in receptor specificity.

To test this hypothesis, several variants were prepared: $Ser_7$peptide 3(1-12)[MCP-1] (SEQ ID NO:11) substitutes the positively charged K residue present in MCP-1, MCP-2, Eotaxin, IL-8 and SDF-1 with the hydroxylated S residue present in MIP-1$\alpha$, MIP1$\beta$ and RANTES. However, this alteration did not markedly alter the selectivity. In particular, this alteration did not decrease the potency of inhibition of MCP-1 signaling, nor increase the potency of inhibition of MIP1$\alpha$ signaling (Table 4). The only detectable change was a modest shift from the moderate CC selectivity of peptide 3(1-12)[MCP-1] (SEQ ID NO:1) to a moderate CXC selectivity of the $Ser_7$peptide 3(1-12)[MCP-1] (SEQ ID NO:11) variant (a selectivity ratio of 0.5). Another variant, $Ser_7Glu_8Glu_9$peptide 3(1-12)[MCP-1] (SEQ ID NO:12) which converts the peptide from being the cognate of the MCP-1 sequence to the cognate of the MIP1$\alpha$ sequence, resulted in a more selective MIP1$\alpha$ inhibitor, although the selectivity ratio for MIP1$\alpha$ versus all other chemokines was only about 3 fold.

None of the peptide 3(1-12)[MCP-1] variants had any detectable activity as an inhibitor of TGF-beta induced migration of THP-1 cells, even at 100 $\mu$M (Table 4). Thus, all these variants were highly selective inhibitors of chemokine-induced signaling. There were no substitutions which altered an amino acid residue in peptide 3(1-12)[MCP-1] to any

other amino acid regions found in the chemokine sequences described above which markedly reduced the potency of the general chemokine inhibition observed. However, certain alterations resulted in a shift in selectivity. For example, the CC selectivity of peptide 3(1-12)[MCP-1] (SEQ ID NO:1) can be converted to CXC selectivity by mutating A to L at position 4 ($x_1$) or by mutating V to I at position 11 ($x_5$). In particular, two variants had greater than 3-fold selectivity for one chemokine over the average $ED_{50}$ for all the others, i.e., $Ile_{11}$peptide 3(1-12)[MCP-1] (SEQ ID NO:13) had weak overall selectivity for IL8 inhibition and $Ser_7Glu_8Glu_9$peptide 3(1-12)[MCP-1] (SEQ ID NO:12) had weak overall selectivity for MIP1α.

In summary, although peptide 3(1-12)[MCP-1] variants varied to a small extent in their $ED_{50}$s and their specificity for either the α family or β family of chemokines, nevertheless, they were all similar to peptide 3(1-12)[MCP-1] (SEQ ID NO:1). The results in Table 6 showed that peptide 3(1-12)[MCP-1] (SEQ ID NO:1) and peptide 3(1-12)[MCP-1] variants inhibited migration induced by MCP-1, MIP1α, IL8 and SDF1α chemokines to a similar extent. While some peptides or peptide variants showed slight preference for CC chemokines, others showed slight preference for CXC chemokines but in no case did the CC-specificity exceed two-fold. Peptide 3(1-12)[MCP-1] (SEQ ID NO:1), peptide 3(1-6)[MCP-1] (SEQ ID NO:8) and peptide 3(7-12)[MCP-1] (SEQ ID NO:9) also showed no significant CC or CXC selectivity.

## Example 3

Identification Preparation and Characterization of Therapeutic Agents of the Invention for *In Vivo* Use

### A. Derivatives

Peptides are generally susceptible to chemical or enzymatic hydrolysis. In particular, peptides are not normally bioavailable by the oral route since they are not stable in the acid and proteolytic environment of the stomach. Thus, chemical or enzymatic hydrolysis leads to a very short *in vivo* half-life for peptides. To extend the half-life of agents susceptible to hydrolysis, *in vitro* active agents are modified in a manner that results in a derivative which may be orally bioavailable, have improved pharmacokinetics, and the administration of which may achieve concentrations in blood that inhibit chemokine activity. For example, cyclic-reverse-D (CRD) peptides may be prepared. CRD peptides are prepared by synthesizing the reverse sequence of the peptide (C-terminal to N-terminal) using the opposite stereoisomer (D-amino acids in place of L amino acids). The resulting peptide is then cyclized via N- and C-terminal cysteine residues. These derivatives retain a very similar steric arrangement of atoms to non-CRD peptide, but are not subject to enzymatic hydrolysis. Other derivatives which may exhibit an extended half-life *in vivo* include thienyl or pyridyl derivatives (e.g., U.S. Patent No. 4,992,463; U.S. Patent No. 5,091,396).

For example, to prepare a peptide 3 derivative, peptide 3(3-12)[MCP-1] was modified according to Jameson et al. (Nature, 368, 744 (1994)), which yielded CRD-$Cys_{13}Leu_4Ile_{11}$peptide 3(3-12)[MCP-1] (Figure 3). CRD-$Cys_{13}Leu_4Ile_{11}$peptide 3(3-12)[MCP-1], which had very similar properties to peptide 3(1-12)(MCP-1] (SEQ ID NO:1) in the *in vitro* assays described hereinabove, was found to be stable against both acid hydrolysis (<10% degradation at pH 2.0 for 2 hrs at 37°C) and enzymatic destruction (5 units trypsin for 2 hrs at 37°C). CRD-$Cys_{13}Leu_4Ile_{11}$peptide 3 (3-12)[MCP-1] was also resistant to hydrolysis *in vivo* and allowed therapeutically useful plasma concentrations to be achieved (> 10 μM 24 hours after a single intraperitoneal dose of 1 mg ofCRD-$Cys_{13}Leu_4Ile_{11}$peptide 3(3-12)[MCP-1] in 250 μl saline).

Cyclic-reverse D (CRD), linear reverse-D (LRD), cyclic forward L (CFL), and linear forward L (LFL) (i.e., the standard form of peptides) derivatives of $Leu_4Ile_{11}$peptide 3 were prepared and their MCP-1 inhibitory activity in the THP-1 transwell assay determined. The results were

| | |
|---|---|
| LFL-$Leu_4Ile_{11}$peptide 3 | 1-5 μM |
| LRD-$Leu_4Ile_{11}$peptide 3 | 200-400 nM |
| CFL-$Cys_{13}Leu_4Ile_{11}$peptide 3 | 500-700 nM |
| CRD-$Cys_{13}Leu_4Ile_{11}$peptide 3 | 5-100 nM |

These results show, somewhat surprisingly, that both cyclization and reverse-D derivatization independently improve activity. This improvement is then additive in the CRD derivative. Thus, cyclization improved activity, possibly by constraining the conformations of the peptide. However, it was not expected that the reverse-D derivatization would be so beneficial, possibly by increasing stability of the molecule.

CRD-$Cys_{13}Leu_4Ile_{11}$peptide 3(3-12)[MCP-1] was found to be a very potent inhibitor of MCP-1 induced THP-1 migration ($ED_{50}$ of about 1-10 nM). This increased potency compared to the parent $Leu_4Ile_{11}$peptide 3(1-12)[MCP-1] (SEQ ID NO:14) may reflect increased stability, even *in vitro,* or it may reflect the increased conformational stability of the peptide. Moreover, this compound binds to the signaling receptor with the same affinity as native full-length MCP-1 but does not signal.

To determine if CRD-$Leu_4Ile_{11}Cys_{13}$peptide 3(3-12)[MCP-1] inhibited or enhanced the proliferation of T or B cells to conconavalin A or tetanus toxoid in culture, proliferation of CD4 T cells and B cells was assessed by CFSE-FITC cell labeling. 50 ng of CRD-$Leu_4Ile_{11}Cys_{13}$peptide 3(3-12) [MCP-1] inhibited ConA proliferation of CD4 T cells by 50% and 5 ng of CRD-$Leu_4Ile_{11}Cys_{13}$peptide 3(3-12) [MCP-1 reduced ConA proliferation of CD4 T cells by < 3%. CRD-$Leu_4Ile_{11}Cys_{13}$peptide 3(3-12)[MCP-1] had no effect on proliferation of B cells to tetanus toxoid.

Computer modeling was employed to determine whether specific amino acid replacements affected the conformation of the peptide derivative CRD-$Leu_4Ile_{11}Cys_{13}$peptide 3(3-12)[MCP-1]. The peptide sequence was entered into HyperChem 5.0 (HyperCube). A minimum energy conformation was sought using the Amber Force Field parameters and the Polak-Ribiere algorithm. The initial model was manipulated both by molecular dynamics simulations (300˚K, 2 nsec) and manual sidechain rotations, followed by geometry optimization, until an apparent global minimum energy conformation was reached. Convergence criterion was <0.01 Kcal/mol Å. A conformation was obtained using this procedure with an energy of about 213.4 kcal/mol.

To test the sensitivity of the model peptide to perturbations, each of the residues except the terminal cysteines forming the disulfide bond was mutated individually from D to L, and the geometry re-optimized, starting with the minimum conformation of the all D peptide. For these perturbations each mutant was first run through the geometry optimization routine, then a molecular dynamics simulation, then another geometry optimization. The resulting mutant peptides were compared to the all-D form by overlaying the disulfide bond and one adjacent atom, and visually assessing the difference between the peptide backbones. The overall conformation was insensitive to change of chirality at positions 2, 3, 4, 8, 9, and 10, but was sensitive to change of chirality at positions 5, 6, and 7. Generally, changes in sidechain position were minor except when the backbone conformation changed significantly. Energies for the mutants varied from -187.9 to -226.1 Kcal/mol, but the energy change (from -213.4 for the starting conformation) did not correlate with conformational change.

In addition, the effect of modifying the aspartate residue at position 9 was examined by converting it sidechain carboxyl group to the D-alanyl amide. A minimum energy conformation of the modified peptide was sought using the same routine as for the chiral mutants, starting from the same minimum energy conformation. Condensation of D-alanine to the residue 9 sidechain carboxyl caused a major change in the conformation of the peptide. This is consistent with the *in vitro* monocyte migration data which demonstrated a significant loss in biological activity of the D-ala peptide relative to CRD-$Leu_4Ile_{11}Cys_{13}$peptide 3(3-12)[MCP-1]. That is, CRD-$Cys_{13}Leu_4Ile_{11}$peptide 3(3-12) was synthesized (left panel of Figure 13) and was found to be 1000-fold more potent as an inhibitor of THP-1 migration induced by MCP-1 than the D-ala derivative thereof (right panel of Figure 13). The D-alanine prevents the salt bridge formation between an aspartic acid and lysine residue, and so renders this derivative inactive even at a concentration of 100 $\mu$M.

Molecular modeling indicated that L-Leu-CRD-$Leu_4Ile_{11}Cys_{13}$peptide 3(3-12)[MCP-1], which is CRD-$Leu_4Ile_{11}Cys_{13}$peptide 3(3-12)[MCP-1] with the D-Leu replaced with an L-Leu, should result in very little change to the conformation of the peptide backbone. *In vitro* migration studies with the L-Leu-derivative showed that it retained functional activity as well. Thus, to select for particular amino acid substitutions which retain the conformation of a biologically active molecule of the invention, molecular modeling may be employed.

The following D-amino acid to L-amino acid changes had no significant impact on the structure of the peptide backbone as assessed by modeling.

| Amino Acid | Position | kcal/mole |
|---|---|---|
| GLN | 2 | -205.5 |
| ILE | 3 | -202.4 |
| TRP | 4 | -222.0 |
| PRO | 8 | -226.1 |
| LEU | 10 | -211.9 |

The following D-amino acid to L-amino acid changes had a significant impact on the structure of the peptide backbone as assessed by this technique.

| Amino Acid | Position | kcal/mole |
|---|---|---|
| LYS | 5 | -200.1 |
| GLN | 6 | -211.6 |
| LYS | 7 | -187.9 |
| ASP | 9 | -214.9 |

B. DARC binding

A further consideration for bioavailability is non-specific binding of the therapeutic agent. Red blood cells have a signaling-deficient chemokine receptor or binding protein, termed the Duffy Antigen Receptor for Chemokines (DARC). Although it does not signal, this receptor has a high affinity for chemokines (10 nM) and may play a role in clearing them from the circulation. Unfortunately, any chemokine receptor antagonist which has a high affinity for DARC may be sequestered by the huge pool of binding sites on red blood cells, and hence be unavailable to inhibit productive chemokine signaling in other tissues. Similarly, agonists which bind DARC with high affinity are unavailable to productively signal through specific chemokine receptors. For *in vivo* use, an agent of the invention preferably has some affinity for DARC, since peptides which do not bind to DARC are rapidly cleared at first pass by glomerular filtration. Thus, preferred agents have DARC binding (affinity constant) in the range 100 nM to 1 mM, more preferably in the range 1 $\mu$M to 100 $\mu$M and even more preferably in the range of 10 to 100 $\mu$M.

Although the interaction of chemokines with DARC is high affinity (5-10 nM association constant), kinetically the interaction is characterized by extremely rapid on and off rates. Consequently, incubation with labeled chemokine leads to saturation of the DARC binding sites, but most of the bound label is lost within minutes of removing the unbound label (>90% loss within 3 minutes). As a result, it is difficult to directly determine the binding of peptides to DARC by assaying direct binding of biotinylated peptide, since the rapid off rates make determination of the amount of bound label impossible or inaccurate.

To overcome this difficulty, the ka for association of DARC with peptide 3(1-12)[MCP-1] (SEQ ID NO:1) and peptide 2(1-15)[MCP-1] (SEQ ID NO:3) was estimated by incubating red blood cells expressing DARC with $^{125}$I-labeled MCP-1 in the presence of varying concentrations of peptide. Nine mls of freshly drawn blood is transferred to a tube containing 1 ml 3.8% sodium citrate, and left at room temperature for 15 minutes. Five mls of anti-coagulated blood is layered over 3.5 ml Polymorphprep (Nycomed Pharma, Oslo), and centrifuged at 500 x g for 35 minutes. The erythrocytes are removed and reconstituted to the original volume with binding medium (PBS + 1 mg/ml fatty acid free BSA, pH 7.4), and centrifuged at 900 x g for 10 minutes. This is repeated four times prior to counting the cell and adjusting the volume to 1 x $10^8$ erythrocytes per well in a "v" bottomed microtiter plate. The cells are sedimented for 5 minutes at 670 x g and resuspended in binding medium containing 0.5 nM $^{125}$-I MCP-1 (specific activity 2000 Ci/mmol; Amersham) in the presence of non-labeled MCP-1 or test agent.

After binding reached equilibrium (30 minutes at 37˚C), the cells are separated from the unbound label by centrifugation for 5 minutes through a sucrose gradient. Counts associated with the cells are then determined by gamma-counting scintigraphy. In the absence of all peptides, the association constant for $^{125}$I-labeled MCP-1 on human red blood cells was 5.45 nM, a value which is in accord with a previous report. Furthermore, Scatchard analysis confirmed the presence of a single high affinity binding site with 500-1000 copies per cell, consistent with the known properties of DARC. Thus, determination of $^{125}$I-MCP-1 binding to red blood cells in this assay in the presence of various concentrations of the peptide(s) allows the association constant of the peptide for DARC to be accurately estimated.

The DARC specificity ratio is also determined. The DARC specificity ratio is defined as the estimated ka for association with DARC divided by the $ED_{50}$ for biological activity. A DARC specificity ratio greater than 1 indicates that a peptide associates poorly with DARC and is bioavailable for modulating chemokine signaling, either as an antagonist or agonist. A DARC specificity ratio of about 1 indicates that the peptide binds DARC and the Thy-1 signaling receptors with similar affinity. Thus, it may be difficult to achieve biologically active (as a chemokine inhibitor) concentrations of such peptides *in vivo* without further modifications of the peptide. A DARC specificity ratio less than 1 indicates much higher affinity for DARC than for chemokine signaling receptors.

Peptide 1[MCP-1] (SEQ ID NO:2) (which does not bind to chemokine receptors but functions in a dominant negative fashion) showed no binding to DARC (estimated ka > 100 $\mu$M). In marked contrast, the weak agonist peptide 2(1-15)[MCP-1] (SEQ ID NO:3) showed high affinity binding to DARC. The association constant for peptide 2(1-15) [MCP1] (SEQ ID NO:3) for chemokine receptors on THP-1 cells was estimated at 2 $\mu$M using competition binding analysis. However, this peptide had an affinity for DARC of less than 500 nM, also assessed by competition binding analysis, using red blood cells. Thus, peptide 2(1-15)[MCP1] (SEQ ID NO:3) binds to THP-1 cell chemokine receptors, although it does not inhibit signaling through the receptors, and it binds DARC even more strongly (DARC selectivity ratio = 0.1-0.2). Thus, peptide 2 is a preferred therapeutic agent for the treatment or prevention of malaria (an action requiring DARC inhibition, but not modulation of chemokine signaling).

Peptides, such as peptide 2(1-15)[MCP-1] (SEQ ID NO:3) which have very high affinity for the DARC receptor, may have strong biological agonist activity *in vivo* (although they are only weak agonists or neutral agonists in *vitro*). Moreover, peptide 2, variants and derivatives thereof may be strongly pro-inflammatory *in vivo,* or strongly exacerbate existing inflammation by preventing DARC from performing the function of binding chemokines. If DARC functions as a sink to remove chemokines from the circulation, then the concentration of chemokines may be markedly increased by the presence of peptide 2. Under conditions where chemokines are being made a released into the circulation (e.g., during inflammation), peptide 2 may exacerbate that inflammation, allow the inflammation to persist longer than in the

absence of the peptide or otherwise change the qualitative nature of the inflammatory reaction. For these reasons, peptides with a low DARC specificity ratio are useful for the treatment of conditions which require improved immune function, or conditions which are characterized by a pathologically inadequate inflammatory response.

MIP1-α has previously been shown to be the only chemokine which does not bind with significant affinity to DARC. Peptide 2(1-9)[MCP-1] had a Duffy affinity of about 50 μM while peptide 2(1-15)[MIP1-α] (SEQ ID NO:5) was a potent receptor binding agent for the MIP1-α receptor and had excellent specificity over DARC. That is, peptide 2(1-15) [MIP1α] (SEQ ID NO:5) did not bind to DARC (association constant > 50 μM) but bound strongly to chemokine receptors on THP-1 cells (association constant = 100-900 nM; number of binding sites is about 150,000 per cell). Moreover, this agent did not inhibit THP-1 cell migration induced by MCP-1, MIP1α, IL-8, or SDF1α. Thus, this latter agent may be particularly useful as a neutral chemokine receptor binding agent *in vivo,* highly selective over DARC.

Peptide 3(1-12)[MCP-1] (SEQ ID NO:1) also binds to DARC, although it binds to DARC with only a similar affinity to which it binds to the chemokine receptors (low μM concentration range). $Leu_4ile_{11}$peptide 3(1-12)[MCP-1] (SEQ ID NO:14) had essentially no DARC binding capacity (and at least 20-fold selectivity for receptors on THP-1 cells), while inhibiting MCP1 induced migration at concentrations around 1 μM. Thus, peptide 3 derivatives, such as $leu_4ile_{11}$peptide 3(1-12)[MCP-1] (SEQ ID NO:14) may achieve antagonist properties *in vivo.*

The shorter fragments of peptide 3[MCP-1] (e.g., peptide 3(7-12)[MCP-1] (SEQ ID NO:9)) showed progressively higher DARC specificity ratios (about 3.0 for peptide 3(7-12)[MCP-1] (SEQ ID NO:9) versus 1.0 for peptide 3(1-12)[MCP-1] (SEQ ID NO:1)), indicating that where chemokine signaling receptor specificity is desired, shorter peptide fragments which retain full chemokine antagonist or agonist activity are in general to be preferred over the full length peptides.

Peptide 3(1-12)[MCP-1] (SEQ ID NO:1) (DARC specificity ratio = 1.00) is unlikely to be useful as a pan-chemokine inhibitor *in vivo,* whereas the $Leu_4Ile_{11}$peptide 3[MCP-1] (SEQ ID NO:14) (DARC specificity ratio = 37.83), or its derivatives such as CRD-$Cys_{13}Leu_4Ile_{11}$peptide 3(3-12)[MCP-1], which bound only weakly to DARC (association constant = 90 μM) but bound very strongly to chemokine receptors on THP-1 cells (association constant = 100-500 nM; number of binding sites is about 150,000 per cell), are a preferred embodiment for the treatment or prevention of atherosclerosis, osteoporosis, and autoimmune diseases, and HIV infection (chemokine signaling receptor binding functions). Moreover, CRD-$Cys_{13}Leu_4Ile_{11}$peptide 3(3-12)[MCP-1] inhibited THP-1 cell migration induced by MCP-1, MIP1α, IL-8, and SDF1, with very similar $ED_{50}$s.

CRD-peptide 2(1-15)[MCP-1] has more functional potency, less Duffy binding activity compared with the LFL derivative. LRD peptide 2(1-15)[MCP-1] had approximately a 100-fold decrease in Duffy binding (25 μM versus 100 μM for LFL).

An alternative approach to preparing agents that are bioavailable is the preparation of non-peptide analogs of chemokines (see Examples 17-22 below). An exemplary non-peptide analog of the invention includes an isostere of WIQ, e.g., a compound of formula (IV), wherein Z=$CH_3$; Y=O; X=$CH_3$; and Ar = indolyl. This compound did not bind to DARC (association constant = > 30 μM) but bound very strongly to chemokine receptors to THP-1 cells (association constant = 100 nM-1 μM; number of binding sites is about 150,000 per cell). This agent inhibited THP-1 cell migration induced by MCP-1, MIP1α, IL-8 and SDF1α with very similar $ED_{50}$s.

Other preferred analogs include an analog of WxQ. For example, using a series wherein x= Gly (H), Ala (Me), EtGly (ethyl), Val (isopropyl), Ile (isobutyl) and alloIle (alloisobutyl), it was found that the most active compounds are those with the smallest alkyl side chains (Gly and Ala) with a trend to Ile being the least active, i.e., WGQ > WAQ >> WEtG = WVQ = WAlloIQ > WIQ.

**TABLE 5**

| PEPTIDE | SEQUENCE | CC SPECIFICITY[a] | DUFFY SELECTIVITY[b] | AVERAGE $ED_{50}$ (μM)[c] |
|---|---|---|---|---|
| Pep2[MCP1] | SYRRITSSKCPKEAV (SEQ ID NO:3) | - | 0.18[d] | 2[e] |
| Pep2[SDF1] | HLKILNTPNCALQIV (SEQ ID NO:4) | - | <1[d] | 1-10[e] |
| Pep2[MIP1α] | DYFETSSQCSKPGV (SEQ ID NO:5) | - | > 100[d] | 1-10[e] |
| Pep2[IL8] | ELRVIESGPHCANTEI (SEQ ID NO:6) | - | < 1[d] | 1-10[e] |
| Pep3 | EICADPKQKWVQ (SEQ ID NO:1) | 1.5 | 1.00 | 10 |
| Pep3[3-12] | CADPKQKWVQ (SEQ ID NO: 7) | 1.2 | 1.21 | 8 |

(continued)

| PEPTIDE | SEQUENCE | CC SPECIFICITY[a] | DUFFY SELECTIVITY[b] | AVERAGE $ED_{50}$ ($\mu$M)[c] |
|---|---|---|---|---|
| Pep3[1-6] | EICADP (SEQ ID NO:8) | 0.7 | 5.32 | 24 |
| Pep3[7-12] | KQKWVQ (SEQ ID NO:9) | 1.0 | 2.94 | 6 |
| $Leu_4$pep3 | EICLDPKQKWVQ (SEQ ID NO:10) | 0.4 | n.d. | 5 |
| $Ser_7$pep3 | EICADPSQKWVQ (SEQ ID NO:11) | 0.5 | 2.00 | 5 |
| $Ile_{11}$pep3 | EICADPKQKWIQ (SEQ ID NO:12) | 0.9 | n.d. | 5 |
| $Leu_4Ile_{11}$pep3 | EICLDPKQKWIQ (SEQ ID NO:13) | 2.0 | 37.83 | 2 |
| $Ser_7Glu_8Glu_9$ | EICADPSEEQVQ (SEQ ID NO:2) | 1.6 | 0.59 | 6 |
| - | KQK (SEQ ID NO:51) | > 100 | 22.34 | 6[f] |
| Pep3[10-12] | WVQ (SEQ ID NO:33) | 1.1 | 24.81 | 2 |
| - | WIQ (SEQ ID NO:34) | 1.0 | n.d. | 2 |
| - | SEE (SEQ ID NO:27) | > 100 | n.d. | 0.8[f] |
| - | KLK (SEQ ID NO:28) | < 0.1 | n.d. | 0.1-10[f] |
| - | KEN (SEQ ID NO:55) | < 0.1 | n.d. | 0.1-10[f] |
| CRD-$Cys_{13}$pep3[3-12] | *-CQVWKQKPDAC-* | n.d. | 8.82 | 0.8 |
| LRD-$Cys_{13}Leu_4Ile_{11}$pep3 [3-12] | *CQVWKQKPDAC* | n.d. | 4.59 | 1 |
| CRD-$Cys_{13}Leu_4Ile_{11}$pep3 [3-12] | *-CQIWKQKPDLC--* | n.d. | > 100 | 0.1 |

Footnotes

[a] 'CC-specificity' is the average inhibitory $ED_{50}$ versus SDF1 and IL8 divided by average inhibitory $ED_{50}$ versus MCP-1 and MIP1$\alpha$.

[b] 'Duffy selectivity' is the estimated ka for binding to red blood cells divided by average inhibitory $ED_{50}$ versus each of the chemokines (except for peptide 2; see footnote d below).

[c] 'Average $ED_{50}$' is the average inhibitory $ED_{50}$ for inhibition of THP-1 migration induced by each of the chemokines (except for peptide 2; see footnote e below).

[d] For the peptide 2 family, the 'average $ED_{50}$' is the estimated ka for binding to THP-1 cells.

[e] For the peptide 2 family, the 'Duffy selectivity' is calculated as the ka for binding to red blood cells divided by the ka for binding THP1-1 cells.

[f] For tripeptide derivatives so marked, the peptide is highly specific for one of the four exemplary chemokines. In these cases, the $ED_{50}$ shown is for inhibition of that chemokine.

n.d. = not determined.

Abbreviations

CRD = Cyclic reverse-D derivative
LRD = Linear reverse-D derivative
CFL = Cyclic derivative of standard L-form peptide
LFL = Standard, linear L-form peptide [NB; all peptides are LFL unless stated otherwise]
Amino acids in italics are D-form amino acids, all others are L-form
-- = Cyclization linking two cysteines so marked

**Example 4**

Preparation and Characterization of Tripeptide Therapeutic Agents of the Invention

To determine whether fragments ofpeptide 3(1-12)[MCP-1] possessed biological activity, fragments of peptide 3 were prepared. Peptide 3(10-12)[MCP-1], i.e., WVQ was found to be a potent inhibitor of all chemokines tested (Table 6). The amino acid residues at positions 10-12 (WVQ) are conserved in many other chemokines, e.g., MCP-3, MIP1$\alpha$,

MIP1β, RANTES, EOTAXIN, and IL8, although SDF1 has the sequence WIQ. WVQ inhibited all four of the exemplary chemokines tested, although, unlike peptide 3(1-12)[MCP-1] (SEQ ID NO:1), it was a more potent inhibitor of all the chemokines other than MCP-1, with $ED_{50}$s around 1 μM. Thus, these tripeptides, WVQ and WIQ, as well as non-peptide analogs based on these tripeptides, are pan-specific chemokine inhibitors. Moreover, it was found that WVQ had good DARC selectivity (i.e., selectivity of 10).

Peptide 3(7-9)[MCP-1], i.e, KQK, did not bind to DARC (association constant = > 50 μM) but bound strongly to chemokine receptors on THP-1 cells (association constant = 500 nM-1 μM; number of binding sites is about 15,000 per cell). This agent inhibited THP-1 cell migration induced by MCP-1, but did not inhibit migration induced by MIP1α, IL-8 or SDF1α. Thus, KQK with an $ED_{50}$ = 2-5 μM was found to be a specific inhibitor of MCP-1, i.e., it had no effect on MIP1α, SDF1α; or IL8 induced activity even at 100 μM. Four tripeptides and a dipeptide of random sequence (RGD, GGR, TTT, APG, and VE) were also tested. None of these significantly inhibited migration induced by any of the chemokines. Thus, the tripeptide KQK was specific for inhibiting MCP-1 activity, showing more than 100-fold specificity for MCP-1 over all the other chemokines tested.

Tripeptide equivalents of KQK from MIP1α, SDF1α and IL8, based on an alignment of conserved cysteine residues in chemokine sequences, were then tested for their inhibition of chemokine-induced THP-1 migration. In each case, the tripeptide was highly specific for its cognate chemokine (> 100-fold specific in each case). For example, SEE, the cognate peptide from MIP-α, showed greater than 100-fold selectivity for MIP1-α over the other chemokines.

Moreover, KLK was a specific and potent inhibitor of SDF1, and KEN was a specific and potent inhibitor of IL8. It is envisioned that tripeptides in which a conservative substitution is made may have the same specificity as the native tripeptide. Moreover, the corresponding tripeptides in other chemokines may be specific for their cognate chemokines.

**TABLE 6**

| | Chemoattractant | | | | |
|---|---|---|---|---|---|
| Tripeptide | | | | | |
| | MCP-1 | MIP1α | RANTES | IL-8 | SDF1α |
| KQK[a] | 95 ± 8[b] | - | - | - | 29 ± 1 |
| SEE | - | 65 ± 3 | - | - | - |
| SES | - | - | 87 ± 4 | - | - |
| KEN | 21 ± 2 | - | - | 70 ± 4 | - |
| KLK | - | - | - | | 87 ± 6 |
| WVQ[c] | 8 μM | 7.5 μM | 1.5 μM | 1 μM | 2 μM |

For each peptide shown (except WVQ), a number indicates the percentage inhibition of migration induced by that chemoattractant by that tripeptide at 100 μM concentration (mean ± range: two experiments). A dash indicates no statistically significant reduction in migration (all combinations of chemoattractant and tripeptide have been tested. The tripeptide WVQ inhibited migration in response to all chemoattractants tested and for this tripeptide the numbers shown are the $ED_{50}$ for the inhibition (mean of at least two determinations). Note that none of the tripeptides shown inhibited TGF-β1 induced migration at 100 μM. The bolded values indicate the inhibition by each peptide of migration, induced by the chemoattractant from which it was derived, i.e., KQK was derived from MCP-1, etc.

[a] The affinity constant for KQK binding to DARC is 15 μM.

[b] The $ED_{50}$ for KQK inhibiting MCP-1 induced migration is 7 μM.

[c] The affinity constant for WVQ binding to DARC is 2 μM.

## Example 5

*In Vivo* Pharmacokinetics and Toxicity

When $^3$H-D-ala peptide 3(1-12)[MCP-1] ($^3$H-D-ala was attached to Asp) was given as an intravenous (IV) or

subcutaneous (SQ) bolus to mice, peak serum concentrations were reached within 1 hour. This radiolabeled peptide was rapidly excreted (approximately 4 hours), primarily via the kidney. Biodistribution data indicated that the primary target organ was the kidney with much smaller amounts detected in blood, liver and intestine. Direct comparison of $^3$H-D-ala peptide 3(1-12)[MCP-1] (no DARC binding and rapidly cleared) and CRD-L-$Leu_4Ile_{11}Cys_{13}$peptide 3(3-12)[MCP-1] (weak Duffy binding and good serum half-life) indicates that agents of the invention may be particularly useful to increase the half-life of other pharmaceutical agents.

A modified $LD_{50}$ technique was used to determine the mouse intravenous $LD_{50}$ value for CRD-$Leu_4Ile_{11}Cys_{13}$peptide 3(3-12)[MCP-1]. The $LD_{50}$ = 11.4 mg/mouse IV, which is 569 mg/kg. This is ten times more than the efficacious dose seen in either the asthma model or the endotoxemia model (see Examples below). Intraperitoneal administration of 11 mg did not result in lethality. Histologically, toxicity was confined to the kidneys and lymphoid tissues.

At the lethal dose, apoptosis of lymphocytes was seen in the spleen and gut-associated lymphoid tissue. The rate limiting toxicity was to the kidney. There was a dose dependent increase in acute renal tubular nephrosis. This is most likely due to the huge intravenous bolus (569 mg/kg) of a small molecular weight peptide which is excreted very rapidly (first pass) by the kidney. Acute tubular nephrosis is also seen in patients with massive release of myoglobin or hemoglobin after crush injuries or massive hemolysis.

Using an in-life phase of an acute rat toxicity study of CRD-$Leu_4Ile_{11}Cys_{13}$peptide 3(3-12)[MCP-1] at 1 or 10 mg (5-50 mg/kg) i.v., no clinically detectable changes associated with test agent administration of doses up to 10 mg were found. That is, all animals remained clinically normal throughout. No dose dependent or clinically significant changes were seen in serum chemistry, CBC, urinalysis, gross necropsy, or histology. In a 7-day repeat dose toxicity study in rats which employed subcutaneous implantation of an osmotic mini-pump that delivered about 43 μg/hr of CRD-$Leu_4Ile_{11}Cys_{13}$ peptide 3(3-12)[MCP-1] continuously for 7 days (7.2 mg), no dose dependent or clinically significant changes were seen in serum chemistry, CBC, urinalysis, gross necropsy, or histology in treated animals.

Thus, toxicity studies indicate that CRD-$Leu_4Ile_{11}Cys_{13}$ peptide 3(3-12)[MCP-1] is safe in rodents at levels greater than 10 times those which demonstrated efficacy (see below). Thus, the systemic administration of an effective pan-chemokine inhibitor is not associated with acute or chronic (up to seven days) side effects. Pan-inhibition of chemokines is therefore a viable strategy for novel anti-inflammatory therapies.

**Example 6**

Use of a CRD-Peptide of the Invention in a Rat Dermal Inflammation Model

To assess the efficacy of an agent of the invention in the prevention of lipopolysaccharide (LPS)- and MCP-1-induced dermal inflammation in the rat, three different doses of CRD-$Leu_4Ile_{11}Cys_{13}$peptide 3(3-12)[MCP-1] were administered. An inflammatory response was elicited by intradermal injection (ventral abdomen) of either 500 ng MCP-1 or 100 ng MCP-1 along with endotoxin-free phosphate-buffered saline vehicle (as a negative control) and bacterial lipopolysaccharide (LPS; as a positive control). Each substance was injected at a different site. The results obtained from animals were compared to CRD-$Leu_4Ile_{11}Cys_{13}$ peptide 3(3-12)[MCP-1] treated, and PBS (diluent control) treated, animals. Thirty minutes prior to intradermal agonist administration, the animals received an intravenous loading dose (3, 30 or 300 mg) and a subcutaneous depot dose (0.1, 1 or 10 mg) (on dorsum) of the pan-chemokine inhibitor CRD-$Leu_4Ile_{11}Cys_{13}$peptide 3(3-12)[MCP-1] (see, for example, Figure 12). Animals were sacrificed at 20-24 hours post injection. Serum and urine were collected. The intradermal sites of agonist injection were collected, bisected and the extent of the inflammatory response was assessed by histopathology and quantitative immunofluorescence (fixed and frozen) (for example, following MCP-1 injection, the number of monocyte/macrophages in the skin was determined using the anti-CD14 (MCA342 from Serotec; clone ED2) at 3 μg/ml overnight at 4˚C. The second antibody was rat anti-mouse FITC (415-096-100 from Jackson ImmunoResearch) at 28 μg/ml for 6 hours at room temperature). In addition, toxicity of CRD-$Leu_4Ile_{11}Cys_{13}$peptide 3(3-12)[MCP-1] was assessed by collection of the following tissue samples in 10% neutral buffered formalin for histologic analysis: lung, liver, kidney, spleen, thymus, heart, and antagonist (test agent) injection site.

The results of a typical experiment are shown in Figures 7A and 7B. Systemic treatment with CRD-$Leu_4Ile_{11}Cys_{13}$ peptide 3(3-12)[MCP-1] completely abolished MCP-1 induced recruitment of monocyte/macrophages. This is consistent with potent inhibition of MCP-1-induced migration seen in *vitro* with this agent. Furthermore, CRD-$Leu_4Ile_{11}Cys_{13}$ peptide 3(3-12)[MCP-1] reduced the number of resident tissue monocyte/macrophages in the site that received PBS alone, and also in untreated skin. This is consistent with a systemic downregulation of monocyte/macrophage recruitment in the 24 hours following a single treatment with CRD-$Leu_4Ile_{11}Cys_{13}$ peptide 3(3-12)[MCP-1]. In contrast, in the study described below, the D-ala derivative (Figure 13) had no effect *in vivo* (p = 0.754), in accord with its lack of *in vitro* activity in the migration assay.

A substantial reduction (>80%) in the number of monocyte/macrophages recruited in response to injected bacterial LPS was also noted. LPS was a stronger inducer of macrophage recruitment than MCP-1 even at 500 ng dose. Previous studies suggested that LPS-mediated macrophage accumulation was heavily dependent on TNF-α (a non-chemokine chemoattractant) since neutralizing antibodies to TNF-α markedly reduced LPS-induced inflammation. However, in endotoxemia models (Example 10) CRD-$Leu_4Ile_{11}Cys_{13}$ peptide 3(3-12)[MCP-1] markedly reduced LPS-induced increases in plasma TNF-α suggesting that chemokines may play a role in the induction of TNF-α, and that both chemokine signaling and TNF-α signaling may be necessary for maximal LPS-induced inflammation.

Although MCP-1 is fairly specific as a monocyte/macrophage chemoattractant, dermal injection of LPS induces recruitment of a broader range of leukocytes, including T- and B-cells and neutrophils. Specific antibodies to rat B-cells (MCA 1432 from Serotec) were used at 10 μg/ml overnight at 4°C to determine whether CRD-$Leu_4Ile_{11}Cys_{13}$ peptide 3(3-12)[MCP-1] affected the recruitment of this leukocyte subpopulation. Secondary antibody was anti-mouse FITC (415-096-100 from Jackson ImmunoResearch, as above). As for monocyte/macrophages, CRD-$Leu_4Ile_{11}Cys_{13}$ peptide 3(3-12)[MCP-1] substantially inhibited the recruitment of B-cells to the site of the LPS injection (Figure 7B).

In another study, three groups (n = 5) of rats were injected subcutaneously with either PBS, "inactive" peptide 3 (the D-ala derivative shown in Figure 13) or CRD-$Leu_4Ile_{11}Cys_{13}$peptide 3(3-12)[MCP-1] (10 mg in 200 μl subcutaneous). Thirty minutes later, PBS, 50 ng LPS and 500 ng MCP-1 were injected into three separate sites on the ventral abdomen of each rat. Animals were sacrificed 24 hours later and the intradermal site was excised, and frozen in OCT embedding media. Five μm sections were collected from each rat intradermal site and stored at -20°C. The number of monocytes (anti-rat CD14, Serotec), T cells, neutrophils (anti-rat granulocytes, Harlan Seralab), B-lymphocytes (anti-rat B cells, Serotec), and MCP-1, IL-8, and TNF-α (anti-mouse TNF-α, R and D Systems) were measured. Eight test sections and two control sections were stained at each site for each animal through a distance of 2.5 mm. The median percentage area was determined for each animal and for each group using NIH image, as the largest variability of staining was between sections rather than in any one section.

Panel A of Figure 14 shows the normal level of monocytes patrolling the rat skin surface. When 500 ng of MCP-1 was injected in the rat abdomen, there is a significant increase in monocyte staining (Figure 14, panel B). These levels remain unaltered if treated with PBS or control peptide. However, when treating the rats with 10 mg of the CRD-$Leu_4Ile_{11}Cys_{13}$peptide 3(3-12) the inflammatory response is abolished (panels C and D of Figure 14).

Sections from the LPS intradermal site (three rats in total) were stained for pro-inflammatory cytokines and cellular infiltrates using quantitative immunofluorescence. Using specific antibodies for neutrophils, monocytes, CD4+ T lymphocytes, and B-lymphocytes, the data showed that the median percentage area stained for each cell type was abolished in each case compared to the PBS and inactive peptide controls (Figure 15). In addition, when TNF-α, IL-8 and MCP-1 were measured in the same sections, TNF-α, IL-8 and MCP-1 were also substantially reduced when rats were treated with the active peptide, a pan-chemokine inhibitor. Furthermore, several groups have shown that using neutralizing antibodies against a variety of individual chemokines did not alter the LPS inflammatory response in a mouse endotoxemia model. Thus, multiple chemokines lie upstream of TNF-α in the inflammatory response pathway, and while inhibition of any of them singly does not inhibit TNF-α upregulation, inhibition of more than one at the same time (such as in response to the pan-chemokine inhibitors of this invention) reduces or abolishes TNF-alpha upregulation.

Thus, the anti-inflammatory effects of CRD-$Leu_4Ile_{11}Cys_{13}$ peptide 3(3-12)[MCP-1] and other peptide 3 derivatives, analogs and variants are not limited to reducing or inhibiting macrophage (monocyte) accumulation but also inhibit recruitment of other leukocyte subsets, e.g., B cells, neutrophils, and CD4+ T lymphocytes, and inhibited the intra-lesional levels of TNF-α, IL-8 and MCP-1.

## Example 7

Use of a CRD-Peptide of the Invention in a Murine Endotoxemia Model

A mouse endotoxemia model is used to screen agents of the invention for *in vivo* functional anti-inflammatory activity in a rapid manner. Female CD-1 mice are injected i.p. (ventral abdomen) with 583 μg LPS. mRNA and protein levels of TNF-α, IFN-γ, IL-4 and MCP-1 and other markers of the inflammatory response are then determined. Thirty minutes prior to LPS administration, the animals were administered one of three different doses of CRD-$Leu_4Ile_{11}Cys_{13}$peptide 3(3-12)[MCP-1] as an intravenous loading dose and a subcutaneous bolus dose (on dorsum). PBS treated animals with and without LPS administration were positive and negative controls. Two hours later, animals were euthanized and serum collected. Serum was separated from the cell pellet and frozen until ELISA analysis of cytokine levels. Lung and liver samples were collected for mRNA analyses and histopathology. CRD-$Leu_4Ile_{11}Cys_{13}$peptide 3(3-12)[MCP-1] demonstrated a dose-dependent decrease in serum TNF-α. Serum levels and mRNA levels of IL-4, IFN-γ and MCP-1 are also determined.

Levels of serum MCP-1 and liver MCP-1 RNA were elevated in LPS treated animals with no modulation by CRD-$Leu_4Ile_{11}Cys_{13}$peptide 3(3-12)[MCP-1]. Levels of serum IL-4 and IFN-γ and liver IL-4 RNA were low or undetectable in all animals. Liver IFN-γ RNA was increased in all LPS treated animals. There was a dose dependent decrease in serum TNF-α in CRD-$Leu_4Ile_{11}Cys_{13}$peptide 3(3-12)[MCP-1] treated animals with statistical significance reached in the high dose group. Liver TNF-α RNA was high in all LPS treated animals.

Thus, CRD-$Leu_4Ile_{11}Cys_{13}$peptide 3(3-12)[MCP-1] treated mice may modulate the immune response via alterations in TNF-$\alpha$ levels.

## Example 8

Use of a CRD-Peptide of the Invention in Normal Monkeys and Mice, and in a Murine Asthma Model

To determine whether increasing doses of CRD-$Leu_4Ile_{11}Cys_{13}$ peptide 3(3-12)[MCP-1] alters the cell number and type of cell within the lungs of normal mice, mice were injected intravenously, intravenously and intratracheally, or intratracheally alone with CRD-$Leu_4Ile_{11}Cys_{13}$ peptide 3(3-12)[MCP-1]. Mice were sacrificed at 20-24 hours post injection. Lungs were collected for isolation of cells, which were subsequently counted and characterized by surface staining for CD3, CD4, CD8, B220, and Mac-1.

The total number of cells isolated from the lungs was higher in all groups receiving a low dose ofCRD-$Leu_4Ile_{11}Cys_{13}$ peptide 3(3-12)[MCP-1] (0.3 $\mu$g IV and/or 10 $\mu$g IT) compared to PBS-treated mice. There were no significant differences in the total number of cells isolated from lungs of mice treated with the high dose CRD-Leu4$Ile_{11}Cys_{13}$ peptide 3(3-12)[MCP-1] compared to PBS controls.

By FACS analysis, high dose CRD-$Leu_4Ile_{11}Cys_{13}$peptide 3(3-12)[MCP-1] significantly reduced the percentages of CD3, CD4, and B220 cells by all routes of administration compared to PBS controls. In contrast, there were not significant differences in the percentages of CD3, CD4, or B220 cells in the groups treated with low dose CRD-$Leu_4Ile_{11}Cys_{13}$ peptide 3(3-12)(MCP-1] by all routes of administration.

Three further studies assessed the ability of two increasing doses of CRD-$Leu_4Ile_{11}Cys_{13}$ peptide 3(3-12) [MCP-1] to reduce the pulmonary inflammatory infiltrate, inhibit IgE antibody increases, and alter the percentages of specific inflammatory cells in the lung and blood in mice challenged intratracheally with ovalbumin. See Gonzalo et al., J.Clin. Invest., 98, 2332 (1996); Gonzalo et al., J. Exp. Med., 188,157 (1998).

In the first study, the chemokine inhibitor was applied both before sensitization and prior to subsequent challenge. Mice were sensitized with 0.1 mg of ovalbumin (OVA) in 100 $\mu$l PBS (diluent control) intraperitoneally. Eight days following sensitization, mice received an intravenous loading dose (0.3 or 30 $\mu$g) and a subcutaneous depo dose (10 $\mu$g or 1 mg) of the pan-chemokine inhibitor CRD-$Leu_4Ile_{11}Cys_{13}$peptide 3(3-12)[MCP-1]. Thirty minutes following CRD-$Leu_4Ile_{11}Cys_{13}$peptide 3(3-12)[MCP-1] administration, mice were challenged with 1% ovalbumin or PBS (diluent control) intratracheally. Twenty-one days following sensitization, mice received a second intravenous loading dose (0.3 or 30 $\mu$g) and a subcutaneous dose (10 $\mu$g or 1 mg) of the pan-chemokine inhibitor CRD-$Leu_4Ile_{11}Cys_{13}$peptide 3(3-12)[MCP-1]. Thirty minutes following CRD-$Leu_4Ile_{11}Cys_{13}$peptide 3(3-12)[MCP-1] administration, mice were challenged with 2% ovalbumin or PBS (diluent control) intratracheally. Mice were sacrificed 3 hours post-ovalbumin challenge on day 21. Lungs were collected for histopathology and for isolation of cells for total cell counts and FACS analysis. PBLs were collected for FACS analysis.

By FACS analysis, there were significantly lower numbers of CD3, CD4, B220, and Mac-1 cells in the lungs of mice treated with both doses of CRD-$Leu_4Ile_{11}Cys_{13}$ peptide 3(3-12)[MCP-1] (0.3 IV/10 $\mu$g subcutaneously or 30 $\mu$g IV/1 mg subcutaneously) compared to mice which received PBS prior to challenge with OVA. The percentage of CD8 cells was similar in all groups. In addition, the total number of cells isolated from lungs of CRD-$Leu_4Ile_{11}Cys_{13}$ peptide 3(3-12)[MCP-1] mice was similar to PBS-treated mice but significantly lower than mice treated with OVA and PBS, suggesting that the agent altered trafficking of inflammatory cells into the lung. In the blood, there were significantly higher percentages of CD3 and CD4 cells and lower percentages of B220 in mice treated with both doses ofCRD-$Leu_4Ile_{11}Cys_{13}$ peptide 3(3-12)[MCP-1] compared to OVA-treated mice (positive control) and to PBS-treated mice (diluent control). Mice treated with the high dose had fewer Mac-1 cells in the PBL compartment compared to all other groups.

Histologically, all mice treated with the high dose CRD-$Leu_4Ile_{11}Cys_{13}$ peptide 3(3-12)[MCP-1] had minimal to no inflammatory infiltrates in the lung, similar to mice treated with PBS alone. Mice that received low dose CRD-$Leu_4Ile_{11}Cys_{13}$peptide 3(3-12)[MCP-1] also had minimal inflammation compared to mice treated with PBS and OVA. Rare eosinophils were seen only in the PBS OVA group (positive control), which is an expected response to OVA sensitization.

IgE levels were significantly higher in mice treated with PBS and OVA compared to all other groups. IgE was not detectable above background in all groups of mice treated with CRD-$Leu_4Ile_{11}Cys_{13}$ peptide 3(3-12)[MCP-1]. Thus, treatment with the pan-chemokine inhibitor CRD-$Leu_4Ile_{11}Cys_{13}$ peptide 3(3-12)[MCP-1] before sensitization and before challenge prevented any inflammatory response being set up in response to OVA.

A second study in the ovalbumin-induced hypersensitivity model was then performed to determine whether CRD-$Leu_4Ile_{11}Cys_{13}$ peptide 3(3-12)(MCP-1] could reduce the inflammatory response to OVA in mice which had been sensitized in the absence of test agent. Mice were sensitized with 0.1 mg of ovalbumin or PBS (diluent control) intraperitoneally. Eight days following sensitization, mice received a subcutaneous dose (10.3 $\mu$g, 103 $\mu$g, or 1.03 mg) of the pan-chemokine inhibitor CRD-$Leu_4Ile_{11}Cys_{13}$ peptide 3(3-12)[MCP-1]. Thirty minutes following CRD-$Leu_4Ile_{11}Cys_{13}$

peptide 3(3-12)[MCP-1] administration, mice were challenged with 1% ovalbumin or PBS (diluent control) intratracheally. Fifteen, eighteen, and twenty-one days following sensitization, mice received subcutaneous doses (10.3 μg, or 103 μg, or 1.03 mg) of the pan-chemokine inhibitor CRD-$Leu_4Ile_{11}Cys_{13}$ peptide 3(3-12)[MCP-1]. Thirty minutes following CRD-$Leu_4Ile_{11}Cys_{13}$ peptide 3(3-12)[MCP-1] administration, mice were challenged with 2% ovalbumin or PBS intratracheally, on day 21. Mice were sacrificed 3 hours post-challenge. Lungs were collected for histopathology and for isolation of cells for total cell counts and FACS analysis. Serum was collected for IgE and IL-4 levels.

Dose-dependent reductions in IgE (60%), IL-4 (85%), total cells in the lung (approximately 50%) and macrophages (74%) were observed (Figure 16A).

By FACS analysis, there were significantly lower percentages of macrophages in the lungs of mice treated with 100 μg of CRD-$Leu_4Ile_{11}Cys_{13}$ peptide 3(3-12)[MCP-1] compared to mice which received PBS only prior to challenge with OVA (Figure 16B). Thus, CRD-$Leu_4Ile_{11}Cys_{13}$ peptide 3(3-12)[MCP-1] alters trafficking of these cells. Histologically, all mice treated with

the high or medium dose CRD-$Leu_4Ile_{11}Cys_{13}$ peptide 3(3-12)[MCP-1] had fewer inflammatory infiltrates in the lung compared to mice that were not treated with the peptide but challenged with OVA (positive control). Mice treated with PBS alone had minimal to no inflammation in the lung. All mice challenged with OVA had eosinophils in the lung. Similar to mice treated with PBS only (negative control), IgE levels were significantly lower in mice treated with CRD-$Leu_4Ile_{11}Cys_{13}$ peptide 3(3-12)[MCP-1] compared to mice treated with PBS and OVA (positive control) (Figure 16B). Likewise, serum IL-4 levels were significantly reduced in mice (Figure 16C). Thus, treatment with CRD-$Leu_4Ile_{11}Cys_{13}$ peptide 3(3-12)[MCP-1] just prior to OVA challenge significantly reduces the inflammatory response to OVA in sensitized mice.

A third study assessed the efficacy of daily dosing with CRD-$Leu_4Ile_{11}Cys_{13}$ peptide 3(3-12)[MCP-1] to reduce OVA-induced pulmonary inflammation. Mice were sensitized with 0.1 mg of ovalbumin or PBS (diluent control) intraperitoneally in the absence of the test agent. Eight days following initial sensitization, mice were treated with either 10.3 μg or 1.03 mg CRD-Leu4$Ile_{11}Cys_{13}$ peptide 3(3-12)[MCP-1], or 1.03 mg of the inactive D-ala peptide, by the subcutaneous route. These treatments were given daily from day 8 through day 21. On day 8, thirty minutes following treatment, mice were challenged intratracheally with 1% ovalbumin or PBS. On days 15, 18, and 21, thirty minutes following treatment, mice were challenged intratracheally with 2% ovalbumin or PBS. Mice were sacrificed 3 hours post-ovalbumin challenge, on day 21. Lungs were collected for histopathology, and for isolation of cells for total cell counts and FACS analysis. Bronchalveolar lavage (BAL) was collected for eicosinoid levels. Serum was collected for IgE and IL-4 levels. Spleens were collected for cytokine recall responses.

There were significantly ($p<0.05$) fewer total cells in the lungs of mice treated with the low dose (10.3 μg) CRD-$Leu_4Ile_{11}Cys_{13}$ peptide 3(3-12)[MCP-1], as compared to positive controls or mice treated with the inactive peptide (Figure 41). By FACS analysis, there were significantly lower numbers of macrophages, and B cells in the lungs of mice treated with the low dose (10.3 μg) of CRD-$Leu_4Ile_{11}Cys_{13}$ peptide 3(3-12)[MCP-1] as compared to positive controls or mice treated with the inactive peptide (Figure 40). In addition, B cells were significantly ($p<0.05$) reduced in mice treated with high dose (1.03 mg) of CRD-$Leu_4Ile_{11}Cys_{13}$ peptide 3(3-12)[MCP-1] as compared to controls (Figure 40). There was no significant differences in T cell number. Histologically, all mice treated with the high or medium dose CRD-$Leu_4Ile_{11}Cys_{13}$ peptide 3(3-12)[MCP-1] had fewer inflammatory infiltrates in the lung compared to mice that were not treated with the peptide but challenged with OVA (positive control) or treated with the inactive peptide, although the inflammation is reduced, not eliminated. Mice treated with PBS alone had minimal to no inflammation in the lung. All mice challenged with OVA had eosinophils in the lung, including those mice treated with CRD-$Leu_4Ile_{11}Cys_{13}$ peptide 3(3-12)[MCP-1].

In a series of *in vitro* experiments, the recall responses of culture splenocytes from the sensitized animals to OVA were performed. Splenocytes from mice treated with CRD-$Leu_4Ile_{11}Cys_{13}$peptide 3(3-12)[MCP-1] produced significantly less IL-4 in response to OVA (as did splenocytes from unsensitized mice) than splenocytes from untreated mice (OVA positive control) or from mice treated with inactive peptide (Figure 40). IgE levels were significantly reduced ($p<0.05$) in mice treated with all doses of CRD-$Leu_4Ile_{11}Cys_{13}$ peptide 3(3-12)[MCP-1] compared to controls (Figure 41). The inactive peptide significantly reduced ($p<0.05$) thromboxane B2 in the BAL (Figure 40). There were no significant differences in eicosinoid levels (thromboxane B2, leukotriene B4, or prostaglandin E2), although different volumes may introduce artifactual differences and large variations within groups.

These results established that CRD-$Leu_4Ile_{11}Cys_{13}$ peptide 3(3-12)[MCP-1], when delivered daily between days 8 and 21, by subcutaneous injection just prior to OVA challenge, reduced the trafficking of macrophages and B cells into the lung following exposure to the antigen OVA. More significantly, CRD-$Leu_4Ile_{11}Cys_{13}$ peptide 3(3-12)[MCP-1] reduced IgE antibody levels in the serum, and IL-4 levels in supernatants from spleen recall cultures, which are strongly associated with asthma. IgE responses are dependent on a Th2 cell response, which produces IL-4 and IL-5. Therefore, the observation that CRD-$Leu_4Ile_{11}Cys_{13}$ peptide 3(3-12)[MCP-1] has an effect on reducing IgE upon challenge with OVA strongly indicates that CRD-$Leu_4Ile_{11}Cys_{13}$ peptide 3(3-12)[MCP-1] reduces IL-4 and IL-5.

In another study, BALB/c mice were sensitized with 0.1 mg of ovalbumin (OVA) or PBS (diluent control) intraperitoneally. Eight days following initial sensitization mice were challenged intratracheally with 1% ovalbumin or PBS (diluent control). On days fifteen, eighteen, twenty one, and thirty four, mice were challenged intratracheally with 2% ovalbumin of PBS. To determine if a single dose of CRD-$Leu_4Ile_{11}Cys_{13}$ peptide 3(3-12)[MCP-1 had an effect on reducing pulmonary inflammation, IL-4 or IgE in mice with an established asthma phenotype, mice were treated with a 100 μg i.v. bolus of CRD-$Leu_4Ile_{11}Cys_{13}$ peptide 3(3-12)[MCP-1] or PBS. Thirty minutes following treatment, mice were challenged intratracheally with 2% OVA and sacrificed 3 hours after OVA challenge. To determine if daily treatment with 100 μg of CRD-$Leu_4Ile_{11}Cys_{13}$ peptide 3(3-12)[MCP-1] caused regression of pulmonary inflammation and reduced IL-4 and IgE levels, mice were injected subcutaneously from day 21 to day 34 with CRD-$Leu_4IleCys_{13}$ peptide 3(3-12) [MCP-1] or PBS. On day 34 mice were re-challenged intratracheally with either 2% OVA or PBS. Mice were sacrificed 3 hours after challenge. Following sacrifice on day 21 or 34, lungs were collected for histopathology and for isolation of cells for total cell counts and identification by FACS analysis. Serum was collected for IgE and IL-4 levels. Spleens were collected for cytokine and antibody recall responses.

Mice treated daily with CRD-$Leu_4Ile_{11}Cys_{13}$ peptide 3(3-12)[MCP-1] for 2 weeks, then re-challenged with OVA, had significantly ($p<0.01$) fewer total cells in the lungs compared to mice treated with PBS and re-challenged with OVA or PBS (positive controls) (Figure 27A). By FACS analysis, there were also significantly lower numbers of macrophages, B cells, and CD4+ T cells in the lungs of mice treated with CRD-$Leu_4Ile_{11}Cys_{13}$ peptide 3(3-12)[MCP-1] compared to positive controls (Figure 27B, C and D). In addition, mice treated with CRD-$Leu_4Ile_{11}Cys_{13}$ peptide 3(3-12)[MCP-1] had significantly ($p<0.05$) reduced levels of serum IgE compared to positive controls (Figure 28 and Table 10). Following stimulation of splenocytes with OVA (no CRD-$Leu_4Ile_{11}Cys_{13}$ peptide 3(3-12)[MCP-1] added), there were significantly ($p<0.05$) reduced levels of IL-4, IgE, and total IgG and IgM in culture supernatants from mice treated in vivo with CRD-$Leu_4Ile_{11}Cys_{13}$ peptide 3(3-12)[MCP-1] compared to positive controls (Figures 30A, B and C; Table 9). Complete suppression of antibody production persisted up to 1 week in recall cultures (Figures 30 Aand B; Tables 8 and 11).

However, following 2 weeks in culture, antibody levels in supernatants from CRD-$Leu_4Ile_{11}Cys_{13}$ peptide 3 (3-12)[MCP-1] treated mice were higher than negative controls, equivalent to mice that were re-challenged with PBS, and significantly ($p<0.05$) lower than mice re-challenged with OVA (Figures 30A and B). Histologically, lungs from mice treated daily with CRD-$Leu_4Ile_{11}Cys_{13}$ peptide 3(3-12)[MCP-1] resembled normal mice and had markedly fewer inflammatory infiltrates in the lung compared to mice that were treated with PBS and re-challenged with OVA (positive control). Mice treated with PBS alone and never challenged with OVA had minimal to no inflammation in the lung. All mice challenged with OVA had eosinophils in the lung. However, those mice treated with CRD-$Leu_4Ile_{11}Cys_{13}$ peptide 3(3-12) [MCP-1] had fewer of any inflammatory cells including eosinophils.

In those mice treated with only a single dose of CRD-$Leu_4Ile_{11}Cys_{13}$ peptide 3(3-12)[MCP-1] prior to challenge with OVA on day 21, and sacrificed on day 21, there was no significant reduction of total cells in the lungs (Figure 29D). However, following stimulation of splenocytes with OVA, there was significantly ($p<0.05$) reduced IL-4 and IgE levels compare to PBS treated mice (Figure 29E and 38).

This established that CRD-$Leu_4Ile_{11}Cys_{13}$ peptide 3(3-12)[MCP-1], when delivered daily for 2 weeks to mice with an established asthma phenotype, caused regression of the pulmonary inflammation. In addition, upon rechallenge with OVA the CRD-$Leu_4Ile_{11}Cys_{13}$ peptide 3(3-12)[MCP-1] treated mice had reduced trafficking of macrophages, B cells, and CD4+T cells into the lungs, suggesting that treatment blocked recurrence of inflammation to the site of antigen exposure. More significantly, CRD-$Leu_4Ile_{11}Cys_{13}$ peptide 3(3-12)[MCP-1] reduced serum IgE antibody levels, and IL-4 levels in supernatants from spleen recall cultures. There was also a striking reduction of total IgG and IgM antibody produced in splenocyte cultures.

**TABLE 7**

| | Total IgM + IgG (1 week splenocyte cultures) | | | | | | | |
|---|---|---|---|---|---|---|---|---|
| **Group** | | **μg/ml Ova** | **m1** | **m2** | **m3** | **m4** | **Avg.** | **STDEV** |
| 1 | Ova on days 8, 15, 18, 21 | 0 | 26.92 | 758.47 | 49.74 | 538.73 | 343.46 | 363.70 |
| 1 | PBS treat 1 × day 21 | 5 | 357.56 | 21.08 | ND | ND | 150.46 | 237/93 |
| 1 | sacrifice day 21 | 50 | 71.72 | 55.4 | 142.98 | 46.54 | 79.16 | 43.80 |
| **Group** | | **μg/ml Ova** | **m1** | **m2** | **m3** | **m4** | **Avg.** | **STDEV** |
| 2 | Ova on days 8, 15, 18, 21 | 0 | 108.00 | 24.97 | 40.65 | 56.17 | 57.45 | 36.03 |
| 2 | CRD-$Leu_4Ile_{11}Cys_{13}$ peptide 3(3- | 5 | 254.36 | 46.56 | ND | ND | 150.46 | 146.94 |

(continued)

| Group | | μg/ml Ova | m1 | m2 | m3 | m4 | Avg. | STDEV |
|---|---|---|---|---|---|---|---|---|
| | 12)[MCP-1] treat 1 × day 21 | | | | | | | |
| 2 | sacrifice day 21 | 50 | 137.57 | 176.41 | 331.79 | 321.23 | 241.75 | 99.24 |
| **Group** | | **μg/ml Ova** | **m1** | **m2** | **m3** | **m4** | **Avg.** | **STDEV** |
| 3 A+B | Ova on days 8, 15, 18, 21 & 35 | 0 | 20.19 | 66.09 | 12.48 | 18.97 | 29.43 | 24.67 |
| 3 A+B | PBS treat BID 14 days | 5 | 1145.29 | 1099.18 | 278.52 | 425.36 | 737.09 | 449.15 |
| 3 A+B | sacrifice day 35 (recent Ova challenge) | 50 | 1424.09 | 1341.16 | 289.83 | 1191.84 | 1061.73 | 523.50 |
| **Group** | | **μg/ml Ova** | **m9** | **m10** | **m11** | **m12** | **Avg.** | **STDEV** |
| 3 C+D | Ova on days 8, 15, 18, 21 | 0 | 20.26 | 24.73 | 13.32 | 24.89 | 20.80 | 5.43 |
| 3 C+D | PBS treat BID 14 days | 5 | 158.69 | 182.09 | 206.16 | 145.68 | 173.15 | 26.67 |
| 3 C+D | sacrifice day 34 (No recent Ova challenge) | 50 | 333.07 | 688.18 | 521.93 | 177.71 | 430.22 | 222.22 |
| **Group** | | **μg/ml Ova** | **m1** | **m2** | **m3** | **m4** | **Avg.** | **STDEV** |
| 4 | Ova on days 8, 15, 18, 21 & 35 | 0 | 15.32 | 25.86 | 14.87 | 12.60 | 17.16 | 5.92 |
| 4 | CRD-$Leu_4Ile_{11}Cys_{13}$ peptide 3(3-12)[MCP] treat BID 14 days | 5 | 189.07 | 249.75 | 148.16 | 68.83 | 163.95 | 75.92 |
| 4 | sacrifice day 35 (recent Ova challenge) | 50 | 338.50 | 679.86 | 487.87 | 161.20 | 416.86 | 220.39 |
| **Group** | | **μg/ml Ova** | **m1** | **m2** | **m3** | **m4** | **Avg.** | **STDEV** |
| 5 | PBS on days 8, 15, 18, 21 | 0 | 26.54 | 15.44 | 21.35 | 16.89 | 20.05 | 5.00 |
| 5 | CRD-$Leu_4Ile_{11}Cys_{13}$ peptide 3(3-12)[MCP-1] treat BID 14 days | 5 | 64.20 | 31.21 | 67.94 | 49.53 | 53.22 | 16.69 |
| 5 | sacrifice day 34 (No recent Ova challenge) | 50 | 86.16 | 80.04 | 189.70 | 89.74 | 111.41 | 52.35 |
| **Group** | | **μg/ml Ova** | **m1** | **m2** | **m3** | **m4** | **Avg.** | **STDEV** |
| 6 | No Ova stim. | 0 | 10.74 | 14.80 | | | 12.77 | 2.87 |
| 6 | PBS treat BID 14 days | 5 | ND | ND | | | | |
| 6 | sacrifice day 35 | 50 | 75.09 | 77.64 | | | 76.36 | 1.80 |

**TABLE 8**

**IL-4 Assays**

| Group | | μg/ml Ova | pg/ml IL-4<br>m1 | m2 | m3 | m4 | Avg. of Group |
|---|---|---|---|---|---|---|---|
| 1 | Ova on days 8, 15, 18, 21 | 0 | 4.90 | 1.30 | 3.40 | 12.60 | 6.08 |

(continued)

| | | | IL-4 Assays | | | | |
|---|---|---|---|---|---|---|---|
| | | | pg/ml IL-4 | | | | |
| **Group** | | **μg/ml Ova** | **m1** | **m2** | **m3** | **m4** | **Avg. of Group** |
| 1 | PBS treat 1 × day 21 | 5 | 16.30 | 23.30 | ND | ND | 19.80 |
| 1 | sacrifice day 21 | 50 | 56.70 | 21.30 | 59.70 | 70.10 | 58.20 |
| **Group** | | **μg/ml Ova** | **m1** | **m2** | **m3** | **m4** | **Avg. of Group** |
| 2 | Ova on days 8, 15, 18, 21 | 0 | 2.81 | 5.60 | 0.25 | 0.45 | 2.89 |
| 2 | CRD-$Leu_4Ile_{11}Cys_{13}$ peptide 3 (3-12)[MCP-1] treat 1 × day 21 | 5 | 3.18 | 8.55 | ND | ND | 5.86 |
| 2 | sacrifice day 21 | 50 | 7.10 | 11.46 | 19.15 | 75.36 | 12.57 |
| **Group** | | **μg/ml Ova** | **m1** | **m2** | **m3** | **m4** | **Avg. of Group** |
| 3 A+B | Ova on days 8, 15, 18, 21 & 35 | 0 | 2.87 | 4.71 | 2.01 | 3.18 | 3.19 |
| 3 A+B | PBS treat BID 14 days | 5 | 31.33 | 49.16 | 15.69 | 22.03 | 29.55 |
| 3 A+B | sacrifice day 35 (recent Ova challenge) | 50 | 51.96 | 76.72 | 52.41 | 46.58 | 50.84 |
| **Group** | | **μg/ml Ova** | **m9** | **m10** | **m11** | **m12** | **Avg. of Group** |
| 3 C+D | Ova on days 8, 15, 18, 21 | 0 | 5.90 | 5.15 | 0.87 | 3.52 | 3.86 |
| 3 C+D | PBS treat BID 14 days | 5 | 16.10 | 7.55 | 7.80 | 6.04 | 9.37 |
| 3 C+D | sacrifice day 34 (No recent Ova challenge) | 50 | 18.46 | 17.23 | 11.17 | 14.80 | 15.42 |
| **Group** | | **μg/ml Ova** | **m1** | **m2** | **m3** | **m4** | **Avg. of Group** |
| 4 | Ova on days 8, 15, 18, 21 & 35 | 0 | 2.61 | 1.11 | 5.08 | 2.88 | 2.92 |
| 4 | CRD-$Leu_4Ile_{11}Cys_{13}$ peptide 3 (3-12)[MCP-1] treat BID 14 days | 5 | 10.40 | 12.77 | 4.59 | 4.45 | 8.05 |
| 4 | sacrifice day 35 (recent Ova challenge) | 50 | 14.38 | 22.08 | 10.25 | 11.09 | 14.45 |
| **Group** | | **μg/ml Ova** | **m1** | **m2** | **m3** | **m4** | **Avg. of Group** |
| 5 | Ova on days 8, 15, 18, 21 | 0 | 9.09 | 3.80 | 12.89 | 6.42 | 8.05 |
| 5 | CRD-$Leu_4Ile_{11}Cys_{13}$ peptide 3 (3-12)[MCP-1] treat BID 14 days | 5 | 5.70 | 4.43 | 10.09 | 5.10 | 6.33 |
| 5 | sacrifice day 34 (No recent Ova challenge) | 50 | 7.02 | 6.10 | 4.74 | 4.59 | 5.61 |
| **Group** | | **μg/ml Ova** | **m1** | **m2** | **m3** | **m4** | **Avg. of Group** |
| 6 | No Ova stim. | 0 | 1.57 | 1.88 | 1.48 | 2.34 | 1.81 |
| 6 | PBS treat BID 14 days | 5 | 4.91 | 3.61 | 5.22 | 3.26 | 4.25 |
| 6 | sacrifice day 35 | 50 | 3.83 | 4.88 | 3.32 | 4.39 | 4.10 |

**TABLE9**

**Total IgE (Serum)**

| | **m1** | **m2** | **m3** | **m4** | **Avg.** | **ng/ml IgE (4X avg.)** |
|---|---|---|---|---|---|---|
| **Group 1** | 1.317 | 1.812 | 1.425 | 2.419 | 1.743 | 6.973 |
| **Group 2** | 1.952 | 1.132 | 2.073 | 0.863 | 1.505 | 6.020 |
| **Group 3 A, B** | 1.419 | 0.881 | 1.477 | 1.295 | 1.268 | 5.072 |
| **Group 3 C, D** | 1.487 | 2.614 | 1.519 | 1.460 | 1.770 | 7.080 |
| **Group 4** | 0.310 | 0.298 | 0.984 | 0.890 | 0.621 | 2.482 |
| **Group 5** | 0.625 | 1.870 | 0.838 | 1.073 | 1.102 | 4.406 |
| **Group 6** | 1.228 | 0.552 | 0.564 | 0.654 | 0.750 | 2.998 |

**TABLE 10**
**Total IgE (1 week splenocyte cultures)**

| Group | | μg/ml Ova | m1 | m2 | m3 | m4 | Avg. | ng/ml (=4) |
|---|---|---|---|---|---|---|---|---|
| 1 | Ova on day | 0 | 0.191 | 0.007 | 0.000 | 0.000 | 0.050 | 0.198 |
| 1 | PBS treat | 5 | 0.161 | 0.000 | ND | ND | 0.081 | 0.322 |
| 1 | sacrifice day | 50 | 0.122 | 0.005 | 0.012 | 0.029 | 0.042 | 0.168 |
| **Group** | | **μg/ml Ova** | **m1** | **m2** | **m3** | **m4** | **Avg.** | **ng/ml** |
| 2 | Ova on day | 0 | 0.000 | 0.051 | 0.007 | 0.009 | 0.017 | 0.067 |
| 2 | CRD-$Leu_4Ile_{11}Cys_{13}$ peptide 3(3-12)[MCP-1] | 5 | 0.041 | 0.023 | ND | ND | 0.032 | 0.128 |
| 2 | sacrifice day | 50 | 0.011 | 0.002 | 0.004 | 0.007 | 0.006 | 0.024 |
| **Group** | | **μg/ml Ova** | **m1** | **m2** | **m3** | **m4** | **Avg.** | **ng/ml** |
| 3 A+B | Ova on day | 0 | 0.022 | 0.034 | 0.009 | 0.007 | 0.018 | 0.072 |
| 3 A+B | PBS treat | 5 | 0.074 | 0.165 | 0.041 | 0.013 | 0.073 | 0.293 |
| 3 A+B | sacrifice day (recent Ova challenge) | 50 | 0.098 | 0.175 | 0.010 | 0.011 | 0.074 | 0.294 |
| **Group** | | **μg/ml Ova** | **m9** | **m10** | **m11** | **m12** | **Avg.** | **ng/ml** |
| 3 C+D | Ova on day | 0 | 0.009 | 0.000 | 0.000 | 0.030 | 0.010 | 0.039 |
| 3 C+D | PBS treat | 5 | 0.000 | 0.000 | 0.000 | 0.031 | 0.008 | 0.031 |
| 3 C+D | sacrifice day (No recent Ova challenge) | 50 | 0.008 | 0.000 | 0.054 | 0.009 | 0.018 | 0.071 |
| **Group** | | **μg/ml Ova** | **m1** | **m2** | **m3** | **m4** | **Avg.** | **ng/ml** |
| 4 | Ova on day | 0 | 0.008 | 0.001 | 0.015 | 0.002 | 0.007 | 0.026 |
| 4 | CRD-$Leu_4Ile_{11}Cys_{13}$peptide 3(3-12)[MCP-1] | 5 | 0.009 | 0.047 | 0.009 | 0.031 | 0.024 | 0.096 |
| 4 | sacrifice day (recent Ova challenge) | 50 | 0.013 | 0.043 | 0.008 | 0.018 | 0.021 | 0.082 |
| **Group** | | **μg/ml Ova** | **m1** | **m2** | **m3** | **m4** | **Avg.** | **ng/ml** |
| 5 | Ova on day | 0 | 0.008 | 0.033 | 0.000 | 0.024 | 0.016 | 0.065 |
| 5 | CRD-$Leu_4Ile_{11}Cys_{13}$ peptide 3(3-12)[MCP-1] | 5 | 0.003 | 0.065 | 0.000 | 0.014 | 0.021 | 0.082 |
| 5 | sacrifice day (No recent Ova challenge) | 50 | 0.000 | 0.020 | 0.000 | 0.001 | 0.005 | 0.021 |
| **Group** | | **μg/ml Ova** | **m1** | **m2** | **m3** | **m4** | **Avg.** | **ng/ml** |
| 6 | No Ova stim. | 0 | 0.000 | 0.014 | 0.004 | lost | 0.006 | 0.024 |
| 6 | PBS treat | 5 | 0.000 | 0.001 | lost | lost | 0.001 | 0.002 |
| 6 | sacrifice day | 50 | 0.004 | 0.007 | lost | lost | 0.006 | 0.022 |

Cynomolgus monkeys were immunized with antigen that is a single chain Fv (scFv) protein. Each animal previously received 3 i.v. injections of the antigen and all developed high serum titers. Five animals were randomly divided into 2 groups. Two animals served as PBS treated controls and 3 were treated i.v. with 50 mg/kg CRD-$Leu_4Ile_{11}Cys_{13}$ peptide 3(3-12)[MCP-1]. Thirty minutes following CRD-$Leu_4Ile_{11}Cys_{13}$ peptide 3(3-12)[MCP-1] treatment all animals were challenged i.v. with 3.5 mg/kg antigen. In addition, animals were challenged intradermal with each of the following in a volume of 50 μl:PBS; 50 μg antigen; 5 μg antigen; 0.5 μg antigen; 500 ng MCP-1; or 50 ng LPS. Serum was collected at 10 minutes, 30 minutes, 60 minutes, 4 hours, and 24 hours. Animals were sacrificed 24 hours after CRD-$Leu_4Ile_{11}Cys_{13}$peptide 3(3-12)[MCP-1] injection and complete necropsy performed and tissues fixed for histopathology. Fixed and frozen skin samples were harvested for histopathology and immunohistochemistry. Blood, spleen, and lymph node were harvested for assessment of cell function in migration assay, proliferation assay, and antigen specific antibody and cytokine recall. Blood was also analyzed pre- and post-CRD-$Leu_4Ile_{11}Cys_{13}$ peptide 3 (3-12)[MCP-1] injection for hematology parameters and serum chemistry. Serum levels were evaluated by an LC-MS method (limit of quantitation = 1 μg/ml) pre-treatment and then at 10 minutes, 30 minutes, 60 minutes, 4 hours and 20-24 hours after injection. Urinalysis was performed pre- and post-CRD-$Leu_4Ile_{11}Cys_{13}$ peptide 3(3-12)[MCP-1] injection.

Compared to PBS treated controls (animal numbers 137 and 144), all three monkeys treated with a single dose

of CRD-$Leu_4Ile_{11}Cys_{13}$ peptide 3(3-12)[MCP-1] (animal numbers 138, 141, and 143) had the lowest detectable levels of antigen specific antibody in recall responses of splenocytes, lymph node cells, and peripheral blood lymphocytes (PBL's from one of the positive control animals was not evaluated due to insufficient cell numbers for culturing) (Figure 33). IL-4 was detected in a single animal (#144) which was one of the PBS treated controls. In the migration assay and the proliferation assay, there was no consistent responder vs non-responder animals. Intradermal injection sites were evaluated histologically,

however, there was no inflammatory response to the 2 positive controls (MCP-1 or LPS) so no further analysis was performed on these samples. No clinically significant changes were noted in the hematology results in any animal. No clinically significant changes were noted in the serum chemistry profiles following treatment with CRD-$Leu_4Ile_{11}Cys_{13}$ peptide 3(3-12)[MCP-1]. The levels of CRD-$Leu_4Ile_{11}Cys_{13}$ peptide 3(3-12)[MCP-1] in the serum were measured using LC-MS. No CRD-$Leu_4Ile_{11}Cys_{13}$peptide 3(3-12)[MCP-1] was detected in monkeys administered PBS, while CRD-$Leu_4Ile_{11}Cys_{13}$ peptide 3(3-12)[MCP-1] was detected in monkeys administered 50 mg/kg CRD-$Leu_4Ile_{11}Cys_{13}$ peptide 3(3-12)[MCP-1]. Urine PH was decreased, and specific gravity increased in some animals in both groups following treatment. There was a small amount of protein in the urine of all three animals treated with CRD-$Leu_4Ile_{11}Cys_{13}$ peptide 3(3-12)[MCP-1]. Some animals in both groups had ketones in the urine. All three treated animals had detectable drug levels for the first 4 hours after treatment (Figure 41). By 20-24 hours, drug levels had fallen below the level of quantitation (<1 $\mu$g/ml).

This study established that CRD-$Leu_4Ile_{11}Cys_{13}$ peptide 3(3-12)[MCP-1], when delivered in a single dose, resulted in reduced antigen specific antibody response in splenocyte, lymph node, and peripheral blood lymphocyte recall responses. There was no consistent response of lack of response in the other assays performed. Small sample size prevents statistical analysis. It is unknown why an intradermal inflammatory response was not invoked at the site of agonist challenge. Thus, a single dose of CRD-$Leu_4Ile_{11}Cys_{13}$ peptide 3(3-12)[MCP-1] prevents antigen specific antibody responses when measured in a recall response in 2 different species.

The data also indicated that CRD-$Leu_4Ile_{11}Cys_{13}$ peptide 3(3-12)[MCP-1] causes regression of established inflammatory responses, thus indicating that chemokine inhibition also results in the reduction of T and B lymphocyte recall responses. T lymphocyte recall responses were measured by the assessment of IL-4 production by cells stimulated with ovalbumin. B lymphocyte recall responses were measured by the assessment of immunoglobulin production by cells stimulated with ovalbumin. In both cases, a single dose of CRD-$Leu_4Ile_{11}Cys_{13}$ peptide 3(3-12)[MCP-1] *in vivo* resulted in a statistically significant reduction in the ability of the cells to produce IL-4, IgG + IgM or IgE in response to antigen challenge.

ovalbumin. In both cases, a single dose of CRD-$Leu_4Ile_{11}Cys_{13}$ peptide 3(3-12)[MCP-1] *in vivo* resulted in a statistically significant reduction in the ability of the cells to produce IL-4, IgG + IgM or IgE in response to antigen challenge.

This is the first time a chemokine inhibitor has been shown to inhibit recall responses in B and T cells to a known allergen. This is very important in the treatment of allergic disease and is also very important in autoimmune diseases in which aberrant increases in IL-4 or Ig contribute to the pathophysiology, e.g., asthma, contact hypersensitivity, allergic rhinitis, rheumatoid arthritis, inflammatory bowel disease, as well as diseases that are mediated by aberrant immunoglobulin production or hypergammaglobulinemia, for example, immune-mediated glomerulonephritis, multiple myeloma (particularly patients with Bence Jones proteins (i.e., immunoglobulin in urine), autoimmune hemolytic anemia, myasthenia gravis, Goodpastures syndrome (anti-glomerular basement membrane induced nephritis), autoimmune uveitis, autoimmune thyroiditis, and autoimmune pancreatic islet cell destruction. Further, the agents of the invention may be useful in indications in which an antibody response to an antigen is to be suppressed (e.g., in conjunction with some immunotherapeutic products which have dose-limiting immunogenicity, such as antibody-based products).

Thus, CRD-$Leu_4Ile_{11}Cys_{13}$ peptide 3(3-12)[MCP-1], when delivered IV and subcutaneously, or subcutaneously alone, altered the trafficking of lymphocytes into the lung following exposure to an antigen. More significantly, CRD-$Leu_4Ile_{11}Cys_{13}$ peptide 3(3-12)[MCP-1] reduced the cellular inflammation in the lung, IgE responses and IL-4 concentration in the serum, which are strongly associated with asthma. IgE responses are dependent on a Th2 T cell response, which produces IL-4 and IL-5. Therefore, the observation that CRD-$Leu_4Ile_{11}Cys_{13}$ peptide 3(3-12)[MCP-1] has an effect on reducing IgE upon challenge with OVA strongly indicates that CRD-$Leu_4Ile_{11}Cys_{13}$ peptide 3(3-12)[MCP-1] may reduce IL-4 and IL-5 also.

## Example 9

Preferred Tripeptides and Analogs Thereof

Preferred tripeptides of the invention include KXK peptides, where X is one of the twenty naturally occurring amino acids, e.g., KQK and KLK, as well as peptides having KXK. As described below, KXK peptides are anti-inflammatory by two distinct mechanisms. Some KXK peptides are TGF-beta activators and others are chemokine antagonists, and a subset are both (see Table 11).

**TABLE 11**

| Peptide | TGF-beta Activator | Chemokine antagonist |
|---|---|---|
| KAK | n.d. | + |
| KCK | n.d. | n.d. |
| KDK | - | + |
| KEK | - | + |
| KFK | ++++ | - |
| KGK | - | - |
| KHK | n.d. | n.d. |
| KIK | ++ | ++ |
| KKK | - | ++++ |
| KLK | +++ | ++++ |
| KMK | n.d. | n.d. |
| KNK | n.d. | +++ |
| KPK | n.d. | n.d. |
| KQK | - | +++++ |
| KRK | n.d. | n.d. |
| KSK | - | - |
| KTK | - | - |
| KVK | n.d. | n.d. |
| KWK | - | - |
| KYK | +++++ | - |

To test whether a KXK tripeptide activates TGF-beta, a direct ELISA-type assay can be used. Recombinant human latent TGF-β1 produced in CHO cells (R&D Systems) was incubated with the test activator. For example, 200 ng of latent TGF-β1 (at 20 μg/ml) was incubated with test peptide at 100 nM final concentration at 37°C for 90 minutes. Following incubation, the TGF-β is incubated with the recombinant extracellular domain of the Type II TGF-β receptor (R2X) which binds only the active and not the latent forms of TGF-β1 (Clin. Chim. Acta, 235, 11 (1995)). For example, 1 μg of purified R2X is coated onto a Maxisorp ELISA plate well in 50 μl of 100 mM sodium carbonate for 2 hours at 4°C, and non-specific protein binding then blocked incubation with 5% sucrose 5% Tween-20 in Tris-buffered saline for 1 hour at room temperature.

The TGF-β sample is then incubated with the coated and blocked wells for 2 hours at room temperature with shaking. Wells are washed 3 times quickly with Tris-buffered saline containing 0.05% Tween-20 between each incubation. If any of the latent TGF-β1 has been activated by the incubation with test peptide, it is captured by the R2X, while remaining latent TGF-β1 is washed away. Captured active TGF-β1 is then detected by incubation with a suitable detection agent, such as a peroxidase conjugated polyclonal anti-TGF-beta antibody. For example, the wells are incubated with 200 μl of BDA19 chicken anti-TGF-β1 antibody coupled to horseradish peroxidase for 90 minutes at room temperature with shaking. Any bound peroxidase is then detected using a suitable chromogenic substrate (e.g., K-BLUE TMB substrate solution). The amount of active TGF-β generated is estimated by interpolation of a standard curve constructed using known amounts of active TGF-β1 (R&D Systems).

Chemokine antagonist activity may be determined using the THP-1 transwell migration assay described above in which the peptide is incubated in the top compartment with the cells while a chemokine is used as a chemoattractant in the lower compartment. Four chemokines were tested: IL-8; SDF-1α; MCP-1 and MIP1α: pluses in Table 11 indicate that the peptide was active as an inhibitor of migration induced by at least one of these four chemoattractant chemokines. The number of pluses is a qualitative indicating of the activity of each peptide in each assay. A minus indicates no detectable activity in the assay, and n.d. indicates that no attempt to estimate the activity of the given peptide in this assay has been made to date.

KFK was as active as RFK (Schultz-Cherry et al., J. Biol. Chem., 270, 7304 (1995)). However, in marked contrast to previous reports, other members of the KXK series were also active as TGF-β activators. For example, the KYK was more active than KFK. Thus, the substitution of arginine for lysine increases the range of amino acids at position 2 which activate TGF-β

KLK and KIK are of particular interest, since these agents are dual-action anti-inflammatory molecules. These tripeptides are specific antagonists of the SDF-1αla receptor CXCR4, and also activate TGF-1β. Thus, KLK, KIK and their analogs and derivatives are therefore likely to be particular useful pharmaceutical agents for the prevention or treatment of a wide range of anti-inflammatory disorders.

For graft eosinophilia, such as that associated with acute transplant rejection, a pan-chemokine inhibitor, or a selective inhibitor of eosinophil recruitment (such as KKK or an analog thereof), may be particularly beneficial. Such agents may be used alone or in conjunction with lower than normal doses of steroids, such as prednisolone, which are used currently to control acute rejection episodes. Severe side-effects are associated with the use of the highest dose of prednisolone (or other steroids) used during acute rejection, and use of agents which reduce or abolish the need to give steroids would be particularly useful.

Analogs of the KXK peptides, e.g., analogs of KQK, are also envisioned. The central chain (in a compound of formula V with R7 as a substituent) is replaced by a general substituent R, where R is the side chain from any of the amino acids. These analogs (for example, the general class of fluoroalkenes of a compound of formula (VI),) are useful for the treatment of a wide variety of diseases where activation of TGF-β and/or inhibition of chemokine signaling are desired. By selecting an appropriate member of this class of molecules, it is possible to engineer the desired properties of the molecule. Thus, selection of KYK analogs provides powerful activation of TGF-β in the absence of chemokine inhibition, while analogs of KLK have both properties. Analogs of KQK have inhibitory action on one or more chemokine receptors but do not activate TGF-β.

Thus, KYK, its analogs, and derivatives may be selected for use in diseases where TGF-β upregulation is particularly beneficial, for example, in atherosclerosis or osteoporosis. In contrast, analogs of KQK may be selected where chemokine inhibition is desired but TGF-βupregulation may not be beneficial, for example, in treatment of HIV infection.

The KXK peptides, and isosteres thereof, may be useful to treat low bone mineral density, where TGF-beta elevation and selective inhibition of MCP-1 are likely to be especially synergistic.

Derivatives or analogs of the KXK class may be used alone or in combination with other therapies for the treatment of inflammatory disorders, or other diseases or indications such as those described herein. For example, derivatives, or analogs of KYK may be used in conjunction with steroids for the treatment of inflammatory conditions, allowing lower than normal doses of steroids to be used reducing the side effects associated with chronic steroid use.

It is also envisioned that conservative substitutions of the amino acids at positions 1 and 3 do not affect the activities of the molecules. Thus, one or both of the lysine side chains (either in a peptide or in an analog such as (VI)) may be substituted with an arginyl side chain or an ornithinyl side chain.

## Example 10

Binding Affinity of the Agents of the Invention

It is also envisioned that moieties other than those exemplified, including analogs of chemokine peptide 2 or 3, variants or derivatives thereof, which bind to DARC and/or chemokine receptors with a specific affinity, e.g., they bind to functional chemokine receptors with high affinity but bind with lower affinity to DARC or bind to DARC with a high affinity but bind to chemokine receptors with lower affinity, may be identified using methods such as those described above. Moreover, the agents of the invention may be useful in functional mapping of chemokine receptors. For example, both chemokine peptide 2 and peptide 3 block binding of the natural chemokine ligands in a competitive manner. However, they do not block binding of one another suggesting that they bind to distinct regions of the receptor and that both of these regions are important for binding of the natural ligand. In addition, peptide 2 is further distinguished from peptide 3 in their differential functional activity. Peptide 3 not only binds to the receptor but also blocks the functional activity of receptor signaling as indicated by inhibition of chemotaxis. Peptide 2 does not inhibit chemotaxis. Thus, these peptides together are particularly useful in identifying regions of chemokine receptors that are important in different functional activities. Once these regions are identified, they can be used to screen combinatorial libraries or compound banks for specific inhibitors to distinct chemokine functions that may be structurally unrelated to the starting compounds, but are functionally related.

In addition, it may be important for chemokines to form dimers to activate the receptor of interest. The peptides of the invention lack the amino terminal domains that are thought to be important for chemokine dimer formation. If dimer formation is required for cell signaling, then the agents of the invention may inhibit activation as they can bind to the receptor but are unable to form dimers, e.g., with native chemokine ligand.

**Example 11**

Peptide 3 Binding Studies

Although a biotinylated peptide 3 derivative was shown to interact with a cell surface molecule on THP-1 cells, peptide 3 did not compete for binding to chemokine receptors in radioligand competition experiments on cell lines expressing cloned chemokine receptors. To determine whether or not peptide 3 binds to chemokine receptors, the THP-1 migration assay was used to assess the inhibitory activity of the biotinylated derivative, and to compare it with the non-labeled version of the same peptide. In addition, a competition ELISA is employed to show whether the labeled peptide contains functional biotin, i.e., whether the biotin is capable of binding streptavidin.

Materials

Iodinated-RANTES and iodinated-MCP-1 (Amersham) were reconstituted in MilliQ water to a concentration of 50 nM (0.1 $\mu$Ci/$\mu$l) with a specific activity of 2000 Ci/mmole. Iodinated-streptavidin was obtained (Amersham) at a stock concentration of 47 nM (0.1 $\mu$Ci/$\mu$l) and a specific activity of 38 $\mu$Ci/$\mu$g (55,000 Da). Cold RANTES and MCP-1 were purchased from R&D Systems (Minneapolis, MN), reconstituted in sterile PBS + 1 mg/ml fatty acid free BSA (FAF-BSA) (Sigma A-6003) at 10 $\mu$g/ml (1.25 $\mu$M). Cold streptavidin (Calbiochem) was reconstituted in sterile MilliQ to a concentration of 4 mg/ml (90 $\mu$M).

FAF-BSA was used throughout. All experiments were performed in binding medium (50 mM HEPES, 1 mM $CaCl_2$, 1 mM $MgCl_2$, 0.5% FAF-BSA pH 7.5) as described by Ruffing et al. (Cell. Immunol., 189, 160 (1998)) unless stated otherwise. Reactions were all performed at 4˚C and all buffers were precooled to 4˚C unless stated otherwise.

The biotinylated peptide was synthesized as an N-terminal biotin conjugate of the sequence n'-CLDPKQKWIQC-c' (Affiniti Research) and confirmed as >90% pure by HPLC. Every molecule has a biotin associated with it. The peptide was reconstituted to 10 mM stock concentration in MilliQ water and stored frozen in small aliquots until required. As a positive control, an N-terminal biotin-labeled derivative of peptide 2(1-15)[MCP-1] was used.

HOS parental cells and CCR1-5 and CXCR4 transformants were obtained from the AIDS Reagent Program and were maintained as described on the supplier data sheet. Selection for the chemokine receptor-expressing plasmid was maintained throughout culture. CHO parental cells and CXCR1 and CXCR2 transformants were obtained form Dr. J. Navarro (Southwestern University, TX, USA) and maintained as described by the supplier under constant selection for expression of the chemokine receptor. All cells were split at about a 1:10 dilution approximately every 4 days, releasing the cells with EDTA solution and reseeding at known density. For experiments, cells were released from the flask with EDTA solution and reseeded into 24 or 12 well plates as described at about 2 x $10^5$ cells/well (4.4 x $10^4$ cells/$cm^2$) 18-24 hours prior to the experiment. At the time of the experiment, all wells were at a nominal density of 3 x $10^5$ cells/well.

Results

Properties of the biotinylated peptide.

Using that standard THP-1 migration bioassay, the biotinylated peptide was compared to the corresponding unlabeled peptide. The chemokine chemoattractant used was MCP-1 at 100 ng/ml in RPMI 1640 + 10% FCS. Migration was allowed to occur for 4 hours through a 5 $\mu$m filter at 37˚C. MCP-1 as the chemoattractant increased the number of cells migrated by almost three fold, and this was inhibited by the presence of the unlabeled peptide in a dose-dependent fashion. The $ED_{50}$ for inhibition was about 9 $\mu$M, consistent with previously reported data for this peptide (2-3 $\mu$M). The labeled peptide also inhibited MCP-1 induced migration, with a similar $ED_{50}$ (about 4 $\mu$M) (Figure 20). This experiment was repeated twice, and there was no statistically significant difference between the labeled and unlabeled peptides. Thus, the addition of the label did not infer with the function of the molecule.

The presence of a biotin moiety in the labeled peptide which was capable of binding streptavidin was confirmed by competition ELISA. Briefly, streptavidin was coated onto ELISA plate wells (Nunc Maxisorp plates) at 10 pmoles per well for 45 minutes in 50 mM sodium carbonate pH 8.5 at 4˚C. After blocking (5% sucrose/5% Tween 20 in TBS), the plate was incubated with various concentrations of labeled peptide 3 (between 0.1 and 1,000-fold molar excess over the coated streptavidin) for 1 hour at room temperature. After washing, any residual free streptavidin was detected by adding a biotinylated mouse IgG (which would only bind if any streptavidin had not been blocked by the labeled peptide). Antimouse peroxidase was used to detect the bound mouse IgG. Under the conditions of the reaction, the binding of labeled peptide 3 to the immobilized streptavidin was functionally irreversible. Thus, the concentration of labeled peptide 3 needed to prevent subsequent binding of biotinylated mouse IgG is a measure of the percentage of the peptide 3 molecules with a functional biotin (i.e., one capable of binding to streptavidin) (Figure 21). The percentage labeling efficiency was estimated to be 100%, consistent with the fact that the biotin had been added co-synthetically.

Characterization of Chemokine-Receptor Over-expressing lines

To establish the kinetics of labeled RANTES binding to CCR5, CCR5 cells and their parental line were exposed to labeled RANTES at 0.05 nM in the absence or presence of a 500-fold excess of unlabeled RANTES. Binding was performed at either 4˚C or room temperature for various periods between 30 minutes and 4 hours. All data was collected in triplicate. Equilibrium was achieved slightly quicker at room temperature than 4˚C, and that in both cases binding was complete by 2 hours (Figure 22A). Furthermore, there were significantly more cold-competable the CCR5 cells than the parental line (although the parental line also showed statistically significant high affinity RANTES binding, suggesting a background level of CCR5 receptors on these cells) (Figure 22B). Based on this experiment, all binding experiments were done at 4˚C for 2 hours.

A Scatchard analysis was performed under the conditions determined to represent equilibrium binding. The purpose of this experiment was to estimate the number of high affinity binding sites (CCR5 receptors) on the cell surface, since the full Scatchard analysis is performed under conditions where the binding was limited by availability of binding sites, not by number of molecules RANTES added. The result indicated about 80,000 binding sites per cell on the parental line and about 200,000-400,000 binding sites per cell on the high expressing CCR5 line. Using this information, the conditions necessary to perform a full 12-point Scatchard analysis at equilibrium, limited by binding site number, were calculated. The conditions chosen were 0.03 nM labeled RANTES in 1.25 ml added to 3 x $10^5$ cells at 4˚C for 2 hours, in the absence or presence of cold RANTES at concentrations between 0.06 and 25 nM (2-fold to 800-fold excess). The Scatchard analysis was performed on the binding data, using the 800-fold excess as a measure of non-specific binding. It was found that the sites had an affinity of about 1 nM (consistent with the expected properties of the CCR5 receptor; kd=0.4 nM), with 100,000 sites per cell on the parental line and 200,000 sites per cell on the CCR5 over-expressing line. This confirms that the plasmid is causing over-expression of the CCR5 receptor in this cell line.

The presence of CCR5 receptors on the CCR5 over-expressing line was confirmed by immunostaining with a directly labeled (FITC-conjugated) anti-CCR5 antibody (Pharmingen; Clone 2D7). At 10 μg/ml, this antibody gave significantly higher staining that an FITC-labeled IgG control antibody (Figure 23) confirming the presence of CCR5 protein on the cell surface. At lower dilutions, no significant staining was seen, but in common with many directly labeled antibodies (where there is no amplification step due to multivalent detection antibody binding) this antibody is recommended for use at concentrations around 25 μg/ml. Based on both the functional data and the immunofluorescence data, it is very likely that the CCR5 line is expressing functional CCR5 receptors.

Using CXCR1 and CXCR2 specific antibodies, flow cytometry data indicated that the CXCR1 line expressed CXCR1 but not CXCR2, while the CXCR2 line expressed CXCR2 but not 1 (Figure 24). As a functional assay for CCR2, an abbreviated Scatchard analysis was performed using radio-iodinated MCP-1 under identical conditions to the full Scatchard analysis using RANTES. No high affinity binding sites were detected on the parental cell line (Ka of highest affinity binding sites >250 nM), or on the CCR-2 over-expressing line. It is likely that the CCR-2 over-expressing line has not maintained the plasmid intact, under as functional MCP-1 binding sites were not detectable.

Binding of Labeled Peptides of the Invention

The binding of labeled peptide 2 and labeled peptide 3 to the parental HOS, parental CHO and 8 chemokine receptor expressing lines was compared. In each case, $^{125}$I radioiodinated streptavidin was used to detect bound biotin-labeled peptide. The amount of streptavidin molecules added was calculated in each case to be approximately 50-fold in excess over the number of chemokine receptor sites. In addition, the number of molecules of labeled peptide added (at 10 μM) was $10^5$-fold in excess over the estimated number of binding sites. Thus, all these experiments were performed under conditions where the number of binding sites as limiting. No detectable binding of labeled peptide 3 to any of the cell lines tested was observed (the number of counts bound was not significantly greater than when no peptide was added). However, peptide 2 apparently bound to all the cell lines, including the parental lines to an approximately equal extent (Figure 25).

To determine whether this apparent binding of peptide 2 was cell dependent, or whether it was binding to the plastic (or the some component of the fetal calf serum used during the cell culture), the following experiment was conducted. Three plates were evaluated: One had HOS parental cells, another had CCR5 over-expressors and the third had no cells but was incubated with the FCS-containing culture medium. Peptide 2, and to a much lesser extent peptide 3, bound to the wells with no cells. Mathematical analysis indicated that all the binding which was observed when cells were present could be accounted for the ability of the peptide to bind to the wells with no cells in them. Furthermore, for peptide 2, this indicated that more than 95% of the peptide 2 was binding to the well, while for peptide 3, about 5-10% of the peptide is binding to the well.

The propensity of these peptide substances to bind to hydrophobic surfaces (such as the plastic of the wells) may also account for much of the variability seen when the experimental protocols are transferred from one site to another (e.g., when different plastic tubes are used to handle the peptide substance).

For peptide 3, there was no evidence for any binding whatsoever to recombinant human chemokine receptors. Any binding which did occur would have a very low affinity (>>10 μM) precluding the possibility that this interaction was responsible for the chemokine inhibitory activity attributed to the peptide 3 class. Conclusions

Thus, it appears that peptide 3 does not bind to human chemokine receptors at any appreciable affinity *in vitro* under the binding conditions normally used for chemokine receptor interaction studies, as well as under several other binding conditions. One possibility is that peptide 3 interacts with the chemokine receptors by collisional coupling, or by a mechanism with very short residence times. The most likely interpretation of the data is that peptide 3 is a functional chemokine inhibitor by a mechanism other than direct receptor antagonism, e.g., by preventing functional receptor ligand interactions, or by binding to a third component (other than receptor or ligand) which is necessary for ligand function. Thus, peptide 3 may bind to a cell surface site/receptor distinct from the known chemokine receptors, bind to extracellular matrix or cell membrane associated components (including, but not limited to, glycosaminoglycans (GAG), e.g., a GAG component of the plasma membrane, glycochalyx, proteoglycans, fibrinogen, chondroitin sulfate, heparin sulfate, keratin sulfate, hyaluronic acid, collagen and sulfated surface moieties), or interfere with signal transduction mechanisms in either a direct or indirect manner.

With respect to the binding of peptide 3 to a distinct receptor or to extracellular matrix or cell membrane associated components, once bound, peptide 3 interferes with chemokine activity, but does not dislodge or hinder ligand binding to the cells. The number of binding sites (receptors) for peptide 3 on THP-1 cells was estimated to be 1000 receptors/cell (Figures 28 and 29). To purify this receptor, a synthetic photoactivatable derivative may be employed for crosslinker aided purification of the receptor, or a ligand blotting approach may be employed. For ligand blotting, cell membrane proteins from THP-1 cells (which have the receptor) and from HOS (a human osteosarcoma cell line) cells (which do not have functional receptor) were separated by gel electrophoresis, then incubated with biotin-labeled peptide 3, preferably after renaturation of the proteins (e.g., using a graded decrease in SDS over a period of time) and then detected with streptavidin peroxidase.

Alternatively, or in addition, a cross linkable affinity probe is synthesized, e.g., biotin-SLDPKQKWIQC-X (L-amino acid forward linear sequence). The purpose of the $Ser_0$ is to leave only one cys residue in the molecule. After synthesis, a photoactivatable crosslinking group is attached through the sulphahydryl group on the remaining $Cys_{13}$ residue, e.g., APDP (N-[4-(p-azidosalicylamido)butyl]-3'-(2'-pyridyldithio)propionamide). This derivative is then incubated with THP-1 cells to allow it to bind the receptor on the THP-1 cells and then the reaction is flashed with UV light which induces covalent crosslinking of the APDP group to the receptor. The cells are then disrupted and membrane proteins extracted and denatured. The protein mix is passed onto a streptavidin column. The peptide and the receptor are then released from the streptavidin column either by full denaturation (e.g., with 10 M urea) or by the use of 2-mercaptoethanol, which will uncouple the APDP from the peptide, freeing the receptor. The purified receptor is then identified by N-terminal sequencing methods, or by tandem nanoelectrospray mass spectroscopy.

After peptide 3 binds to its receptor, it may block proteins required to effect the response (e.g., block specific integrins needed for chemokine-induced migration but not fMLP or TGFb induced migration), down regulate a chemokine receptor, or interfere with signal transduction mechanisms. Interference with signal transduction mechanisms can be detected in either a direct or indirect manner (for example, using assays for measuring intracellular calcium flux, cAMP, pI3, kinase activity, and DAG). In particular, with respect to calcium flux, CRD-$Leu_4Ile_{11}Cys_{13}$ peptide 3(3-12)[MCP-1] did not block MCP-1 induced calcium flux, but did block SDF-1 induced calcium flux (Figure 38).

## Example 12

Model of Ischaemia Reperfusion Injury

The intraluminal thread (ILT) model is representative of clinical ischaemia reperfusion injury, it gives a focal ischemic lesion and is widely used in the pharmaceutical industry to test candidate compounds for neuroprotective efficacy. Fed, male Sprague Dawley rats (Charles Rivers, approximately 330 g) were anaesthetized with 2% halothane in 70/30 % $N_2O/O_2$ and the left middle cerebral artery occluded (MCAo) for 90 minutes using the ILT approach. The MCAo is achieved by the placement of a 3/0 nylon suture into the internal carotid artery and advancing it approximately 18 mm from the carotid bifurcation such that its tip is positioned approximately 1 mm beyond the origin of the MCA. After the required ischemic period, the nylon suture is withdrawn into the external carotid artery and the lesion is reperfused from the normal antegrade direction.

Throughout the surgical period, rectal temperature was monitored and maintained at 37˚C with a heated blanket and feedback rectal temperature probe and arterial blood gases were measured prior to and immediately post MCAo. During the ischemic period the rats were recovered to allowing neurological deficit assessment prior to reperfusion. The neurological deficit score can be used to indirectly assess the 'quality' of the occlusion, a spontaneous right side-circling gait represents a good occlusion while animals which showed a low neurological deficit were removed from further study.

After 90 minutes of ischaemia, the rats were reanesthetized and the ILT withdrawn. The collector of the data

was blinded to the identity of the test compound. Either solution A or B (PBS or 0.5 mg/ml of CRD-$Leu_4Ile_{11}Cys_{13}$ peptide 3(3-12)[MCP-1] in PBS, respectively) was administered by jugular venepuncture at a dose of 2 mg/kg. A femoral vein cannula was inserted, tunneled under the skin and exteriorized at the back of the neck. This was used to administer compound A or B by constant intravenous infusion (0.5 mg/kg/hr) over the following 72 hours. The 3 day time frame of the experiment encompasses the maximal inflammatory response typically seen in rats using this model.

The ischemic damage was assessed by conventional magnetic resonance imaging (T2, diffusion and proton density sequences) on day 1, 2 and 3 post MCAo. For all MR procedures anaesthesia was induced and maintained at 1% halothane v/v in oxygen. Rectal temperature was maintained at 37˚C. MRI was performed at 4.7T using a SIS-200 imaging spectrometer (Spectroscopy Imaging Systems, Fremont, CA, USA) and a home-built 75 mm diameter 8-legged birdcage radio frequency coil. 25 contiguous coronal slices starting at the level of the eyes, running rostral to caudal through the brain, were acquired using a 128*128 acquisition matrix covering a field of view of 4*4 cm. Each slice was 0.9 mm thick.

After the final MR analysis at 72 hour post ictus the rats were decapitated, the brains removed, stored fresh in cryo-protecting medium at -70˚C prior to quantitative immunohistochemical analysis of infiltrating leukocytes (Figure 34). Animals treated with CRD-$Leu_4Ile_{11}Cys_{13}$ peptide 3(3-12)[MCP-1] had a 50% reduction ($p < 0.0001$) in the mean infarct volume at all time points.

Brains were sectioned every 100 μm, stained for neutrophils, and images from four regions of the brain on each of the lesion and contralateral sides were collected. There was a complete suppression of neutrophil accumulation (measured by quantitative immunofluorescence) in the penumbra of the lesion (Figure 47). Regions 2 and 3 overlap the core of the infarct and more neutrophils were observed in sections in the penumbra regions. This is likely to be because the penumbra region remains fully perfused but the infarcted region is largely necrotic with little or no blood supply. Region 1 is the most penumbral and had the highest neutrophil number. The inhibition by the test agent is statistically significant in the penumbra ($p < 0.01$ in region 4 and $p = 0.02$ in region 1). There was no significant neutrophil accumulation or effect of the agent on the contralateral side of the brain.

## Example 13

Pharmacokinetics of $^3H$ -CRD-L-$Leu_4Ile_{11}Cys_{13}$ peptide 3(3-12)[MCP-1] and labeled CRD peptides in SD rats and mice

### Labeled Peptide

Male (n=5, ave. body weight: 305 grams) and female (n=5, ave. body weight: 251 grams) SD rats were obtained from Hilltop Laboratories with surgically cannulated jugular veins. Animals were housed in Nalgene metabolic cages for the duration of the study, and given food and water ad libitum.

Radiolabeled CRD-L-$Leu_4Ile_{11}Cys_{13}$ peptide 3(3-12)[MCP-1] peptide (molecular weight about 1360, 2.0 Ci $mmol^{-1}$, 302.3 μCi $mL^{-1}$ in sterile water for injection (SWI)), and non-labeled peptide were prepared. Doses of 100 μg total peptide and 20 μCi of $^3H$-CRD-L-$Leu_4Ile_{11}Cys_{13}$ peptide 3(3-12)[MCP-1] in 200 μl of PBS were prepared, taking into account the impact of tritiated peptide mass on the total dose. Each rat was injected i.v. in the lateral tail vein with a weighed syringe containing approximately 200-300 μl of the peptide solution. The syringe was re-weighed, and the injection amount calculated for each individual dose. Radiolabeled counting standards were prepared from the same solution.

Serial blood samples (about 300-400 μL/timepoint) were removed via the jugular catheter at 0.17, 0.5, 1, 2, 4, 6, 20, 28, and 48 hours post-injection, and immediately placed into MICROTAINER® serum separator tubes (Becton Dickinson). The catheter was flushed with a small amount (20 μL) of heparin solution to prevent clotting, and blood volume was replaced with 400 μL of saline at each sampling. The serum tubes were centrifuged, and the resulting serum (100 μL) and cell pellet (weighed) were placed onto sample cones for processing by a Model 307 Packard sample oxidizer. The remaining serum was aliquoted and frozen for later analysis. Urine was sampled in time intervals of 0-4, 4-6, 6-10, 10-20, 20-28, 28-48, and 48-72 hours. 100 μL of each individual urine collection was placed onto sample cones for oxidation, and the remainder frozen for later analysis by HPLC and LC-MS. At 72 hours, the rats were anesthetized, exsanguinated by cardiac punctured, euthanized by cervical dislocation, and dissected to estimate the biodistribution of $^3H$-CRD-L-$Leu_4Ile_{11}Cys_{13}$ peptide 3(3-12)[MCP-1]. Processed samples were counted in the Packard.

### Serum Pharmacokinetics

The radiolabel disappeared from serum quite rapidly in both males and females. Linear plots of concentration versus time for both genders are shown in Figure 37A. The concentration at 10 minutes post injection was 1238 ± 138 ng $mL^{-1}$ for females (corresponding to 12.45 ± 1.39% of the injected dose (i.d.)) and 1141 ± 283 ng $mL^{-1}$ for males (corresponding to 14.38 ± 3.46% i.d.). While the absolute concentration values appear less for the males, the fact that

the dose was not adjusted to body mass easily accounts for this differential, as the percent injected dose values indicate. By four hours post injection, only about 0.1% of the injected dose is present in blood. While data is reported at 6 hours and beyond, the amount of radioactivity present in these samples was less than twice background levels. This low level of activity cannot be characterized with regard to structure, nor is it robust enough for consideration as part of the kinetic modeling.

The vast majority of the radioactivity present in blood resides in the serum fraction (<12% of the total counts were present in the unwashed cell pellet at 10 minutes), although this ratio changes over time (Figures 38B and 38C). Cell pellet data is of low reliability (due to low counts) between 4 and 28 hours. The 48 hour data is suggestive of a "rebound" in cell-associated radioactivity, but represents an extremely low amount of material. It is unknown whether this radioactivity reflects the presence of intact peptide.

The data indicate a biphasic clearance in both sets of animals. Curve-fitting pharmacokinetic analyses were done using a basic two-compartment biexponential model to the serum data. The actual time and concentration data is entered into a fitting program (PK Analyst, MicroMath Scientific Software. Salt Lake City, UT), and is fitted according to model selection (e.g., i.v. bolus, two-compartment distribution). Calculated concentration values are generated, and a curve is fit to the data.

From the fit generated above, the following pharmacokinetic parameters were generated:

| | | | |
|---|---|---|---|
| Elimination half-life: | 0.19 hr | alpha-phase $T_{1/2}$: | 0.07 hr |
| $\alpha$: 9.59 | | beta-phase $T_{1/2}$: | 0.29 hr |
| $\beta$: 2.42 | | | |
| Maximal concentration | 1,710 ng $mL^{-1}$ | | |
| A: 48.88 $\mu$g/mL | | | |
| B: 63.64 $\mu$g/mL | | | |
| AUC | 31.43 (gg-hr)/mL | | |
| Maximal concentration/ dose | 0.000017 | | |
| Vd | 0.36 L / kg | | |

Urinary Excretion

Sampling of the female rats' urine was subject to an experimental handling error. Only rat number 5 showed a pattern consistent with excretion in the male rats, eliminating >75% of the injected dose in the first 4 hours. The urine of male rats, in which the handling error was corrected for all but rat #14, yield the most consistent data (Figure 37D). The majority of the administered dose was eliminated in the urine over the first 4 hours (86.6 +/- 16.5% i.d.). A subsequent experiment has confirmed that female rats administered either 1.0 or 10.0 mg of $^{3}$H-CRD-L-$Leu_4Ile_{11}Cys_{13}$ peptide 3 (3-12)[MCP-1] also excrete >80% i.d., in this case measured over the first 2 hours after injection. This high rate of urinary excretion is consistent with the rapid elimination of $^{3}$H-CRD-L-$Leu_4Ile_{11}Cys_{13}$ peptide 3(3-12)[MCP-1] from blood, and the low organ retention values measured in subsequent dissected rats.

Urine from male rats was filtered through a 0.2 $\mu$ filter (without decreasing the amount of radioactivity per unit volume), and analyzed by gradient reverse-phase HPLC with online radiodetection. The co-elution of the injected preparation (as a standard) with the 0-4 hour urine from rats 12 and 16 was observed. A single peak of radioactivity was seen in both urine samples, consistent with excretion of the $^{3}$H-CRD-L-$Leu_4Ile_{11}Cys_{13}$ peptide 3(3-12)[MCP-1] as an intact moiety. For definitive structural characterization of the excreted molecule, all urine samples from the male rats were analyzed by LC-MS-MS.

Using electrospray ionization (ESI), an LCQ ion trap mass spectrometer (Thermoquest Finnigan, San Jose, CA) is operated in the positive ion mode with the heated capillary set to 200°C, and 4.25 kV applied to the electrospray needle. The sheath gas flow rate is set to 50 units, while the auxiliary gas is turned off. The data are acquired with a maximum ion time of 500 ms and 1 total microscan. The analysis is performed using two scan events, a full scan MS with m/z [280-1500] and a data dependent full scan MS/MS with m/z [125-1300] generated by fragmentation of the doubly charged ion with *m*/*z* 680.1 set to an isolation width of 2.0 amu and a collisional energy of 28%.

HPLC grade solvents ('Baker Analyzed') are purchased from J. T. Baker, Phillipsburg, NJ, and formic acid, 99%, ACS, is purchased from Sigma, St. Louis, MO. A Zorbax Eclipse XDB-C 18 3.0 × 150 mm, 3.5 micron ('Zorbax', Hewlett-Packard, Palo Alto, CA) equipped with a 'SafeGuard' guard column containing a C18 cartridge (Phenomenex, Torrence, CA) is operated at a column temperature of 35°C and a maximum pressure of 400 bar. An HP1100 binary system (Hewlett-Packard, Palo Alto, CA) generates a gradient at a flow rate of 0.500 ml/min from 2% B (acetonitrile) in water/0.1% formic acid (A) from 0.0 to 0.8 min, then ramping up to 20% B for 5 min. The organic content is further

increased at 6 min to 95% B at 6.5 min, combined with an increase in flow rate to 0.800 ml/min at 6.55 min to elute lipophilic contaminants or metabolites. After 1.45 min, the column is re-equilibrated at a flow rate of 0.800 ml/min at 2% B for 2 min. 10 μl of each sample is injected using an HP1100 autosampler (Hewlett-Packard, Palo Alto, CA). With these conditions CRD-$Leu_4Ile_{11}Cys_{13}$ peptide 3(3-12)[MCP-1] elutes at a retention time of 5.17 min.

The standard analytes are prepared by adding different levels of $^3$H-CRD-L-$Leu_4Ile_{11}Cys_{13}$ peptide 3(3-12)[MCP-1] to rat urine filtrated through a 'Sterile Acrodisc 13 0.2 μm' filter (Gelman Sciences, Prod. #4454). The following levels of 3H-CRD-L-$Leu_4IleCys_{13}$ peptide 3(3-12)[MCP-1] standards were each injected three times and analyzed to generate a standard curve: 0 μg/ml (no standard added), 0.5 μg/ml, 1.0 μg/ml, 2.0 μg/ml, 3 μg/ml, 5 μg/ml, and 10 μg/ml. Rat urine samples were collected at different time points after i.v. injection of a single dose of 100 μg $^3$H-CRD-L-$Leu_4Ile_{11}Cys_{13}$ peptide 3(3-12)[MCP-1] containing 86.4% of unlabeled and 13.6% of tritium-labeled compound. Samples were analyzed after filtration as described above. As the detection method only allows for the analysis of the unlabeled fraction of the sample, the overall concentration was calculated by multiplication of the measured concentration by 100/86.4.

Only samples from the first 2-3 collection periods (out to 20 hours post administration) were evaluable within the detection limits of the mass spectrometer. While there were some experimental inconsistencies (variability in the assayed amounts with the counting data, probably due to method errors and the low sample concentrations) the data show that >80% of the injected radioactivity in the urine possesses a mass consistent with the intact, cyclized structure. In these analyses, the linear peptide was assayed as a control. The linear form was found to be rapidly cyclized in room temperature urine. Similar incubations in rat serum gave the same result, confirming that the preferred conformation of the peptide in these biological fluids appears to be the cyclized compound. Given the propensity to cyclize in the serum, it is unlikely that $^3$H-CRD-L-$Leu_4Ile_{11}Cys_{13}$ peptide 3(3-12)[MCP-1] underwent significant *in* vivo reduction to the linear form and was oxidized back to the cyclized form in the urine, but this possibility cannot be ruled out until serial serum analysis confirms the presence of the intact $^3$H-CRD-L-$Leu_4Ile_{11}Cys_{13}$ peptide 3(3-12)[MCP-1] at all early (0-6 hour) timepoints. LC-MS-MS analysis of the serum at 10 minutes post-injection confirmed the presence of intact $^3$H-CRD-L-$Leu_4Ile_{11}Cys_{13}$ peptide 3(3-12)[MCP-1].

Summary for Labeled Peptide

The pharmacokinetic profile of a relatively small, highly polar molecule is consistent with the above parameter values and the structure of $^3$H-CRD-L-$Leu_4Ile_{11}Cys_{13}$ peptide 3(3-12)[MCP-1]. For comparative purposes, the antibiotic amikacin (a relatively small (MW about 580), polycationic aminoglycoside) exhibits a similar pharmacokinetic profile ($T_{1/2}$ about 2 hours, 98% urinary excretion, $V_d$ = 0.27 L / kg). Molecules within this range of distribution volumes may be distributing rapidly through the extracellular fluid. $^3$H-CRD-L-$Leu_4Ile_{11}Cys_{13}$ peptide 3(3-12)[MCP-1] appears to be rapidly and nearly quantitatively cleared via renal excretion. Serum concentrations after i.v. bolus dosing decline quickly, with less than 1% i.d. remaining in the blood by 2 hours. Given the balance between the decline in serum/whole blood and the increase in cumulative urinary excretion over the same interval, it is difficult to imagine that a significant fraction of $^3$H-CRD-L-$Leu_4Ile_{11}Cys_{13}$ peptide 3(3-12)[MCP-1] is sequestered in any tissue. Biodistribution data from another study seems to bear out this supposition. At 3 hours post injection, only about 11% i.d. could be accounted for in the 25 tissues and organs assayed.

A mouse study was conducted wherein female BALB/c mice received a subcutaneous dose of 10 μg $^3$H-CRD-L-$Leu_4Ile_{11}Cys_{13}$ peptide 3(3-12)[MCP-1] (see Figures 39-40). At 30 minutes post injection, the average serum concentrations were 503 ng/g, which compare favorably with the 591 ng/g recorded at the same timepoint in female rats. The biodistribution data at 30 minutes for these mice are similar to those values for the rat study in process. Both species exhibit similar blood clearance and distribution profiles.

Unlabeled peptide

Material and Methods

Chemicals and Reagents

HPLC grade water and acetonitrile ("Baker Analyzed") were purchased from J. T. Baker (Phillipsburg, NJ). Formic acid (99%, ACS), trifluoroacetic acid (99+%, spectrophotometric grade), and 1,2-ethanedithiol (90+%) were purchased from Sigma (St. Louis, MO). Phosphate-Buffered Saline (Dulbecco's Phosphate-Buffered Saline, 1x) was purchased from GibcoBRL (Grand Island, NY). Nitrogen used as a sheath gas for mass spectrometric analysis, was drawn from a liquid nitrogen cylinder (99.998% purity) purchased from Byrne Specialty Gases (Seattle, WA). Heparin sodium for injection, USP, 1000 units per ml, was purchased from Provet (Seattle, WA). Serum for preparation of calibration standards was prepared freshly from blood of female Sprague-Dawley rats (average body weight: 265 g)

obtained from Hilltop Lab Animals (Scottdale, PA).

Peptides

CRD-$Leu_4Ile_{11}Cys_{13}$ peptide 3(3-12)[MCP-I] has a molecular mass of 1357.7 Da (monoisotopic), is amidated at the C-terminus, and was manufactured as triacetate salt with a purity >97% (Multiple Peptide Systems, San Diego, CA).

CRD-L-$Leu_4Ile_{11}Cys_{13}$ peptide 3(3-12)[MCP-1] has a molecular mass of 1357.7 Da (monoisotopic) and is a diastereomer of CRD-$Leu_4Ile_{11}Cys_{13}$ peptide 3(3-12)[MCP-1]. The diastereomer was synthesized by solid phase peptide synthesis inserting L-leucine instead of D-leucine as follows: Peptide synthesis was performed on an ABI Applied Biosystems Peptide Synthesizer 430A (Foster City, CA) using Fmoc chemistry with Rink amide resin (PE Applied Biosystems, Foster City, CA). After completion of the synthesis, the linear peptide was cleaved from the resin with trifluoracetic acid/1,2-ethanediothiol/water (95/2.5/2.5). The peptide was purified by preparative RP-HPLC using a Dynamax HPLC system with photo diode array detection (Rainin Instrument Company, Inc., Woburn, MA) applying a water/acetonitrile gradient containing 0.1% trifluoracetic acid. The correct molecular mass of the isolated linear peptide (1359.7 Da, monoisotopic) was verified by ESI/MS analysis of the infused compound on a Finnigan LCQ ion trap (San Jose, CA). The linear peptide was cyclized oxidatively by stirring a highly diluted aqueous solution of peptide (0.05-0.1 mg/ml) at pH 8.5 at room temperature in an open flask for two days to form head to tail disulfide bonds. The intramolecularly linked peptide was purified by preparative RP-HPLC as described above. The resulting trifluoroacetate salt was dissolved in water and filtered through a column packed with a 36 fold molar excess of pre-washed AG 1-X2 strong anion exchange resin, analytical grade, 200-400 mesh, acetate form (Bio-Rad Laboratories, Richmond, CA). The eluting peptide triacetate salt was collected and lyophilized. The correct molecular mass of the diastereomer was verified by ESI/MS analysis of the infused compound on a Finnegan LCQ ion trap (San Jose, CA). The purity was assessed by RP-HPLC coupled with phot diode array detection, and ESI/MS analysis of the infused compund as described above..

LC-MS analysis

Chromatographic separation was carried out on an HP series 1100 system comprising a degasser, binary pump, auto sampler, and column compartment (Hewlett-Packard, Palo Alto, CA). The chromatograph was fitted with a Zorbax Eclipse XDB-C18 3.0 $\times$ 150 mm, 3.5 micron column (Hewlett-Packard, Palo Alto, CA) and a Phenomenex "SafeGuard" guard column with a C18 cartridge (Torrance, CA), and operated at a maximum pressure of 400 bar with a column temperature of 35°C. A flow rate of 500 $\mu$l/min was employed. The mobile phase utilized for separation of CRD-$Leu_4Ile_{11}Cys_{13}$ peptide 3(3-12)[MCP-I] and CRD-L-$Leu_4Ile_{11}Cys_{13}$ peptide 3(3-12)[MCP-1] was composed of water containing 1% formic acid (buffer A) and acetonitrile (buffer B). The gradient applied was 2% B for 2 min rising to 17% B within 0.5 min, then running isocratically for 7.5 min. A wash step was appended with 95% B for 3 min at an increased flow rate of 800 $\mu$l/min, followed by a re-equilibration step at 2% B for 3 minutes. Per sample, 10 $\mu$l was injected. CRD-$Leu_4Ile_{11}Cys_{13}$ peptide 3(3-12)[MCP-I] eluted at a retention time of 8.3 minutes, and CRD-L-$Leu_4Ile_4Cys_{13}$ peptide 3 (3-12)[MCP-1] eluted at 8.9 minutes. The analytical column was interfaced with a Thermoquest/Finnigan LCQ ion trap (San Jose, CA) using ESI. The mass spectrometer was operated in the positive ion mode with the heated capillary set to 200°C, and 4.25 kV applied to the electrospray needle. The sheath gas flow rate was set to 55 units, while the auxiliary gas flow was turned off. The data were acquired in a full scan MS mode (*m*/*z* [335-1400 Da/z]) using automoated gain with 1 microscan and a maximum ion time of 500 ms. The HPLC effluent was directed to waste for the first 6 minutes of the analysis, and then introduced into the electrospray source for 4 minutes.

Preparation of Calibration Standards and Samples

Two primary stock solutions of CRD-$Leu_4Ile_{11}Cys_{13}$ peptide 3(3-12)[MCP-I] triacetate salt and CRD-L-$Leu_4Ile_{11}Cys_{13}$ peptide 3(3-12)[MCP-1] triacetate salt were prepared at a concentration of 11.3 mg/ml in water. Three working solutions of CRD-$Leu_4Ile_{11}Cys_{13}$ peptide 3(3-12)[MCP-I] triacetate salt were prepared by diluting the primary stock solution ten-fold with water, followed by two hundred-fold serial dilution steps. Additionally, a 1.13 mg/ml working solution of CRD-L-$Leu_4Cys_{13}$ peptide 3(3-12)[MCP-1] triacetate salt was prepared by dilution of the primary stock solution with water. Serum was prepared freshly from blood of untreated rats into Microtainer serum separator tubes (Becton Dickinson and Co., Franklin Lakes, NJ), followed by centrifugation for 10 minutes at 16,000 g, and sampling of the upper layer.

To generate a standard curve, thirteen calibration standards were prepared by adding 2 $\mu$l of one of the CRD-$Leu_4Ile_{11}Cys_{13}$ peptide 3(3-12)[MCP-I] triacetate salt working solutions and 2 $\mu$l of the CRD-L-$Leu_4Ile_{11}Cys_{13}$ peptide 3(3-12)[MCP-1] triacetate salt working solution to 96 $\mu$l of freshly prepared serum from untreated rats. To determine levels of CRD-$Leu_4Ile_{11}Cys_{13}$ peptide 3(3-12)[MCP-I] in treated rats, serum was isolated from blood collected at a series of time points post peptide injection, and 2 $\mu$l of internal standard CRD-L-$Leu_4Ile_{11}$ $Cys_{13}$ peptide 3(3-12)[MCP-1]

triacetate salt working solution was added to 98 μl of each serum sample from CRD-$Leu_4Ile_{11}Cys_{13}$ peptide 3(3-12) [MCP-I]-treated rats. Each of the calibration standards and rat serum samples was mixed with 400 μl of ice-cold acetonitrile and centrifuged for 10 minutes at 16,000 g. 400 μl of the supernatants were transferred into Eppendorf centrifuge tubes (Fisherbrand polypropylene, Fisher Scientific, Pittsburgh, PA), dried overnight under vacuum in a Savant Speed Vac (Holbrook, NY), and reconstituted in 100 μl of HPLC grade water. Samples were spun for 10 min at 16,000 g and 70 μl transferred into 100 μl glass inserts placed into 2 ml HPLC vials (Hewlett-Packard, Palo Alto, CA) for LC-MS analysis. The calibration standards' final concentrations were 0.008, 0.04, 0.08, 0.2, 0.4, 0.8, 2, 4, 8, 20, 40, 80, and 200 μg/ml of CRD-$Leu_4Ile_{11}Cys_{13}$ peptide3(3-12)[MCP-1]. All samples contained 16 μg/ml of internal standard CRD-L-$Leu_4Ile_{11}Cys_{13}$ peptide 3(3-12)[MCP-1]. A blank sample was prepared by spiking 4 μl of water into 96 μl of serum from untreated rats followed by liquid/liquid extraction and reconstitution for LC-MS analysis as described above.

Quantitative Analysis

Using the LCQuan program (Finnigan, San Jose, CA), levels of CRD-$Leu_4Ile_{11}Cys_{13}$ peptide 3(3-12)[MCP-I] were determined by monitoring the summation of ion currents of the triply, doubly, and singly charged ions of CRD-$Leu_4Ile_{11}Cys_{13}$ peptide 3(3-12)[MCP-I] and CRD-L-$Leu_4Ile_{11}Cys_{13}$ peptide 3(3-12)[MCP-1] *m/z* 453.6 $[M + 3H]^{3+}$, m/z 680.0 $[M + 2H]^{2+}$, and *mlz* 1358.7 $[M+H]^{+}$) extracted from the full scan total ion chromatogram using an isolation width of 3 u. Thirteen calibration standards, each containing 16 μg/ml of CRD-L-$Leu_4Ile_{11}Cys_{13}$ peptide 3(3-12)[MCP-1] and prepared in serum as described above, were analyzed in triplicate. After averaging the three data points for each standard, the calibration curve was constructed by plotting the peak area ratios of analyte to CRD-$Leu_4Ile_{11}Cys_{13}$ peptide 3(3-12)[MCP-I] to internal standard CRD-L-$Leu_4Ile_{11}Cys_{13}$ peptide 3(3-12)[MCP-1] against the concentration ratios of CRD-$Leu_4Ile_{11}Cys_{13}$ peptide 3(3-12)[MCP-I] to CRD-L-$Leu_4Ile_{11}Cys_{13}$ peptide 3(3-12)[MCP-1]. A non-weighted linear regression line was fitted to the concentration ratios of CRD-$Leu_4Ile_{11}Cys_{13}$ peptide 3(3-12)[MCP-I] to CRD-L-$Leu_4Ile_{11}Cys_{13}$ peptide 3(3-12)[MCP-1] covering a concentration range of 200 ng/ml - 200 μl/ml of CRD-$Leu_4Ile_{11}Cys_{13}$ peptide 3(3-12)[MCP-I]. The lower limit of quantitation (LLOQ) was determined as the level of the least concentrated calibration standard with a signal to noise ratio greater than 3:1. The accuracy of the method was calculated as % relative error (RE), and the precision as % relative standard deviation (RSD).

Pharmacokinetic Analysis

Five female Sprague-Dawley rats (average body weight: 265 g) with surgically cannulated jugular veins were obtained from Hilltop Lab Animals (Scottdale, PA) and housed in a facility approved by the Association for the Assessment and Accreditation of Laboratory Animal Care. All experiments involving the use of animals were approved by the Institutional Animal Care and Use Committee. During the study, animals were housed in Nalgene metabolic cages (Nalgene Company, Rochester, NY) and supplied with food and water ad *libitum.*

Sixty mg of CRD-$Leu_4Ile_{11}Cys_{13}$peptide 3(3-12)[MCP-I] triacetate salt was dissolved in 1.8 ml of phosphate-buffered saline and 300 μl was injected into the lateral tail vein of each rat (8.8 mg). The exact amounts of CRD-$Leu_4Ile_{11}Cys_{13}$peptide 3(3-12)[MCP-I] injected into each animal were calculated based on the syringe weights pre- and post-injection.

From each of the five rats, serial blood samples of approximately 300 μl were removed via the jugular catheter before treatment (pre-bleed), and at 0.08, 0.25, 0.5, 1, 2, 4, 6, 24, 48, and 72 hours post injection. The removed blood was replaced with an equal volume of phosphate-buffered saline, followed by injection of approximately 30 μl of heparin as anti-coagulant. Blood samples were immediately placed into serum separator tubes, and processed and analyzed as described above. Urine was sampled at intervals of 0-4, 4-6, 6-10, 10-28, 28-48, and 48-72 hours and filtered through Acrodisc filters (Sterile Acrodisc 13, 0.2 μm, Gelman Sciences).

Concentrations of CRD-$Leu_4Ile_{11}Cys_{13}$ peptide 3(3-12)[MCP-I] were determined by LC-MS analysis as described above (data for urine samples not shown). Curve-fitting pharmacokinetic analysis was performed with a simulation program (PKAnalyst, MicroMath Scientific Software, Salt Lake City, UT) employing a basic biexponential two-compartment model with bolus input and first order output (Model #7) following the equation: $Conc_{(Time)} = A \bullet e^{-\alpha\text{-}Time} + B \bullet e^{-\beta \bullet Time}$.

Results

LC-MS analysis

The mass spectrometric parameters were optimized for the detection of CRD-$Leu_4Ile_{11}Cys_{13}$peptide 3(3-12) [MCP-I] and CRD-L-$Leu_4Ile_{11}Cys_{13}$ peptide 3(3-12)[MCP-1] at a flow rate of 500 μl/min, and the ESI positive mass spectra generated showed the triply, doubly, and singly charged molecular ions with m/z 453.6 ($[M + 3H]^{3+}$), *mlz* 680.0 ($[M + 2H]^{2+}$), and *mlz* 1358.7 ([M + H]). The most abundant ions observed throughout the analyzed concentration range

were either the $[M + 3H]^{j+}$ or the $[M + 2H]^{2+}$ ions. Base-line separation of CRD-$Leu_4Ile_{11}Cys_{13}$ peptide 3(3-12)[MCP-I] and CRD-L-$Leu_4Ile_{11}Cys_{13}$peptide 3(3-12)[MCP-1] in rat serum was achieved by reversed-phase HPLC (RP-HPLC) with CRD-$Leu_4Ile_{11}Cys_{13}$ peptide 3(3-12)[MCP-I] eluting first at 8.33 (± 0.04, n = 108) minutes and CRD-L-$Leu_4Ile_{11}Cys_{13}$peptide 3(3-12)[MCP-1] eluting at 8.94 (± 0.05, n = 108) minutes. All samples prepared in serum as well as samples isolated from serum of peptide-treated animals exhibited two minor species which pre-eluted CRD-$Leu_4Ile_{11}Cys_{13}$ peptide 3(3-12)[MCP-I] and CRD-L-$Leu_4Ile_{11}Cys_{13}$ peptide 3(3-12)[MCP-1]. One of the minor species was formed from CRD-$Leu_4Ile_{11}Cys_{13}$ peptide 3(3-12)[MCP-I] while the other was formed from CRD-L-$Leu_4Ile_{11}Cys_{13}$ peptide 3(3-12)[MCP-1]. To account for this reaction, CRD-$Leu_4Ile_{11}Cys_{13}$peptide 3(3-12)[MCP-I] was integrated as peaks of the corresponding minor species and CRD-$Leu_4Ile_{11}Cys_{13}$ peptide 3(3-12)[MCP-I] while CRD-L-$Leu_4Ile_{11}Cys_{13}$ peptide 3(3-12)[MCP-1] was integrated as the peaks of the corresponding minor species of CRD-L-$Leu_4Ile_{11}Cys_{13}$ peptide 3(3-12)[MCP-1] and CRD-L-$Leu_4Ile_{11}Cys_{13}$ peptide 3(3-12)[MCP-1]. No interfering peaks were detected in the blank serum samples.

Quantitative Analysis

Initial attempts to quantify known amounts of CRD-$Leu_4Ile_{11}Cys_{13}$ peptide 3(3-12)[MCP-I] spiked into deproteinated rat serum samples by single ion monitoring of the doubly or triply charged species, or by selected ion monitoring detecting the product ions generated from the doubly or triply charged species, yielded highly variable results with a RSD > 20%. When applying SIM to integrate the summed ion currents of the singly, doubly, and triply charged species of the same samples, the reproducibility was significantly improved to a RSD < 15% (data not shown). Hence, levels of CRD-$Leu_4Ile_{11}Cys_{13}$ peptide 3(3-12)[MCP-I] in rat serum were quantified by LC-MS in the SIM mode. In absence of a stable isotope, the diastereomer CRD-L-$Leu_4Ile_{11}Cys_{13}$ peptide 3(3-12)[MCP-1] was included as internal standard. The levels of the three least concentrated calibration standards analyzed containing 0.008, 0.04, and 0.08 μg/ml of CRD-$Leu_4Ile_{11}Cys_{13}$ peptide 3(3-12)[MCP-I] in serum, respectively, were not sufficiently detectable and the LLOQ was determined as <0.2 μg/ml (2 ng per injection). There was a RSD of less than 12% over the calibration range and a RE within ±11% for intra-day precision and accuracy. The response for the concentration ratios of CRD-$Leu_4Ile_{11}Cys_{13}$ peptide 3(3-12)[MCP-I] to CRD-L-$Leu_4Ile_{11}Cys_{13}$ peptide 3(3-12)[MCP-1] in deproteinated rat serum plotted against the concentration ratios of CRD-$Leu_4Ile_{11}Cys_{13}$peptide 3(3-12)[MCP-I] to CRD-L-$Leu_4Ile_{11}Cys_{13}$ peptide 3(3-12)[MCP-1] was linear over a range of 200 ng/ml to 200 μg/ml of CRD-$Leu_4Ile_{11}Cys_{13}$ peptide 3(3-12)[MCP-I] ($r^2$= 0.9996, slope 0.8488 ± 0.005843. The curve's Y-intercept of 0.02653 ± 0.02416 indicated an insignificant amount of interfering impurities.

As the diastereomers present the same protonation sites, thei ionization efficiences were expected to be similar. However, the serum calibration curves' slope was calculated as 0.8488 (± 0.005843) suggesting a difference in sensitivites, which may be the result of a difference in the competition for charge between CRD-$Leu_4Ile_{11}Cys_{13}$ peptide 3 (3-12)[MCP-I] and CRD-L-$Leu_4Ile_{11}Cys_{13}$ peptide 3(3-12)[MCP-1].

Pharmacokinetic Analysis

The method was applied to determine the serum profiles of five rats treated with a single i.v. bolus dose of 8.8 mg of CRD-$Leu_4Ile_{11}Cys_{13}$ peptide 3(3-12)[MCP-I]. With exception of one sample collected from rat #5 at 15 minutes post-injection, which exhibited a RSD of 17.7%, the analysis of all rat samples showed good precision with a RSD lower than 10%. The resulting five animals' serum concentration curves paralleled each other closely with increasing % STDEV at the later sampling time points approaching completion of elimination. With serum concentrations declining below LLOQ within four hours, CRD-$Leu_4Ile_{11}Cys_{13}$ peptide 3(3-12)[MCP-I] exhibited very rapid elimination kinetics suggesting an average serum half-life time of approximately 10 minutes. The means of all serum data sets were processed through a curve-fitting pharmacokinetic simulation program (PKAnalyst) to assess serum pharmacokinetics. When employing a basic biexponential two-compartment model with bolus i.v. input and first order output, generated and predicted data were in good agreement ($r^2$ = 0.9999). Using the quantitative method described above, urine levels of CRD-$Leu_4Ile_{11}Cys_{13}$ peptide 3(3-12)[MCP-I] were measured in samples collected at several time points post-injection to generate a cumulative urinary excretion profile. A total of about 70% of the administered dose was recovered over 24 hours, and greater than 55% was excreted over the first two hours. This high rate of urinary excretion is consistent with the rapid elimination of CRD-$Leu_4Ile_{11}Cys_{13}$ peptide 3(3-12)[MCP-I] from the vascular compartment and in agreement with the pharmacokinetic profiles of small, ionic, and highly polar molecules.

Discussion

ESI positive mass spectra of biomolecules, such as peptides and proteins, usually show a characteristic pattern of multiply charged species due to the presence of a number of basic sites accessible to protonation. The intensity ratio

of the ions in a series of different charge states can fluctuate considerably, which renders the quantitation of multiply charged compounds by LC-MS using single ion or selective reaction monitoring difficult. When using SIM to generate a total area under the curve from the summed ion currents of all detected charge states, the reproducibility of the quantitative data can be notably improved. Carrascal et al., J. Pharm. Bio. Ana., 17, 1129 (1998); Clarke et al., FEBS Lett, 430,419 (1998).

Along with a rugged analytical method, quantitative measurements of high accuracy further require the inclusion of a suitable internal standard. For mass spectrometric analysis, an ideal internal standard is analytically distinguishable from the analyte, yet, exhibits nearly identical physical and chemical properties to correct for analyte loss during sample preparation, for matrix effects, and for drifting ion currents of the mass spectrometer. One class of compounds that meets these criteria very well is stable isotope analogs. However, stable isotope analogs are often difficult to synthesize, expensive, and frequently contain isotopic impurities that interfere with the analysis. Structurally related molecules that differ in molecular weight as well as physical and chemical properties are often used as internal standards when stable isotopes are not readily available. For quantitative peptide analysis by LC-ESI/MS for instance, peptide isoforms have been used as internal standards (Carrascal et al., 1998). However, in this case the inclusion of two internal standards was necessary to compensate for variable recoveries after sample processing as well as instrumental inconsistencies.

Like stable isotope analogs, some steroisomers, including atropisomers and diastereomers, can be analytically distinguished despite similar physical and chemical properties (March, Advanced Organic Chemistry. Reactions, Mechanism, and Structure, pp. 94-116, John Wiley & Sons, Inc., NY (1992)) and, therefore, may qualify well as internal standards. To further improve the reproducibility of the quatitative ESI/MS data, SIM can be applied to generate a total area under the curve from the summed ion currents of all detected charge states.

The peptide diastereomers CRD-$Leu_4Ile_{11}Cys_{13}$peptide 3(3-12)[MCP-I] and CRD-L-$Leu_4Ile_{11}Cys_{13}$ peptide 3 (3-12)[MCP-1] have the same amino acid composition, sequence, and hydrophobicity, thus, were expected to partition and react similarly during sample preparation. While RP-HPLC afforded base-line separation throughout a concentration range spanning three log scales, the diastereomers could be analyzed using the same mass spectrometric method as they show identical ESI mass spectra. In result, the calibration curve generated showed good precision and accuracy throughout a broad dynamic range. Application of this method to the quantitative analysis of CRD-$Leu_4Ile_{11}Cys_{13}$ peptide 3(3-12)[MCP-I] in serum samples collected from treated rats showed good reproducibility, and predicted serum pharmacokinetics were in agreement with the pharmacokinetic profiles of small, ionic, and highly polar molecules.

In conclusion, the diasteromer CRD-L-$Leu_4Ile_{11}Cys_{13}$ peptide 3(3-12)[MCP-1], which was readily synthesized, proved a suitable substitute for a stable isotope analog as internal standard. Moreover, interference due to partial overlap of co-eluting compounds or isotopic impurities introduced with stable isotope analogs did not present a problem. Hence, a facile and reliable quantitation of a multiply charged peptide in biological fluids was achieved using LC-MS in the SIM mode combined with the inclusion of a diasteromer as internal standard.

## Example 14

Inhibition of a T Cell-Dependent Antibody Response

Treatment with CRD-$Leu_4Ile_{11}Cys_{13}$ peptide 3(3-12)[MCP-1] prevented a T cell-dependent antibody response but not a T cell-independent antibody response in mice immunized with sheep red blood cells (Figure 36).

## Example 15

Exemplary peptide 3 Sequences

Preferred peptide 3 agents of the invention include reverse sequences made with D-amino acids, e.g., aqiwkqkpdlc, cqiwkqkpdlc, dqiwkqkpdlc, eqiwkqkpdlc, fqiwkqkpdlc, gqiwkqkpdlc, hqiwkqkpdlc, iqiwkqkpdlc, kqiwkqkpdlc, lqiwkqkpdlc, mqiwkqkpdlc, nqiwkqkpdlc, pqiwkqkpdlc, qqiwkqkpdlc, rqiwkqkpdlc, sqiwkqkpdlc, tqiwkqkpdlc, vqiwkqkpdlc, wqiwkqkpdlc, yqiwkqkpdlc, as well as agents having a L-amino acid in each of the bolded positions, e.g., Cqiwkqkpdlc. It is also preferred that the reverse sequences are cyclized. Other preferred peptide 3 agents of the invention include, but are not limited to, those which have one of the 19 D-amino acids in the second position of peptide3(3-12)[MCP-1], e.g., caiwkqkpdlc, cciwkqkpdlc, cdiwkqkpdlc, and cyiwkqkpdlc, as well as agents having a L-amino acid in the second position of peptide3(3-12)[MCP-1], e.g., cQiwkqkpdlc. Other preferred peptide 3 agents of the invention have one of the 19 D-amino acids in the each of the positions of peptide3(3-12)[MCP-1], as well as agents having a L-amino acid in those positions, e.g., cqawkqkpdlc, cqywkqkpdlc, cqIwkqkpdlc, cqiwkqkpdla, cqiwkqkpdlc, cqiwkqkpdld, cqiwkqkpdly, and andcqiwkqkpdlc. The entire series of peptide 3 variants are tested for their chemokine inhibitory activity and those which are positive are tested for their potency ($ED_{50}$).

**Example 16**

Inhibition of MCP-1 Binding to Heparin-Coated Agarose Beads

Chemokines bind to glycosaminoglycans on the cell surface, which may facilitate chemokine receptor binding and subsequent signaling. To determine if CRD-$Leu_4Ile_{11}Cys_{13}$ peptide 3(3-12)[MCP-1], a pan-chemokine inhibitor, would competitively inhibit $^{125}$I-MCP-1 from binding to heparin-coated agarose beads, a protocol described by Hoogerwerf et al. (Biochemistry, 36, 13570 (1997)) was employed. Competition with increasing concentrations of approximately $10^{-10}$ to $10^{-7}$ M MCP-1 resulted in an increase in bound $^{125}$I-MCP-1, consistent with MCP-1 multimer formation. This was followed by a monophasic displacement curve at concentrations of approximately $10^{-7}$ to $10^{-5}$ M MCP-1. In contrast, CRD-$Leu_4Ile_{11}Cys_{13}$ peptide 3(3-12)[MCP-1] at concentrations from $10^{-11}$ to $10^{-4}$ M did not result in displacement of MCP-1 from heparin-coated agarose beads, and concentrations of CRD-$Leu_4Ile_{11}Cys_{13}$ peptide 3(3-12)[MCP-1] as high as $10^{-3}$ M failed to inhibit MCP-1 binding to heparin to the same degree as MCP-1. This suggests that CRD-$Leu_4Ile_{11}Cys_{13}$ peptide 3(3-12)[MCP-1] does not function as a chemokine inhibitor through competitive inhibition of heparin binding sites at physiologically relevant concentrations (Figure 42).

**Example 17**

Synthesis of 3-(undec-10-enoylamino)-dihydropyridine-2,6(1*H*,2*H*)-dione(a compound of formula XV wherein $R_1$ is 9-decenyl; $R_2$ is hydrogen, $R_3$ and $R_4$ together with the atoms to which they are attached are a six membered heterocyclic ring comprising five carbon atoms and N(H); $R_5$ is hydrogen; and the center marked by * has the (S) configuration)

L-Glutamine (14.62 g, 0.1 mol) and KOH (11.2 g, 0.2 mol) were dissolved in water (200 mL) at 20˚C. A solution of 10-undecenoyl chloride (20.3 g, 0.1 mol) in THF (200 mL) was then added over 10 minutes. The reaction was stirred for 16 hours at 20˚C and was then reduced *in vacuo* to a volume of approximately 250 mL. Concentrated HCl *(circa* 12M) was added dropwise until the solution was pH 1. The white solid precipitate was collected by filtration. This solid was washed with water (100 mL) and diethyl ether (100 mL). The solid was then dissolved in refluxing toluene (500 mL) and the excess water present was removed by distillation at ambient pressure using a Dean-Stark trap. The solution was then allowed to cool to 20˚C. After 16 hours, the white solid precipitate was collected *via* filtration and recrystallized from EtOAc (500 mL). This solid was dried *in vacuo* to give N-α-undec-10-enoyl-glutamic acid (9.36 g, 30%) as a white powder.

*N*-α-Undec-10-enoyl-glutamic acid (8.00 g, 25.6 mmol) was dissolved in a mixture of THF (200 mL) and DMF (20 mL) and the solution was cooled to 0˚C. *N*-Hydroxysuccinimide (2.94 g, 25.6 mmol) was added, followed by DCC (5.26 g, 25.6 mmol). The reaction was stirred at 0˚C for 1 hour, and was then allowed to warm to 25˚C and was stirred at this temperature for 24 hours. The reaction was filtered, and the filtrate was partitioned between EtOAc (400 mL) and 0.5 M $HCl_{(aq)}$ (300 mL). The organic layer was washed twice more with 0.5 M $HCl_{(aq)}$ (300 mL), dried over $Na_2SO_4$and reduced *in vacuo* to give a white solid which was chromatographed ($SiO_2$ / EtOAc) to give a white powder. This material was recrystallized from EtOAc to give the title compound as a white crystalline solid (3.14 g, 42%).

**Example 18**

Alternate synthesis of 3-(undec-10-enoylamino)-dihydropyridine-2,6(1*H*, 2*H*)-dione

(S)-3-(tert-Butoxycarbonylamino)glutarimide (a compound of formula XV wherein $R_1$ is *tert*-butoxy; $R_2$ is hydrogen, $R_3$ and $R_4$ together with the atoms to which they are attached are a six membered heterocyclic ring comprising five carbon atoms and N(H); $R_5$ is hydrogen; and the center marked by * has the (S) configuration; 228 mg, 1 mmol) was dissolved in $CH_2Cl_2$ (2 mL) and the solution was cooled to 0˚C. Trifluoroacetic acid (1 mL) was added dropwise, and the reaction was stirred for 1 hour. The reaction was then *reduced in vacuo* to give crude (S)-glutarimidyl-3-ammonium trifluoroacetate as a thick oil. This material was dissolved in DMF (1.5 mL) and $(i-Pr)_2NEt$ (1.5 mL), and the resulting solution was added to a solution of 10-undecenoic acid (1 mmol) and BOP (442 mg, 1 mmol) in DMF at 25˚C. This reaction was stirred for 24 hours, and was then partitioned between EtOAc (30 mL) and 0.5 M $HCl_{(aq)}$ (20 mL). The organic layer was then washed twice more with 0.5 M $HCl_{(aq)}$ (20 mL), dried over $Na_2SO_4$ and reduced *in vacuo* to give a white solid which was chromatographed ($SiO_2$/ EtOAc) to give the title compound.

**Example 19**

Synthesis of yohimbamide (Y-II; a compound of formula (XIV) wherein $R_1$ is amino; $R_2$ is hydroxy; $R_3$ is hydrogen; $R_4$ is hydrogen- and n is 0)

Yohimbine (2 g) was added to freshly prepared sodamide (prepared by adding 3.6 g of sodium metal to approximately 100 ml liquid ammonia under reflux). The mixture was stirred for 5-6 hours, then the condenser was removed and the liquid ammonia allowed to vaporize. The residual material was dissolved in a mixture of warm water and ethyl acetate (~1:1). After shaking, the solvents were separated and the ethyl acetate was removed using a rotary evaporator. The resulting orange powder was dried *in vacuo* to give the title Yohimban-17-amide (Y-II; 1.8 g; 90%), the structure of which was confirmed by NMR.

**Example 20**

Synthesis of lysergyl-glutamine (L-II, a compound of formula XVII wherein $R_1$ is methyl- and $R_2$ is glutamine linked through the amine nitrogen to form an amide)

Lysergic acid (2 g) was dissolved in dry DMF and activated by addition of 1.8 g PyBOP. The reaction was stirred at room temperature for 1 hour, and 1.4 g of Fmoc-L-glutamine was added. After 16 hours, the reaction mixture was filtered and the filtrate partitioned between ethyl acetate (400 ml) and 0.5 M sodium hydroxide (200 ml). The organic layer was washed twice more with alkali, dried over sodium sulphate, and reduced *in vacuo* to give a white solid, which was recrystallized from ethyl acetate to give Lysergyl-glutamine (L-II; 1.1 g; 55%) as a white crystalline solid. Compound L-II was found to possess solubility properties similar to those of valium. Accordingly, L-II can conveniently be formulated for administration in combination with carriers that are known to be useful for formulating valium. For example, L-II can be administered in a pharmaceutical composition comprising a suitable polyhydroxy carrier (e.g. polypropylene glycol). The compound L-II can also be administered in a pharmaceutical composition comprising a polyhydroxy carrier and a suitable alcohol (e.g. a composition comprising about 10%-50% polypropylene glycol and about 5%-15% ethanol; preferably about 40% polypropylene glycol and 10% ethanol).

**Example 21**

*In vitro* and *in vivo* evaluation of peptide analogs

WGQ inhibits THP-1 migration induced by MCP-1 with an $ED_{50}$ of about 1.75 μM (Figure 43). Fourteen analogs of WGQ were synthesized and evaluated in a transwell THP-1 migration assay (Figure 43). A structurally related compound, thalidomide, was also evaluated. All compounds were reconstituted in DMSO.

Figure 43 shows the effect of these compounds at 100 μM and the percentage inhibition of THP-1 migration induced by MCP-1. For example, the N-undec-10-enoyl series inhibited THP-1 migration induced by MCP--1 back to control levels, while the benzoyl series was inactive at 100 μM (less than 40% inhibition). Interestingly, thalidomide at 100 μM also inhibited THP-1 migration induced by MCP-1.

For compounds which inhibited THP-1 migration by greater than 50%, an $ED_{50}$ was determined (Figure 45). One compound N-undec-10-enoyl aminotetrahydropyridinedione (NR58,4 or A-I hereinafter) inhibited THP-I migration with a more than ten-fold greater potency (1-100 nM) than WGQ. The majority of the other compounds which inhibited THP-1 migration by greater than 50%, have an $ED_{50}$ of about 10 to 20 μM, with the exception of the benzyl-glutamide analogs which have an $ED_{50}$ of about 40 μM (Figure 45). Thalidomide had an $ED_{50}$ of approximately 50 μM.

To determine whether NR58,4 and thalidomide reduced inflammation in the murine sub-lethal endotoxemia model, 36 female CD-1 adult mice were divided into groups of 6 (Table 12). Each animal received a pretreatment (200 ul) by sub-cutaneous injection into the scruff at the back of the neck. All pre-treatment agents were administered at 1 mg per animal (approximately 40-50 mg/kg). After 30-45 minutes, animals in groups 2 through 6 received 750 μg bacterial lipopolysaccharide (LPS) in 300 μl PBS by intraperitoneal injection. Animals in group 1 received PBS vehicle alone.

After 3 hours (2 hours post-LPS), all animals were sacrificed by terminal anaesthesia followed by cardiac puncture. Approximately 1 ml of blood was withdrawn and serum was prepared. Blood was allowed to clot at room temperature for approximately 2 hours, then the clot was spun out and the serum aliquoted and stored at -20°C until tested. Serum from each animal was assayed in duplicate for TNF-α using the Quantikine M kit (R&D Systems) calibrated by interpolation of the standard curve.

There was very little TNF-α in the unchallenged animals (<10 pg/ml), but massive stimulation in response to LPS (about 1500 pg/ml) (Figure 46 and Table 12). A dose-dependent inhibition of this LPS stimulation was observed with CRD-$Leu_4Ile_{11}Cys_{13}$ peptide 3(3-12)[MCP-1]. As expected, the negative control compound (inactive D-ala derivative) had no effect. The dose response for CRD-$Leu_4Ile_{11}Cys_{13}$ peptide 3(3-12)[MCP-1 gives a half-maximal dose of approximately 100 μg per animal. The potassium salt of NR58,4 at 1 mg per animal reduced TNF-α by just over 60%. However, NR58,4 suppressed TNF-α release generation to an equal or greater extent than CRD-$Leu_4Ile_{11}Cys_{13}$ peptide 3(3-12)[MCP-1], suggesting that NR58,4 which has similar potency as a chemokine inhibitor *in vitro* may also have similar anti-inflammatory properties *in vivo.*

The mice showed no obvious acute toxicity during treatment with the drug, although irritation was noted at the site of injection of the NR58,4, which may be due to the relatively high pH of the injected solution (the potassium salt was used).

Thus, NR58,4 is at least as effective an anti-inflammatory agent as CRD-$Leu_4Ile_{11}Cys_{13}$ peptide 3(3-12)[MCP-1] (it is active at <100 nM and likely ≤10 nM). Moreover, NR58,4, as a lipophilic molecule, may have a substantially longer residence *time in vivo.* Interestingly, thalidomide was also effective at lowering TNF-α levels *in vivo,* although it is questionable as to whether this effect is mediated through chemokine inhibition, since thalidomide is more than 4 orders of magnitude less potent as a chemokine inhibitor *in vitro* compared with NR58,4.

**TABLE 12**

| | Pretreatment/ Treatment | TNF-α (μg/ml) |
|---|---|---|
| GROUP 1 | PBS/PBS | 4 ± 1 |
| GROUP 2 | PBS/LPS | 1348 ± 125 |
| GROUP 3a | 3.14.3 (100 μg)/LPS | 1270 ± 270 |
| GROUP 3b | 3.14.3 (1 mg)/LPS | 845 ±110* |
| GROUP 4 | 3.14.4 (1 mg)/LPS | 1167 ± 305 |
| GROUP 5 | NR58,4/LPS | 552 ± 25* |
| GROUP 6 | Thalidomide /LPS | 793 ± 40* |
| * = $p < 0.05$ (Student's t-test on log-transformed data) | | |

The $ED_{50}$ (the concentration required to inhibit by 50% the migration induced by MCP-1 in the chemotaxis assay) for a number of agents is summarized in Table 13.

**TABLE 13**

| **Compound** | **$ED_{50}$ v MCP-1** |
|---|---|
| Thalidomide | 50 μM |
| Yohimbine | 30 nM |
| α-Yohimbine | 500 nM |
| Glutethimide | 50 μm |
| Phenytoin | 10 μM |
| Oxymetazoline | 75 μM |
| Yohimbamide (Y-II) | 0.5 nM |
| NR58,4 (A-I) | 2 nM |
| Lysergyl-glutamine (L-II) | 5 nM |

For each agent tested, a single "wide-range" assay was performed testing concentrations of the agent between 100 pM and 100 μM (Figure 51) and MCP-1, RANTES or MIP-1α. Similar data has been generated for SDF-1α and IL-8 (see Table 14). Once an estimate of the working range has been obtained in these "wide-range" assays, "narrow-range" assays are performed 2 logs either side of the estimated $ED_{50}$ (Figure 51). This is repeated three times to obtain an accurate estimate of the functional $ED_{50}$.

**Example 22**

Characterization of the Binding Specificity of the Agents of the Invention

Materials and Methods

For α2-adrenoreceptor binding, membranes (A-213, RBI/Sigma)) are diluted into binding buffer (75 mM Tris pH 7.4, 12.5 mM $MgCl_2$ and 2 mM EDTA). For example, 10 μl of membranes are diluted in 500 μl of binding buffer (i.e., 1:50 dilution), which is aliquoted into tubes. Cold competitor or test competitor is added, for example, 5 μl of 1 mM oxymetazoline (O-110, RBI/Sigma, 1 mM stock in MilliQ water). Radioligand is then added ($^3$H-RS-79948-197, TRK 1036, Amersham, 50 μCi in 250 μl, 2.1 μM at 95 Ci/mmol). For competition studies, 1 nM is used. A 100x stock (i.e., 100 nM) is made by diluting the commercial stock 1:20 in binding buffer, and 5 μl is added to every tube. Control tubes have radioligand but no membranes. The mixtures are vortexed, then spun very briefly. The mixtures are then incubated for 1 hour at 27°C in a water bath. The mixture is filtered through GF/C filters over a vacuum. Each filter is pre-soaked in polyethyleneimine (0.3% in water; add 1 ml of 10% stock (P-261; RBI/Sigma) to 33 ml of MilliQ water). The tubes are washed with 2-3 ml of wash buffer (50 mM Tris pH 7.4) and the wash filtered. The filters are then rinsed with 3 × 5 ml of wash buffer, air dried, placed in a scintillation vial and 4.5 ml of scintillant added. The activity on the filter is then determined.

For SHT1a serotonin receptors, membranes (S-160, RBI/Sigma) are diluted into binding buffer (50 mM Tris pH 7.4 10 mM $MgCl_2$ and 0.5 mM EDTA, with ascorbate at 1 mg/ml added immediately before use), e.g., 10 μl of membranes in 500 μl assay volume (i.e., 1:50 dilution). Cold competitor or test competitor is then added, e.g., 5 μl of 100x stock solution such as 5 μl of 1 mM oxymetazoline. Radioligand ($^3$H-8-OH-DPAT, TRK850, Amersham, 250 μCi in 250 μl; 4.5 μM at 222 Ci/mmol) is then added. For competition studies, ligand is employed (e.g., a 100x stock (i.e., 150 nM) is prepared by diluting the commercial stock 1:30 in binding buffer). Control tubes are prepared which have radioligand but no membranes. The tubes are vortexed then spun briefly. The mixtures are incubated for 1 hour at 4°C. The mixtures are filtered through GF/C filters pre-soaked in polyethyleneimine (0.3% in MilliQ water). The tubes are washed with 2-3 ml of wash buffer and filtered. The filter is washed with 3 x 5 ml of wash buffer, and air dried. 4.5 ml of scintillant is added to the dried filter in a scintillation vial and the filter counted on a tritium channel.

For dopamine receptor binding, membranes (D-179, RBI/Sigma) are diluted into binding buffer (75 mM Tris pH 7.4, 12.5 mM $MgCl_2$ and 2 mM EDTA). For example, 10 μl of membranes are diluted in 500 μl of binding buffer (i.e., 1:50 dilution), which is aliquoted into labelled tubes. Cold competitor or test competitor is added, (e.g., 5 μl of 100x stock solution; 5 μl of 1 mM haloperidol solution (H-100, RBI/Sigma in DMSO). Radioligand is then added ($^3$H-Spiperone, TRK 818, Amersham, 250 μCi in 250 μl, 10.5 μM at 95 Ci/mmol). For competition studies, 0.5 nM is used. A 100x stock (i.e., 50 nM) is made by diluting the commercial stock 1:210 in binding buffer, and 5 μl is added to every tube. Control tubes have radioligand but no membranes. The mixtures are vortexed, then spun very briefly. The mixtures are then incubated for 1 hour at 27°C in a water bath. The mixture is filtered through GF/C filters over a vacuum. Each filter is pre-soaked in polyethyleneimine (0.3% in water; add 1 ml of 10% stock (P-261; RBI/Sigma) to 33 ml of MilliQ water). The tubes are washed with 2-3 ml of wash buffer (50 mM Tris pH 7.4) and the wash filtered. The filters are then rinsed with 3 × 5 ml of wash buffer, air dried, placed in a scintillation vial and 4.5 ml of scintillant added. The activity on the filter is then determined.

For opioid receptor binding, membranes (O-116, RBI/Sigma) are diluted into binding buffer (75 mM Tris pH 7.4, 12.5 mM $MgCl_2$ and 2 mM EDTA). For example, 10 μl of membranes are diluted in 500 μl of binding buffer (i.e., 1:50 dilution), which is aliquoted into labelled tubes. Cold competitor or test competitor is added (e.g. 5 μl of 100x stock solution; 5 μl of 100 μM naltrindole solution (N-115, RBI/Sigma in DMSO). Radioligand is then added ($^3$H-Diprenorphine, TRK 1060, Amersham, 250 μCi in 250 μl, 10.5 μM at 95 Ci/mmol). For competition studies, 0.5 nM is used. A 100x stock (i.e., 50 nM) is made by diluting the commercial stock 1:210 in binding buffer, and 5 μl is added to every tube. Control tubes have radioligand but no membranes. The mixtures are vortexed, then spun very briefly. The mixtures are then incubated for 1 hour at 27°C in a water bath. The mixture is filtered through GF/C filters over a vacuum. Each filter is pre-soaked in polyethyleneimine (0.3% in water; add 1 ml of 10% stock (P-261; RBI/Sigma) to 33 ml of MilliQ water). The tubes are washed with 2-3 ml of wash buffer (50 mM Tris pH 7.4) and the wash filtered. The filters are then rinsed with 3 × 5 ml of wash buffer, air dried, placed in a scintillation vial and 4.5 ml of scintillant added. The activity on the filter is then determined.

Results

A competitive binding assay was used to determine the binding of A-I to chemokine receptors. These experiments were performed under 'Horuk' conditions (Hesselgesser et al., J. Biol. Chem., 273, 15687 (1998)), except that CHO cells expressing CCR-2 or CXCR-2 (AIDS Reagent Program, MRC London) were used. In each case, the specific

binding of 0.25 nM labeled ligand to the cells was determined as the counts bound which were competable by 100 nM cold ligand. In each experiment, A-I was added from a stock solution in DMSO (with <0.1% DMSO final concentration in the assay).

Two typical experiments are shown in Figure 53, in which A-I inhibits MCP-1 binding to CCR-2 and IL-8 binding to CXCR-2 with $k_i$ concentrations similar to the functional $ED_{50}$s reported in Table 14. Similar data was obtained using PBS as the incubation buffer.

**TABLE 14**

| Summary of Properties of A-I (NR58,4) | | |
|---|---|---|
| **Assay** | **$ED_{50}$** | **I Notes** |
| *In vitro* | | |
| MCP-1 chemotaxis assay | 2 nM | |
| MIP1α chemotaxis assay | 0.2 nM | |
| RANTES chemotaxis assay | 0.3 nM | |
| SDF1α chemotaxis assay | 8 nM | |
| IL-8 chemotaxis assay | < 10 nM | |
| fMLP chemotaxis assay | > 100 μM | fMLP is not a chemokine |
| Binding to CCR-2 | 5 nM | |
| Binding to α2-adrenoceptors | > 100 μM | No binding detected |
| Binding to HT-1a serotonin receptors | 80 μM | |
| Binding to D2s dopamine receptors | > 100 μM | No binding detected |
| Binding to ∂-opioid receptors | - | Not yet determined |
| *Physical* | | |
| Molecular weight | 293 | Free acid |
| PKa (approximate) | 11 | Very weak acid |
| Solubility in water | negligible | |
| Solubility in water (potassium salt) | 5 mg/ml | Solution at pH 11 |
| Solubility in DMSO | > 200 mg/ml | |
| *In vivo* | | |
| Effect on LPS-induced TNFα *in vivo* (1 mg s.c. injection) | 65% inhibition | |

The specificity of some of the agents against related G-protein coupled 7TM receptors, e.g., α2-adrenoreceptors, 5-HT-1α serotonin receptors, D2s dopamine receptors, and δ-opioid receptors, is shown in Table 15. In all cases 100 μM was the highest concentration tested. Where '>100 μM' is shown, no significant effect was observed at any concentration (including 100 μM).

Thus, CRD-$Leu_4Ile_{11}Cys_{13}$ peptide 3(3-12)[MCP-1], A-I, yohimbamide (Y-II), L-II and WVQ are highly specific for chemokines over the other receptor types tested. In contrast, although yohimbine and thalidomide are chemokine inhibitors, they have greater activity against one (or more) other G-protein coupled receptors tested. For example, yohimbine is selective for adrenoreceptors over chemokines and thalidomide for dopamine receptors over chemokines. A-I was the most selective (approximately 40,000 fold selective for MCP-1 inhibition over serotonin receptor binding). The most potent agent against MCP-1 (Y-II) was less selective, showing only 1,500 fold selectivity for chemokine inhibition of alpha-adrenoreceptor binding.

**TABLE 15**

| Chemokine specificity of various inhibitors | | | | | |
|---|---|---|---|---|---|
| **Compound** | **MCP-1**[a] | **α2-A**[β] | **5-HT1a**[c] | **D2s**[d] | **∂-opioid**[ε] |
| NR58-3.14.3 | 10 nM | >100 μM | >100 μM | >100 μM | >100 μM |
| NR58,4 (A-I) | 2 nM | >100 μM | 80 μM | >100 μM | n.d.[f] |
| Yohimbamide (Y-II) | 0.5 nM | 770 nM | 5 μM | 70 μM | n.d. |
| Lysergyl-glutamine (L-II) | 5 nM | n.d. | 1 μM | n.d. | n.d. |
| WVQ | 1 μM | >100 μM | >100 μM | n.d. | n.d. |
| Yohimbine | 30 nM | 4 nM | 2 μM | n.d. | n.d. |
| Thalidomide | 50 μM | >100 μM | n.d. | >100 μM | n.d. |
| LII | 5 nM | n.d. | 1 μM | n.d. | n.d. |

[a] MCP-1 = $ED_{50}$ versus MCP-1 induced chemotaxis
[b] α2-A = $ED_{50}$ for competition of RS79943 binding to α2-adrenoceptors
[c] 5-HT1a = $ED_{50}$ for competition of 8-OH-DPAT binding to S-HT1a receptors
[d] D2s = $ED_{50}$ for competition of spiperone binding to short splice variant D2 dopamine receptor
[e] ∂-opioid = $ED_{50}$ for competition of diprenorphine binding to ∂-opioid receptors.
[f] n.d. = not yet determined.

## Example 23

Plasma Half-Life of a Glucose Conjugate

To determine whether sugar conjugates of an agent of the invention had increased plasma half-lives, a glucose conjugate of CRD-$Leu_4Ile_{11}Cys_{13}$ peptide 3(3-12)[MCP-1] was prepared and administered to rats. The conjugate was synthesized by mixing D-glucose (50 mM) and CRD-$Leu_4Ile_{11}Cys_{13}$ peptide 3(3-12)[MCP-1] (1 mM) in phosphate buffered saline and incubating at room temperature for 16 hours. The reaction mixture was then subject to analytical HPLC which indicated a yield of about 30%. The material was then treated with cyanoborohydride to effect reduction of the schiffs base linkage and stabilize the glucosides for purification. The stabilized glucosides were then subjected to preparative HPLC and the mono- and di-substituted glucosides prepared separately. The monoglucoside was obtained at >90% purity in approximately 10% yield, and it is likely that the material is a mixture of Lys5-glucoside and Lys-7 glucoside.

The CRD-$Leu_4Ile_{11}Cys_{13}$ peptide 3(3-12)[MCP-1] monoglucoside was injected into 3 rats (5 mg per rat via s.c. route in sterile PBS). Blood samples were withdrawn through an i.v. cannula at various time points, and CRD-$Leu_4Ile_{11}Cys_{13}$ peptide 3(3-12)[MCP-1] glucoside was quantitated in the serum. Using a one-compartment fit to the data suggested, the T1/2α for the CRD-$Leu_4Ile_{11}Cys_{13}$ peptide 3(3-12)[MCP-1] monoglucoside was determined to be approximately 7 hours, which represents an increase of more than 25-fold compared to the unmodified cyclic peptide (T1/2α = 15 minutes).

## Example 24

Agonist Activity

To evaluate the agonist activity ofCRD-$Leu_4Ile_{11}Cys_{13}$ peptide 3(3-12)[MCP-1], CRD-Leu4$Ile_{11}Cys_{13}$ peptide 3(3-12)[MCP-1] was placed in the lower compartment of the migration chamber at various concentrations and 50,000 THP-1 cells were placed in the upper compartment. The number of cells in the lower compartment after 4 hours was then determined using the vital dye MTT. No agonist activity was detected in the concentration range where inhibitory effects on chemokine-induced migration are seen (<1 μM). At higher concentrations (1 μM and 10 μM), there may be a small stimulation of migration in this experiment, although the magnitude of this migratory response was less than that seen with 3.25 ng/ml recombinant MCP-1. CRD-$Leu_4Ile_{11}Cys_{13}$ peptide 3(3-12)[MCP-1] is unlikely to have any agonist activity that is significant at concentrations attainable *in vivo*.

The concentration of MIP-1α in the bottom of the chemotaxis chamber was varied in the presence or absence of a half-maximally active dose of the inhibitor in the upper chamber of the plate (with the cells). Consistent with previous

observations, increasing the concentration of MIP-1a in the absence of inhibitor first stimulates migration (at concentrations up to approximately 5 ng/ml) and thereafter further increases in MIP-1α decrease migration. At 100 ng/ml MIP-1α, there is no stimulation of migration (Figure 49). This biphasic (or bell-shaped curve) is seen with any pro-migratory agent which is purely chemotactic (rather than chemokinetic), similar to the chemokines. At higher concentrations in the bottom of the well, diffusion rapidly destroys the gradient across the membrane, such that the receptors all around the target cells are fully occupied by the chemokine. In the absence of a gradient, chemotactic agents do not stimulate movement. When the dose-response to MIP1α is performed side by side in the presence of a sub-maximal dose of the inhibitor, a similar biphasic (or bell-shaped curve) is seen. However, the dose response curve is shifted markedly to higher concentrations (this assay is not sufficiently accurate to allow the extent of the shift to be estimated, but it lies in the range of 5-100 fold). This experiment therefore provides some evidence for a competitive mode of action for the inhibitor acting against MIP1α-induced chemotaxis. The experiment shown is typical of three replicate experiments, and was also performed using MCP-1.

## Examples 25

### Binding Affinity of the Agents of the Invention

It is also envisioned that moieties other than those exemplified, including analogs of chemokine peptide 2 or 3, variants or derivatives thereof, which bind to DARC and/or chemokine receptors with a specific affinity, e.g., they bind to functional chemokine receptors with high affinity but bind with lower affinity to DARC or bind to DARC with a high affinity but bind to chemokine receptors with lower affinity, may be identified using methods such as those described above. Moreover, the agents of the invention may be useful in functional mapping of chemokine receptors. For example, both chemokine peptide 2 and peptide 3 block binding of the natural chemokine ligands in a competitive manner. However, they do not block binding of one another suggesting that they bind to distinct regions of the receptor and that both of these regions are important for binding of the natural ligand. In addition, peptide 2 is further distinguished from peptide 3 in their differential functional activity. Peptide 3 not only binds to the receptor but also blocks the functional activity of receptor signaling as indicated by inhibition of chemotaxis. Peptide 2 does not inhibit chemotaxis. Thus, these peptides together are particularly useful in identifying regions of chemokine receptors that are important in different functional activities. Once these regions are identified, they can be used to screen combinatorial libraries or compound banks for specific inhibitors to distinct chemokine functions that may be structurally unrelated to the starting compounds, but are functionally related.

In addition, it may be important for chemokines to form dimers to activate the receptor of interest. The peptides of the invention lack the amino terminal domains that are thought to be important for chemokine dimer formation. If dimer formation is required for cell signaling, then the agents of the invention may inhibit activation as they can bind to the receptor but are unable to form dimers, e.g., with native chemokine ligand.

## Examples 26

### Anti-HIV Activity of the Agents of the Invention

To demonstrate that the agents of the invention inhibit HIV binding and infection of cells, human T-cell derived Jurkat cells were incubated with an infectious T-tropic HIV isolate in the presence of (i) no inhibitor, (ii) peptide C (Table 17) as an inactive control peptide, (iii) 100 μM peptide 3(1-12)[MCP-1] (SEQ ID NO:1), or (iv) 100 ng/ml SDF-1, which should bind to and block all CXCR-4 receptors. After 3 weeks in culture, viral replication was assessed by a reverse transcriptase assay of the culture medium. Peptide 3(1-12)[MCP-1] (SEQ ID NO:1) was found to be an effective inhibitor of Jurkat cell infection by HIV.

Since peptide 2(1-15)[MCP1] (SEQ ID NO:3) binds to chemokine receptors on the surface of Jurkat cells (Ka = 250 nM; B = 55,000 sites/cell) and THP-1 cells (Ka = 300 nM; B = 130,000 sites/cell), but does not inhibit productive signaling by chemokines, it is possible that peptide 2(1-15)[MCP-1] (SEQ ID NO:3) binds and inhibits an epitope used by HIV for cell entry but not by MCP-1 for signaling. To test this hypothesis, the same HIV infection assay described above was employed to test whether peptide 2(1-15)[MCP-1] (SEQ ID NO:3) inhibits HIV infection of Jurkat cells. At 100 μM, peptide 2(1-15)[MCP-1] (SEQ ID NO:3) was more effective than peptide 3(1-12)[MCP-1] (SEQ ID NO:1), and as effective as SDF1α, in preventing virus entry.

Peptide 2 derivatives (Figure 52) are better inhibitors of Jurkat T cell infection by HIV (a CXCR4 mediated event) than peptide 3 derivatives, while surprisingly peptide 3 is a better inhibitor of THP-1 cell infection (a CCR-5 mediated event). Thus, combinations of peptide 2 and peptide 3 may be particularly useful for anti-HIV therapy, e.g., to inhibit productive infections by both M-tropic and T-tropic isolates.

Moreover, as LRD peptide 2(1-15)[MCP-1] had a 100 nM affinity constant or lower for CCR5/CXCR4 and a

100 fold decrease in Duffy binding relative to LFL peptide 2[MCP-1], LRD derivatives may be more efficacious than their LFL counterparts (25 μM versus 100 μM for LFL).

Current therapies for inhibition of HIV focus on the virus, for example reverse transcriptase inhibitors or viral protease inhibitors. These therapies are only effective for a limited period. In each case, the efficacy is reduced because the virus is undergoing rapid replication, and there is selection in favor of mutants which are resistant to the inhibitors. Although combination therapies are more effective, they are unlikely to result in clearance of the virus from an infected individual. Eventually, mutant virus will arise which circumvents the drug cocktail and progression will again occur in the now drug-resistant individual. Thus, strategies which are based on co-receptor inhibition (i.e., viral entry) target a host protein, rather than a virus protein, may have increased efficacy as more extensive mutations in the virus may be necessary to circumvent an inhibited co-receptor. Indeed, the resistance to infection of CCR-5Δ32 homozygotes suggests that the virus cannot readily adapt to use of an alternative co-receptor, at least while the virus population is small. Thus, the agents of the invention may be used in conjunction with other anti-viral therapies, e.g., protease inhibitors, reverse transcriptase inhibitors, or a combination thereof.

Preferably, a Ser10 variant of peptide 2(1-15)[MCP-1] (SYRRITSSKSPKEAV), or its LRD $Cys_0Ser_{10}Cys_{16}$ derivative (cvaekpsksstirrysc) or CRD derivative, is employed. DARC binding of SYRRITSSKSPKEAV is in the range 20 μM to 100 μM and activity in the range 1-100 nM as an anti-HIV agent.

**Example 27**

Rapid Screening Method for Infectivity

Current assays for HIV infection *in vitro* are time consuming and lack reproducibility. For example, infection is often monitored by the production of viral reverse transcriptase (RT) activity using a radiolabelled RT substrate. Unfortunately, RT production is low, even when a laboratory adapted HIV strain is used to infect a high permissive line such as the Jurkat human T cell line. As a result, it is necessary for the infected cells to be cultured for two or more weeks to allow sufficient infection to occur for RT production be measurable. In addition to being time consuming, this assay has a number of other disadvantages: most importantly, it relies on multiple rounds of secondary infection to increase the viral titer sufficiently for RT activity to become detectable. As a result, small differences in primary infection are magnified, and since primary infection frequency is low, stochastic differences between identically treated wells become significant. The assay therefore requires many replicate wells for each analysis, with as many as 24 replicates being routinely used. For example, in a typical assay groups of 24 wells of Jurkat cells in 96-well plates are infected with replicate aliquots of HIV virus stock, with one group receiving treatment with peptide 2 as a chemokine co-receptor inhibitor, another group receiving SDF-1α (the CXCR-4 natural ligand) and a third group is untreated. After three weeks, the cells were harvested and RT activity measured. The co-efficient of variation in the untreated wells was 37%. As a result, although peptide 2 inhibited RT activity by 75%, this was significant only with p=0.02 because of the high well to well variability. This necessitates the use of many replicate making the assay cumbersome for screening purposes.

An alternative method is to use direct visualization of the HIV proteins, for example, by immunofluorescence microscopy. Unfortunately, even the most highly expressed HIV proteins (such as p24gag) are present at fairly low levels in cells. Thus, direct detection the earliest stages after infection has been difficult and error prone. Therefore, the following method was employed to enhance the sensitivity of immunofluorescence, allowing the number of HIV infected cells to be accurately determined between 24 hours and 72 hours after infection. Furthermore, the signal to noise ratio of this technique allows automated counting of the infected cells using image analysis software.

For THP-1 cells, the cells are adhered to glass multiwell slides (for example, 16-well chamber slides; Nunc) using PMA and hydrocortisone. The cells are then exposed to virus in the chamber slide in the presence of various test agents. For non-adherent cells such as Jurkat cells, infections are carried out in, for example, 96-well culture plates as for RT assays, but prior to analysis the cells are attached to glass slides using a cytospin apparatus in accordance with the manufacturer's instructions. The infected cells on the glass slides are fixed between 24 hours and 72 hours after infection, for example, by immersing the slides in ice cold acetone for 90 seconds. Other methods of fixation compatible with quantitative immunofluorescence may also be used (see J. Histochem Cytochem., 44, 1043 (1997) for a discussion of quantitative immunofluorescence procedures). Following fixation, non-specific binding of proteins to the cells is blocked, e.g., by incubation in 3% w/v fatty acid free bovine serum albumin in phosphate buffered saline (3% FAF-BSA in PBS) for 30 minutes at room temperature. Alternatively, other blocking solutions (e.g., 5% sucrose, 5% Tween-20 in PBS) may be used. The blocked sections are then stained for HIV protein, for example, using a specific antiserum to p24gag. Slides are incubated with the antiserum at a suitable concentration (usually in the range 1-100 μg/ml of specific IgG) in 3% FAF-BSA in PBS. Antibodies to other HIV antigens may be used, although relatively highly expressed antigens such as p24gag are preferred.

This incubation should be left on for at least 16 hours. Traditional immunofluorescence procedures use primary antibody incubation periods typically 1-2 hours in length, but longer incubation increases signal without increasing

background (J. Histochem. Cytochem., 44, 1043 (1997)). The incubation may be left on for up to 36 hours without deleterious effects on the signal to noise ratio. Unbound antibody is then washed off. Typically, this involves 3 x 3 minute washes in PBS, although other washing regimens may be used (see J. Histochem. Cytochem., 44, 1043 (1997)) for a comparison of washing methods). Normally, second antibody labelled with an appropriate fluorophore is then used to detect the unbound primary antibody. However, to prevent primary antibody from falling off the antigen, primary antibody is post-fixed to the section. This may be achieved, for example, by incubating the slide in freshly prepared 4% paraformaldehyde in PBS for 10 minutes at room temperature. After three further washes, e.g., 3 x 3 minutes in PBS, the slides are exposed to a secondary antibody specific for the species of the primary antibody coupled to an appropriate fluorophore (for example, antirabbit-IgG FITC conjugate at 1-100 μg/ml). A non-specific nuclear stain should be included in this incubation. For example, Hoescht 33342 at 1-100 ng/ml could be used, or propidium iodide at 1-100 ng/ml. This incubation is for a minimum of about 4 hours, preferably at least 8 hours and may be left up to 24 hours without detrimental effect on the signal to noise ratio. Slides are then washed, for example, 3 x 3 minutes in PBS, to remove unbound second antibody and mounted with a suitable mounting medium such as Citifluor AF1. Slides are left at least about 18 hours after mounting but less than about 72 hours in a dark box following mounting prior to analysis.

Analysis may be performed manually using any suitable microscope with epifluorescence visualization capability and appropriate filter sets to allow examination of the fluorescence of the secondary antibody fluorophore selected (e.g., FITC) and the non-specific nuclear staining selected (e.g., Hoescht 33342) separately. The number of cells in each field of view is determined by counting nuclei using filters to visualize the non-specific nuclear stain. The number of cells infected with HIV in the same field of view is then determined by switching the filter set to visualize the fluorophore coupled to the secondary antibody. In each case, the number of cells may be determined by manual counting. Alternatively, image analysis software (for example, OpenLab software: Improvision, U.K.) may be used to apply a consistent threshold to each image and count the number of separate objects above that threshold. Deagglomeration algorithms, standard in the field of image analysis, may be applied if required according to the density of the cells on the slides. Provided that constant set of illumination conditions are used during image acquisition and that a constant threshold is applied, the fraction of HIV stained cells may be rapidly and accurately determined without reference to subjective considerations.

## Examples 28

Use of the Agents of the Invention to Block gp120 Binding

A number of studies, for example using chimeric receptors, have begun to localize the binding site for the HIV envelope protein gp120 and the chemokine ligands on the CCR5 receptor. These reports suggest that the N-terminal region is important for both gp 120 and chemokine binding (Wells et al., Methods, 10, 126 (1996); Ross et al., J Virol., 72,1918 (1998); Alkhatib et al., J. Biol. Chem., 272, 19771 (1997); Dragic et al., J. Virol., 72, 279 (1998); Monteclaro et al., J. Biol. Chem., 272, 23186 (1997)) but that the two bind to overlapping but non-identical sites involving not only the N-terminal region but also the extracellular loops of the receptor (Ross et al., J. Virol., 72, 1918 (1998); Farzan et al.,J. Biol. Chem., 212, 6854 (1997)).

Methods

Peptides. The peptides were prepared by Affinity (Exeter, U.K.) by standard solid phase chemistry, followed by reverse phase HPLC purification to greater than 95% purity. Peptide 2 (derived from amino acids 28-42 of mature human MCP-1) has the sequence SYRRITSSKCPKEAV (SEQ ID NO:3). Peptide 3 (derived from amino acids 51-62 of mature human MCP-1) has the sequence EICADPKQKWVQ (SEQ ID NO:1). Labeled peptides were synthesized with an N-terminal biotin moiety. Peptides were also synthesized corresponding to the sequence of the full-length V3 loop (including terminal cysteine residues) of gp120 from HIV-1 IIIb and HIV-1 BaL. All peptides were prepared as TFA salts and dissolved in sterile MilliQ to give 10 mM stock solutions which were stored at -20˚C until used.

Binding assays. Cells (either Jurkat T-cells or THP-1 cells) were grown in RPMI 1640 medium supplemented with 10% fetal calf serum, 2 mM glutamine, 20 μM β-mercaptoethanol, 100 U/ml penicillin and 100 μg/ml streptomycin and maintained between $2 \times 10^5$ and $1 \times 10^6$ cells/ml. Prior to performing a binding assay, cells were spun out (100 × g; 4 minutes) and washed 3 times in ice-cold PBS. A volume of cell suspension in PBS containing $10^6$ cells (for Jurkat cells) or $4 \times 10^5$ cells (for THP-1 cells) was pipetted into each well of a V-bottom 96-well plate (Gibco BRL) and spun out (100 x g; 4 minutes). Triplicate wells were then resuspended in 100 μl binding medium (PBS pH 7.2 containing 0.1% fatty acid-free bovine serum albumin (BSA)) containing labeled peptide in the presence or absence of various concentrations of unlabeled peptide. The plate was then incubated on ice for 90 minutes. Cells were washed 3 times with 380 μl of ice-cold PBS, spinning out the cells each time (100 × g; 4 minutes), and resuspended in 100 μl binding medium containing streptavidin-peroxidase (Amersham International) at 1:1000 dilution. Cells were incubated for a further 15

minutes on ice to allow labeling of any bound biotinylated peptide, then washed 4 times as above. Cells were then incubated with 200 μl TMB substrate (K-Blue, Bionostics) for 20 minutes at room temperature, and the reaction stopped by addition of 50 μl 2 M HCl. The plate was spun (3,000 × g; 3 minutes) and 200 μl of the colored product was transferred to an empty 96-well ELISA plate and the absorbance at 450 nm determined. For each assay, the absorbance of a blank reaction (no peptide added) was subtracted from all readings before further analysis.

For Scatchard analysis, the absorbance readings at 450 nm were calibrated, by reference to a standard curve in which known amounts of labeled peptide were coupled to derivatized sepharose beads (activated thiol-sepharose 4B; Sigma T8512) via the cysteine residue in each peptide. Briefly, known amounts (1 fmol to 1 pmol) of biotin-labeled peptide were incubated with 1 mg of activated thiol-sepharose beads in 100 μl of PBS at 37°C for 1 hour. Free activated thiols were then blocked by addition of cysteine to a final concentration of 10 mM. The peptide-conjugated beads were then washed 5 times in PBS and treated exactly as for the cells described above. The absorbance at 450 nm obtained when a known amount of peptide was coupled to the beads was used to construct a standard curve which was used to convert the absorbance readings obtained with cells into number of molecules of labeled peptide which had bound. This method has a sensitivity <1 fmol of labeled peptide bound, allowing Scatchard analysis of binding even where the affinity is moderate or the number of binding sites relatively small.

Binding experiments with labeled peptide 2 were performed with 10 nM labeled peptide in each reaction. Labeled peptide 3 was used at 250 nM and labeled peptides from the V3 loop of gp120 from HIV-1 were used at 100 μM, unless stated otherwise.

Immunefluorescence detection of $p24^{gag}$

Jurkat cells following HIV infection were attached to 8-well chamber slides (Becton-Dickinson) by spinning the slides using a plate rotor in a Labofuge centrifuge (Haeraeus) at 3,000 x g for 5 minutes. Attached Jurkat cells or THP-1 cells were then fixed by dipping the slides into ice-cold 70% ethanol for 90 seconds. Non-specific binding was blocked by incubation with 3% fatty acid-free BSA in TBS for 1 hour at room temperature. Cells were incubated with the mouse monoclonal anti-HIV-1 $p24^{gag}$ antibody EH12E1 (Ferns et al., L Gen. Virol., 68, 1543 (1987); AIDS Reagent Program, NIBSC) at 10 μg/ml in 3% BSA in TBS at room temperature overnight. Unbound antibody was removed with 3 × 3 minute washes in PBS, and bound antibody was then fixed to the slide by incubation with 3.8% phosphate-buffered formalin pH 7.2 for 10 minutes at room temperature, followed by 3 further 3 minute washes in PBS. Bound antibody was visualized using donkey anti-mouse IgG FITC conjugate (715-095-150; Jackson Immunoresearch) at 30 μg/ml in 3% BSA/TBS + 1 ng/ml Hoescht 33342 for 6 hours at room temperature. Twelve fields of view (100 × magnification) were captured from each well of the chamber slide using an Olympus Provis AX electronic microscope connected to a Power Macintosh 8500, running OpenLab image analysis software (Improvision), under both FITC illumination conditions (NIBA filter block; λex = 470-490 nm, dichroic mirror = 505 nm, λem = 515-550 nm) and UV illumination conditions (Chroma 31000; λex = 340-380 nm, dichroic mirror = 400 nm, λem = 435-485 nm). Images were acquired with a Hamamatsu C4742-05 monochrome digital camera with 10 bit depth in a 1280-1024 pixel field connected to a DIG Snapper frame grabber. The exposure time, amplifier gain and offset values were controlled by the OpenLab software and were held constant throughout the experiment. A background (an image captured without a slide under the objective) was digitally subtracted from every image. A threshold was then applied to each image which was the lowest threshold that detected <1% of the pixels of an image of uninfected cells stained under identical conditions. The number of objects exceeding this threshold in each field of view were counted. This process, for example, detected 9 objects in the field of view shown in the upper panel of Figure 54D and 1 object in the field of view shown in the lower panel. A similar procedure was used to determine the total number of nuclei in the same field of view, using the image captured under UV illumination conditions. The ratio of positively stained objects to nuclei in each field of view was reported as the percentage of cells stained for $p24^{gag}$.

Results

Binding of peptides to THP-1 and Jurkat cells

To test whether peptide 2 and peptide 3 were likely to bind to the same or to different sites on the chemokine receptors, the effect of unlabeled peptide 2 on the binding of biotinylated peptide 3 and vice versa was analyzed. The affinity constant for peptide 3 binding to THP-1 cells was similar in the presence (8 μM) and absence (7 μM) of 100 μM peptide 2. Similarly, the affinity constant for peptide 2 binding was unaffected by the presence of 100 μM peptide 3 (250 μM in each case). These data suggest that peptide 2 and peptide 3 bind to distinct sites on the chemokine receptors. Similar data was obtained using Jurkat T-cells.

Effect of peptides on gp120 binding

The binding of gp120 to chemokine receptors is likely to involve sequences in the V3 loop of gp 120 (Ross et al., Proc. Nat'l Acad. Sci. U.S.A., 95, 7682 (1998); Cocchi et al., Nat. Med.,2, 1244 (1996); Jiang et al., Nat. Med., 3, 367 (1997)). Therefore peptide sequences were synthesized from the V3 loop of the M-tropic BaL strain and the T-tropic IIIB strain and analyzed the binding of these biotinylated peptides to the THP-1 and Jurkat cells. Specific binding of gp120:V3(BaL) to THP-1 cells was detected at 100 μM (Table 17), although the combination of low number of binding sites per cell and moderate affinity of the interaction precluded accurate Scatchard analysis of the binding. In contrast, specific binding of gp120:V3(BaL) to Jurkat cells was not detected even at concentrations up to 500 μM (Table 17). These observations are consistent with the hypothesis that gp120:V3(BaL) binds specifically to CCR5, which is expressed on the surface of the THP-1 monocytic cells but not the Jurkat T-cells.

TABLE 17

| | ΔA450nm | | |
|---|---|---|---|
| | alone | +100-fold excess unlabeled peptide | Specific binding |
| Jurkat T-cells | | | |
| gp120:V3(IIIb) | 0.878 ± 0.013 | 0.189 ± 0.010 | 0.689 |
| gp120:V3(BaL) | 0.167 ± 0.019 | 0.168 ± 0.020 | <0 |
| THP-1 cells | | | |
| gp120:V3(IIIb) | 0.268 ± 0.024 | 0.131 ± 0.011 | 0.137 |
| gp120:V3(BaL) | 0.642 ± 0.009 | 0.107 ± 0.015 | 0.535 |

N-terminally biotinylated peptides corresponding to the V3 loop sequence (including the terminal cysteines, residues 303-339 in gp120(IIIb)) from HIV IIIb of BaL were incubated at 100 μM with Jurkat cells ($10^6$ cells per reaction) or THP-1 cells ($4 \times 10^5$ cells per reaction) at 4°C in binding medium. Bound peptide was then labeled with streptavidin-peroxidase and visualized using the chromogenic substrate TMB. Each reaction was performed in triplicate both in the absence or presence of 100-fold excess of the same peptide lacking the biotin label, to estimate the contribution of non-specific binding.

Specific binding of gp120:V3(IIIb) at 100 μM to both Jurkat T-cells and THP-1 cells was detected, but again the low number of binding sites per cell and moderate affinity of the interaction precluded accurate Scatchard analysis of the binding. There was approximately 5-fold greater specific binding to the Jurkat cells than the THP-1 cells (Table 17). These observations are consistent with the hypothesis that gp120:V3(IIIb) binds specifically to CXCR4, which is expressed on both THP-1 and Jurkat cells, but at higher levels on the T-cell line.

Effect of peptides on HIV infection *in vitro*

HIV infection of Jurkat T-cells using the laboratory-adapted T-tropic isolate IIIb was monitored using two different assays. Firstly, Jurkat T-cells in 96-well plates were pre-treated with either peptide 2, peptide 3, vehicle (as a negative control) or SDFIa (as a positive control) for 1 hour, then exposed to HIV virus ($10^6$ $TCID_{50}$) and pulsed at 2-3 day intervals with peptide, SDF1α or medium alone as appropriate. After two weeks in culture, the extent of viral infection was assayed by measuring the reverse transcriptase activity in the supernatant, as a measure of viral replication in the culture. Peptide 2 (100 μM) but not peptide 3 (100 μM) markedly inhibited virus replication following HIV exposure (Figure 54A), suggesting this peptide had inhibited HIV infectivity in *vitro.* No effect was seen on cell viability. Reverse transcriptase activity was inhibited by an average of 75% in six similar experiments, and in each case the inhibition achieved with peptide 2 was similar to that with SDF-1α.

HIV infection of Jurkat T-cells was also monitored by high sensitivity quantitative immunofluorescence detection of viral $p24^{gag}$expression. Jurkat cells were infected with HIV in the presence or absence of peptide 2 (100 μM), peptide 3 (100 μM) or SDF1α (100 ng/ml) as described above. Approximately 48 hours after infection, the cells were attached to glass slides using a cytospin and then fixed by emersion in ice-cold 70% ethanol for 90 seconds. Expression of $p24^{gag}$was determined using quantitative immunofluorescence as previously described (Mosedale et al., J. Histochem. Cytochem., 44, 1043 (1996)), except that the primary antibody was post-fixed to the section using paraformaldehyde to increase the sensitivity of the technique. Viral infectivity was expressed as the number of cells stained for $p24^{gag}$expressed as a proportion of the total number of cells (detected using Hoechst 33342 nuclear dye). Consistent with the reverse transcriptase assay results, peptide 2 and SDF1α inhibited viral infectivity by more than 80% (Figure

54B), while peptide 3 was ineffective.

Infection of THP-1 cells with M-tropic isolates does not generate high levels of virus particles and hence the reverse transcriptase assay is not sufficiently sensitive to monitor the progress of the infection. However, it was possible to assess HIV infectivity of THP-1 cells using high sensitivity immunofluorescent detection of $p24^{gag}$. THP-1 cells were differentiated with hydrocortisone and PMA, then treated with TNFα, resulting in adherent monolayers on glass chamber slides. The THP-1 cells were then treated with either peptide 2 (100 μM), peptide 3 (100 μM), MIP1α (100 ng/ml) or SDF1α (100 ng/ml) as for the Jurkat cells. THP-1 cells were infected with HIV strain MN at a concentration previously validated to produce easily detectable infection and growth for 72 hours prior to fixation and staining for $p24^{gag}$.Peptide 2 inhibited HIV infectivity of THP-1 cells by 28 ± 5% ($p < 0.05$; Mann-Whiney U test), while peptide 3 inhibited infection of THP-1 cells by more than 80% (Figure 54C, D).

Table 18 summarizes the DARC binding, ED50s for chemokine and gp120, as well as the percent of virus inhibition by various peptide 2 sequences.

Discussion

Two different oligopeptide sequences from human MCP-1 are able to inhibit cellular infection by HIV-1 isolates *in vitro.* One of these sequences, peptide 2, inhibited both M-tropic and T-tropic infection, suggesting that, unlike agents which specifically target individual chemokine receptors (Cairns et al., Nat. Med., 4, 563 (1998); Simmons et al., Science, 226, 276 (1997)), it may be possible to simultaneously inhibit usage of a wide range of related co-receptors using a single molecule. Moreover, this peptide substantially reduces CXCR4-dependent HIV infection of Jurkat T-cells (assayed either by reverse transcriptase production (75 ± 10% inhibition) or immunofluorescence staining for $p24^{gag}$ (80 ± 3% inhibition). Peptide 2 also reduces CCR5-dependent HIV infection of THP-1 cells (28% ± 5% inhibition, assayed by immunofluorescence staining). However, peptide 2 did not inhibit chemokine signaling through the receptors, since both SDF1α and MIP1α were fully chemotactic for THP-1 cells in the presence of peptide 2. A second peptide, spanning amino acids 51 to 62 of human MCP-1, did not inhibit CXCR4-dependent HIV infection of Jurkat T-cells (despite inhibiting SDF1α-mediated chemotaxis) but strongly inhibited CCR5-mediated HIV infection of THP-1 cells (83 ± 7% inhibition assayed by immunofluorescence staining).

**TABLE 18**

| Peptide 2 Sequences | DARC binding (μM) | MCP-1 ED-50 (μM) | MIPla ED-50 (μM) | SDF1a ED-50 (μM) | gp120 ED-50 (μM) | % virus inhibition |
|---|---|---|---|---|---|---|
| SYRRITSSKCPKEAV | 0.1 | ns | ns | ns | 0.4 | 85 |
| Vaekpcksstirrys | 18 | ns | ns | ns | 3 | 72 |
| HLKILNTPNCALQIV | 19 | 10 | 40 | 7 | 35 | 42 |
| DYFETSSQCSKPGV | ns | ns | ns | ns | >100 | n.d. |
| vgpkscqsstefyd | ns | ns | ns | ns | ns | ns |
| SYRRITSSKC | 22 | ns | ns | ns | 60 | ns |
| CPKEAV | >100 | ns | ns | ns | ns | ns |
| SYRRI | ns | ns | ns | ns | ns | ns |
| TSSKC | ns | ns | ns | ns | ns | ns |
| DYFETSSQC | ns | ns | ns | ns | ns | ns |
| CSKPGV | ns | ns | ns | ns | ns | ns |
| CSYRRITSSKSPKEAVC | ns | ns | ns | ns | >100 | ns |
| cvaekpsksstirrysc | >100 | ns | ns | ns | ns | ns |

(continued)

| Peptide 2<br><br>Sequences | DARC binding<br><br>(μM) | MCP-1 ED-50<br><br>(μM) | MIPIa ED-50<br><br>(μM) | SDF1a ED-50<br><br>(μM) | gp120 ED-50<br><br>(μM) | % virus<br><br>inhibition |
|---|---|---|---|---|---|---|
| =cvaekpsksstirrysc= | ns | ns | ns | ns | ns | ns |
| Notes: ns = not significant (assay performed, but peptide was not significantly different from control)<br>n.d. = not determined<br>>100 = ED50 not determined | | | | | | |

Consistent with extensive previous studies using chemokine receptors with selected amino acid substitutions (for example, see Ross et al., J. Virol., *supra;* Alkhatib et al., *supra;* Dragic et al., *supra* and the references therein), the results described herein suggest that HIV gp120 binds to both CXCR4 and CCR5 at a site overlapping with, but not identical to, the chemokine ligand binding site. Both Ross et al. (1995) and Dragic et al. (1998) highlight the importance of residues in the amino terminal region of CCR5 for gp120 binding, although both studies recognize that residues in the other extracellular loops of the receptor are likely to play a role in productive binding of gp120. Since peptide 2 blocks gp120:V3 loop peptides from binding to cells, this tentatively suggests that peptide 2 may be binding to a site in the N-terminal region of the receptor (see Figure 16). This is consistent with the studies of Wells and colleagues who showed that the residues in the peptide 2 region of CC-chemokines interacted with the N-terminus of the chemokine receptors (Monteclaro et al., *supra;* Lusti-Narasimhan et al., J. Biol. Chem., 211, 3148 (1996); Lusti-Narasimhan et al., J.Biol. Chem., 270, 2716 (1995)). Interestingly, however, they showed that the tyrosine residue at position 2 in the peptide 2 sequence is important for determining receptor specificity: chemokines with leucine at this position bound to CXCR receptors, while chemokines with tyrosine bound to CCR receptors. The results described herein suggest that as an isolated 15mer peptide (as opposed to in the context of the whole chemokine), the tyrosine-containing sequence from MCP-1 is able to interact with both CXCR and CCR receptors. Indeed, the tyrosine-containing peptide 2 was a more effective inhibitor of CXCR4-dependent T-tropic HIV infection than of CCR5-dependent M-tropic infection. It will be interesting to determine the effect, if any, of introducing a Tyr->Leu substitution into the peptide 2 sequence.

Although the studies by Wells and colleagues implicate the N-terminal region of CCR5 in chemokine ligand binding (Monteclaro et al., *supra;* Lusti-Narasimhan et al., J. Biol. Chem., 271, 3148 (1996); Lusti-Narasimahn et al., L Biol. Chem., 270, 2716 (1995)), peptide 2 inhibits gp120:V3 loop binding without affecting chemokine binding and signaling. This is consistent with the work of Ross et al. (1998) who found that some amino acid substitutions in the N-terminal region inhibited gp120 binding but not binding and signaling by the natural chemokine ligand. In marked contrast, peptide 3 binds to region on CCR5 which is required for both gp120:V3 loop binding and for chemokine binding and signaling. Experiments with peptide 3, therefore, indicate a difference in the properties of CXCR4 and CCR5: peptide 3 inhibits SDF1α signaling, but does not affect gp120:V3 loop binding or CXCR4-dependent HIV infection. Taken together, these observations suggest that the gp120 binding site and chemokine ligand binding site are more distinct on the CXCR4 receptor (Figure 16), although binding of the full-length chemokine is still able to prevent gp120 binding (Cocchi et al., Science, 270, 1811 (1995); Zaguny et al., Proc. Nat'l Acad. Sci U.S.A., 95, 3857 (1998); Wells et al., Methods, 10, 126 (1996)).

In addition, the observations reported herein are consistent with both peptide 2 and peptide 3 binding directly to the chemokine receptors and inhibiting gp120:V3 loop binding and HIV-1 infection. Moreover, the peptides inhibit gp120:V3 loop binding. However, the affinity of the interaction between the isolated V3 loop peptide and cells was of only moderate affinity and may not be a relevant model for the intact virus particle docking with the chemokine receptors. This is consistent with data suggesting that other regions of gp120, such as the V1/V2 loops, are also important in co-receptor binding (Ross et al., Proc.Nat'l Acad. Sci. U.S.A., 95, 7682 (1998)). The inhibition of infectivity could result from a number of different mechanisms: the peptides may act as simple competitive inhibitors for virus particle binding to the coreceptor. Alternatively, the peptides may induce internalization and inhibit recycling of chemokine receptors as previously described for AOP-RANTES (Mack et al., L Exp. Med., 187, 1215 (1998)), although this mechanism could not account for the inhibition of gp120:V3 loop binding, since these binding experiments were performed at 4°C.

In summary, oligopeptide sequences derived from the chemokine MCP-1 are able to inhibit HIV infection *in vitro* to a similar extent to full-length chemokines. Furthermore, one of the sequences (peptide 2), uniquely among chemokine antagonists described to date, inhibits both M-tropic and T-tropic HIV infection raising the possibility that single agents may simultaneously block the ability of HIV to utilize a variety of chemokine co-receptors and hence provide a more complete block on HIV infectivity.

All publications, patents and patent applications are incorporated herein by reference. While in the foregoing

specification this invention has been described in relation to certain preferred embodiments thereof, and many details have been set forth for purposes of illustration, it will be apparent to those skilled in the art that the invention is susceptible to additional embodiments and that certain of the detailed herein may be varied considerably without departing from the basic principles of the invention.

The following items further characterise the invention:

1. A method of preventing or inhibiting an indication associated with a chemokine-induced activity, comprising: administering to a mammal afflicted with, or at risk of, the indication an amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);
or a pharmaceutically acceptable salt thereof;
or a combination thereof;
effective to prevent or inhibit said activity, wherein the chemokine is not IL8 or NAP-2.

2. A method to inhibit the activity of more than one chemokine, comprising:

administering to a mammal in need thereof an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);

or a pharmaceutically acceptable salt thereof;
or a combination thereof.

3. A method of preventing or inhibiting an indication associated with hematopoietic cell recruitment, comprising: administering to a mammal at risk of, or afflicted with, the indication an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);
or a pharmaceutically acceptable salt thereof;
or a combination thereof.

4. A method of preventing or inhibiting an indication associated with histamine release from basophils or mast cells, comprising administering to a mammal at risk of, or afflicted with, the indication an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);
or a pharmaceutically acceptable salt thereof;
or a combination thereof.

5. A method to modulate the chemokine-induced activity of hematopoietic cells at a preselected physiological site, comprising: administering to a mammal a dosage form comprising an effective amount of:

1) a compound of formula (XIII), (XIV), (X), (XI), (XIX), or (XII);
2) a saccharide conjugate of a chemokine peptide 3, a variant thereof, a derivative thereof, a compound of

formula (IV), a compound of formula (V), a compound of formula (VI), a compound of formula (XIII), a compound of formula (XIV), a compound of formula (X), a compound of formula (XI), a compound of formula (XII), or a compound of formula (XIX);
or a pharmaceutically acceptable salt thereof;
or a combination thereof;
wherein the dosage form is linked to a site targeting moiety.

6. A method to prevent or inhibit stroke, comprising:

administering to a mammal an effective amount of:

1) a compound of formula (XIII), (XIV), (X), (XI), (XIX), or (XII);
2) a saccharide conjugate of, chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);

or a pharmaceutically acceptable salt thereof;
or a combination thereof.

7. A therapeutic method to prevent or treat a vascular indication, comprising: administering to a mammal in need of such therapy an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);
or a pharmaceutically acceptable salt thereof;
or a combination thereof;
wherein the indication is coronary artery disease, myocardial infarction, unstable angina pectoris, atherosclerosis or vasculitis.

8. A therapeutic method to prevent or treat low bone mineral density, comprising:

administering to a mammal in need of such therapy an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);

or a pharmaceutically acceptable salt thereof;
or a combination thereof.

9. A therapeutic method to prevent or treat an autoimmune disease, comprising: administering to a mammal in need of such therapy an effective amount of:

1) a compound of formula (XIII), (XIV), (X), (XI), (XIX), or (XII); or
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), or a compound of formula (XII), or a compound of formula (XIX);
or a pharmaceutically acceptable salt thereof;
or a combination thereof.

10. A method of suppressing tumor growth in a vertebrate animal, comprising: administering to said vertebrate an

effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);
or a pharmaceutically acceptable salt thereof;
or a combination thereof.

11. A method for preventing or treating psoriasis in a mammal, comprising:

administering to the mammal an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);
or a pharmaceutically acceptable salt thereof;
or a combination thereof.

12. A method to enhance wound healing, comprising:

administering an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);

or a pharmaceutically acceptable salt thereof;
or a combination thereof.

13. A method of treating a mammal afflicted with, or at risk of, an indication associated with chemokine-induced activity, comprising:

administering to the mammal an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);

or a pharmaceutically acceptable salt thereof;
or a combination thereof.

14. A therapeutic method to prevent or treat asthma, comprising:

administering to a mammal in need of such therapy an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);

or a pharmaceutically acceptable salt thereof;
or a combination thereof.

15. The method of item 1 wherein the amount inhibits a product or intermediate in the arachidonic acid pathway.

16. The method of item 15 wherein leukotriene is inhibited.

17. The method of item 15 wherein thromboxane is inhibited.

18. The method of item 15 wherein prostaglandin is inhibited.

19. A method of preventing or inhibiting an indication associated with elevated TNF-α, comprising: administering to a mammal afflicted with, or at risk of, the indication an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);
or a pharmaceutically acceptable salt thereof;
or a combination thereof.

20. A therapeutic method to prevent or treat organ transplant rejection, comprising:

administering to a mammal in need of such therapy an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);

or a pharmaceutically acceptable salt thereof;
or a combination thereof.

21. A therapeutic method to prevent or treat rheumatoid arthritis, comprising:

administering to a mammal in need of such therapy an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);

or a pharmaceutically acceptable salt thereof;
or a combination thereof.

22. A therapeutic method to prevent or treat allergy, comprising:

administering to a mammal in need of such therapy an effective amount of:

1) a compound of formula (XIII), (XIV), (X), (XI), (XIX), or (XII);
2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);

or a pharmaceutically acceptable salt thereof;
or a combination thereof.

23. A compound of formula (XIII), (XIV), (X), (XI), (XIX), or (XII), or a pharmaceutically acceptable salt thereof, that binds to the Duffy antigen of red blood cells with a decreased affinity relative to the corresponding chemokine.

24. A compound of formula (XIII), (XIV), (X), (XI), (XIX), or (XII), or a pharmaceutically acceptable salt thereof

25. A saccharide conjugate of : a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX); or a pharmaceutically acceptable salt thereof.

26. A compound of formula (XIII), (XIV), (X), (XI), (XIX), or (XII), or a pharmaceutically acceptable salt thereof, that binds to the Duffy antigen of red blood cells with a decreased affinity relative to the corresponding chemokine peptide or chemokine.

27. The saccharide conjugate of item 25 wherein the compound binds to the Duffy antigen of red blood cells with a decreased affinity relative to the corresponding chemokine peptide or chemokine.

28. A method to identify an agent that inhibits antigen-induced recall response, comprising:

a) administering to a mammal which is sensitized to an antigen an agent selected from 1) a compound of formula (XIII), (XIV), (X), (XI), (XIX), or (XII); and 2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), or a compound of formula (XII), or a compound of formula (XIX);
or a pharmaceutically acceptable salt thereof;
or a combination thereof; and
b) detecting or determining whether the agent inhibits the recall response.

29. A method of preventing or inhibiting a recall response to an antigen, comprising: administering to a mammal which is sensitized to the antigen an amount of an agent effective to inhibit or decrease IL-4 levels in the mammal; wherein the agent is selected from: 1) a compound of formula (XIII), (XIV), (X), (XI), (XIX), or (XII); and 2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);
or a pharmaceutically acceptable salt thereof;
or a combination thereof.

30. A method of preventing or inhibiting a recall response to an antigen, comprising: administering to a mammal which is sensitized to the antigen an amount of an agent effective to inhibit or decrease immunoglobulin levels in the mammal;
wherein the agent is selected from: 1) a compound of formula (XIII), (XIV), (X), (XI), (XIX), or (XII); and 2) a saccharide conjugate of a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), or a compound of formula (XIX);
or a pharmaceutically acceptable salt thereof;
or a combination thereof.

31. A method of suppressing the immune response of a mammal subjected to a therapy which employs an immunogenic therapeutic molecule, comprising:

administering to the mammal an amount of an agent effective to inhibit antigen-induced recall response to the immunogenic therapeutic molecule;
wherein the agent is selected from: 1) a compound of formula (XIII), (XIV), (X), (XI), (XIX), and (XII); and 2) a saccharide conjugate of a chemokine peptide 3, a variant thereof, a derivative thereof, a compound of formula (IV), a compound of formula (V), a compound of formula (VI), a compound of formula (XIII), a compound of

formula (XIV), a compound of formula (X), a compound of formula (XI), a compound of formula (XII), and a compound of formula (XIX);
or a pharmaceutically acceptable salt thereof;
or a combination thereof.

32. A method to increase the *in vivo* half-life of compound selected from a chemokine peptide 3, a variant thereof, a derivative thereof, a compound of formula (IV), a compound of formula (V), a compound of formula (VI), a compound of formula (XIII), a compound of formula (XIV), a compound of formula (X), a compound of formula (XI), a compound of formula (XII), and a compound of formula (XIX) comprising linking a saccharide to the compound.

33. The saccharide linked compound of item 33.

34. A method to increase or enhance a chemokine-associated inflammatory response in a mammal, comprising: administering to the mammal an amount of a compound of formula (XIII), (XIV), (X), (XI), (XIX), or (XII), or a combination thereof effective to increase or enhance said response.

35. A method to augment an immune response, comprising: administering to a mammal an immunogenic moiety and an amount of a compound of formula (XIII), (XIV), (X), (XI), (XIX), or (XII), or a combination thereof, wherein the amount is effective to augment the immune response of the mammal to the immunogenic moiety.

36. A therapeutic method to prevent or inhibit lentiviral infection or replication, comprising: administering to a mammal in need of such therapy an effective amount of a compound of formula (XIII), (XIV), (X), (XI), (XIX), or (XII), or a combination thereof.

37. The method of item 36 wherein the lentivirus is HIV.

38. The method of item 37 further comprising administering an antiviral agent before, during and/or after the administration of the compound or a combination.

39. A method of inhibiting a parasitic infection in a vertebrate animal, comprising: administering to the animal an effective amount of a compound of formula (XIII), (XIV), (X), (XI), (XIX), or (XII), or a combination thereof.

40. The method of item 39 wherein the animal is a human with malaria.

41. A method to increase or enhance hematopoietic cell-associated activity at a tumor site, comprising: administering an effective amount of a compound of formula (XIII), (XIV), (X), (XI), (XIX), or (XII), or a combination thereof.

42. An immunogenic composition comprising an immunogenic moiety and an amount of a compound of formula (XIII), (XIV), (X), (XI), (XIX), or (XII), or a combination thereof.

43. Use of a compound of formula (XIII), (XIV), (X), (XI), (XIX), or (XII), or a combination thereof for the manufacture of a medicament for the treatment of a pathological condition or symptom in a mammal which is associated with a chemokine-induced activity.

44. A pharmaceutical composition comprising a compound of formula (XIII), (XIV), (X), (XI), (XIX), or (XII), which binds to the Duffy antigen on red blood cells and a pharmaceutically acceptable carrier.

45. The pharmaceutical composition of item 44 wherein compound is associated with a biologically active molecule.

46. The pharmaceutical composition of item 45 wherein the biologically active molecule is a peptide, polypeptide or protein.

47. The pharmaceutical composition of item 46 wherein the biologically active molecule is human growth hormone or insulin.

48. The pharmaceutical composition of item 45 wherein the biologically active molecule is immunogenic.

49. The pharmaceutical composition of item 45 wherein the biologically active molecule when it is not associated

with the compound is rapidly cleared from the circulation.

50. The pharmaceutical composition of item 45 wherein the compound and the biologically active molecule are covalently bonded.

51. The pharmaceutical composition of item 45 wherein the compound is associated with a detectable molecule.

52. A method to modulate the pharmacokinetics of a biologically active molecule, comprising: associating a compound of formula (XIII), (XIV), (X), (XI), (XIX), or (XII), which binds to the Duffy antigen on red blood cells with the biologically active molecule, wherein the pharmacokinetic properties of the associated biologically active molecule are different than the pharmacokinetic properties of the biologically active molecule alone.

53. A method to alter the pharmacokinetics of a biologically active molecule in a mammal, comprising: administering to the mammal the biologically active molecule in association with a compound of formula (XIII), (XIV), (X), (XI), (XIX), or (XII), which binds to the Duffy antigen on red blood cells.

54. A compound of formula (XIII), (XIV), (X), (XI), (XIX), or (XII) that binds to the Duffy antigen of red blood cells with a decreased affinity relative to the corresponding chemokine.

55. A compound of formula (XIII), (XIV), (X), (XI), (XIX), or (XII) that binds to the Duffy antigen of red blood cells with a decreased affinity relative to the corresponding chemokine peptide or chemokine.

56. A moiety that binds to the Duffy antigen of red blood cells with at least about a ten-fold reduced affinity for the Duffy antigen relative to the binding of moiety to at least one chemokine receptor.

57. A moiety that binds to the Duffy antigen of red blood cells with at least about a ten-fold increased affinity for the Duffy antigen relative to the binding of the moiety to at least one chemokine receptor.

58. A method to identify a region of a chemokine receptor which binds to a chemokine peptide, a variant, derivative or analog thereof comprising:

(a) contacting a chemokine receptor with an amount of the chemokine peptide, a variant, derivative or analog thereof so as to result in a complex between the receptor and the chemokine peptide, a variant, derivative or analog thereof; and
(b) determining which region of the receptor is bound to the chemokine peptide, variant, derivative or analog thereof.

59. A method to identify a molecule which binds to a region of a chemokine receptor that is bound by a specific chemokine peptide, a variant, derivative or analog thereof, comprising:

(a) contacting the region with a population of molecules; and
(b) detecting or determining whether at least one molecule of the population of molecules specifically binds to the region.

60. A method to identify a molecule that binds to a chemokine receptor but which molecule does not form a heterodimer with at least one chemokine that binds to the receptor, comprising:

(a) contacting the chemokine receptor with the molecule so as to form a complex between the receptor and the molecule;
(b) contacting the complex with at least one chemokine; and
(c) detecting or determining whether the molecule in the complex forms a heterodimer with the chemokine.

61. A method to identify a molecule that binds to a chemokine receptor but which molecule does not form a heterodimer with a chemokine that binds to the receptor, comprising:

(a) contacting the chemokine receptor with the molecule and at least one chemokine; and
(b) detecting or determining whether the molecule forms a heterodimer with the chemokine.

62. A method to identify an agent which inhibits chemokine activity but does not compete with native chemokine for its receptor, comprising:

(a) contacting cells with the agent, wherein the cells comprise receptors that bind native chemokine; and
(b) detecting or determining whether the agent specifically binds to a receptor which is not the receptor which binds native chemokine and inhibits chemokine activity.

## Claims

**1.** A compound of formula (X) or (XIV):

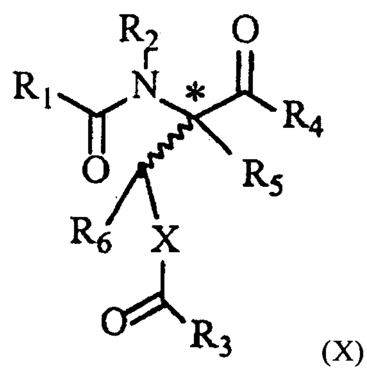

(X)

wherein
$R_1$ is $(C_1-C_{15})$alkyl, $(C_2-C_{15})$alkenyl, $(C_2-C_{15})$alkynyl, $(C_1-C_5)$alkoxy, aryl, heteroaryl, aryl$(C_1-C_6)$alkyl, heteroaryl $(C_1-C_6)$alkyl; and $R_2$ is hydrogen or $(C_1-C_{15})$alkyl; or $R_1$ and $R_2$ together with the atoms to which they are attached are a five or six membered heterocyclic ring comprising four or five carbon atoms, optionally substituted on carbon with oxo, and optionally substituted with a fused benzo group; $R_3$ is hydroxy, $(C_1-C_6)$alkoxy, or $N(R_a)(R_b)$; and $R_4$ is hydroxy, $(C_1-C_6)$alkoxy, or $N(R_a)(R_b)$; or $R_3$ and $R_4$ together with the atoms to which they are attached are a five or six membered heterocyclic ring comprising four or five carbon atoms and $N(R_c)$; $R_5$ is hydrogen or $(C_1-C_6)$alkyl; $R_6$ is hydrogen or oxo; X is a direct bond, N(H), or methylene ($-CH_2-$); each $R_a$ and $R_b$ is independently hydrogen, $(C_1-C_6)$alkyl, $(C_1-C_6)$alkanoyl, $(C_1-C_6)$alkoxycarbonyl, aryl, aryl$(C_1-C_6)$alkyl, aryl$(C_1-C_6)$alkoxy, aryl$(C_1-C_6)$alkanoyl, aryl$(C_1-C_6)$alkoxycarbonyl, heteroaryl, heteroaryl$(C_1-C_6)$alkyl, heteroaryl$(C_1-C_6)$alkoxy, heteroaryl$(C_1-C_6)$alkanoyl, or heteroaryl$(C_1-C_6)$alkoxycarbonyl; and $R_c$ is hydrogen or $(C_1-C_6)$alkyl; wherein any $(C_1-C_{15})$alkyl, $(C_2-C_{15})$alkenyl, $(C_2-C_{15})$alkynyl, or $(C)-C_{15})$alkoxy of $R_1$ is optionally substituted with one or more substituents selected from the group consisting of halo, cyano, nitro, carboxy, trifluoromethyl, trifluoromethoxy, $(C_1-C_6)$alkyl, $(C_1-C_6)$alkoxy, $(C_1-C_6)$ alkanoyl, $(C_1-C_6)$alkanoyloxy, $(C_1-C_6)$alkoxycarbonyl, hydroxy, oxo, and $N(R_a)(R_b)$; and wherein any aryl, heteroaryl, or benzo of $R_1$, $R_a$, and $R_b$, is optionally substituted with 1, 2, 3, or 4 substituents independently selected from the group consisting of halo, cyano, hydroxy, nitro, trifluoromethyl, trifluoromethoxy, $(C_1-C_6)$alkyl, $(C_1-C_6)$alkoxy, $(C_1-C_6)$ alkanoyl, $(C_1-C_6)$alkanoyloxy, $(C_1-C_6)$alkoxycarbonyl, sulfino ($SO_2H$), sulfo ($SO_3H$), and methylenedioxy;

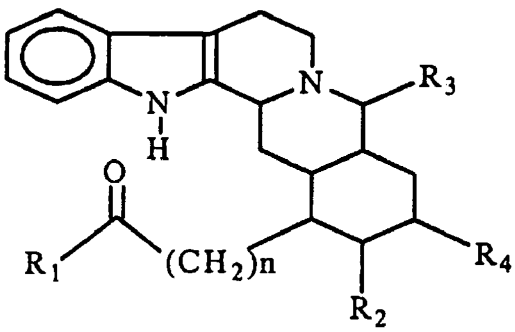

(XIV)

wherein

$R_1$ is O(Ra) wherein Ra is H, $(C_1\text{-}C_6)$alkyl, $(C_1\text{-}C_6)$alkanoyl, $(C_1\text{-}C_6)$alkanoyloxy, $(C_6\text{-}C_{10})$aryl or $(C_6\text{-}C_{10})$heteroaryl; or N(Rb)(Rc) wherein each Rb and Rc is independently H or $(C_1\text{-}C_6)$alkyl; $R_2$ is O(Ra) wherein Ra is H, $(C_1\text{-}C_6)$ alkyl, $(C_1\text{-}C_6)$alkanoyl, $(C_1\text{-}C_6)$alkanoyloxy, $(C_6\text{-}C_{10})$aryl or $(C_6\text{-}C_{10})$heteroaryl; or N(Rb)(Rc) wherein each Rb and Rc is independently H or $(C_1\text{-}C_6)$alkyl; $R_3$ is H, C(=O) or C(=S); $R_4$ is H, C(=O), C(=S), O(Ra), or N(Rb)(Rc); each Ra is H, $(C_1\text{-}C_6)$alkyl, $(C_1\text{-}C_6)$alkanoyloxy, $(C_1\text{-}C_6)$alkanoyl, $(C_6\text{-}C_{10})$aryl or $(C_6\text{-}C_{10})$heteroaryl; each Rb and Rc is independently H or $(C_1\text{-}C_6)$alkyl; and n is an integer between 0 and 6, inclusive; wherein any $(C_6\text{-}C_{10})$aryl, $(C_1\text{-}C_6)$ alkyl, $(C_1\text{-}C_6)$alkanoyloxy, $(C_6\text{-}C_{10})$heteroaryl or $(C_1\text{-}C_6)$alkanoyl or the benzo ring in formula (XIV) is optionally substituted with at least one substituent (e. g. 1, 2, 3, or 4) selected from the group consisting of halo, cyano, nitro, trifluoromethyl, trifluoromethoxy, $(C_1\text{-}C_6)$alkyl, $(C_1\text{-}C_6)$alkoxy, $(C_1\text{-}C_6)$alkanoyl, $(C_1\text{-}C_6)$alkanoyloxy, $(C_1\text{-}C_6)$alkoxy-carbonyl, methoxydioxy, hydroxy, C (=O), sulfino ($SO_2H$) sulfo ($SO_3H$), and N(Rb)(Rc);

or alternatively wherein

$R_1$ is $O(R_a)$, an amino acid, or $N(R_b)(R_c)$; $R_2$ is $O(R_a)$ or $N(R_b)(R_c)$; $R_3$ is H, oxo or thioxo; $R_4$ is H, oxo, thioxo, O $(R_a)$ or $N(R_b)(R_c)$; n is 0, 1, 2, 3, 4, 5 or 6; each $R_a$ is independently hydrogen, $(C_1\text{-}C_6)$alkyl, $(C_1\text{-}C_6)$alkanoyl, aryl, heteroaryl, or a saccharide; $R_b$ and $R_c$ are each independently H or $(C_1\text{-}C_6)$alkyl; wherein any $(C_1\text{-}C_6)$alkyl, $(C_1\text{-}C_6)$ alkanoyl, $(C_1\text{-}C_6)$alkanoyloxy, aryl or heteroaryl is optionally substituted with one or more (e. g. 1, 2, 3, or 4) substituents selected from the group consisting of halo, cyano, nitro, trifluoromethyl, trifluoromethoxy, $(C_1\text{-}C_6)$alkyl, $(C_1\text{-}C_6)$alkoxy, $(C_1\text{-}C_6)$alkanoyl, $(C_1\text{-}C_6)$alkanoyloxy, $(C_1\text{-}C_6)$alkoxycarbonyl, hydroxy, oxo, sulfino ($SO_2H$) sulfo ($SO_3H$), and $N(R_b)(R_c)$;

or a pharmaceutically acceptable salt thereof.

**2.** A compound of formula (X) or (XIV) as defined in claim 1, or a pharmaceutically acceptable salt thereof, that binds to the Duffy antigen of red blood cells with a decreased affinity relative to the corresponding chemokine peptide or chemokine.

**3.** A pharmaceutical composition comprising a compound of formula (X) or (XIV) as defined in claim 1, which binds to the Duffy antigen on red blood cells, and a pharmaceutically acceptable carrier.

**4.** The pharmaceutical composition of claim 3, wherein the compound is associated with a biologically active molecule such as a peptide, polypeptide, protein, for example human growth hormone or insulin.

**5.** The pharmaceutical composition of claim 4, wherein the biologically active molecule is immunogenic.

**6.** The pharmaceutical composition of claim 4, wherein the biologically active molecule when it is not associated with the compound is rapidly cleared from the circulation.

**7.** The pharmaceutical composition of claim 4, wherein the compound and the biologically active molecule are covalently bonded.

**8.** The pharmaceutical composition of claim 4, wherein the compound is associated with a detectable molecule.

**9.** A saccharide conjugate of: a chemokine peptide 3, a variant thereof, a derivative thereof, a compound of formula (X) or (XIV) as defined in claim 1; or a pharmaceutically acceptable salt thereof.

**10.** The saccharide conjugate of claim 9, wherein the compound binds to the Duffy antigen of red blood cells with a decreased affinity relative to the corresponding chemokine peptide or chemokine.

**11.** The compound of either of claim 1 or claim 2, or the saccharide conjugate of either of claim 9 or claim 10, or a pharmaceutically acceptable salt of either, or a combination thereof, for use in any of the group of methods consisting of: a method of preventing or inhibiting an indication associated with a chemokine-induced activity (for example, a product or intermediate in the arachidonic acid pathway, such as inhibiting leukotriene, thromboxane or prostaglandin); a method to inhibit the activity of more than one chemokine; a method of preventing or inhibiting an indication associated with hematopoietic cell recruitment; a method of preventing or inhibiting an indication associated with histamine release from basophils or mast cells; a method to modulate the chemokine-induced activity of hematopoietic cells at a preselected physiological site; a method to prevent or inhibit stroke; a therapeutic method to prevent or treat coronary artery disease, myocardial infarction, unstable angina pectoris, atherosclerosis or vasculitis; a therapeutic method to prevent or treat low bone mineral density; a therapeutic method to prevent or treat an autoimmune disease; a method of suppressing tumor growth in a vertebrate animal; a method for preventing or treating psoriasis in a mammal; a method to enhance wound healing; a method of treating a mammal afflicted with, or at risk of, an indication associated with chemokine-induced activity; a therapeutic method to prevent or treat asthma; a method of preventing or inhibiting an indication associated with elevated TNF-$\alpha$; a therapeutic method to prevent or treat organ transplant rejection; a therapeutic method to prevent or treat rheumatoid arthritis; a therapeutic method to prevent or treat allergy; a method to identify an agent that inhibits antigen-induced recall response in a mammal which is sensitized to an antigen; a method of preventing or inhibiting a recall response to an antigen in a mammal which is sensitized to the antigen; a method of preventing or inhibiting a recall response to an antigen in a mammal which is sensitized to the antigen; and a method of suppressing the immune response of a mammal subjected to a therapy which employs an immunogenic therapeutic molecule.

**12.** Use of the compound of either of claim 1 or claim 2, or the saccharide conjugate of either of claim 9 or claim 10, or a pharmaceutically acceptable salt of either, or a combination thereof, in the preparation for a medicament for any of the group of methods consisting of: a method of preventing or inhibiting an indication associated with a chemokine-induced activity (for example, a product or intermediate in the arachidonic acid pathway, such as inhibiting leukotriene, thromboxane or prostaglandin); a method to inhibit the activity of more than one chemokine; a method of preventing or inhibiting an indication associated with hematopoietic cell recruitment; a method of preventing or inhibiting an indication associated with histamine release from basophils or mast cells; a method to modulate the chemokine-induced activity of hematopoietic cells at a preselected physiological site; a method to prevent or inhibit stroke; a therapeutic method to prevent or treat coronary artery disease, myocardial infarction, unstable angina pectoris, atherosclerosis or vasculitis; a therapeutic method to prevent or treat low bone mineral density; a therapeutic method to prevent or treat an autoimmune disease; a method of suppressing tumor growth in a vertebrate animal; a method for preventing or treating psoriasis in a mammal; a method to enhance wound healing; a method of treating a mammal afflicted with, or at risk of, an indication associated with chemokine-induced activity; a therapeutic method to prevent or treat asthma; a method of preventing or inhibiting an indication associated with elevated TNF-$\alpha$; a therapeutic method to prevent or treat organ transplant rejection; a therapeutic method to prevent or treat rheumatoid arthritis; a therapeutic method to prevent or treat allergy; a method to identify an agent that inhibits antigen-induced recall response in a mammal which is sensitized to an antigen; a method of preventing or inhibiting a recall response to an antigen in a mammal which is sensitized to the antigen; a method of preventing or inhibiting a recall response to an antigen in a mammal which is sensitized to the antigen; and a method of suppressing the immune response of a mammal subjected to a therapy which employs an immunogenic therapeutic molecule.

**13.** A method to increase the *in vivo* half-life of compound selected from a compound of formula (X) or (XIV), comprising linking a saccharide to the compound.

**14.** The compound of either of claim 1 or claim 2, or a combination thereof, for use in any of the group of methods consisting of: a method to increase or enhance a chemokine-associated inflammatory response in a mammal; a method to augment an immune response; a therapeutic method to prevent or inhibit lentiviral infection or replication (for example, HIV infection or replication), optionally in combination with an antiviral agent; a method of inhibiting a parasitic infection in a vertebrate animal, such as a human with malaria; a method to increase or enhance hematopoietic cell-associated activity at a tumor site; and treatment of a pathological condition or symptom in a mammal which is associated with a chemokine-induced activity.

**15.** Use of the compound of either of claim 1 or claim 2, or a combination thereof, in the preparation of a medicament for any of the group of methods consisting of: a method to increase or enhance a chemokine-associated inflammatory

response in a mammal; a method to augment an immune response; a therapeutic method to prevent or inhibit lentiviral infection or replication (for example, HIV infection or replication), optionally in combination with an antiviral agent; a method of inhibiting a parasitic infection in a vertebrate animal, such as a human with malaria; a method to increase or enhance hematopoietic cell-associated activity at a tumor site; and treatment of a pathological condition or symptom in a mammal which is associated with a chemokine-induced activity;.

**16.** An immunogenic composition comprising an immunogenic moiety and an amount of a compound of formula (X) or (XIV) as defined in either of claim 1 or claim 2, or a combination thereof.

**17.** A compound of formula (X) or (XIV) as defined in claim 1, for use in a method to modulate the pharmacokinetics of a biologically active molecule, for example in a mammal, wherein the compound binds to the Duffy antigen on red blood cells with the biologically active molecule, and wherein the method comprises associating the compound with the biologically active molecule wherein the pharmacokinetic properties of the associated biologically active molecule are different than the pharmacokinetic properties of the biologically active molecule alone.

**18.** Use of a compound of formula (X) or (XIV) as defined in claim 1, in the preparation of a medicament for modulating the pharmacokinetics of a biologically active molecule, for example in a mammal, wherein the compound binds to the Duffy antigen on red blood cells with the biologically active molecule, and wherein the modulation comprises associating the compound with the biologically active molecule wherein the pharmacokinetic properties of the associated biologically active molecule are different than the pharmacokinetic properties of the biologically active molecule alone.

TOP COMPARTMENT

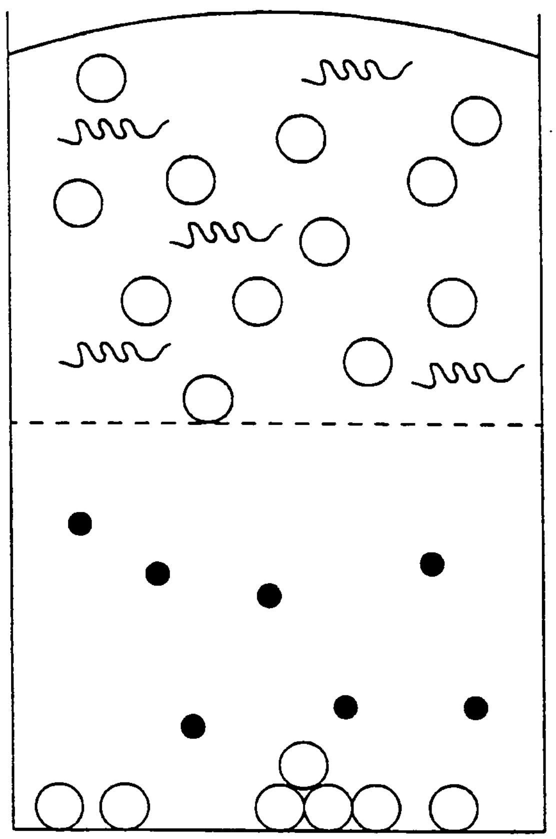

LOWER COMPARTMENT

FIG. 1

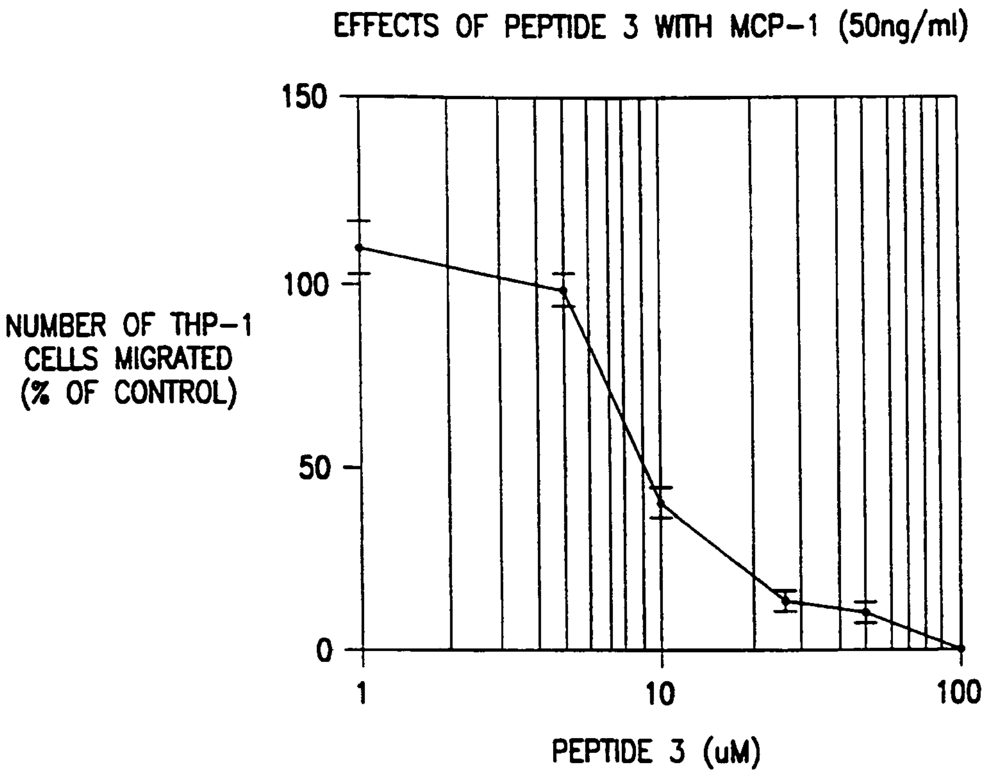
EFFECTS OF PEPTIDE 3 WITH MCP-1 (50ng/ml)
150
100
50
0
NUMBER OF THP-1 CELLS MIGRATED (% OF CONTROL)
1
10
100
PEPTIDE 3 (uM)

FIG. 2

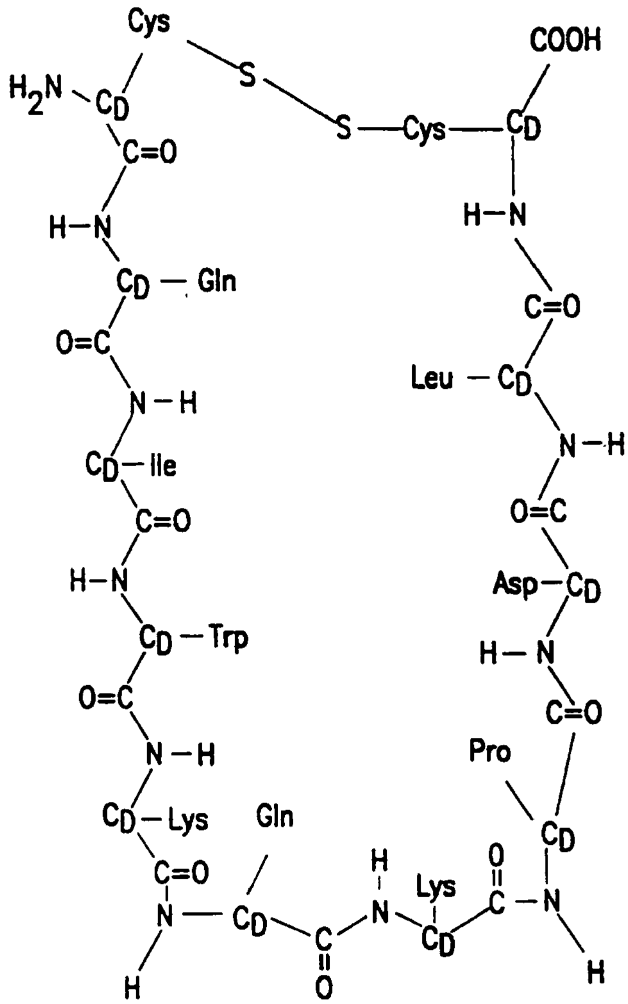

FIG. 3

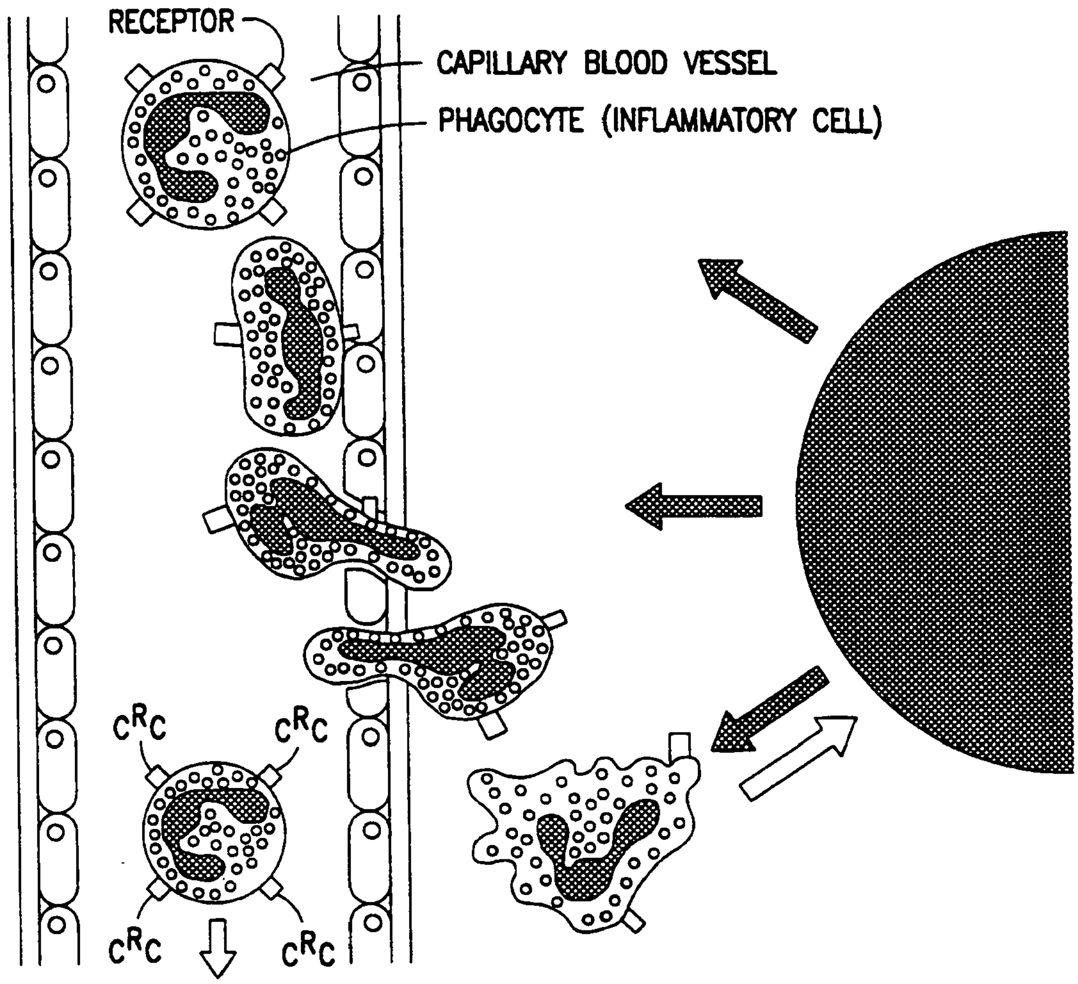
RECEPTOR
CAPILLARY BLOOD VESSEL
PHAGOCYTE (INFLAMMATORY CELL)
C^R C
C^R C
C^R C
C^R C

FIG. 4

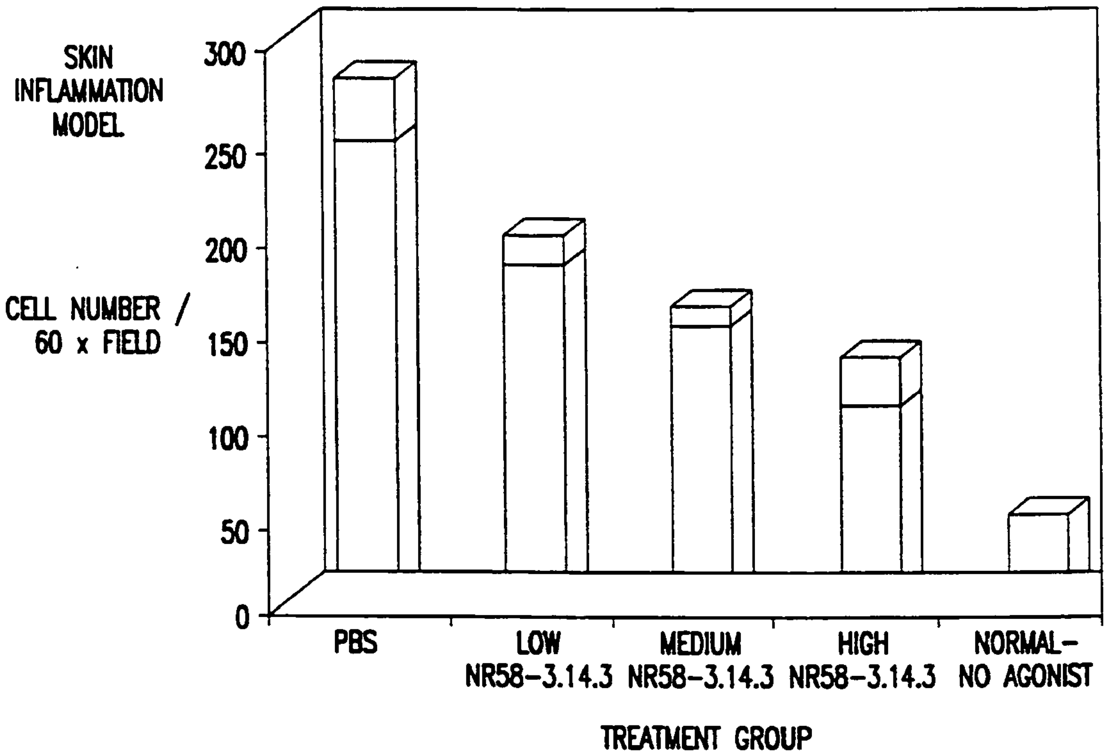
SKIN INFLAMMATION MODEL
CELL NUMBER / 60 x FIELD
300
250
200
150
100
50
0
PBS
LOW NR58-3.14.3
MEDIUM NR58-3.14.3
HIGH NR58-3.14.3
NORMAL- NO AGONIST
TREATMENT GROUP

FIG. 5A

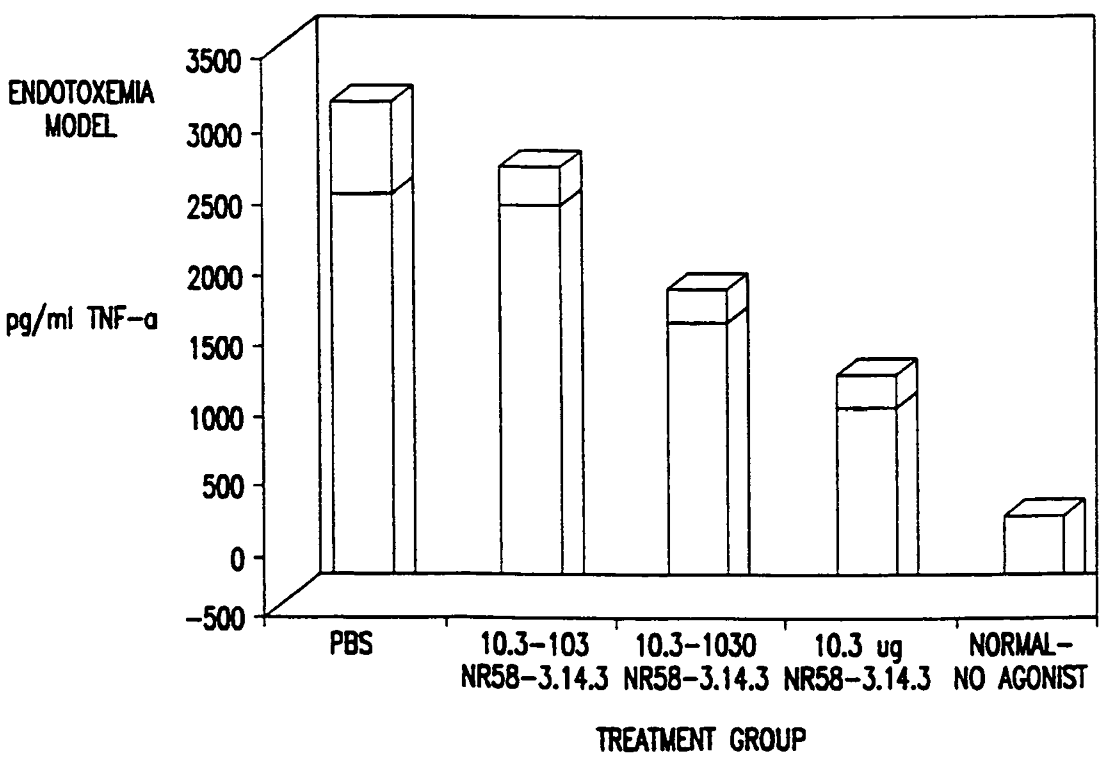
ENDOTOXEMIA MODEL
pg/ml TNF-a
3500
3000
2500
2000
1500
1000
500
0
-500
PBS
10.3-103 NR58-3.14.3
10.3-1030 NR58-3.14.3
10.3 ug NR58-3.14.3
NORMAL- NO AGONIST
TREATMENT GROUP

FIG. 5B

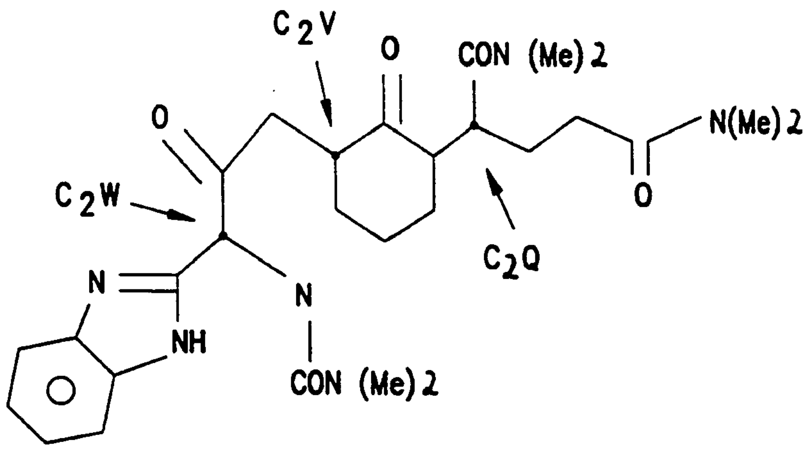

FIG. 6

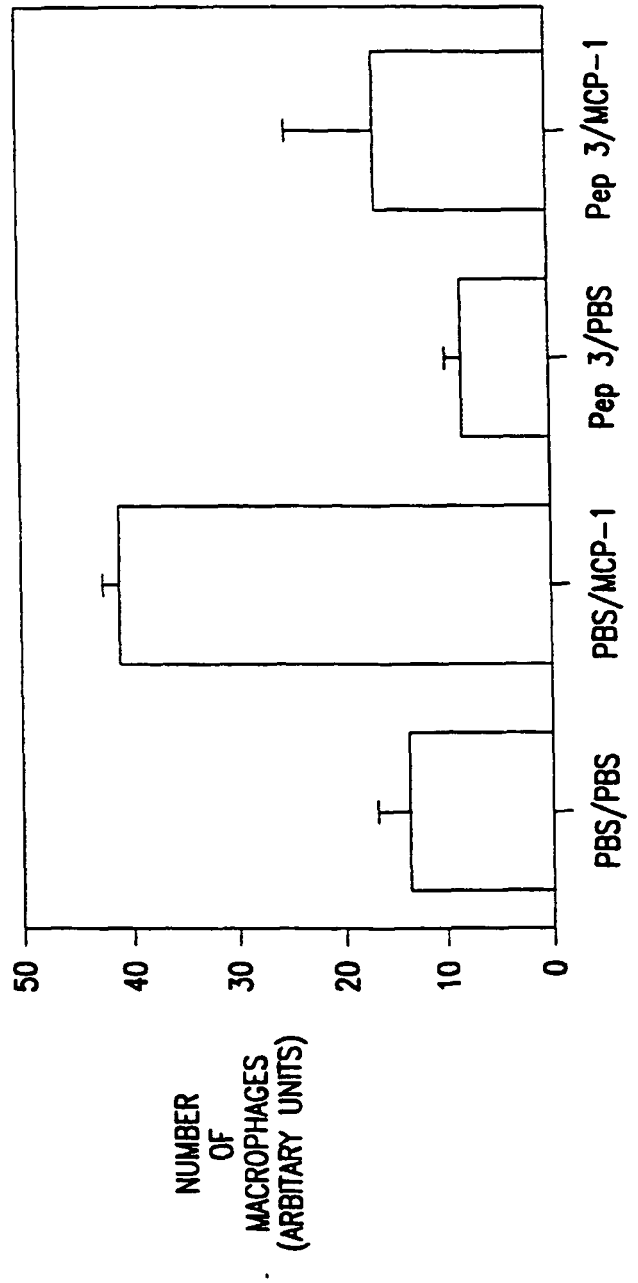
NUMBER OF MACROPHAGES (ARBITARY UNITS)
50
40
30
20
10
0
PBS/PBS
PBS/MCP-1
Pep 3/PBS
Pep 3/MCP-1

FIG. 7A

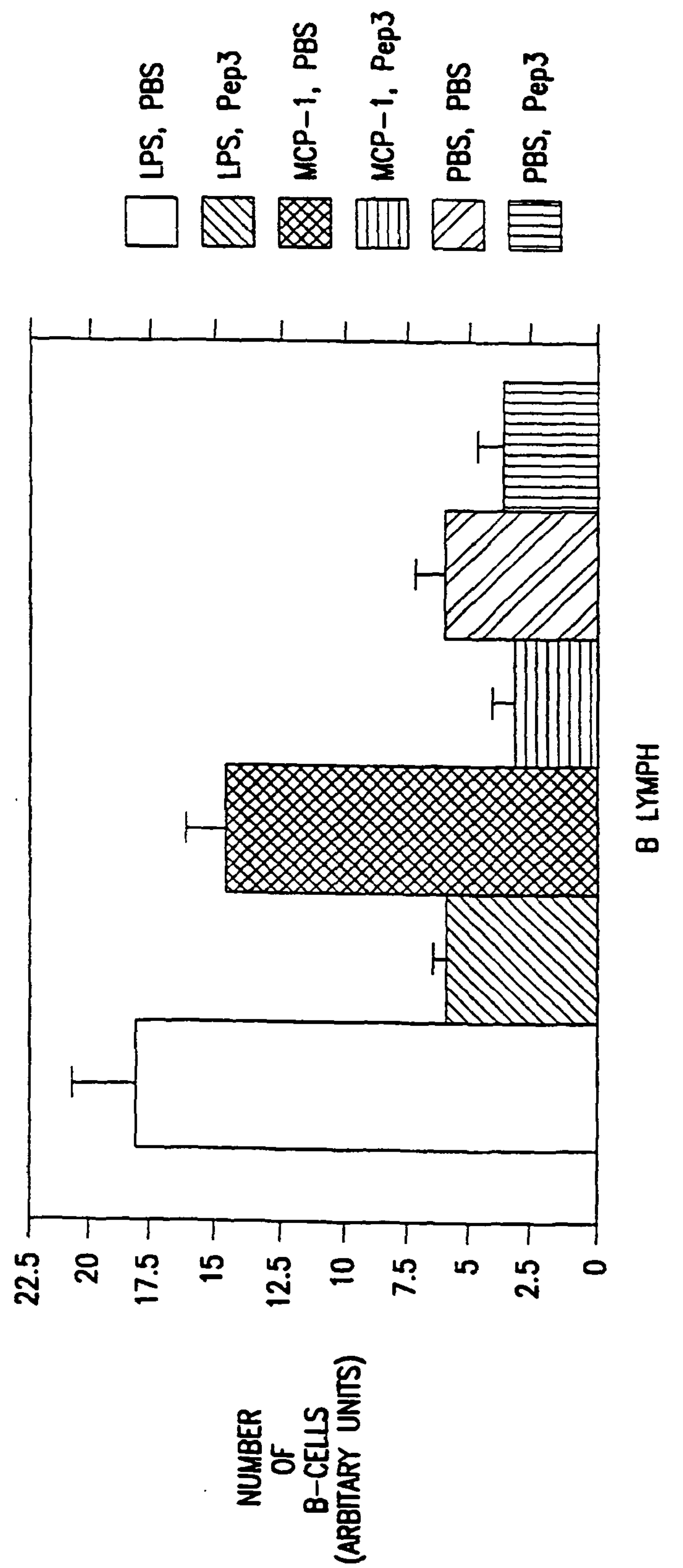
NUMBER
OF
B-CELLS
(ARBITARY UNITS)
22.5
20
17.5
15
12.5
10
7.5
5
2.5
0
B LYMPH
LPS, PBS
LPS, Pep3
MCP-1, PBS
MCP-1, Pep3
PBS, PBS
PBS, Pep3

FIG. 7B

| Amino Acid | Codon |
|---|---|
| Phe | UUU, UUC |
| Ser | UCU, UCC, UCA, UCG, AGU, AGC |
| Tyr | UAU, UAC |
| Cys | UGU, UGC |
| Leu | UUA, UUG, CUU, CUC, CUA, CUG |
| Trp | UGG |
| Pro | CCU, CCC, CCA, CCG |
| His | CAU, CAC |
| Arg | CGU, CGC, CGA, CGG, AGA, AGG |
| Gln | CAA, CAG |
| Ile | AUU, AUC, AUA |
| Thr | ACU, ACC, ACA, ACG |
| Asn | AAU, AAC |
| Lys | AAA, AAG |
| Met | AUG |
| Val | GUU, GUC, GUA, GUG |
| Ala | GCU, GCC, GCA, GCG |
| Asp | GAU, GAC |
| Gly | GGU, GGC, GGA, GGG |
| Glu | GAA, GAG |

FIG. 8

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro | pro |
| His (H) | asn; gln; lys; arg | arg |
| Ile (I) | leu; val; met; ala; phe norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala | leu |
| Pro (P) | gly | gly |
| Ser (S) | thr | thr |
| Thr (T) | ser | ser |
| Trp (W) | tyr | tyr |
| Tyr (Y) | trp; phe; thr; ser | phe |
| Val (V) | ile; leu; met; phe; ala; norleucine | leu |

FIG. 9

**Peptide 3**

LFL peptide 3(1-12)[MCP-1]: Residues 50-61 of mature hMCP-1
E-I-C-A-D-P-K-Q-K-W-V-Q
L amino acids

LFL peptide 3(3-12)[MCPI] Residues 52-61 of mature hMCP-1
C-A-D-P-K-Q-K-W-V-Q
L amino acids

LFL peptide 3(1-6)[MCP1]: Residues 50-55 of mature hMCP-1
E-I-C-A-D-P
L amino acids

LFL peptide 3(7-12)[MCP1]: Residues 56-61 of mature hMCP-1
K-Q-K-W-V-Q
L amino acids

LFL $Leu_4$peptide3(1-12)[MCP-1]
E-I-C-L-D-P-K-Q-K-W-V-Q
L amino acids

LFL $Ser_7$peptide3(1-12)[MCP-1]
E-I-C-A-D-P-S-Q-K-W-V-Q
L amino acids

LFL $Ile_{11}$peptide3(1-12)[MCP-1]
E-I-C-A-D-P-K-Q-K-W-I-Q
L amino acids

LFL $Leu_4Ile_{11}$peptide3(1-12)[MCP-1]
E-I-C-L-D-P-K-Q-K-W-I-Q
L amino acids

CFL $Cys_0Leu_4Ile_{11}Cys_{13}$peptide3(1-12)[MCP-1]
C-E-I-C-L-D-P-K-Q-K-W-I-Q-C
L amino acids

LRD $Leu_4Ile_{11}$ peptide 3(1-12)[MCP-1]
q-i-w-k-q-k-p-d-l-c-i-e
D amino acids

FIG. 10

CRD $Cys_0Leu_4Ile_{11}Cys_{13}$peptide 3(1-12)[MCP-1]
c-q-i-w-k-q-k-p-d-l-c-i-e-c
D amino acids

LFL $Ser_7Glu_8Glu_9$peptide3(1-12)[MCP1):Residues 50-61 of mature hMIP1α
E-I-C-A-D-P-S-E-E-W-V-Q
L amino acids

LFL peptide3(10-12)[MCP-1]
W-V-Q
L amino acids

CFL $Cys_0Cys_4$ peptide3(10-12)[MCP-1]
C-W-V-Q-C
L amino acids

LRD peptide3(10-12)[MCP-1]
q-v-w
D amino acids

LFL peptide3(7-9)[MCP-1]
K-Q-K
L amino acids

LRD peptide3(7-9)[MCP-1]
k-q-k
D amino acids

LFL peptide 3(7-9)[MIP1α](MIP1α specific inhibitor)
S-E-E
L amino acids

LRD peptide3(7-9)[MIP1α] (MIP1α specific inhibitor)
e-e-s
D amino acids

LFL peptide3(7-9)[IL-8](IL-8 specific inhibitor)
K-E-N
L amino acids

LRD peptide3(7-9)[IL-8](IL-8 specific inhibitor)
n-e-k
D amino acids

FIG. 10 CONTINUED

LFL peptide3(7-9)[SDF-1α](SDF-1α specific inhibitor)
K-L-K
L amino acids

LRD peptide3(7-9)[SDF1α] (SDF-1α specific inhibitor)
k-l-k
D amino acids

LFL $Leu_4Ile_{11}Cys_{13}$ peptide3(3-12)[MCP-1]
L-D-P-K-Q-K-W-I-Q-C
L amino acids

CRD $Leu_4Ile_{11}Cys_{13}$ peptide3(3-12)[MCP-1]
c-q-i-w-k-q-k-p-d-l-c
D amino acids

$^3$H-Ala CRD-$Leu_4Ile_{11}$ $Cys_{13}$ peptide 3(3-12)[MCP-1](D-Ala attached to Asp residue of CRD-$Leu_4Ile_{11}Cys_{13}$ peptide 3(3-12)[MCP-1])

$^3$H-L-Leu LRD $Cys_{13}$ peptide3(3-12)[MCP-1]
c-q-i-w-k-q-k-p-d-L-c
D and L amino acids

LFL SES
S-E-S
L amino acids

LFL KKK
K-K-K
L amino acids

LFL $Cys_4$ peptide3(10-12)[MCP-1]
W-V-Q-C
L amino acids

LRD $Cys_4$ peptide3(10-12)[MCP-1]
c-q-v-w
D amino acids

LFL $Ile_{11}Cys_{13}$peptide3(10-12)[MCP-1]
W-I-Q-C
L amino acids

FIG. 10 CONTINUED

LRD $Cys_{13}Ile_{11}$peptide3(10-12)[MCP-1]
cqiw
D amino acids

LRD peptide3(7-12)[MCP-1]
q-v-w-k-q-k
D amino acids

CFL $Cys_0Cys_{13}$peptide3(7-12)[MCP-1]
C-K-Q-K-W-V-Q-C
L amino acids

CRD $Cys_0Cys_{13}$peptide3(7-12)[MCP-1]
c-q-v-w-k-q-k-c
D amino acids

LFL peptide3(10-12)[RANTES]
WVR
L amino acids

LRD peptide3(10-12)[RANTES]
rvw
D amino acids

LFL peptide3(10-12)[SDF-1]
W-I-Q
L amino acids

FIG. 10 CONTINUED

| Sequence | Mol Wt. | | Duffy Binding ED-50 | MCP-1 ED-50 | MIP-1α ED-50 | RANTES ED-50 | SDF-1α ED-50 | IL-8 ED-50 | Other Data |
|---|---|---|---|---|---|---|---|---|---|
| AQPDAINAPVTCC | 1302 | | 90μM | ns | ns | - | ns | ns | |
| SYRRITSSKCPKEAV | 1725 | | 100nM | ns | ns | - | ns | - | |
| vaekpckastirrys | 1725 | | 18μM | ns | ns | - | ns | - | |
| HLKILNTPNCALQIV | 1677.3 | | 19μM | 10μM | 40μM | - | 7μM | - | |
| DYFETSSQCSKPGV | 1549 | | >100μM | ns | ns | - | ns | - | |
| vgpkscqestefyd | 1549 | | >100μM | ns | ns | - | ns | - | |
| SYRRITSSKC | 1097.4 | | 22μM | ns | ns | - | ns | - | |
| CPKEAV | 645.8 | | >100μM | ns | ns | - | ns | - | |
| SYRRI | 693.9 | | >100μM | ns | ns | - | ns | - | |
| TSSKC | 525.7 | | >100μM | ns | ns | - | ns | - | |
| DYFETSSQC | 1079.2 | | >100μM | ns | ns | - | ns | - | |
| CSKPGV | 589.8 | | >100μM | ns | ns | - | ns | - | |

FIG. 11

| | | | | | | | | | |
|---|---|---|---|---|---|---|---|---|---|
| BICADPKQKWVQ | 1445 | | 6µM | 8µM | 7.5µM | - | 13.5µM | 10µM | |
| CADPKQKWVQ | 1202 | | - | 8µM | 6.5µM | - | 9µM | 8.5µM | |
| cqvwkqkpdac | 1305 | | 3µM | 100nM | - | - | - | - | |
| cqvwkqkpdac | 1305 | | 40µM | 30nM | - | - | - | - | |
| BICADP | 647 | | - | 25µM | 20µM | - | 18.5µM | 16µM | |
| KQKWVQ | 816 | | 15µM | 7µM | 5µM | - | 5.5µM | 5µM | |
| BICLDPKQKWVQ | 1487 | | - | 8µM | 7µM | - | 2.5µM | 3µM | |
| BICADPSQKWVQ | 1404 | | 25µM | 7µM | 5.5µM | - | 4µM | 3µM | |
| BICADPKQKWIQ | 1459 | | - | 5.5µM | 35µM | - | 7µM | 2µM | |
| BICLDPKQKWIQ | 1501 | | 90µM | 2µM | 2µM | - | 4µM | 3.5µM | |
| WVQ | 431.5 | | 1µM | 8µM | 7.5µM | 1.5µM | 2.25µM | 1µM | |
| KQK | 464.5 | | 50µM | 7µM | >100µM | >100µM | >100µM | >100µM | |
| SBB | 399.4 | | >100µM | >100µM | - | >100µM | >100µM | >100µM | |
| KEN | 425.4 | | >100µM | >100µM | >100µM | >100µM | >100µM | | |

FIG. 11 CONTINUED

| | | | | | | | | | |
|---|---|---|---|---|---|---|---|---|---|
| KLK | 516.6 | | >100µM | >100µM | >100µM | >100µM | - | >100µM | |
| cqiwkqkpdlc | 1359 | | >100µM | 1nM | - | - | 350nM | 10nM | Note 1 |
| cqiwkqkpalc | 1448 | | - | >100µM | - | - | - | - | Note 2 |
| cqiwkqkpdlc | 1472.2 | | - | 10nM | - | - | - | - | |
| ses | 357.3 | | >100µM | >100µM | - | - | - | - | |
| kkk | 609.8 | | >100µM | - | - | - | - | - | |

NOTE 1: In vivo effect-abolishes macrophages in an in vivo intradermal study by 500ng MCP-1. 300 ug iv and 10mg sq 30 minutes prior to MCP-1; D-ala ("a") is attached to D-asp ("d").

NOTE 2: In same study as note 1 above, no effect on macrophages seen.

FIG. 11 CONTINUED

STUDY DESIGN TABLE

| GROUP | ANIMAL# | N | RX | RX DOSE/ROUTE T=30 MIN | DERMAL AGONIST | DERMAL AGONIST DOSE (ng IN 50 ul) T=0 | HOUR OF SACRIFICE |
|---|---|---|---|---|---|---|---|
| 1 | 1,2,3 | 3 | PBS | 200 ul:LV<br>200 ul:SQ BACK | PBS<br>LPS<br>MCP-1<br>MCP-1 | 0<br>50<br>100<br>500 | 20-24 |
| 2 | 4,5,6 | 3 | NR58-3.14.3 | 3 ug:LV<br>100 ug:SQ BACK | PBS<br>LPS<br>MCP-1<br>MCP-1 | 0<br>50<br>100<br>500 | 20-24 |
| 3 | 7,8,9 | 3 | NR58-3.14.3 | 30 ug:LV<br>1 mg:SQ BACK | PBS<br>LPS<br>MCP-1<br>MCP-1 | 0<br>50<br>100<br>500 | 20-24 |
| 4 | 10,11,12 | 3 | NR58-3.14.3 | 300 ug:LV<br>10 mg:SQ BACK | PBS<br>LPS<br>MCP-1<br>MCP-1 | 0<br>50<br>100<br>500 | 20-24 |

FIG. 12

CYCLISED REVERSE -D-
DERIVATIVES OF PEPTIDE 3

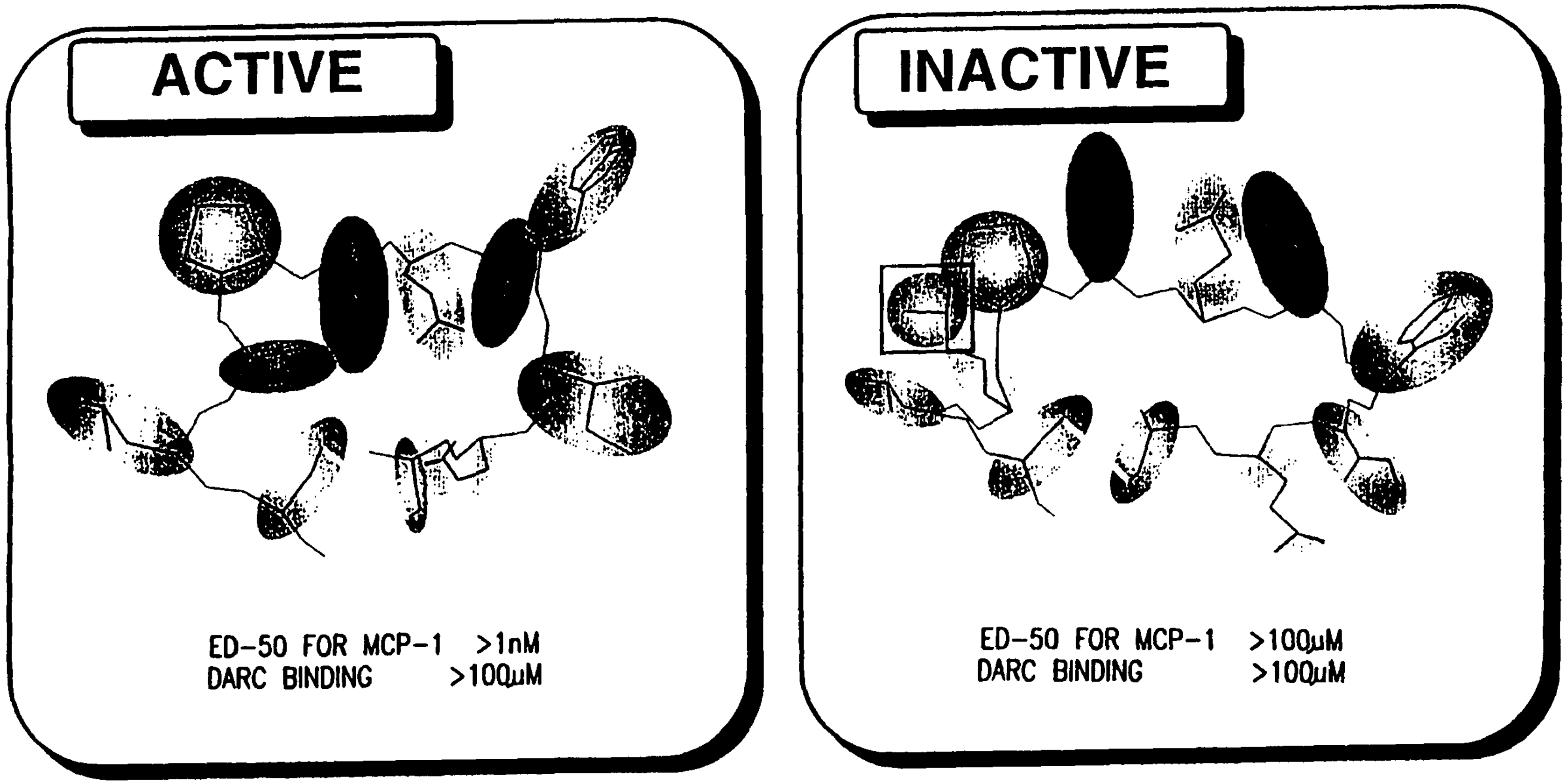


FIG. 13

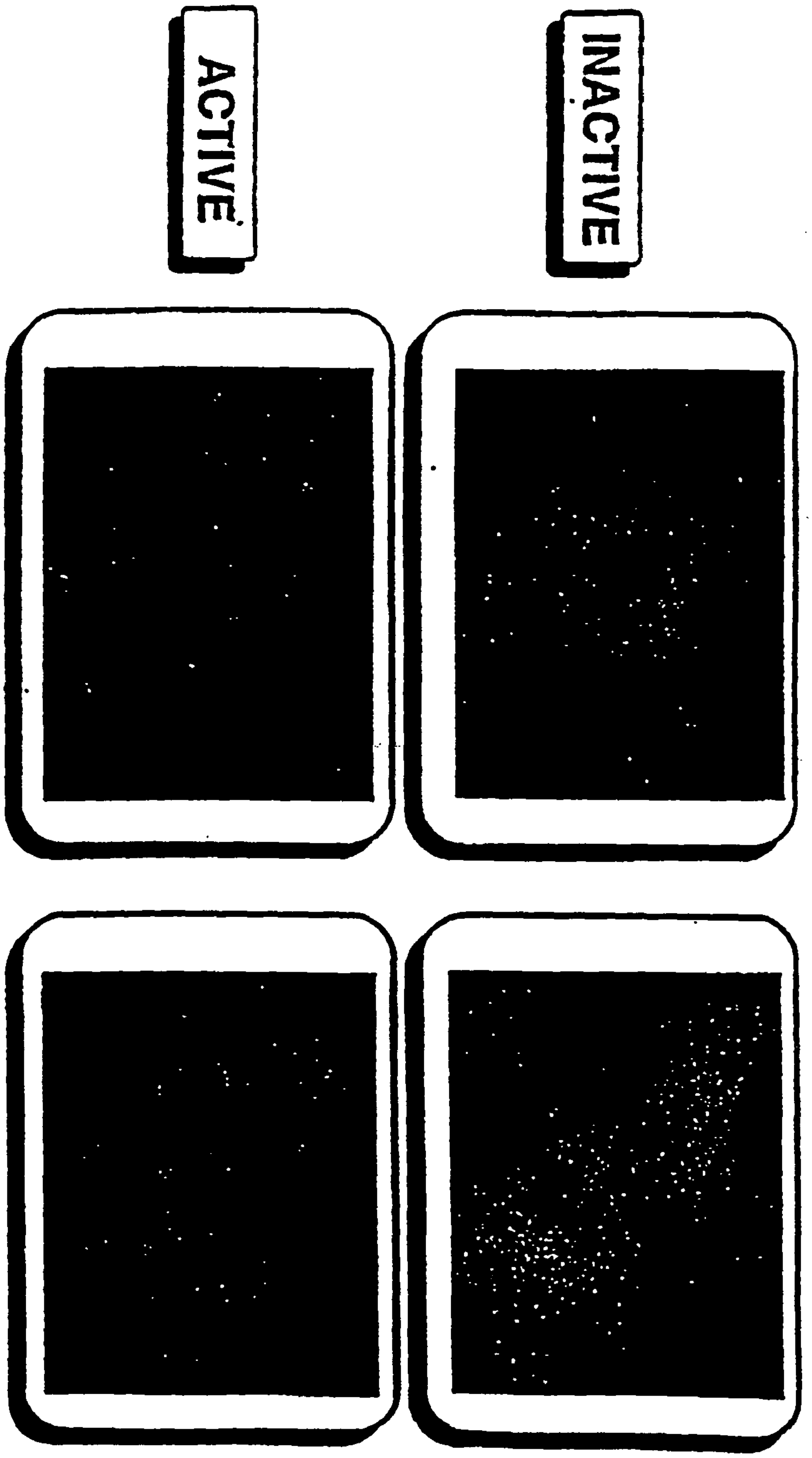


Fig 14

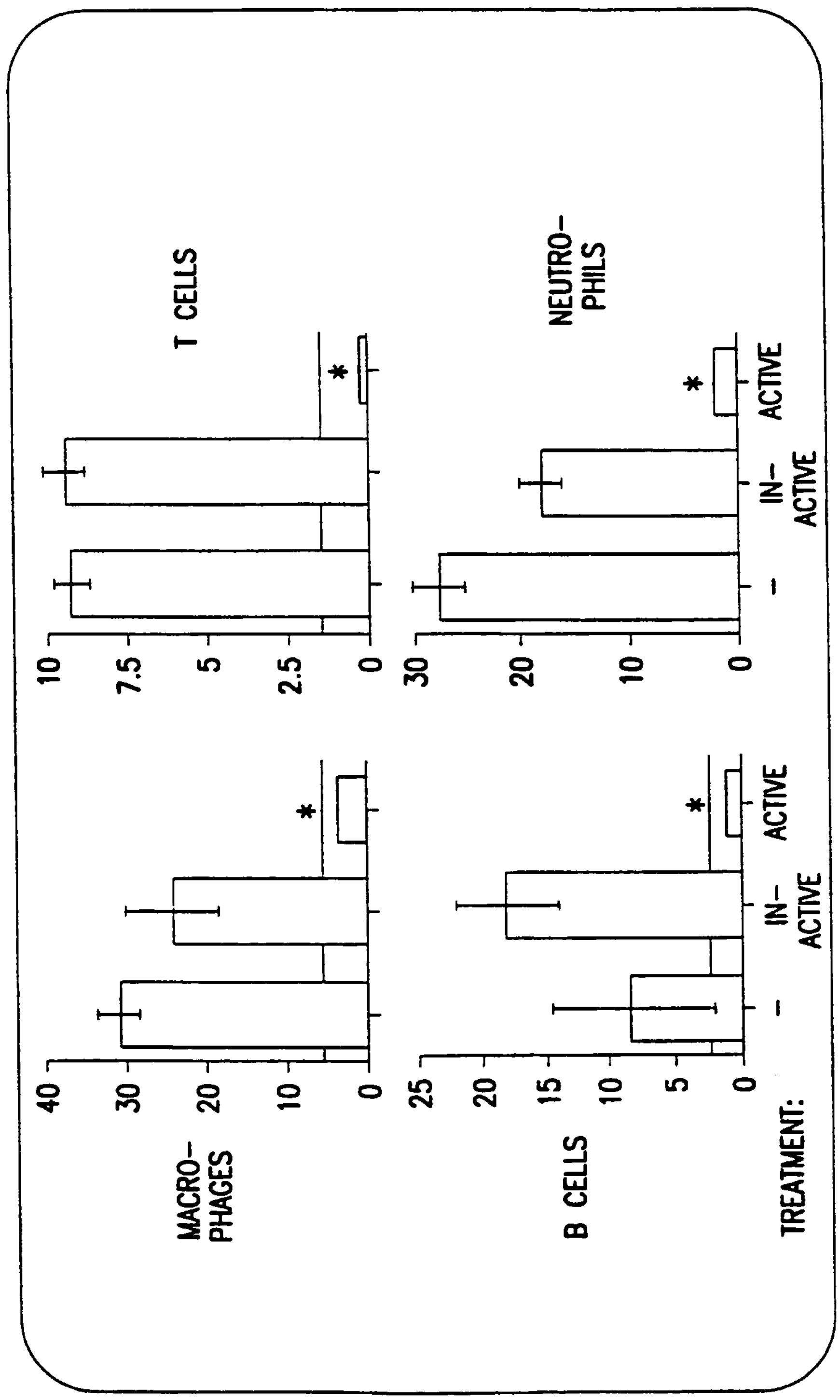


* p<0.05 VERSUS LPS-CHALLENGED, UNTREATED

FIG. 15A

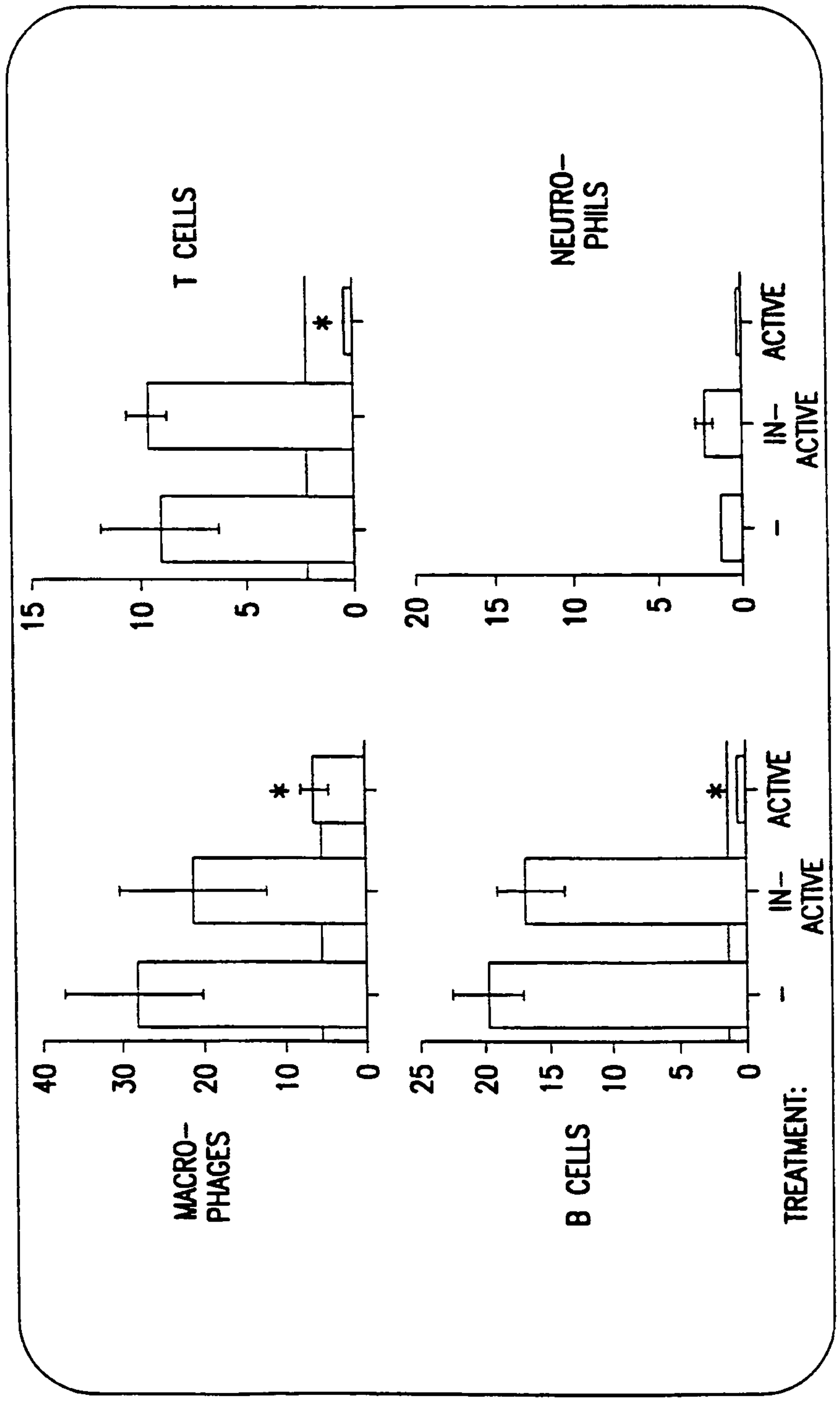


* p<0.05 VERSUS MCP1-CHALLENGED, UNTREATED

FIG. 15B

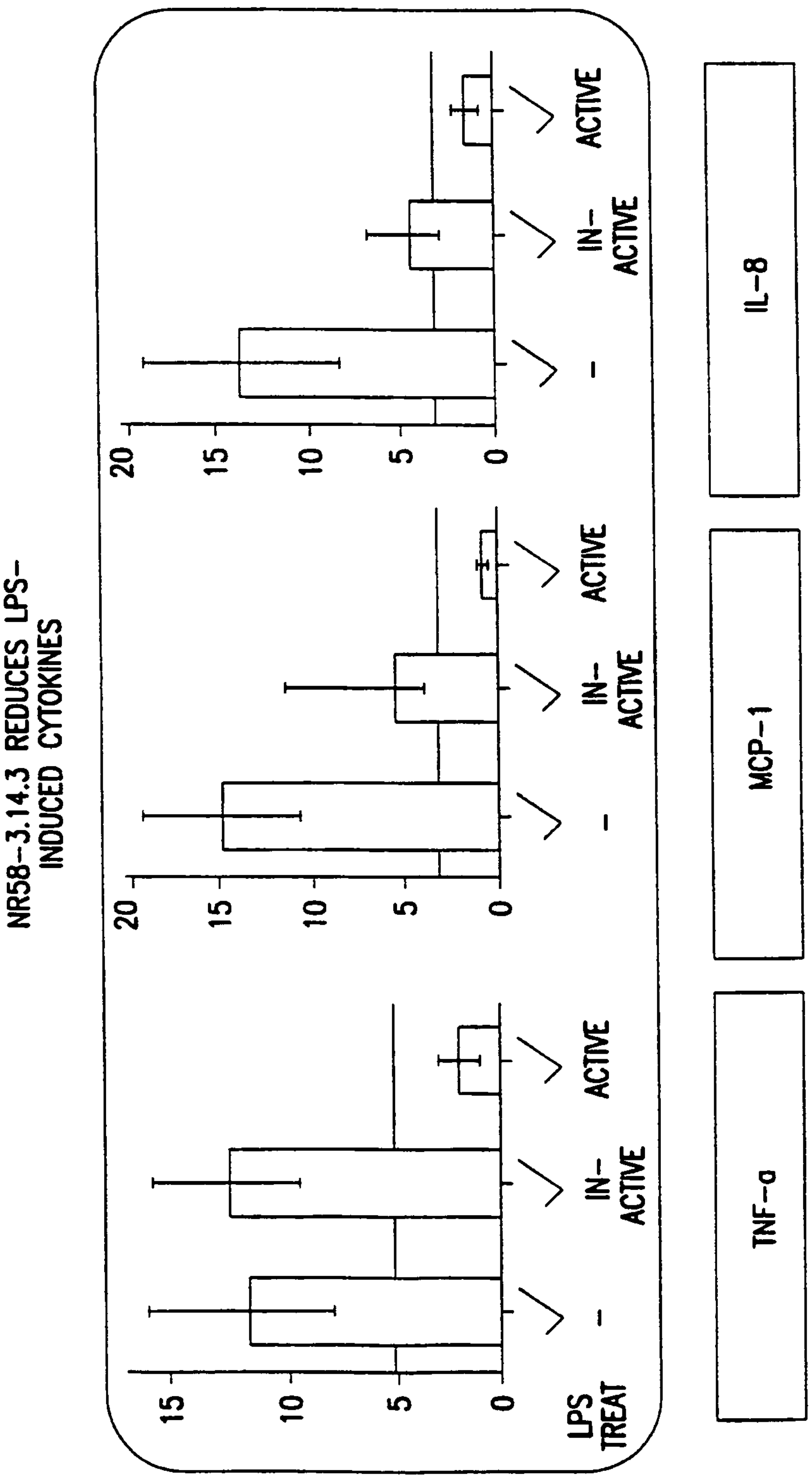
NR58-3.14.3 REDUCES LPS-INDUCED CYTOKINES
15
10
5
0
LPS
TREAT
–
IN-ACTIVE
ACTIVE
TNF-α
20
15
10
5
0
–
IN-ACTIVE
ACTIVE
MCP-1
20
15
10
5
0
–
IN-ACTIVE
ACTIVE
IL-8

FIG. 15C

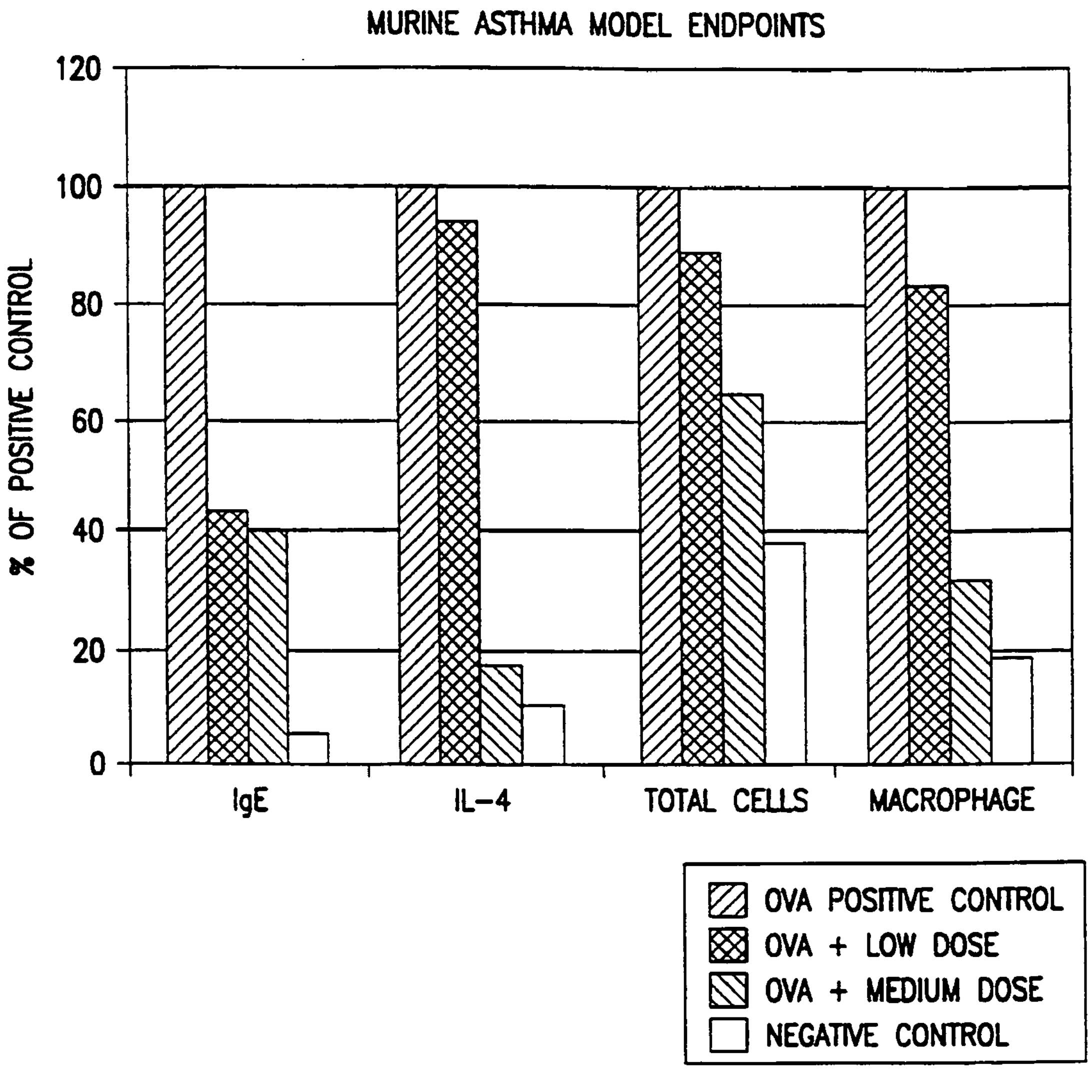
MURINE ASTHMA MODEL ENDPOINTS
120
100
80
60
40
20
0
% OF POSITIVE CONTROL
IgE
IL-4
TOTAL CELLS
MACROPHAGE
OVA POSITIVE CONTROL
OVA + LOW DOSE
OVA + MEDIUM DOSE
NEGATIVE CONTROL

FIG. 16A

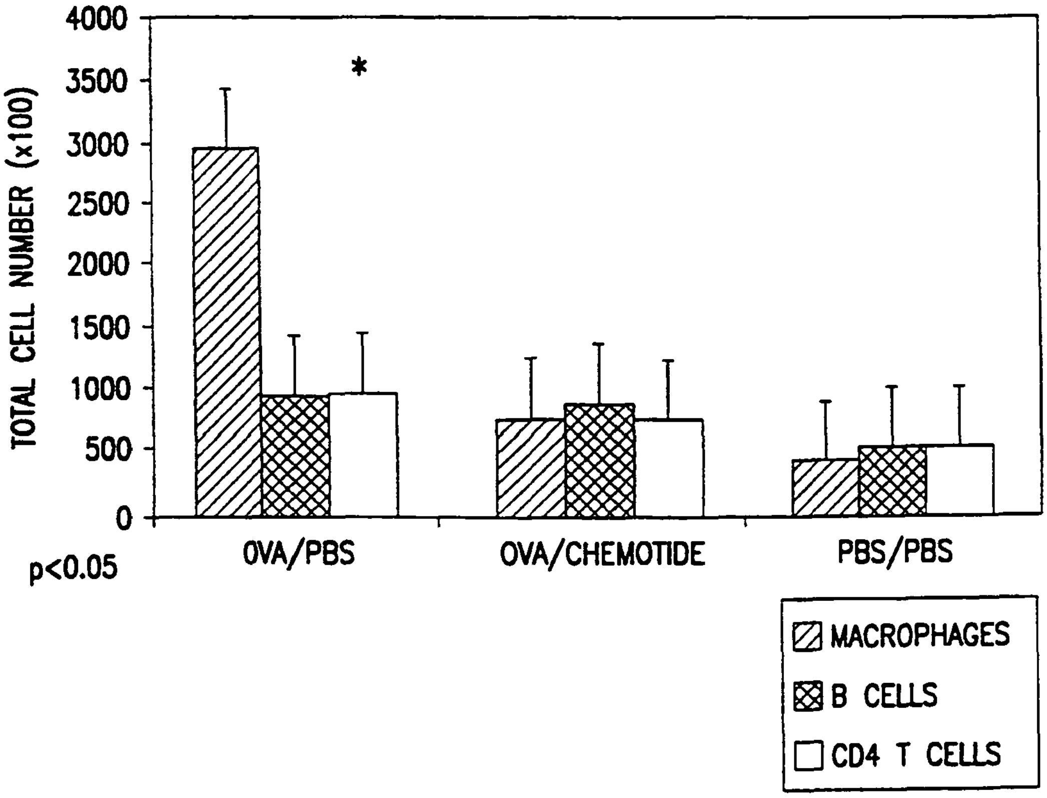
4000
3500
3000
2500
2000
1500
1000
500
0
TOTAL CELL NUMBER (x100)
*
p<0.05
OVA/PBS
OVA/CHEMOTIDE
PBS/PBS
MACROPHAGES
B CELLS
CD4 T CELLS

FIG. 16B

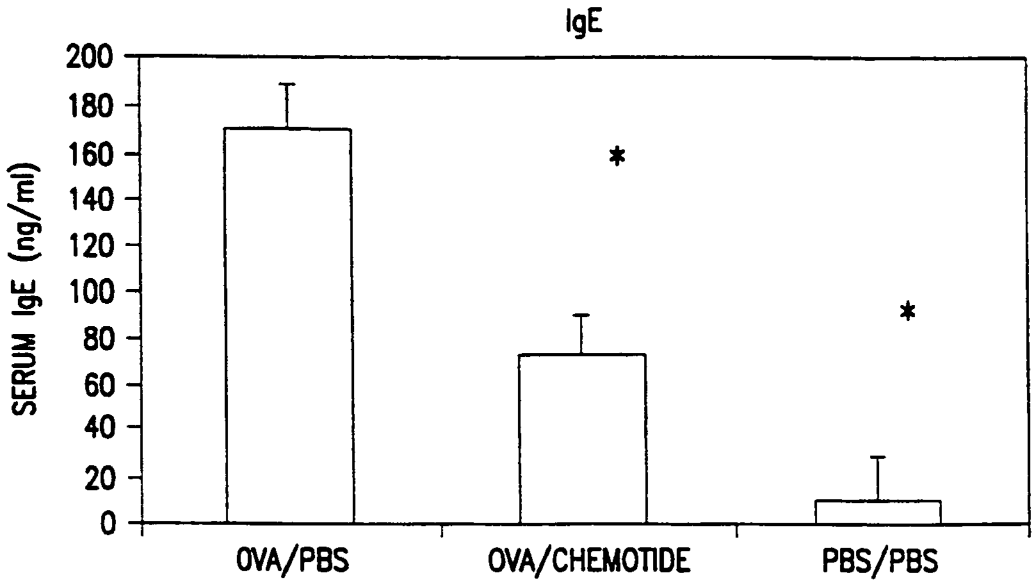


*p<0.05 COMPARED TO POSITIVE CONTROL-OVA

FIG. 16C

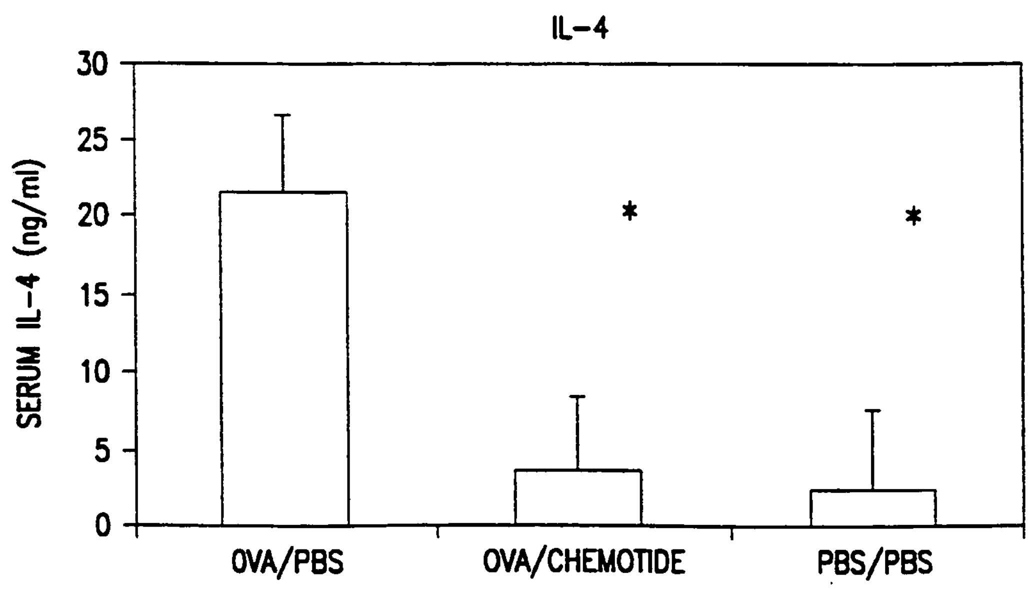


*p<0.05 COMPARED TO POSITIVE CONTROL-OVA

FIG. 16D

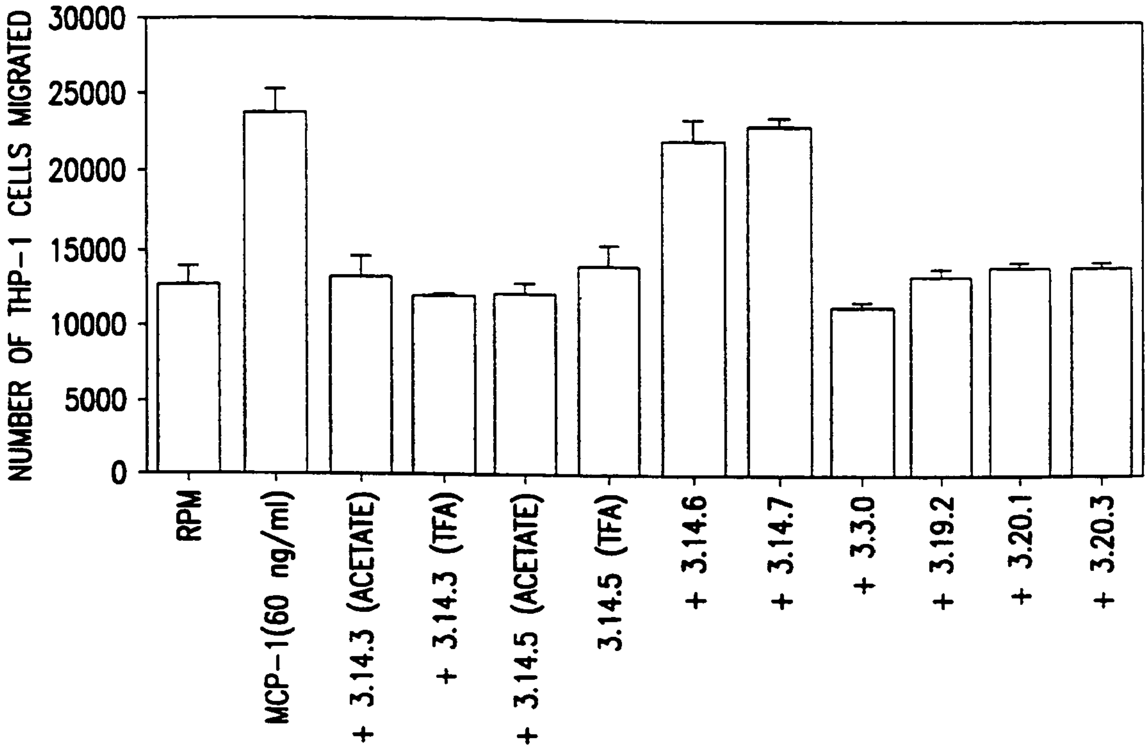
NUMBER OF THP-1 CELLS MIGRATED
30000
25000
20000
15000
10000
5000
0
RPM
MCP-1(60 ng/ml)
+ 3.14.3 (ACETATE)
+ 3.14.3 (TFA)
+ 3.14.5 (ACETATE)
3.14.5 (TFA)
+ 3.14.6
+ 3.14.7
+ 3.3.0
+ 3.19.2
+ 3.20.1
+ 3.20.3

FIG. 17

| Common Name | Sequence | L/D | Direction |
|---|---|---|---|
| Peptide 1 | AQPDAINAPVTCC | L | Forward |
| Peptide 2 | SYRRITSSKCPKEAV | L | Forward |
| Peptide 2 /control/ | SYRRITSSKCPKEAV | L | Forward |
| Biotin-Peptide 2 | biotin-SYRRITSSKCPKEAV | L | Forward |
| LRD-peptide 2 | vaekpcksstirrysc | D | Reversed |
| Cys10 (DTT)-Peptide 2 | SYRRITSSKC*PKEAV | L | Forward |
| Peptide 2 /dimer/ | SYRRITSSKC*PKEAV | L | Forward |
| Peptide 2 (SDP-1) | HLKILNTPNCALQIV | L | Forward |
| | | | |
| Peptide 2 (MIP-1a) | DYFETSSQCSKPGV | L | Forward |
| LRD-Peptide 2 (MIP-1a) | vgpkscqsstefyd | D | Reversed |
| Peptide 2 (IL-8) | ELRVIESQPHCANTEI | L | Forward |
| Peptide 2 (1-9) | SYRRITSSK | L | Forward |
| Peptide 2 (10-15) | CPKEAV | L | Forward |
| Peptide 2 (1-5) | SYRRI | L | Forward |
| Peptide 2 (6-10) | TSSKC | L | Forward |
| Peptide 2(1-9) (MIP-1a) | DYFETSSQC | L | Forward |
| Cys0Ser10Cys16-Peptide 2 | CSYRRITSSKSPKEAVC | L | Forward |
| LRD-Cys0Ser10Cys16-Peptide 2 | cvaekpsksstirrysc | D | Forward |
| CRD-Cys0Ser10Cys16-Peptide 2 | cvaekpsksstirrysc | D | Reversed |
| LariatFL-Cys0-Peptide 2 | CSYRRITSSKCPKEAV | L | Forward |
| LariatFL-Cys16-Peptide 2 | SYRRITSSKCPKEAVC | L | Forward |
| LariatRD-Cys0-Peptide 2 | vaekpcksstirrysc | D | Reversed |
| LariatRD-Cys16-Peptide 2 | cvaekpcksstirrys | D | Reversed |
| Ser16-Peptide 2 (SDF-1) | HLKILNTPNSALQIV | L | Forward |
| CRD-Cys0Ser10Cys16-Peptide 2 (SDF-1) | cviqlasnptaliklhc | D | Reversed |
| Peptide 3 | EICADPKQKWVQ | L | Forward |
| Peptide 3 (3-12) | CADPKQKWVQ | L | Forward |
| LRD-Peptide 3 (3-12) | cqvwkqkpdac | D | Reversed |
| CRD-Peptide 3 (3-12) | cqvwkqkpdac | D | Reversed |
| Peptide 3 (1-6) | EICADP | L | Forward |
| Peptide 3 (7-12) | KQKWVQ | L | Forward |
| LRD-Peptide 3 (3-12) | qvwkqk | D | Reversed |
| Leu4-Peptide 3 | EICLDPKQKWVQ | L | Forward |
| Peptide 3 (MIPla) | EICADPSEEWVQ | L | Forward |
| Peptide 3 (10-12) | WVQ | L | Forward |
| Peptide 3 (7-9) | KQK | L | Forward |
| Peptide 3(7-9), carbonate | KQK | L | Forward |

FIG. 18

| | | | |
|---|---|---|---|
| Peptide 3(7-9) (MIP1a) | SEE | L | Forward |
| Peptide 3(7-9) (IL-6) | KEN | L | Forward |
| Peptide 3(7-9) (SDP1) | KLK | L | Forward |
| Peptide 3(7-9) (SDP1)-acetate | KLK | L | Forward |
| CRD-Leu4IleIlCys13-Peptide 3 (3-12) | cqiwkqkpdlc | D | Reversed |
| CRD-Leu4IleIlCys13-Peptide 3 (3-12) /D-Ala/ | cqiwkqkpd*lc | D | Reversed |
| CRD-Leu4IleIlCys13 Peptide3 (3-12) /L-Leu4/acetate | cqiwkqkpdLc | D | Reversed |
| CRD-Tyr-2pAla-1pAla0Leu4 IleIlCys13-Peptide 3 (3-12) | YβAβAcqiwkqkpdlc | D/L | Reversed |
| CRD-Leu4 IleIlCys13βAla14βAla15Tyr16-Peptide 3 (3-12) | cqiwkqkpnlcβAβAY | D/L | Reversed |
| CRD-Leu4IleIlCys13-Peptide 3 (3-12) /L-IleIl/ | cqIcwkqkpdlc | D/L | Reversed |
| Peptide 3(7-9) (MIPIβ) | SES | L | Forward |
| Peptide 3(7-9) Eotaxin) | KKK | L | Forward |
| Cys13-Peptide 3(10-12) | WVQC | L | Forward |
| Cys13-Peptide 3(10-12) (SDP1) | WIQC | L | Forward |
| CRD-Leu4Cys13-Peptide 3 (3-12) | cqvwkqkpdlc | D | Reversed |
| CFL-Cys4Cys13-Peptide3 (7-12) | CKQKWVQC | L | Forward |
| CRD-Cys4Cys13-Peptide3 (7-12) | cqvwkqkc | D | Reversed |
| Peptide 3(10-12) (RANTES) | WVR | L | Forward |
| Peptide 3(10-12) (SDP1) | WIQ | L | Forward |
| Peptide 3 (7-9) (RANTES) | EKK | L | Forward |
| Biotin-Leu4 IleIl Peptide 3 (3-12) | biotin-CLDPKQKWIQ | L | Forward |
| Ala8-Peptide 3(7-9-amide | KAK | L | Forward |
| Cys8-Peptide 3(7-9)-amide | KCK | L | Forward |
| Asp8-Peptide 3(7-9)-amide | KDK | L | Forward |
| Glu8-Peptide 3(7-9)-amide | KEK | L | Forward |
| Phe8-Peptide 3(7-9) | KFK | L | Forward |
| Gly8-Peptide 3(7-9)-amide | KGK | L | Forward |
| His8-Peptide 3(7-9)-amide | KHK | L | Forward |
| Ile8-Peptide 3(7-9)-amide | KIK | L | Forward |
| Met8-Peptide 3(7-9)-amide | KMK | L | Forward |
| Asn8-Peptide 3(7-9)-amide | KNK | L | Forward |
| Pro8-Peptide 3(7-9)-amide | KPK | L | Forward |
| Arg8-Peptide 3(7-9)-amide | KRK | L | Forward |
| Ser8-Peptide 3(7-9) | KSK | L | Forward |
| Thr8-Peptide 3(7-9)-amide | KTK | L | Forward |
| Val8-Peptide 3(7-9)-amide | KVK | L | Forward |

FIG. 18 CONTINUED

| | | | |
|---|---|---|---|
| Trv8-Peptide 3(7-9) | KWK | L | Forward |
| Tyr8-Peptide 3(7-9) | KYK | L | Forward |
| | | | |
| Glyll-Peptide 3 (10-12) | WGQ | L | Forward |
| Alall-Peptide 3 (10-12) | WAQ | L | Forward |
| EthylGlyll-Peptide 3 (10-12) | W(EtGl)Q | L | Forward |
| Allolle11-Peptide 3 (10-12) | W(AlloI)Q | L | Forward |
| LRL-Peptide 3(7-9) (MIPla) | EES | L | Forward |
| Peptide 3(10-11) | WV | L | Forward |
| APG | APG | L | Forward |
| Furin substrate | pERTKR-AMC | L | Forward |
| GD | GD | L | Forward |
| GGR | GGR | L | Forward |
| Peptide F1 | SGPSIVHRKCF | L | Forward |
| Peptide F1-biotin | biotin-SGPSIVHRKCF | L | Forward |
| Peptide F2 | MCEEEDSTAL | L | Forward |
| Peptide F3 | SGPSIVHRKCFGMCEE EDSTAL | L | Forward |
| Peptide HIV-1 env V3 loop (MN) | CTRPNYNKRKRIHIGP GRAFYTTKNIIGTIRQA HC | L | Forward |
| Peptide HIV-A (1-24) | NNTRKSIRIQRGPGRA FVTIGKIG | L | Forward |
| Peptide HIV-A (11-20) | RGPGRAFVTI | L | Forward |
| Peptide HIV-A (11-20) biotin | biotin-RGPGRAPVTI | L | Forward |
| Peptide HIV-A (8-22) | RIQRGPGRAFVTIGK | L | Forward |
| Peptide T1 | LYIDFRKDLGWKW | L | Forward |
| Peptide T2 | HEPKGYHANFC | L | Forward |
| Peptide T3 | VYYVGRK | L | Forward |
| RGD | RGD | L | Forward |
| RGD | RGD | L | Forward |
| TGF-β1 (78) | CVPQALEPLPIVYYVG RKPHVEQLSNMIVRSC | L | Forward |
| TGF-β1 (pre 266) | ERAQHLQSSRHRRC | L | Forward |
| TGF-β1 (pre 274) | SRHRRALDTNYC | L | Forward |
| TGF-β1 (pre 40) | LVKRKRIEAIRC | L | Forward |
| TGF-β1 (pre-25) | RPAAGLSTCKTIDME | L | Forward |
| TTT | TTT | L | Forward |
| VE | VE | L | Forward |

FIG. 18 CONTINUED

| Name | Description | If not stated |
|---|---|---|
| Nterm | N-terminal modification (e.g. Biotin) | -NH2 |
| FORMAT | Is it linear/cyclised/reversed/D or L | LFL |
| $Mut_xMut_y$ | Substitutions from human chemokine sequence | None |
| X | Peptide region (1,2 or 3) | - |
| a-b | Number of residues included (from - to) | Full length |
| Chemokine | Parental chemokine from which sequence came | MCP-1 |
| Cterm | C-terminal modification (e.g. amide) | -COOH |
| extra | Extra information (e.g. dimer, D-ala addition etc) | None |
| Salt | Salt of peptide (e.g. TFA. acetate, citrate, carbonate) | TFA |

FIG. 18 CONTINUED

ED-50's of Peptides 1 to 3 and its derivatives with CC and CXC Chemokines

| Peptide | Sequence | MCP-1 | IL-8 | fMLP |
|---|---|---|---|---|
| Peptide 1[1-13] | AQPDAINAPVTCC | >100 | >100 | >100 |
| Peptide 2[1-15] | SYRRITSSKCPKEAV | >100 | >100 | >100 |
| Peptide 3[1-12] | EICADPKQKWVQ | 8 ± 2 | 10 ±1 | >100 |
| Peptide 3[1-6] | EICADP | 25 ± 5 | 16 ± 2 | >100 |
| Peptide 3[7-12] | KQKWVQ | 7 ± 1 | 5 ± 2 | >100 |
| Peptide 3[3-12] | CADPKQKWVQ | 8 ± 1 | 9 ± 4 | >100 |
| $Leu_4$ Peptide 3[1-12] | EICLDPKQKWVQ | 8 ± 2 | 3 ± 1 | >100 |
| $Ser_7$ Peptide 3[1-12] | EICADPSQKWVQ | 7 ± 1 | 3 ± 2 | >100 |
| $Ile_{11}$ Peptide 3[1-12] | EICADPKQKWIQ | 6 ± 3 | 2 ± 1 | >100 |
| $Leu_4$ $Ile_{11}$ Peptide 3[1-12] | EICLDPKQKWIQ | 2 ± 0 | 4 ± 2 | >100 |

FIG. 19

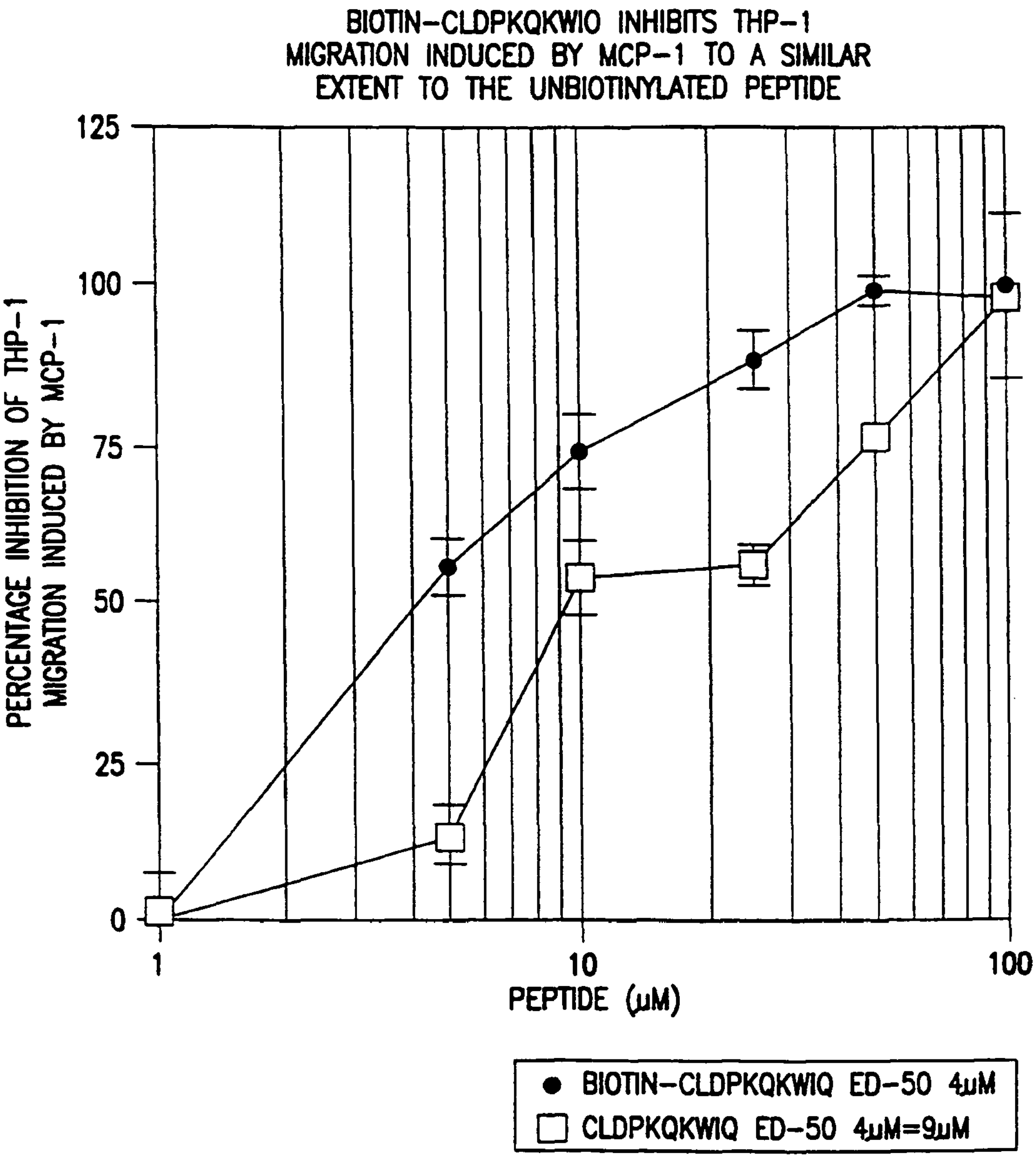
BIOTIN-CLDPKQKWIO INHIBITS THP-1 MIGRATION INDUCED BY MCP-1 TO A SIMILAR EXTENT TO THE UNBIOTINYLATED PEPTIDE
PERCENTAGE INHIBITION OF THP-1 MIGRATION INDUCED BY MCP-1
125
100
75
50
25
0
1
10
100
PEPTIDE (μM)
BIOTIN-CLDPKQKWIQ ED-50 4uM
CLDPKQKWIQ ED-50 4uM=9uM

FIG. 20

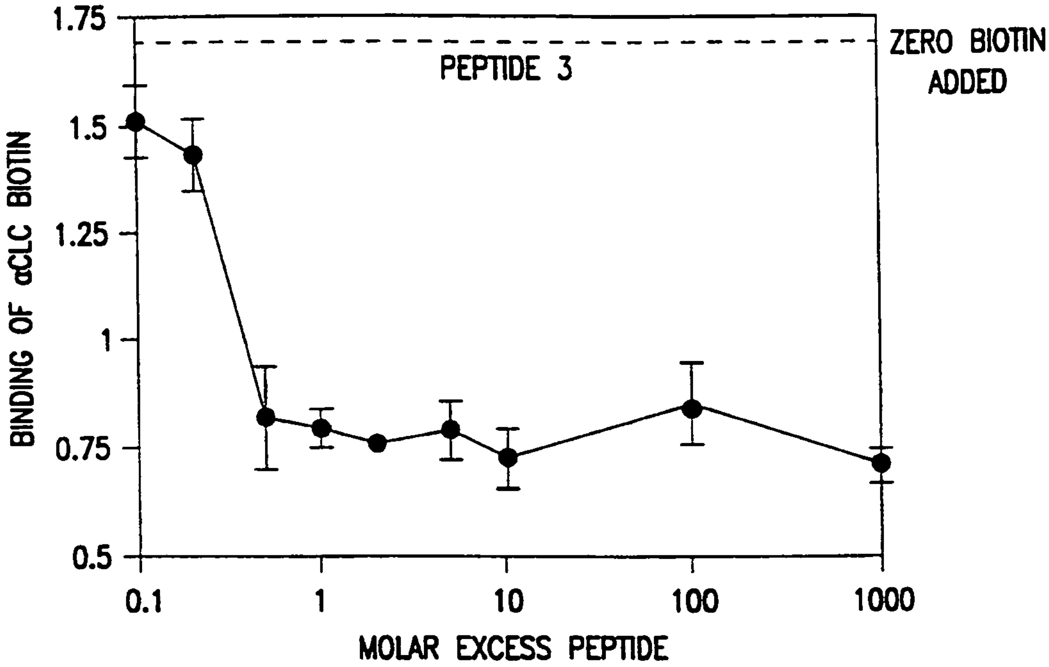


FIG. 21A

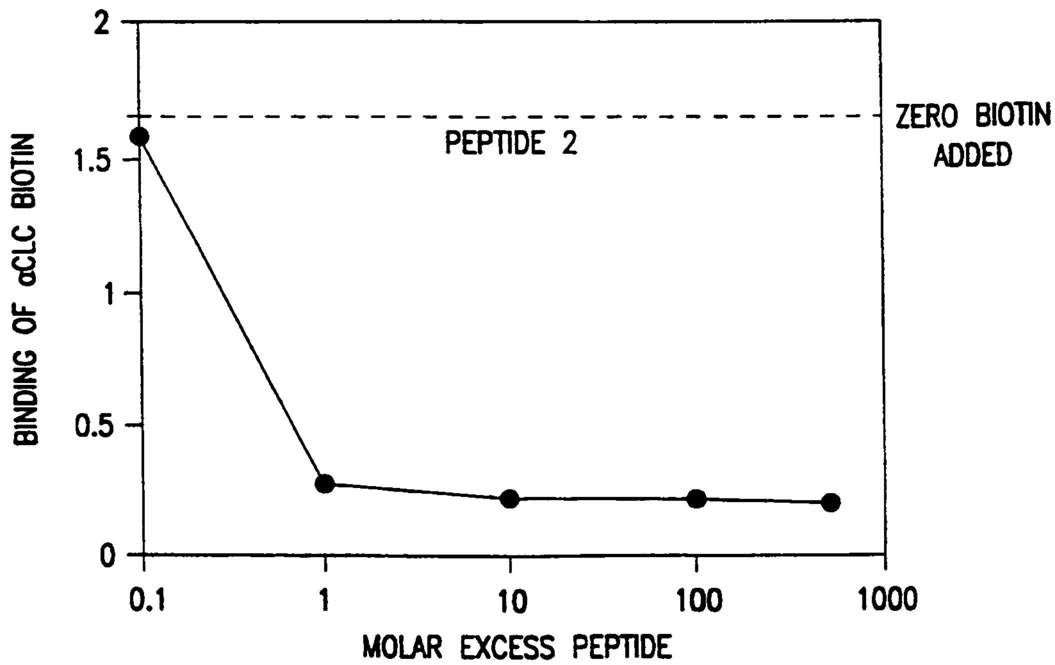


FIG. 21B

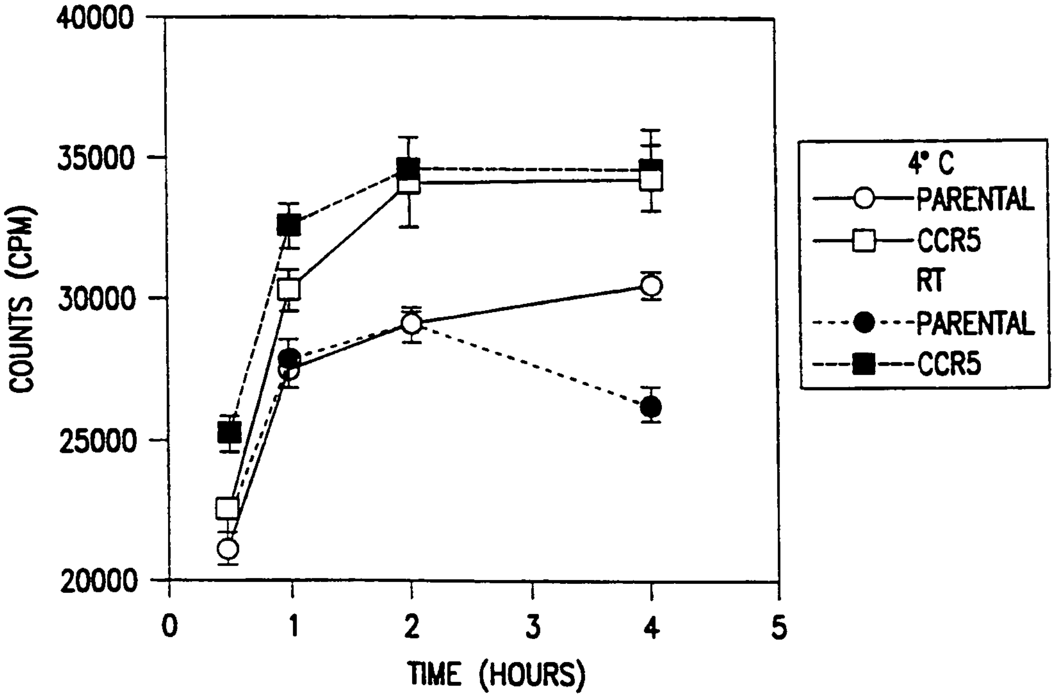


FIG. 22A

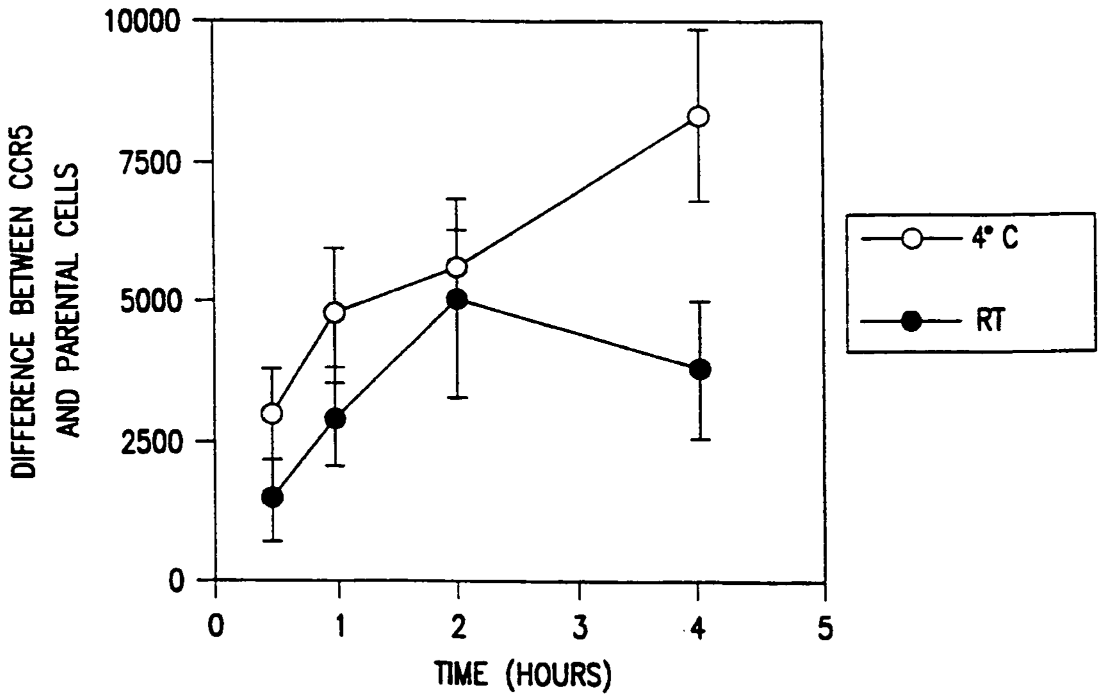


FIG. 22B

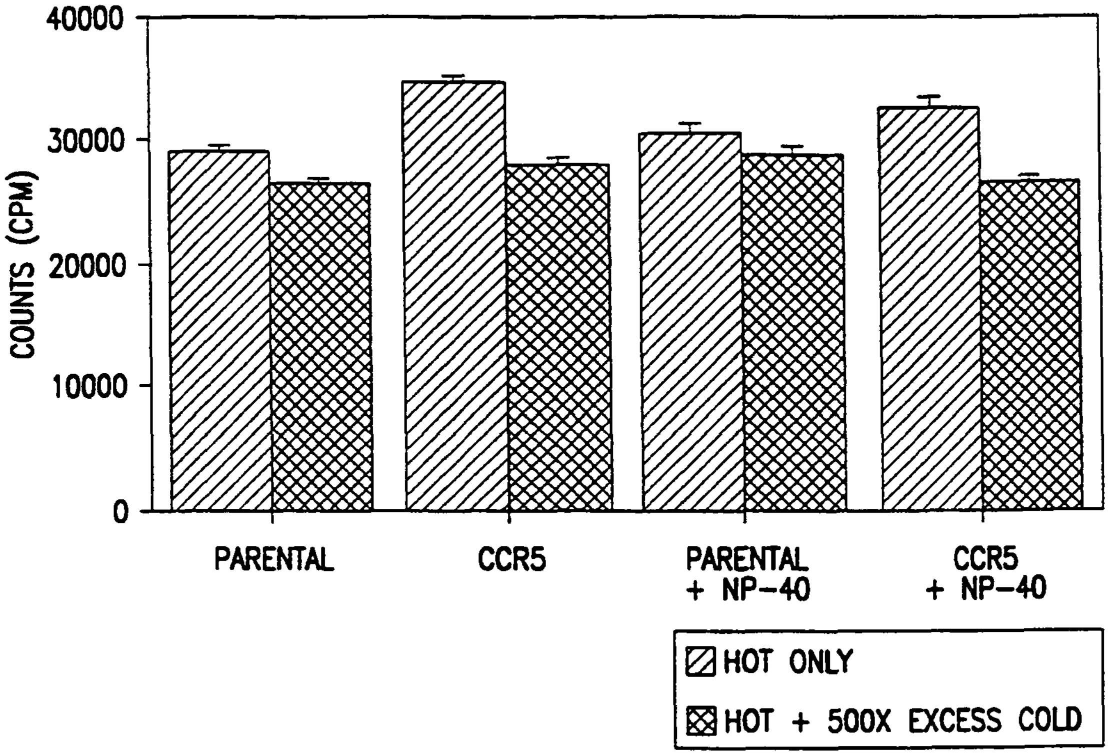


FIG. 22C

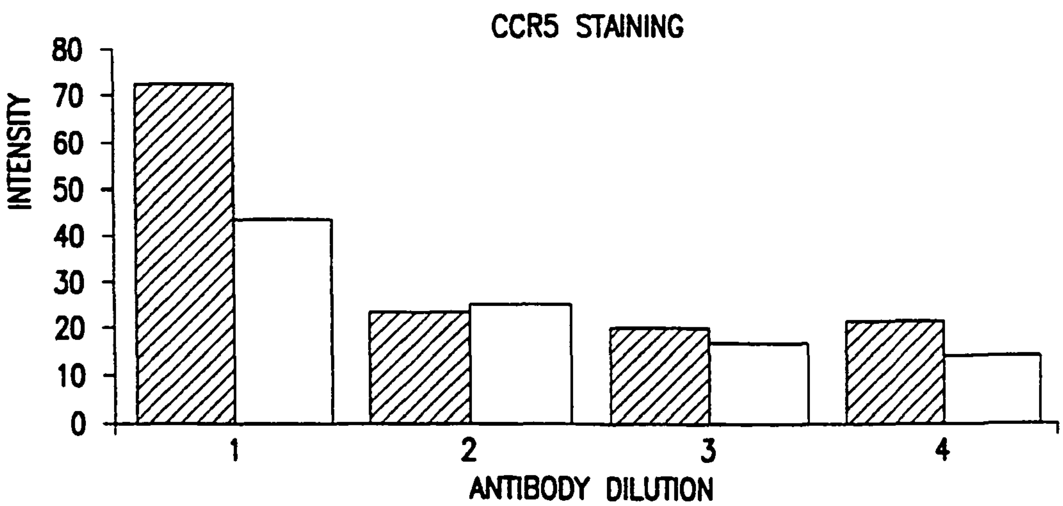


FIG. 23

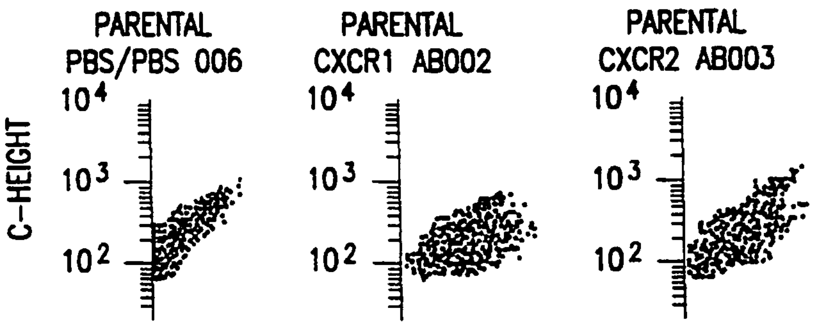


FIG. 24A

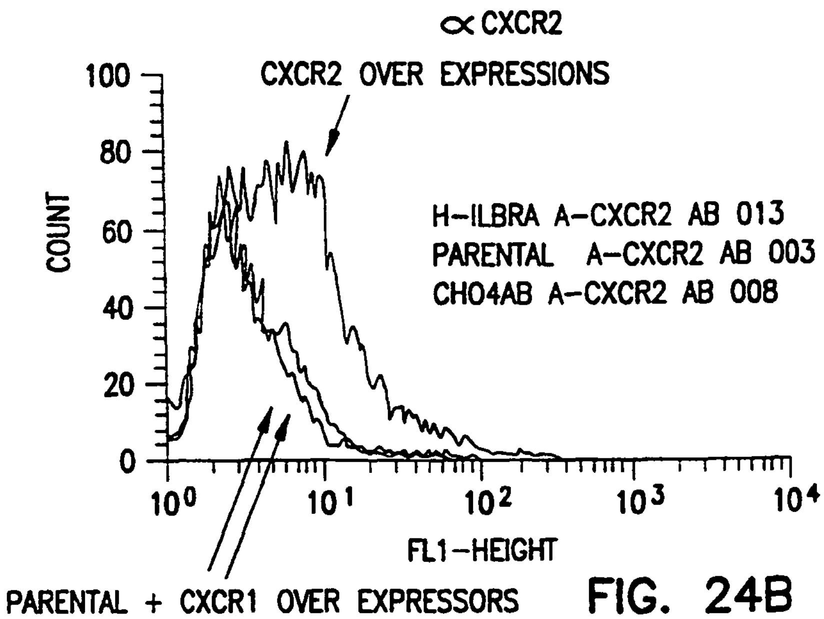


FIG. 24B

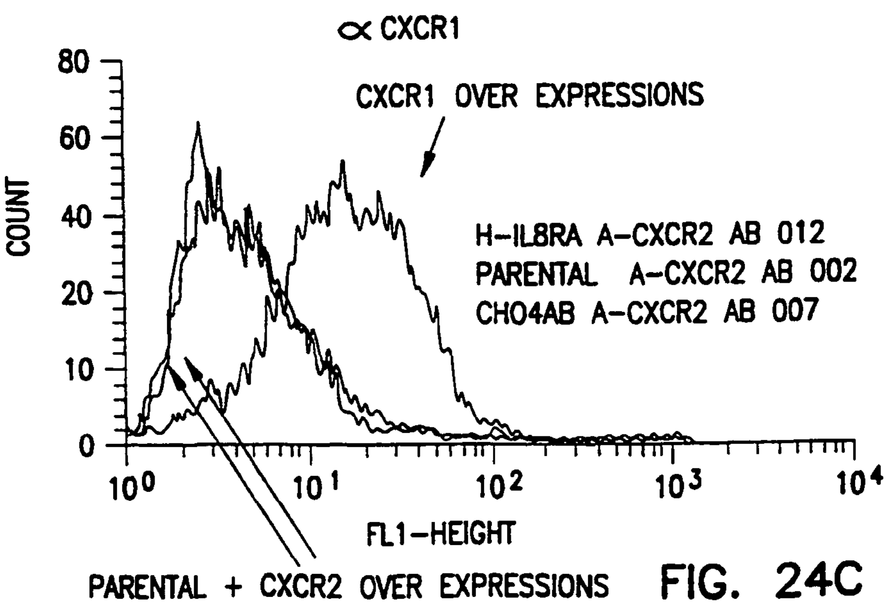


FIG. 24C

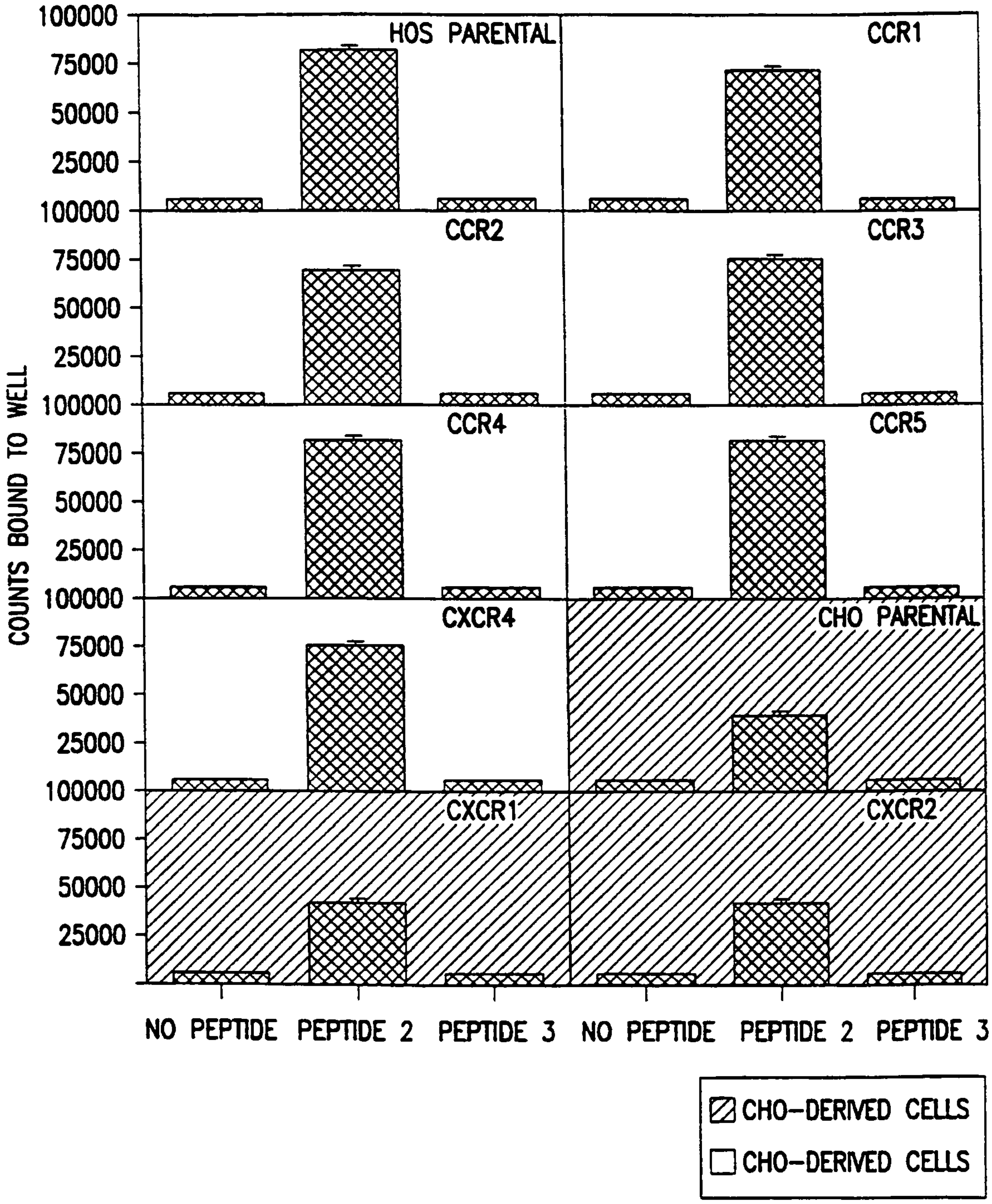

COUNTS BOUND TO WELL
100000
75000
50000
25000
HOS PARENTAL
CCR1
CCR2
CCR3
CCR4
CCR5
CXCR4
CHO PARENTAL
CXCR1
CXCR2
NO PEPTIDE
PEPTIDE 2
PEPTIDE 3
CHO-DERIVED CELLS
CHO-DERIVED CELLS


FIG. 25

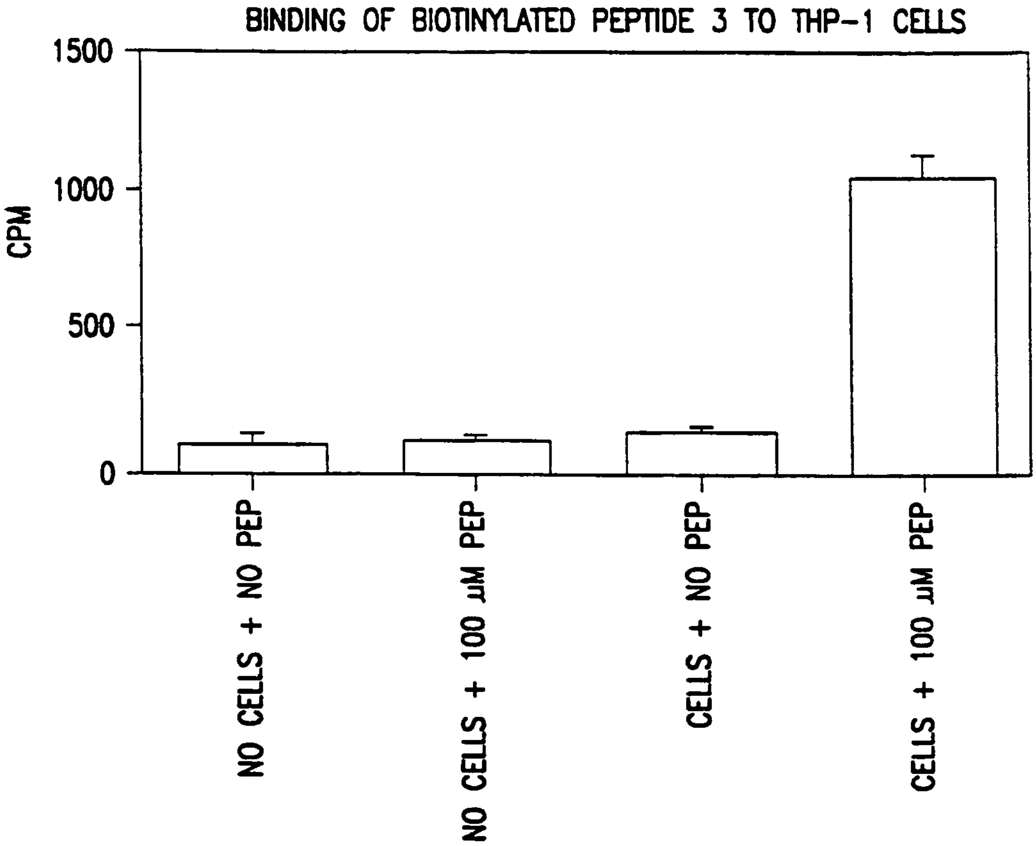


FIG. 26A

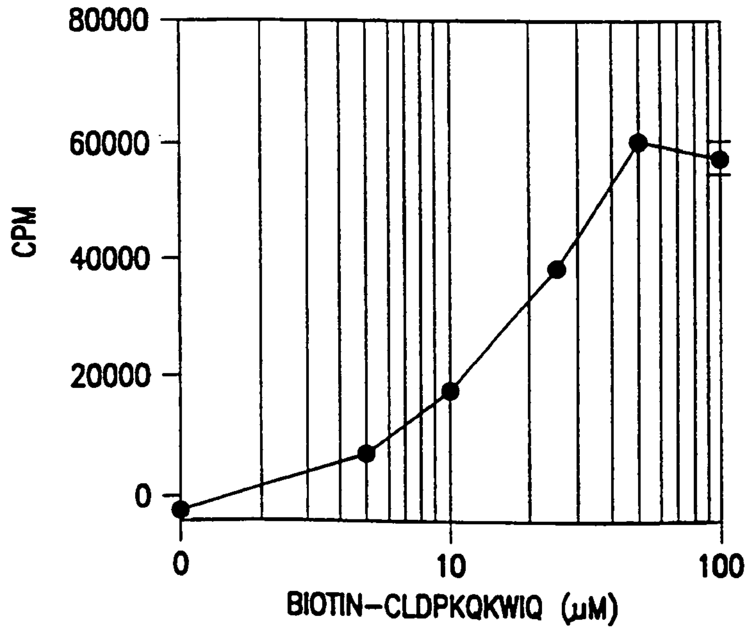


FIG. 26B $K_A$ =15 μM

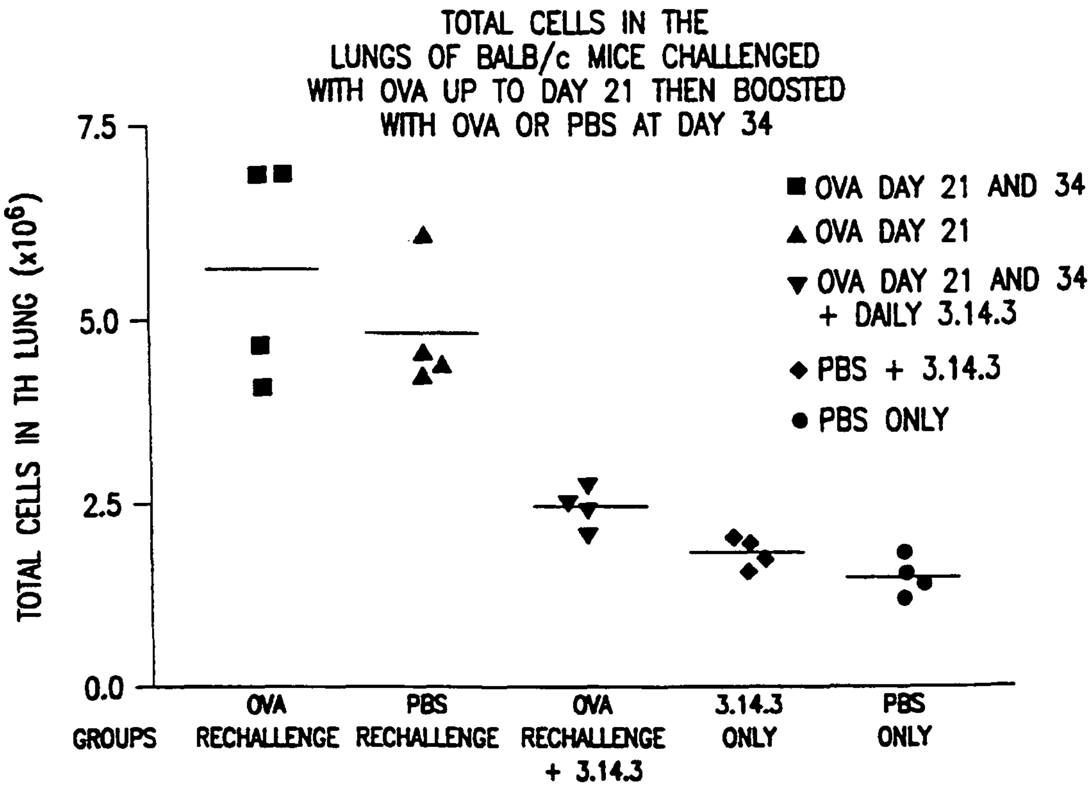


FIG. 27A

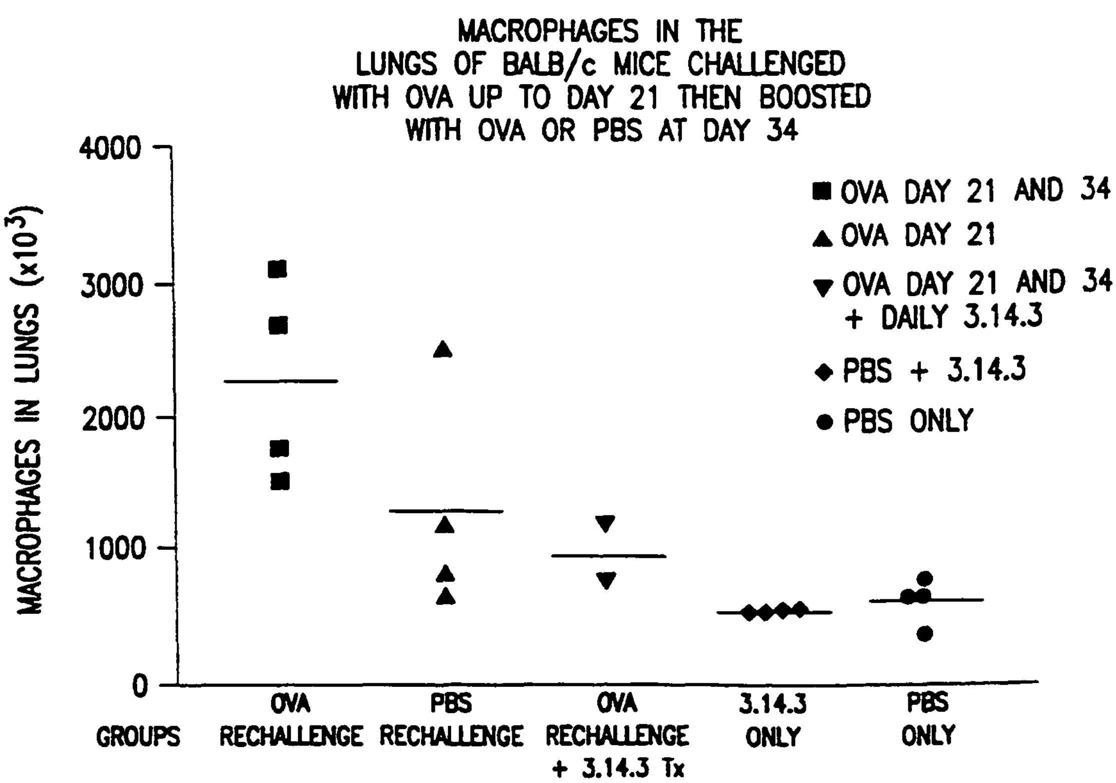


FIG. 27B

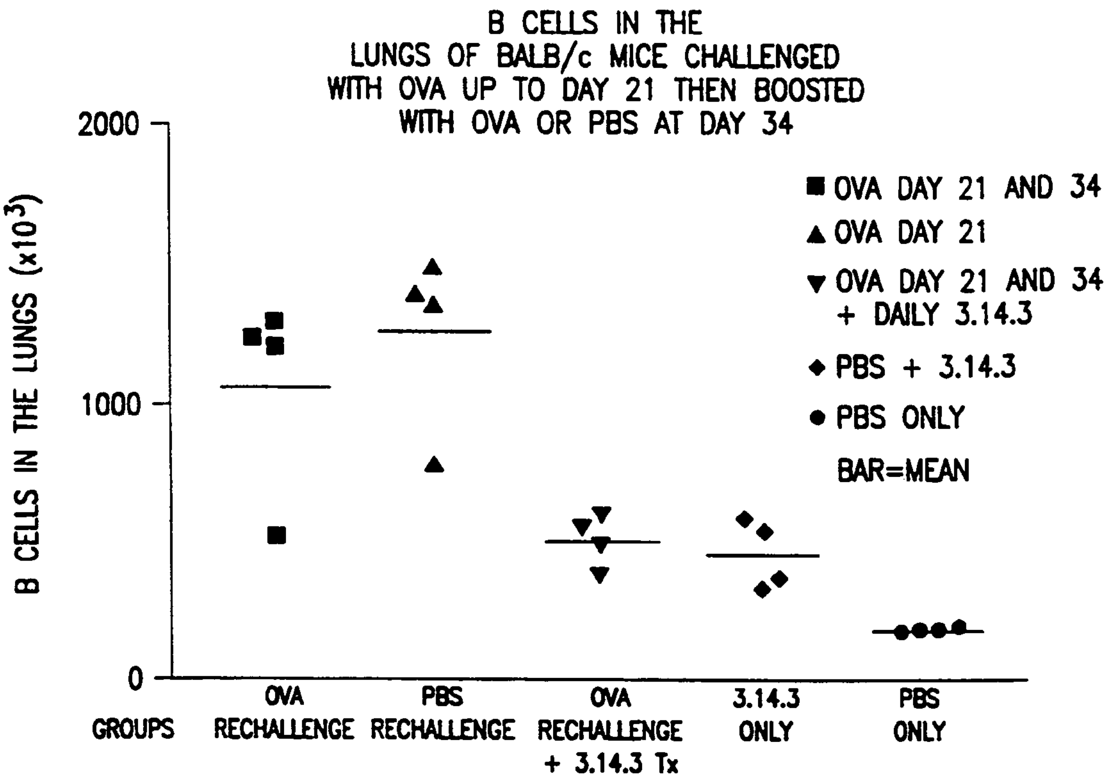


FIG. 27C

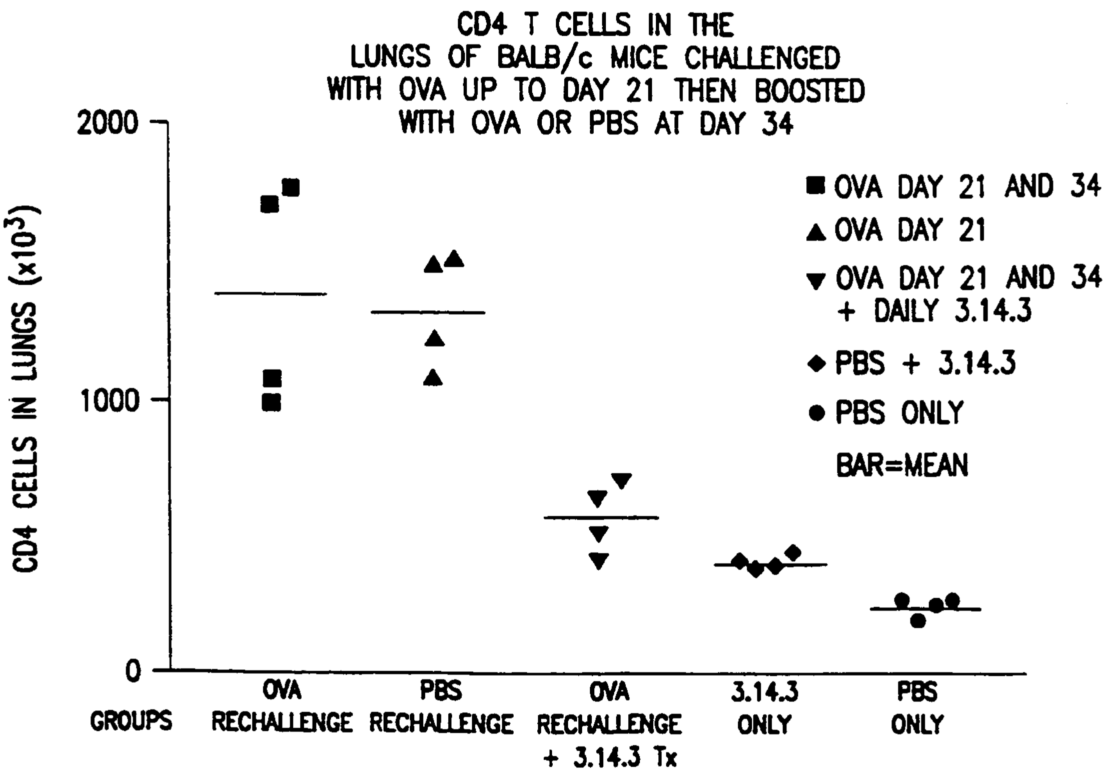


FIG. 27D

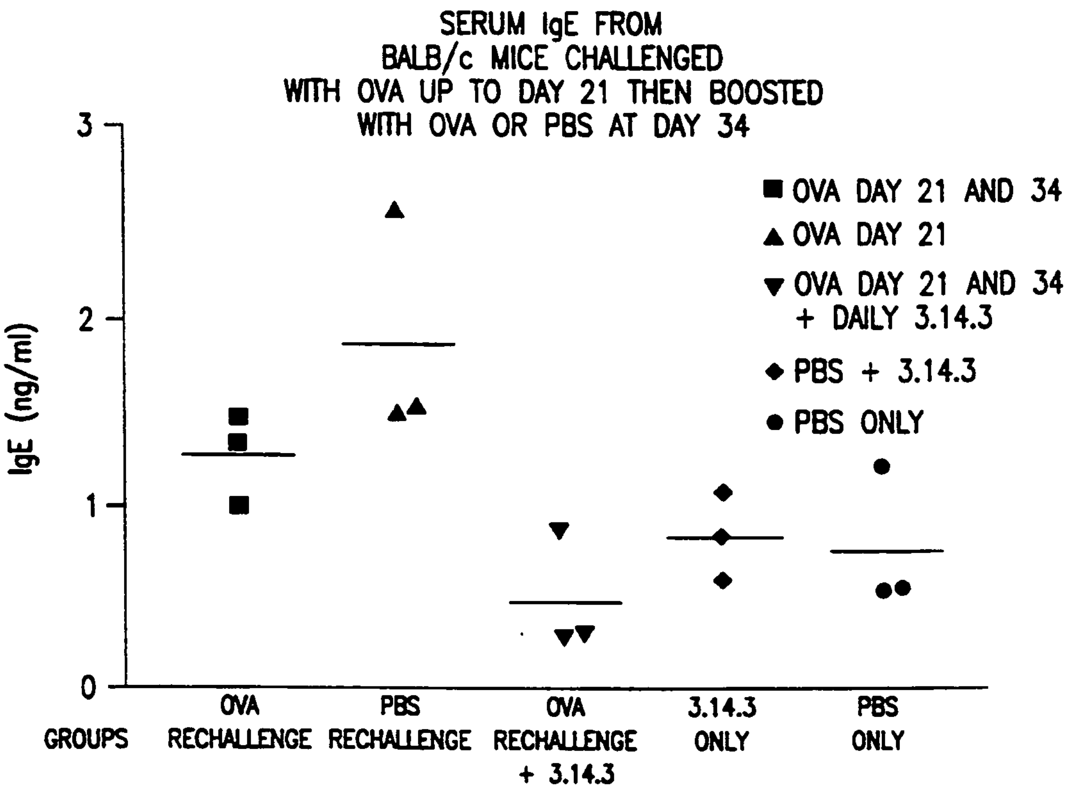
SERUM IgE FROM
BALB/c MICE CHALLENGED
WITH OVA UP TO DAY 21 THEN BOOSTED
WITH OVA OR PBS AT DAY 34
IgE (ng/ml)
0
1
2
3
OVA DAY 21 AND 34
OVA DAY 21
OVA DAY 21 AND 34 + DAILY 3.14.3
PBS + 3.14.3
PBS ONLY
GROUPS
OVA RECHALLENGE
PBS RECHALLENGE
OVA RECHALLENGE + 3.14.3
3.14.3 ONLY
PBS ONLY

FIG. 28

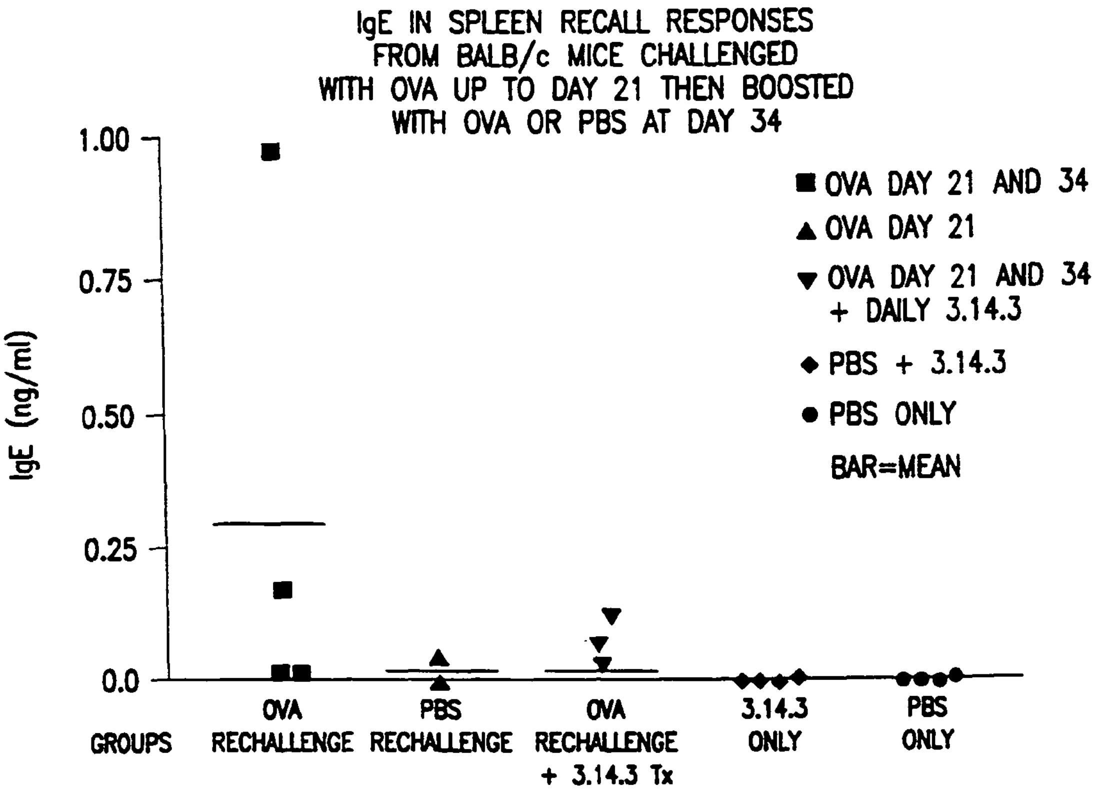


FIG. 29A

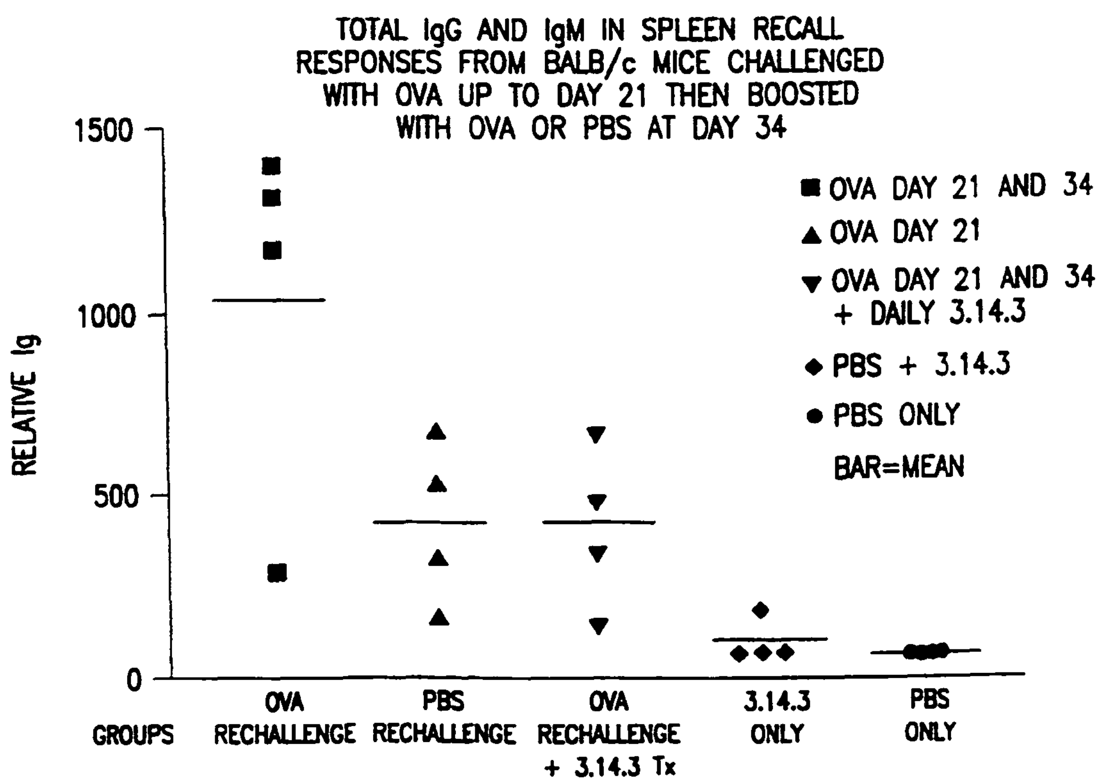


FIG. 29B

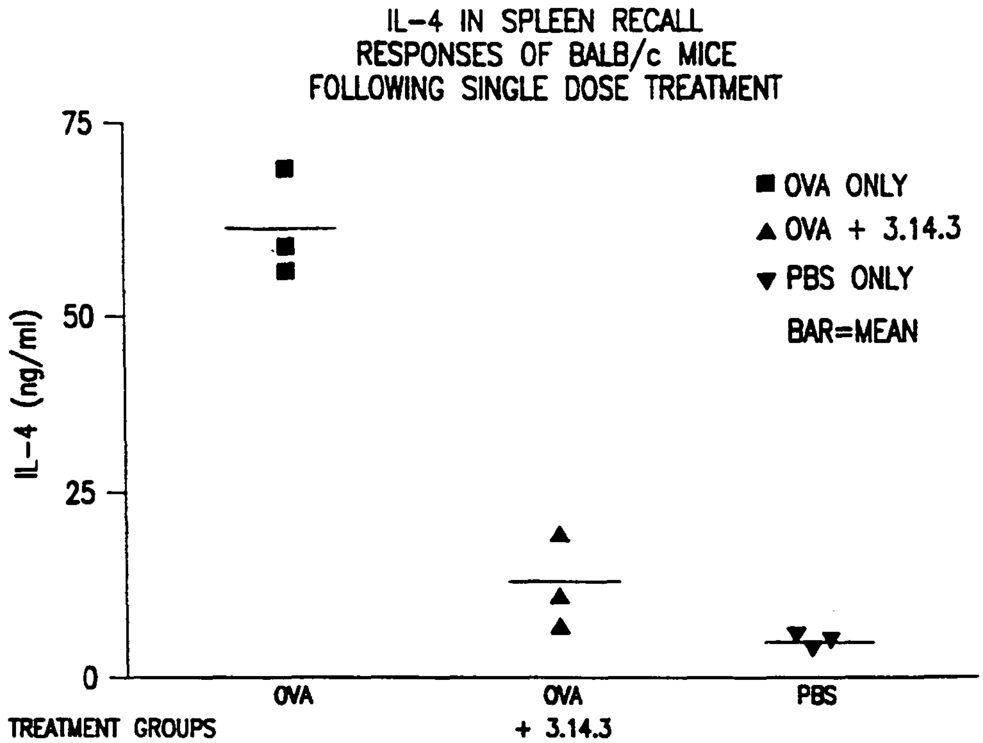


FIG. 30A

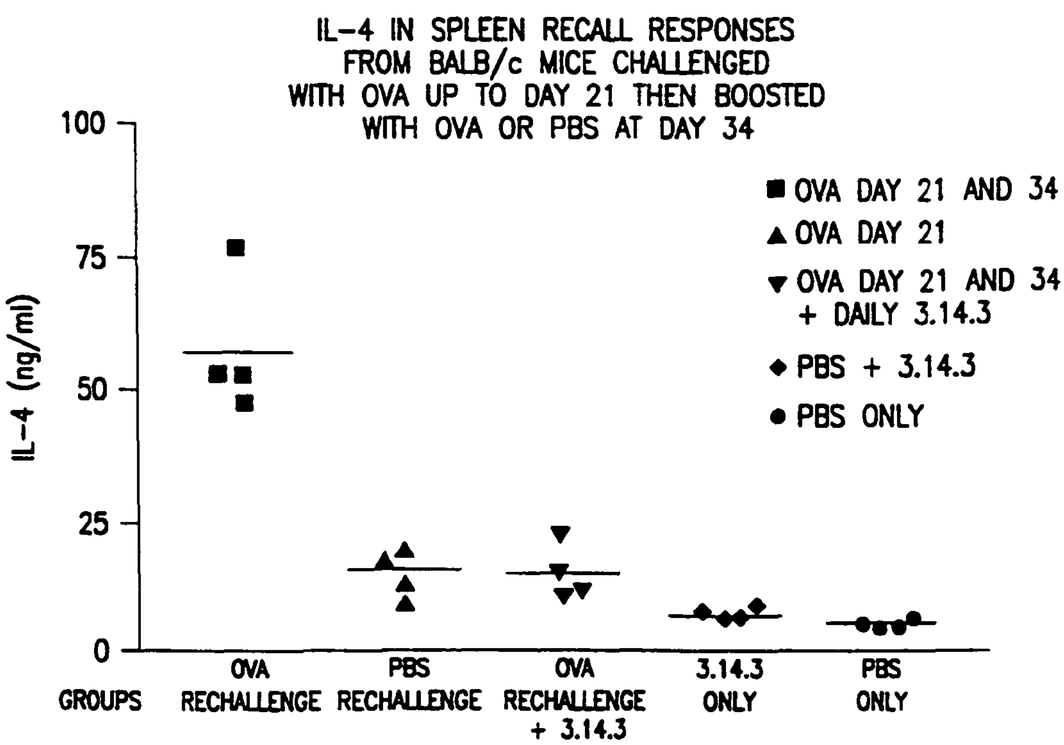


FIG. 30B

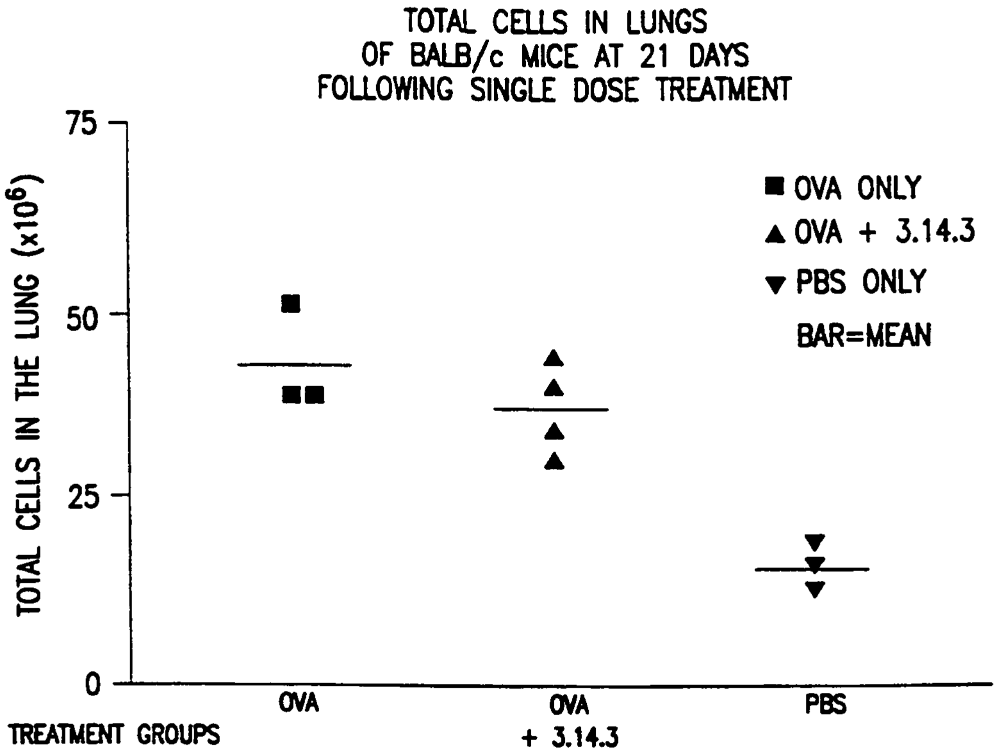
TOTAL CELLS IN LUNGS
OF BALB/c MICE AT 21 DAYS
FOLLOWING SINGLE DOSE TREATMENT
TOTAL CELLS IN THE LUNG (x10^6)
75
50
25
0
■ OVA ONLY
▲ OVA + 3.14.3
▼ PBS ONLY
BAR=MEAN
OVA
OVA + 3.14.3
PBS
TREATMENT GROUPS

FIG. 31

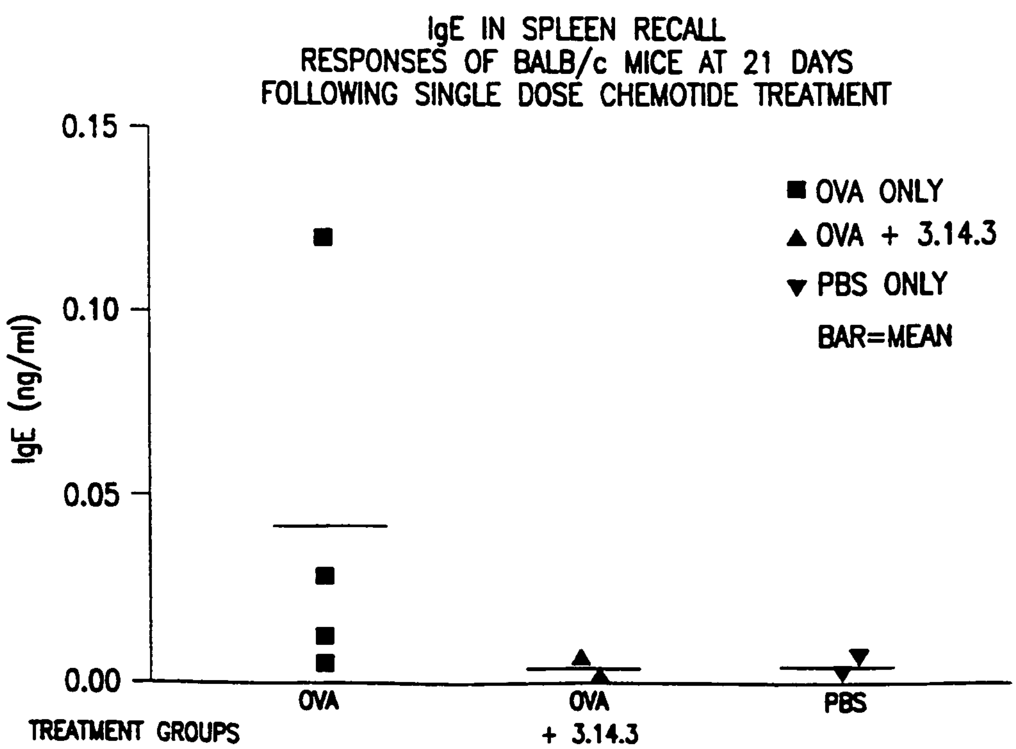
IgE IN SPLEEN RECALL
RESPONSES OF BALB/c MICE AT 21 DAYS
FOLLOWING SINGLE DOSE CHEMOTIDE TREATMENT
IgE (ng/ml)
0.15
0.10
0.05
0.00
■ OVA ONLY
▲ OVA + 3.14.3
▼ PBS ONLY
BAR=MEAN
OVA
OVA + 3.14.3
PBS
TREATMENT GROUPS

FIG. 32

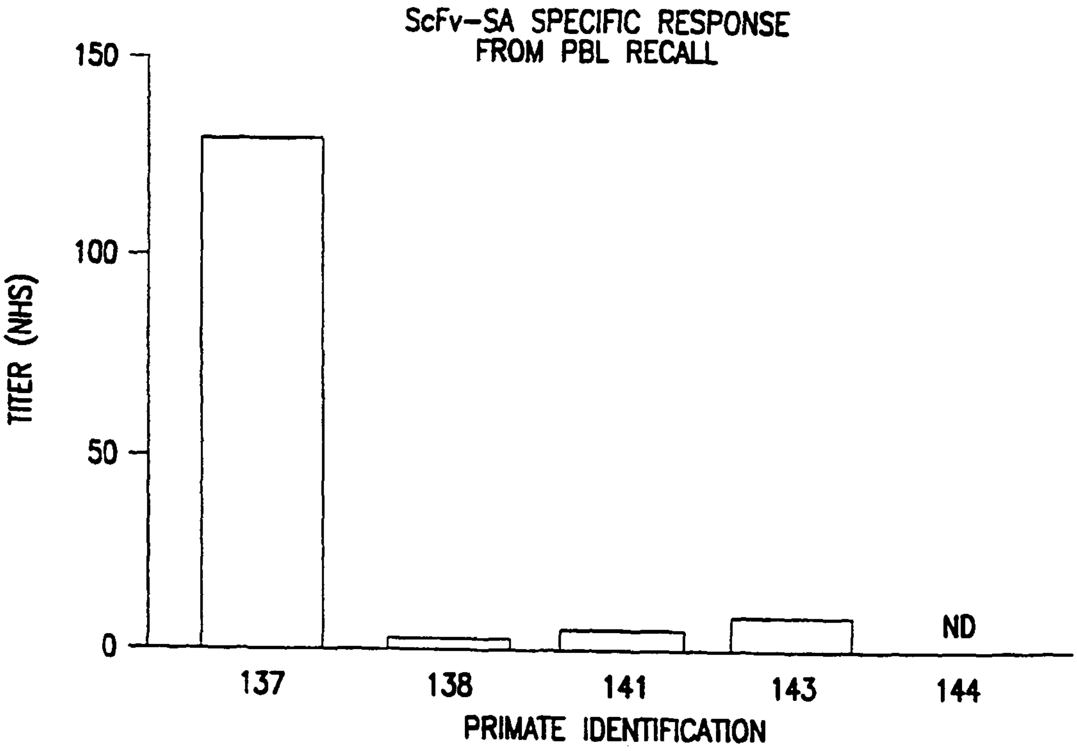


FIG. 33A

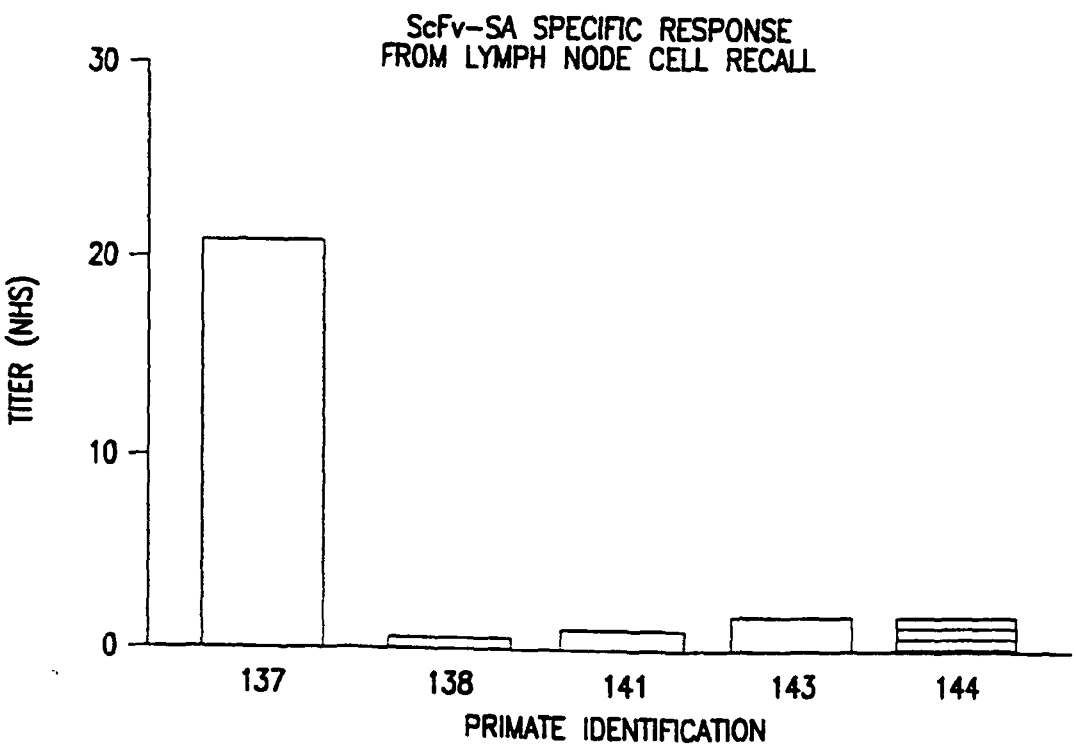


FIG. 33B

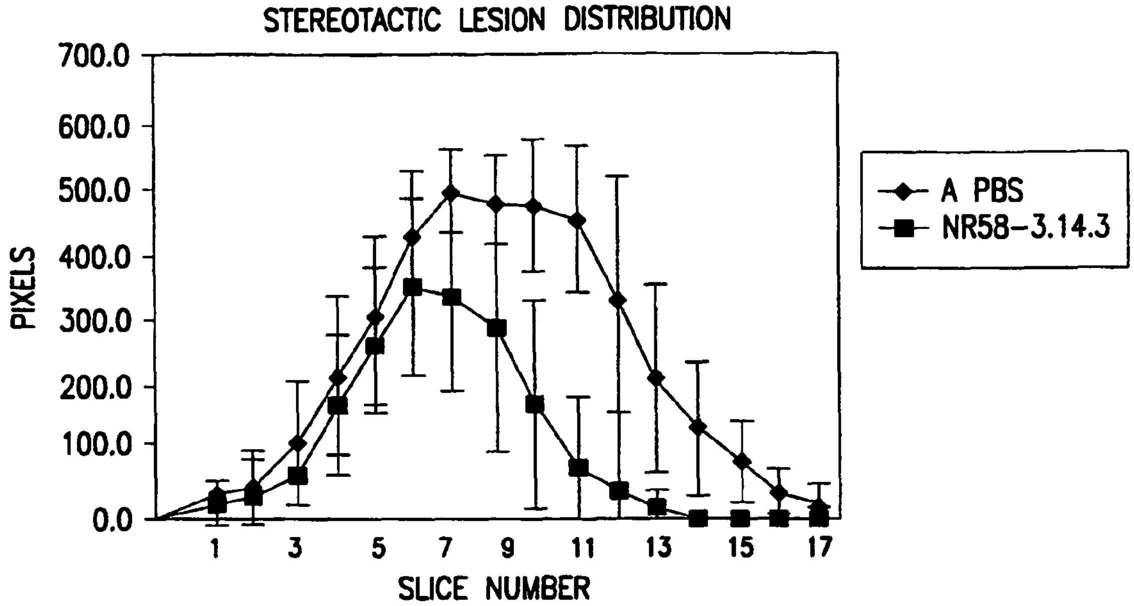


TOTAL LESION VOLUME AT 24 HOUR POST OCCLUSION

| GROUP | MEAN | VARIANCE | GROUP | GROUP |
|---|---|---|---|---|
| A | 2613 | 41761 | 204 | 19.9 |
| B | 126 | 26824 | 163 | 15.9 |

COMPARISON OF MEAN BY STUDENT'S UNPAIRED T-TEST A,B
MEAN DIFF. = 134.7
DF = 208
T = 5.2
P = <0.001

FIG. 34A

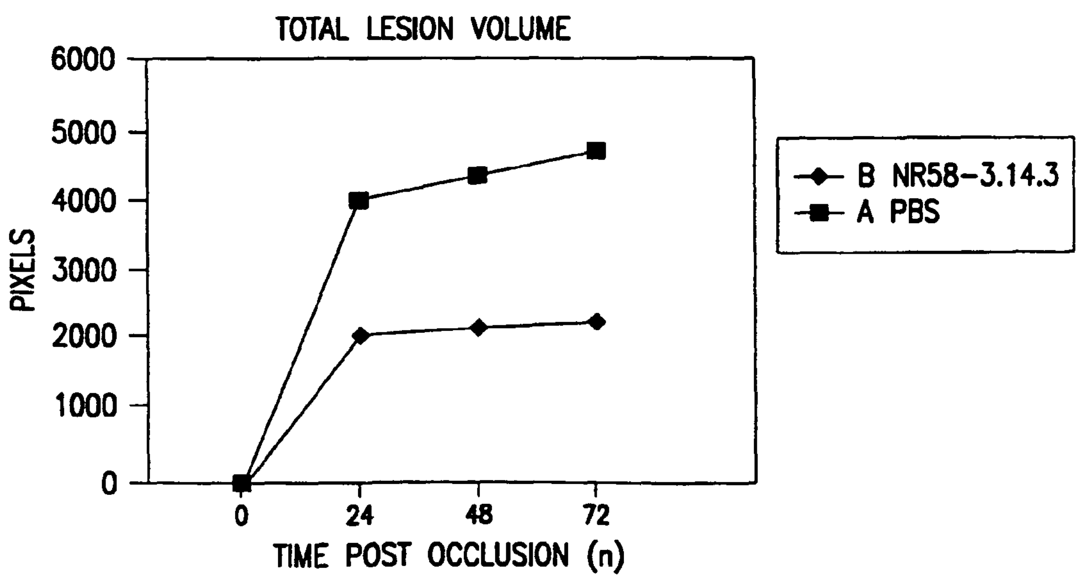


FIG. 34B

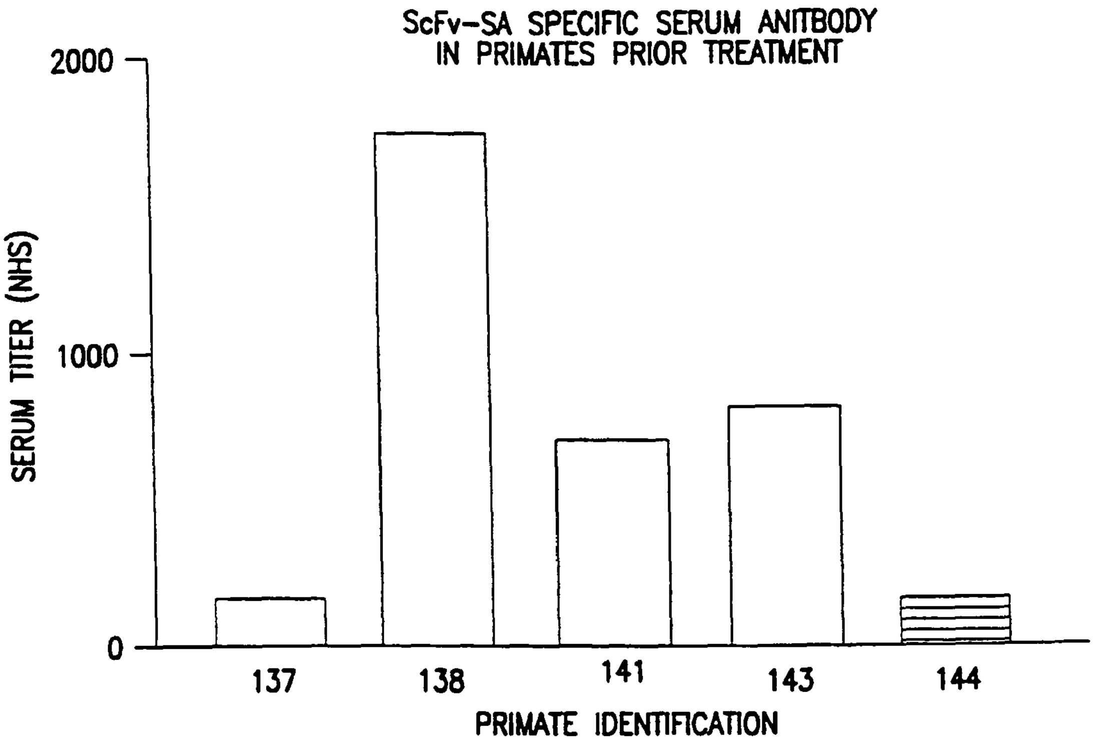


FIG. 34C

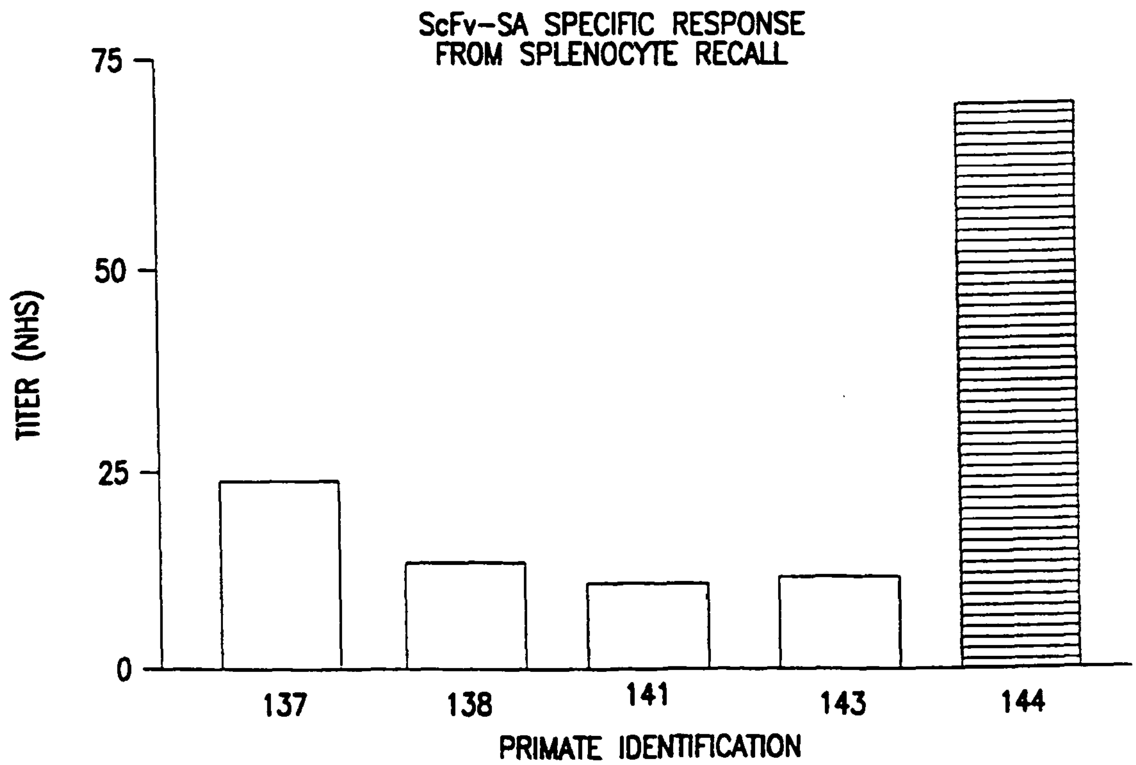


FIG. 34D

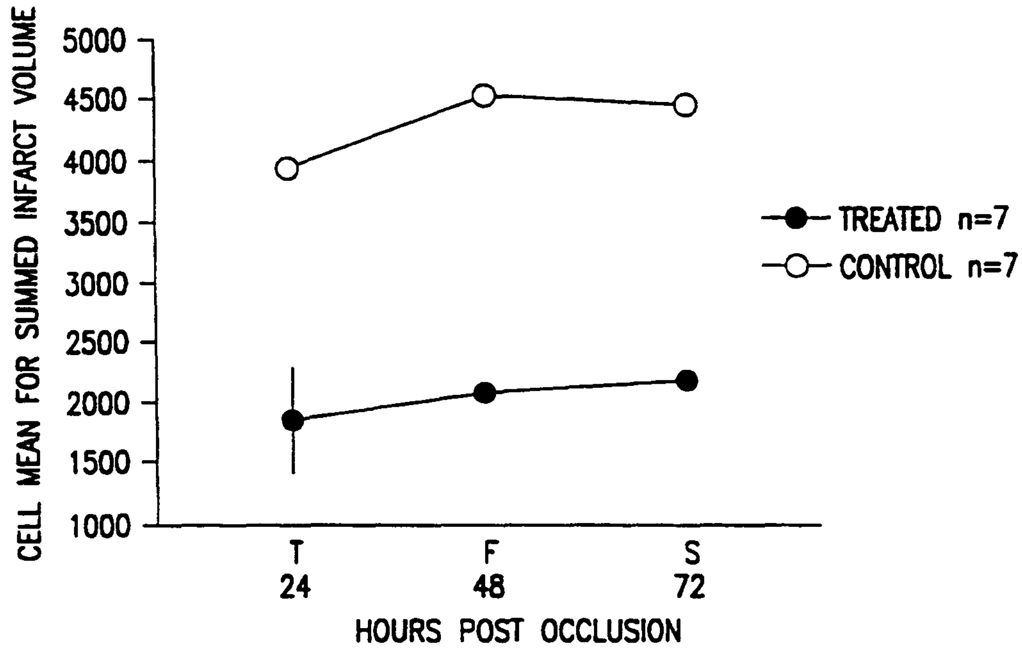


FIG. 34E

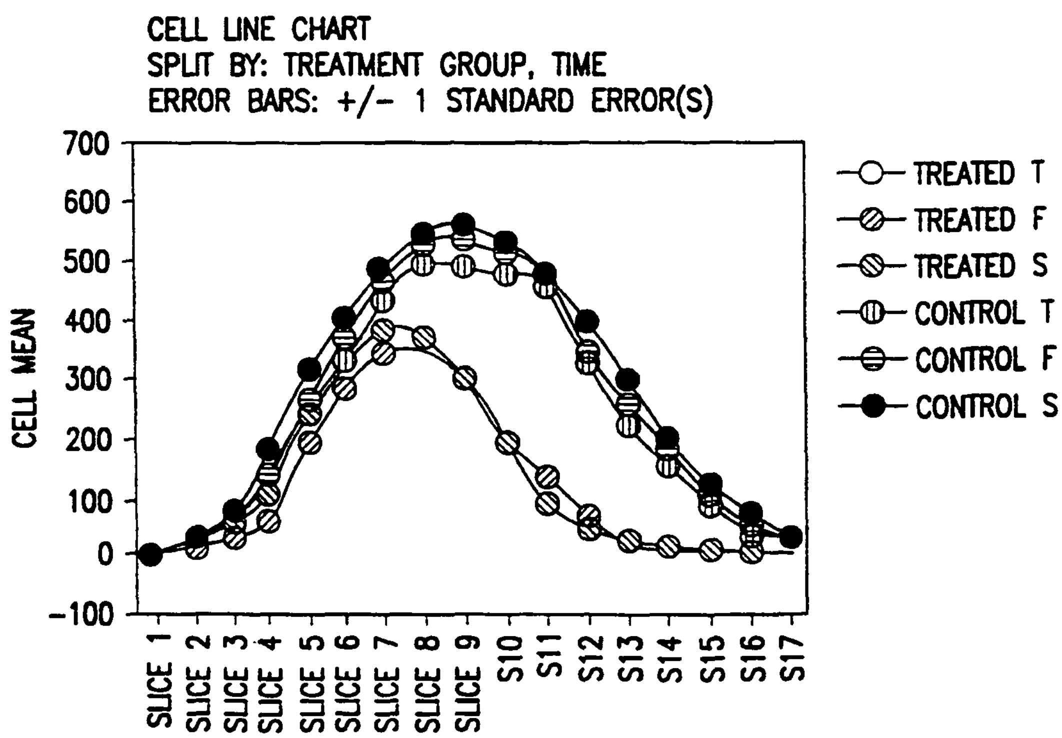


FIG. 34F

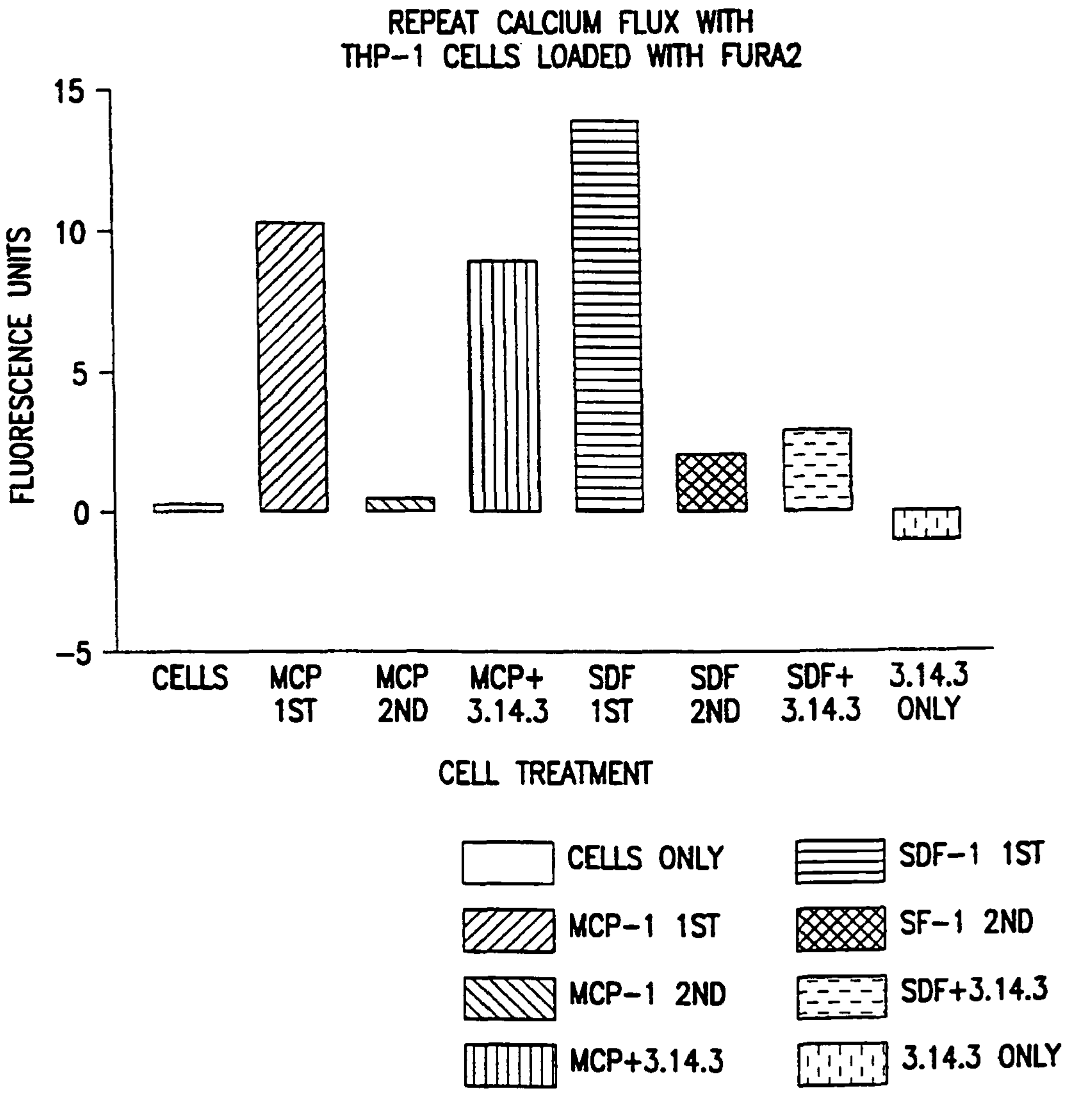
REPEAT CALCIUM FLUX WITH
THP-1 CELLS LOADED WITH FURA2
FLUORESCENCE UNITS
15
10
5
0
-5
CELLS
MCP 1ST
MCP 2ND
MCP+ 3.14.3
SDF 1ST
SDF 2ND
SDF+ 3.14.3
3.14.3 ONLY
CELL TREATMENT
CELLS ONLY
MCP-1 1ST
MCP-1 2ND
MCP+3.14.3
SDF-1 1ST
SF-1 2ND
SDF+3.14.3
3.14.3 ONLY

FIG. 35

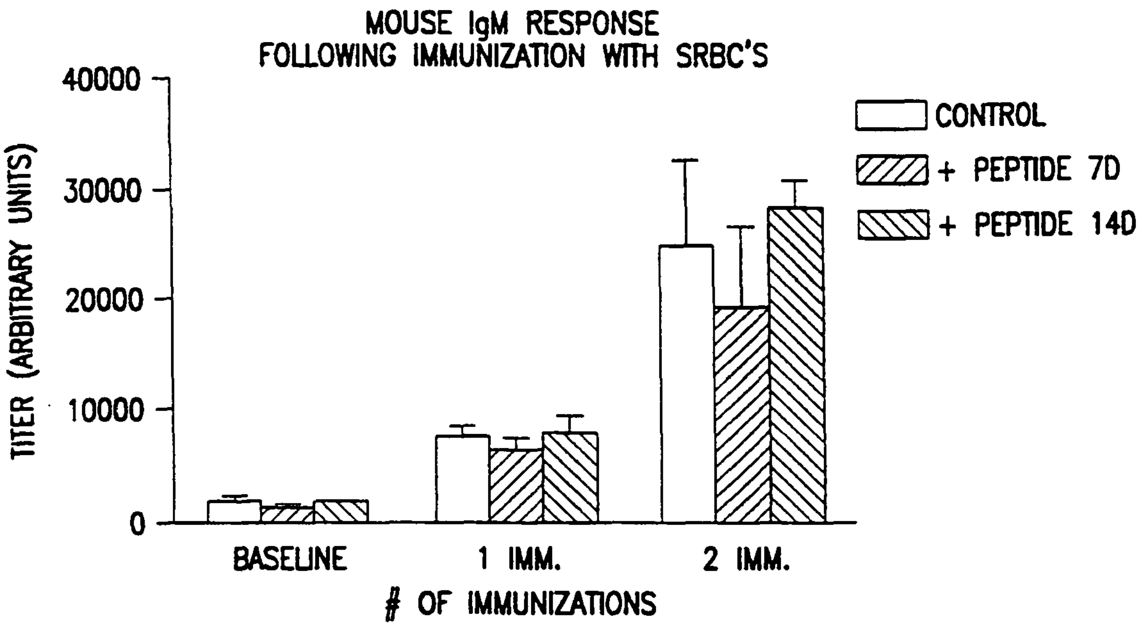


FIG. 36A

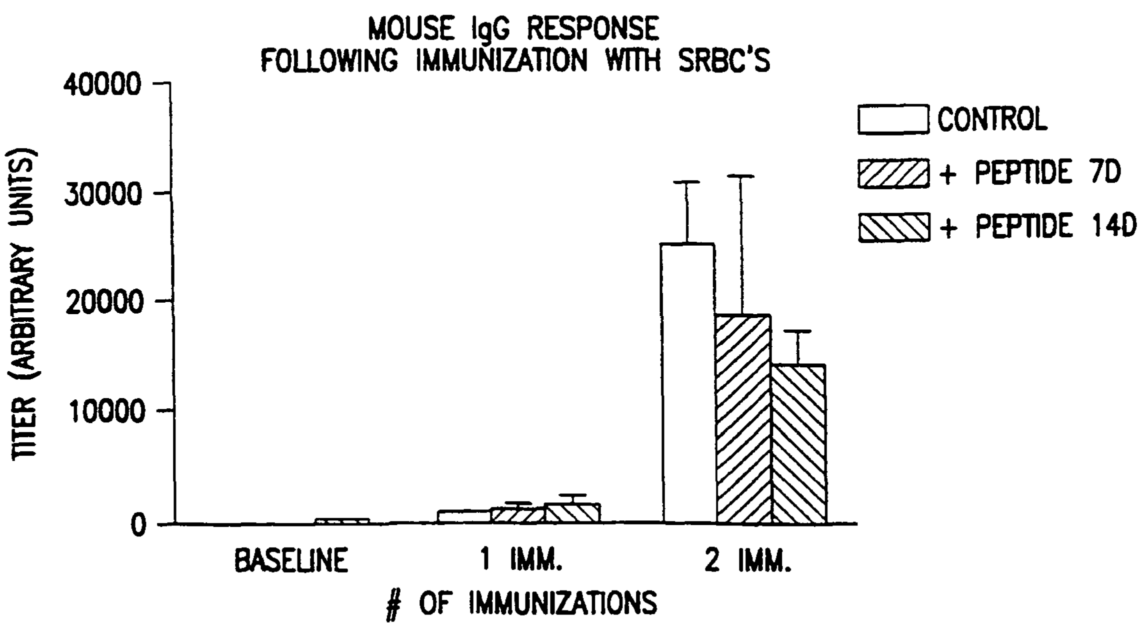


FIG. 36B

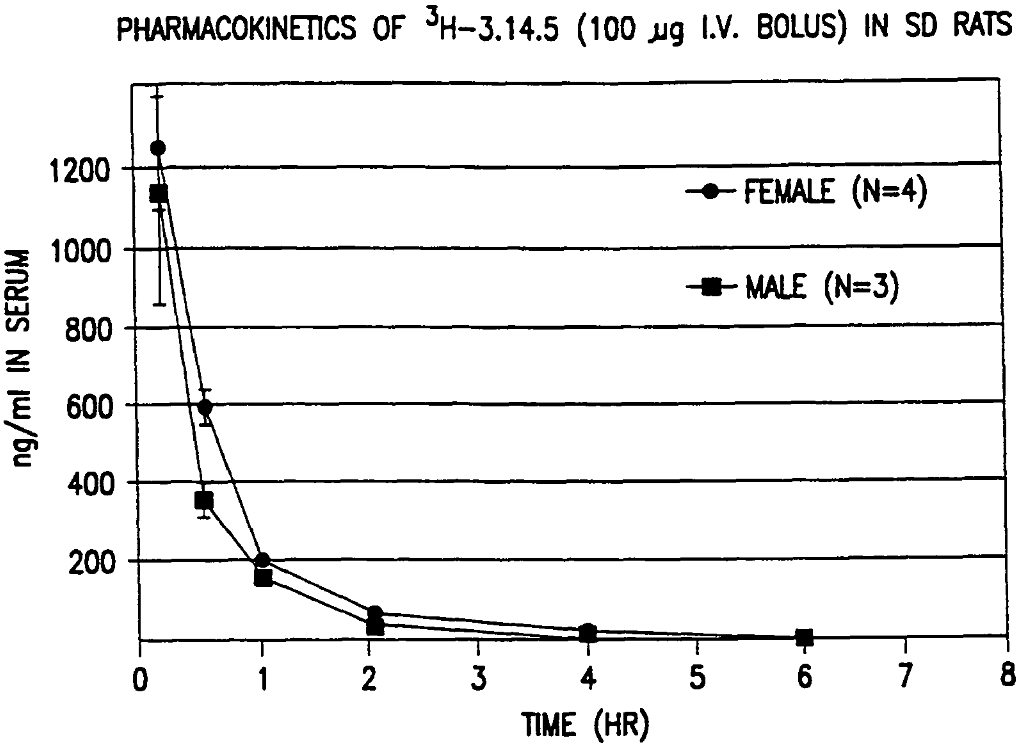


FIG. 37A

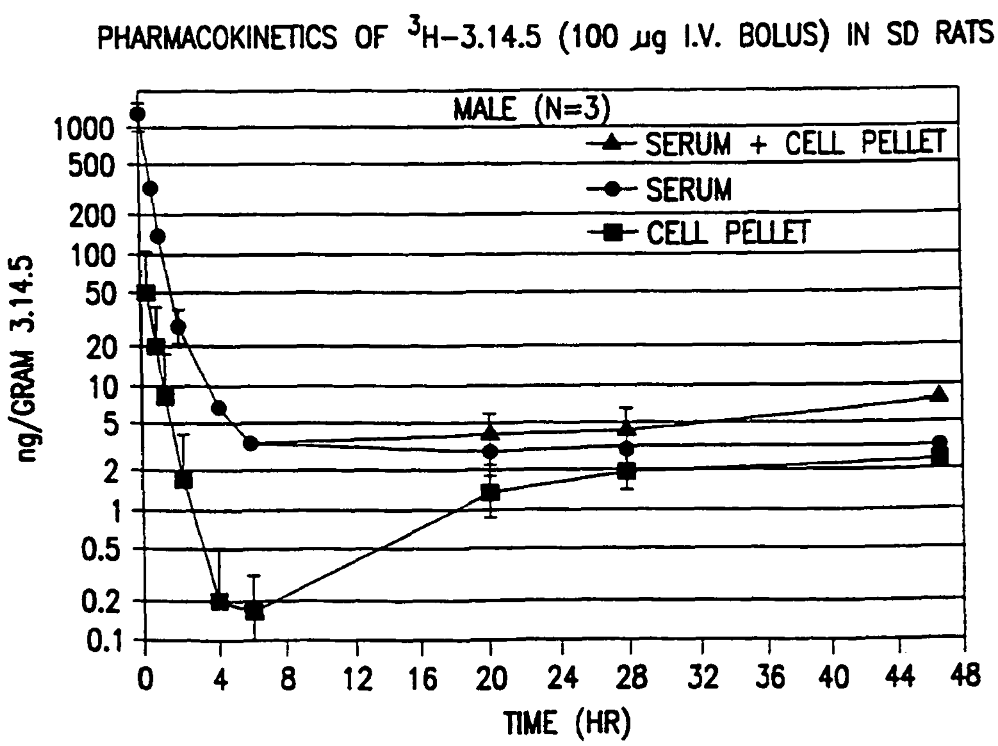


FIG. 37B

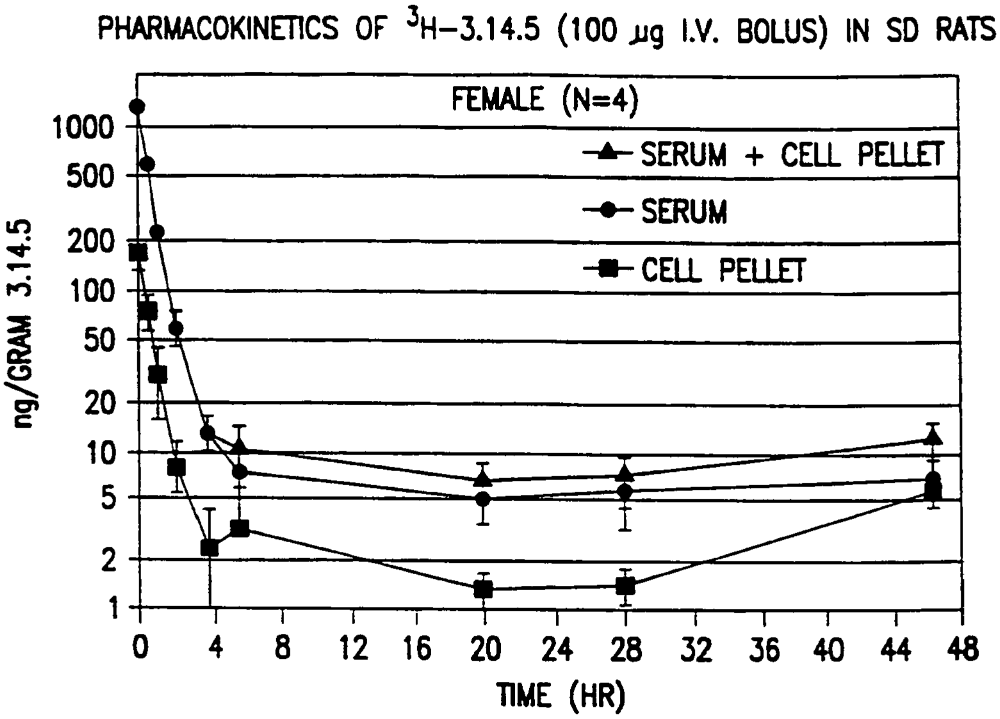


FIG. 37C

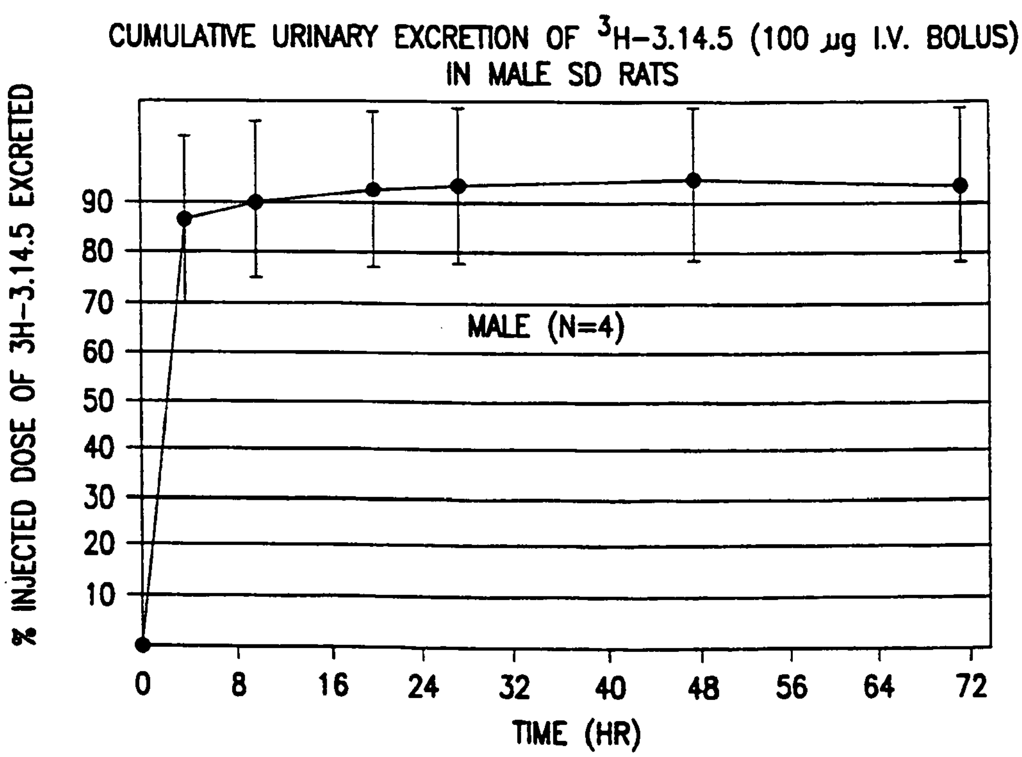


FIG. 37D

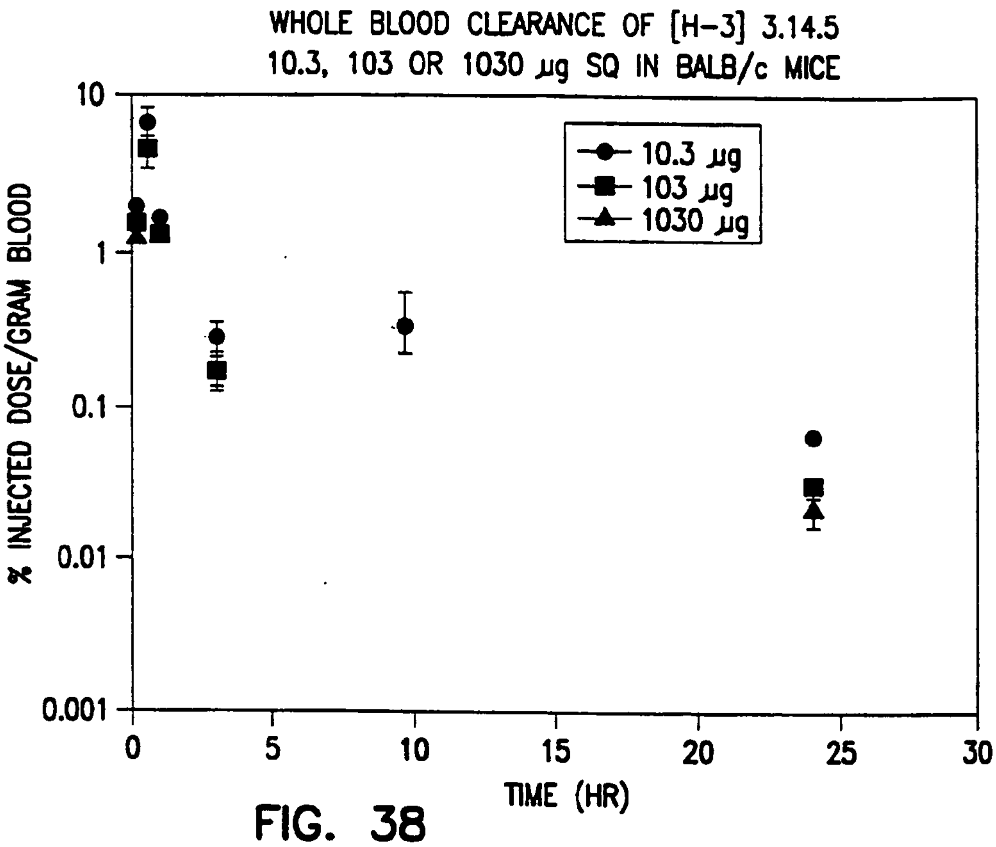


FIG. 38

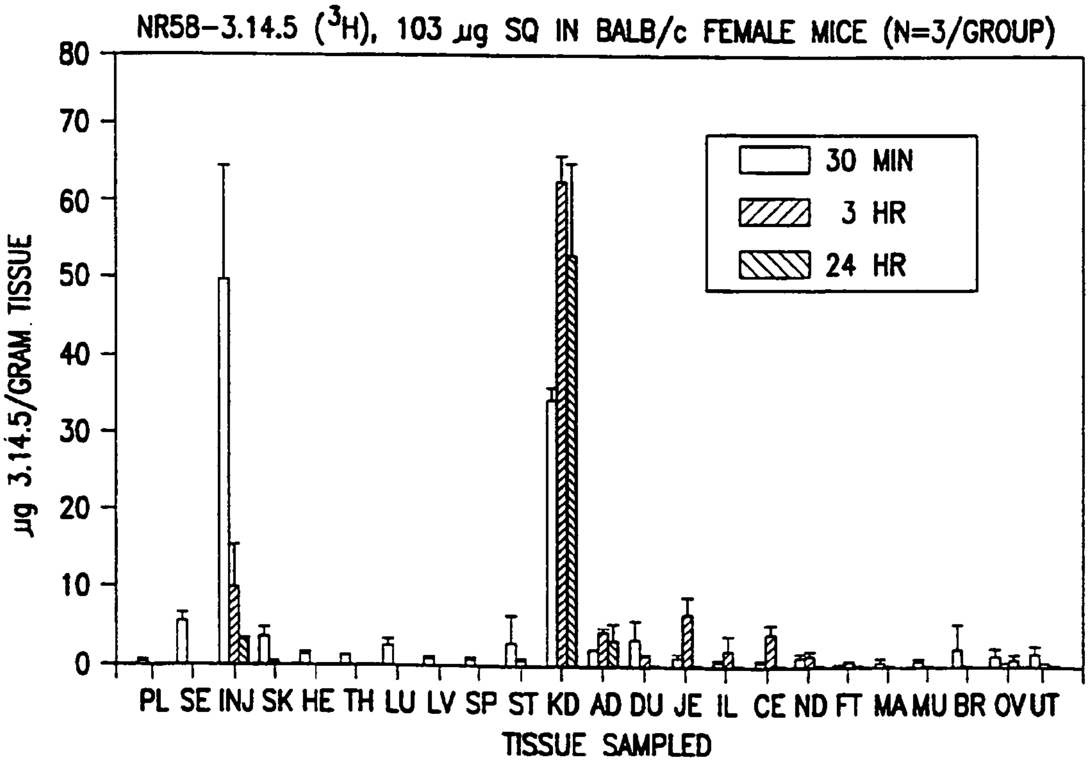


FIG. 39A

SUMMARY

SQ in BALB/c mice

| | Group 1-103 μg %ID/G 30 minute | SD | Group 2-103 μg %ID/G 30 minute | SD | Group 3-103 μg %ID/G 30 minute | SD | Group 4-103 μg %ID/G 3 Hour | SD | Group 5-103 μg %ID/G 3 Hour | SD | Group 6-103 μg %ID/G 3 Hour | SD | Group 7-103 μg %ID/G 24 Hour | SD | Group 8-103 μg %ID/G 24 Hour | SD | Group 9-1030 μg %ID/G 24 Hour | SD |
|---|---|---|---|---|---|---|---|---|---|---|---|---|---|---|---|---|---|---|
| Blood Pallet | 0.79 | 0.16 | 0.47 | 0.09 | 0.32 | 0.07 | 0.05 | 0.02 | 0.02 | 0.00 | 0.01 | 0.00 | 0.03 | 0.01 | 0.04 | 0.05 | 0.01 | 0.01 |
| Serum | 6.20 | 1.84 | 5.54 | 0.81 | 4.21 | 0.79 | 0.24 | 0.05 | 0.19 | 0.04 | 0.14 | 0.02 | 0.03 | 0.00 | 0.01 | 0.00 | 0.00 | 0.00 |
| Injection Site | 30.64 | 34.24 | 48.42 | 14.15 | 69.95 | 25.19 | 15.48 | 8.85 | 3.59 | 5.37 | 32.40 | 44.27 | ..54 | 1.45 | 3.38 | 0.27 | 2.54 | 1.44 |
| Skin | 4.64 | 1.99 | 3.50 | 1.28 | 1.55 | 0.62 | 0.35 | 0.08 | 0.48 | 0.21 | 0.29 | 0.03 | 0.19 | 0.03 | 0.14 | 0.03 | 0.12 | 0.01 |
| Heart | 1.64 | 0.59 | 1.51 | 0.39 | 1.03 | 0.19 | 0.18 | 0.04 | 0.10 | 0.01 | 0.08 | 0.02 | 0.09 | 0.03 | 0.08 | 0.02 | 0.03 | 0.01 |
| Thymus | 1.43 | 0.48 | 1.41 | 0.05 | 0.89 | 0.76 | 0.18 | 0.06 | 0.15 | 0.03 | 0.10 | 0.01 | 0.11 | 0.05 | 0.07 | 0.02 | 0.04 | 0.01 |
| Lung | 2.79 | 0.52 | 2.81 | 0.72 | 1.38 | 0.12 | 0.35 | 0.11 | 0.25 | 0.04 | 0.19 | 0.01 | 0.14 | 0.01 | 0.10 | 0.03 | 0.07 | 0.01 |
| Liver | 1.15 | 0.36 | 1.20 | 0.30 | 0.59 | 0.12 | 0.18 | 0.07 | 0.13 | 0.03 | 0.10 | 0.06 | 0.21 | 0.05 | 0.18 | 0.02 | 0.11 | 0.02 |
| Spleen | 1.16 | 0.29 | 0.30 | 0.25 | 0.59 | 0.02 | 0.22 | 0.04 | 0.20 | 0.04 | 0.16 | 0.07 | 0.21 | 0.04 | 0.17 | 0.02 | 0.11 | 0.01 |
| Stomach | 1.37 | 0.28 | 2.99 | 3.38 | 2.18 | 1.78 | 1.10 | 0.39 | 0.46 | 0.37 | 0.52 | 0.09 | 0.12 | 0.02 | 0.07 | 0.02 | 0.06 | 0.01 |
| Kidneys | 24.20 | 12.53 | 21.98 | 1.94 | 16.31 | 0.06 | 68.14 | 5.13 | 60.34 | 3.29 | 27.47 | 1.29 | 52.58 | 9.20 | 51.74 | 11.29 | 25.54 | 3.75 |
| Adrenals | 6.68 | 0.13 | 2.25 | 0.15 | 2.40 | 0.42 | 8.05 | 4.15 | 4.28 | 0.59 | 2.16 | 1.54 | 5.37 | 5.85 | 3.23 | 2.17 | 2.20 | 1.10 |
| Duodenum | 1.73 | 0.43 | 3.34 | 2.30 | 1.40 | 1.31 | 6.12 | 2.53 | 1.42 | 0.16 | 1.33 | .061 | 0.47 | 0.01 | 0.28 | 0.03 | 0.20 | 0.07 |
| Jejunum | 1.02 | 0.20 | 1.28 | 0.50 | 1.52 | 0.73 | 9.47 | 3.47 | 6.57 | 2.14 | 2.50 | 1.76 | 0.76 | 0.08 | 0.31 | 0.03 | 0.22 | 0.02 |
| Ileum | 1.02 | 0.23 | 0.76 | 0.27 | 0.63 | 0.12 | 4.34 | 3.13 | 2.22 | 1.67 | 6.37 | 5.94 | 0.37 | 0.14 | 0.21 | 0.03 | 0.15 | 0.05 |
| Cacum & Colon | 0.77 | 0.11 | 0.68 | 0.08 | 0.45 | 0.04 | 2.36 | 0.71 | 4.08 | 1.21 | 3.59 | 5.55 | 1.38 | 0.35 | 0.34 | 0.02 | 0.50 | 0.26 |
| Mesenteric Nodes | 1.02 | 0.74 | 1.30 | 0.48 | 0.54 | 0.46 | 2.18 | 1.83 | 1.42 | 0.64 | 1.83 | 1.70 | 0.37 | 0.14 | 0.15 | 0.04 | 0.10 | 0.07 |
| Abdominal Fat | 1.08 | 6.00 | 0.24 | 0.05 | 0.11 | 0.54 | 0.15 | 0.12 | 0.33 | 0.29 | 0.10 | 0.07 | 0.08 | 0.02 | 0.03 | 0.01 | 0.03 | 0.01 |
| Bone Marrow | 3.76 | 0.54 | 0.56 | 0.51 | 0.10 | 0.31 | 0.17 | 0.05 | 0.25 | 0.05 | 0.19 | 0.22 | 0.17 | 0.04 | 0.04 | 0.01 | 0.04 | 0.01 |
| Skeletal Muscle | 0.70 | 0.04 | 0.34 | 0.17 | 0.02 | 0.10 | 0.14 | 0.04 | 0.09 | 0.03 | 0.04 | 0.00 | 0.06 | 0.02 | 0.03 | 0.00 | 0.02 | 0.01 |
| Brain | 0.24 | 0.59 | 2.08 | 3.07 | 2.45 | 0.02 | 0.03 | 0.01 | 0.07 | 0.09 | 0.01 | 0.00 | 0.03 | 0.01 | 0.06 | 0.09 | 0.00 | 0.00 |
| Ovaries | 1.62 | 0.64 | 1.21 | 1.00 | 0.23 | 2.45 | 0.21 | 0.04 | 0.30 | 0.28 | 2.10 | 3.43 | 0.15 | 0.02 | 0.57 | 0.90 | 0.06 | 0.01 |
| Uterus | 1.82 | | 1.52 | 0.85 | | 0.23 | 0.41 | 0.27 | 0.33 | 0.04 | 0.11 | 0.09 | 0.53 | 0.65 | 0.10 | 0.09 | 0.11 | 0.02 |

FIG. 39B

| | | | | | | URINE | 35.16 | 62.55 | | 0.01 | 50.15 | 35.5538.03 | | | | | | |
|---|---|---|---|---|---|---|---|---|---|---|---|---|---|---|---|---|---|---|
| | Group 1-10 3 µg nMOL/ G 30 minute | SD | Group 2-103 µg nMOL/ G 30 minute | SD | Group 3-103 µg nMOL/ G 30 minute | SD | Group 4-103 µg nMOL/ G 3 Hour | SD | Group 5-103 µg nMOL/ G 3 Hour | SD | Group 6-103 µg nMOL/ G 3 Hour | SD | Group 7-103 µg nMOL/ G 24 Hour | SD | Group 8-103 µg nMOL/ G 24 Hour | SD | Group 9-1030 µg nMOL/ G 24 Hour | SD |
| Blood Pallet | 0 053 | 0.011 | 0.318 | 0.058 | 2.137 | 0 487 | 0 003 | 0.001 | 0.012 | 0.003 | 0 080 | 0.019 | 0 002 | 0.001 | 0 025 | 0.035 | 0 055 | 0 043 |
| Serum | 0 415 | 0.123 | 3.707 | 0.545 | 28.204 | 5.283 | 0.016 | 0.002 | 0.128 | 0.028 | 0.367 | 0.154 | 0.002 | 0.000 | 0.010 | 0 001 | 0 026 | 0 005 |
| Injection Site | 6 057 | 2.292 | 32.410 | 9.473 | 468.257 | 165.612 | 1.035 | 0.391 | 5.418 | 3.595 | 215.383 | 295.316 | 0.243 | 0.097 | 2.245 | 0.180 | 16.369 | 9 824 |
| Skin | 0.304 | 0.133 | 2.345 | 0.857 | 10.382 | 4.137 | 0.024 | 0.005 | 0.318 | 0.141 | 1.331 | 0.222 | 0.012 | 0.002 | 0 091 | 0.021 | 0 305 | 0 089 |
| Heart | 0.110 | 0.040 | 1.010 | 0.258 | 6.880 | 1.291 | 0.012 | 0.003 | 0.063 | 0.009 | 0.515 | 0.104 | 0.005 | 0.002 | 0.036 | 0.016 | 0 235 | 0.044 |
| Thymus | 0 035 | 0 032 | 0.342 | 0.034 | 5.974 | 5.055 | 0.012 | 0.002 | 0.107 | 0.019 | 0.539 | 0.075 | 0.007 | 0.003 | 0.049 | 0 015 | 0 249 | 0 056 |
| Lung | 0 187 | 0.035 | 1.882 | 0.481 | 13.271 | 0 784 | 0 024 | 0.007 | 0.177 | 0.027 | 1.276 | 0 059 | 0.008 | 0 000 | 0 065 | 0 022 | 0 483 | 0 076 |
| Liver | 0 077 | 0.024 | 0.800 | 0.204 | 3.975 | 0 820 | 0.012 | 0.005 | 0.128 | 0.021 | 0.887 | 0.419 | 0.014 | 0.004 | 0.121 | 0.014 | 0.727 | 0 125 |
| Spleen | 0 077 | 0.020 | 0.602 | 0.165 | 3.962 | 0 133 | 0.015 | 0.003 | 0.137 | 0.027 | 1 056 | 0.441 | 0.014 | 0.003 | 0.112 | 0.014 | 0 707 | 0 088 |
| Stomach | 0 082 | 0.019 | 0 002 | 2 247 | 14.820 | 11.889 | 0.074 | 0.025 | 0.510 | 0.249 | 3.483 | 0.572 | 0.005 | 0.001 | 0.048 | 0 012 | 0.429 | 0 035 |
| Kidneys | 1.620 | 0.839 | 22.075 | 1.237 | 102.503 | 0.426 | 4.628 | 6.410 | 40.513 | 2.205 | 183.392 | 8.628 | 3.519 | 0.616 | 34.637 | 7 560 | 171.609 | 25.195 |
| Adrenals | 0 447 | 0.009 | 1.503 | 0.303 | 18.102 | 2.827 | 0.539 | 0.278 | 2.865 | 0.394 | 14.485 | 10.964 | 0.383 | 0.392 | 2.162 | 1 453 | 14 762 | 7.371 |
| Duodenum | 0 116 | 0.029 | 2.256 | 1.542 | 11.388 | 8.787 | 0.409 | 0.169 | 0.349 | 0.109 | 5.590 | 4.103 | 0.032 | 0.001 | 0.187 | 0.018 | 1.346 | 0 494 |
| Jejunum | 0 053 | 0.013 | 0.360 | 0.332 | 10.164 | 4.872 | 0.634 | 0.232 | 4.385 | 1.435 | 17.404 | 11.755 | 0.050 | 0.005 | 0.210 | 0.022 | 1 486 | 0 151 |
| Ileum | 0 068 | 0.015 | 0.500 | 0.183 | 4.203 | 0 826 | 0.291 | 0.210 | 1.439 | 1.118 | 42.809 | 39.756 | 0.025 | 0.009 | 0.139 | 0.017 | 1 054 | 0 342 |
| Cacum & Colon | 0 053 | 0 007 | 0.458 | 0.056 | 3.027 | 0 261 | 0.158 | 0.048 | 2.718 | 0 808 | 84 392 | 37.151 | 0.083 | 0.024 | 0.228 | 0.014 | 3 361 | 1 760 |
| Mesenteric Nodes | 0 089 | 0.049 | 0.869 | 0.308 | 6 755 | 3 101 | 0 146 | 0.122 | 0.351 | 0.429 | 10.390 | 11.393 | 0.025 | 0 009 | 0.102 | 0 025 | 0 681 | 0.502 |
| Abdominal Fat | 0.072 | 0.047 | 0.163 | 0 034 | 6.027 | 3.647 | 0.00 | 0.008 | 0.221 | 0.195 | 0 850 | 0.438 | 0.004 | 0.001 | 0.020 | 0 005 | 0 179 | 0 092 |
| Bone Marrow | 0 252 | 0 402 | 0 373 | 0.343 | 2.223 | 2 095 | 0 011 | 0.004 | 0.130 | 0.043 | 1 253 | 1 485 | 0.011 | 0.002 | 0 024 | 0 004 | 0.276 | 0 060 |
| Skeletal Muscle | 0 047 | 0 036 | 0.559 | 0 116 | 3 587 | 0 700 | 0 009 | 0.003 | 0 060 | 0 019 | 0.294 | 0.015 | 0.002 | 0.001 | 0 022 | 0 003 | 0 107 | 0 049 |
| Brain | 0 016 | 0 002 | 1.391 | 2 057 | 1 000 | 0 123 | 0 002 | 0 001 | 0.049 | 0 059 | 0 082 | 0.021 | 0 002 | 0.001 | 0.042 | 0 060 | 0 030 | 0 006 |
| Ovaries | 0 109 | 0 039 | 0.305 | 0.668 | 15.333 | 16.491 | 0.014 | 0.003 | 0.201 | 0.185 | 14.059 | 22.958 | 0.010 | 0 001 | 0.381 | 0 605 | 0 519 | 0 070 |
| Uterus | 0 129 | 0 043 | 1.017 | 0.570 | 7.873 | 1 511 | 0 027 | 0 018 | 0.222 | 0.025 | 0 746 | 0.572 | 0.036 | 0.043 | 0.070 | 0.059 | 0 727 | 0 148 |

FIG. 39B CONTINUED

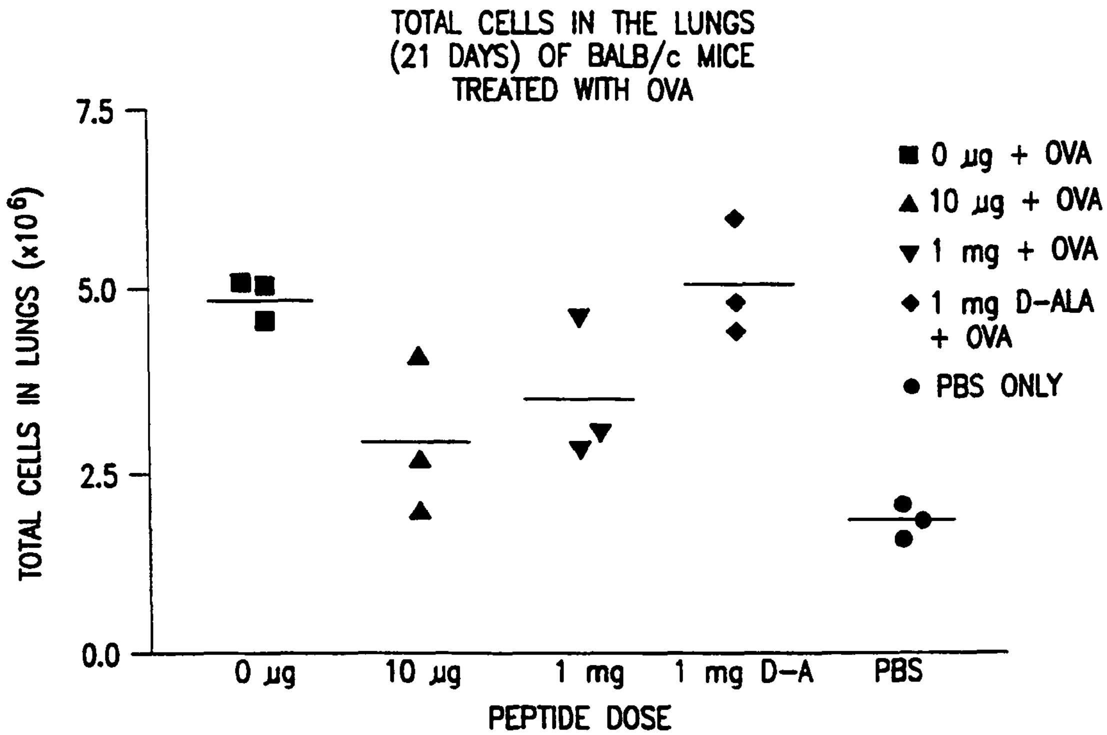


FIG. 40A

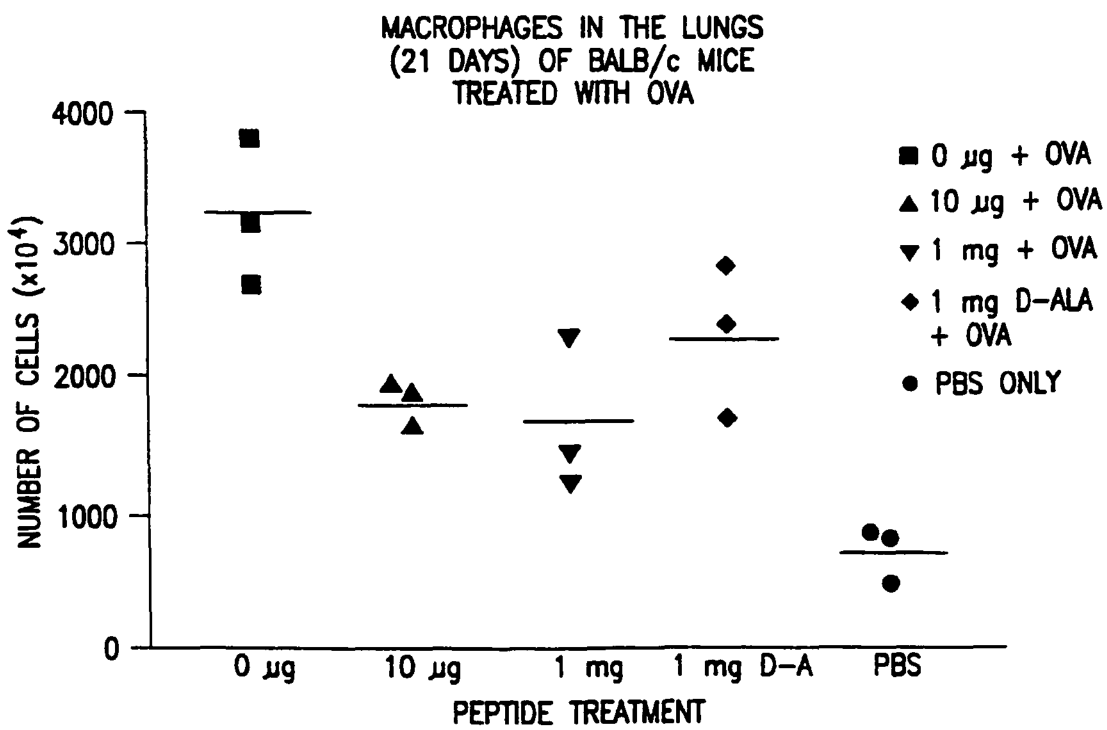


CELL NUMBER = PRODUCT OF TOTAL CELL POPULATION FROM LUNG X% OF CELL POPULATION FROM FACS

FIG. 40B

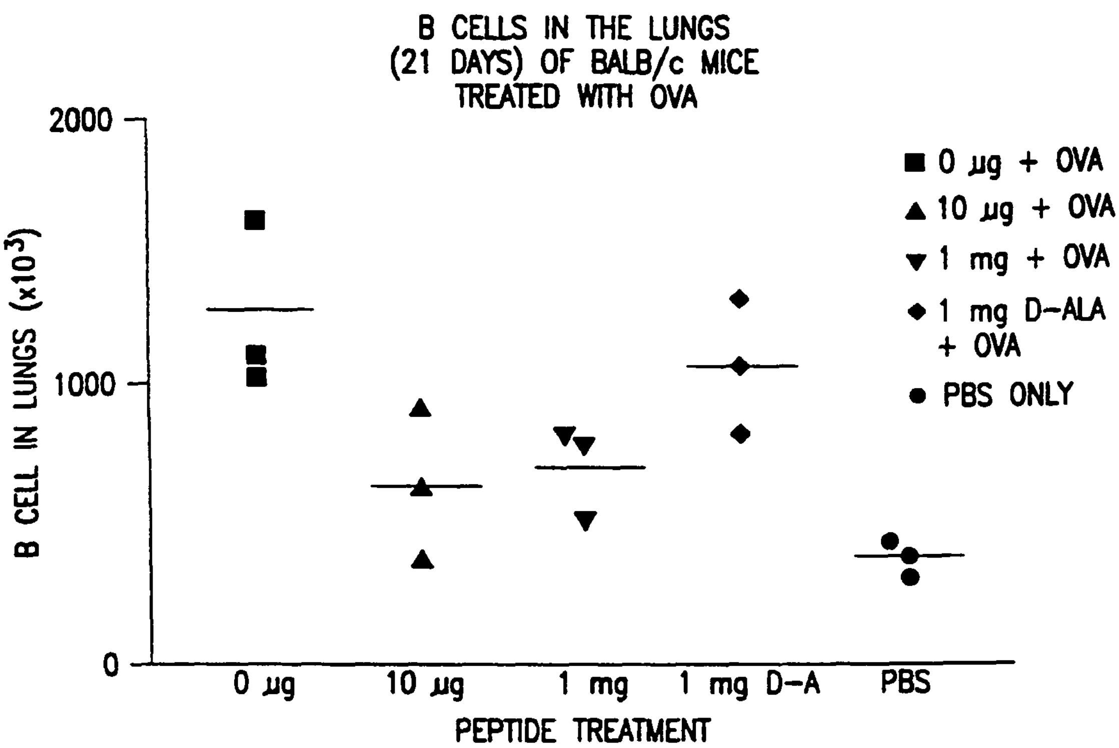
B CELLS IN THE LUNGS
(21 DAYS) OF BALB/c MICE
TREATED WITH OVA
2000
1000
0
B CELL IN LUNGS ($x10^3$)
0 µg + OVA
10 µg + OVA
1 mg + OVA
1 mg D-ALA
+ OVA
PBS ONLY
0 µg
10 µg
1 mg
1 mg D-A
PBS
PEPTIDE TREATMENT

FIG. 40C

CULTURED SERUM CELLS STIMULATED
WITH ANTIGEN (OVALBUMIN)

| | GROUP | MOUSE | 1ST ASSAY IL-4, pg/ml | AVG. | 2ND ASSAY IL-4, pg/ml | AVG. |
|---|---|---|---|---|---|---|
| 1 | POSITIVE CONTROL | m1 | 619 | 474 | 730 | 427 |
| | | m2 | 330 | | 123 | |
| | | m3 | CONTAMINATED | | CONTAMINATED | |
| | | | 204.3538598 | | | |
| 2 | 10 ug NR58-3.14.3 | m1 | CONTAMINATED | 115 | CONTAMINATED | 91 |
| | | m2 | 144 | | 104 | |
| | | m3 | 86 | | 78 | |
| | | | 41.01219331 | | | |
| 3 | 10 mg NR58-3.14.3 | m1 | 154 | 67 | 82 | 89 |
| | | m2 | 33 | | 135 | |
| | | m3 | 13 | | 51 | |
| | | | 85.55992052 | | | |
| 4 | 10 mg NR58-3.14.4 | m1 | 175 | 191 | 76 | 158 |
| | | m2 | 198 | | 316 | |
| | | m3 | 200 | | 81 | |
| | | | 16.26346697 | | | |
| 5 | NEGATIVE CONTROL | m1 | 19 | 9 | 9 | 9.5 |
| | | m2 | 0 | | 0 | |
| | | m3 | 8 | | 10 | |

| | | |
|---|---|---|
| POSITIVE CONTROL | 474 | 427 |
| LOW DOSE ACTIVE PEPTIDE | 115 | 91 |
| HIGH DOSE ACTIVE PEPTIDE | 67 | 89 |
| HIGH DOSE INACTIVE PEPTIDE | 191 | 158 |
| NEGATIVE CONTROL | 9 | 9 |

FIG. 40D

| | SERUM IgE GROUP | MOUSE | SERUM IgE ng/ml | BAL THROMBOXANE pg/ml | BAL LTB4 pg/ml | BAL PGE2 pg/ml |
|---|---|---|---|---|---|---|
| 1 | POSITIVE CONTROL-OVA | M1 | 160 | 12.7 | 2.6 | 93.1 |
| | | M2 | 145 | 10.3 | 2.81 | 25.4 |
| | | M3 | 180 | 34 | 29 | 116.8 |
| | | AVE | 161.6666667 | 19 | 11.47 | 78.43333333 |
| | | STDEV | 17.55942292 | 13.04568894 | 15.18178843 | 47.43230264 |
| 2 | 10 ug NR58-3.14.3 | M1 | 80 | 19.6 | 4.9 | 2.7 |
| | | M2 | 74 | 6.85 | 1.6 | 21.2 |
| | | M3 | 115 | 17.1 | 4.6 | 31.7 |
| | | AVE | 89.66666667 | 14.51666667 | 3.666666667 | 18.53333333 |
| | | STDEV | 22.14347157 | 6.75617002 | 1.800925688 | 14.68275633 |
| 3 | 1 mg NR58-3.14.3 | M1 | 65 | 5 | 4 | 109 |
| | | M2 | 98 | 8.1 | 1.1 | 11.7 |
| | | M3 | 38 | 4.7 | 4.8 | 196 |
| | | AVE | 67 | 5.933333333 | 3.3 | 55.33523484 |
| | | STDEV | 30.0499584 | 1.882374387 | 1.946792233 | 87.67841161 |
| 4 | 1 mg NR58-3.14.4 | M1 | 132 | 4.1 | 8.7 | 83 |
| | | M2 | 152 | 2.5 | 3.5 | 1.3 |
| | | M3 | 168 | 1.7 | 1.3 | 2 |
| | | AVE | 150.6666667 | 2.766666667 | 4.5 | 28.76666667 |
| | | STDEV | 18.03699901 | 1.222020185 | 3.8 | 46.96874848 |
| 5 | NEGATIVE CONTROL-PB | M1 | 14 | 6.9 | 1.3 | 0.1 |
| | | M2 | 12 | 7.2 | 1.5 | 1.1 |
| | | M3 | 3 | 8 | 0.4 | 0.1 |
| | | AVE | 7.366666667 | 7.366666667 | 1.066666667 | 0.433333333 |
| | | STDEV | 6.859465277 | 0.56862407 | 0.585946528 | 0.577350269 |

FIG. 40E

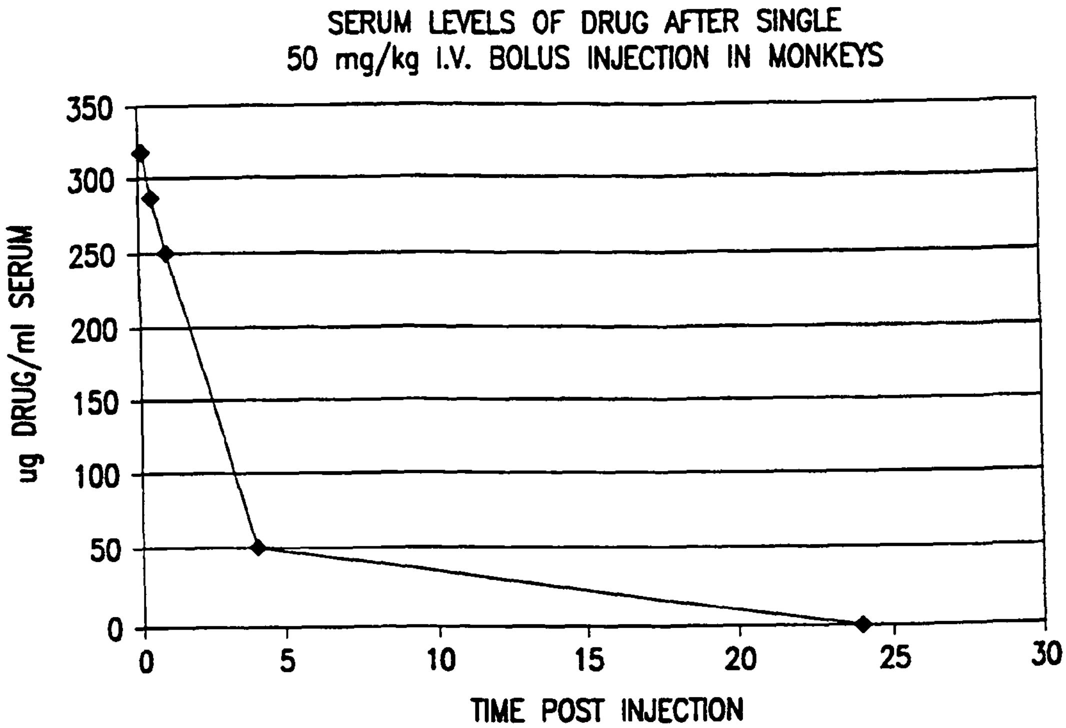
SERUM LEVELS OF DRUG AFTER SINGLE
50 mg/kg I.V. BOLUS INJECTION IN MONKEYS
ug DRUG/ml SERUM
350
300
250
200
150
100
50
0
0
5
10
15
20
25
30
TIME POST INJECTION

FIG. 41

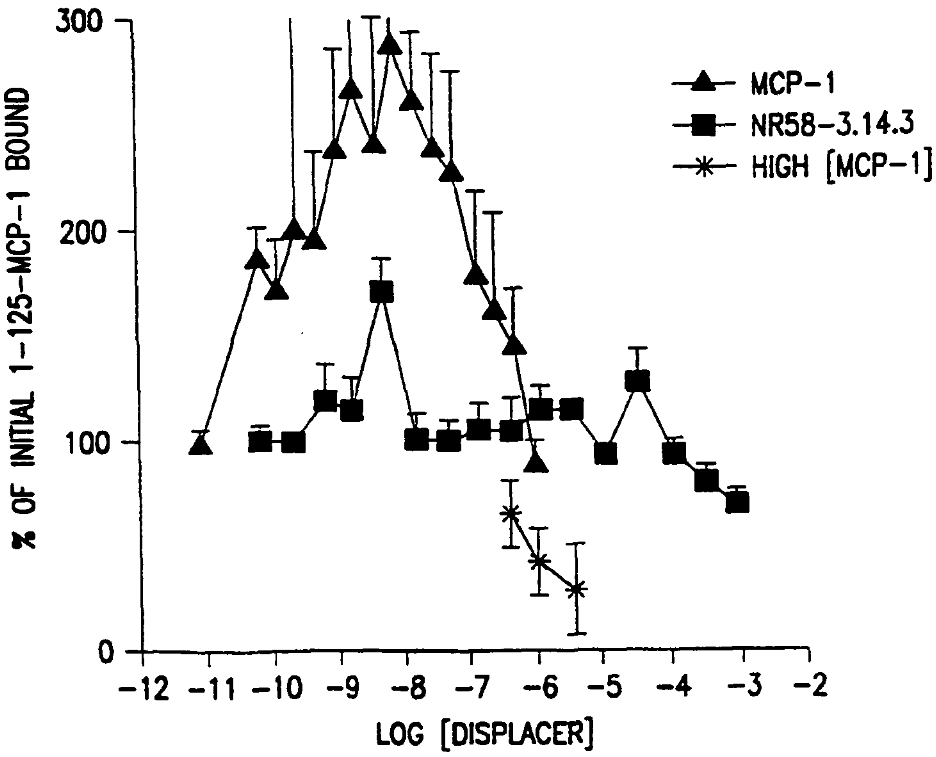
% OF INITIAL 1-125-MCP-1 BOUND
300
200
100
0
MCP-1
NR58-3.14.3
HIGH [MCP-1]
-12 -11 -10 -9 -8 -7 -6 -5 -4 -3 -2
LOG [DISPLACER]

FIG. 42

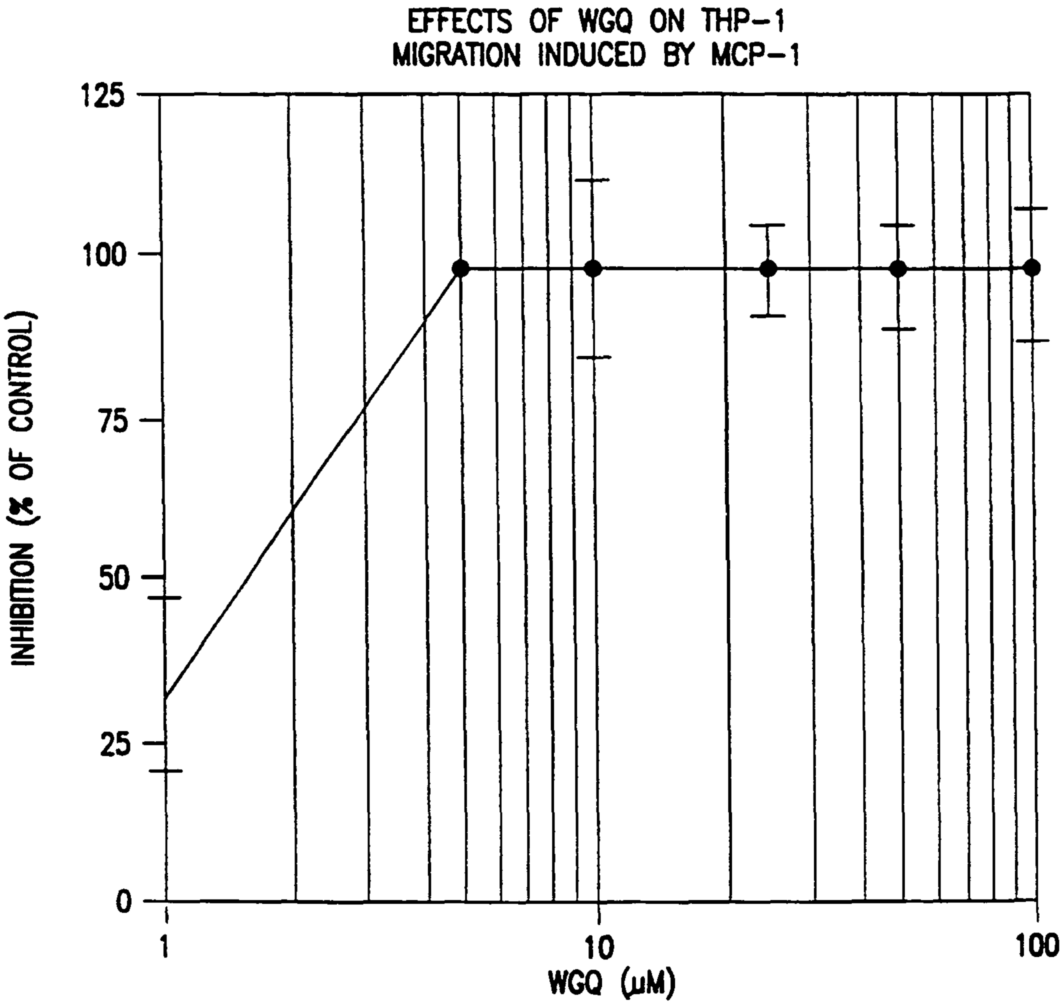


ED-50 = ~1.75 µM

FIG. 43

| | Glutamine | Glutamide | Benzyl-glutamide | Pyroglutamate |
|---|---|---|---|---|
| N-undec-10-enoyl | 58,1<br>>100% | 58,2<br>>100% | 58,3<br>90 ± 2% | 58,4<br>>100% |
| BOC | 58,5<br>>100% | 58,6<br>80 ± 10% | 58,7<br>34 ± 17% | 58,8<br>Not made |
| Benzoyl | 58,9<br>21 ± 13% | 58,10<br>28 ± 13% | 58,11<br>31 ± 17% | 58,12<br>39 ± 4% |
| -Glycylglutamine | 58,13<br>31 ± 35% | 58,14<br>36 ± 4% | 58,15<br>83 ± 2% | |
| | N-tert<br>Butylcarbonyl | N-Benzoyl | N-Z | |

FIG. 44

| | Glutamine | Glutamide | Benzyl-glutamide | Pyroglutamate |
|---|---|---|---|---|
| N-undec-10-enoyl | 58,1<br>13 µM | 58,2<br>15 µM | 58,3<br>40 µM | 58,4<br><100nM |
| BOC | 58,5<br>5-20 µM | 58,6<br>45 µM | 58,7<br>N/D | 58,8<br>Not made |
| Benzoyl | 58,9<br>N/D | 58,10<br>N/C | 58,11<br>N/D | 58,12<br>N/D |
| -Glycylglutamine | 58,13<br>N/D | 58,14<br>N/D | 58,15<br>38 µM | |
| | N-tert<br>Butylcarbonyl | N-Benzoyl | N-Z | |

FIG. 45

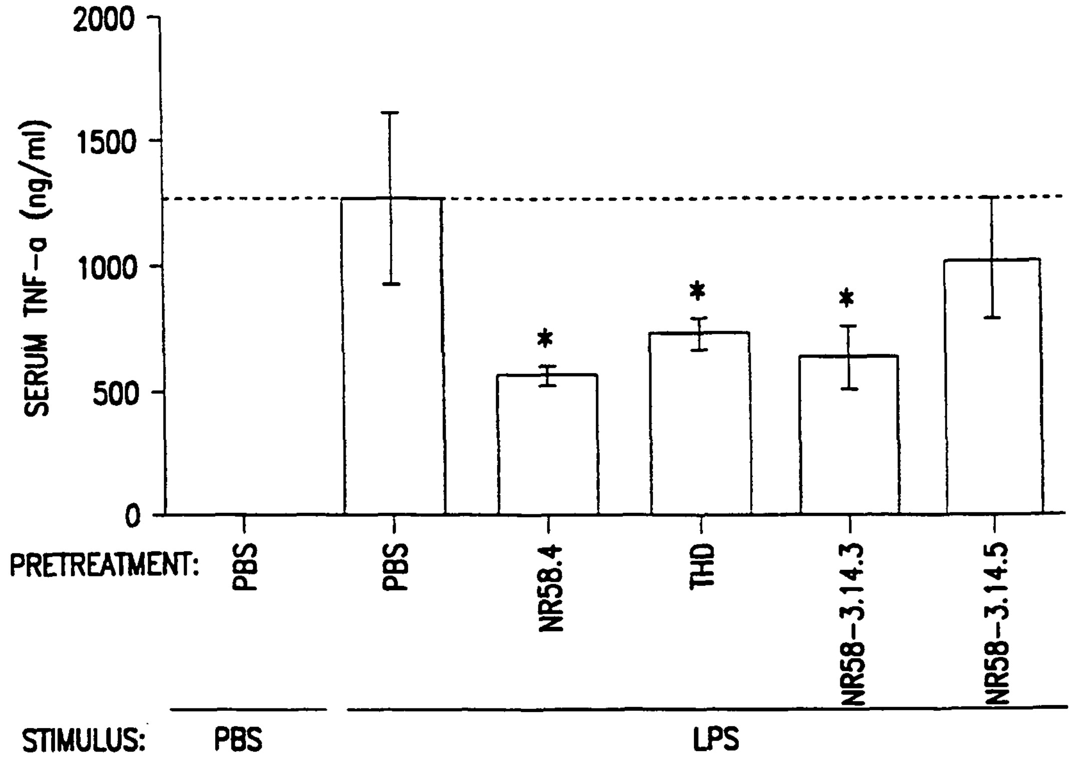


* p<0.05; MANN-WHITNEY U-TEST VERSUS PBS/LPS (n=6 PER GROUP)

FIG. 46

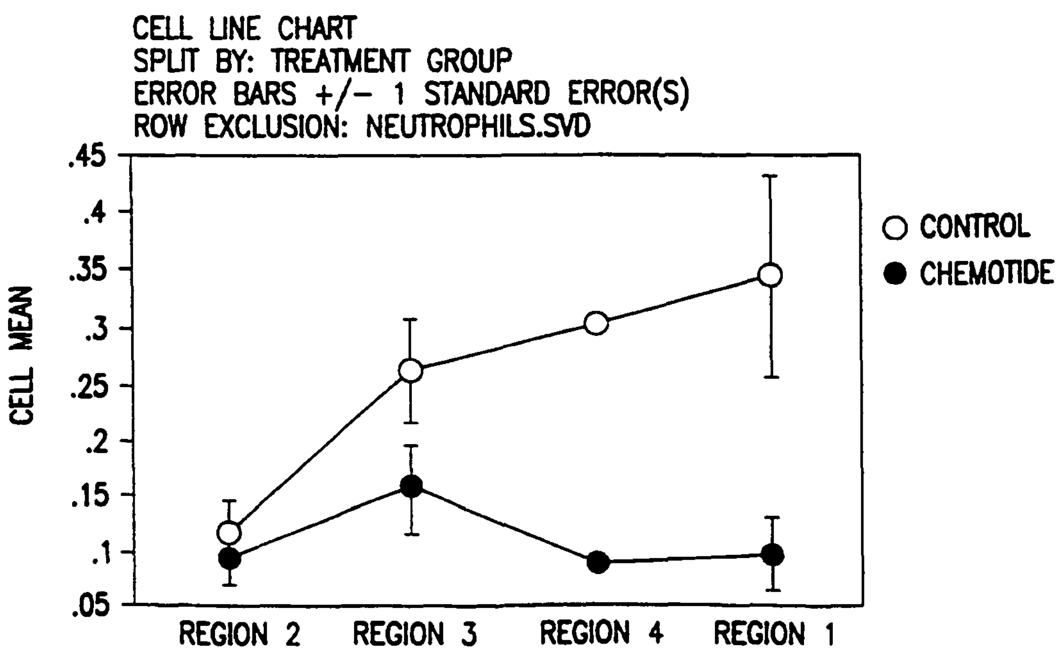


FIG. 47A

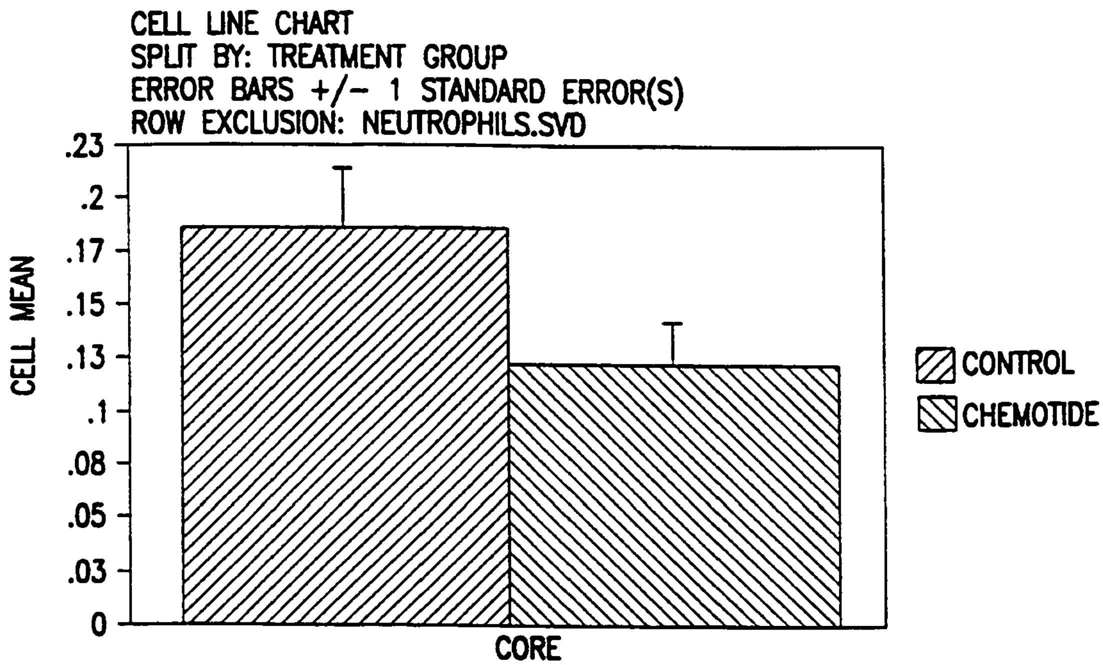


FIG. 47B

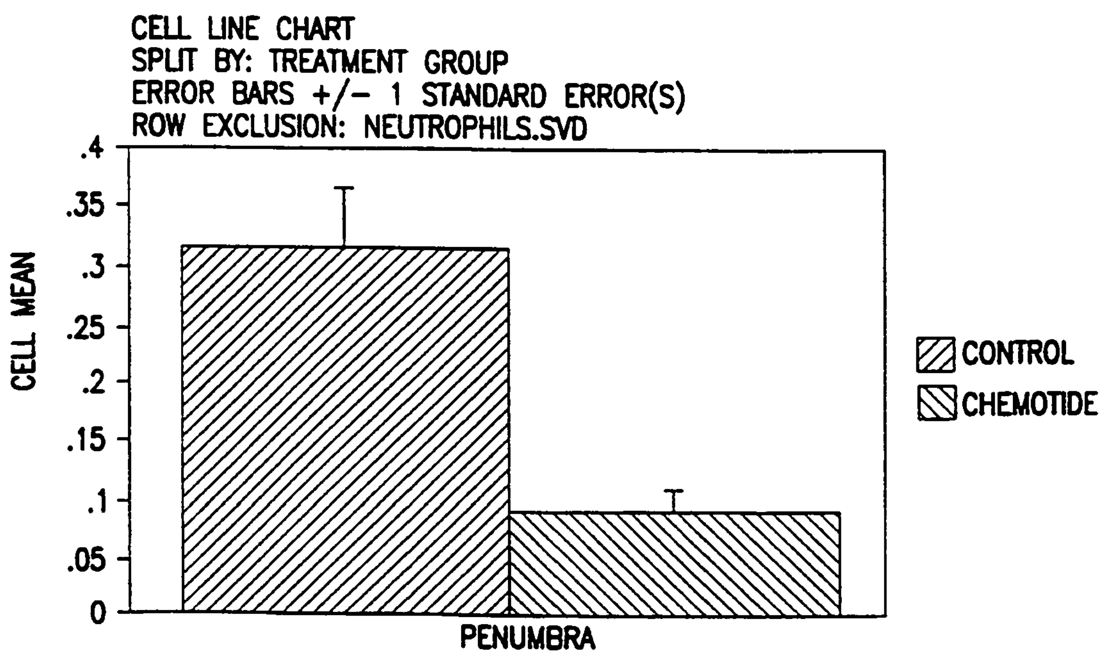


FIG. 47C

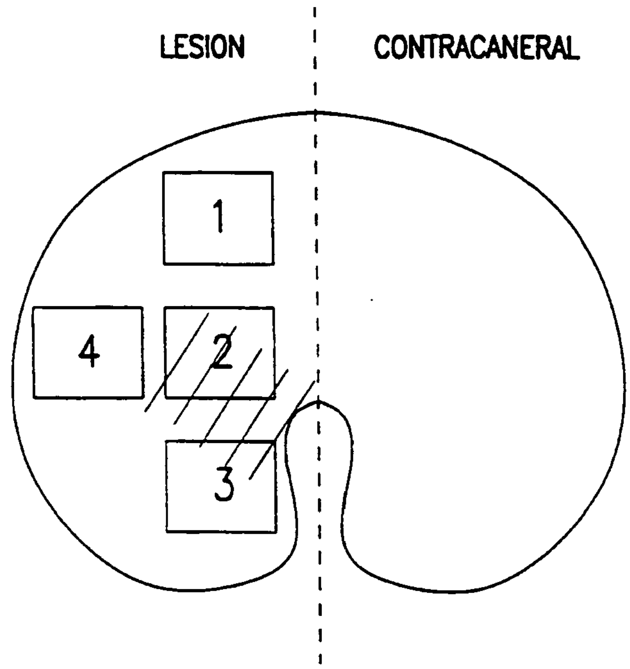


FIG. 47D

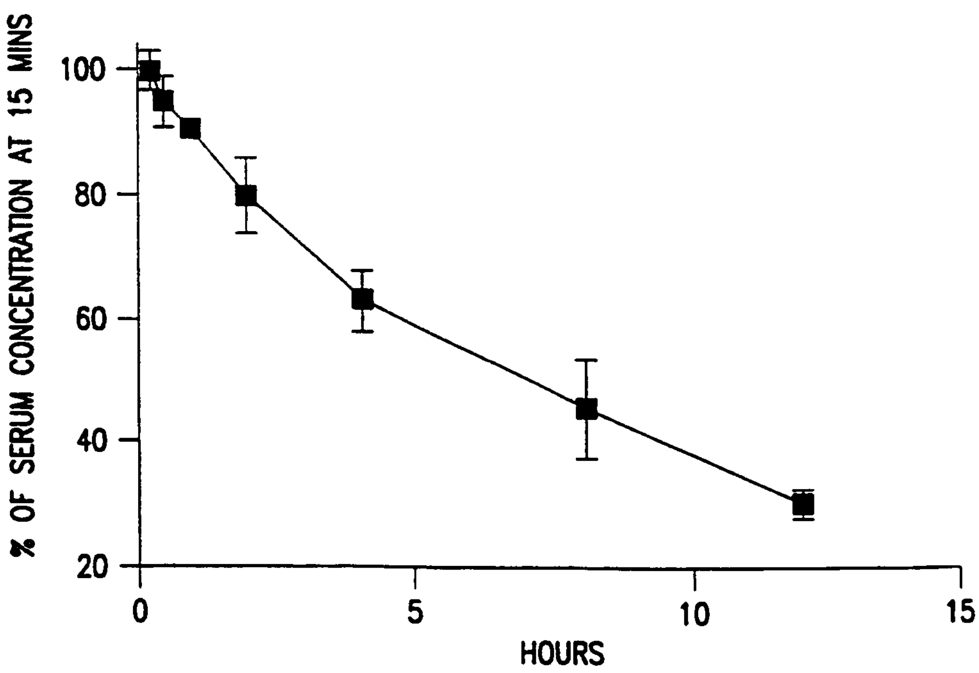


FIG. 48

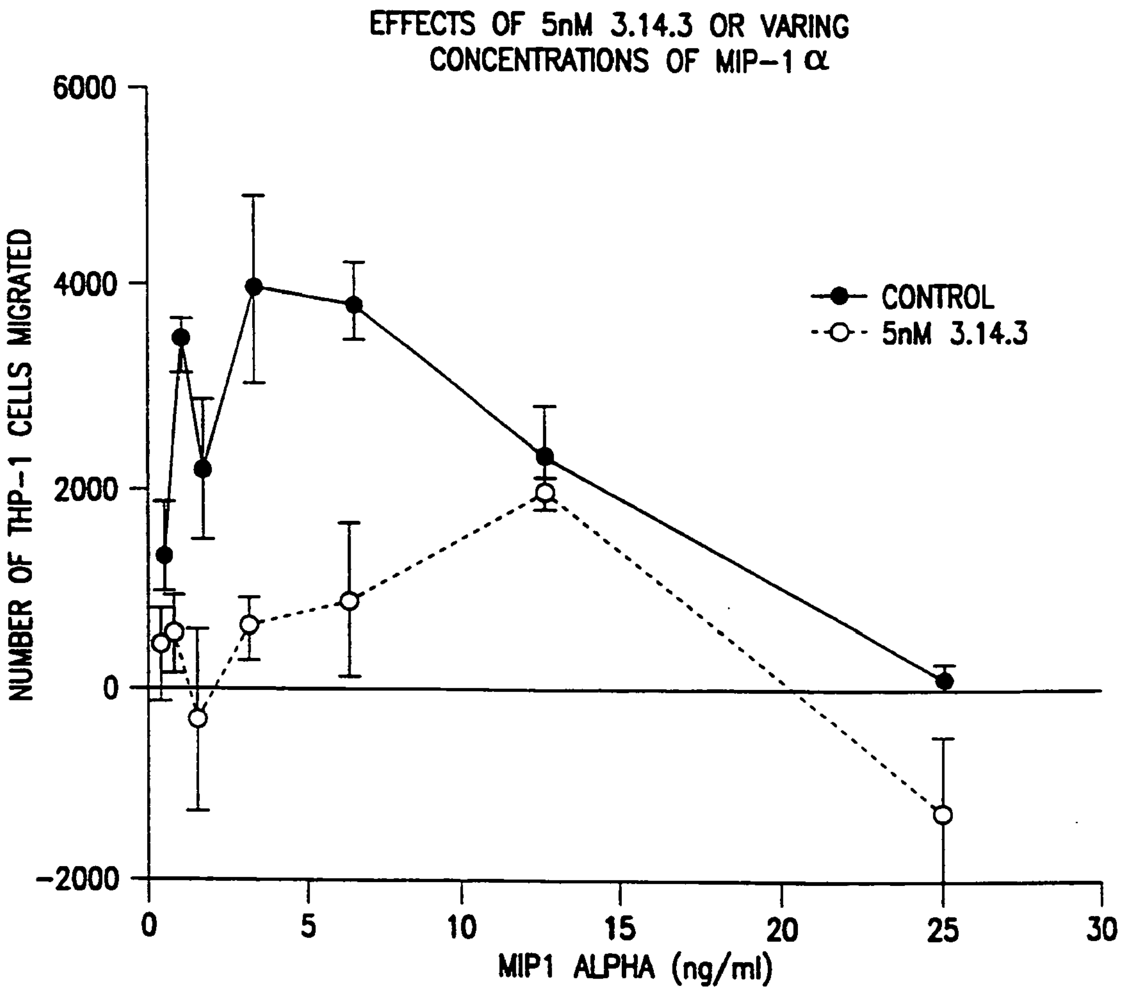
EFFECTS OF 5nM 3.14.3 OR VARING CONCENTRATIONS OF MIP-1 α
NUMBER OF THP-1 CELLS MIGRATED
6000
4000
2000
0
-2000
0
5
10
15
20
25
30
MIP1 ALPHA (ng/ml)
CONTROL
5nM 3.14.3

FIG. 49

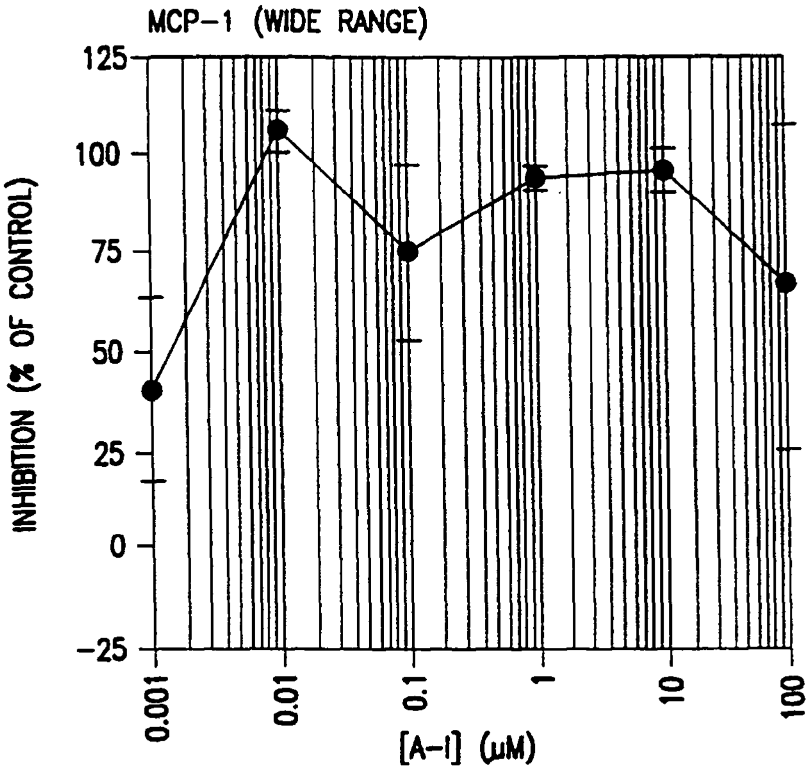


FIG. 50A

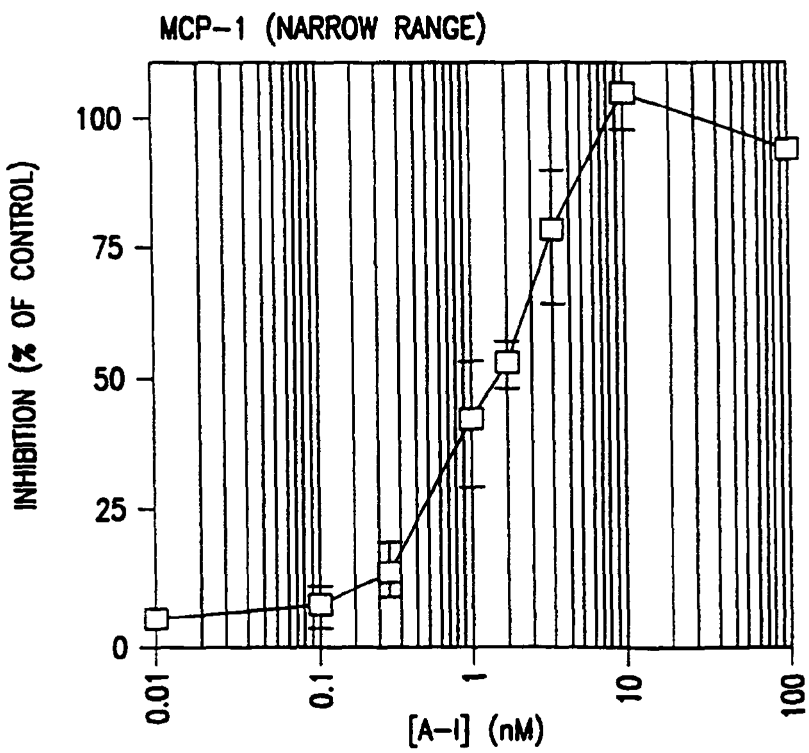


FIG. 50B

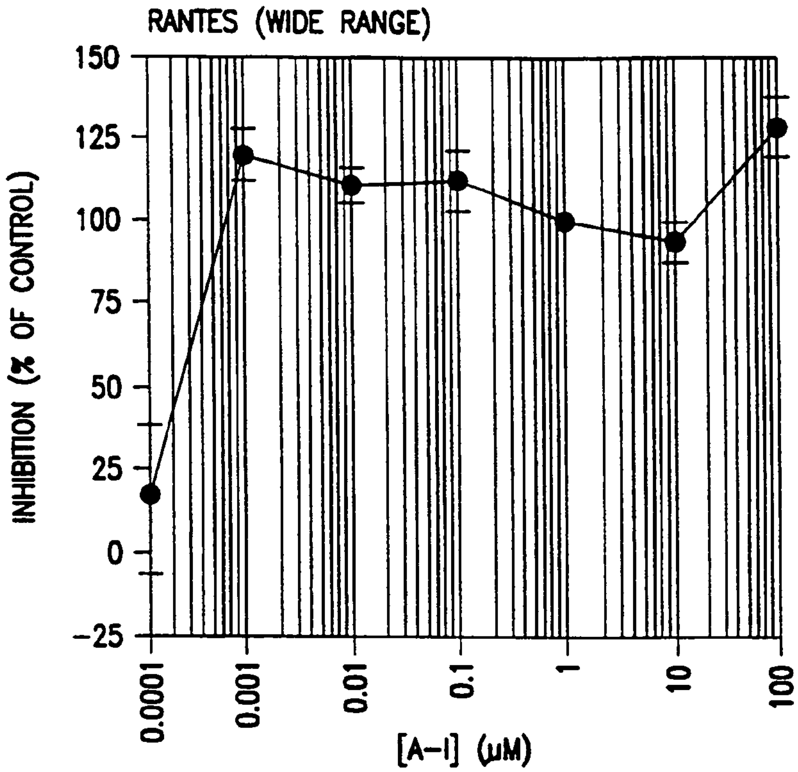


FIG. 50C

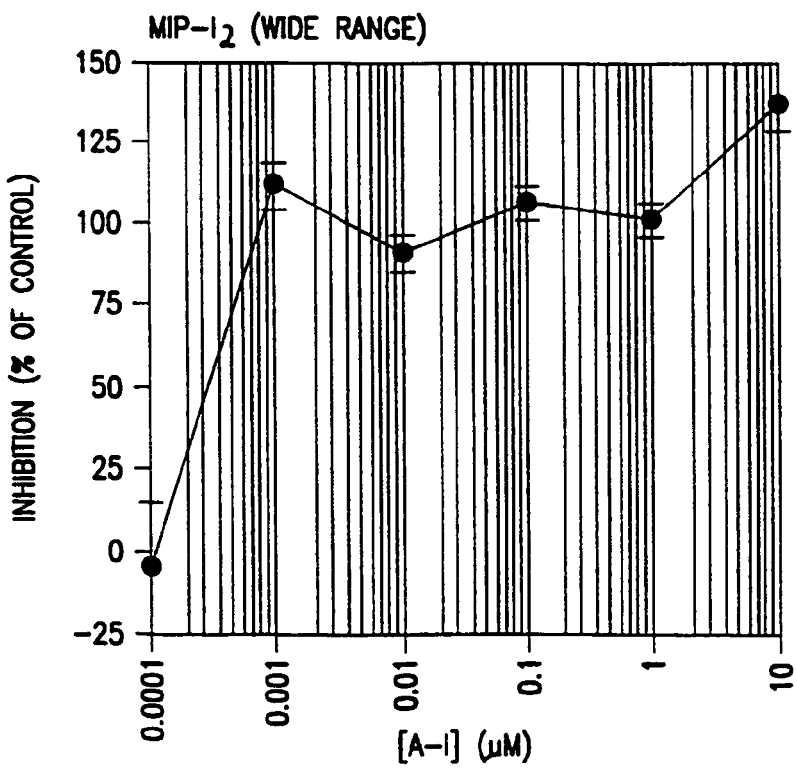


FIG. 50D

COMPETITIVE BINDING OF A-I CHEMOKINE RECEPTORS

MCP-1/CCR-Z

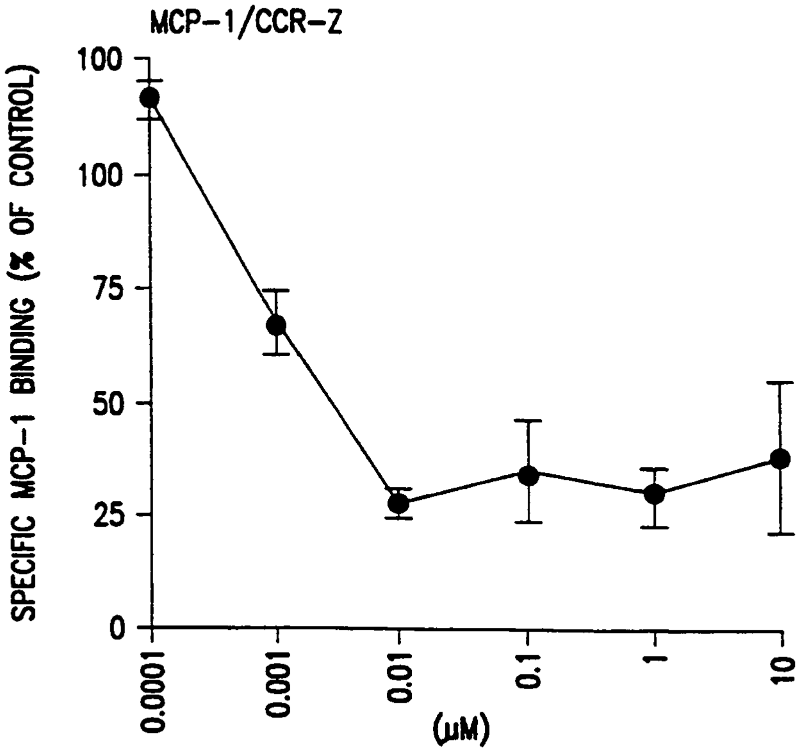


FIG. 51A

COMPETITIVE BINDING OF A-I CHEMOKINE RECEPTORS

IL-8/CCR-2

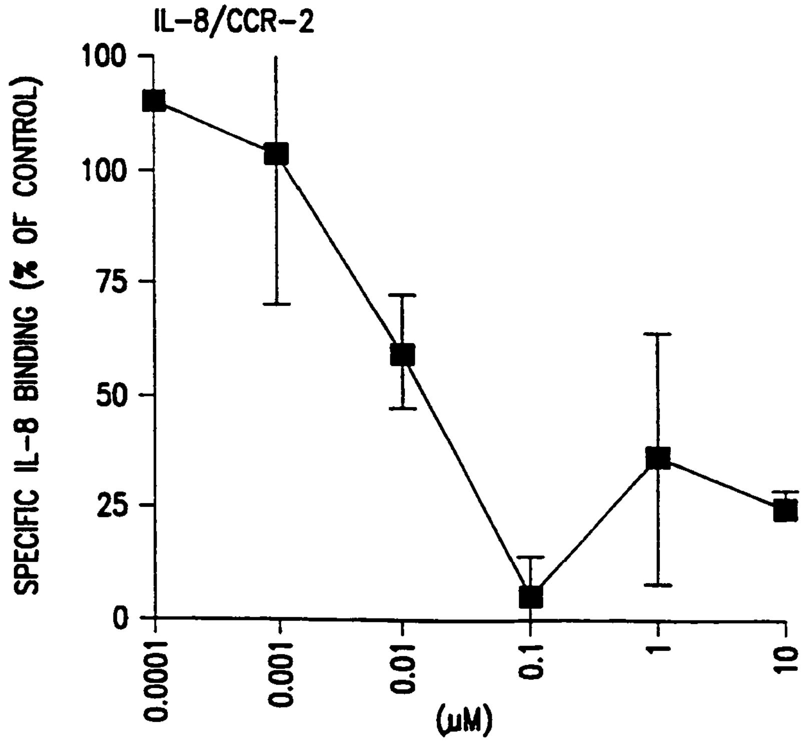


FIG. 51B

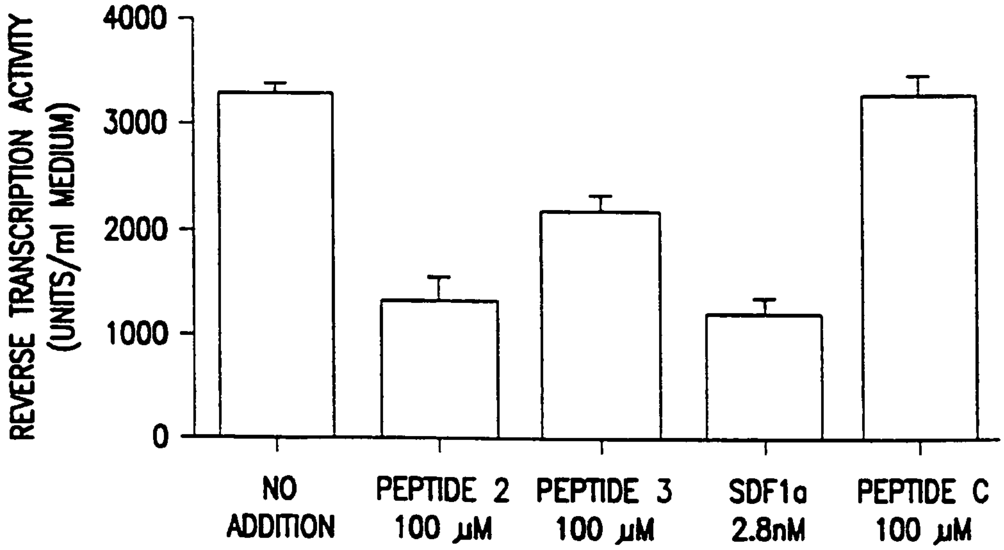
REVERSE TRANSCRIPTION ACTIVITY
(UNITS/ml MEDIUM)
4000
3000
2000
1000
0
NO ADDITION
PEPTIDE 2 100 μM
PEPTIDE 3 100 μM
SDF1α 2.8nM
PEPTIDE C 100 μM

FIG. 52

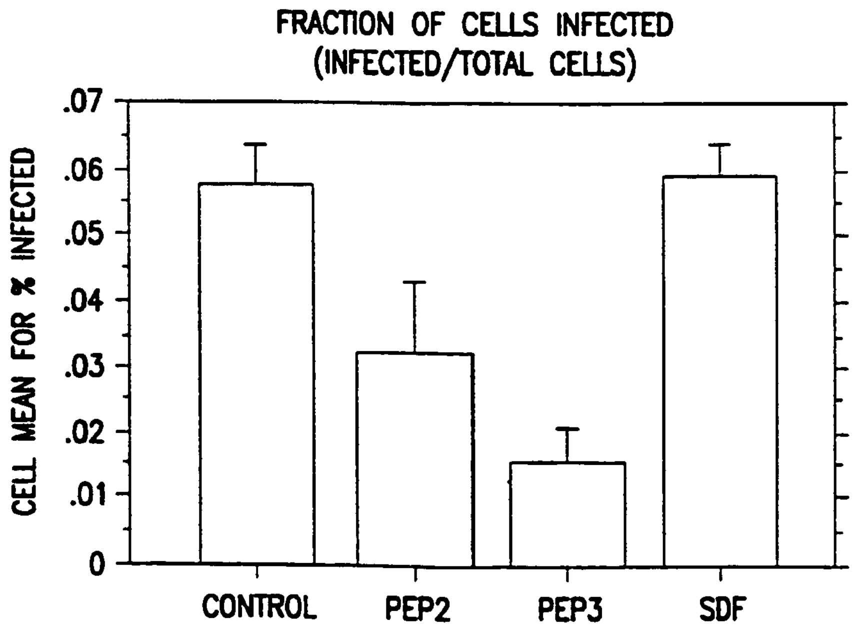
FRACTION OF CELLS INFECTED
(INFECTED/TOTAL CELLS)
CELL MEAN FOR % INFECTED
.07
.06
.05
.04
.03
.02
.01
0
CONTROL
PEP2
PEP3
SDF

FIG. 53

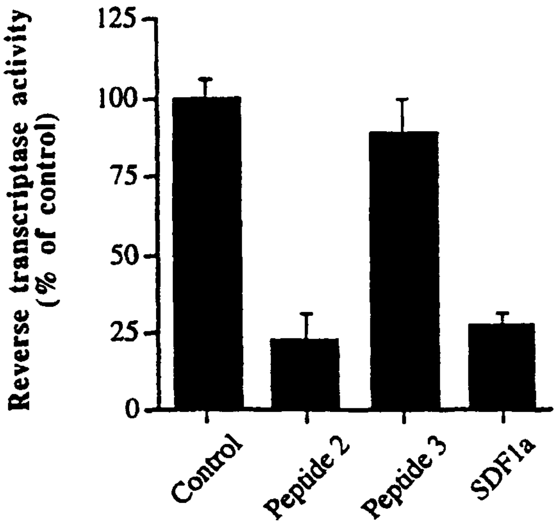


FIG. 54A

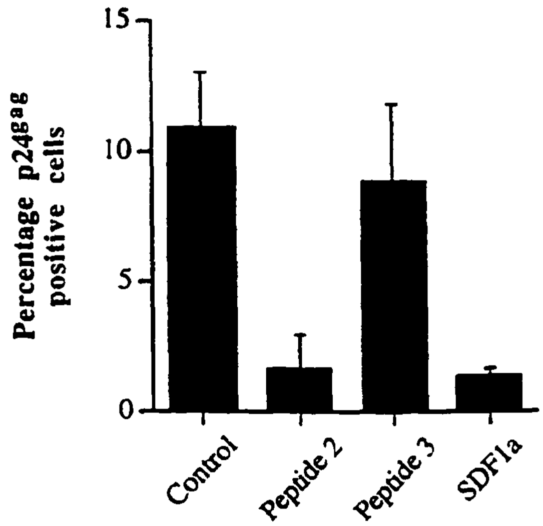


FIG. 54B

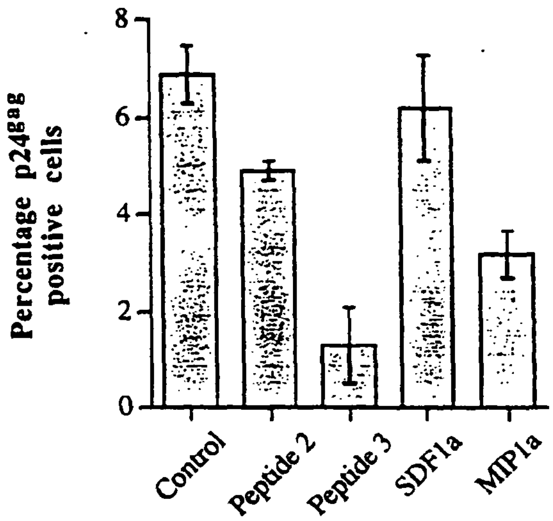


FIG. 54C

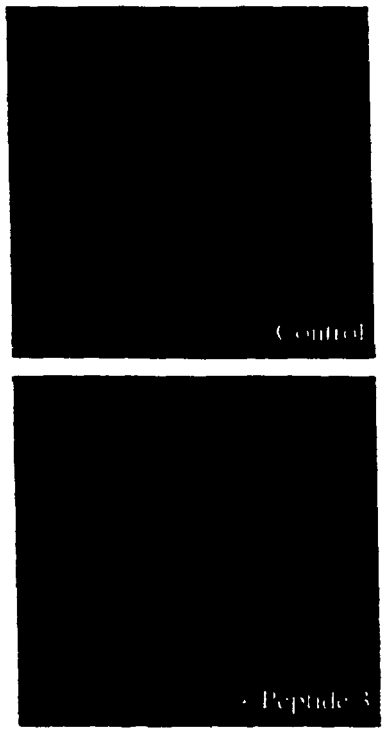


FIG. 54D

# ALKAPHORE SKELETONS

(XVI)

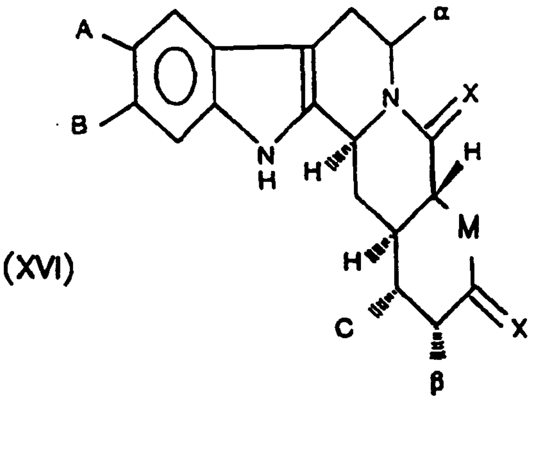

(XVII)

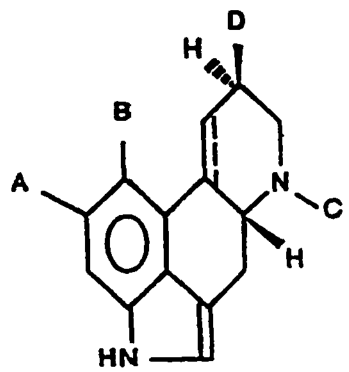

(XVIII)

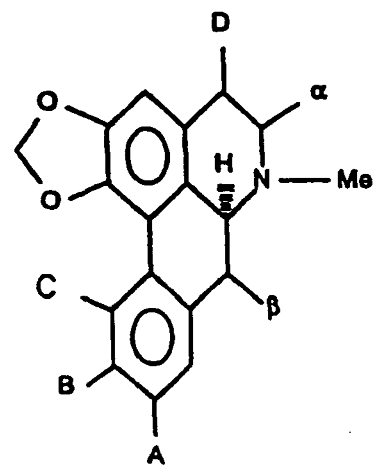

FIG. 55

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 27119299 A
- EP 00904325 A
- US 5459128 A
- US 5807482 A
- WO 9854326 A
- WO 9729192 A
- WO 9634005 A
- WO 9703995 A
- WO 9722698 A
- US 4683195 A
- US 5661132 A
- US 5848956 A
- US 4992463 A
- US 5596078 A
- US 5091396 A
- EP 45665 A
- US 4877868 A
- US 5175343 A
- US 5213787 A
- US 5120526 A
- US 5202169 A
- US 4897255 A
- US 4879225 A
- EP 0411893 A2
- US 5625126 A, N. Lonberg
- US 5545806 A
- US 5569825 A
- US 5545807 A, Surani
- US 5650150 A
- US 4863713 A
- US 5578287 A
- US 5630996 A
- US 5624896 A
- US 5616690 A
- WO 9515978 PCT
- WO 9640098 A
- US 5202118 A
- WO 9302556 A
- US 5580859 A
- US 5560922 A
- US 4140122 A
- US 4383529 A
- US 4051842 A
- US 4093709 A
- US 4675189 A


### Non-patent literature cited in the description


- **Zhang et al.** *J. Biol_ Chem.,* 1994, vol. 269, 15918
- **Zhang et al.** *Mol. Cell. Biol.,* 1995, vol. 15, 4851
- **Baxter et al.** *J.Am. Chem. Soc.,* 1990, vol. 112, 7682-7692
- **Szantay et al.** *Chem. Ber.,* 1976, vol. 109, 1737-1748
- **Toke et al.** *J. Org. Chem.,* 1993, vol. 38, 2496-250002501-2509
- **Toke et al.** *J. Org. Chem.,* 1973, vol. 38, 2501-2509
- **Toke et al.** *Chem. Ber.,* 1969, vol. 102, 3249-3259
- **Chianelli et al.** *Nucl, Med Comm,* 1997, vol. 18, 437
- **Kledal et al.** *Science,* 1997, vol. 277, 1656
- **Rollins et al.** *Blood,* 1997, vol. 90, 404
- **Dayhoff, M. O.** Atlas of Protein Sequence and Structure. National Biomedical Research Foundation, 1972, vol. 5, 101-110
- **Smith ; Waterman.** *Adv. Appl. Math.,* 1981, vol. 2, 482
- **Needleman ; Wunsch.** *J. Mol Biol.,* 1970, vol. 48, 443
- **Pearson ; Lipman.** *Proc. Natl_ Acad_ Sci. (USA.),* 1988, vol. 85, 2444
- **Kumar et al.** *Circulation,* 1997, vol. 95, 693
- **Stark et al.** *Athero., Thrombosis. and Vascular Biology,* 1997, vol. 17, 1614
- **Posner et al.** *Bone,* 1997, vol. 21, 321
- **Sozzani et al.** *J. Leukoc. Biol.,* 1997, vol. 62, 30
- **Van Setten et al.** *Pediatr. Res.,* 1998, vol. 43, 759
- **Ogata et al.** *J. Pathol.,* 1997, vol. 182, 106
- **Gong et al.** *J. Exp. Med,* 1997, vol. 186, 131
- **Zeyneloglu et al.** *Am. J. Obstet. Gynecol.,* 1998, vol. 179, 438
- **Mazzucchelli et al.** *J Pathol.,* 1996, vol. 178, 201

- **Gillitzer et al.** *Arch. Dermatol. Res.,* 1992, vol. 284, 26
- **Yu et al.** *Lab Investig.,* 1994, vol. 71, 226
- **Johnstone et al.** *J. Neuroimmunol.,* 1999, vol. 93, 182
- **Volejnikova et al.** *Am. J. Pathol),* 1997, vol. 150, 1711
- **Sugiyama et al.** *Eur. Respir. J.,* 1995, vol. 8, 1084
- **Dvorak et al.** *L Allergy Clin. Immunol.,* 1996, vol. 98, 355
- **Galli et al.** *Ciba Foundation Symposium,* 1989, vol. 147, 53
- **Gesualdo et al.** *Kidney International,* 1997, vol. 51, 155
- **Sach et al.** *Nephrol, Dial. Transplant,* 1997, vol. 12, 315
- **Zheng et al.** *Cell Biochem.,* 1998, vol. 70, 121
- **Ghirnikar et al.** *J.Neurosci Res.,* 1996, vol. 46, 727
- **Berman et al.** *J. Immunol.,* 1996, vol. 156, 3017
- **Lukacs et al.** *Adv. Immunol.,* 1996, vol. 62, 257
- **Robbins et al.** Pathologic Basis of Disease
- **Del Papa et al.** *Arthritis Rheum.,* 1996, vol. 39, 758
- **Jacobs et al.** *Am. J. Pathol.,* 1997, vol. 150, 2033
- **Iida et al.** *Thorax,* 1994, vol. 52, 431
- **Car et al.** *Am. J. Respir. Crit. Care Med.,* 1994, vol. 149, 655
- **Zisman et al.** *J._ Clin._ Invest.,* 1997, vol. 99, 2832
- **Moll.** *Biol Abs.,* 1997, vol. 104, 21765
- **Imai et al.** *J. Biol. Chem.,* 1997, vol. 272, 15036
- **Uguccioni et al.** *Eur. J. Immunol.,* 1995, vol. 25, 64
- **Loetscher et al.** *J. Exp. Med.,* 1996, vol. 184, 569
- **Weber et al.** *L Immunol.,* 1995, 4166
- **Uguccioni et al.** *Eur. J. Immunol,* 1995, vol. 25, 64
- **Pereri et al.** *J. Exp Med,* 1988, 1547
- **Clark-Lewis et al.** *J. Biol. Chem.,* 1994, vol. 269, 16075
- **Forssmann et al.** *FEBS Lett.,* 1997, vol. 408, 211
- **Sozzani et al.** *J. Leukoc. Biol.,* 1995, vol. 57, 788
- **Berkhout et al.** *J. Biol. Chem.,* 1997, vol. 272, 16404
- **Porreca et al.** *J Vasc. Res.,* 1997, vol. 34, 58
- **Sambrook et al.** Molecular Cloning: A Laboratory Manual. Cold Spring Harbor, 1989
- **Mullis et al.** *Cold Spring Harbor Symp. Quant.Biol.,* 1987, vol. 51, 263
- **Erlich.** PCR Technology. Stockton Press, 1989
- **Lawn et al.** *Nucleic Acids Res.,* 1981, vol. 9, 6103
- **Goeddel et al.** *Nucleic Acids Res.,* 1980, vol. 8, 4057
- **Adelman et al.** *DNA,* 1983, vol. 2, 183
- **Crea et al.** *Proc. Natl. Acad Sci U.S.A.,* 1978, vol. 75, 5765
- **Viera et al.** *Meth. Enzymol.,* 1987, vol. 153, 3
- **Sambrook et al.** Molecular Cloning: A Laboratory Manual. Cold Spring Harbor Laboratory Press, 1989, 4.21-4.41
- **Paigen.** *Arteriosclerosis,* 1990, vol. 10, 316
- **Lukacs.** Adv. Immunol. Academic Press, 1996, 257-304
- **Lloyd et al.** *J. Leuko. Biol.,* 1997, vol. 185, 1371
- **Tam et al.** *Kid. Int.,* 1996, vol. 49, 715
- **Volejnikova.** *Am. J.Pathol.,* 1997, vol. 150, 1711
- **Ghinikar et al.** *J. Neurosci Res.,* 1996, vol. 46, 727
- **Ransoholf et al.** *J. Leuko Biol.,* 1997, vol. 62, 645
- **Kaul et al.** *Am. J. Trop. Med. Hyg.,* 1995, vol. 58, 240
- **Ajeubar et al.** *J. Leuko. Biol.,* 1998, vol. 63, 108
- **Furukawa et al.** *Lupus,* 1997, vol. 6, 193
- **Suzuki et al.** *J. Heart & Lung Transpl.,* 1967, vol. 16, 1141
- **Abbott et al.** *Arch Surg.,* 1964, vol. 89, 645
- **Corry et al.** *Transpl.,* 1973, vol. 16, 343
- **Dworkin et al.** *J. Heart Lung Transpl.,* 1991, vol. 10, 591
- **Laden et al.** *Arch. Path.,* 1972, vol. 93, 240
- **Mitchell et al.** *Transpl.,* 1990, vol. 49, 835
- **Burhardt et al.** *Rheum. Int.,* 1997, vol. 17, 91
- **Elson et al.** *Gastroenter.,* 1998, vol. 109, 1344
- **Hayes et al.** *Arterio. Thromb. Vasc. Biol.,* 1998, vol. 18, 397
- **Wang et al.** *Arterio. Thromb.,* 1991, vol. 11, 1166
- **Wegner et al.** *Science,* 1990, vol. 247, 456
- **Brahn.** *Ciinorth and Rel. Res.,* 1991, vol. 265, 42
- **Wooley.** *Curr.Op. Rheum.,* 1991, vol. 3, 407
- **Gay et al.** *Curr. Op. Rheum.,* 1995, vol. 7, 199
- **Beamer et al.** *Blood,* 1998, vol. 86, 3220
- **Nakaguma.** *Int. 1. Exp. Path.,* 1998, vol. 76, 65
- **Nanney et al.** *J. Invest. Dermat.,* 1996, vol. 106, 1169
- **Nickoff et al.** *AJP,* 1995, vol. 146, 580
- **Sundberg et al.** *Pathobiol.,* 1997, vol. 65, 271
- **Wolf et al.** *Int J. Dermat.,* 1998, vol. 30, 448

- **Conti et al.** *Blood,* 1997, vol. 89, 4120
- **Gonzalo et al.** *JCI,* 1996, vol. 98, 2332
- **Teiyeira et al.** *JCI,* 1997, vol. 100, 1657
- **Ceri et al.** *Allergy,* 1997, vol. 52, 739
- **Freed.** *Eur. Res. J.,* 1998, vol. 8, 1770
- **Griffiths-Johnson et al.** *Meth. Enzy.,* 1991, vol. 288, 241
- **Herz et al.** New Horizons in Allergy Immunoth. Plenum Press, 1996, 25-32
- **Kane.** *Eur. Resp. J.,* 1991, vol. 7, 555
- **J. Sambrook et al.** Molecular Cloning- A Laboratory Manual. Cold Spring Harbor Laboratory Press, 1989
- **Stewart et al.** Solid Phase Peptide Synthesis. W. H. Freeman Co, 1969
- **Merrifield.** *J. Am. Chem. Soc,* 1963, vol. 85, 2149
- **Meienhofer.** Hormonal Proteins and Peptides. Academic Press, 1973, vol. 2, 48-267
- **Bavaay ; Merrifield.** The Peptides. Academic Press, 1980, vol. 2, 3-285
- **Clark-Lewis et al.** *Meth Enzymol.,* 1997, vol. 231, 233
- **Simmons et al.** *Science,* 1997, vol. 276, 276
- **Jameson et al.** *Nature,* 1994, vol. 368, 744
- **Fauchere, J.** *Adv. Dru Res.,* 1986, vol. 15, 29
- **Veber ; Freidinger.** *TINS,* 1985, 392
- **Evans et al.** *J. Med. Chem,* 1987, vol. 30, 1229
- **Spatola, A. F.** Chemistry and Biochemistry of Amino Acids, Peptides, and Proteins. Marcel Dekker, 1983, 267
- **Spatola, A. F.** Peptide Backbone Modifications. *Vega Data,* March 1983, vol. 1 (3
- **Morley, J. S.** *Trends Pharm. Sci.,* 1980, 463-468
- **Hudson, D. et al.** *Int. J. Pept. Prot. Res.,* 1979, vol. 14, 177-185
- **Spatola, A. F. et al.** *Life Sci,* 1986, vol. 38, 1243-1249
- **Hann, M. M.** *J. Chem. Soc. Perkin Trans,* 1982, vol. I, 307-314
- **Almquist, R. G. et al.** *J. Med. Chem.,* 1980, vol. 23, 1392-1398
- **Jennings-White, C. et al.** *Tetrahedron Lett,* 1982, vol. 23, 2533
- **Holladay, M. W. et al.** *Tetrahedron Lett.,* 1983, vol. 24, 4401-4404
- **Hruby, V. J.** *Life Sci.,* 1982, vol. 31, 189-199
- **House et al.** *J. Org. Chem.,* 1975, vol. 40, 1460
- **House et al.** *Org.Chem.,* vol. 31, 3128
- **House et al.** *J. Org. Chem.,* 1971, vol. 36, 2361
- **Rubottom.** *J. Org. Chem.,* 1979, vol. 44, 1731
- **Green et al.** *Adv. Pest control Res.,* 1960, vol. 3, 129
- **Eschenmoser ; Winter.** *Science,* 1977, vol. 196, 1410
- **Lygo ; Rudd.** *Tetrahedron Lett.,* 1995, vol. 36, 3577
- **Yuan ; Hruby.** *Tetrahedron Lett.,* 1997, vol. 38, 3853
- **Hayashi et al.** *Chemistry Letters,* 1980, 935-938
- **Visweswariah et al.** *Synthesis,* 1982, 309-310
- *Chemistry Letters,* 1980, 935-940
- **Gillies et al.** *L Immunol. Meth.,* 1989, vol. 125, 191
- **Gillies et al.** *Biotechnol,* 1989, vol. 2, 798-804
- **Gillies et al.** *J. Immunol Methol.,* 1989, vol. 125, 191
- **Varki et al.** *Cancer Res.,* 1984, vol. 44, 681
- **Okabe et al.** *Cancer Res,* 1989, vol. 44, 5273
- Monoclonal Antibody Therapy to Cancer: Preclinical Models and Investigations. **Morgan ; Foon.** Basic and Clinical Tumor Immunology. Kluwer Academic Publishers, vol. 2
- **Uhr.** *J. of Immunol.,* 1984, vol. 133, i-vii
- **Boring et al.** *Nature,* 1998, vol. 394, 894
- **Doranz et al.** *Cell,* 1996, vol. 85, 1149
- **Ross et al.** *Proc Natl. Acad Sci USA,* 1998, vol. 95, 7682
- **Cairns et al.** *Nat. Med.,* 1998, vol. 4, 563
- **Bischoff et al.** *J. Exp. Med.,* 1992, vol. 175 (5), 1271
- **Zisman et al.** *J. Clin. Invest.,* 1997, vol. 99, 2832
- **Kumar et al.** *Circulation,* 1994, vol. 90, 1427
- **Gong et al.** *J. Exp Med.,* 1997, vol. 186, 131
- **Plater-Zyberk et al.** *Immune. Lett.,* 1997, vol. 57, 117
- **Wilder.** *Clin- Rheumat.,* 1996, vol. 10, 259
- **Pardoll et al.** *Immunity,* 1995, vol. 3, 165
- **Stevenson et al.** *Immunol. Rev.,* 1995, vol. 145, 211
- **Molling.** *J. Mol, Med.,* 1997, vol. 75, 242
- **Donnelly et al.** *Ann. N.Y. Acad. Sci.,* 1995, vol. 772, 40
- **Yang et al.** *Mol. Med. Today,* 1996, vol. 2, 476
- **Abdallah et al.** *Biol. Cell,* 1995, vol. 85, 1
- *Polymer Letters,* 1980, vol. 18, 293
- **Cowsar et al.** Poly(Lactide-Co-Glycolide) Microcapsules for Controlled Release of Steroids. *Methods Enzymology,* 1985, vol. 112, 101-116
- **Eldridge et al.** Biodegradable and Biocompatible Poly(DL-Lactide-Co-Glycolide) Microspheres as an Adjuvant for Staphylococcal Enterotoxin B Toxoid Which Enhances the Level of Toxin-Neutralizing Antibodies. *Infection and Immunity,* 1991, vol. 52, 2978-2986

- **Cohen et al.** Controlled Delivery Systems for Proteins Based on Poly(Lactic/Glycolic Acid) Microspheres. *Pharmaceutical Research,* 1991, vol. 8 (6), 713-720
- **Sanders et al.** Controlled Release of a Luteinizing Hormone-Releasing Hormone Analogue from Poly(D,L-Lactide-Co-Glycolide) Microspheres. *J. Pharmaceutical Science,* 1984, vol. 73 (9), 1294-1297
- **Tice et al.** Biodegradable Controlled-Release Parenteral Systems. *Pharmaceutical Technology,* 1984, 26-35
- **Beck et al.** Poly(DL-Lactide-Co-Glycolide)/Norethisterone Microcapsules: An Injectable Biodegradable Contraceptive. *Biol. Reprod.,* 1983, vol. 28, 186-195
- **Gonzalo et al.** *J.Clin. Invest.,* 1996, vol. 98, 2332
- **Gonzalo et al.** *J. Exp. Med.,* 1998, vol. 188, 157
- *Clin. Chim. Acta,* 1995, vol. 235, 11
- **Schultz-Cherry et al.** *J. Biol. Chem.,* 1995, vol. 270, 7304
- **Ruffing et al.** *Cell. Immunol.,* 1998, vol. 189, 160
- **Carrascal et al.** *J. Pharm. Bio. Ana.,* 1998, vol. 17, 1129
- **Clarke et al.** *FEBS Lett,* 1998, vol. 430, 419
- **March.** Advanced Organic Chemistry. Reactions, Mechanism, and Structure. John Wiley & Sons, 1992, 94-116
- **Hoogerwerf et al.** *Biochemistry,* 1997, vol. 36, 13570
- **Hesselgesser et al.** *J. Biol. Chem.,* 1998, vol. 273, 15687
- *J. Histochem Cytochem.,* 1997, vol. 44, 1043
- *J. Histochem. Cytochem.,* 1997, vol. 44, 1043
- **Wells et al.** *Methods,* 1996, vol. 10, 126
- **Ross et al.** *J Virol.,* 1998, vol. 72, 1918
- **Alkhatib et al.** *J. Biol. Chem,* 1997, vol. 272, 19771
- **Dragic et al.** *J. Virol.,* 1998, vol. 72, 279
- **Monteclaro et al.** *J. Biol. Chem.,* 1997, vol. 272, 23186
- **Ross et al.** *J. Virol.,* 1998, vol. 72, 1918
- **Farzan et al.** *J. Biol. Chem.,* 1997, vol. 212, 6854
- **Ferns et al.** *L Gen. Virol.,* 1987, vol. 68, 1543
- **Ross et al.** *Proc. Nat'l Acad. Sci. U.S.A.,* 1998, vol. 95, 7682
- **Cocchi et al.** *Nat. Med.,* 1996, vol. 2, 1244
- **Jiang et al.** *Nat. Med.,* 1997, vol. 3, 367
- **Mosedale et al.** *J. Histochem. Cytochem.,* 1996, vol. 44, 1043
- **Simmons et al.** *Science,* 1997, vol. 226, 276
- **Lusti-Narasimhan et al.** *J. Biol. Chem.,* 1996, vol. 211, 3148
- **Lusti-Narasimhan et al.** *J.Biol. Chem.,* 1995, vol. 270, 2716
- **Lusti-Narasimhan et al.** *J. Biol. Chem.,* 1996, vol. 271, 3148
- **Lusti-Narasimahn et al.** *L Biol. Chem.,* 1995, vol. 270, 2716
- **Cocchi et al.** *Science,* 1995, vol. 270, 1811
- **Zaguny et al.** *Proc. Nat'l Acad. Sci U.S.A.,* 1998, vol. 95, 3857
- **Ross et al.** *Proc.Nat'l Acad. Sci. U.S.A.,* 1998, vol. 95, 7682
- **Mack et al.** *L Exp. Med.,* 1998, vol. 187, 1215